# EUROPEAN PATENT SPECIFICATION

(11) **EP 2 970 363 B1**
(45) Date of publication and mention of the grant of the patent: **08.07.2020**
(21) Application number: 14767960.9
(22) Date of filing: 14.03.2014
(51) Int. Cl.: C07H 21/02, C07H 21/04, C12N 5/04, C12N 5/10, C12N 15/00

(54) **COMPOSITIONS AND METHODS TO CONTROL INSECT PESTS**
ZUSAMMENSETZUNGEN UND VERFAHREN ZUR INSEKTENSCHÄDLINGSBEKÄMPFUNG
COMPOSITIONS ET PROCÉDÉS POUR CONTRÔLER DES INSECTES RAVAGEURS

(30) Priority: 14.03.2013 US 201361785680 P; 14.03.2013 US 201313831230
(43) Date of publication of application: 20.01.2016
(60) Divisional application: 20174779.7
(73) Proprietor: Pioneer Hi-Bred International, Inc., Johnston, IA 50131-1014 (US); E. I. du Pont de Nemours and Company, Wilmington, DE 19805 (US)
(72) Inventor: HU, Xu, Johnston, IA 50131 (US); PRESNAIL, James, Saînt Louis, MO 63108 (US); RICHTMAN, Nina, Granger, IA 50109 (US); DIEHN, Scott, West Des Moines, IA 50265 (US); VAN ALLEN, Michelle, Urbandale, IA 50322 (US); PROCYK, Lisa, Ankeny, IA 50023 (US)
(74) Representative: J A Kemp LLP
(86) International application number: PCT/US2014/029805
(87) International publication number: WO 2014/153254

(56) References cited:
- WO-A1-2011/025860
- US-A1- 2012 164 205
- DATABASE GENBANK [Online] 08 February 2011 'Diabrotica virgifera virgifera midguts cDNA', XP055286628 Retrieved from NCBI Database accession no. CN497524.1

### SEQUENCE LISTING

<110> Pioneer Hi-Bred International Diehn, Scott Harnack, Michelle Hu, Xu Presnail, James Richtman, Nina Procyk, Lisa
<120> Novel RNAi Gene Targets from the Western Corn Rootworm Diabrotica virgifera
<130> 36446.0007P1
<150> 61/785,680
   <151> 2013-03-14
<160> 731
<170> PatentIn version 3.5
<210> 1
   <211> 593
   <212> DNA
   <213> Diabrotica virgifera
<220>
   <221> misc_feature
   <222> (1)..(593)
   <223> RNAi target sequence
<220>
   <221> misc_feature
   <222> (480)..(480)
   <223> n can be any nucleotide
<220>
   <221> misc_feature
   <222> (495)..(495)
   <223> n can be any nucleotide
<400> 1
   tgacttcaca cttccagatg gttctagagc agcaatagtg aataacataa atgacatgga      60
   aagggtttac aatgattttg gttccaaatg gatgttggag gatgtgttag atcctcaaaa     120
   gggtagagca ttgttccaca gggaattcgg aaggacttca gcgacttaca acaacaaaac     180
   gttcaagcct caattcctaa tgaatccgga agatttccta ccaaccaaca gatctacaga     240
   tcttaagcga atatccgaaa tatgtcctct aaagttgtac gaatgttact acgattacgc     300
   tatgacggtt gacagggatt tggcgcatta caccaagaat tataaagcca cgatctatca     360
   atataaagaa acgacaagga ggaaagttgt gtcttgcgga gtactagaaa caccgagatt     420
   cggacgaaag agtactttcc tgttcgtgcc cggtacgaag gttacctatg aatgcatccn     480
   agagttcgtg ttggntaggt gatcagcgga gagagtgtca agcggatgga acatggaata     540
   ttcctgaata cggatacaca tattgcttac gtcagcagga atactctcaa cga            593
<210> 2
   <211> 22
   <212> DNA
   <213> Artificial Sequence
<220>
   <223> primer
<220>
   <221> misc_feature
   <222> (1)..(22)
   <223> forward primer
<400> 2
   tgacttcaca cttccagatg gt 22
<210> 3
   <211> 22
   <212> DNA
   <213> Artificial Sequence
<220>
   <223> primer
<220>
   <221> misc_feature
   <222> (1)..(22)
   <223> reverse primer
<400> 3
   tcgttgagag tattcctgct ga 22
<210> 4
   <211> 5689
   <212> DNA
   <213> Diabrotica virgifera
<220>
   <221> misc_feature
   <222> (1)..(5689)
   <223> WCRW transcript ta0120.010_diavv
<400> 4
   tgttcatcac caacgctgac caacgtgaga gtagcaactt tttttgatgt gatgtgcgcc 60
   agtttgtagt gccaaataac caaaatgtgg ttcaagtact ttgttatttt tgttttgttt 120
   agtgttagcg ttaaaggact agatggtgat cttccaccag atgtcgaaac tatcgatgtg 180
   gagactacga atgtaagtaa cgcggatcca gacgcaggga aacctattcc ggtggaagat 240
   acgcctaatg gagtccctcc agtgaataca aactatgaag tagataaatc cttagttaat 300
   gtaaccaata gtttggtaaa tgtgactaaa aatcccagac ggacttatca gacagttaat 360
   aaaatgggca aatattcgac attttatgct gcagattacg atcctatgac ctcagacaca 420
   gctccaccag acactgacca acgaggagga ggaggaactc catacactat atcagaaccg 480
   agactggcag aaataaggaa acattttatg tacccatttt tcgataaagg aggcagtgat 540
   aataacttgg gagatttcca aaaggacatt caatcttcca ttcctcaggt gcacaagaat 600
   ttaaacttcc agttgccttt ctttggattc aggtataatt acacaagagt ttcagtgaat 660
   gggtatctgg aattcagcga tccacctcca aattatgact atccattagt atttccaatt 720
   aaagaatggc ccaaaaagaa tgatcctgcc tttattggca ttttcttcag cagatgcaga 780
   atcggtaatc tacgtgatgg agacattgat caaagaacac ccggggtcta ttttagaatg 840
   gaaagagatt taagaacgag gcaagacaga atgggtgtag aaattagaga aagacttaag 900
   tgggatatca gaacaggagt cattggatca gaaacattcg atcctaagca cgccgttatt 960
   gtcacttgga aaaatgttac tttcactgga ggattcgcta atgctaaata caagacaaac 1020
   accttccaaa tggttcttgc aactgacgaa gtcttcactt acgcgatgtt caactacctc 1080
   aacttggact ggacctccca cactgaagcg ggtggtgatt cccaaaacgg ggaaggtggt 1140
   gtcagtgcct atgttggttt taacgctgga aacggtacca gaagctacga atacaatcct 1200
   tacagtcaag cttccgtcat tcgagatctc acatcagttg gatttggaaa tggttttaag 1260
   ggaagacata ttttccgtat agacgaagac attttgcttg gatcgtgcaa caaagatata 1320
   gatggtgcca accttcccct gaaattcgct cctgaaagtg gtaacatgtt gggtggaaca 1380
   gtggtaaata taactggtcc atgcttcaat ttgaatgata ggattcgatg caagtttgac 1440
   gtatcaaatg aagtatttgg gtatgttgtt gataaaaaca gagctatctg tgtacaacct 1500
   caactttatg ccgaaggatg ggtgaatctt caaattgctg ttaattccga agcattcaag 1560
   tggaagggga agtattatgt agaatcacct gctagtgcga ctcaaaaaat cttctttaag 1620
   gacatgaaat tacacgaaaa atctccgagc gaattaaaaa tcacttggga gaagcagaat 1680
   ttgaccacaa acgacaacgc taacatccga atttctctct ggggttacag agaaacgact 1740
   atgaaaccag tgtttgttta tatcaccgat atcgctgata atgttcaaaa cattggagaa 1800
   cacacgattg ttccttctca gtttagaact agagtcaatc aatatctgac tgatattaag 1860
   tttggattcc tccaaattaa tttaactgag tctatcacag ttaatacata tactacaagc 1920
   cagagcagca tagaattaac accggtaata tggagccgac ccattccact cggttggtac 1980
   ttccagttcc aatgggaaaa ccagtacggt aaaaattggc ccaaatatct atgtgacgat 2040
   tggctgagaa cggacagata tctaaaaaac ttcgcccacg aacttgctca atgtccctgc 2100
   accgtcgaac aggctttagc tgacaagggc aggtttatgc cagacttcga ttgcgacaaa 2160
   gattcgaacc cgatttgtta ctataacaac caagcacttc attgtgtgaa aactggatct 2220
   cccactttag aaggctccga acaacaatgt tgctatgata aaaacggctt tttgatgctc 2280
   tcttacgatc agcaatgggg ttctagtccg agacgttgcc acaacttggg taaaatgcct 2340
   tacaatgaag cgacaaaagt gcctactctc tcacaatggt ttaatgacat ggtacccaaa 2400
   tatttgtgtt gtctttggca agacgagcaa gctgtgggat gtgaaacact gagatttgaa 2460
   cggagaccta cacaggattg tgtagcttac caagcaccag gtgtagcagg agtctacggt 2520
   gatccacact ttgttacttt cgacgatgta gaatatacgt ttaatggaaa gggagaattc 2580
   gcattggtga aatcagttac gcaaacggac aatctggaaa tccaaggaag atttgagcaa 2640
   atggacccta acacttatgg tgaggttaga gctactcagc tgacttctgt agtagctaaa 2700
   ggtaataaca caatagcaat tgaagtcaga aggagacctc tggattctag atggagatat 2760
   aggttggatg ttatagcaga taatagaaga ctttattttg atagaccctc actaaaattc 2820
   cagcacttcc aaggtgttac tatttacact ccaagctaca tccttaatca atcagaagtc 2880
   atcattatgt ttgacaatgg agctggaatg gaggttgtcg acaatcaagg attcatgagt 2940
   gcaagagtat ttttgccttg gtcgtttata aataaaacca taggcctttt tggaaattgg 3000
   agttttaaca aagaagatga cttcacactt ccagatggtt ctagagcagc aatagtgaat 3060
   aacataaatg acatggaaag ggtttacaat gattttggtt ccaaatggat gttggaggat 3120
   gtgttagatc ctcaaaaggg tagagcattg ttccacaggg aattcggaag aacttcagcg 3180
   acttacaaca acaaaacgtt caagcctcaa ttcctaatga atccggaaga tttcctacca 3240
   accaacagat ctacagatct taagcgaata tccgaaatat gtcctctaaa gttgtacgaa 3300
   tgttactacg attacgctat gacggttgac agggatttgg cgcattacac caagaattat 3360
   aaagccacga tctatcaata taaagaaacg acaaggagga aagttgtgtc ttgcggagta 3420
   ctagaaacac cgagattcgg acgaaagagt actttcctgt tcgtgcccgg tacgaaggtt 3480
   acctatgaat gcatccaaga gttcgtgttg gtaggtgatc agcggagaga gtgtcaagcg 3540
   gatggaacat ggaatattcc tgaatacgga tacacatatt gcttacgtca gcaggaatac 3600
   tctcaacgac aagctggttt ggcttccggg ataattatgg ctattcttat cccgctcatc 3660
   ttgcttttcg tctacctggc gtatcgtttc ttacaaagaa ggcagaaaga aaaagaatct 3720
   gaaaaattac aagagcaaca gttcatggaa tcacaaagga gagcccaaga agcagctact 3780
   aagaaattaa cagaagctga atatgcttca gaagatgaag acagtaatgt tacaagcacg 3840
   ggagcaaaag aaacaaccgt atactaacac ctatagaata taatgattca acctatgaat 3900
   tgtaatattt taaagtttaa tctttgtcaa aactattata taaaataagt actatattat 3960
   gttgtagtta atatttaata ggacttttat tttttattgt atttcagatt atatttcatg 4020
   cagagtgtga tttagattat atttcatgca aagtgtagag tttcagatat agccttttta 4080
   accacaacgt aatccgattc ttatagtaaa ttcaacatta tttaagatac tttttcattg 4140
   aattgtatac ctatctaatc ctgcaagacc cacatatctt tttcccaatt aaaagagtaa 4200
   ataggaagat acgaaagcac aaagttaaaa taaattaacg aatttcataa aacccagtag 4260
   aaagctatgt tctgcagtta ttatctaaat tagaatacta ttaagtaacc atatcaccct 4320
   tctcaagttt acattaacat caagatctac tggtggcatc cataggtagg tgcctactcg 4380
   tatatttagt aaacaacagt ttgttgatcc atcaacttct tgttggtaag attcagctca 4440
   acgttgaggg caaccggccc ttcttcttaa agttccatct tctatcagag gttggatatc 4500
   ataatgggtt tggtcactat gttggctgct gctctgaata gttgtaataa actacagtta 4560
   aaccattatc taaggttctt tagccaggag atgcgtcttc ttccttggat ccttccttgc 4620
   ataataattc tcagcaactc atatttttct cctctggtaa tgtgacccaa atattccagt 4680
   tttctatgtt tttatactgt tcataatttc taactcctta tttaaacgtc gtagcacttc 4740
   aacattggta atcctttgaa cccactgaat tttcaatatt cttctgtaac accacatttc 4800
   gaacgcttct atttttctta tgtgttgtct cttcaacgtc caagcttcca ttccgtatag 4860
   taatatagaa aatatgtagc atcttagagc cctcaatctg agaggcaact gaaggtcttt 4920
   gtttgtaagc agtgtcttca tttataaatg cttgccttgc agtttcaatt cggactttaa 4980
   tttctttgct ttggtcattt ttgtcatcaa cccaggttcc cagatattta tatgtctaac 5040
   tttttctatt tgggtttgct ctctatcatt aacctttcat ttccatgttg tgtttttgag 5100
   acaatcataa atttagtttt tttcttgttt atttttagtc cgtatcgaat gcaataatcg 5160
   ttaattctat ttagcaattc ttgtagcgat cccagggtgt ctgccattat aaggatgtca 5220
   tcagcgaatc ttgtgttatt tactattttt ccgtttatag agattctatc acttagctcc 5280
   gctatcgctt cctgaaatat ggcttcactg tacacgttaa acagtagcgg cgacagaaca 5340
   caaccctgtc ttactcctct ctttatatca atattctcgg attcttgttc ttctatcttt 5400
   atattggcct tttgattcca atacagattg atgataattc gcaaatctcg taaaaaagca 5460
   gctctcattt aatttaacga agaaccatgt gtaggaaacc catcagcaga atgtagcagg 5520
   ctctggttgc caataataaa gaagtcagca acaagaatat accaacatta acggattagt 5580
   agaaagttgg ggtcacttct tcttttggca tgataagcct agatgggtct ttgcctgtct 5640
   ggctatgttc cattcagatc tctcttgtgc tcttctccat tgtctgttc 5689
<210> 5
   <211> 446
   <212> DNA
   <213> Diabrotica virgifera
<220>
   <221> misc_feature
   <222> (1)..(446)
   <223> RNAi target sequence
<400> 5
   aagagtagcg atgggtggtt tacctcaagg aggaatatta agccctttgt tatatttaat        60
   ttacacttct gatatagaaa aaaatttaaa ctcaacaaaa attttacaat ttgcagatga       120
   tgtagttatt tatcaagaaa acattaaaat agaaaatgca gtcaaatcca ttgaagaagg       180
   agacaaacat attaaaatat ggagtgaatt acatggacta aatatatctg attccaaaac       240
   caaattatgt atttttacaa gaaaacgaaa agaaataccc aatcacatct taataaacaa       300
   tatatcctat cctgtacaaa gttatgttaa atatttgggt attactctgg atagacagct       360
   tctctggaaa gaacatatag acaatatagt taaaaaaaca gaaaaaggaa taaaccttct       420
   tcgattcgta tcacacttac gttggg                                            446
<210> 6
   <211> 22
   <212> DNA
   <213> Artificial Sequence
<220>
   <223> primer
<220>
   <221> misc_feature
   <222> (1)..(22)
   <223> forward primer
<400> 6
   aagagtagcg atgggtggtt ta 22
<210> 7
   <211> 22
   <212> DNA
   <213> Artificial Sequence
<220>
   <223> primer
<220>
   <221> misc_feature
   <222> (1)..(22)
   <223> reverse primer
<400> 7
   cccaacgtaa gtgtgatacg aa 22
<210> 8
   <211> 7388
   <212> DNA
   <213> Diabrotica virgifera
<220>
   <221> misc_feature
   <222> (1)..(7388)
   <223> WCRW transcript ta0145.001_diavv
<400> 8
   catatattcc aacgtttttt actcaaaaga aaggtagtta taaaattagg tagataaaga      60
   gatagaagat aatgatttat tatcattaat taaacctaga tttggaatta aattcgaagt     120
   attacatgta aaaagaatta tgcggaaagt gattcaagat aatggtaata ctaattatag     180
   taaaaacagg aaccgtaatt gtcactttta aaggtcagtc tataccaaaa aatgtaataa     240
   tagaaaaaaa tgatatacga ggtggaaaat tatacaccca gaataatcca atgtttaaag     300
   tgtttgagat ttgggcatat aagtgcccaa tgtagaggaa aagataggtg tgaaagatgt     360
   ggcgaagaac acaacaaatc taattgttcc aatccgaata atttactttg tgtattgtgc     420
   aaaggaaaac acagcgctac tgacaaggga gcagattgta cagaaagaca aaaacaggaa     480
   tatattaaaa aaattatgaa taatgaaaat ataacatatt atgaagctaa taataaatat     540
   aaaaaatgtt attcaacagt aagtaaagac caagaaaata cttcaaataa atatacaata     600
   tctaaacgaa aaagatcaag tagtattgat tctagtagtg tagataaaga aacaatggaa     660
   gcacgcagaa aaattttgca aacaccaaga atacaaagtc agccttgtta taattttaat     720
   aatcccattt attctaacac aacacaaaca caaaacgata atcaaaataa tataggagaa     780
   ataatagtaa actttatttc agcaacatta aatcaaatta atcacaattt agatcaaaaa     840
   acgttggaat tattaaaaaa caatatttca caattattat tacctcgtga tggctaacgt     900
   ttcatttctt caatggaatg cgagatcaat taaaacaaat aagggttatt tagaaaaatt     960
   cttgtatgac aataaaatag atataggatt aatatcagaa acttggttaa aaaaaagtaa    1020
   ttttattaac tttactggtt acaatgtctt taggaatgat agagatgatg gatatggtgg    1080
   agtagccatc ccttccaaaa aattaataaa attttacaac agtcctactt ttcaccaaat    1140
   taatgacata atgtgcaata gcatatcaat taaaattcaa tccggaaaaa agttaaacat    1200
   ttattcaata tacgtaaaac caaatcttaa aatcactcta aatgaatgga aaaagttttt    1260
   taatagtcta gagaagcctt ttataattgg tggtgatttt aatagccaca attatgtttg    1320
   gggttgtagt actgtagata ctataggaaa aaatctgtta gaagctattg aggactgtaa    1380
   tcttgtaatt ttaaataatg gtaaagaaac tttgatgcgt agaccgaata gcaataataa    1440
   atctgcaata gatcttacaa tatgctcagc aaatattagt cattttttcg attgggatgt    1500
   tgtggacgag acattaggat caaatcattt tgctattatt attaagtcaa atattaatgt    1560
   taataaagcg gaatgtgtaa atacaaaatt ccaatggaac ataaataaag cggattggtc    1620
   tcttttctct tctatcactg ataatttcat ggaaatagat aataatttaa attaccaaca    1680
   atttttaaat aaattaaacg aatattgtaa gatatgtata ccggagaaga gaacagggac    1740
   taatccacga tttagaaaaa cttggtggaa tgacaattgt aaaaaggtaa tagaagaaca    1800
   aaaactagct ttacgcaagt ttaaactaca gtctaatata caaaattata taaattataa 1860
   taaatgtgta gcgaaaacca aaaaagttgt attagagagt aagcgtaatg catggagaaa 1920
   tttttgtaaa actttaaaca aaaatacacc aattaaagat atgtggaatc aagccaaacg 1980
   attacaaaac atttttaaac cacaagtaaa tccagtgact gaaggagaat gggttgagga 2040
   gtttttaaga aaattatgtc cagataatat attttcaaaa attaatgtaa tggaaagaaa 2100
   aaactctatg catccacttt caattccatt tagtttctgt gaattagaaa taagccttaa 2160
   gaataagaaa aatacttctc caggtataga taatgtacat tatattatgt tggccaattt 2220
   acatcaaaaa gggaaagaaa cactattaaa tttttttaat caaatatggt tatcagaaga 2280
   tattccagat aactggagag agtaccgggt gattcctatt ctcaaaacaa atcggaatcc 2340
   tgattcagca gaatcttata gaggtatttc attgagctcc tgcataacaa aaacactgga 2400
   aagaatgata aaacttaggc tcgaaagttg gctagaaaaa aacaataaat tatcacaaac 2460
   acaatttgga tttcgaaaaa atcattctac tgtggaagct gtgagtcatt tggtaacaga 2520
   tataaattta gcgtttacga aaaattcttc agtaatcgct cttttattag atgttgaagc 2580
   agcatacgac aacgttaatt taaatatact atataacaaa atgatacaaa taggtctgcc 2640
   agaatgcttc tgtcaaaaaa taataaaatt gtatgactgt agaaaaattt atatatcggt 2700
   aaataataac acatttggtc caagagtagc gatgggtggt ttacctcaag gaggaatatt 2760
   aagccctttg ttatatttaa tttacacttc tgatatagaa aaaaatttaa actcaacaaa 2820
   aattttacaa tttgcagatg atatagttat ttatcaagaa aacattaaaa tagaaaatgc 2880
   agtcaaatcc attgaagaag gagacaaaca tattaaaata tggagtgaat tacatggact 2940
   aaatatatct gattccaaaa ccaaattatg tatttttaca agaaaacgaa aagaaatacc 3000
   caatcacatc ttaataaaca atatatccta tcctgtacaa agttatgtta aatatttggg 3060
   tattactctg gatagacagc ttctctggaa agaacatata gaccatatag ttaaaaaaac 3120
   agaaaaagga ataaaccttc ttcgattcgt atcacactta cgttggggag cagatccaaa 3180
   tatttctttg ttgttattta aaaataatat aagagctata ttcgactacg gatcaatatt 3240
   atacagtgac gcatcacagt gtcatttaaa taaaatagac aaaatcatta ataaatccct 3300
   tagattgtgt ataggagccc taagaagtac accgataaat aatctacaag ttgaatgctg 3360
   tgaaatgccg atggatataa gacgaaaaaa acttggcatc aaccttttag ttagattgta 3420
   tgaaaaaaag gcaagtttaa tttccaaaat acatcaactg tttttagcag acttgacaga 3480
   aaaatattgg ataaaaaaga aaactctaaa tatggtagat ttatattcaa aaatgacaat 3540
   atatgataaa caactttata aatatgacat aaacccaaat tataatattg actttaatag 3600
   tatggaacaa gttcaggtat actttttaag ggactatagc aagttgccta tatctttaag 3660
   aaaattagtg tttatctccg acagttcaca aatgttcaaa gattattgta tgctatatac 3720
   agatgcatca aaaaatattg aaggtgtggg atgtgcctat tgggatagca gtaataaaat 3780
   tagtaaaatg tttaaactaa attctattat gagtatatac acagctgaat taatagcgat 3840
   aatggaagca ctaaaatatt tatctaatat aaatcattca aagtttataa tttttagtga 3900
   cagtaaaagt tctcttagta aaattagtgc tataaatcct aaatcaaaag tgaactacat 3960
   tgaattatat atcataaata aaattaaaga acttcagcaa caaggaaagt ctgttaagtt 4020
   ggcatgggtt aaaagccact gtggaataac tggaaatgaa atggtagatg aattggctaa 4080
   aaacgcggtg acacaaggag tattaaccca ttatctttgt acgccaaaag atattgaatc 4140
   aaatttattc aaagagatta agaaagaatg gaaagaaagg tatattgatg aaaacgtaaa 4200
   tacaggaaac cactacagat caatcagaaa ctatataacg gataaacctt ggttttctaa 4260
   aatggagtcg acaaaagata tgataagaac catatgcaga atgagatttg accactgtct 4320
   tactccatca tatttattta aaattaatat agctgatagt ccacaatgta tttgtggaca 4380
   gttgggcaat ctacaacaca aaattttgac ctgttctctt atctctaata aagttaatat 4440
   gtttttaaat tcactgtatt ctttgactga tgtccaccat cccattaatt taaattattt 4500
   attaacatta gaaaataatg agttggtata ccgactattg tataaacata ttttagatat 4560
   taagcttaaa ttgtaattgt aaaatataga gtaagtattt cttgtaaatt gttatatgtt 4620
   aaaagaaaaa gaaaagaaaa gaaaaaaaaa gaaaagaaat ataaaaaaat aataaaataa 4680
   aataaaaaaa aaaataataa ataaataaaa aaataataaa aaaagaaaga aaaaatataa 4740
   aaaagatata taaaaaaaat agaaaaaaaa atataataat taaaaaaata taaaaaaaaa 4800
   tatgtagata aaaatgaatg acgaacttgg acagtccata gtaaaatagc tttcgaatga 4860
   agtagagggc taaagaagaa tgttgcagtt tttgctgtgg aactctgaga acatcatttg 4920
   gaaaaaaatt aataaaatat atatataata aaaattatta aaaaaaatac aaaaataatt 4980
   atttattagt agaattgttt attatattag tattagtttt aggataagat acgaaaaaat 5040
   gactaataaa ataaaaaata gtaaaattgg cgaatggatt tagtgtccgc agtcatataa 5100
   acccaaacaa acaacaaaag atcaacgaat accataaatc tgagtaatat taattgattt 5160
   ttgaagaaat cgtgtcaaaa ttatttttat tatcaatcta atagtaaact taaagccagt 5220
   agaagaacaa ccggttttta ctggacatta tttagaccag tcaaatggtt taggttctat 5280
   atgagtagaa taacaagtta aaacaattat atattatcaa aaaattgaaa aaaggatcgg 5340
   ctattttatg gtaaagggtt ttttggtcaa tttacttatt aaatgttaag gaggtaaaca 5400
   ttttaaaatg gaataaattg ttaatcaatt taaaaaaaaa attctacgag ttttaaattt 5460
   gcgtcaaaag gaggcttttg tagtggaaaa tatgtgataa aaatttcaaa actattcgtt 5520
   aaatagtttt taaacaattc aatttgctta ttataacttt gcgaaaatga agaatctttt 5580
   acaactaaaa atattttacc acatctcatt ttgaaggttt ttcaataaat caaccgattc 5640
   ttttatcagt ttttttgata aactatataa caaacatcag cattttgcaa ggattgagac 5700
   tattgatggg agcacatgtt atccacttat tttacttttt ataatacaat attacaataa 5760
   atctaactta aaatataaaa tttgggatat ggcatttaaa aatattaatt tcattaccgc 5820
   cgaatttaga tttagatgcg cttaaattca ttaataaaat ttagattaaa ccattacttg 5880
   aactagcttt acaaacaaag agagcaacag aatttgacca aaaaatatat acacctgctt 5940
   atgagatcag tcagtggcgt gaagcatgta cgtgatcgcg atcatgactg ggacagcgat 6000
   cacataatag ggcttttcat tcacaatcat ttgtttcgag cttctgtcat gtgtcacata 6060
   atattaatat acagggtgag tcatgaggac tcctacctcg tatagaggcc cctatgggga 6120
   acaacaaacg accactaaaa agtgtctgct cccattgttc aacaatatac agggcgagtt 6180
   cgcattttga cagaaattca tattcgtcac aattcctgaa cggtcagatc gatgtgtctc 6240
   ccattctggc caatcgccac actactacca cccaatcaac taattcattc aaactagaaa 6300
   aaaacaaggc ccgccccaaa aaaattagtt cgtttgggtc ccagaaaaaa cttcaccctg 6360
   tatacactcc ttgaaaactc caatatgaat tttacaaatt agacaaatag gcaattaaaa 6420
   tggcatattt atttttttcc ccacacgact actcaatttt tattaaaaaa tcaaatttgt 6480
   caaaatcgga attttaacct aaaaataaaa aaaattaaat cagcgtttaa ctcgctacaa 6540
   ctctgttcca ttttaatatt tttttctgaa atttttacag cacatacctc ttaccattgt 6600
   gaagactatg aaaaattgtt gtagactttc agtcttcttc tcgtaaaagt tatgaatttt 6660
   taaaaataaa aggtgcagat tcgtgaattg caaagttaaa tcgcaaaaat taagtgaaaa 6720
   aatttaaaat ttatctattt gatcacgtct atgttaaact atagagtccc aaaagaagtg 6780
   ttatgggaag ttttagatct agacatgtta tagaaaaaaa aaatggtaaa acttttaatt 6840
   tgaggaaaaa tgttgttttt gtaaattaat ttttgtgaaa atcgttaatt tttttaaatc 6900
   tatgcaaaaa ctttgccaaa ctttttatgc atagtcaaat ttgatttttt aactaaaaaa 6960
   ctaagtaatc gtgtggggaa aaaataaata tgccatttca atcgcctacc tgtctccctg 7020
   taaaattcat attagagccc tctaaaaaac gttattttta ctttttaagg tttttcgttt 7080
   taaatttttt cctaagaccc aaacaaacta atttttcaaa tccgggcctg atttctctcc 7140
   agtttgaaca aatcagccga ttgggtgata acacaaatta aatatttgct caaaaaaatt 7200
   catttttgta ataaaagcta tttttttcaa tgcatggttg actttaccaa actttcaatt 7260
   attcatagtc aaatttgatt tttttataaa aaatcaagta atcgtgtagg gaaaaaataa 7320
   tatggaacag agttgtagcg agttaaaccg tgagttcatt tttttttcat ttttaggtta 7380
   aaattccg 7388
<210> 9
   <211> 445
   <212> DNA
   <213> Diabrotica virgifera
<220>
   <221> misc_feature
   <222> (1)..(445)
   <223> RNAi target sequence
<400> 9
   gtttcgtttc atcatcattt gttgtaattt ttgtagtaaa gcacattaaa aaaaaaatct 60
   gttttaaagc catggctgat gaagaatttg acgaaaatga tgtagcagat gatttcgatg 120
   acgacgtaga ggatgataat atcgaagaac tcgaacaacc cgaggaagat ggagataaca 180
   tcgatatcct tgctccagga caagcaggag gtggtgtacc aaaaaacaag aggataacaa 240
   ctaaatatat gaccaaatat gagagagcca gagtattagg tactagagcc ttgcaaatag 300
   ccatgtgtgc cccagttatg gttgaactag atggtgaaac tgatcctctg caaattgcca 360
   tgaaggaatt aaaacagaga aagattccaa ttattattag aagatattta cctgaccatt 420
   cctatgaaga ttggggaata gacga 445
<210> 10
   <211> 20
   <212> DNA
   <213> Artificial Sequence
<220>
   <223> primer
<220>
   <221> misc_feature
   <222> (1)..(20)
   <223> forward primer
<400> 10
   gtttcgtttc atcatcattt 20
<210> 11
   <211> 22
   <212> DNA
   <213> Artificial Sequence
<220>
   <223> primer
<220>
   <221> misc_feature
   <222> (1)..(22)
   <223> reverse primer
<400> 11
   tcgtctattc cccaatcttc at 22
<210> 12
   <211> 544
   <212> DNA
   <213> Diabrotica virgifera
<220>
   <221> misc_feature
   <222> (1)..(544)
   <223> WCRW transcript ta02059.001_diavv
<400> 12
   ggtttcgttt catcatcatt tgttgtaatt tttgtagtaa agcacattaa aaaaaaaatc      60
   tgttttaaag ccatggctga tgaagaattt gacgaaaatg atgtagcaga tgatttcgat     120
   gacgacgtag aggatgataa tatcgaagaa ctcgaacaac ccgaggaaga tggagataac     180
   atcgatatcc ttgctccagg acaagcagga ggtggtgtac caaaaaacaa gaggataaca     240
   actaaatata tgaccaaata tgagagagcc agagtattag gtactagagc cttgcaaata     300
   gccatgtgtg ccccagttat ggttgaacta gatggtgaaa ctgatcctct gcaaattgcc     360
   atgaaggaat taaaacagag aaagattcca attattatta gaagatattt acctgaccat     420
   tcctatgaag attggggaat agacgagctc attattatag atcactagat tgtaattttt     480
   atgtggatat tatttaatac ataggttttt ataacaaaaa aaaaaaaaaa aaaaaaaaaa     540
   aaaa                                                                  544
<210> 13
   <211> 563
   <212> DNA
   <213> Diabrotica virgifera
<220>
   <221> misc_feature
   <222> (1)..(563)
   <223> RNAi target sequence
<400> 13
   ttggtgattc tggtgtagga aaaagtaact tattgtcaag atttactaga aatgaattca 60
   atttggagtc taaatctact ataggtgtcg aattcgctac cagaagtata caggtggatg 120
   gtaaaactat taaagcacaa atatgggaca cggcaggtca agagagatac cgagcaatca 180
   cggcggccta ttatcgtggt gcagtcggtg ctctactcgt ctacgatatc gccaaacatc 240
   tgacatacga aaacgttgaa cgttggttgc gcgaattgcg cgaccacgca gatcagaaca 300
   ttgtgatcat gttggtcgga aacaaatccg atctgaggca tttgcgcgcc gtgctgacag 360
   aagaagctaa ggctttcgcc gaaagaaacg gcctcagctt catcgagact tcagccctag 420
   attcgaccaa tgtcgataat gctttccaaa atatactcac agagatctat agaattgttt 480
   cccaaaaaca gatcagagac cctccggaaa ataatatgat gccccaacac atgatgaacc 540
   aaaaaatgat gcaaccagac atg 563
<210> 14
   <211> 22
   <212> DNA
   <213> Artificial Sequence
<220>
   <223> primer
<220>
   <221> misc_feature
   <222> (1)..(22)
   <223> forward primer
<400> 14
   ttggtgattc tggtgtagga aa 22
<210> 15
   <211> 22
   <212> DNA
   <213> Artificial Sequence
<220>
   <223> primer
<220>
   <221> misc_feature
   <222> (1)..(22)
   <223> reverse primer
<400> 15
   catgtctggt tgcatcattt tt 22
<210> 16
   <211> 689
   <212> DNA
   <213> Diabrotica virgifera
<220>
   <221> misc_feature
   <222> (1)..(689)
   <223> WCRW transcript ta47051.001_diavv
<400> 16
   agttgtcctt attggtgatt ctggtgtagg aaaaagtaac ttattgtcaa gatttactag      60
   aaatgaattc aatttggagt ctaaatctac tataggtgtc gaattcgcta ccagaagtat     120
   acaggtggat ggtaaaacta ttaaagcaca aatatgggac acggcaggtc aagagagata     180
   ccgagcaatc acggcggcct attatcgtgg tgcagtcggt gctctactcg tctacgatat     240
   cgccaaacat ctgacatacg aaaacgttga acgttggttg cgcgaattgc gcgaccacgc     300
   agatcagaac attgtgatca tgttggtcgg aaacaaatcc gatctgaggc atttgcgcgc     360
   cgtgctgaca gaagaagcta aggctttcgc cgaaagaaac ggcctcagct tcatcgagac     420
   ttcagcccta gattcgacca atgtcgataa tgctttccaa aatatactca cagagatcta     480
   tagaattgtt tcccaaaaac agatcagaga ccctccggaa aataatatga tgccccaaca     540
   catgatgaac caaaaaatga tgcaaccaga catgatgatg aataacgaaa tgatgaacga     600
   aaaaatgatg atgccccaac acatgatgaa ccaaaaaatg atgcaaccag acatgatgat     660
   gaataacgaa atgatgaacg aaaaaatga                                       689
<210> 17
   <211> 204
   <212> DNA
   <213> Diabrotica virgifera
<220>
   <221> misc_feature
   <222> (1)..(204)
   <223> RNAi target sequence
<400> 17
   cccaatgccg agatatattg ataccgaaca aggcggatcc caagccagat ttttgttgtc      60
   gaaagtaaat ccaagtcaaa ctcataacaa catgtattcc tacggaggtg attctggagc     120
   tccagttttg acagatgatg tatcccttca agtattcatg gaccatctaa agaaattggc     180
   agtttcgtcc acagcataat acct                                            204
<210> 18
   <211> 20
   <212> DNA
   <213> Artificial Sequence
<220>
   <223> primer
<220>
   <221> misc_feature
   <222> (1)..(20)
   <223> forward primer
<400> 18
   cccaatgccg agatatattg 20
<210> 19
   <211> 20
   <212> DNA
   <213> Artificial Sequence
<220>
   <223> primer
<220>
   <221> misc_feature
   <222> (1)..(20)
   <223> reverse primer
<400> 19
   aggtattatg ctgtggacga 20
<210> 20
   <211> 2636
   <212> DNA
   <213> Diabrotica virgifera
<220>
   <221> misc_feature
   <222> (1) .. (2636)
   <223> WCRW transcript ta01733.001_diavv
<400> 20
   gcacgaggca gacacgacat cgaatgacgt ggaagttcaa attctgaaac aattaggtgt      60
   aatttttagg agctcaataa tagtgttatt tacatgatag aatcctaata atatatattg     120
   aggaatttcc ttagggaatt ccgacttgta atcttcaaaa atgagcacat atgaagagta     180
   tatacaacaa aatgaagatc gagatgggat tagatttacc tggaatgtat ggccttcaag     240
   cagaattgaa gctacccgtc tcgtagtacc cttagcttgt ctgtaccagc ctataaagga     300
   acgtctggat cttccaccaa tacaatatga ccctgtttta tgtactagaa atacttgtag     360
   agcaatatta aacccactgt gtcaggtaga ttatcgagca aaactctggg tatgcaactt     420
   ttgtttccag agaaatccat ttccacctca atatgctgct atttcagaac aacatcaacc     480
   agcggaattg atgcctatgt tttccaccat tgaatacaca ataactagag ctcaatgttt     540
   accaccaata tttttgtatg ttgttgacac ctgcatggat gatgaagaac tgggttccct     600
   gaaagactca ttgcaaatgt cccttagttt gttgccacct aatgcgttaa taggactaat     660
   aacatttggg aaaatggttc aagttcatga acttggcact gaaggttgta gtaagtcata     720
   tgtgttcaga ggtacaaaag atcttagtgc taaacaggtt caagaaatgc tgggaatagg     780
   caaagtggct ttaggtcagc aagcccctca acagccaggg cagcctctaa gacctgggca     840
   aatgcaacct actgttgttg caccaggaag caggtttcta caacctgtat ccaaatgcga     900
   tatgaatcta acagacctaa taggagaaca acagaaagat ccttggcctg ttcatcaggg     960
   taaaaggtat ttaagatcta caggtgtagc tttatcgatt gccattggtt tgttagaatg    1020
   tacatattcc aatactggcg cccgagttat gctatttgtt ggaggacctt gctcacaagg    1080
   acctggtcag gtagttaatg atgatttaaa acagcctatt agatcacatc atgatattca    1140
   gaaagataat gcaaaatata tgaagaaagg tattaaacat tatgatgcgt tagcaatgag    1200
   agccgcaact aatggtcact ctgttgatat ttattcttgt gctttggatc agacaggtct    1260
   gatggaaatg aagcaatgct gtaattctac tgggggacac atggtaatgg gggattcatt 1320
   taattcttcc ttgtttaagc aaactttcca acgtgtgttt accagagatc aaaaaagtga 1380
   tctgaaaatg gcatttaacg gtactttgga agtgaagtgt tcccgagaat taaaagttca 1440
   aggaggtatc ggttcgtgtg tatcacttaa cgtgaagagc cccttggttt ccgacacaga 1500
   aataggaatg ggtaatactg tgcaatggaa aatgtgtact ttaacgccaa gtactaccat 1560
   gtctttattc tttgaggtcg taaatcaaca ttctgctccc atacctcaag gtggtagagg 1620
   ttgtatacaa tttattacgc agtaccagca ttcaagtggt caaagaaaaa tcagagtaac 1680
   aacagtggct cgaaattggg ctgacgcaac tgctaatata caccatatca gtgccggatt 1740
   cgatcaagaa gctgctgctg taataatggc taggatggcc gtttataggg cagaatctga 1800
   tgatagtcca gatgttctta gatgggttga cagaatgctg attagattgt gtcaaaaatt 1860
   cggagaatac aataaggacg accccaattc attcagactt ggtcaaaact tcagtcttta 1920
   cccacagttc atgtatcact taagaagatc tcaatttctt caagtattca ataattctcc 1980
   ggacgagact tcattctaca gacacatgtt gatgagggaa gatcttactc aatctttgat 2040
   aatgattcaa cctattttgt atagttatag tttcaatggt ccaccagagc ctgtattact 2100
   agatactagc tccattcaac ctgacagaat attacttatg gatactttct tccaaatatt 2160
   aattttccat ggagagacta tcgcccaatg gcgtagttta aaatatcaag acatgccaga 2220
   atatgaaaac tttagacagc tactacaggc tccagtagat gatgcacaag aaattttgca 2280
   aactaggttc ccaatgccga gatatattga taccgaacaa ggcggatccc aagccagatt 2340
   tttgttgtcg aaagtaaatc caagtcaaac tcataacaac atgtattcct acggaggtga 2400
   ttctggagct ccagttttga cagatgatgt atcccttcaa gtattcatgg accatctaaa 2460
   gaaattggca gtttcgtcca cagcataata cctatatatt acaattagat acatttgaca 2520
   taatacagtt tttgaattta ttcaatatat tatattttaa gcttaatttt tttgtatatt 2580
   tatttcatag atagtttata tatttggtaa tgtgatacaa taaatttttg ttttcc 2636
<210> 21
   <211> 599
   <212> DNA
   <213> Diabrotica virgifera
<220>
   <221> misc_feature
   <222> (1)..(599)
   <223> RNAi target sequence
<400> 21
   acaaatgtgg tggtatcgac aaacgtacca tcgaaaagtt cgaaaaagaa gcccaagaaa 60
   tgggtaaagg ttcattcaaa tatgcctggg tactcgacaa acttaaggcc gagagagaac 120
   gtggtattac cattgatatt gctttgtgga aattcgaaac agctaaatac tatgtaacca 180
   tcattgatgc ccctggacac agagatttca ttaagaacat gatcactggt acatcacaag 240
   ctgactgtgc tgtactcatt gttgcagctg gtactggtga atttgaagca ggtatttcaa 300
   agaatggaca aacacgtgaa catgctcttc ttgctttcac ccttggtgta aaacaactta 360
   ttgttggtgt caacaaaatg gactcgactg aaccagcata cagtgaatca cgtttcgagg 420
   aaatcaagaa ggaagtatcc tcatacatca agaaaattgg ttacaaccca gctgccgttg 480
   ctttcgtacc aatttcagga tggcacggag acaacatgtt agaaggatct gacaagatgc 540
   catggttcaa gggatggcaa atcgaacgta aagaaggaaa agctgaagga aagtgcttg 599
<210> 22
   <211> 22
   <212> DNA
   <213> Artificial Sequence
<220>
   <223> primer
<220>
   <221> misc_feature
   <222> (1)..(22)
   <223> forward primer
<400> 22
   acaaatgtgg tggtatcgac aa 22
<210> 23
   <211> 22
   <212> DNA
   <213> Artificial Sequence
<220>
   <223> primer
<220>
   <221> misc_feature
   <222> (1)..(22)
   <223> reverse primer
<400> 23
   caagcacttt ccttcagctt tt 22
<210> 24
   <211> 1698
   <212> DNA
   <213> Diabrotica virgifera
<220>
   <221> misc_feature
   <222> (1)..(1698)
   <223> WCRW transcript ta04714.004_diavv
<400> 24
   gggatcttcc tttttaattt gctcgtcgta cggtgtggac ctcgcgcctg gtgttacatc 60
   ccaagtagtg ttccttttat tctaagttta atttcgaaca gttgcattta ctttatttcc 120
   aaacaatcaa aatgggtaaa gaaaagattc atattaacat cgttgtcatt ggacacgtag 180
   attctggtaa atctactact actggacatt taatttacaa atgtggtggt atcgacaaac 240
   gtaccatcga aaagttcgaa aaagaagccc aagaaatggg taaaggttca ttcaaatatg 300
   cctgggtact cgacaaactt aaggccgaga gagaacgtgg tattaccatt gatattgctt 360
   tgtggaaatt cgaaacagct aaatactatg taaccatcat tgatgcccct ggacacagag 420
   atttcattaa gaacatgatc actggtacat cacaagctga ctgtgctgta ctcattgttg 480
   cagctggtac tggtgaattt gaagcaggta tttcaaagaa tggacaaaca cgtgaacatg 540
   ctcttcttgc tttcaccctt ggtgtaaaac aacttattgt tggtgtcaac aaaatggact 600
   cgactgaacc agcatacagt gaatcacgtt tcgaggaaat caagaaggaa gtatcctcat 660
   acatcaagaa aattggttac aacccagctg ccgttgcttt cgtaccaatt tcaggatggc 720
   acggagacaa catgttagaa ggatctgaca agatgccatg gttcaaggga tggcaaatcg 780
   aacgtaaaga aggaaaagct gaaggaaagt gcttgattga ggctttggat gctatccttc 840
   ccccatctcg tccaactgag aaacccctcc gtcttccact ccaggatgtc tacaaaattg 900
   gtggtattgg aacagtacct gtaggtcgtg ttgaaactgg tgtattgaaa cctggtatgg 960
   ttgtagtatt cgctccagcc aatttgacga ctgaagtaaa atccgttgaa atgcatcacg 1020
   aagccctcca ggaagcagta cctggtgaca atgttggttt caacgtcaag aatgtctctg 1080
   ttaaagaatt gcgtcgtggt tacgtagctg gagacaccaa gaacaaccca cccagaggag 1140
   cttcagactt cactgcccaa gtcattgttc tcaaccaccc tggtcaaatc tcaaacggat 1200
   atacccctgt actcgactgt cacacagctc acattgcctg caaattcgct gaaatcaaag 1260
   aaaaggttga ccgtcgttct ggtaagacca ctgaagaaaa tcccaaagcc atcaaatctg 1320
   gtgatgccgc cattgtcaac ttggtaccca ccaagcccat gtgtgtagaa tcattccaag 1380
   aattcccacc ccttggacgt tttgctgtcc gtgacatgag gcaaaccgtt gcagtaggag 1440
   tcatcaagag cgttgccttc aaagatccca gcgccggaaa agtcacaaaa gctgcagaaa 1500
   aagcccaaaa gaagaaatag tctatttgga ctccactact taaataattt tgatatgttt 1560
   tgtttctttt atatattaat attggagtat caatgtattt ttaataacta atggacttat 1620
   tacttgcatt gcattatttg tgatctgtaa attaaacttg gacaaataaa gaaaaaaatt 1680
   aaaaaaaaaa aaaaaaaa 1698
<210> 25
   <211> 550
   <212> DNA
   <213> Diabrotica virgifera
<220>
   <221> misc_feature
   <222> (1)..(550)
   <223> RNAi target sequence
<400> 25
   ccagggaaag agagaagtac gataatttag cagatttgta tgctgtaata aacactttgc      60
   aacaattaga gaaggcatat attcgagatt gtgtagtggc aaaggaatat acaggagctt     120
   gtagtaaatt attagttcag tataaggctg cgtttcgaca agtcaagagt gatgaatttc     180
   ctacaccaga cgcctttgtt aagaagtaca ggcttgactg tcctgcagct ttagaacgta     240
   tcaaggaaga taggccaatc actatcaaag acgacaaagg caacactagt cgatgcattg     300
   ctgatatcgt atcgctattc atcactataa tggacaaact gcggttggac attcgttcga     360
   tggataactt acatcctgac gtgagggact tagtagatac tatgaacaga cttagcattc     420
   tgccatcgga tttcgatgga aaggtaaaag tggctgaatg gttaaatact ttaaatgaaa     480
   tgcaggcctc tgatgaatta tccgaagctc aagtgaggca actgttgttt gatcttgaaa     540
   gtgcgtatgc                                                            550
<210> 26
   <211> 22
   <212> DNA
   <213> Artificial Sequence
<220>
   <223> primer
<220>
   <221> misc_feature
   <222> (1)..(22)
   <223> forward primer
<400> 26
   ccagggaaag agagaagtac ga 22
<210> 27
   <211> 22
   <212> DNA
   <213> Artificial Sequence
<220>
   <223> primer
<220>
   <221> misc_feature
   <222> (1)..(22)
   <223> reverse primer
<400> 27
   gcatacgcac tttcaagatc aa 22
<210> 28
   <211> 769
   <212> DNA
   <213> Diabrotica virgifera
<220>
   <221> misc_feature
   <222> (1)..(769)
   <223> WCRW transcript ta01620.001_diavv
<400> 28
   atataaatag aaaaatgtca gcagaaaatc gccctgagct gtatgaggaa gtcaagctat      60
   atcacaatgc cagggaaaga gagaagtacg ataatttagc agatttgtat gctgtaataa     120
   acactttgca acaattagag aaggcatata ttcgagattg tgtagtggca aaggaatata     180
   caggagcttg tagtaaatta ttagttcagt ataaggctgc gtttcgacaa gtcaagagtg     240
   atgaatttcc tacaccagac gcctttgtta agaagtacag gcttgactgt cctgcagctt     300
   tagaacgtat caaggaagat aggccaatca ctatcaaaga cgacaaaggc aacactagtc     360
   gatgcattgc tgatatcgta tcgctattca tcactataat ggacaaactg cggttggaca     420
   ttcgttcgat ggataactta catcctgacg tgagggactt agtagatact atgaacagac     480
   ttagcattct gccatcggat ttcgatggaa aggtaaaagt ggctgaatgg ttaaatactt     540
   taaatgaaat gcaggcctct gatgaattat ccgaagctca agtgaggcaa ctgttgtttg     600
   atcttgaaag tgcgtatgcg tcctttaata aagtccttca taattctgcc taaatgtacg     660
   agatagttgt tatgattgtt acacgtttat gtaaatatta taagcaaaaa aaattgagct     720
   agatagttat tggcaatgta tgcatttatc aattttaacc ctaattgga                 769
<210> 29
   <211> 578
   <212> DNA
   <213> Diabrotica virgifera
<220>
   <221> misc_feature
   <222> (1)..(578)
   <223> RNAi target sequence
<400> 29
   aatccaaaag ctgaagtggc tagagctgca caagccctag ccgttaatat atcagcggca      60
   aaaggcattc aagatgtaat gaagtcaaac cttggtccca aaggaacagt gaaaatgttg     120
   gtatctggtg ccggcgatat aaaaataacc aaagatggaa acgttctatt gcacgaaatg     180
   caaattcaac accccacggc ttcccttatt gctcgagcct caactgccca agatgatatg     240
   accggcgacg gcactacatc gactgtgtta gtcataggtg aactattaaa acaagcagat 300
   atttacatag cagaaggtct ccaccctaga ttgttaacag atggctttga taaggctagg 360
   acaaaagcgt tggaagtact ggagaaaatg aagatttcaa ttgaagcgaa caaagaaact 420
   ttaacagata tttgcaggac gagtttaaga acaaagctgc atcaaaaact ggctgatgtt 480
   ttgacagacg tttgtgtgga tgctgttcta gctataaaga aagatggcaa acctgttgat 540
   cttcatatgg ttgaattaat ggaaatgcag cacaaaac 578
<210> 30
   <211> 20
   <212> DNA
   <213> Artificial Sequence
<220>
   <223> primer
<220>
   <221> misc_feature
   <222> (1)..(20)
   <223> forward primer
<400> 30
   aatccaaaag ctgaagtggc 20
<210> 31
   <211> 20
   <212> DNA
   <213> Artificial Sequence
<220>
   <223> primer
<220>
   <221> misc_feature
   <222> (1)..(20)
   <223> reverse primer
<400> 31
   gttttgtgct gcatttccat 20
<210> 32
   <211> 1864
   <212> DNA
   <213> Diabrotica virgifera
<220>
   <221> misc_feature
   <222> (1)..(1864)
   <223> WCRW transcript ta00896.001_diavv
<400> 32
   gggggtcaaa aattttgggt tggctcaaca cgagttttct attttatttg aaaaaatagt 60
<210> 33
   <211> 709
   <212> DNA
   <213> Diabrotica virgifera
<220>
   <221> misc_feature
   <222> (1)..(709)
   <223> RNAi target sequence
<400> 33
   gaaaatgcca accaaagtct tcgagaagag ctgtccttta aggatcagat ccatcaacaa      60
   cagttgacag aaactcgtac tcgtcgtcaa gtagaaatca gcgaaatcga cggtcgcctg     120
   gcagaaaaat atgaggctaa gcttcaagag gctctacaag aactcagaga tcaatatgaa     180
   agtcaaatgg ccaacaaccg tggcgaaatc gagttgcttt acgaacagaa gattaagaac     240
   ttgcaatcag ctaatgacag acatgctgcc tctgcctccg gtgctcttga cgaattgaga     300
   caagttagaa ccagaatcga tactttgaat tctcgaatta gcgatttaga gaaccagaac     360
   gctggcttag cagctcgtgc tagagatttg gagaagttgt tagagaatga gcgcatgcgc     420
   catgtggaag acctcgcaat gttagaaaga gaattgcata gactccgaga agaaatggct     480
   aatcaacttc aagaatacca ggatcttatg gacattaagg tttctcttga tttagagata     540
   gctgcttatc ggaaactgct cgaatctgaa gaagctcgtc ttaatattac cccaacaggt     600
   agtgaagttc atgaagtaac tcacagtagg agccaacgca gctcgtcgca acgtcgcact     660
   ccagtcagat tgggagccaa aagaaagcga acactactcg aagaaagcc                709
<210> 34
   <211> 22
   <212> DNA
   <213> Artificial Sequence
<220>
   <223> primer
<220>
   <221> misc_feature
   <222> (1)..(22)
   <223> forward primer
<400> 34
   gaaaatgcca accaaagtct tc 22
<210> 35
   <211> 22
   <212> DNA
   <213> Artificial Sequence
<220>
   <223> primer
<220>
   <221> misc_feature
   <222> (1)..(22)
   <223> reverse primer
<400> 35
   ggctttcttc gagtagtgtt cg 22
<210> 36
   <211> 2662
   <212> DNA
   <213> Diabrotica virgifera
<220>
   <221> misc_feature
   <222> (1)..(2662)
   <223> WCRW transcript ta03354.001_diavv
<400> 36
   aaaaaccgtt aaacgccgct cccatcatat catctccaaa aatagtttga ttacctcggg      60
   tagaccgcga cataatttgt gcttcgaccg atctctctct ttgagttgtc gcttttcgag     120
   gtatttagtt gcaggcgtgg tgtatacgtt gaaatagtga tttttgttaa aagtgagtaa     180
   atatggcatc gaaatcaaaa aaagggtcaa caccgttagc ttcacagcgg ccggcgagcc     240
   ccttaagtcc taccagacat tctagattac aggagaagca tgaacttcag aatcttaacg     300
   accgtttggc gggatatata gatcgagtaa ggtatttaga aggtgaaaac aacagattaa     360
   cgagggaaat acaaagtacc caggaaacag taacaaggga agtttcaaac attaaatcta     420
   tgtatgacca tgaattatca gatgctagaa aactattaga cgagacccac cgtgaaaaag     480
   cacgtttaga gatagatgtt aaacgtcttt tagaagagaa tgacgaactc aagtctgagt     540
   tagcaaagaa gaacaaggat ttggctctag ctgagaattc tgccaggata tacgagacca     600
   gatgtaacga gttacagctc aaagcaaatc aagcggctgc tgatgctaag aaagctattg     660
   cagatgctaa ggaacttgag aaagaacgtg ataagctacg caaacttctt gataatcatc     720
   gtaaacagtt agaagaagaa tcattagcga gagtggaagt cgaaaatgcc aaccaaagtc     780
   ttcgagaaga gctgtccttt aaggatcaga tccatcaaca acagttgaca gaaactcgta     840
   ctcgtcgtca agtagaaatc agcgaaatcg acggtcgcct ggcagaaaaa tatgaggcta     900
   agcttcaaga ggctctacaa gaactcagag atcaatatga aagtcaaatg gccaacaacc     960
   gtggcgaaat cgagttgctt tacgaacaga agattaagaa cttgcaatca gctaatgaca    1020
   gacatgctgc ctctgcctcc ggtgctcttg acgaattgag acaagttaga accagaatcg    1080
   atactttgaa ttctcgaatt agcgatttag agaaccagaa cgctggctta gcagctcgtg    1140
   ctagagattt ggagaagttg ttagagaatg agcgcatgcg ccatgtggaa gacctcgcaa    1200
   tgttagaaag agaattgcat agactccgag aagaaatggc taatcaactt caagaatacc    1260
   aggatcttat ggacattaag gtttctcttg atttagagat agctgcttat cggaaactgc 1320
   tcgaatctga agaagctcgt cttaatatta ccccaacagg tagtgaagtt catgaagtaa 1380
   ctcacagtag gagccaacgc agctcgtcgc aacgtcgcac tccagtcaga ttgggagcca 1440
   aaagaaagcg aacactactc gaagaaagcc aagagtcaag catgaatgac tatacggtga 1500
   acagctcctc aaaaggtgac attgaagtcg ctgatgtcga tccagaagga cactatgtta 1560
   agttgcacaa taaaagtaat caagaagtcg cacttggagg atggcaagtc attagacgtg 1620
   ttggtgagga ggaaactaca ttcaaatttc acaggtcact aaagcttgaa ggtaatggat 1680
   ttgtaacaat atggagctct gacctcaaca aagatcatga acctccaact aatttagtta 1740
   tgaagagtca gaaatgggtt gtaggtgata acatggtcac cacattattg aataataacg 1800
   gagatgaagt agctgtctcc gaaagagtta aaaggcaatt aacaagctcc catctaagac 1860
   acagagaact agcagctggc tacctctcag aagaactcca ccatcaacag ggtgatccac 1920
   aagtagatga aagatgtagg cttatgtaaa gttaacacat ctcatttaat ttttttattt 1980
   acagttaagg ttggaatgtt acagaagtag gttgtgctcc ttacttacgc tttttaaact 2040
   attttttaag tgatgttctt aatattataa gattctagga cagaaggaac acaacttata 2100
   ctgttattga caaaaaactt actgaaaatt gcaattagac aacagtagta agtttaatac 2160
   taagaataca ggatgaggta ataattatta ttctttatat aaactgtctt tttaggagtt 2220
   ttatatatat tttttaaata tcttgcatat aagtcttgaa ttacagttgt catttttata 2280
   aatttaaacc tgaaaaatac attatttcgc attaatacct catcctgttc gatagtttct 2340
   taagatagat aacaaactgc cagttcgata agttttcttt taactaacac aattgtttat 2400
   tagtataata taattttgaa aaattactgt actgttgtcc ccaaaacatt aaagtgttct 2460
   gcatttattt aaagaataat aaacttttta tattaatggt agttcttttg tttttgaggt 2520
   ttttcactca gctgtttacg cttgttttag ttagataggt gttgcaaaaa ctatagggat 2580
   aaaaatctag atttggtttc agttacttgt attatatttt attgtatatc tttattcttg 2640
   atcttcgaaa cttaaatctg ta 2662
<210> 37
   <211> 635
   <212> DNA
   <213> Diabrotica virgifera
<220>
   <221> misc_feature
   <222> (1)..(635)
   <223> RNAi target sequence
<400> 37
   aatggcttta cacgtcccta aagcaccggg ctttgcccag atgttaaaag aaggagccag 60
<210> 38
   <211> 22
   <212> DNA
   <213> Artificial Sequence
<220>
   <223> primer
<220>
   <221> misc_feature
   <222> (1)..(22)
   <223> forward primer
<400> 38
   aatggcttta cacgtcccta aa 22
<210> 39
   <211> 22
   <212> DNA
   <213> Artificial Sequence
<220>
   <223> primer
<220>
   <221> misc_feature
   <222> (1)..(22)
   <223> reverse primer
<400> 39
   aaccagaccc caaaacttta ca 22
<210> 40
   <211> 1866
   <212> DNA
   <213> Diabrotica virgifera
<220>
   <221> misc_feature
   <222> (1)..(1866)
   <223> WCRW transcript ta04788.001_diavv
<400> 40
   ggaactctat ggtcttcagt cactctaaat aaaatcctat aacctttttt gggatttcaa      60
   ttcgaattaa ataaatttct tcagaaacat acaagaaaca aatacagaca atggctttac     120
   acgtccctaa agcaccgggc tttgcccaga tgttaaaaga aggagccagg cactattctg     180
   gtttggaaga agctgttata agaaacataa tagcatgcaa ggaatttgca caatccgtaa     240
   aaactgccta tggtccaaat ggtatgaata aaatggtcat taatcacatt gagaaacaat     300
   ttgtcacaag tgatgcagct acaattatgc gagaactaga tatagaacac ccagcagcca     360
   aactcatgat actaggaagt caaatgcaag attctgaggt gggtgatgga actaactttg     420
   tgattatact tgctggagca cttttggagc atgctgaaga actaattcgt cttggagtaa     480
   ctcccacaga aatagcagaa ggttatgaga aagcactaga caaatgtcta gaaatccttc     540
   ctaatcttat ttgctacaca atcaaggatt tcagaaacat cgaagaagtc aaaaagggca     600
   tacaaacctc aattcaatct aagcagtatg gcaatgaaga gtttctatca aaacttattg     660
   cagaagcttg tgtgtcaatt ttgccagaag aaacaacatt taatgttgat aatgttagaa     720
   tatgtaaagt tttggggtct ggtttacata actctcaggt tgtacaaggt atggttttca     780
   aaagacaagt tgaaggggaa ataactaaag cagaaaaagc taaaattgct gtattcagct     840
   gtgctgttga tattatgcaa acagaaacta aaggaactgt acttatcaaa actgcagatg     900
   aacttatgaa ctttagtagg ggagaagaaa atcttcttga attacaaatc aaatgtatag     960
   cagatgctgg agctaaagtt gtagttgcag gagccaaatt tggagatatg gctctacatt    1020
   acttacataa atatggcatg atggttgtca ggttgaactc caaatttgat ttgagaagat    1080
   tatgcaaaac tgttggtgct acttgtcttc cacgcctaac agctcccaca tctcaagaac    1140
   ttggttacgc agatattgta tgtgtagaag aattgggtga tacccctata gtttctttca    1200
   gattggaggg caaagaatca agaatttcca caattgtcat cagaggtgct actgacaatt    1260
   acatggatga cattgaaaga gcaattgatg atggagttaa tacatttaag ggaatctctc    1320
   gagatggcag atttgttcct ggtgccggcg ccgttgaagc agagttagct gttcagctga    1380
   ctaaatatgc agacactctt ccaggcttag aacagtatgc tgtcagaaaa ttcgcatctg    1440
   cccttgaaag ttttcccaaa acgttggcag ataatagtgg acacaaatcc acagcagtat    1500
   tagaaaaaat attggaagct caccagaatg gtcagaaaaa tgttggagtt gacatagaag    1560
   ctgaaagtgc agtatgtgac gctttggaaa agaacatctt ggacctttat caatgtaaat    1620
   attggggact gaagtatgct gtaggagcag cagcaaccat tttaagagtc gaccaaatta    1680
   ttatggcgaa aagggcaggt gggccaaaag ttaggcaacc aaagggaagt gatgacgaat 1740
   cttgaaatcg ttaattttgc gcttggtttc tcgtatacac ttaaattcac cttacaactc 1800
   acttgtatta atctgttaat ttaatttttt aattaatatt tcttaaccaa aaaaaaaaaa 1860
   aaaaaa 1866
<210> 41
   <211> 579
   <212> DNA
   <213> Diabrotica virgifera
<220>
   <221> misc_feature
   <222> (1)..(579)
   <223> RNAi target sequence
<400> 41
   ccagagactt catatcccaa ccaagactca cttacaaaac tgtatctggt gtcaacggtc 60
   cactagtaat cctcgaagat gtcaaatttc ctaagttcaa cgaaattgtc caactcagat 120
   tatctgacgg aagtttaaga tcaggtcaag tacttgaagt cagcggttcc aaggctgtag 180
   tacaagtatt cgaaggtaca tcaggtatcg atgccaaaca cactgtctgt gagttcacag 240
   gagatattct cagaactcca gtctctgagg atatgttggg aagagtattc aacggctctg 300
   gaaaacctat tgacaaaggt cccccaattc tggctgaaga ctacctagat attcaaggac 360
   aacccatcaa cccatggtcc cgtatctacc cagaggaaat gatccaaact ggtatcactg 420
   ccatcgatgt gatgaactcg attgcccgtg gtcagaagat tcccattttc tcagctgctg 480
   gtctgccaca taacgatatt gctgcccaaa tttgtaggca agccggtctt gtcaaaatcc 540
   caggcaaatc agttttggat gaccacgaag acaactttg 579
<210> 42
   <211> 22
   <212> DNA
   <213> Artificial Sequence
<220>
   <223> primer
<220>
   <221> misc_feature
   <222> (1)..(22)
   <223> forward primer
<400> 42
   ccagagactt catatcccaa cc 22
<210> 43
   <211> 22
   <212> DNA
   <213> Artificial Sequence
<220>
   <223> primer
<220>
   <221> misc_feature
   <222> (1)..(22)
   <223> reverse primer
<400> 43
   caaagttgtc ttcgtggtca tc 22
<210> 44
   <211> 2471
   <212> DNA
   <213> Diabrotica virgifera
<220>
   <221> misc_feature
   <222> (1)..(2471)
   <223> WCRW transcript ta00620.009_diavv
<400> 44
   tcttaaactt ccgtcagata atctgagttg ctaatttttt actgtctttg atagacgtct      60
   cctgtacacg taaaaatggc ttcgacgata tcggcaagac aggctaacag agaacatgtt     120
   ttggccgtat ccagagactt catatcccaa ccaagactca cttacaaaac tgtatctggt     180
   gtcaacggtc cactagtaat cctcgaagat gtcaaatttc ctaagttcaa cgaaattgtc     240
   caactcagat tatctgacgg aagtttaaga tcaggtcaag tacttgaagt cagcggttcc     300
   aaggctgtag tacaagtatt cgaaggtaca tcaggtatcg atgccaaaca cactgtctgt     360
   gagttcacag gagatattct cagaactcca gtctctgagg atatgttggg aagagtattc     420
   aacggctctg gaaaacctat tgacaaaggt cccccaattc tggctgaaga ctacctagat     480
   attcaaggac aacccatcaa cccatggtcc cgtatctacc cagaggaaat gatccaaact     540
   ggtatcactg ccatcgatgt gatgaactcg attgcccgtg gtcagaagat tcccattttc     600
   tcagctgctg gtctgccaca taacgatatt gctgcccaaa tttgtaggca agccggtctt     660
   gtcaaaatcc caggcaaatc agttttggat gaccacgaag acaactttgc catcgtgttc     720
   gctgctatgg gtgttaacat ggagactgct agattcttca aacaagattt cgaagaaaat     780
   ggttctatgg aaaacgtatg tttgttcttg aacttggcta acgatccaac catcgaaaga     840
   atcatcaccc cacgtcttgc tctaacagcc gctgaattct tggcttacca gtgcgaaaaa     900
   cacgtgttag tcattttaac tgacatgtct tcatacgctg aagctttgcg tgaggtatct     960
   gccgccagag aggaagtacc cggtcgtcgt ggtttccccg gttacatgta caccgatttg    1020
   gccaccatct acgaacgtgc cggtcgtgtc gaaggccgta acggttccat cactcaaatc    1080
   cccatcttga ctatgcctaa cgacgatatt actcacccta ttcccgattt gacaggttat 1140
   attaccgaag gacagatcta cgtcgaccgt caattgcaca acaggcagat ctacccacca 1200
   attaacgtac tgccatctct ctcacgtttg atgaagtctg ctatcggcga aggtatgacc 1260
   aggaaggacc actctgacgt ttctaaccag ttgtatgctt gctatgctat cggtaaggac 1320
   gtccaggcca tgaaagccgt agtaggtgaa gaagctctaa cacccgacga tcttctctac 1380
   ctcgagttcc taacgaaatt cgaaaagaac ttcatcaccc aaggaaacta cgaaaaccgc 1440
   accgtcttcg agtctttgga catcggctgg caactactcc gtatcttccc taaggaaatg 1500
   ttgaaacgta tccctgcaag cacattggcg gaattctatc caagagactc tcgtcactag 1560
   ttggtttatg tataaaattt atatatattt aggaagtttg ttaggtttgt tacaaatatg 1620
   tccacttatt gtatcgatta ttttcaattg agagatattt taccaaaaca atttgagccg 1680
   atgagattct acggtgtaat ttttgtaaat acttaaagag cgtgtttcaa aaatgcgctt 1740
   aatataatat attatcaata cagagcaacc tttttttctt tattgttttt ttcactccaa 1800
   agttcttgaa ctcctaaaag gagcatagag cttcagtgaa aaggcgccat cccctcattc 1860
   caagactttc cttgacctct taccaattgt tggaaaaatt ataactcaga ttaattttat 1920
   taggtgacag ttccataagt aaaaatatgc aatggctata tattgcgtac taaacttaag 1980
   aagaaaaatt gtgtcgctct ggaagaaaga ttacttgcat aagaatgaaa atcagtgtca 2040
   tattttgcgt ttatgacatc cctagacgat actatttttt cgttactata ttttttttaa 2100
   agagatcaag agcaacaatt tggagactga tattcttatg cgagttgttt tcctactttg 2160
   tcctattttg aagagtctac aattatttac ttaccatttg attaaattag atgaacataa 2220
   gacaatacta tatttttatc atgtaagttg atattcccta tgtaaattat tggcacaatg 2280
   taacctccaa cttgatttat acatttattt tacttttgtt tgtctatttt ttctgtaata 2340
   gagtggaaga attaacattc cattaaatgt cattgcccaa tgtataaagc aatatacctc 2400
   ttaccattcg atgctttttc agtattttaa ttctagatgg tgaacggaga tttttcttga 2460
   tgtaggtttt g 2471
<210> 45
   <211> 670
   <212> DNA
   <213> Diabrotica virgifera
<220>
   <221> misc_feature
   <222> (1)..(670)
   <223> RNAi target sequence
<400> 45
   tcgtaatttt tggttgtgct tgagaaaatc gtcaaacatc caaaatggtg aacttcaccg 60
<210> 46
   <211> 22
   <212> DNA
   <213> Artificial Sequence
<220>
   <223> primer
<220>
   <221> misc_feature
   <222> (1)..(22)
   <223> forward primer
<400> 46
   tcgtaatttt tggttgtgct tg 22
<210> 47
   <211> 22
   <212> DNA
   <213> Artificial Sequence
<220>
   <223> primer
<220>
   <221> misc_feature
   <222> (1)..(22)
   <223> reverse primer
<400> 47
   tcaactctga cttcacccat tg 22
<210> 48
   <211> 4535
   <212> DNA
   <213> Diabrotica virgifera
<220>
   <221> misc_feature
   <222> (1)..(4535)
   <223> WCRW transcript ta02030.005_diavv
<400> 48
   ggggggcaga gagtcggtct ctttctttca cgggagtgcg ccatcgttgg cgtatcgtaa 60
   tttttggttg tgcttgagaa aatcgtcaaa catccaaaat ggtgaacttc accgtagatg 120
   aaattagggc catgatggac aaaaagagga acatccgtaa catgtccgtc attgcccatg 180
   tggaccatgg taaatctaca ttaacagatt ctctagtatc aaaagctggt attattgctg 240
   gtgccaaagc tggagaaacc agattcacag acacaaggaa agatgaacag gaacgttgta 300
   ttaccatcaa atcaactgcc atatctatgt tcttcgagtt agatgataaa gatcttgtgt 360
   tcatcaccaa cgctgaccaa cgtgagaaag atgtgaaagg tttcttgatt aaccttattg 420
   actctcctgg acacgttgat ttctcatcag aagtaacagc tgcccttcgt gtcactgatg 480
   gtgcccttgt agtagtagat tgtgtatctg gtgtatgtgt acagactgaa actgtactcc 540
   gtcaagctat tgcagaacgt attaagccaa ttttattcat gaacaagatg gaccgtgccc 600
   tcttagaatt acagttggaa tctgaagaac tttaccagac tttccaacgt attgttgaaa 660
   acgtcaacgt cattattgcc acctacaacg atgattcagg tccaatgggt gaagtcagag 720
   ttgaccccag caaaggttca gttggttttg gatcaggtct ccacggatgg gctttcactc 780
   tcaaacaatt ctctgaaatg tatgccgaga aattcaagat tgatgtagtt aaactaatga 840
   accgtttgtg gggtgagaac ttcttcaacc ccaaaaccaa gaaatggtcc aaacaaaagg 900
   atgatgataa caaacgttca ttctgtatgt acatcttaga tccaatctac aagatttttg 960
   actccatcat gaactacaag aaagaagaat atgaagcact ccttccaaaa ttgggtattg 1020
   ccttaaaaca tgaagataaa gacaaagatg gtaaacaatt attgaaagta gttatgcgta 1080
   cctggttgcc agctggagaa gctttacttc agatgatagc cattcactta ccatcacctg 1140
   tggtagcaca gaaatacaga atggaaatgt tgtacgaagg acctcatgat gatgaagcag 1200
   ctgttggtat caagaactgt gatccaaatg cacctcttat gatgtatgta tctaaaatgg 1260
   tacccacttc tgacaaaggt cgtttctatg cttttggtcg tgtattctct ggtaaagtag 1320
   ctactggtat gaaagcccgt atcatgggac ccaactacac ccctggcaaa aaggaagatt 1380
   tatatgaaaa ggctatccaa cgtaccattc tcatgatggg tcgttatgtt gaagctattg 1440
   aagatgtccc atctggtaac atctgtggtt tggttggtgt tgatcaattc ttggttaaga 1500
   ctggtaccat taccactttc aaggatgccc acaacttgaa ggtcatgaaa ttcagtgtat 1560
   ctcctgtagt acgtgttgct gttgaaccta agaaccctgc tgatttgcca aaacttgtag 1620
   aaggtttgaa acgtttgacc aaatccgatc ctatggtcca atgtatcatt gaagaatctg 1680
   gtgagcacat cattgctggt gccggagaat tgcatcttga aatttgcctt aaggatcttg 1740
   aagaagatca tgcctgcatt cccattaaga aatctgaccc tgttgtatcc taccgtgaga 1800
   cagtaagtga agagtctgat cagatgtgcc tctcaaaatc acccaacaaa cacaacagat 1860
   tgttcatgaa agcttgtcct atgcctgatg gacttgctga agatattgat gatggtaaag 1920
   ttaacccacg tgatgacttc aaagtccgtg cccgttatct tactgagaaa tatgactatg 1980
   atataactga agcccgtaag atctggtgct ttggacctga tggtactgga cccaacatcc 2040
   ttgttgattg tactaaggga gtgcaatatc ttaatgaaat caaagactct gtggtagctg 2100
   gtttccaatg ggcaaccaaa gaaggtgtct tgtctgaaga aaacttgaga ggtgttagat 2160
   tcaacattta tgatgttacc ttacacgctg atgctatcca tcgtggaggt ggtcaaatca 2220
   ttccaacaac ccgtaggtgt ttgtatgctt gtttaatgac agctgcaccc agacttatgg 2280
   aacctatcta caagtgtgaa attatgtgtc ctgaagttgc tgttggtggt atttatggtg 2340
   tattgaacag aagacgtggt cacgtatttg aagaagctca aatcgctggt acacctatgt 2400
   tcttagtaaa agcttactta cctgtaaacg aatcattcgg tttcactgct gatttacgtt 2460
   ccaacactgg aggtcaggcc ttcccacaat cagtcttcga tcactggcaa atcttacctg 2520
   gtgacccaat ggaacccagt accaaaccct atggaattgt gcaggacaca cgtaaaagaa 2580
   agggtcttaa agaaggactt ccagacctgg cacaatatct ggataaatta taaacaacta 2640
   agaaacttaa tttatgtaca gattatttaa taaaattatt tcaatttact caacggtttt 2700
   tatagttaat tgtatttttg atatttttat tctgataagt tttcagttct ctcaaattga 2760
   tggcaacact agaaatgaaa taaactaaat aatctgacta atattttatg tttgttatat 2820
   atatttttgt taacagtcca gatatttgta tatttattaa tttgacatca aaagtaaatc 2880
   tgatgaaaca tttttgagta tttatttgtg gatacaaatt cgtcttgttt tatttttact 2940
   cccttgaaac attttattag tgtaattttt atttgagcag tactgattaa tgttaagtat 3000
   attttttgga tatgtgtata ataaaactca gttttgtaag gaatctagtt ttctttaatg 3060
   tgggtatcca aatcaattac gttaagtaaa taaaatacta tacccaaagt tatacaaggt 3120
   tagagagtaa acaataaaac attaaatatt ctgtacatac attctagact cggataagat 3180
   gaccgctgaa aataaatggg ccctcttgta ctactatatc tggtacatag tgttactaca 3240
   gaactagagg attttttaaa attgaaggag ctaataccac atcagaaaaa cacccacatg 3300
   ctttataaaa cattaggaag gaaagagtca ttacgtataa aaattggagc atactttaca 3360
   gtaaatgaaa caaagaaacc ttatctaaac aaatattcac aaagtcctgg ataaattcat 3420
   taagatcaat cacattcaat ataacagaat ttcttcattg ttttgaactc tttgtagata 3480
   tctcttggaa acattaatat gctgattgga attctttaat tattttatga aaatattatg 3540
   gttgatttat ggcacctgac tgatttcaga tctccaaata cacattagga aatcatgatg 3600
   aagaaaccat ttttattttt ccatgtagca aattttctat agaaatatta gcttgctaat 3660
   atccactaac tagtagaagt ttgtaatgtc acatttattt aaagaaatgg caggatagca 3720
   aggattttat tttctttaaa aatgataaat tgcatacctt tgaagaagac aaaatattac 3780
   tcctatacat ttcatacaaa caataaaaag aagaaaccac gataccagat gaggaaaaac 3840
   atggaattca gttcatttag aattgattgt tttgaaaagc ttaaactaat tgatttttgc 3900
   aagttgacag ctgacaagga atgctcctat acaaactgaa agatcgacga ttaggatatt 3960
   ttttaaaggt tttgtaatga gtaacatatt cctaggagta tatcttgaga cataatgtga 4020
   aagatctaaa atatcctatg gggcagattc ctgttgtatt taaaaaaagg gctgcaaatt 4080
   aatagccact aaagctgact ttgctgtttt tgaaacaaaa agtctgcgaa ttaataacca 4140
   ctgaggctga ctttgctgtt ttttaaagaa aaggttgtta attaataacc ggtgagacta 4200
   accctgatgt ttttgaaaga aaggggttgc taattcataa tccatggcgc aaactctact 4260
   gtttttgaaa taagagattt ttgagaaatt gtactgaaaa tgtgcctgtt cgtattaata 4320
   tgaactgaaa acaacaaagg caaatcaggg atctaaccta ccgatcttta aaaggctgtt 4380
   cccaaaaata aatgattctg atggtttcat ggacttaaga gtgaaatcaa gagaaggtgt 4440
   ctgttcaata tagaaagttt tggggatgaa attctttctc tctggaatag tttctgttat 4500
   tcaaggtgtt aaactaagaa tatttcagga taaaa 4535
<210> 49
   <211> 528
   <212> DNA
   <213> Diabrotica virgifera
<220>
   <221> misc_feature
   <222> (1)..(528)
   <223> RNAi target sequence
<220>
   <221> misc_feature
   <222> (499)..(499)
   <223> n can be any nucleotide
<220>
   <221> misc_feature
   <222> (500)..(500)
   <223> n can be any nucleotide
<400> 49
   cgtcaaaagt atttgtcgca tcaactgaag acatgagaaa ataatttttt acaacataat        60
   attttcagat attaaatcaa gatggtacta gcagatttag gtcgtaaaat tacgaccgcc       120
   ttgcagtctt taagcaaggc aacaattatt aatgaagagg tcttaaatgg aatgctaaat       180
   gatatttgtc gggcactcat tgaggctgat gttaatatca aattagttaa atctctcagg       240
   gaaaatgtta aatctgttat tgactttgat gaaatggctg gtggtctcaa caagaggagg       300
   atgatccaaa gtgcagtatt taaagagctt gtcaaattgg tagacccagg agtaaagcca       360
   tatcaacctg ttaaaggcaa accaaatgtt attatgtttg ttggtttaca aggttctggt       420
   aaaacaacca cttgtaccaa actagcttat cattatcaga aaaagaactg gaaatcatgt       480
   ttggtatgtg cagatacann cagagctggt gcctatgatc aggtaaaa                    528
<210> 50
   <211> 22
   <212> DNA
   <213> Artificial Sequence
<220>
   <223> primer
<220>
   <221> misc_feature
   <222> (1)..(22)
   <223> forward primer
<400> 50
   cgtcaaaagt atttgtcgca tc 22
<210> 51
   <211> 22
   <212> DNA
   <213> Artificial Sequence
<220>
   <223> primer
<220>
   <221> misc_feature
   <222> (1)..(22)
   <223> reverse primer
<400> 51
   ttttacctga tcataggcac ca 22
<210> 52
   <211> 1927
   <212> DNA
   <213> Diabrotica virgifera
<220>
   <221> misc_feature
   <222> (1)..(1927)
   <223> WCRW transcript ta05283.001_diavv
<400> 52
   ggggggtcgc atcaactgaa gacatgagaa aataattttt tacaacataa tattttcaga 60
<210> 53
   <211> 605
   <212> DNA
   <213> Diabrotica virgifera
<220>
   <221> misc_feature
   <222> (1)..(605)
   <223> RNAi target sequence
<400> 53
   aggctgtaag agaacctatg cagtctggaa ttaaggctgt agattctctt gtaccaattg 60
   gtcgtggtca gcgtgagttg atcattggtg atcgtcaaac tggtaaaact gctttggcta 120
   ttgatacaat catcaaccaa aaacgattca acgatgctga agacgaaaag aagaaattgt 180
   actgcattta cgtcgccatc ggtcaaaaga gatctactgt ggctcaaatc ttgaagcgtc 240
   tcacagacac tggtgccatc aactacacaa tcattgtatc agctactgca tctgatgctg 300
   cacctttgca atacctcgcc ccatactccg gatgtgctat gggtgaattt ttcagagata 360
   atggcaaaca tgccctcatc atctatgatg atttgtccaa acaagccgta gcctaccgtc 420
   agatgtctct gttgcttcgt cgtcctccag ggcgtgaagc ctaccctggt gatgtattct 480
   accttcactc ccgtcttttg gaacgtgccg ccaagatgtc tgaagcccac ggtggtggtt 540
   cattgactgc cttgcccgta attgaaaccc aagctggtga tgtatcagcc tacattccaa 600
   ccaat 605
<210> 54
   <211> 23
   <212> DNA
   <213> Artificial Sequence
<220>
   <223> primer
<220>
   <221> misc_feature
   <222> (1)..(23)
   <223> forward primer
<400> 54
   aggctgtaag agaacctatg cag 23
<210> 55
   <211> 22
   <212> DNA
   <213> Artificial Sequence
<220>
   <223> primer
<220>
   <221> misc_feature
   <222> (1)..(22)
   <223> reverse primer
<400> 55
   attggttgga atgtaggctg at 22
<210> 56
   <211> 2003
   <212> DNA
   <213> Diabrotica virgifera
<220>
   <221> misc_feature
   <222> (1)..(2003)
   <223> WCRW transcript ta00263.005_diavv
<400> 56
   ggggggatcg tgcgcagttc ggcctattcg ggtactcgtt cagtgttcgt cacttaattt      60
   tcagtactgg ggcgactggc aaaaaaatgt ctttgctttc cgcacgatta gctgcctcat     120
   tggcaagaaa tgcccctaaa aatgtaacac agattgcggg agctgtcttc cctgctgcat     180
   ccgtcgcagc ccggaacttc catgctactc cacaaaacaa aggtgctgaa atttcatcca     240
   tccttgaaga gcgcatcctt ggtgctgcac caaaagccaa cctcgatgaa acaggacgag     300
   tcttaagtat tggtgacggt atcgcccgtg tatatgggtt gaacaacatc caagccgatg     360
   agatggtgga attctcctcc ggtctcaaag gtatggccct taacttggaa cctgacaacg     420
   ttggtgttgt agtatttggt aacgacaaac tcattaagga aggtgacatt gtcaagagaa     480
   ctggtgccat tgtagatgtg cctgtaggtg accaaatttt gggacgtgta gttgatgcct     540
   taggtaaccc aattgatggc aaaggaccat tgaacaccaa acaacgtttc cgtgtaggta     600
   tcaaagcccc tggtatcatt ccacgtatct ctgtaagaga acctatgcag tctggaatta     660
   aggctgtaga ttctcttgta ccaattggtc gtggtcagcg tgagttgatc attggtgatc     720
   gtcaaactgg taaaactgct ttggctattg atacaatcat caaccaaaaa cgattcaacg     780
   atgctgaaga cgaaaagaag aaattgtact gcatttacgt cgccatcggt caaaagagat     840
   ctactgtggc tcaaatcttg aagcgtctca cagacactgg tgccatcaac tacacaatca     900
   ttgtatcagc tactgcatct gatgctgcac ctttgcaata cctcgcccca tactccggat     960
   gtgctatggg tgaatttttc agagataatg gcaaacatgc cctcatcatc tatgatgatt    1020
   tgtccaaaca agccgtagcc taccgtcaga tgtctctgtt gcttcgtcgt cctccagggc    1080
   gtgaagccta ccctggtgat gtattctacc ttcactcccg tcttttggaa cgtgccgcca    1140
   agatgtctga agcccacggt ggtggttcat tgactgcctt gcccgtaatt gaaacccaag    1200
   ctggtgatgt atcagcctac attccaacca atgtcatttc catcactgat ggacaaatct    1260
   tcttggaaac tgaattgttc tacaaaggta tcagacctgc cattaacgta ggtttgtctg 1320
   tatccagagt aggatcagct gctcaaacca aggccatgaa acaggttgct ggttccatga 1380
   aactggaatt ggcccaatac cgtgaagtag ctgcttttgc ccaattcggt tccgatcttg 1440
   atgctgctac tcagcaattg ttgaacagag gtgtacgtct tactgaattg ttgaaacaag 1500
   gtcaatacgt acccatggct attgaagaac aagtagccat catctactgt ggtgtcagag 1560
   gacatttgga caaagttgat ccatcaaaga ttacagtatt tgaaaaacaa ttctcagaac 1620
   acattaagac tagccacaaa aacatcttgc agagcattgc caaggaagga aaaatcacag 1680
   acgatactga tgcccaactc aagaaagtcg ttcaagattt cttggccagc ttctctggtt 1740
   aaatataaat aatacaatta ttattaatac ctaaatcgtc acttttgttg atgtgatctg 1800
   taaacgttta agaacttcat gagctgtaat cctttcaaat tagaataatt aggcaggact 1860
   cctatgttat ccaataaggc acgtgaaaga attttctttt ctccatttta caatatttat 1920
   cgatcattgt tttttaagga tatcttattt gtaaaaataa aaaataaact ttatattttt 1980
   taaaaaaaaa aaaaaaaaat cgg 2003
<210> 57
   <211> 583
   <212> DNA
   <213> Diabrotica virgifera
<220>
   <221> misc_feature
   <222> (1)..(583)
   <223> RNAi target sequence
<400> 57
   gcacgaggct ttattagaca gaggtattca tcccataagg attgctgatg gtttcgagct 60
   agcttgtcaa acagctgtag ctcatttgga aaaaattgca gaactatttc cggtatcagc 120
   agaaaacact gaaccattaa taaaagttgc aatgaccact ctgggatcta aaatcattaa 180
   caagtgtcat aaacagatgg cagaaatagc tgttaatgca gtcttagctg ttgcagattt 240
   agaacagaaa gatgttaatt ttgaacttat taaagttgaa ggcaaggttg gtggaaaatt 300
   ggaagacaca acattagtta aaggtgtagt tattgacaaa acaatgtccc atccacaaat 360
   gcccaaagaa ctaaaagatg taaaattggc tattctcacc tgcccgttcg aaccaccaaa 420
   accaaagacc aaacataagt tggatgtcag cagtgtggaa gattataagg aactaagaca 480
   gtatgaacaa gaaaagttca aagaaatcgt tggtttggta aagaaagctg gagctacatt 540
   agcaatttgt caatggggct tcgatgatga agccaatcac tta 583
<210> 58
   <211> 23
   <212> DNA
   <213> Artificial Sequence
<220>
   <223> primer
<220>
   <221> misc_feature
   <222> (1)..(23)
   <223> forward primer
<400> 58
   gcacgaggct ttattagaca gag 23
<210> 59
   <211> 22
   <212> DNA
   <213> Artificial Sequence
<220>
   <223> primer
<220>
   <221> misc_feature
   <222> (1)..(22)
   <223> reverse primer
<400> 59
   taagtgattg gcttcatcat cg 22
<210> 60
   <211> 1889
   <212> DNA
   <213> Diabrotica virgifera
<220>
   <221> misc_feature
   <222> (1)..(1889)
   <223> WCRW transcript ta05506.001_diavv
<400> 60
   aaaaatatca aatgtcagtt ttctgtcact ttcatgggag tagccagttc gaaacaaaaa      60
   taaaatttta attaatctgg aacatctccc aattttttag aaaacttaca ttttcaatat    120
   gagtgctata ccaggaagta ttgcatttga tgaatatgga cgacccttca taattcttag     180
   agaccaagat aagcaaaaga ggttgaccgg gatagatgca cttaagtcac atattttggc     240
   agcgagacaa atcgctcaaa ctataagcac ttctttgggt cccaaaggtt tagacaagat     300
   gatggtgtca cctgacggtg aagtaacagt cactaatgat ggtgctacta ttttgaaaat     360
   gatggatgtt gaacatgaag tcgctaaatt gctagtacag ctatcacagt cccaagatga     420
   tgaaattggt gatggtacca caggtgttgt agttttggct ggtgccctgt tagagcaagc     480
   aggatcttta ttagacagag gtattcatcc cataaggatt gctgatggtt ttgagctagc     540
   ttgtcaaaca gctgtagctc atttggaaaa aattgcagaa ctatttccag tatcagcaga 600
   aaacactgaa ccattaataa aagttgcaat gaccactctg ggatctaaaa tcattaacaa 660
   gtgtcataaa cagatggcag aaatagctgt taatgcagtc ttagctgttg cagatttaga 720
   acagaaagat gttaattttg aacttattaa agttgaaggc aaggttggtg gaaaattgga 780
   agacacaaca ttagttaaag gtgtagttat tgacaaaaca atgtcccatc cacaaatgcc 840
   caaagaacta aaagatgtaa aattggctat tctcacctgc ccgttcgaac caccaaaacc 900
   aaagaccaaa cataagttgg atgtcagcag tgtggaagat tataaggaac taagacagta 960
   tgaacaagaa aagttcaaag aaatcgttgg tttggtaaag aaagctggag ctacattagc 1020
   aatttgtcaa tggggcttcg atgatgaagc caatcactta ttgttacaac aagaattacc 1080
   agctgtaaga tgggttggag gtccagaaat tgaacttata gcaatagcta ctgggggtag 1140
   gatagttccc cgttttgaag agcttactcc tgaaaaactt ggtcatgctg gtttggtacg 1200
   agaaattgca tttggcacca ctaaggatag aatgttggtt attgaagaat gttcaaattc 1260
   acgggcagtt acgattcttg tcagaggagg taatcaaatg ttagtagctg aagccaagag 1320
   aagtctccat gatgcattat gtgttgtcag gagtttagtt caagaaccaa gagtagtgta 1380
   tggtggtgga gcagcagaaa ttgcctgctg tttggcagtc gcggctcaag cagatcaact 1440
   agcttccttg gaacagtacg cctatcgtgc atttgctgaa gccttagaaa gtataccact 1500
   tgctttggct gataacagtg ggctatctgc gatccatacg ctaggagaag tcaaggctaa 1560
   acaagcagct acaggagatc cagctctcgg tatcgattgt atgaatactg gaaacctcaa 1620
   catgagagaa caacatgtta tcgaatcact cagatcaaaa agacaacaat ttttgttggc 1680
   cacccagttg gttaaaatga tattgaaaat tgacgatgta cgccaacctg catcgggata 1740
   aaatgacact tatttttgta ctaccggcgt taatttctaa ttctcactaa taatgacttt 1800
   tataagttga tctgcgtaat gcttctttgt tcttataatc actgaaagta ttaatataat 1860
   ttatagaaaa aaaaaaaaaa aaaatgcga 1889
<210> 61
   <211> 588
   <212> DNA
   <213> Diabrotica virgifera
<220>
   <221> misc_feature
   <222> (1)..(588)
   <223> RNAi target sequence
<400> 61
   agagaacagt cagcacaagc agttattgat atcgatgatt tagataattt ggctacacct        60
   gaaagtatat tgttgagtgc tgtttctgga gaagatgccc aagaccgttc agatcgtaca       120
   attcttactc cgtgggttaa gttcctttgg gaatcttact gtcaatgttt ggaacttttg       180
   cgtactaatg cacatgttga gaacttatat catgatatag ccaggatggc tttccagttt       240
   tgtttgaaat ataatagaaa aacagaattt agaaaattat gtgataaatt gaggaaacat       300
   ttagaggata tctgcaaatt accaacacaa atggcaaatg tttctatgtc taaaccagaa       360
   acccaacaac ttaatcttga aacgaggctc catcaactag attttgctat tcagatggag       420
   ttatggcaag aagcctacaa ggccatcgaa gacatccata acttgatgaa tttgtccaag       480
   aagagtccag taccaaaaac catggcaaac tattaccaga aactagccat ggtgttctgg       540
   aaagctggta attatctatt ccatgctgct gctttgttca agttcttc                    588
<210> 62
   <211> 22
   <212> DNA
   <213> Artificial Sequence
<220>
   <223> primer
<220>
   <221> misc_feature
   <222> (1)..(22)
   <223> forward primer
<400> 62
   agagaacagt cagcacaagc ag 22
<210> 63
   <211> 22
   <212> DNA
   <213> Artificial Sequence
<220>
   <223> primer
<220>
   <221> misc_feature
   <222> (1)..(22)
   <223> reverse primer
<400> 63
   gaagaacttg aacaaagcag ca 22
<210> 64
   <211> 3733
   <212> DNA
   <213> Diabrotica virgifera
<220>
   <221> misc_feature
   <222> (1)..(3733)
   <223> WCRW transcript ta05000.002_diaw
<400> 64 tattataaaa aaa 3733
<210> 65
   <211> 716
   <212> DNA
   <213> Diabrotica virgifera
<220>
   <221> misc_feature
   <222> (1)..(716)
   <223> RNAi target sequence
<400> 65
   ttgaaaaaga tagggctcca aagtcctatt gccaacaaaa aaacaacgca agacattaaa      60
   caactttttt caagcccatc tgtatttaaa aaaacagtgc aaaatgtttc tgtgggactg     120
   gtttacggga atgctgggat atctaggatt gtggaagaaa cctggaaaac tattattctt     180
   aggactggat aacgcaggca aaactaccct tctacatatg ctcaaggatg acagactggc     240
   ccagcatctt cccacgttac atcccacatc agaggagctt tccattggta acatgaggtt     300
   tacgacgttc gatttggggg gccacgagca ggctaggaga gtgtggaggg actactttcc     360
   agcagtcgat gccatagtgt tccttgttga tgccaacgac agctcaagat ttgtagaaag     420
   ccaggaacag ctaaatgccc tcctctcaga cgaaactcta tcaaactgtc caatacttat     480
   cttaggtaat aaaattgatc tcccaggtgc tgcttcggaa gatgaattac gaactagatt     540
   cggcttgttt ggccaaacca caggcaaagg caaagtagcc agaaatgatc tacccggtag     600
   gcctctagaa ctatttatgt gctctatact caaaagacaa ggttatggag aaggtttccg     660
   ttggttggca caatatatcg attaattatg tatttttcca tttcgttctg tcattg         716
<210> 66
   <211> 22
   <212> DNA
   <213> Artificial Sequence
<220>
   <223> primer
<220>
   <221> misc_feature
   <222> (1)..(22)
   <223> forward primer
<400> 66
   ttgaaaaaga tagggctcca aa 22
<210> 67
   <211> 22
   <212> DNA
   <213> Artificial Sequence
<220>
   <223> primer
<220>
   <221> misc_feature
   <222> (1)..(22)
   <223> reverse primer
<400> 67
   caatgacaga acgaaatgga aa 22
<210> 68
   <211> 2438
   <212> DNA
   <213> Diabrotica virgifera
<220>
   <221> misc_feature
   <222> (1)..(2438)
   <223> WCRW transcript ta00303.001_diavv
<400> 68
   ggggggaaaa agatagggct ccaaagtcct attgccaaca aaaaaacaac gcaagacatt 60
   aaacaacttt tttcaagccc atctgtattt aaaaaaacag tgcaaaatgt ttctgtggga 120
   ctggtttacg ggaatgctgg gatatctagg attgtggaag aaatctggaa aactattatt 180
   cttaggactg gataacgcag gcaaaactac ccttctacat atgctcaagg atgacagact 240
   ggcccagcat cttcccacgt tacatcccac atcagaggag ctttccattg gtaacatgag 300
   gtttacgacg ttcgatttgg ggggccacga gcaggctagg agagtgtgga gggactactt 360
   tccagcagtc gatgccatag tgttccttgt tgatgccaac gacagctcaa gatttgtaga 420
   aagccaggaa cagctaaatg ccctcctctc agacgaaact ctatcaaact gtccaatact 480
   tatcttaggt aataaaattg atctcccagg tgctgcttcg gaagatgaat tacgaactag 540
   attcggcttg tttggccaaa ccacaggcaa aggcaaagta gccagaaatg atctacccgg 600
   taggcctcta gaactattta tgtgctctat actcaaaaga caaggttatg gagaaggttt 660
   ccgttggttg gcacaatata tcgattaatt atgtattttt ccatttcgtt ctgtcattga 720
   gttaggatat taatgtttga ggaactattg gcaacactgc aactacctga ttcatttcag 780
   atcttaggta ctttacataa atatctaaat atagatgttg gcaatgtaat tttgaacaac 840
   agtatataca ttcaatgtaa attatatatt tttagttaag ttaccttctt aaatggtgtt 900
   tgagtggcta tggtactgaa atagtttgac tttttgtgtt cttcgataac taaaaatgat 960
   ttcttgtgga aagttacact cagaattaca tagttaactt ctttatcagc agttgttgtc 1020
   aagatttcca ttttgagacg attgttttgt aaattaggtg ggaattttta aaatgttgga 1080
   ctgtttttta acatgccttt ctcaactttt gttacaaata atgttttgtc cagttgtaaa 1140
   tgttttattt aactagagac tttctaggtt gtgttaataa tagtcattgg tcaaatacga 1200
   tagaagccaa aatttgaaga aaaggaacaa tgtactaggg tggtccgata agtattttgc 1260
   cttcaaaaga aaagtggcga tttatttttc aatattgtct ccttctagct caatacacgt 1320
   gacccagcga ggcttcaccc agattcagcg gcttcaattt ctgaaggtct gtaaagtaga 1380
   catcaatagc agcgatgaac tcactaaatc gacttacatt tctgcccgac gagtgacttt 1440
   tcaaatttgg aaacgaaaag aaatcgcaca gggccaaatc tggagaatac agtggatggg 1500
   atagtagtta gtagtccgaa tcgaaaaatt tggccatggc gacggcgaag gtgtgagcgg 1560
   gtgcgttgtc gtggaagagt actttttatc ttccccaatt ggggctgttt tttctgcaag 1620
   tcggtgtcga atcgacccaa taattcgcca aagtaaagcc ctgtgaccgt tttgcccttt 1680
   ttttcaggaa gctgatatat atcacacttt gtgaatccca gaaaacggtg gtcatcactt 1740
   ttcggtcgtt aggacagtct ttgccttctt cggagtatgt tcgcggggcg acgtccactg 1800
   tatcaactgt tccttggttt ctggtgagta ctggtggatc caagtttcat cgatgattat 1860
   gaaacaatgt ataaacaata ggaaggttta atttatttct aattctgagt aggaatggtc 1920
   acttaaaggt tttgtaattt aacttaagta cctaatgaat ttattttgga ctttttcaag 1980
   cttggctata tggacttcat aagcgggtga ccaaacaact gagcaatact caagaacaga 2040
   tctaactagg gaaatgtaaa tcagtctgat tgaattaatg ttatgcaaac atctagaagt 2100
   tctaataatg aagcctaaca tgtgaaatgc cgcaccaacc acatcaacac aatgcgcata 2160
   aacaaaataa tgaagaatat aactcaaaaa acagatacag cacaggtgac acaacacaca 2220
   gcagtcaagc acaaagtgaa agcacaaaaa actacaaaac caaaaaacaa gaacaaaatc 2280
   acaggcaaat atcaaacgaa gtgaattatt caacaaacca aaaataaacc aaatgccaca 2340
   cccagtcaaa acaaaacaac gaaggttgag attttgacat agataggcca tctacaagaa 2400
   tgtattacat atacagggtg cgccaagcct ctggtttt 2438
<210> 69
   <211> 619
   <212> DNA
   <213> Diabrotica virgifera
<220>
   <221> misc_feature
   <222> (1)..(619)
   <223> RNAi target sequence
<400> 69
   tcggtcgatt taggtagtag gtgtaataaa ttttgaaagt gataaagaca gtttgtgata 60
   aactgtagtg cagttctttt cgtttatttt ctcacctctc acataacaat taaactgaaa 120
   caatggcccc cccgggtatc gggaattcgg tggagatccg ccccgaggcg acagcatcgt 180
   cggcagagag gagcagtagc aacggagtcg ctacattcca aaaggacatc gtcgttttgg 240
   atgggggctt cgctactcaa ttaggctgcc acgtccaaca accgattgat ggagacgtcc 300
   tatggagttc caggttcttg gccaccaatc cagaggctgt tattgatact cacttagatt 360
   ttctcagagc tggtgcggat gtaattatta ctaacacata tcaagcagat gtcaacttgt 420
   ttgccaaaca tctcaatatg accaaagaag aaggatacaa cttaataaaa gactcagtga 480
   agatggccaa aacagctgtt gaaagatatt taaaagaata tccggaagca agaaaacctc 540
   ttatagctgg ttcagtgggt ccttacggag cttcactaca tgatggttcg gaatacacag 600
   gagcctatgc gaaaactac 619
<210> 70
   <211> 23
   <212> DNA
   <213> Artificial Sequence
<220>
   <223> primer
<220>
   <221> misc_feature
   <222> (1)..(23)
   <223> forward primer
<400> 70
   tcggtcgatt taggtagtag gtg 23
<210> 71
   <211> 22
   <212> DNA
   <213> Artificial Sequence
<220>
   <223> primer
<220>
   <221> misc_feature
   <222> (1)..(22)
   <223> reverse primer
<400> 71
   gtagttttcg cataggctcc tg 22
<210> 72
   <211> 1246
   <212> DNA
   <213> Diabrotica virgifera
<220>
   <221> misc_feature
   <222> (1)..(1246)
   <223> WCRW transcript ta08045.004_diavv
<400> 72
   ggtcactcgt cgccgactgc cgacttgatc ggtcgattta ggtagtaggt gtaataaatt      60
   ttgaaagtga taaagacagt ttgtgataaa ctgtagtgca gttcttttcg tttattttct     120
   cacctctcac ataacaatta aactgaaaca atggcccccc cgggtatcgg gaattcggtg     180
   gagatccgcc ccgaggcgac agcatcgtcg gcagagagga gcagtagcaa cggagtcgct     240
   acattccaaa aggacatcgt cgttttggat gggggcttcg ctactcaatt aggctgccac     300
   gtccaacaac cgattgatgg agacgtccta tggagttcca ggttcttggc caccaatcca     360
   gaggctgtta ttgatactca cttagatttt ctcagagctg gtgcggatgt aattattact     420
   aacacatatc aagcagatgt caacttgttt gccaaacatc tcaatatgac caaagaagaa     480
   ggatacaact taataaaaga ctcagtgaag atggccaaaa cagctgttga aagatattta     540
   aaagaatatc cggaagcaag aaaacctctt atagctggtt cagtgggtcc ttacggagct     600
   tcactacatg atggttcgga atacacagga gcctatgcga aaactacttc ggcagaaacc     660
   atgagagaat ggcacgggcc cagaataaaa gccttggtgg aaggaggagt tgatctctta     720
   gcaattgaaa ctattccgaa tgcagttgaa gctgagatgt tggtccagtt aataaaagaa     780
   gaatatccta atgttaaagc gtggcttgct tttagtgtag cgcaagacgg aaaatcaata     840
   gcctacggag aagacttcaa aacaatcgcc cgaaaatgtt atgactccaa tcctgagcaa     900
   ttagtagcca taggaatcaa ctgcacggcc ccacgactaa tcgaacccct tctcaccggg     960
   ataaatgatg atagaaaaga caacccccga ccacttattg tctaccctaa cagcggagaa    1020
   agttacaatg tcgaactcgg ggatgattcc agatggcata accgagaaaa ctgcgagcca    1080
   gtagacacaa atattcacaa atggttagat ctgggagtca cgtgggtcgg aggctgctgt    1140
   agaacctacg ccgtggatgt cacaagaata cgcgaccaag tgcagttatg gaaggagaaa    1200
   cagtagctaa actttgaatt tatattttaa tttttatttt taataa                   1246
<210> 73
   <211> 680
   <212> DNA
   <213> Diabrotica virgifera
<220>
   <221> misc_feature
   <222> (1)..(680)
   <223> RNAi target sequence
<400> 73
   attgtcaacc tggaaaacga cttgtcgaag tcgcatagtt tttataagtt taaataaact        60
   aaattaaata taaatacttc gagaatgcaa taattattat tctttaacta gacccacagc       120
   ttattaatta gcagaagtag tagcagactt atactaacta gcataaggag aaacatatta       180
   acatagcatg gcagacttca tagattctga agcagaagaa agtagtgagg aggaggaatt       240
   agatcatagg gatcgtaaaa aagcccaaaa agccaaagtt gtagatagtt cagatgaaga       300
   tgatgaagat gatgacgaaa gactgagaga ggaattaaag gatttgattg atgataatcc       360
   tattgaagaa agtgatgctg agtctgatgc ttcaggaagg gaaaaacgta agaaatctga       420
   cgacgaggat ttggatgatc gactggaaga tgaagattat gatttgcttg aagaaaattt       480
   gggtgttaaa gttgaaagaa ggaaattcaa gcgactgcgg cgttttgaag atgaagaaag       540
   tgaaggagaa gaagaacatg atcctgaaca agatagggaa caaattgcta tggatatatt       600
   ttcagatgat gacgatgaaa gacgatcaga acgaagtcac aggcctgccg tcgaacaaga       660
   aacttatggt gtaggcgagg                                                   680
<210> 74
   <211> 22
   <212> DNA
   <213> Artificial Sequence
<220>
   <223> primer
<220>
   <221> misc_feature
   <222> (1)..(22)
   <223> forward primer
<400> 74
   attgtcaacc tggaaaacga ct 22
<210> 75
   <211> 22
   <212> DNA
   <213> Artificial Sequence
<220>
   <223> primer
<220>
   <221> misc_feature
   <222> (1)..(22)
   <223> reverse primer
<400> 75
   cctcgcctac accataagtt tc 22
<210> 76
   <211> 6054
   <212> DNA
   <213> Diabrotica virgifera
<220>
   <221> misc_feature
   <222> (1)..(6054)
   <223> WCRW transcript ta02840.001_diavv
<400> 76
   agtttaaata aactaaatta aatataaata cttcgagaat gcaataatta ttattcttta      60
   actagaccca cagcttatta attagcagaa gtagtagcag acttatacta actagcataa     120
   ggagaaacat attaacataa catggcagac ttcatagatt ctgaagcaga agaaagtagt     180
   gaggaggagg aattagatca tagggatcgt aaaaaagccc aaaaagccaa agttgtagat     240
   agttcagatg aagatgatga agatgatgac gaaagactga gagaggaatt aaaggatttg     300
   attgatgata atcctattga agaaagtgat gctgagtctg atgcttcagg aagggaaaaa     360
   cgtaagaaat ctgacgacga ggatttggat gatcgactgg aagatgaaga ttatgatttg     420
   cttgaagaaa atttgggtgt taaagttgaa agaaggaaat tcaagcgact gcggcgtttt     480
   gaagatgaag aaagtgaagg agaagaagaa catgatcctg aacaagatag ggaacaaatt     540
   gctatggata tattttcaga tgatgacgat gaaagacgat cagaacgaag tcacagacct     600
   gccgtcgaac aagaaactta tggtgtaggc gaggaagaag aggaagggga gtactcggat     660
   gccgatgatt ttatagttga cgatgacggt agaccgatag ctgaaaaaaa aaaaaaaaaa     720
   aaaaaaaaaa aaacagatgc cgctctccaa gaggctcaag aaaccttcgg tgtcgatttt     780
   gattatgatg aatttagtaa atacgatgaa gatgattacg aagatgaaga ggaggaggat     840
   gacgaatacg aggaagatga tgtagagaaa aggaaacggc ctaaaaagac ttcaaagaaa     900
   aaaccgacga agaaatccat ttttgaagtg tatgaaccta gtgaacttaa aagagggttc     960
   tttaccgatc tcgataatga aatccgaaac actgatattc ccgaaagaat gcaacttcgt    1020
   gatgttccaa tcaccgctgt tccggatgac tcaactgaac ttgatgatga agcagaatgg    1080
   atttacaggc aagcgttttg taacagaact gtttccaatg tggattctca tttatcatca    1140
   gaggcaagag agaaattaaa gaagactcat catgccatcg gaaaaatcag aaaagcatta    1200
   gattttataa gaaatcaaca attagaagta ccgtttattg ctttttatag aaaggagtat    1260
   gttcaaccgg aacttaatat taacgatttg tggaaagtat ataaatacga tgcaaagtgg    1320
   tgccaattga aaacacgcaa agaaaacctc ttgaagcttt ttgaaaaaat gaggtcatat    1380
   caaactgacc acataatgaa agatccagat gcaccaattc cagacaacct tcgtatcatg    1440
   actgagtccg acattgagcg attgaaaaat gttcaaaccg ccgaagagtt aaatgacgtt    1500
   cacaatcatt tcattttata ttatgctgca gatttgccag ccatgcatgc cgcgtggaga    1560
   gtcaaagaaa gagaaaggag aagacaggaa agaaaggagg ctagacttaa gctcatcgct    1620
   gaaagtgaag aaggtgctga aattcctgaa gagcctgagg aaattgacga tgatgaacca    1680
   gaagctgaaa ccttaaaata cgccaataga tcaggcagct atgcactatg taataaagga    1740
   ggtttgggtc ctctagcgaa gaaatttggt ttaacgcctg aagaatttgc cgaaaacctg 1800
   agagataatt atcagaggca cgaagtagat caagaacata tagaacctgt agaagtcgct 1860
   aaagaattcg tatcgccgaa atttcctaca gtggaagaag tgttgcaagc tactaaacac 1920
   atggtagctt tacaaatagc aagagaacca ttagtaagga aatgcgtaag agaaattttc 1980
   tttgaacgag ctagattaaa cgtttatcca accaaaaagg gtgtgaaagt tatagatgaa 2040
   gctcataatt gctatagtat gaagtatgta aaaaataaac cagtaagaga tcttgcaggc 2100
   gaccaatttt taaaattatg tttagccgaa gaggagaacc tccttactat taccatcaat 2160
   gaccatattc aaggaaacac tactaacaat tacattgatg aagtcaaaca attatatata 2220
   aaggatgaat tcagcaaaca cgttcaggat tggaatgcgc taagaatgga atcggtagaa 2280
   agggctttaa cgaaaagtgt cttaccagat ttaagatctg aattaaaacg aacgttgctc 2340
   acagaggcta aagaattcgt attaaaagct tgctgtagaa aattatataa ttggataaag 2400
   attgctccat acgcaataac ttttcccgat gaagacgaag atgattggga tacatccaaa 2460
   ggtgttagaa ctatgggtgt agcatacgta ccagagcaca cagtatcagc ttttacctgt 2520
   atttcagcac cagacggaga tataactgat tatctcagat taccaaatat tctaaaaaga 2580
   aaaaatagct ttcgaactga agaaaaactt atgaaggaag ctgatcttca agcactgaaa 2640
   aatttcatat tccttaaaaa gcctcatgtg atagcagtag ggggtgagtc cagagaagcc 2700
   ttaatgatcg ctgatgatat cagaggagta ataagtgaac taatagaatc cgaccaattc 2760
   ccacagatta gggttgaaat tattgataat gaattagcca aagtgtacgc aaattccatt 2820
   aaaggttcaa ctgatttcag agattatcca gagttgttaa ggcaagctat ttcattagct 2880
   cgaagaatgc aagatccttt ggttgaattt tcccaattat gtaatagcga tgaggaaatt 2940
   ttgagtctga ggtttcatcc cttgcaggaa caagtccaga aagaagaact actagaagct 3000
   ctctgtttag aatttgtcaa cagaacaaat gaagtaggtg tagatataaa tcttgccgtt 3060
   cagcagattc ataaaaagta gtttagttca attcatatgc ggtctaggac cgcgtaaagg 3120
   tcaagcgtta cttaaagttc tgaaacaaac taatcagagg cttgaaaaca gaacccaatt 3180
   ggttacattt tgtcatatgg gtccaaaagt ttttattaat tgttctggat tcataaagat 3240
   tgataccaat agtttaggag acagtactga ggcatatgtt gaaatattgg atggctctcg 3300
   agttcatccc gaaacttatg aatgggcacg aaaaatggct gtggatgctt tagaatatga 3360
   cgatgatgag ggagctaatc cggcgggagc tttagaggaa attctcgagg cgccagagag 3420
   gttaaaagat cttgacttgg atgcatttgc ggaggaattg gaaagacaag gaatttggta 3480
   acaagagtat aacattgtat gacattagag cagaactgaa ctcgcgatat aaagatttga 3540
   gacaaccttt tcgttctgca aatcctgaag aactattcga tatgcttact aaagaaactc 3600
   ccgaaacatt ttatattgga aaaatggtta catctaccgt gtttggcatt gcaaggagaa 3660
   aaccaaagtc agaccagctc gatcaagcta atccggtccg taatgacgaa actggtttgt 3720
   ggcagtgccc cttctgtttg aaaaatgatt ttcctgaatt atctgatgta tggaatcatt 3780
   ttgatgcagg agcatgtcct ggtcaagcta ctggagttaa actaagactg gataatggta 3840
   tattaggtta tatttatata aaaaatataa gcgacaaacc agttgctaat ccggaagaaa 3900
   gagtaggcat aggacaatta attcactgta gaataataaa aattgacgta gagaagttta 3960
   gtgttgattg tacatcgaaa tctagcgatc ttgctgataa aaatcatgaa tggagacctc 4020
   aacgagatcc tcattatgat caagaacgtg aagacaaaga caatcgatta gaagccgaga 4080
   agaagaaaga gaaacagcgt atgacgtaca tcaagagggt tatcgttcat ccggcgttcc 4140
   ataacatatc ctatgccgaa gccgaaaaat gtatggttaa tatggaccag ggtgaagtta 4200
   tcattaggcc ttcgaggaag ggcccggaac aatcgaacat aacgaagaag gttaaggaag 4260
   gaacatatca acacaaagat actaaagaaa aacgaaaact gaacaatggt acattaggaa 4320
   aatcattatg gattggaaat gaagaatttg aagatcttga tgaaataata gctaggcacg 4380
   ttacaccaat ggcggcgcat gctagagatt tactgtattt taggtattac aaagatttcc 4440
   aaggtggtca taaagataag gctgaggaat atctaaaaga tgaaaaaaag aaaaatgctt 4500
   ctaaaattca ttatgtagtt agtgctgcta agaatatacc tggtaaattt cttctttcgt 4560
   accttccacg caacaaagtt agacacgaat atgtaacagt tactccagaa ggctttcggt 4620
   ttagacaaca aatgtttgat tccgtttctt cgttatttaa atggttcaaa gaacatttcc 4680
   gggagcctcc accaggcggt gcaacaccag gtagcacgcc aaggatggct tcaagcagaa 4740
   ctggatatgg aagtgccaca cccgcgtata gtatgaataa tgaagctatt caaagagttg 4800
   ctcaaaattt accaagtcat gtagttcaag cgttatctgc tgccaccaac caaactccac 4860
   attatcccca cacccctggt tatggaggca attatattaa tacgccttac actccaagtg 4920
   gtcaaactcc atacatgact ccgtacgcta caccccatac gcagcaaact ccccgctatg 4980
   gtcatcaaac accttcccaa cacatggcga gctcagcacc acaaggtctc aacaatccct 5040
   ttttacatcc tggcgcggtg actccctccc aacgaactcc tatttatcgc aaccatcctg 5100
   cacaatctcc agtaatgctt cctacaagcc ctgtaccaag tccaggttcc cagagttcat 5160
   acagtagtca tttaagtcat aatcagcgaa gtggaagtta tgctgaatcc ttaagattcc 5220
   aacctcccga atcgccgaga agctcagtga gtaatagaag ctttcaaact gatagatacg 5280
   gcggtgatag gtatggtaaa ggaggaagtc atagatatgg tggaagctca aacgaagata 5340
   gatatggtaa gggaggagga ggaaatgaaa atacggattg gcagaaagct gcagaagcat 5400
   gggctcgatc tagatctact ccgagaagtg atggccgtaa tactccaaga tctgtaggtc 5460
   aacgaactcc tagatacgac aacgacgccg aacgttctag aatgaaacac ctcagcaaga 5520
   gcccgaggtc tgttaggtct actcctcgaa ccaatacatc tccacattct atgtccctag 5580
   gtgatgctac ccctctgtat gacgaaagta tctaaattac tttgtatttg gttatatagt 5640
   agggtaacag tgatattgta aatgctaaaa tcaacaatac agattcgcga tattaagtca 5700
   caatgacgac ctctcgtgta tttcggctga actagcatcg aggggtcttt tcaatgtcaa 5760
   acgctgagcg cacatgcatt tgaaacaagt aggcaatatt cacttattta tttttacatg 5820
   ttttaaatta attattgggt aatataaaat gattaacatg gaatcttcac gtttcaataa 5880
   aagttggcgt cgatattgta tataaaatta aaaaaaatat gtaaaaataa ataatattac 5940
   ctacttgtct caaatgcacg tgcgcccagc gtttgacatt gaaatcaccc tcaaagctag 6000
   ttcagacgaa atagacgaga ggtcgtcgtt gtgacttaac atcgtgaata gaaa 6054
<210> 77
   <211> 319
   <212> DNA
   <213> Diabrotica virgifera
<220>
   <221> misc_feature
   <222> (1)..(319)
   <223> RNAi target sequence
<400> 77
   tggtaaggga ggcaaagtta agggaaaagc aaagtcccga tcaaatcgtg ctggtttaca 60
   atttcctgta ggtcgtattc atcgtttatt gagaaaagga aattatgccg aaagagttgg 120
   tgctggagct cctgtatact tggcagctgt tatggaatat ttagctgctg aagttttgga 180
   attggcagga aatgcagcta gagatagcaa aaagacccgt ataattccta gacatttaca 240
   attggccata agaaatgacg aggaattgaa caaattactg tcaggagtta ccatcgccca 300
   aggtggagta ttgcctaat 319
<210> 78
   <211> 22
   <212> DNA
   <213> Artificial Sequence
<220>
   <223> primer
<220>
   <221> misc_feature
   <222> (1)..(22)
   <223> forward primer
<400> 78
   tggtaaggga ggcaaagtta ag 22
<210> 79
   <211> 22
   <212> DNA
   <213> Artificial Sequence
<220>
   <223> primer
<220>
   <221> misc_feature
   <222> (1)..(22)
   <223> reverse primer
<400> 79
   attaggcaat actccacctt gg 22
<210> 80
   <211> 456
   <212> DNA
   <213> Diabrotica virgifera
<220>
   <221> misc_feature
   <222> (1)..(456)
   <223> WCRW transcript ta07804.001_diavv
<400> 80
   gggggaaggg aggggagtat tcacttgatc ttcaaggtag attaacgcaa gtagaaatct      60
   aaaacatgtc tggacgtggt aagggaggca aagttaaggg aaaagcaaag tcccgatcaa     120
   atcgtgctgg tttacaattt cctgtaggtc gtattcatcg tttattgaga aaaggaaatt     180
   atgccgaaag agttggtgct ggagctcctg tatacttggc agctgttatg gaatatttag     240
   ctgctgaagt tttggaattg gcaggaaatg cagctagaga taacaaaaag acccgtataa     300
   ttcctagaca tttacaattg gccataagaa atgacgagga attgaacaaa ttactgtcag     360
   gagttaccat cgcccaaggt ggagtattgc ctaatataca agcagtactt ttacctaaaa     420
   agaccgaaaa aaaaaaaaaa aaaaaaaaaa aaaaaa                              456
<210> 81
   <211> 759
   <212> DNA
   <213> Diabrotica virgifera
<220>
   <221> misc_feature
   <222> (1)..(759)
   <223> RNAi target sequence
<400> 81
   atcatcctct gtgtttccgt tttgaactta acttgtattt agtttaaaaa ataatcatgt 60
   ctagaggaag cagtgcaggt tttgaccgac acataacaat tttctcgccc gaaggccgac 120
   tctatcaagt agagtatgct tttaaagcca ttaaccaagc cggccccact tcggtagcag 180
   tccgaggagt agatgctgcg gcgtgtgtga cccagagaaa gatcccggat aagctgattg 240
   atcccaacac aattacacat ctgttccagt taacagaaca cactggatgt gtgatgactg 300
   gcatgattgc tgacagcaag tcccaggtgc agagagctag atatgaggct gccgagttca 360
   aatataagtt tggatatgag atgccaatcg atgccttgtg taggagagta tcggatattt 420
   cccaggttta tacgcagaat gctgagatga gacctttggg ttgctccatg cttctgatag 480
   gatatgacca agaaatggga ccatgtgtcc acaaagctga ccctgctggc tattactgtg 540
   gctacagagc agtaagtgta ggatccaaac aaactgaagc caacagctac ctagagaaga 600
   agctaaagaa gaaaactgat ctgtccaaag atgaagtcat ccaattggca atcagttgtc 660
   tttcaagtgt tttatctgta gattttaaac caacagaagt cgaagttggg gttgttacta 720
   aggatgagcc caagtttagg aaattgacgg aacaagaaa 759
<210> 82
   <211> 22
   <212> DNA
   <213> Artificial Sequence
<220>
   <223> primer
<220>
   <221> misc_feature
   <222> (1)..(22)
   <223> forward primer
<400> 82
   atcatcctct gtgtttccgt tt 22
<210> 83
   <211> 22
   <212> DNA
   <213> Artificial Sequence
<220>
   <223> primer
<220>
   <221> misc_feature
   <222> (1)..(22)
   <223> reverse primer
<400> 83
   tttcttgttc cgtcaatttc ct 22
<210> 84
   <211> 957
   <212> DNA
   <213> Diabrotica virgifera
<220>
   <221> misc_feature
   <222> (1)..(957)
   <223> WCRW transcript ta04410.001
<400> 84
   ggggccccgg gggacagaag acagaacgtg acaacagaaa aatatagaaa atcatcctct      60
   gtgtttccgt tttgaactta acttgtattt agtttaaaaa ataatcatgt ctagaggaag     120
   cagtgcaggt tttgaccgac acataacaat tttctcgccc gaaggccgac tctatcaagt     180
   agagtatgct tttaaagcca ttaaccaagc cggccccact tcggtagcag tccgaggagt     240
   agatgctgcg gcgtgtgtga cccagagaaa gatcccggat aagctgattg atcccaacac     300
   aattacacat ctgttccagt taacagaaca cactggatgt gtgatgactg gcatgattgc     360
   tgacagcaag tcccaggtgc agagagctag atatgaggct gccgagttca aatataagtt     420
   tggatatgag atgccaatcg atgccttgtg taggagagta tcggatattt cccaggttta     480
   tacgcagaat gctgagatga gacctttggg ttgctccatg cttctgatag gatatgacca     540
   agaaatggga ccatgtgtct acaaagctga ccctgctggc tattactgtg gctacagagc     600
   agtaagtgta ggatccaaac aaactgaagc caacagctac ctagagaaga agctaaagaa     660
   gaaaactgat ctgtccaaag atgaagtcat ccaattggca atcagttgtc tttcaagtgt     720
   tttatctgta gattttaaac caacagaagt cgaagttggg gttgttacta aggatgagcc     780
   caagtttagg aaattgacgg aacaagaaat agatttccat ctgacagcta ttgctgagag     840
   ggattagata gattttttat attcactctt tgtaatgtta agaaatggtc aaattttgag     900
   ttaagtaaga aataaatttt tctcttataa aaaaaaaaaa aaaaaaaaaa aaaaaaa        957
<210> 85
   <211> 587
   <212> DNA
   <213> Diabrotica virgifera
<220>
   <221> misc_feature
   <222> (1)..(587)
   <223> RNAi target sequence
<400> 85
   aactttgtgt gttttggcag gtccgatggc tgcaataaga aaaaaattgg tgattgttgg     60
   tgacggagca tgtggaaaaa catgtctttt aatagtattt agtaaagatc agttcccaga    120
   agtatacgtg cccactgtat tcgaaaacta tgtggcggat attgaagtag atggaaagca    180
   ggtagaactc gccttatggg ataccgcagg tcaagaagat tatgatcgtc tgcggcccct    240
   ttcatatcca gacaccgatg tgattttaat gtgcttctca gtagactccc ccgactcatt    300
   agaaaacatc ccagaaaaat ggaccccaga ggtaaaacac ttttgtccaa atgtgccaat    360
   tattctggta ggcaataaga aggatctgcg taacgaccct actaccatta acgaacttaa    420
   aaagatgaaa caagaaccag taaaacctca agacggaaga tccatggcag agaaaatcaa    480
   tgcttttgca tatctagaat gttctgccaa aagtaaggag ggtgtaagag aggtttttga    540
   aaatgctacc cgagctgcac tgcaagtcaa aaagaagaag aagcccc                  587
<210> 86
   <211> 22
   <212> DNA
   <213> Artificial Sequence
<220>
   <223> primer
<220>
   <221> misc_feature
   <222> (1)..(22)
   <223> forward primer
<400> 86
   aactttgtgt gttttggcag gt 22
<210> 87
   <211> 20
   <212> DNA
   <213> Artificial Sequence
<220>
   <223> primer
<220>
   <221> misc_feature
   <222> (1)..(20)
   <223> reverse primer
<400> 87
   ggggcttctt cttctttttg 20
<210> 88
   <211> 1530
   <212> DNA
   <213> Diabrotica virgifera
<220>
   <221> misc_feature
   <222> (1)..(1530)
   <223> WCRW transcript ta15897.001_diavv
<400> 88
   ggggatttaa tgtccgccat tttcgttcgt agttgaatag gcgttaactt tgtgtgtttt      60
   ggcaggtccg atggctgcaa taagaaaaaa attggtgatt gttggtgacg gagcatgtgg     120
   aaaaacatgt cttttaatag tatttagtaa agatcagttc ccagaagtat acgtgcccac     180
   tgtattcgaa aactatgtgg cggatattga agtagatgga aagcaggtag aactcgcctt     240
   atgggatacc gcaggtcaag aagattatga tcgtctgcgg cccctttcat atccagacac     300
   cgatgtgatt ttaatgtgct tctcagtaga ctcccccgac tcattagaaa acatcccaga     360
   aaaatggacc ccagaggtaa aacacttttg tccaaatgtg ccaattattc tggtaggcaa     420
   taagaaggat ctgcgtaacg atcctactac cattaacgaa cttaaaaaga tgaaacaaga     480
   accagtaaaa cctcaagacg gaagatccat ggcagagaaa atcaatgctt ttgcatatct     540
   agaatgttct gccaaaagta aggagggtgt aagagaggtt tttgaaaatg ctacccgagc     600
   tgcactgcaa gtcaaaaaga agaagaagcc ccgttgtgta atgttttgag ccactacata     660
   ttctatattt tatattaata atcgttacgt gtgttaatta atttcaaggg ggcttctgca     720
   tgatgtattg actaacaaaa taaaccccta cttttctctt cagttctatt tgttcaaact     780
   ccttttctcc ttgtggacaa atttaagtat taagttatat tttcatatgt atttaaccca     840
   ttgtttattg gttcttagag ttacacgacc cagaaattta attttaaaac aacacaacga     900
   tgtagcaagt tactttttag agtttaatat agaatagatc attattttgt ttctttattc     960
   cccaccaaac tagtattgca cattgcaatt atttggctat gaattttaat tgattaatgt    1020
   aaaattttga tttttgaaat atccttagtt gtctcgattt ttgatgaact tccagtgagg    1080
   ggtgtgtaac ttataagctt tttttataca atggtaaatg atataatcag tgtcatgtgc    1140
   acaaaactta ttgaacgtcc acttagatca tcattgtagt actaatttac aagcatacgt    1200
   tgattcattt tatagtattt atattttata acctgatcaa tatattttgt ttcaaaaatt    1260
   tttttctgta tctatagtta tcttttacag atattcagag tacccattga tttttattta    1320
   gttcaatgac tgcagcacat ttattatgtt ttgattagaa gtcactgcaa atgaaacaag    1380
   tggattaaaa cttttgacta acagatacac aaaaaaatgt ttgtgttgca gttattgcac    1440
   tgggactaaa aggactaaca gtgatgtcat ctcttgtgta agggtagtta ctaccacctg    1500
   ttgtccttta agtctcaaaa ttaattttga                                     1530
<210> 89
   <211> 444
   <212> DNA
   <213> Diabrotica virgifera
<220>
   <221> misc_feature
   <222> (1)..(444)
   <223> RNAi target sequence
<400> 89
   cggcgttgta aaccttttgt aaacattaaa cattttgaaa attactgtat tgctgcagcg       60
   atttacgtag aagtatttta agatgaatgc gcctccgacc tttgaatcgt ttttattata      120
   cgatggagag aaaaagatca ttctagaaca agatacgaaa gttccaaatg cagccatttt      180
   tactatcaac aaagaggacc acacacttgg taatacgatc agaaaccaac ttttaaaaga      240
   tcccaacgta ttattcgctg gttacaaact acctcatcca ttggaacaca aatctgtctt      300
   gcgaattcaa acaacatcag actattcacc tcaagaagcc ttaatgcacg ctatcactga      360
   tttaatttca gaattgtcct tgttcgaaga aagatttaag gaagcgatca aagaaaagaa      420
   ggaaggattg gattaggcag tgaa                                             444
<210> 90
   <211> 21
   <212> DNA
   <213> Artificial Sequence
<220>
   <223> primer
<220>
   <221> misc_feature
   <222> (1)..(21)
   <223> forward primer
<400> 90
   cggcgttgta aaccttttgt a 21
<210> 91
   <211> 22
   <212> DNA
   <213> Artificial Sequence
<220>
   <223> primer
<220>
   <221> misc_feature
   <222> (1)..(22)
   <223> reverse primer
<400> 91
   ttcactgcct aatccaatcc tt 22
<210> 92
   <211> 575
   <212> DNA
   <213> Diabrotica virgifera
<220>
   <221> misc_feature
   <222> (1)..(575)
   <223> WCRW transcript ta06570.001_diavv
<400> 92
   caaataacag gaaattgaca ctgacagttc gttggacagg aatgattttt tcggcgttgt 60
<210> 93
   <211> 453
   <212> DNA
   <213> Diabrotica virgifera
<220>
   <221> misc_feature
   <222> (1)..(453)
   <223> RNAi target sequence
<400> 93
   ctggttgtca taggttgtac gcagttaaat tttttgtgat aaaacttagt gttcctttgt 60
   tatcaattaa catatataaa aatagtaatg gctagtcgga agaagacttt gttgaaggtc 120
   accatcctgg gtgattccag tgtgggaaag acttccttga tgaaccagta cgtaaatagg 180
   aaattctcca accaatacaa agcgaccata ggtgctgatt tccttacaaa agaactcaca 240
   gtggacgatc gaactgttac aatgcaaata tgggatactg caggtcagga aaggtttcag 300
   tccttaggtg tagcctttta cagaggggca gactgctgtg tgttagtatt tgatgtaact 360
   tcccccacct cattcaaatc cctggacagt tggcgagacg aattcctcat ccaagcatcg 420
   ccccgagagc cagaaaactt cccattgacc tca 453
<210> 94
   <211> 22
   <212> DNA
   <213> Artificial Sequence
<220>
   <223> primer
<220>
   <221> misc_feature
   <222> (1)..(22)
   <223> forward primer
<400> 94
   ctggttgtca taggttgtac gc 22
<210> 95
   <211> 22
   <212> DNA
   <213> Artificial Sequence
<220>
   <223> primer
<220>
   <221> misc_feature
   <222> (1)..(22)
   <223> reverse primer
<400> 95
   tgaggtcaat gggaagtttt ct 22
<210> 96
   <211> 640
   <212> DNA
   <213> Diabrotica virgifera
<220>
   <221> misc_feature
   <222> (1)..(640)
   <223> WCRW transcript ta40942.001_diavv
<400> 96
   tagtaatggc tagtcggaag aagactttgt tgaaggtcac catcctgggt gattccagtg        60
   tgggaaagac ttccttgatg aaccagtacg taaataggaa attctccaac caatacaaag       120
   cgaccatagg tgctgatttc cttacaaaag aactcacagt ggacgatcga actgttacaa       180
   tgcaaatatg ggatactgca ggtcaggaaa ggtttcagtc cttaggtgta gccttttaca       240
   gaggggcaga ctgctgtgtg ttagtatttg atgtaacttc ccccacctca ttcaaatccc       300
   tggacagttg gcgagacgaa ttcctcatcc aagcatcgcc ccgagagcca gaaaacttcc       360
   cattgacctc aagcgttatt atccttataa tacccttggt accttaataa ctataagtac       420
   aagaagattc cactataata ttgttacaaa ttaagaaagt aacatattag atatattcaa       480
   tcctttatat ctctgaaaat attaaaaaaa gcatacatct gatttacaaa agaaataagt       540
   tgtaaaaatg cataagctct cagctgtaaa tgtttttttg tgtcctttag tattattcca       600
   gtgtttttaa tatatctcat aatattggaa tattttttaa                             640
<210> 97
   <211> 601
   <212> DNA
   <213> Diabrotica virgifera
<220>
   <221> misc_feature
   <222> (1)..(601)
   <223> RNAi target sequence
<400> 97
   gtgaacgcca ttgtgaactc taacgacttt ggaaccaagc tgaagtaatc cgtgattttt        60
   tgtgccatcg gtgtttccta gtacttttta ttcacaagtt taaattcaaa aaatcatggc       120
   tggcgaacaa gatatgccca ccttcaaatg cgtcttggtc ggagatggag gtactggcaa       180
   aactaccttt gtaaaaagac acatgactgg tgaattcgaa aagaaatatg tagcaacctt       240
   aggagtagaa gttcaccctt tagttttcca cacaaataga ggacaaattc gttttaacgt       300
   atgggacaca gcaggccaag aaaagtttgg tggtctgcgt gatggatact acatccaagg       360
   acaatgtgcc ataattatgt ttgatgttac atctagagtc acttacaaaa atgttccaaa       420
   ctggcacaga gatcttgtta gagtttgtga aaatatccca attgtattgt gtggtaacaa       480
   agttgacatc aaagatagga aagttaaggc aaagagcatt gtcttccata ggaagaagaa       540
   tttacaatac tatgacatct ctgcaaaatc caactacaac tttgaaaaac ccttcttgtg       600
   g                                                                       601
<210> 98
   <211> 22
   <212> DNA
   <213> Artificial Sequence
<220>
   <223> primer
<220>
   <221> misc_feature
   <222> (1)..(22)
   <223> forward primer
<400> 98
   gtgaacgcca ttgtgaactc ta 22
<210> 99
   <211> 22
   <212> DNA
   <213> Artificial Sequence
<220>
   <223> primer
<220>
   <221> misc_feature
   <222> (1)..(22)
   <223> reverse primer
<400> 99
   ccacaagaag ggtttttcaa ag 22
<210> 100
   <211> 1075
   <212> DNA
   <213> Diabrotica virgifera
<220>
   <221> misc_feature
   <222> (1)..(1075)
   <223> WCRW transcript ta05966.001_diavv
<400> 100
   tatttggcgc gcattcctga cacctacgcg ggtcttcttg tgaacgccat tgtgaactct      60
   aacgactttg gaaccaagct gaagtaatcc gtgatttttt gtgccatcgg tgtttcctag     120
   tactttttat tcacaagttt aaattcaaaa aatcatggct ggcgaacaag atatgcccac     180
   cttcaaatgc gtcttggtcg gagatggagg tactggcaaa actacctttg taaaaagaca     240
   catgactggt gaattcgaaa agaaatatgt agcaacctta ggagtagaag ttcacccttt     300
   agttttccac acaaatagag gacaaattcg ttttaacgta tgggacacag caggccaaga     360
   aaagtttggt ggtctgcgtg atggatacta catccaagga caatgtgcca taattatgtt     420
   tgatgttaca tctagagtca cttacaaaaa tgttccaaac tggcacagag atcttgttag     480
   agtttgtgaa aatatcccaa ttgtattgtg tggtaacaaa gttgacatca aagataggaa     540
   agttaaggca aagagcattg tcttccatag gaagaagaat ttacaatact atgacatctc     600
   tgcaaaatcc aactacaact ttgaaaaacc cttcttgtgg ttggctagga aactcattgg     660
   agatcccaac ttagaattcg tagctgcacc tgccttgctt cctccagaag tccagatgga     720
   tccccaatgg cagcaacaaa tcgaaaagga tcttaaagaa gcctcagaaa ctgcactgcc     780
   cgatgatgac gaagatttat aaactcatta accaattact aaaactctgt catcgttcca     840
   aagcgcgtgt gtaacaattg atatcttaca agacttaagt taaattacat tttatgtatt     900
   tctgaagcag aactgttgat atttttatat aaatgttttc taaatttgtt gaaaggtttg     960
   tctggagtct ggacgtatgt tcttcaatat attagtggta tggttgttag agtgttagag    1020
   tcagcaagct accgtaacag ttcttgtcac ataggagtgt agtcttaatt tcaat         1075
<210> 101
   <211> 614
   <212> DNA
   <213> Diabrotica virgifera
<220>
   <221> misc_feature
   <222> (1)..(614)
   <223> RNAi target sequence
<400> 101
   caacaatttt cgcatctttc tgtctgttcc cggcgttctt tgaaaaagtt ttcaataata 60
   ctctaaaaaa tggcagacgc tgatgatcta ttagattatg aagatgagga acagacagaa 120
   caaaccgcaa ctgaaacggc aactacagag gtacagaaaa agggtgtcaa gggcacatat 180
   gtatcaatac acagttctgg gtttagagat tttctgttaa aaccagcaat tctcagagct 240
   atagtggact gcgggttcga acatccttca gaagttcaac atgaatgtat tcctcaagct 300
   gtcattggca tggatattct gtgccaagct aaatccggta tgggaaaaac ggctgttttt 360
   gtattagcta cactccaagt aatagatcct acagaaaatg ttgtatatgt tctcgtcatg 420
   tgccatacca gagagttagc cttccagata agcaaagagt acgaacgttt cagtaaatat 480
   atgcccaata ttaaagtagg ggtcttcttt ggtggcttgc ctatccagaa agatgaggaa 540
   acgttaaaaa ataattgccc gcatatcgtt gtgggtactc caggaagaat tttagcattg 600
   gtcagatcga aaaa 614
<210> 102
   <211> 22
   <212> DNA
   <213> Artificial Sequence
<220>
   <223> primer
<220>
   <221> misc_feature
   <222> (1)..(22)
   <223> forward primer
<400> 102
   caacaatttt cgcatctttc tg 22
<210> 103
   <211> 22
   <212> DNA
   <213> Artificial Sequence
<220>
   <223> primer
<220>
   <221> misc_feature
   <222> (1)..(22)
   <223> reverse primer
<400> 103
   tttttcgatc tgaccaatgc ta 22
<210> 104
   <211> 1515
   <212> DNA
   <213> Diabrotica virgifera
<220>
   <221> misc_feature
   <222> (1)..(1515)
   <223> WCRW transcript ta03347.002-diavv
<400> 104
   ttggaaaacg caatatatct atagaaaaca agcggtttat ttgccgccat tttgacaagc      60
   ttacgtacct acatttttaa ttcgttgttt tatttttagt tttcaataat actctaaaaa     120
   atggcagacg ctgatgatct attagattat gaagatgagg aacagacaga acaaaccgca     180
   actgaaacgg caactacaga ggtacagaaa aagggtgtca agggcacata tgtatcaata     240
   cacagttctg ggtttagaga ttttctgtta aaaccagcaa ttctcagagc tatagtggac     300
   tgcgggttcg aacatccttc agaagttcaa catgaatgta ttcctcaagc tgtcattggc     360
   atggatattc tgtgccaagc taaatccggt atgggaaaaa cggctgtttt tgtattagct     420
   acactccaag taatagatcc tacagaaaat gttgtatatg ttctcgtcat gtgccatacc     480
   agagagttag ccttccagat aagcaaagag tacgaacgtt tcagtaaata tatgcccaat     540
   attaaagtag gggtcttctt tggtggcttg cctatccaga aagatgagga aacgttaaaa     600
   aataattgcc cgcatatcgt tgtgggtact ccaggaagaa ttttagcatt ggtcagatcg     660
   aaaaaactta atctcaaaca tctaaagcat tttattttgg atgaatgtga taaaatgttg     720
   gagttattag acatgagacg tgatgttcaa gaaatatatc gtaacactcc ccacgaaaaa     780
   caagtcatga tgttcagtgc caccttaagt aaagaaatta gaccagtttg caagaaattt     840
   atgcaagatc ccatggaagt atatgttgac gacgaagcta aattaacatt gcatggtcta     900
   cagcaacatt atgtaaaact aaaagaaaac gaaaagaata aaaagttatt tgagttacta     960
   gatgttttgg agttcaacca ggttgtcatt ttcgtaaaat ccgtacaaag gtgtgtggcc    1020
   ctggcacaat tactgactga acagaacttc ccagcgatag gaattcacag aggaatggac    1080
   caaaaagaac gactgtcccg gtatgaacaa ttcaaggatt tccaaaagag gattctagta    1140
   gccacaaact tatttggacg tggaatggat atcgaaagag tgaacatcgt cttcaactat    1200
   gacatgcccg aagattctga cacttacctg cacagagtag cccgagctgg acgtttcggt    1260
   accaaaggtc tagccattac tttcgtatca gaagagggag atgccaaaat attgaacgaa    1320
   gtacaagaaa gatttgatgt taatattact gaattacctg acgaaataga tctcagttca    1380
   tacattgaag gacgataaga atttcaagat ctttgaagaa ttaattttaa gatttagaat    1440
   aagatttcca ctaggtttaa cgatttcaat ttgtattatg tttcaatgtt attgcattag    1500
   tattaaaata aatgg                                                     1515
<210> 105
   <211> 683
   <212> DNA
   <213> Diabrotica virgifera
<220>
   <221> misc_feature
   <222> (1)..(683)
   <223> RNAi target sequence
<400> 105
   ggcatcttgt ctcgtttatc gtaactcaaa ttaaacgtaa agatgtcggg aggaatcgag      60
   gctttaagcc ttaaagagga cgatgttacc aagatgttgg cggcttccac ccatcttgga     120
   acagcaaatg ttgattttca aatggaacag tatgtataca aacgtcgtca tgacggtact     180
   cacatcatca acttaaggaa aacgtgggaa aagattctct tggcagctcg tgccattgtt     240
   gctattgaac accctggtga agttttcgta atttcatctc gtccccatgg ccagcgtgct     300
   gttcttaagt ttgctgctca cactggagct acccctattg ctggaagatt tactcctggt    360
   gcttttacca atcagatcca agctgcattc agggaacctc gtctgttgat cgtcactgac     420
   cctgcttttg atcaccaacc tatcactgaa gcctcttacg taaacattcc agttattgct     480
   ttgtgcaata ctgattctcc attacgtttt gttgatattg ccattcccgg aaacaacaaa     540
   gcctcacaca gtattggttt actgtggtgg cttttggcta gagaggttct ccgtcttaga     600
   ggaatcattc cacgggatac caaatgggat gttgtagtag atcttttctt ctaccgtgaa     660
   ccagaagagg ctgaaaagga aga                                             683
<210> 106
   <211> 22
   <212> DNA
   <213> Artificial Sequence
<220>
   <223> primer
<220>
   <221> misc_feature
   <222> (1)..(22)
   <223> forward primer
<400> 106
   ggcatcttgt ctcgtttatc gt 22
<210> 107
   <211> 22
   <212> DNA
   <213> Artificial Sequence
<220>
   <223> primer
<220>
   <221> misc_feature
   <222> (1)..(22)
   <223> reverse primer
<400> 107
   tcttcctttt cagcctcttc tg 22
<210> 108
   <211> 822
   <212> DNA
   <213> Diabrotica virgifera
<220>
   <221> misc_feature
   <222> (1)..(822)
   <223> WCRW transcript ta15911.001_diavv
<400> 108
   gggggacaga gacaggacat aagcattaaa agggacgtaa aaacaacaaa ggggctcttt      60
   ctcgtgcaaa taacccaagt ggcatcttgt ctcgtttatc gtaactcaaa ttaaacgtaa     120
   agatgtcggg aggaatcgag gctttaagcc ttaaagagga cgatgttacc aagatgttgg     180
   cggcttccac ccatcttgga acagcaaatg ttgattttca aatggaacag tatgtataca     240
   aacgtcgtca tgacggtact cacatcatca acttaaggaa aacgtgggaa aagattctct     300
   tggcagcccg tgccattgtt gctattgaac accctggtga agttttcgta atttcatctc     360
   gtccctatgg ccagcgtgct gttcttaagt ttgctgctca cactggagct acccctattg     420
   ctggaagatt tactcctggt gcttttacca atcagatcca agctgcattc agggaacctc     480
   gtctgttgat cgtcactgac cctgcttttg atcaccaacc tatcactgaa gcctcttacg     540
   taaacattcc agttattgct ttgtgcaata ctgattctcc attacgtttt gttgatattg     600
   ccattcccgg aaacaacaaa gcctcacaca gtattggttt actgtggtgg cttttggcta     660
   gagaggttct ccgtcttaga ggaatcattc cacgggatac caaatgggat gttgtagtag     720
   atcttttctt ctaccgtgaa ccagaagagg ctgaaaagga agaacaagct gccaaagaag     780
   ctgtggctgc tgttgtcaaa cctgtagata tcccagctcc tc                        822
<210> 109
   <211> 559
   <212> DNA
   <213> Diabrotica virgifera
<220>
   <221> misc_feature
   <222> (1)..(559)
   <223> RNAi target sequence
<400> 109
   ggatggttta ttgtctggaa gctcattcta agtagattcc gttttattaa agaactgttg 60
<210> 110
   <211> 22
   <212> DNA
   <213> Artificial Sequence
<220>
   <223> primer
<220>
   <221> misc_feature
   <222> (1)..(22)
   <223> forward primer
<400> 110
   ggatggttta ttgtctggaa gc 22
<210> 111
   <211> 22
   <212> DNA
   <213> Artificial Sequence
<220>
   <223> primer
<220>
   <221> misc_feature
   <222> (1)..(22)
   <223> reverse primer
<400> 111
   tattttggct caatttcgac ag 22
<210> 112
   <211> 903
   <212> DNA
   <213> Diabrotica virgifera
<220>
   <221> misc_feature
   <222> (1)..(903)
   <223> WCRW transcript ta05802.003-diavv
<400> 112
   aaatatgcac ttgtgacggc ctttgaccta aatgtttagt taggaacaaa atggcgaaat      60
   gttggaactg ataaacatca ctgccttttt gtaatagtta agaattagat ttttaattgg     120
   aaaattgtaa tgtagcaaat tacttaagcg cctaactcca agacccatta atgcattcag     180
   gttaaacagt ttattttatt taggatggtt tattgtctgg aagctcattc taagtagatt     240
   ccgttttatt aaagaactgt tgaagggagc aagtgactct acaattacgg aattgaaaaa     300
   cactaggagt aaactcaaga ggacaagaaa ggactgattt atcttctcaa aatgaatatt     360
   cgatgtgcaa aaccagaaga tcttatgaac atgcagcact gcaatctgtt atgtctacca     420
   gaaaactatc agatgaaata ttacttttat catggcctta gttggcccca actaagttat     480
   gtagctgaag acgaaaaagg taacatagtt ggttatgtcc tagcaaaaat ggaagaagac     540
   caagaagatc tcaagcatgg tcacataacc tccctggcag tgaagcggtc ccacaggcga     600
   ttagggctag cccagaaact gatggatcaa gcttccgaag ccatggtcga atgttttgac     660
   gcaaaatatg tttcgctgca tgttcgtaaa agcaatcgcg ccgctttaaa tctatacaaa     720
   aactctttga aatttgaaac tgtcgaaatt gagccaaaat attatgcaga tggtgaagat     780
   gcatattcca tgagacgaga cttatctgaa ttcgctaaga aagttaaacc taagtctgag     840
   actaaaaacg aagaatagtg taattatttg taatttttgt atttatagcg aatataatat     900
   aca                                                                    903
<210> 113
   <211> 368
   <212> DNA
   <213> Diabrotica virgifera
<220>
   <221> misc_feature
   <222> (1)..(368)
   <223> RNAi target sequence
<400> 113
   ggttgcagaa gatttcacaa aactttatta tgatactctg gataaacgac ggcatttgat        60
   agcaagactg tatttggaga cgggattatt atcatggaat ggtaatggtg taactgggaa       120
   tgaaaatatt ttgaaattta tgattgattt accaccgtca tcttttacta tacaaactct       180
   agacgcacag cctgttttag attctgcagt aaatggacaa ctgaccttca tcactcaagt       240
   gactggcagt gtaaaatacc aagagaaaac accaaaaacc tttcagcgaa acttcataat       300
   tacagcacag ggcgataaat ggaaaattgt cagtgattgt atgaggctgc aagaaccatt       360
   aaacgcta                                                                368
<210> 114
   <211> 21
   <212> DNA
   <213> Artificial Sequence
<220>
   <223> primer
<220>
   <221> misc_feature
   <222> (1)..(21)
   <223> forward primer
<400> 114
   gttgcagaag atttcacaaa a 21
<210> 115
   <211> 22
   <212> DNA
   <213> Artificial Sequence
<220>
   <223> primer
<220>
   <221> misc_feature
   <222> (1)..(22)
   <223> reverse primer
<400> 115
   tagcgtttaa tggttcttgc ag 22
<210> 116
   <211> 470
   <212> DNA
   <213> Diabrotica virgifera
<220>
   <221> misc_feature
   <222> (1)..(470)
   <223> WCRW transcript ta08838.001_diavv
<400> 116 actaacaact gtatttttaa gtattttgat aaataaagta attggtagac 470
<210> 117
   <211> 589
   <212> DNA
   <213> Diabrotica virgifera
<220>
   <221> misc_feature
   <222> (1)..(589)
   <223> RNAi target sequence
<400> 117
   acctggctga tgcaatagat ttggtaaaag ggttttctac ctataaattt acaatatatt 60
   attataaata gactaaactc atcaataaaa aagtttaagc aaatagccat atagctacaa 120
   tggccgaaga cgatgcaatc tttgatccaa ccctcaaaaa aaagaaaaag aagaagaaga 180
   ccactttcga cattgatgct gctctagctg aaggtaacat tgaaaatgat tcagggaatg 240
   ccccagctgc agaagctgat aatattgaat caaaacctga cgatgatcag gatgatggaa 300
   atgtggacat tgatttagat ttcactaaaa agaaaaagaa gaagaaaaag cctttctcag 360
   ctgatgattt agattcttcc ccagctgata ataaagctga aactactcaa gatggagctg 420
   gggacaatga tgggggtatg gatgataact ttgatttaga tatggacttt agtaaaaaga 480
   agaagaagaa aaccaagaaa cctaagaatt tagacgaact tattgcagaa actgaggaga 540
   aggatgatga tcaggagaat gtagatgaaa attcaacaca atgggtcaa 589
<210> 118
   <211> 22
   <212> DNA
   <213> Artificial Sequence
<220>
   <223> primer
<220>
   <221> misc_feature
   <222> (1)..(22)
   <223> forward primer
<400> 118
   acctggctga tgcaatagat tt 22
<210> 119
   <211> 22
   <212> DNA
   <213> Artificial Sequence
<220>
   <223> primer
<220>
   <221> misc_feature
   <222> (1)..(22)
   <223> reverse primer
<400> 119
   ttgacccatt gtgttgaatt tt 22
<210> 120
   <211> 1418
   <212> DNA
   <213> Diabrotica virgifera
<220>
   <221> misc_feature
   <222> (1)..(1418)
   <223> WCRW transcript ta00006.001_diavv
<400> 120
   ggggtggaga agggtttcgt gtgaacagcc gttgcacacg agtcagtcga tcctaagccc      60
   taggcgaaag ccgatgttga tgtggttggg ggacaacctg gctgatgcaa tagatttggt     120
   aaaagggttt tctacctata aatttacaat atattattat aaatagacta aactcatcaa     180
   taaaaaagtt taagcaaata gccatatagc tacaatggcc gaagacgatg caatctttga     240
   tccaaccctc aaaaaaaaga aaaagaagaa gaagaccact ttcgacattg atgctgctct     300
   agctgaaggt aacattgaaa atgattcagg gaatgcccca gctgcagaag ctgataatat     360
   tgaatcaaaa cctgacgatg atcaggatga tggaaatgtg gacattgatt tagatttcac     420
   taaaaagaaa aagaagaaga aaaagccttt ctcagctgat gatttagatt cttccccagc     480
   tgataataaa gctgaaacta ctcaagatgg agctggggac aatgatgggg gtatggatga     540
   taactttgat ttagatatgg actttagtaa aaagaagaag aagaaaacca agaaacctaa     600
   gaatttagac gaacttattg cagaaactga ggagaaggat gatgatcagg agaatgtaga     660
   tgaaaattca acacaatggg tcaattccga tcgggactac acatatgacg aattactaaa     720
   ccgtgccttt gaaataatga gagataaaaa tccagatatg gctgccggta agaaacagaa     780
   gttcgtcatg agacctcctc aagtagtcaa aatcggcgcc aaaaagacgt catttgcgaa     840
   tttcacagag atatgcaaaa tgcttcatcg tcaacccaag catcttttag attttttact      900
   agctgaattg ggtaccagcg ggtctgtgga cggtaataat cagctcatca ttaaaggaag     960
   gtttcaacag aaacaaattg aaaatgtact caggagatac atcaaggaat acgtcacttg    1020
   tcatacatgt agatccccag atacaattct tcaaaaagac actagattgt tcttcttaca    1080
   atgtgaaacg tgtggttcta ggtgttctgt tgctagtatt aaatcaggtt tccaggctgt    1140
   cacttccaaa cgtgctgcaa ttcgagccaa gacagcatag gaaccttaga tattgcaata    1200
   caataataaa taggcactgt tacagaagaa atttcaaagt gctaaagaat aggtgttgta    1260
   tgtgttatat aatttgtact ataaatctag ctgaattgtt aaatggaatt aacaatcata    1320
   ttaatatcaa caattttaat tatataactt ttttattgat tattaaattt taataaacag    1380
   taaataatta tttatatttt tcgtccataa aaaaaaaa                              1418
<210> 121
   <211> 610
   <212> DNA
   <213> Diabrotica virgifera
<220>
   <221> misc_feature
   <222> (1)..(610)
   <223> RNAi target sequence
<400> 121
   gtagcggaac cgtcaaaaag tcgggccctc gcaagagagt tcgtcggggt aacccaaaag      60
   gacctggaga cgtcgtcgag agatccggga agaatctcgc actcttctcg aacaagccga     120
   ccgtgctcgt cgccaagctg aacaagaatt gggagatgct cacgaacaat tgaacgatct     180
   tggagcccaa aacgcttcct tgtctgccgc caagaggaaa ttggaaaccg aattgcaaac     240
   cctccactca gaccttgacg aacttctcaa cgaagccaag aactccgaag agaaggccaa     300
   gaaggctatg gtagatgccg ctcgtctcgc tgatgaactc cgcgctgaac aagaccatgc     360
   ccaaacacaa gagaaactcc gtaaagctct cgaagctcaa atcaaggacc tccaagtacg     420
   cttggacgaa gctgaagcca atgcccttaa aggaggaaag aaggctattg ccaaattgga     480
   acaacgtgtt agagaattag aaaatgaatt agacggtgaa caaaggcggc acgcggatgc     540
   ccaaaagaac ttgagaaagt ctgaacgtcg tatcaaggaa cttagcttcc aagctgaaga     600
   agaccgcaag                                                            610
<210> 122
   <211> 21
   <212> DNA
   <213> Artificial Sequence
<220>
   <223> primer
<220>
   <221> misc_feature
   <222> (1)..(21)
   <223> forward primer
<400> 122
   gtagcggaac cgtcaaaaag t 21
<210> 123
   <211> 20
   <212> DNA
   <213> Artificial Sequence
<220>
   <223> primer
<220>
   <221> misc_feature
   <222> (1)..(20)
   <223> reverse primer
<400> 123
   cttgcggtct tcttcagctt 20
<210> 124
   <211> 669
   <212> DNA
   <213> Diabrotica virgifera
<220>
   <221> misc_feature
   <222> (1)..(669)
   <223> WCRW transcript ta41130.001_diavv
<400> 124
   gggcacaccc attgggcgaa agggaatccg gttcctattc cggaacccgg tagcggaacc      60
   gtcaaaaagt cgggccctcg caagagagtt cgtcggggta acccaaaagg acctggagac     120
   gtcgtcgaga gatccgggaa gaatctcgca ctcttctcga acaagccgac cgtgctcgtc     180
   gccaagctga acaagaattg ggagatgctc acgaacaatt gaacgatctt ggagcccaaa     240
   acgcttcctt gtctgccgcc aagaggaaat tggaaaccga attgcaaacc ctccactcag     300
   accttgacga acttctcaac gaagccaaga actccgaaga gaaggccaag aaggctatgg     360
   tagatgccgc tcgtctcgct gatgaactcc gcgctgaaca agaccatgcc caaacacaag     420
   agaaactccg taaagctctc gaagctcaaa tcaaggacct ccaagtacgc ttggacgaag     480
   ctgaagccaa tgcccttaaa ggaggaaaga aggctattgc caaattggaa caacgtgtta     540
   gagaattaga aaatgaatta gacggtgaac aaaggcggca cgcggatgcc caaaagaact     600
   tgagaaagtc tgaacgtcgt atcaaggaac ttagcttcca agctgaagaa gaccgcaaga     660
   accacgaga                                                             669
<210> 125
   <211> 628
   <212> DNA
   <213> Diabrotica virgifera
<220>
   <221> misc_feature
   <222> (1)..(628)
   <223> RNAi target sequence
<400> 125
   ccagtattta gaccggcatc tcgtttttcc cttattggaa tttctgtccg caaaaggaat      60
   ttacaatgag accgaactgt taaaagccaa attagatatt ctaagcaaga ctaatatgat     120
   agattatgct attgatatca gaaaacaact gtaccctgat caggaaatac ccgaggacct     180
   aaaacagagg cgtaatcatg ttgtgcagca gctagcagag ttgcaagaag aggttaagcc     240
   tatccttaag attatggaat ctgaagaagt tatgaagaat atggaaaaca tgagagattc     300
   gaaaacactt attacatatt tgtcaaaaga attcaagttt gaaattgaat tgatcgacag     360
   tcttcacaaa ctagccaaat acagatacga ctgtggcaac tacacagtat ctacatccta     420
   cctgtacttc tgtatgctgg tattacctcc taatgacagg aactatctta gtgccttgtg     480
   gggaaaattc gcttcagaaa ttctgtacca aaactgggat tcagccctgg aagatctcaa     540
   caaactgagg gaatatattg attccagtcc caatcagttt tctggcaaca gtttgcagct     600
   tctccaacag aggacttggt tgatccat                                        628
<210> 126
   <211> 22
   <212> DNA
   <213> Artificial Sequence
<220>
   <223> primer
<220>
   <221> misc_feature
   <222> (1)..(22)
   <223> forward primer
<400> 126
   ccagtattta gaccggcatc tc 22
<210> 127
   <211> 22
   <212> DNA
   <213> Artificial Sequence
<220>
   <223> primer
<220>
   <221> misc_feature
   <222> (1)..(22)
   <223> reverse primer
<400> 127
   atggatcaac caagtcctct gt 22
<210> 128
   <211> 1547
   <212> DNA
<220>
   <221> misc_feature
   <222> (1)..(1547)
   <223> WCRW transcript ta05495.001_diavv
<400> 128
   tttttagagg ctacttctcg tagctctgct tgttgaattt taaaatattg aaagtagtaa      60
   atattgaaga aagtattaat aacacaatca tggcaaagtt cgacttaact tcgaagatgg     120
   gccagtattt agaccggcat ctcgtttttc ccttattgga atttctgtcc gcaaaaggaa     180
   tttacaatga gaccgaactg ttaaaagcca aattagatat tctaagcaag actaatatga     240
   tagattatgc tattgatatc agaaaacaac tgtaccctga tcaggaaata cccgaggacc     300
   taaaacagag gcgtaatcat gttgtgcagc agctagcaga gttgcaagaa gaggttaagc     360
   ctatccttaa gattatggaa tctgaagaag ttatgaagaa tatggaaaac atgagagatt     420
   cgaaaacact tattacatat ttgtcaaaag aattcaagtt tgaaattgaa ttgatcgaca     480
   gtcttcacaa actagccaaa tacagatacg actgtggcaa ctacacagta tctacatcct     540
   acctgtactt ctgtatgctg gtattacctc ccaatgacag gaactatctt agtgccttgt     600
   ggggaaaatt cgcttcagaa attctgtacc aaaactggga ttcagccctg gaagatctca     660
   acaaactgag ggaatatatt gattccagtc ccaatcagtt ttctggcaac agtttgcagc     720
   ttctccaaca gaggacttgg ttgatccatt ggtctctctt tgtgtttttc aatcatgtta     780
   tgggcagaga attgattatt gaaatgttct tgtatagacc acactacctc aacgccatcc     840
   agaccatgtg tcctcatatc ttgaggtatt tggctacagc tgtaattatt aatcgtggaa     900
   ggagatcagc actgaaggac ttagttaaag ttattcagca agaaagctac acttacagag     960
   atcccattac agagtttgtg gaacaccttt acgtcaactt cgatttcgat ggagcccgtc    1020
   aaaaattaca cgaatgccaa acagttttac taaacgactt cttcctaatt tcctgtttag    1080
   acgaattcgt agaaaacgcc agacttatga tcttcgagac attctgcaga attcatcaat    1140
   gcatcagtat aggcatgtta gccgaaaagc tcaacatgaa tccagacgaa gctgaatgct    1200
   ggatcgtgaa tttgatcaga aacgcacgat tggatgctaa gatcgattct aagttagggc    1260
   atgtggttat gggtgctcaa cctttatcgc cataccagca acttattgaa aaaattgatt    1320
   ccctttcggt caggtcagaa actctgtgtg ctcttataga tagaaagctc agagcgagga    1380
   ctgacgtaag tttctaaagg tacgttaacg aaatgatgca agaatttgtc atttttgtta    1440
   cagatacgtt ggggaaatcc agatttttaa atatctgtta ttttgtaata attttattgg    1500
   atacaaataa aagatctttc cagtatgtca aaaaaaaaaa aaaaaaa                  1547
<210> 129
   <211> 619
   <212> DNA
   <213> Diabrotica virgifera
<220>
   <221> misc_feature
   <222> (1)..(619)
   <223> RNAi target sequence
<400> 129
   tcacattcca gttgtttaga gcacttagta gagatataca cctttaagca tgtcgtgttc        60
   agcgatatat attttagatg taaaaggaaa agtcttaatc tctaggaact atagaggcga       120
   tatagaccta ggtgtaatag agaagtttat gcctttatta atggaaaaag aagaagaagg       180
   gcttctaact ccaattttgc aaatccctga atgcaccttc tcatatatta aaaccaacaa       240
   tctatatata gtaagcacta cgaagaaaaa tggtaatatc gctcttgtat ttgtattttt       300
   acataaaatc gttcaagtaa tgactgaata ttttaaagaa ttagaagaag aaagtatcag       360
   agataatttt gtcgttattt atgagttgtt ggacgaactt ttagactttg gctatcctca       420
   aacaactgat agcaaaattc ttcaagaata catcactcaa gaaggtcata aactagagat       480
   acaaccaaga attcctgtag ctgtgacaaa tgcagtttca tggcgttctg agggaatcaa       540
   atacaggaaa aatgaagtat tcttagatgt aatcgagtca gtaaatcttt tagcaagtgc       600
   aaatggcaat gtactaaga                                                    619
<210> 130
   <211> 20
   <212> DNA
   <213> Artificial Sequence
<220>
   <223> primer
<220>
   <221> misc_feature
   <222> (1)..(20)
   <223> forward primer
<400> 130
   tcacattcca gttgtttaga 20
<210> 131
   <211> 20
   <212> DNA
   <213> Artificial Sequence
<220>
   <223> primer
<220>
   <221> misc_feature
   <222> (1)..(20)
   <223> reverse primer
<400> 131
   tcttagtaca ttgccatttg 20
<210> 132
   <211> 2131
   <212> DNA
   <213> Diabrotica virgifera
<220>
   <221> misc_feature
   <222> (1)..(2131)
   <223> WCRW transcript ta08478.001_diavv
<400> 132
   agtaaataat aatgattata atatggtttg caaatagtta aatgcagtat gtgattttct      60
   gaagaagtga agaggttata tcacattcca gttgtttaga gcacttagta gagatataca     120
   cctttaaaca tgtcgtgttc agcgatatat attttagatg taaaaggaaa agtcttaatc     180
   tctaggaact atagaggcga tatagaccta ggtgtaatag agaagtttat gcctttatta     240
   atggaaaaag aagaagaagg gcttctaact ccaattttgc aaatccctga atgcaccttc     300
   tcatatatta aaaccaacaa tctatatata gtaagcacta cgaagaaaaa tggtaatatc     360
   gctcttgtat ttgtattttt acataaaatc gttcaagtaa tgactgaata ttttaaagaa     420
   ttagaagaag aaagtatcag agataatttt gtcgttattt atgagttgtt ggacgaactt     480
   ttagactttg gctatcctca aacaactgat agcaaaattc ttcaagaata catcactcaa     540
   gaaggtcata aactagagat acaaccaaga attcctgtag ctgtgacaaa tgcagtttca     600
   tggcgttctg agggaatcaa atacaggaaa aatgaagtat tcttagatgt aatcgagtca     660
   gtaaatcttt tagcaagtgc aaatggcaat gtactaagaa gcgaaatagt gggagcaata     720
   aaaatgagag tgtatctttc tggtatgcct gaactgagat taggtctcaa tgataaagta     780
   ttattcgaga gtacaggacg tggtaaatct aaatcggtag agttagatga tgttaagttc     840
   catcaatgtg taagattatc aagattcgag atagatagaa caatctcttt tattccacca     900
   gatggtgaat ttgaattaat gtcttatcgt ttaaataccc atgtgaaacc attaatatgg     960
   attgaatctg ttatagaaag acatgcgcat agtagagtgg agtacatgat taaagctaaa    1020
   tcgcaattca agagacgttc tactgctaac aatgttgaaa taatcattcc agtgccacat    1080
   gatgcagact ctccgaagtt taaaaccacc atcggcagtg tcaagtatgc ccctgaacaa    1140
   aatgccataa cttggagtat caaatcattc ccaggggtaa aagagtattt gatgagagcc    1200
   cactttggtt tacctagtgt tgaatgtgaa gatactgagg gtaaaccacc tattcaggtc    1260
   aaatttgaaa ttccatattt tacaacttct ggaatacaag tcagatactt gaagattata    1320
   gagaaaagtg ggtatcaagc tctaccatgg gtgagatata ttacacagaa tggtgactac 1380
   cagttgcgta ctaattaagc gttgttattg tacttttctg ctttttaagt tagaatataa 1440
   tatattttta cttatacttt atttgtaggg tcttataaaa ctttgtacta tgtagcacta 1500
   tattcagtta atatatttta aaataaacat tactttttca tgtactgttc tcacaattca 1560
   ttataacttg tttcagcatt atctatttca aaatatttaa agtatgtact taaacatttg 1620
   acattgaata aatttaccta ttttattctt aaattttggg tgctttgaga caagctcagc 1680
   ggttggacaa ctgagtaagc ggtagcgttt agacaacaca atgacaagcc actgttaaat 1740
   ttactaagcg tattttcttt ctgctgtgta actcagtggt tgcttgggta cagtacgcaa 1800
   taatgagttt aaattttttt aaacttttca taaaatattc aattgaccca aaaaatggat 1860
   ttacagtgga ataagagaag tacattttgg tactgtacga gaatatttat ttctagaagt 1920
   attatgtaaa tattatgcaa atatgtttat tactactgga tctaagccgt ttttgtagtt 1980
   aatgtcagcc actgaggtcg tctgaatgta cccattggtt atcctgtctt ttatcaagtt 2040
   gccatgcctt caagcgtcgg aaaagttttg attttaagag agtaggcgca aaatttaact 2100
   ggattcattt ttttcgaatc ctgagaaaac t 2131
<210> 133
   <211> 660
   <212> DNA
   <213> Diabrotica virgifera
<220>
   <221> misc_feature
   <222> (1)..(660)
   <223> RNAi target sequence
<400> 133
   acctccaaaa gaccatcaag aaacaatccc tgcaactcac cgaaatccag gctcactatg 60
   acgaaatcca acgtcaattg caagttactc ttgaccaact cagcgtatca caaagaaggg 120
   tacaggcact taccgccgaa tctgaagaaa tcaggagcaa ttatgaaggt gctcttcgtg 180
   gtaagaggca agccgagcaa atgtacgaag aagctggcac tcgcatcaac gagctcacca 240
   ccatcaacgt caacctttct tcaatgaagg tcaagattga acaagaactg tcctcagttc 300
   ttgctgacta cgatgaaatc accaaggaac tccgcatttc tgatgaacgc taccaacgtg 360
   tacaggccga actcaaacat accgtcgaaa tcctacatga agagcaagag cgcgtggtca 420
   agatcgaagc tatcaagaag agtctggaaa tcgaagtcaa gaacctctca gtccgtttgg 480
   aagaagtcga agccaacgct atcgttggag gaaaacgtat catcagcaaa ctcgaagctc 540
   gtatcaggga ttgcgaagct gaactcgatg aagaaaagag acgccacgcc gaaaccatca 600
   agatcttgcg caagaaggaa cgcactgtta aggaagtcat gatccaatgt gaagaggacc 660
<210> 134
   <211> 22
   <212> DNA
   <213> Artificial Sequence
<220>
   <223> primer
<220>
   <221> misc_feature
   <222> (1)..(22)
   <223> forward primer
<400> 134
   acctccaaaa gaccatcaag aa 22
<210> 135
   <211> 21
   <212> DNA
   <213> Artificial Sequence
<220>
   <223> primer
<220>
   <221> misc_feature
   <222> (1)..(21)
   <223> reverse primer
<400> 135
   gtcctcttca cattggatca t 21
<210> 136
   <211> 3369
   <212> DNA
   <213> Diabrotica virgifera
<220>
   <221> misc_feature
   <222> (1)..(3369)
   <223> WCRW transcript ta00254.002-diavv
<400> 136
   aatcaagtat attcaaatgg gaaacaagcc acaatttcac ctaaaaacga ctttatcgac      60
   gtctcgacgc tcatatacga acgactctgc caccactgtc aggcggcgca ggcacgcgga     120
   tttcaatctt gaaattttcg acggtcagtg caccaacgac acgactatac attcacaaaa     180
   aagcaaaaaa atctcttgaa gatggcttct tcctctgtgg cgaggacctc caaatactct     240
   taccgttcca caggagctgc aggaggcagt gctgacgtca gcatcgaata cagcgcggat     300
   ctgacagcgc tttctaggct tgaggataaa attcgcctac ttcaggatga cctagaaatt     360
   gaacgagaac ttagatctag aatcgaaaga gaaaaagccg accttagtgt acaagtaatc     420
   caattatcag aacgtctaga agaagctgag ggtggtgcag aaagtcagtt tgaaattaac 480
   aaaaaacgtg acacagaatt gctgaagctt cgcaaactat tagaagatgt acacatagag 540
   tcagaagaaa ctgctctcat cctcaagaag aaacatcaag aaatcgttgt cgacttccaa 600
   gaacagattg attccttagc taaagcaaaa tcaagagccg aaaaagaaaa gggcaaattc 660
   caagccgaaa tctaccagct catcgcccag gtcgaaagcg taaccaaaga acgcttgatt 720
   tccatcaaac aatgtgagaa actagaaatc accgtcaacg agctcaacat cagaatcgag 780
   gagcttaacc gttccgtcgt tgacatcacc tcccacaaga cccgtctctc ccaagagaac 840
   atcgagctca tcaaggaagt ccaagacctc aaggtcaata tcgaaaacgc catctacctc 900
   aagagccagc tcgccggcca actagaggat gcccgccgcc gtgctgaaga cgatgaaaga 960
   agacgctccc ttttggaggc caacctccac caggtcgaga tcgaacttga atctgtccgt 1020
   gtgcaacttg aggaagaaag cgaagctcgt cttgaccttg aacgtcaatt ggtcaaatcc 1080
   caaggtgagg ttcaagtcta caaatccaaa tatgaaaccg aagtcgttgc tcgtgctgaa 1140
   gaagttgaag aaatccgtcg caaattcacc atcagaatcc aagaacaaga agaacacatt 1200
   gaagccctct tggtcaaggt caacaacctc gagaagcaaa agtcccgcct ccaatccgaa 1260
   gtcgaggttc ttatcattga cttggagaaa gcaaataaca ccgctagaga acttcaaaaa 1320
   cgcgttgaac aactcgagag aatcaatatt gacttgaaga cccgtctcga tgaaacagtt 1380
   caattgtacg agcaaagcca acgtgacctc cgtaacaagg ttactgaatt gcaacgcgct 1440
   gtacacgaat tggacaaaac tcgcgaactg aaggaccaac ttgccagaga aaacaagaaa 1500
   ttatgcgacg atctcagcga ctccaagatt accattaccg agcttactcg tcgtctccat 1560
   gaactcgact tggaagttcg ccgtttggaa aatgaacgtg atgaacttac tgcagcctac 1620
   aaagaagctg aagccggacg taaagctgaa gaacaacgtg cccaacgcct tgccgccgaa 1680
   cttggtcaat tccgtcacga agccgaaaaa cgtcttcaag aaaaagacga agaaattgaa 1740
   gctatccgca aggcaaccag catcgaaatt gaacagctca atgcccgcgt cgttgaagcc 1800
   gaaaccaggc tcaagactga ggtcacccgc atcaagaaga agttgcagat ccaaatcacc 1860
   gagctcgaga tgtccttgga ctccgccaac aaactcaaca tcgacctcca aaagaccatc 1920
   aagaaacaat ccctgcaact caccgaaatc caggctcact atgacgaaat ccaacgtcaa 1980
   ttgcaagtta ctcttgacca actcagcgta tcacaaagaa gagtacaggc acttaccgcc 2040
   gaatctgaag aaatcaggag caattatgaa ggtgctcttc gtggtaagag gcaagccgag 2100
   caaatgtacg aagaagctgg cactcgcatc aacgagctca ccaccatcaa cgtcaacctt 2160
   tcttcaatga aggtcaagat tgaacaagaa ctgtcctcag ttcttgctga ctacgatgaa 2220
   atcaccaagg aactccgcat ttctgatgaa cgctaccaac gtgtacaggc cgaactcaaa 2280
   cataccgtcg aaatcctaca tgaagagcaa gagcgcgtgg tcaagatcga agctatcaag 2340
   aagagtctgg aaatcgaagt caagaacctc tcagtccgtt tggaagaagt cgaagccaac 2400
   gctatcgttg gaggaaaacg tatcatcagc aaactcgaag ctcgtatcag ggattgcgaa 2460
   gctgaactcg atgaagaaaa gagacgtcac gccgaaacca tcaagatctt gcgcaagaag 2520
   gaacgcactg ttaaggaagt catgatccaa tgtgaagagg accaaaagaa catcgctcta 2580
   ttgcaagaaa ctttagacaa ggctaattcg aagattacta ttttcaagag acaactacaa 2640
   gaagtcgagg gtgtatctag ccaaagcata actcgcgtac gtcgattcca gagggaattg 2700
   gaagccgccg aagaccgtgc cgaaaccgcc gaaagcaatc tgtccctcat ccgcgccaaa 2760
   caccgcactt tcgtcaccac cagcacagta cctggctccc aagtctactt ggtacaagaa 2820
   caacgagcat cttccgaaat ctaatcatct cctattctag ctactaataa actaccccat 2880
   ccggccgaat aacaacgtca aagacattta tccatacagc agcgacatct gcgaaggaaa 2940
   acttgagttg gaagactcgt ttcccttcgt cagtttcttg ctgtgtgtaa acttgtcttt 3000
   gggctcgcga catggtggaa gacatcatca tacaagatca tttttttttg ttcttcgtgc 3060
   acataattaa ataatattta ccactctctt tgaaatatta ttattattat tataacgaaa 3120
   cgtacatatt tcattaaata aattaataaa atgagtaagc gatttatatt tgtttgcaat 3180
   cgcacgatga gtccatggta tggaaatttg taggtttcta ataaactgtt atgttatgta 3240
   attgtactta tatcactgat gtgtgcttct attagaggta caagggttat tggaatgtaa 3300
   tttataagtt tttgaatgta ccgtgtaaga ataaattgct tataaataaa aaaaaaaaaa 3360
   aaaaatcgg 3369
<210> 137
   <211> 589
   <212> DNA
   <213> Diabrotica virgifera
<220>
   <221> misc_feature
   <222> (1)..(589)
   <223> RNAi target sequence
<400> 137
   cgatttatca gcttcccaat tctctcctga tggaagagta tttcaagttg aatatgcaat      60
   gaaagcagtt gaaaatagtg gcaccgtaat aggcctccga ggtacagatg gcattgtatt     120
   ggctgctgaa aagctcatta tgtcaaaatt gcatgaacca agtacaaata aacgaatttt     180
   caacattgat aaacacatag gaatggcatt ttcaggctta atagctgatg caaggcaaat     240
   cgttgagatt gctagaaaag aagcatcaaa ttatagacat caatatggtt caaatattcc     300
   tcttaaatac ctaaatgata gagtaagcat gtacatgcat gcatacactt tatacagtgc     360
   tgttagacca tttggttgca gtgtcatctt ggccagttat gaagatagtg acccatctat     420
   gtatctgatt gatccatctg gagttagcta tggatacttt ggatgtgcta caggtaaagc     480
   aaaacagtct gcaaagactg aaatagaaaa attgaagatg gggaatctaa catgcaaaga     540
   acttgttaaa gaagcagcca aaatcattta tttggtccat gatgagctg                589
<210> 138
   <211> 22
   <212> DNA
   <213> Artificial Sequence
<220>
   <223> primer
<220>
   <221> misc_feature
   <222> (1)..(22)
   <223> forward primer
<400> 138
   cgatttatca gcttcccaat tc 22
<210> 139
   <211> 22
   <212> DNA
   <213> Artificial Sequence
<220>
   <223> primer
<220>
   <221> misc_feature
   <222> (1)..(22)
   <223> reverse primer
<400> 139
   cagctcatca tggaccaaat aa 22
<210> 140
   <211> 956
   <212> DNA
   <213> Diabrotica virgifera
<220>
   <221> misc_feature
   <222> (1)..(956)
   <223> WCRW transcript ta06609.001_diavv
<400> 140
   aaaaaatcaa aatgagttct attggaactg ggtacgattt atcagcttcc caattctctc      60
   ctgatggaag agtatttcaa gttgaatatg caatgaaagc agttgaaaat agtggcaccg     120
   taataggcct ccgaggtaca gatggcattg tattggctgc tgaaaagctc attatgtcaa     180
   aattgcatga accaagtaca aataaacgaa ttttcaacat tgataaacac ataggaatgg     240
   cattttcagg cttaatagct gatgcaaggc aaatcgttga gattgctaga aaagaagcat     300
   caaattatag acatcaatat ggttcaaata ttcctcttaa atacctaaat gatagagtaa     360
   gcatgtacat gcatgcatac actttataca gtgctgttag accatttggt tgcagtgtca     420
   tcttggccag ttatgaagat agtgacccat ctatgtatct gattgatcca tctggagtta     480
   gctatggata ctttggatgt gctacaggta aagcaaaaca gtctgcaaag actgaaatag     540
   aaaaattgaa gatggggaat ctaacatgca aagaacttgt taaagaagca gccaaaatca     600
   tttatttggt ccatgatgag ctgaaggata agaattttga actggaactt tcatgggtat     660
   gcaaagatac gaatggttta cataccaaag tgcctgaatc agtgtttgct gatgcagaaa     720
   aagctgccaa acaagcaatg gaagcagatt cagaatcaga tacagaagat atgtaataac     780
   tacatttagt ttttaatatt tcgctgatgg tggctgttct tacaatattt cgtgtgttat     840
   gttcatatat tatgtaatac tgtgagaatt tccatttcaa ggataggttt ataacttttt     900
   tttctaataa atacataact ttatgtcaaa aaaaaaaaaa aaaaaaaaaa aaaaaa         956
<210> 141
   <211> 577
   <212> DNA
   <213> Diabrotica virgifera
<220>
   <221> misc_feature
   <222> (1)..(577)
   <223> RNAi target sequence
<400> 141
   aattggtgca gatacacgtt tgagtactgg attttccatt tataccagag aacaaaacaa 60
   acttttccca ctatcaggca ctactgtttt gggttgtgca ggatgttggt gtgacactct 120
   aacattaacc agaatcctta aatctcgcat gcagatgtac caacaagagc ataacaaaac 180
   aatgtctaca actgcatgtg cccagatgtt gtcaaccatg ctctactaca agagattctt 240
   tccttattat atatcaaaca ttctagtagg tttagataat gaaggaaagg gctgtgttta 300
   cagctatgat cctattggac attgtgaaaa agctacgtat agagcaggtg gttcagctgg 360
   agctcttctt cagcctctgt tggacaatca aattggacag aagaacatgc ttaaaacatc 420
   tggggaacct cttagtcagg agaaagctct gtctaccctt aaagatgtat ttatttctgc 480
   tgctgaaaga gacatctaca ctggagatag cgtacttata aatattatta ctaaagatgg 540
   agtaaaggaa gagtccttcc agttgagacg ggattag 577
<210> 142
   <211> 22
   <212> DNA
   <213> Artificial Sequence
<220>
   <223> primer
<220>
   <221> misc_feature
   <222> (1)..(22)
   <223> forward primer
<400> 142
   aattggtgca gatacacgtt tg 22
<210> 143
   <211> 22
   <212> DNA
   <213> Artificial Sequence
<220>
   <223> primer
<220>
   <221> misc_feature
   <222> (1)..(22)
   <223> reverse primer
<400> 143
   ctaatcccgt ctcaactgga ag 22
<210> 144
   <211> 913
   <212> DNA
   <213> Diabrotica virgifera
<220>
   <221> misc_feature
   <222> (1)..(913)
   <223> WCRW transcript ta03835.001_diavv
<400> 144
   gggggcagtt atttcgactt ttcatgcttg tcataaaata aaattaaaat atatccggcg      60
   aggtgttgac tagcggattt ttttagattc aacaatctta ttttataaaa taattagtta     120
   aaatgatgca aacagctaat aatgcatatt atcccgatta ttccactgct ccaatgcaac     180
   gtcaaattaa cccctatgca gataatggag ggagtgtagt agcaatagca ggtgaagact     240
   ttgtaataat tggtgcagat acacgtttga gtactggatt ttccatttat accagagaac     300
   aaaacaaact tttcccacta tcaggcacta ctgttttggg ttgtgcagga tgttggtgtg     360
   acactctaac attaaccaga atccttaaat ctcgcatgca gatgtaccaa caagagcata     420
   acaaaacaat gtctacaact gcatgtgccc agatgttgtc aaccatgctc tactacaaga     480
   gattctttcc ttattatata tcaaacattc tagtaggttt agataatgaa ggaaagggct     540
   gtgtttacag ctatgatcct attggacatt gtgaaaaagc tacgtataga gcaggtggtt     600
   cagctggagc tcttcttcag cctctgttgg acaatcaaat tggacagaag aacatgctta     660
   aaacatctgg ggaacctctt agtcaggaga aagctttgtc tacccttaaa gatgtattta     720
   tttctgctgc tgaaagagac atctacactg gagatagcgt acttataaat attattacta     780
   aagatggagt aaaggaagag tccttccagt tgagacggga ttagaagcaa gtggttttgt     840
   ttatattttc ttatgtgaaa ttcaaatata ctttctaaat aaacaaaaaa aaaaaaaaaa     900
   aaaaaaaaaa aaa                                                        913
<210> 145
   <211> 542
   <212> DNA
   <213> Diabrotica virgifera
<220>
   <221> misc_feature
   <222> (1)..(542)
   <223> RNAi target sequence
<400> 145
   tcttcgttca ataccagctt cagtgatgat ccccagtctg actacacctc ctgaagaacc        60
   atctcttgac atggcaagtg ccaatgtatt tgtgacaaac ttcacacatt cttccttgct       120
   catattgggc ttgaagttgg catctacgta accataaaca taactggaac ctgatcctcc       180
   gattgacact tcttgtctaa cacacatccc accaattggt atggaatata cttgtccgcc       240
   tttcttttta tcccaacctg ctaccagtat accagccatt agcgaatctc tataattgta       300
   gcaaagttct tggaaaatgg cggcacctac ttgtactttg ggttcttcac caagttccat       360
   accatgaaaa ttaagatgat aagcaacaat gtctgcaatt gcttgtgtat ctgctgcaga       420
   tcctgaacga caacagtata tatggtcagt gactttggtg agtttgtctg ctacccggtt       480
   tgcaatgtag gccccagtag ttgtgcgaga atctgctcct ataacaacgc ctccatcaaa       540
   ct                                                                      542
<210> 146
   <211> 22
   <212> DNA
   <213> Artificial Sequence
<220>
   <223> primer
<220>
   <221> misc_feature
   <222> (1)..(22)
   <223> forward primer
<400> 146
   tcttcgttca ataccagctt ca 22
<210> 147
   <211> 22
   <212> DNA
   <213> Artificial Sequence
<220>
   <223> primer
<220>
   <221> misc_feature
   <222> (1)..(22)
   <223> reverse primer
<400> 147
   agtttgatgg aggcgttgtt at 22
<210> 148
   <211> 850
   <212> DNA
   <213> Diabrotica virgifera
<220>
   <221> misc_feature
   <222> (1)..(850)
   <223> WCRW transcript ta02145.001_diavv
<400> 148
   attgcagggg gactggcaaa atattgcatt gtaggattac aagtgtaatt tttctaagct 60
   tgtattcttt tcattaaact tgtaaaaaat gcctcttatg actgagtatg gtgcaatgac 120
   tggtcctaat gattggagag atgccgctca cagtacagga acctctatta tggccgcgga 180
   gtttgatgga ggcgttgtta taggagcaga ttctcgcaca actactgggg cttacattgc 240
   aaaccgggta gcagacaaac tcaccaaagt cactgaccat atatactgtt gtcgttcagg 300
   atctgcagca gatacacaag caattgcaga cattgttgct tatcatctta attttcatgg 360
   tatggaactt ggtgaagaac ccaaagtaca agtaggtgcc gccattttcc aagaactttg 420
   ctacaattat agagattcgc taatggctgg tatactggta gcaggttggg ataaaaagaa 480
   aggcggacaa gtatattcca taccaattgg tgggatgtgt gttagacaag aagtgtcaat 540
   cggaggatca ggttccagtt atgtttatgg ttacgtagat gccaacttca agcccaatat 600
   gagcaaggaa gaatgtgtga agtttgtcac aaatacattg gcacttgcca tgtcaagaga 660
   tggttcttca ggaggtgtag tcagactggg gatcatcact gaagctggta ttgaacgaag 720
   agttgtttta ggaaatgaat tgcccaaatt ttatgaaggt taattgatta ttttttaagc 780
   tttggtatgt tttggtatat attaaaataa aaaaatcagt tttacactaa aaaaaaaaaa 840
   aaaaaatcgg 850
<210> 149
   <211> 559
   <212> DNA
   <213> Diabrotica virgifera
<220>
   <221> misc_feature
   <222> (1)..(559)
   <223> RNAi target sequence
<400> 149
   ctgaattgac caacaaattg gattcaatta gagggttttt acattctcgt ttacgtaccg      60
   ctacactcag gaacgacttt gaaggtcaag ctgtgtttat aaattgcctg ttgagaaatt     120
   atttgcatta ttcgttgtat gatcaagctg acaaactagt gagtaaatgc gtatttcctg     180
   aaactgcaag taacaatgaa tgggccagat tcttatacta cttgggcaga attaaggcgg     240
   caagattaga atacagtgta gcacataaac atcttgtgca ggccatgaga aaagcacccc     300
   agaatgctgc cattggtttc agacaggttg ttcaaaagct tactgttgta gttgaactac     360
   tgctaggaga tataccagaa cgacagattt ttagacaatc tagtatgagg cactcccttg     420
   ccccatactt tcaactaaca caagcagtta gaatgggcaa tttgcaaaga tttggtgagg     480
   tgttagagaa cttcggccca caatttagac aagatcatac atttacacta attttgagac     540
   tgcgccacaa tgttattaa                                                  559
<210> 150
   <211> 22
   <212> DNA
   <213> Artificial Sequence
<220>
   <223> primer
<220>
   <221> misc_feature
   <222> (1)..(22)
   <223> forward primer
<400> 150
   ctgaattgac caacaaattg ga 22
<210> 151
   <211> 22
   <212> DNA
   <213> Artificial Sequence
<220>
   <223> primer
<220>
   <221> misc_feature
   <222> (1)..(22)
   <223> reverse primer
<400> 151
   ttaataacat tgtggcgcag tc 22
<210> 152
   <211> 1711
   <212> DNA
   <213> Diabrotica virgifera
<220>
   <221> misc_feature
   <222> (1)..(1711)
   <223> WCRW transcript ta08208.001_diavv
<400> 152
   ggggggaggg gattgtcaaa tagttgtcag cgttacacag cgaatatttt cctcatcagt      60
   tcaatattaa caataaattg ttttgttgaa attgaaatca caaattactt tataaaatgg     120
   taactctaga agacgttgaa atgaaaaatg cagacagtcc tccaggatta gaagctggtg     180
   atacaaagaa agataccgac ctacaaagtg taatagaaat tcgtgaacat gcaagacaaa     240
   tagaaaaatc agtcacaagt aaagaaaacc gtttcatctt acgagtttta cgttgcttgc     300
   ccaacactag aaggaagctt aatggactgg tgctgagaag cctcattact caaatatatc     360
   ctgtagctga acgtgatgcc ctccttagtt tcgtcgagga agcttctgga gaactcgacg     420
   ccacccagtc acgagcaaga tcagctgtta agtcacctgt tcccgaggta gatacatata     480
   taaatctttt aatactagta cgtttaattg ataccaataa gttagttgaa gcagagcgct     540
   gttctcaagc tcttatgaat aaaataacta accaaaacag acgtactata gatcatattg     600
   ctgccaagtg ttatttctat cactcaagag ttgctgaatt gaccaacaaa ttggattcaa     660
   ttagagggtt tttacattct cgtctacgta ccgctacact caggaacgac tttgaaggtc     720
   aagctgtgct tataaattgc ctgttgagaa attatttgca ttattcgttg tatgatcaag     780
   ctgacaaact agtgagtaaa tgcgtatttc ctgaaactgc aagtaacaat gaatgggcca     840
   gattcttata ctacttgggc agaattaagg cggcaagatt agaatacagt gtagcacata     900
   aacatcttgt gcaggccatg agaaaagcac cccagaatgc tgccattggt ttcagacagg     960
   ttgttcaaaa gcttactgtt gtagttgaac tactgctagg agatatacca gaacgacaga    1020
   tttttagaca atctagtatg aggcactccc ttgccccata ctttcaacta acacaagcag    1080
   ttagaatggg caatttgcaa agatttggtg aggtgttaga gaacttcggc ccacaattta    1140
   gacaagatca tacatttaca ctaattttga gactgcgcca caatgttatt aaaacagcta    1200
   taagatccat aggattatca tactccagaa tatctcccca agacatagct aagaaactgg    1260
   ggctggattc agctgaagat gcagaattta tagtagcaaa agcaatcaga gatggggtga    1320
   tagaagctac acttgatcca cagggaggtt acatgaggag caaagaaagt actgacatct    1380
   actgcacaaa ggaacctcaa ctggcgtttc atcaaagaat atcgttttgc ttagatttgc     1440
   acaatcaaag tgtcaaagct atgagatatc cccccaaggc gtatggcaaa gaacttgaat     1500
   cagcagaaga gagaagggaa agagaacagc aggatttgga gttggccaag gaaatggctg     1560
   aagaagatga tgatggtttt ccttaagttg gtgtagttat caatattaat tttataacat     1620
   ttttgctttt gtatggtatt tatagacagt agatttatca aaatatactg agtaaaactc     1680
   caaaaaaaaa aaaaaaaaaa aaaaaaaaaa a                                    1711
<210> 153
   <211> 473
   <212> DNA
   <213> Diabrotica virgifera
<220>
   <221> misc_feature
   <222> (1)..(473)
   <223> RNAi target sequence
<400> 153
   atgtgtgatg actggcatga ttgctgacag caagtcccag gtgcagagag ctagatatga       60
   ggctgccgag ttcaaatata agtttggata tgagatgcca atcgatgcct tgtgtaggag      120
   agtatcggat atttcccagg tttatacgca gaatgctgag atgagacctt tgggttgctc      180
   catgcttctg ataggatatg accaagaaat gggaccatgt gtccacaaag ctgaccctgc      240
   tggctattac tgtggctaca gagcagtaag tgtaggatcc aaacaaactg aagccaacag      300
   ctacctagag aagaagctaa agaagaaaac tgatctgtcc aaagatgaag tcatccaatt      360
   ggcaatcagt tgtctttcaa gtgttttatc tgtagatttt aaaccaacag aagtcgaagt      420
   tggggttgtt actaaggatg agcccaagtt taggaaattg acggaacaag aaa             473
<210> 154
   <211> 22
   <212> DNA
   <213> Artificial Sequence
<220>
   <223> primer
<220>
   <221> misc_feature
   <222> (1)..(22)
   <223> forward primer
<400> 154
   atgtgtgatg actggcatga tt 22
<210> 155
   <211> 22
   <212> DNA
   <213> Artificial Sequence
<220>
   <223> primer
<220>
   <221> misc_feature
   <222> (1)..(22)
   <223> reverse primer
<400> 155
   tttcttgttc cgtcaatttc ct 22
<210> 156
   <211> 879
   <212> DNA
   <213> Diabrotica virgifera
<220>
   <221> misc_feature
   <222> (1)..(879)
   <223> WCRW transcript 454run3_isotig15710
<400> 156
   gtgacaacag aaaaatatag aaaatcatcc tctgtgtttc cgttttgaac ttaacttgta      60
   tttagtttaa aaaataatca tgtctagagg aagcagtgca ggttttgacc gacacataac     120
   aattttctcg cccgaaggcc gactctatca agtagagtat gcttttaaag ccattaacca     180
   agccggcccc acttcggtag cagtccgagg agtagatgct gcggcgtgtg tgacccagag     240
   aaagatcccg gataagctga ttgatcccaa cacaattaca catctgttcc agttaacaga     300
   acacactgga tgtgtgatga ctggcatgat tgctgacagc aagtcccagg tgcagagagc     360
   tagatatgag gctgccgagt tcaaatataa gtttggatat gagatgccaa tcgatgcctt     420
   gtgtaggaga gtatcggata tttcccaggt ttatacgcag aatgctgaga tgagaccttt     480
   gggttgctcc atgcttctga taggatatga ccaagaaatg ggaccatgtg tctacaaagc     540
   tgaccctgct ggctattact gtggctacag agcagtaagt gtaggatcca aacaaactga     600
   agccaacagc tacctagaga agaagctaaa gaagaaaact gatctgtcca aagatgaagt     660
   catccaattg gcaatcagtt gtctttcaag tgttttatct gtagatttta aaccaacaga     720
   agtcgaagtt ggggttgtta ctaaggatga gcccaagttt aggaaattga cggaacaaga     780
   aatagatttc catctgacag ctattgctga gagggattag atagattttt tatattcact     840
   ctttgtaatg ttaagaaatg gtcaaatttt gagttaagt                            879
<210> 157
   <211> 451
   <212> DNA
   <213> Diabrotica virgifera
<220>
   <221> misc_feature
   <222> (1)..(451)
   <223> RNAi target sequence
<400> 157
   agataggcgg tttggtattc aagcccaaac ggtagctaca aatttccaaa aaaatctttg      60
   aaatgggacc acatttatat gtgggtcttc caggattagc cacagatacc caaacagtta     120
   tggaaaaact ccgtttccga aaaaacttgt acgaacttaa ggaaaatcga aaaatatctc     180
   caaaagtatt tgcctctatg atatcaaata tgttgtatga aaaaagattt gggccatttt     240
   ttgtagaacc tgtagtagct ggacttctac ctaatactta tgaacccttt atctgtaata     300
   tggatttaat tggttgtata aaccaacctt cagactttgt tgttggtgga acagcgtcag     360
   cacagttgta tggtatgtgt gaagcacttt gggagcctaa cctaggacct gaggatcttt     420
   ttgaaaccat ctctcaagct ctcatcaatg c                                    451
<210> 158
   <211> 22
   <212> DNA
   <213> Artificial Sequence
<220>
   <223> primer
<220>
   <221> misc_feature
   <222> (1)..(22)
   <223> forward primer
<400> 158
   agataggcgg tttggtattc aa 22
<210> 159
   <211> 22
   <212> DNA
   <213> Artificial Sequence
<220>
   <223> primer
<220>
   <221> misc_feature
   <222> (1)..(22)
   <223> reverse primer
<400> 159
   gcattgatga gagcttgaga ga 22
<210> 160
   <211> 829
   <212> DNA
   <213> Diabrotica virgifera
<220>
   <221> misc_feature
   <222> (1)..(829)
   <223> WCRW transcript ta03399.001_diavv
<400> 160
   gtttgcagag agtgtattag tgtattgtat tttagtattt tacaattctt tgaattgcag     60
   attatttagc ggtttagtac aaaaacctga agtaaattct ataacggaaa gtgcttaaat    120
   tttagtggta aaatgtccat tctagcttac aatggtggtg ctatggtggc gatgaaggga    180
   gaaaactgtg tagcaattgc agcagatagg cggtttggta ttcaagccca aacagtagct    240
   acaaatttcc aaaaaatctt tgaaatggga ccacatttat atgtgggtct tccaggatta    300
   gccacagata cccaaacagt tatggaaaaa ctccgtttcc gaaaaaactt gtacgaactt    360
   aaggaaaatc gaaaaatatc tccaaaagta tttgcctcta tgatatcaaa tatgttgtat    420
   gaaaaaagat ttgggccatt ttttgtagaa cctgtagtag ctggacttct acctaatact    480
   tatgaaccct ttatctgtaa tatggattta attggttgta taaaccaacc ttcagacttt    540
   gttgttggtg gaacagcgtc agcacagttg tatggtatgt gtgaagcact ttgggagcct    600
   aacctaggac ctgaggatct ttttgaaacc atctctcaag ctctcatcaa tgcctttgac     660
   agagatgcca tttctggttg gggagctaca gtgtacatca tagaaaaaaa taaggtgact    720
   gtaaaaaatt tgaagacacg tatggactag ttaaatcttg ttttttcatt tacatttaat    780
   tgataaaata aatgaaacta tttgataaaa aaaaaaaaaa aaaatgcga                 829
<210> 161
   <211> 530
   <212> DNA
   <213> Diabrotica virgifera
<220>
   <221> misc_feature
   <222> (1)..(530)
   <223> RNAi target sequence
<400> 161
   cttcttcgac taggaggtgg aatgccaggt ttaactcagg caccgccgcc ttctgatgct      60
   cctgttgtgg atactgccga acaagtatac atatcctcct tagcactatt aaaaatgttg     120
   aagcacggcc gagctggagt acccatggaa gtaatgggtc ttatgttagg agaattcgta     180
   gatgactata ccgttcgtgt aatcgatgta tttgctatgc ctcagactgg taccggtgtc     240
   agtgtagaag ctgctgatcc agtgtttcaa acaaaaatgc ttgacgtgtt aagacagact     300
   ggtaggccag aaatggttgt ggggtggtat cattcccatc caggtttcgg atgttggttg     360
   tctggagtgg atatcaacac ccagcaaagt tttgaggctc tcacggaaag agctgtagca     420
   gtagtagttg atccaatcca gtcagtgaaa ggcaaagtcg ttattgatgc ctttaggtta     480
   ataaatccga atatgatggt attgggccaa gaacctcgcc aaactacatc                530
<210> 162
   <211> 22
   <212> DNA
   <213> Artificial Sequence
<220>
   <223> primer
<220>
   <221> misc_feature
   <222> (1)..(22)
   <223> forward primer
<400> 162
   cttcttcgac taggaggtgg aa 22
<210> 163
   <211> 22
   <212> DNA
   <213> Artificial Sequence
<220>
   <223> primer
<220>
   <221> misc_feature
   <222> (1)..(22)
   <223> reverse primer
<400> 163
   gatgtagttt ggcgaggttc tt 22
<210> 164
   <211> 1190
   <212> DNA
   <213> Artificial Sequence
<220>
   <223> primer
<220>
   <221> misc_feature
   <222> (1)..(1190)
   <223> WCRW transcript ta04924.001_diavv
<400> 164
   tgacagatga cttgtaaggg ggaaaactaa aagtcatact cacggttgtt tttgtaaaac      60
   ttgtaggtaa cgaattttcg tttttagtga attaacaaaa caacagagtg attttccaac     120
   aagaataact tatttattag tttcattaat ttgataaaca ataaaatgga tagacttctt     180
   cgactaggag gtggaatgcc aggtttaact caggcaccgc cgccttctga tgctcctgtt     240
   gtggatactg ccgaacaagt atacatatcc tccttagcac tattaaaaat gttgaagcac     300
   ggccgagctg gagtacccat ggaagtaatg ggtcttatgt taggagaatt cgtagatgac     360
   tataccgttc gtgtaatcga tgtatttgct atgcctcaga ctggtaccgg tgtcagtgta     420
   gaagctgttg atccagtgtt tcaagcaaaa atgcttgaca tgttaagaca gactggtagg     480
   ccagaaatgg ttgtggggtg gtatcattcc catccaggtt tcggatgttg gttgtctgga     540
   gtggatatca acacccagca aagttttgag gctctctcgg aaagagctgt agcagtagta     600
   gttgatccaa tccagtcagt gaaaggcaaa gtcgttattg atgcctttag gttaataaat     660
   ccgaatatga tggtattggg ccaagaacct cgccaaacta catccaactt aggacatcta     720
   cagaaaccat cagtccaggc tttgattcat ggtctaaatc ggcattacta ctccatcagt     780
   attaattaca gaaagaatga attggaacaa aaaatgctac taaatctcca taaaaaatcc     840
   tggatggatg gtctaacttt agcagattac tctgaaaatt gtagtgtgaa tgaaaagact     900
   gtagctgata tgttagaact agccaagaat tacaacaagg cattagaaga ggaggagaaa     960
   atgactccag aacagctcgc tattaaaaat gttggcaaac aagatcctaa gaggcattta    1020
   gaagaaaagg ttgatatttt gatgaccaat aatatcgtcc agtgcttggg agctatgcta    1080
   gatacagttg tttttaagta attgctggat ctcttttttt actataattg caacttattt    1140
   ataagatatt ttttcaataa aaacttttat tatgaatttg tgttgcgcag               1190
<210> 165
   <211> 654
   <212> DNA
   <213> Diabrotica virgifera
<220>
   <221> misc_feature
   <222> (1)..(654)
   <223> RNAi target sequence
<400> 165
   acgatttttg aggttgtcgg ctagaagttc gcaaagtaaa tctaaattcc aacaattaaa 60
   ccgaaatagt tctttaaaaa agatgtttcg aaaccaatac gatagtgatg tgacagtgtg 120
   gagcccccag ggccgtctgc atcaggtaga atatgccatg gaagcagtta acctaggttc 180
   agccacagtg ggactgaaaa gtaaaactca tgctgtactt atcgctttga agagggcttc 240
   ttcagaactg tctgcctacc agaagaaaat tatcaacata gacgatcaca ttggtatcac 300
   catctcggga cttacagctg atgctaggat ccttagtaga tatatgcgaa acgaatgctt 360
   gaattataag tactctttgg atacttatat gccattgaat aggcttatca gtattttagg 420
   taacaaaatg cagacatgta cccaaagata cgaccgcaga ccatttggtg taggtcttct 480
   agtagctggt tatgacgaat ctggttctca tatctaccaa acgtgccctt ctgctaattt 540
   ctacgattgc aaagctatgt ccattggagc tcgatcccag agtgccagga catacttaga 600
   aaaacatctg gatgaattgc catctagttc tttagaagaa cttatcaaac atgg 654
<210> 166
   <211> 22
   <212> DNA
   <213> Artificial Sequence
<220>
   <223> primer
<220>
   <221> misc_feature
   <222> (1)..(22)
   <223> forward primer
<400> 166
   atttttgagg ttgtcggcta ga 22
<210> 167
   <211> 22
   <212> DNA
   <213> Artificial Sequence
<220>
   <223> primer
<220>
   <221> misc_feature
   <222> (1)..(22)
   <223> reverse primer
<400> 167
   ccatgtttga taagttcttc ta 22
<210> 168
   <211> 1142
   <212> DNA
   <213> Diabrotica virgifera
<220>
   <221> misc_feature
   <222> (1)..(1142)
   <223> WCRW transcript ta04466.001_diavv
<400> 168
   gggggagggg gacagtcgac gtagcattac aaacgatttt tgaggttgtc ggctagaagt      60
   tcgcaaagta aatctaaatt ccaacaatta aaccgaaata gttctttaaa aaagatgttt     120
   cgaaaccaat acgatagtga tgtgacagtg tggagccccc agggccgtct gcatcaggta     180
   gaatatgcca tggaagcagt taacctaggt tcagccacag tgggactgaa aagtaaaact     240
   catgctgtac ttatcgcttt gaagagggct tcttcagaac tgtctgccta ccagaagaaa     300
   attatcaaca tagacgatca cattggtatc accatctcgg gacttacagc tgatgctagg     360
   atccttagta gatatatgcg aaacgaatgc ttgaattata agtactcttt ggatacttat     420
   atgccattga ataggcttat cagtatttta ggtaacaaaa tgcagacatg tacccaaaga     480
   tacgaccgca gaccatttgg tgtaggtctt ctagtagctg gttatgacga atctggttct     540
   catatctacc aaacgtgccc ttctgctaat ttctacgatt gcaaagctat gtccattgga     600
   gctcgatccc agagtgccag gacatactta gaaaaacatc tggatgaatt gccatctagt     660
   tctttagaag aacttatcaa acatggattg agggctctta gagatactct acctccagag     720
   gttgacctca caacaaaaaa tgtctccata ggatacactg gcaaaggtca gacctttaaa     780
   atcctcgaag aagaagaaac ttccccctac ttgtccctta ttgagggtga agaacgtaga     840
   ggaggaggag ctgcagctgc tgcaattgag gaccttcctg cagatccgtt gagagatgat     900
   gcgggggacg agcccaggga tgcagtccca gctgtggaaa cttctgccat ggataccaca     960
   gaatagaagg attagataaa gcattaaagc cacccgttga tcaggagagt gggtattgga    1020
   aaggaatatt gccgtggctg tctctgttag tctaaaacag actgagacag tcacggcaga    1080
   acatcgtttg ttatatccat actgtatttt ttaagctttt aataatattt tgattaataa    1140
   at                                                                    1142
<210> 169
   <211> 558
   <212> DNA
   <213> Diabrotica virgifera
<220>
   <221> misc_feature
   <222> (1)..(558)
   <223> RNAi target sequence
<400> 169 atcctgggtg gtgttgat 558
<210> 170
   <211> 22
   <212> DNA
   <213> Artificial Sequence
<220>
   <223> primer
<220>
   <221> misc_feature
   <222> (1)..(22)
   <223> forward primer
<400> 170
   tctgtgcaaa aacaaactga ca 22
<210> 171
   <211> 22
   <212> DNA
   <213> Artificial Sequence
<220>
   <223> primer
<220>
   <221> misc_feature
   <222> (1)..(22)
   <223> reverse primer
<400> 171
   atcaacacca cccaggatta aa 22
<210> 172
   <211> 1578
   <212> DNA
   <213> Diabrotica virgifera
<220>
   <221> misc_feature
   <222> (1)..(1578)
   <223> WCRW transcript ta04816.001_diavv
<400> 172
   gggggtttta aattttcgta gacacgattg ttcagtagtt caaaattcat tattattaat      60
   gttctgtgca aaaacaaact gacaagtttg acataagtga gaagggacat agcggttgtc     120
   aacaattttg caagttgtgt aaattgttaa tttgaaggaa gcttttaact tttaagtaaa     180
   attaactgaa aatggctaca gttttgtgtc cagaaatacc aacgcctggt ttttcattcg     240
   aaaattgtaa aagaaatgca cttctggaaa ataagggcct tgctcttcca aaagccacta     300
   aaacaggtac tacgatcgta ggaatagtct acaaagatgg aattattctt ggagcagata     360
   ctagagctac agaagacacc acagttgcag acaagaactg tgaaaagatt cacttccttg     420
   cacccaatat gtattgttgt ggtgctggta ctgcagcaga tacagaaatg accactcaga     480
   tggtctctgc taaattagaa ctccacagat tatacacaaa ccgagttact agagtgtgta     540
   cagcaaacca attgttaaaa caatatctat ttcgctacca gggatatatt ggggctgctt     600
   taatcctggg tggtgttgat attgaaggac cacatctata tatgatttat ccacatggat     660
   ccagtgttga tcttccttat gctacaatgg gttctggttc cttagcagcc atatctgtct     720
   ttgaatcccg ctggaagcct caaatggaag aggaagatgg tgtccaacta gtaagagatg     780
   ctattgcagc tggtatattc aatgatctag gttcaggttc caatgtagac atctgtgtaa     840
   taagaaaggg ttcagtagac tacaaacgaa cctatgacga ggctaacaaa aagggagtta     900
   aacaaggatc ttatagatat caaagaggaa ccactgcagt gctcagtacc caggtggtgg     960
   atgttgagga agtctcagtg attcccattg aagctgaaga aatggatgtt gcttaaatat    1020
   tacagttaaa taaatgtcat tgtattcttt ttggtattta ctttaatata aaaataaaaa    1080
   ataaatataa cagggtttaa atgttcaata atatagtaaa ttttaattta aaaaaatatt    1140
   ttttcttaac aattacaata gccacatcta aatagatttg ctactcaagg atgttgagtt    1200
   aataggatca gtcagtctat aaaactaaag aacacattta ttttgatcat tactttgggt    1260
   ttgatagctg ccctttatta tatgttaatt ttaagtacaa tctatataac cctaaagttg    1320
   tttacccaaa aatcattttt atcattttcg agagtaaata ttgttcaatt ttcacatgtc    1380
   aaaaatatta ttatattttt tactgtgttt aatcttttat tacaaccctt aaggtactag    1440
   tacacttcag aagaacaaaa ataatcattt ttttaaagaa ttttcctctc agaacctgta    1500
   ttaaaaatga acacaaaacc tgtcacatac taatatctaa ctctcagaga gtacaagaaa    1560
   tatatctctt ttcattca                                                  1578
<210> 173
   <211> 473
   <212> DNA
   <213> Diabrotica virgifera
<220>
   <221> misc_feature
   <222> (1)..(473)
   <223> RNAi target sequence
<400> 173
   aatataccga aaatgccgag tcaagaatta acaactaata aagtaatagt ccaccctttg        60
   gttcttctaa gtgtggtgga ccattttaat cgcatgggca aaatcggcaa tcaaaagcgt       120
   gttgttggtg ttcttcttgg ctgttggcgt gctaaaggag tcttagatgt ttctaacagt       180
   tttgcagttc cttttgacga agatgacaag gataagtctg tgtggttctt ggaccatgat       240
   tacttagaaa acatgtatgg aatgttcaaa aaagttaatg ccagggaacg agtagtaggt       300
   tggtaccaca ccggccccaa acttcatcaa aatgatattg ccattaatga gttaatcaga       360
   aggtattgtc caaattccgt tctagttatc atagacgcta aaccaaaaga tttaggttta       420
   ccaactgaag cttatcaagc tgtagaagaa gtacacgatg acggttcacc tac              473
<210> 174
   <211> 22
   <212> DNA
   <213> Artificial Sequence
<220>
   <223> primer
<220>
   <221> misc_feature
   <222> (1)..(22)
   <223> forward primer
<400> 174
   aatataccga aaatgccgag tc 22
<210> 175
   <211> 22
   <212> DNA
   <213> Artificial Sequence
<220>
   <223> primer
<220>
   <221> misc_feature
   <222> (1)..(22)
   <223> reverse primer
<400> 175
   gtaggtgaac cgtcatcgtg ta 22
<210> 176
   <211> 1221
   <212> DNA
   <213> Diabrotica virgifera
<220>
   <221> misc_feature
   <222> (1)..(1221)
   <223> WCRW transcript ta03894.001_diavv
<400> 176
   tagttaattg ggaacaaaga ttttattaga ataatatacc gaaaatgccg agtcaagaat      60
   taacaactaa taaagtaata gtccaccctt tggttcttct aagtgtggtg gaccatttta     120
   atcgcatggg caaaatcggc aatcaaaagc gtgttgttgg tgttcttctt ggctgttggc     180
   gtgctaaagg agtcttagat gtttctaaca gttttgcagt tccttttgac gaagatgaca     240
   aggataagtc tgtgtggttc ttggaccatg attacttaga aaacatgtat ggaatgttca     300
   aaaaagttaa tgccagggaa cgagtagtag gttggtacca caccggcccc aaacttcatc     360
   aaaatgatat tgccattaat gagttaatca gaaggtattg tccaaattcc gttctagtta     420
   tcatagacgc taaaccaaaa gatttaggtt taccaactga agcttatcaa gctgtagaag     480
   aagtacacga tgacggttca cctacatcta aaacatttga gcatgttcct agtgaaatcg     540
   gagctgagga agctgaagaa gttggtgttg aacacttatt gagagatatt aaagacacaa     600
   ctgttggtac tttgtcacag agaataacga atcagctttt agggttaaaa ggtctccatt     660
   cacagttacg tgatattagg gactatttgg tacaagtgtg tggtaaccag cttccaatca     720
   atcaccaaat catctaccaa ttacaagaca tatttaatct tttgcccgat ataaatcagg     780
   atgcatttaa tacatcgttt tacgtgaaaa ataacgatca aatgttagtc gtttatctag     840
   cagctttagt tagatctatt gtcgctctac acaatcttat taacaacaaa ctaacaaata     900
   gagacgcgga agaaggaaaa aaggaggata gtaagaaaga aagcaaagat aaggacaagg     960
   ataaagagaa ggaaaaggat aaagatgcta agaaggacga gaagaaaaag taattgtgta    1020
   ttatatctgg ttctacattc aaatgttctc atgttgtgtt ttcaaacacg atattttaat    1080
   ggtttcaaca ctttccattt gattcgagta taataattta ttttcatttt atatgctagt    1140
   ttagtatata cctaacttcc ttactaagtt tgatttatat ttaggcaata aagttcttta    1200
   tacaaaaaaa aaaaaaaaaa a                                              1221
<210> 177
   <211> 553
   <212> DNA
   <213> Diabrotica virgifera
<220>
   <221> misc_feature
   <222> (1)..(553)
   <223> RNAi target sequence
<400> 177
   ttaattctgc tgctcctcct ttgtggcata acggccccgt acctggagaa ttttacaatt        60
   ttcccggcaa acagaccagc tattccaacc ctataacaca tacacagtct cccgttacaa       120
   catcaacttc tgtattagct attacttacg ataaaggagt attgatagcc ggagatctgg       180
   ttgcttcata cggatcttta gctaggtacg gaaattgccc gagggttatt aaagtcaatc       240
   agaatatcat cttgggagcc ggaggagact atgctgattt ccaatacgtc aagagtttca       300
   ttgagcaaaa aataattgat gaagaatgca ttgatgatgg actaaaaatg aaaccaaaat       360
   cattgtactg ctggcttact agagtaatgt accaacgcag gagtaaatta gacccttttt        420
   ggaataatct ggttatagga ggaatccagg atggagtccc ctttcttgga accgtagata       480
   aattgggaac tgcatataca gacaaagtta tatgtacagg ttatggagcc cacatagcct       540
   tgcctcttct gag                                                          553
<210> 178
   <211> 22
   <212> DNA
   <213> Artificial Sequence
<220>
   <223> primer
<220>
   <221> misc_feature
   <222> (1)..(22)
   <223> forward primer
<400> 178
   ttaattctgc tgctcctcct tt 22
<210> 179
   <211> 22
   <212> DNA
   <213> Artificial Sequence
<220>
   <223> primer
<220>
   <221> misc_feature
   <222> (1)..(22)
   <223> reverse primer
<400> 179
   ctcagaagag gcaaggctat gt 22
<210> 180
   <211> 925
   <212> DNA
   <213> Diabrotica virgifera
<220>
   <221> misc_feature
   <222> (1)..(925)
   <223> WCRW transcript ta01874.001_diavv
<400> 180
   aaacaagtca tcgttgaatt ttctgaagtt agagattctt tgattacagt ttgttgtatt      60
   aaatagaaaa tgtttaattc tgctgctcct cctttgtggc ataacggccc cgtacctgga     120
   gaattttaca attttcccgg caaacagacc agctattcca accctataac acatacacag     180
   tctcccgtta caacatcaac ttctgtatta gctattactt acgataaagg agtattgata     240
   gccggagatc tggttgcttc atacggatct ttagctaggt acagaaattg cccgagggtt     300
   attaaagtca atcagaatat catcttggga gccggaggag actatgctga tttccaatac     360
   gtcaagagtt tcattgagca aaaaataatt gatgaagaat gcattgatga tggactaaaa     420
   atgaaaccaa aatcattgta ctgctggctt actagagtaa tgtaccaacg caggagtaaa     480
   ttagaccctt tttggaataa tctggttata ggaggaatcc aggatggagt cccctttctt     540
   ggaaccgtag ataaattggg aactgcatat acagacaaag ttatatgtac aggttatgga     600
   gcccacatag ccttgcctct tctgagagac tctttagata aaaatggagc tcttaacttg     660
   gccgatgcca aaactctgat tcacaaatgc atggaagtat tgttctatag agatgccaga     720
   agttttccta aatatcagct tggtgttatt gataaagatg atggtgttag tattgagggg     780
   cctatttcag tcgaagagaa ctggagcatt gcccatatga cataattttt cttaatttcg     840
   tttattttgt attgaaacta tattatacat tcatggaagt ctgtgtgaat aaatttttga     900
   taaaactaaa aaaaaaaaaa aaaaa                                           925
<210> 181
   <211> 420
   <212> DNA
   <213> Diabrotica virgifera
<220>
   <221> misc_feature
   <222> (1)..(420)
   <223> RNAi target sequence
<400> 181
   acaagcaatg gcgaaagtaa atgaagtggc taatttgtac aagcagttaa aatcagaatg        60
   gaataccaac aacccaaatt taagtaaatg tgaaaaacta ctctcaactc ttaagcttga       120
   gctcaccaat ctaatgtttc tgccgattac cacatctact gcgtccaaac aagaactact       180
   tctagccaga gatgttctag agataggagt ccaatggagt atagcagcca atgatattac       240
   atcttttgaa agatacatgg cacaattaaa atgttattac ttcgattata acaaccaact       300
   accagaatca ccatttaagt accaactgct tggattaaac ttgttattcc tgctgtcaca       360
   aaaccgagta gcagaatttc acactgaatt ggaactttta cctgccgatc atattcagaa       420
<210> 182
   <211> 22
   <212> DNA
   <213> Artificial Sequence
<220>
   <223> primer
<220>
   <221> misc_feature
   <222> (1)..(22)
   <223> forward primer
<400> 182
   acaagcaatg gcgaaagtaa at 22
<210> 183
   <211> 22
   <212> DNA
   <213> Artificial Sequence
<220>
   <223> primer
<220>
   <221> misc_feature
   <222> (1)..(22)
<400> 183
   ttctgaatat gatcggcagg ta 22
<210> 184
   <211> 1040
   <212> DNA
   <213> Diabrotica virgifera
<220>
   <221> misc_feature
   <222> (1)..(1040)
   <223> WCRW transcript ta06150.001_diavv
<400> 184
   gacagctgtc acaaatattc aacggattcc atttgttcct agtcatagtg taatttgtgg      60
   aagaattttg ttcgagtatt tatagctaaa attctgaaga tttaattaat taaacaagca     120
   atggcgaaag taaatgaagt ggctaatttg tacaagcagt taaaatcaga atggaatacc     180
   aacaacccaa atttaagtaa atgtgaaaaa ctactctcaa ctcttaagct tgagctcacc     240
   aatctaatgt ttctgccgat taccacatct actgcgtcca aacaagaact acttctagcc     300
   agagatgttc tagagatagg agtccaatgg agtatagcag ccaatgatat tacatctttt     360
   gaaagataca tggcacaatt aaaatgttat tacttcgatt ataacaacca actaccagaa     420
   tcaccattta agtaccaact gcttggatta aacttgttat tcctgctgtc acaaaaccga     480
   gtagcagaat ttcacactga attggaactt ttacctgccg atcatattca gaatgatgtg     540
   tacatcagac atcccttgtc tattgaacaa tatttaatgg aagggagtta taataaaata     600
   tttctggcta aaggtaatgt acctgctaag agttataact tctttatgga tattttgctg     660
   gacaccataa gagaagaaat tgcagactgt ttatcagtag cctatgaaaa gatctcagta     720
   aaagacgtag ccagaatgct gtacctgcca aacgaagaag ctgctaaaac atttgccaaa     780
   aaggctttcc agctcaaacc gtggaagctc aacaatgact ttttccactt tcttcctgaa     840
   gagaagaccc acgaacccat accctcacag gaactggcac agcaggctat agagtatgcc     900
   aaggaacttg aaatgattgt ctaataggtg tatttaattc ttatatttag gattttttta      960
   tataaaaatc aatatttaat aaacaaacaa tttatttgtt ttgtacttta aaaaaaaaaa     1020
   aaaaaaaaaa aaaaaatgcg                                                1040
<210> 185
   <211> 716
   <212> DNA
   <213> Diabrotica virgifera
<220>
   <221> misc_feature
   <222> (1)..(716)
   <223> RNAi target sequence
<220>
   <221> misc_feature
   <222> (608)..(608)
   <223> n can be any nucleotide
<220>
   <221> misc_feature
   <222> (645)..(645)
   <223> n can be any nucleotide
<400> 185
   cattacatag cgctttttgt tattgcaaat tgtcggcccg agttattttg tacaatggag       60
   tgtctacttg gtatcaaatt taatgattac gttcttgttg ctgcagatat gactgcagcg      120
   caaagcataa ttgtaatgaa aacagatgaa aacaagcttc ataaactttc caataagctt      180
   gttatggctg tttctggtga gtctggggat actacacagt ttgcagagta tatagcaaaa      240
   aatattcaac tttacaaaat gaggaatacc tatgaattga gtcccaaaga agctgcagca      300
   tttactagaa gaaatttggc agatgcactc agaagcagaa gtgcatacca tgtaaacctt      360
   cttctagctg gatatgatga caaagaaggt cctcagctat attacatgga ctacctagca      420
   tccatagcaa gcgttgatta tgctgctcat ggttatggag gattcttttc actgtcaatt      480
   atggatcgta attatcggca gaaccttagt aaagaagaag gttatgaact tttgaaaaaa      540
   tgtgtgaaag aagttcagaa aagattgatc atcaatttgc ctaactttaa ggtgcagtgt      600
   gttgatanaa atggcatcca ggatatgcct aatatcactg ctaangattt ggtttaattt      660
   agtttgatat aagttatgta ttttttttta ttattaaata aactgaattt tcttgt          716
<210> 186
   <211> 20
   <212> DNA
   <213> Artificial Sequence
<220>
   <223> primer
<220>
   <221> misc_feature
   <222> (1)..(20)
   <223> forward primer
<400> 186
   cattacatag cgctttttgt 20
<210> 187
   <211> 20
   <212> DNA
   <213> Artificial Sequence
<220>
   <223> primer
<220>
   <221> misc_feature
   <222> (1)..(20)
   <223> reverse primer
<400> 187
   acaagaaaat tcagtttatt 20
<210> 188
   <211> 872
   <212> DNA
   <213> Diabrotica virgifera
<220>
   <221> misc_feature
   <222> (1)..(872)
   <223> WCRW transcript ta01941.001_diavv
<400> 188
   aaaaatgggg ggctatcatt acatagcgct ttttgttatt gcaaattgtc ggcccgagtt      60
   attttgtaca atggagtgtc tacttggtat caaatttaat gactacgttc ttgttgctgc     120
   agatatgact gcagcgcaaa gcataattgt aatgaaaaca gatgaaaaca agcttcataa     180
   actttccaat aagcttgtta tggctgtttc tggtgagtct ggggatacta cacagtttgc     240
   agagtatata gcaaaaaata ttcaacttta caaaatgagg aatacctatg aattgagtcc     300
   caaagaagct gcagcattta ctagaagaaa tttggcagat gcactcagaa gcagaagtgc     360
   ataccatgta aaccttcttc tagctggata tgatgacaaa gaaggtcctc agctatatta     420
   catggactac ctagcatcca tagcaagcgt tgattatgct gctcatggtt atggaggatt     480
   cttttcactg tcaattatgg atcgtaatta tcggcagaac cttagtaaag aagaaggtta     540
   cgaacttttg aaaaaatgtg tgaaagaagt tcagaaaaga ttgatcatca atttgcctaa     600
   ctttaaggtg cagtgtgttg ataaaaatgg catccaggat atgcctaata tcactgctaa     660
   ggatttggtt taatttagtt tgatataagt tatgtatttt tttattatta aataaactga     720
   attttcttgt aaaaaaaaaa aaaaaagtag catttatttc tatattagga caagaaatta     780
   gatagggtgg tatatgcttg taacaaaatt tactaattat acggtaaacg ccaccctact     840
   agatacatag gaacattgag ctattacagt cc                                   872
<210> 189
   <211> 364
   <212> DNA
   <213> Diabrotica virgifera
<220>
   <221> misc_feature
   <222> (1)..(364)
   <223> RNAi target sequence
<220>
   <221> misc_feature
   <222> (274)..(274)
   <223> n can be any nucleotide
<400> 189
   ttggcatgac tgtgcaaata gtcggtccat tgtgcatgag taaagtgatt ctaaccataa      60
   cttcaaaacg ctttagtttt aaatatttgt gaacattaat aacagaaaca gttcattcaa     120
   aataatctat aagaatgggc gatacaaagg tagaacctac aaaggtggat aaaacaaagg     180
   ggactgagga caaaaacaaa aatgaactgt cggaagaaga caaacagctt caagatgaat     240
   taaatttgtg tgttgagaaa ctccaaggat aacnatgaaa agagatacct acttgctttg     300
   caaagcttta tcaaatcata ttcaaagcct caactacttc gatgacttct gttcctaaag     360
   ccgt                                                                  364
<210> 190
   <211> 22
   <212> DNA
   <213> Artificial Sequence
<220>
   <223> primer
<220>
   <221> misc_feature
   <222> (1)..(22)
   <223> forward primer
<400> 190
   ttggcatgac tgtgcaaata gt 22
<210> 191
   <211> 22
   <212> DNA
   <213> Artificial Sequence
<220>
   <223> primer
<220>
   <221> misc_feature
   <222> (1)..(22)
   <223> reverse primer
<400> 191
   acggctttag gaacagaagt ca 22
<210> 192
   <211> 3066
   <212> DNA
   <213> Diabrotica virgifera
<220>
   <221> misc_feature
   <222> (1)..(3066)
   <223> WCRW transcript ta44362.001_diavv
<400> 192
   attgaatgct tggcatgact gtgcaaatag tcggtccatt gtgcatgagt aaagtgattc      60
   taaccataac ttcaaaacgc tttagtttta aatatttgtg aacattaata acagaaacag     120
   ttcattcaaa ataatctata agaatgggcg atacaaaggt agaacctaca aaggggactg     180
   aggacaaaaa caaaaatgaa ctgtcggaag aagacaaaca gcttcaagat gaattaaatt     240
   tgtgtgttga gaaactccag gataacgatg aaaagagata cctacttgct ttgcaagctt     300
   tatcaaatca tattcaagcc tcaactactt cgatgacttc tgttcctaag ccgttaaaat     360
   ttatgaggcc ccattacgaa accatgaaga cagtacatga aaaaattact gatcctgata     420
   ctaaaaaagt ctgtgcagat attatatctg ttttagctat gacaatggga gaaggcagag     480
   aatgtttgaa ataccgtctt ctttctaatt tggataaaat tggcgaatgg ggacatgaat     540
   atgttaggca tttggcagga gagattgcag gagaatgggc cgagactgat tttatagtga     600
   acactgaaat tagggaaaaa cttatcttac taacaaaaca aattgtgcca tataatatgg     660
   cacacaatgc agaagcagaa gcatgtgact tgcttatgga aattgaaagg cttgatctac     720
   tagaacagta tgttgatgaa aacatctacc atagagtgtg cttatatctc accagctgtg     780
   taccctatgt ggcagatcct gagaatacta ctctactgca gactgctctt ttactgttta     840
   gaaagtttga tcagtatccc caggcactga gattagcaat gcagttaaat gactttggat     900
   taattgaaga aatttttgtc aactgcccag attcggacgt acaaaaacaa ctggcattta     960
   tgctaggacg acaacagata tatgttgaaa ttcctgagca tacgcctgaa tacgaagatc    1020
   taatcgagat aatggcaaac tgtcatctta ataaccactt cttgaacctc gctcgcgagt    1080
   tggacattat ggatcctaaa acccccgaag acgtttacaa atcccattta gaaaatagtc 1140
   gtcctccatt tggtggaaac caagttgatt cagccagaca aaacttagct gccagctttg 1200
   ttaacggttt tgttaatgct gcttttggtc aagataagct cttaatggaa gatgggaata 1260
   aatggcttta caaaaacaaa gaacatggaa tgcttagtgc tactgcttct ttgggactaa 1320
   ttcttttatg ggatgtagac ggtggtctta ctcctatcga taaatacttg tactcttctg 1380
   aagaccatat caagtctggc gctttattag cttgtggcat tgtaaactgc ggtgtcagga 1440
   atgaatgtga tcccgcactt gcacttttaa gtgattatgt tcttcacaac acaaacataa 1500
   tgagaatagg agctattgta ggtctaggtt tagcgtatgt tggttcaaat agggaagcag 1560
   tattatctct aatcacgcct gtgctatctg accctaaatc aaatatggag gtcattggaa 1620
   tggcggctct agcatgcgga atgatttctg caggatctgg taactcacat gtgacaacat 1680
   caataatgca agttcttatg gaaaagtccg aaatagaatt aaaggacact tacgccagat 1740
   ttttgcctct gggtcttgga ttgtgtcatt taggtaaaca agaaagcatt gacacaatta 1800
   tagcagcatt agaagttata cctgaccctt tcagatccat ggcgactact atggtcgata 1860
   tttgcgctta tgcaggcaca ggaaatgttt taaaagtaca acatttgtta catatatgtt 1920
   cagaacatta cgatgctcct gaaactcctg acaaaacttc aaaggacaaa gataaaaaag 1980
   aagcagagaa agaaaaagat aaggatcttt cagctagaca agctattgcg gttttgggta 2040
   tagctttaat tagcatggga gaagacattg gtagcgagat ggctttccgg acatttggtc 2100
   atttactaag atactgcgaa ccagcgatca gacgtgctgt tcccttagcc ctaggcttaa 2160
   tctcagtttc aaatccaaaa ttaagtattc tcgatacttt gagtaaattt tcacatgata 2220
   gcgatgctga agtggctcat aatgccatct ttgcgatggg tctggtggga gctggtacta 2280
   acaatgcaag gttagcagct atgctaagac agttagcaca gtaccatgcc aaagatccca 2340
   ataatttatt tatggttcgc atcgcccagg gtctcactca tcttggcaaa ggaactttaa 2400
   ctctcagtcc attccacagt gataggcagc tgatgagtcc agtagctgta gctggtctaa 2460
   tggcgacact catcggcttc ttggatgtta aaaacattat attggggaag tcacactact 2520
   tactgtacac cttagcagct gcaatgcaac caaggatgtt agtcacattt gacgatgagc 2580
   taaatcctct accagtgcct gtcagagttg gattggcagt ggatgttgtt ggccaagcag 2640
   gaaaaccaaa gactataaca ggtttccaaa cccataccac tcctgtgctt ctggcttatg 2700
   gtgagcgcgc tgagctcgca actgaagaat acattgcatt gacgccaatt atggaaggat 2760
   ttgttatttt acgaaaaaat cctgaattta attcataaat atattatttc cgtcattgtt 2820
   tatcatgttc cgcaattttt tgatttaata tacttttttg tattaatcga tatttattaa 2880
   atattgctca cgttttgatt gaaaacccta attgttcata acctcttcaa tatctgtata 2940
   tttgcattga aaatatctga atctgtatat tttaagaaaa aatcgcgaat ttaattcata 3000
   aatatagata aaagtatagg gacaaaatta tggagcccat ttctctaaag tggacgattt 3060
   tggaga 3066
<210> 193
   <211> 358
   <212> DNA
   <213> Diabrotica virgifera
<220>
   <221> misc_feature
   <222> (1)..(358)
   <223> RNAi target sequence
<220>
   <221> misc_feature
   <222> (267)..(267)
   <223> n can be any nucleotide
<400> 193
   cagcagtggt acagcaatca aatgtaaacg attatttact aaaaaaacaa caagaaagta 60
   ctaaagaatt aggctcagaa tgggccgaac ttgaagaact tcataataaa aaattgtggc 120
   atcagttgac tctaaaattg ctgaatttta ttaagaaacc agaactacag aagaatgata 180
   acctcattca actgtacaat aactttattc aaagttttga aaataaaatc aatcctttgt 240
   cattggttga aattgttgct atagtantcc aacaattcaa aaatccaaaa gatgctgtcg 300
   cttttcttga aaagactgaa cctaaggtta aaattaattc aagatgccca aaacttgt 358
<210> 194
   <211> 21
   <212> DNA
   <213> Artificial Sequence
<220>
   <223> primer
<220>
   <221> misc_feature
   <222> (1)..(21)
   <223> forward primer
<400> 194
   cagcagtggt acagcaatca a 21
<210> 195
   <211> 21
   <212> DNA
   <213> Artificial Sequence
<220>
   <223> primer
<220>
   <221> misc_feature
   <222> (1)..(21)
   <223> reverse primer
<400> 195
   acaagttttg ggcatcttga a 21
<210> 196
   <211> 1241
   <212> DNA
   <213> Diabrotica virgifera
<220>
   <221> misc_feature
   <222> (1)..(1241)
   <223> WCRW transcript ta04679.001_diavv
<400> 196
   taaaagcgaa aatggctgca gcagtggtac agcaatcaaa tgtaaacgat tatttactaa      60
   aaaaacaaca agaaagtcct aaagaattag gctcagaatg ggccgaactt gaagaacttc     120
   ataataaaaa attgtggcat cagttgactc taaaattgct gaattttatt aagaaaccag     180
   aactacagaa gaatgataac ctcatacaac tgtacaataa ctttattcaa agttttgaaa     240
   ataaaatcaa tcctttgtca ttggttgaaa ttgttgctat agtagtccaa caattcaaaa     300
   atccaaaaga tgctgtcgct tttcttgaaa agactgaacc taaggttaaa attaattcag     360
   atgcccaaaa cttgtgcaaa gttttagctg ggcagatata tatcgaaaag ttgaacgatt     420
   tagatgctac taaaaagata attgaagaag tggaatctac attggacaat gctgatggtg     480
   ttactccagt tcatggaaga ttttatttac ttgcctcaca atattataga attcaaggaa     540
   accatgctca atattatagg acagcattga gatatcttgg atgcatagag ttggatacaa     600
   taacatcaga agtgaaagtg caacacgctt tctttttggg tttggctgct ctattaggcg     660
   aaggtgtcta taacttaggt gaattactcg cacatcccgt tttggagtcc ctaaaatcaa     720
   cagaaaatgc gtggttggta gaattgttgt atgcttttaa ttctggcaat attaaaaagt     780
   ttgagtccat gaaaccgcag tgggaagcta tagcagatct tgcagctgaa gaattatttt     840
   tacgacaaaa aatatccctg ttgtgtctta tggagatgac atttaataga ccatcgcaca     900
   acaggcaatt aacatttgca gaaatcgcaa aagaaacaaa attacctttg aatgaaattg     960
   aattattgat tatgaaagcg ttgtcccaag gactagtcaa aggagccatt gatcaggttg    1020
   ccaataccgt aaatatgacc tgggttcaac ctagagtttt ggattgtacg cagatttcca    1080
   acatgattac taggcttaac gattggtgta aacatgttga taccatggag caattgctgg    1140
   aagaaaaagc tagtgaaata ttaacattgt aatttgtaat tattaataat ttatatattc    1200
   gtccttaatt tttttttaag gttttctcac tgataataaa a                         1241
<210> 197
   <211> 410
   <212> DNA
   <213> Diabrotica virgifera
<220>
   <221> misc_feature
   <222> (1)..(410)
   <223> RNAi target sequence
<400> 197
   tggaggtgat aacgaggtac aacgtactat gttagaactt atcaatcagt tggatggttt      60
   tgatcctaga ggtaacatta aagtacttat ggctacaaat cgtccagata ctctagatcc     120
   tgcccttatg agacctggtc gtttggatcg taaagttgaa tttggacttc cagaccttga     180
   aggaagaact catattttca aaattcatgc taggtccatg tctgttgaaa gagatattcg     240
   ttttgaactt ttggctaggt tgtgtccaaa ttccactgga gctgaaatca gaagtgtttg     300
   taccgaagct ggtatgttcg ctataagagc aaggaggaaa gtagctaccg aaaaagactt     360
   cttagaagct gtaaataaag taattaagtc atacgccaag ttctctgcta                410
<210> 198
   <211> 22
   <212> DNA
   <213> Artificial Sequence
<220>
   <223> primer
<220>
   <221> misc_feature
   <222> (1)..(22)
   <223> forward primer
<400> 198
   tggaggtgat aacgaggtac aa 22
<210> 199
   <211> 22
   <212> DNA
   <213> Artificial Sequence
<220>
   <223> primer
<220>
   <221> misc_feature
   <222> (1)..(22)
   <223> reverse primer
<400> 199
   tagcagagaa cttggcgtat ga 22
<210> 200
   <211> 1452
   <212> DNA
   <213> Diabrotica virgifera
<220>
   <221> misc_feature
   <222> (1)..(1452)
   <223> WCRW transcript ta07921.001_diavv
<400> 200
   ttatttaaaa ctaaatctat gaaaaatatt gttcttaaaa tatttttcat agatttagtt      60
   ttaaataaag acaattaaga tgccggacca tttaggagat gatatgcgaa aaataaaaga     120
   acagaaagag gagcccgaaa aggaaattaa agccctggat gaaggtgata tagcacttct     180
   taaaacctat ggacaagggc aatacacaaa gacaattaaa acatgtgaag aagatatcca     240
   aacgataatc aaaagagtaa atgaattaac tggtataaaa gaatctgata ctggtttagc     300
   tccaccagct ttatgggatt tggcagctga caaacaaacc ttacaaaatg agcaaccctt     360
   acaagtggcc cgttgtacga aaattatcaa cgctgataca gatgatccca aatacattat     420
   aaacgttaaa cagtttgcca aattcgtagt agatttggca gactctgtgg cccctacaga     480
   catagaagaa ggcatgagag ttggagtcga ccgaaataag tatcaaattc atattcctct     540
   acctccaaaa attgatccca ctgtgactat gatgcaagtt gaggagaagc ctgatgttac     600
   atacagtgat gttggtggct gcaaggaaca aattgaaaag ttgagagaag ttgttgaaac     660
   acctttactc catcctgaaa aatttgtaaa acttggtatt gaaccaccaa aaggcgttct     720
   cctttttggc ccccctggaa ctggtaaaac tctttgtgct agagcagtag ccaatagaac     780
   agatgcctgc tttattcgtg taattggttc tgaactggtt cagaagtacg taggagaggg     840
   agcaagaatg gttagagaac ttttcgaaat ggctaggtct aagaaggcct gcttgatatt     900
   ctttgatgaa attgatgcca ttggtggagc cagatttgat gatggcgctg gaggtgataa     960
   cgaggtacaa cgtactatgt tagaacttat caatcagttg gatggttttg atcctagagg    1020
   taacattaaa gtacttatgg ctacaaatcg tccagatact ctagatcctg cccttatgag    1080
   acctggtcgt ttggatcgta aagttgaatt tggacttcca gaccttgaag gaagaactca    1140
   tattttcaaa attcatgcta ggtccatgtc tgttgaaaga gatattcgtt ttgaactttt    1200
   ggctaggttg tgtccaaatt ccactggagc tgaaatcaga agtgtttgta ccgaagctgg    1260
   tatgttcgct ataagagcaa ggaggaaagt agctaccgaa aaagacttct tagaagctgt    1320
   aaataaagta attaagtcat acgccaagtt ctctgctaca ccaagatata tgacttacaa    1380
   ttaattttaa agattttcta attatcgaat tatttaaatt gcgtttctaa ataaataatc    1440
   cttttttagt gt                                                         1452
<210> 201
   <211> 458
   <212> DNA
   <213> Diabrotica virgifera
<220>
   <221> misc_feature
   <222> (1)..(458)
   <223> RNAi target sequence
<400> 201
   tctcctgaag ggaggttgta tcaggtggag tatgccatgg aggctatttc tcatgcaggt      60
   acttgtctgg gcattttagc aaatgatgga attttgttgg ctgctgagag gcgtaacact     120
   aacaaattat tagatgaagt ttttatttca gaaaaaatat acaaactaaa tgatgatatg     180
   gtttgtagtg tggctggaat aacatcagat gctaatgttt taacaaatga attgagactt     240
   ataggtcaaa gatatttatt tcagtatgcc gaatctattc cttgcgaaca gttagtttcc     300
   tggctttgtg atgtcaagca ggcatacact cagtatggag gtaaacgtcc atttggtgtt     360
   tcaatattat atatgggatg ggataaacat tatggatacc agctatatca gtcagatccc     420
   agtggcaact attctggttg gaaagctacc tgcatagg                             458
<210> 202
   <211> 22
   <212> DNA
   <213> Artificial Sequence
<220>
   <223> primer
<220>
   <221> misc_feature
   <222> (1)..(22)
   <223> forward primer
<400> 202
   tctcctgaag ggaggttgta tc 22
<210> 203
   <211> 22
   <212> DNA
   <213> Artificial Sequence
<220>
   <223> primer
<220>
   <221> misc_feature
   <222> (1)..(22)
   <223> reverse primer
<400> 203
   cctatgcagg tagctttcca ac 22
<210> 204
   <211> 892
   <212> DNA
   <213> Diabrotica virgifera
<220>
   <221> misc_feature
   <222> (1)..(892)
   <223> WCRW transcript ta04612.001_diavv
<400> 204
   tttaaatttt ctaggctcag tatttttagc tatattgagt atcaactaaa actatggctc      60
   gtcgatatga taccagaacc acaatattct ctcctgaagg gaggttgtat caggtggagt     120
   atgccatgga ggctatttct catgcaggta cttgtctggg cattttagca aatgatggaa     180
   ttttgttggc tgctgagagg cgtaacacta acaaattatt agatgaagtt tttatttcag     240
   aaaaaatata caaactaaat gatgatatgg tttgtagtgt ggctggaata acatcagatg     300
   ctaatgtttt aacaaatgaa ttgagactta taggtcaaag atatttattt cagtatgccg     360
   aatctattcc ttgcgaacag ttagtttcct ggctttgtga tgtcaagcag gcatacactc     420
   agtatggagg taaacgtcca tttggtgttt caatattata tatgggatgg gataagcatt     480
   atggatacca gctatatcag tcagatccca gtggcaacta ttctggttgg aaagctacct     540
   gcataggcaa caacagttca gcagcaattt caagtttgaa acaagaatat aaagaaggtc     600
   aaatgacttt gaaggatgca aagacgttag cagtaaaggt tctggcaaaa acattggata     660
   tggcaaaact aacttcagaa aaagtggaaa tggctacact tacccgcaaa aatgataaaa     720
   ccatgattaa aattttaagc ggcaaggaag tagaagaact gatttcagag tatgaaaaaa     780
   gtgaagctgc cgcagaagct agcaaaaaag agcagaaggc atcagcttaa ttacatttat     840
   ttggttttgt ttaggttcac taagtatgtc attgtcacaa tatgaataaa tg             892
<210> 205
   <211> 453
   <212> DNA
   <213> Diabrotica virgifera
<220>
   <221> misc_feature
   <222> (1)..(453)
   <223> RNAi target sequence
<400> 205
   tgcagcttat agtggctgaa aagagtcaaa atcttagacg tttgcaagct cagagaaatg      60
   aactcaatgc caaagttcgt atgttgagag aagaactgat gttacttcaa gagcaaggaa     120
   gctatgtcgg agaagttgta aagcccatgg ataagaaaaa agtccttgtg aaagttcatc 180
   cagaaggaaa atttgtagtt gacattgaca aaaacataga cataaatgat gttactccaa 240
   attgtcgagt agccctcaga aatgaatctt acacattgca caaaatactt ccaaacaagg 300
   ttgatccttt agtttccttg atgatggtgg agaaagtccc tgattcaact tatgaaatgg 360
   taggaggact tgacaagcaa attaaagaaa tcaaagaagt aattgaactt ccagttaaac 420
   atccagaact ttttgatgct cttggtattg cac 453
<210> 206
   <211> 22
   <212> DNA
   <213> Artificial Sequence
<220>
   <223> primer
<220>
   <221> misc_feature
   <222> (1)..(22)
   <223> forward primer
<400> 206
   tgcagcttat agtggctgaa aa 22
<210> 207
   <211> 22
   <212> DNA
   <213> Artificial Sequence
<220>
   <223> primer
<220>
   <221> misc_feature
   <222> (1)..(22)
   <223> reverse primer
<400> 207
   gtgcaatacc aagagcatca aa 22
<210> 208
   <211> 1322
   <212> DNA
   <213> Diabrotica virgifera
<220>
   <221> misc_feature
   <222> (1)..(1322)
   <223> WCRW transcript ta01297.001_diavv
<400> 208
   aaactcccgg agtgcaaaaa actatcaaga atgacggtaa ttaacaagat ggaggtggac 60
<210> 209
   <211> 488
   <212> DNA
   <213> Diabrotica virgifera
<220>
   <221> misc_feature
   <222> (1)..(488)
   <223> RNAi target sequence
<400> 209
   gttaagatgc cggaccattt aggagatgat atgcgaaaaa taaaagaaca gaaagaggag      60
   cccgaaaagg aaattaaagc cctggatgaa ggtgatatag cacttcttaa aacctatgga     120
   caagggcaat acacaaagac aattaaaaca tgtgaagaag atatccaaac gataatcaaa     180
   agagtaaatg aattaactgg tataaaagaa tctgatactg gtttagctcc accagcttta     240
   tgggatttgg cagctgacaa acaaacctta caaaatgagc aacccttaca agtggcccgt     300
   tgtacgaaaa ttatcaatgc tgatacagat gatcccaaat acattataaa cgttaaacag     360
   tttgccaaat tcgtagtaga tttggcagac tctgtggccc ctactgacat agaagaaggc     420
   atgagagttg gagtcgaccg aaataagtat caaattcata ttcctctacc tccaaaaatt     480
   gatcccac                                                              488
<210> 210
   <211> 22
   <212> DNA
   <213> Artificial Sequence
<220>
   <223> primer
<400> 210
   gttaagatgc cggaccattt ag 22
<210> 211
   <211> 22
   <212> DNA
   <213> Artificial Sequence
<220>
   <223> primer
<220>
   <221> misc_feature
   <222> (1)..(22)
   <223> reverse primer
<400> 211
   gtgggatcaa tttttggagg ta 22
<210> 212
   <211> 1425
   <212> DNA
   <213> Diabrotica virgifera
<220>
   <221> misc_feature
   <222> (1)..(1425)
   <223> WCRW transcript 454run3_isotig16670
<400> 212
   aaaatttgct tgtaatcttg cacaaaaata ttttaagaac aatatttttc atagatttag      60
   ttttaaataa agacaattaa gatgccggac catttaggag atgatatgcg aaaaataaaa     120
   gaacagaaag aggagcccga aaaggaaatt aaagccctgg atgaaggtga tatagcactt     180
   cttaaaacct atggacaagg gcaatacaca aagacaatta aaacatgtga agaagatatc     240
   caaacgataa tcaaaagagt aaatgaatta actggtataa aagaatctga tactggttta     300
   gctccaccag ctttatggga tttggcagct gacaaacaaa ccttacaaaa tgagcaaccc     360
   ttacaagtgg cccgttgtac gaaaattatc aacgctgata cagatgatcc caaatacatt     420
   ataaacgtta aacagtttgc caaattcgta gtagatttgg cagactctgt ggcccctaca     480
   gacatagaag aaggcatgag agttggagtc gaccgaaata agtatcaaat tcatattcct     540
   ctacctccaa aaattgatcc cactgtgact atgatgcaag ttgaggagaa gcctgatgtt     600
   acatacagtg atgttggtgg ctgcaaggaa caaattgaaa agttgagaga agttgttgaa     660
   acacctttac tccatcctga aaaatttgta aaacttggta ttgaaccacc aaaaggcgtt     720
   ctcctttttg gcccccctgg aactggtaaa actctttgtg ctagagcagt agccaataga     780
   acagatgcct gctttattcg tgtaattggt tctgaactgg ttcagaagta cgtaggagag     840
   ggagcaagaa tggttagaga acttttcgaa atggctaggt ctaagaaggc ctgcttgata     900
   ttctttgatg aaattgatgc cattggtgga gccagatttg atgatggcgc tggaggtgat     960
   aacgaggtac aacgtactat gttagaactt atcaatcagt tggatggttt tgatcctaga    1020
   ggtaacatta aagtacttat ggctacaaat cgtccagata ctctagatcc tgcccttatg    1080
   agacctggtc gtttggatcg taaagttgaa tttggacttc cagaccttga aggaagaact    1140
   catattttca aaattcatgc taggtccatg tctgttgaaa gagatattcg ttttgaactt    1200
   ttggctaggt tgtgtccaaa ttccactgga gctgaaatca gaagtgtttg taccgaagct    1260
   ggtatgttcg ctataagagc aaggaggaaa gtagctaccg aaaaagactt cttagaagct    1320
   gtaaataaag taattaagtc atacgccaag ttctctgcta caccaagata tatgacttac    1380
   aattaatttt aaagattttc taattatcga attatttaaa ttgcg                    1425
<210> 213
   <211> 553
   <212> DNA
   <213> Diabrotica virgifera
<220>
   <221> misc_feature
   <222> (1)..(553)
   <223> RNAi target sequence
<220>
   <221> misc_feature
   <222> (485)..(485)
   <223> n can be any nucleotide
<400> 213
   caaaggaact cgcagagctt ataaaggcca ccaggccttt tttgagttta ataagcaaag    60
   caaaagctgc taaattggta aggtctctag ttgactactt cctagattta gaagctggta   120
   ttggtatcga agtacaactc tgcaaagaat gtatagaatg ggcaaaggaa gagagaagaa   180
   cttttctacg tcagtccctt gaagccaggc tgattgctct ttattttgac actggaatgt   240
   acagcgaagc gctacaactg gaatcgacct tactgaaaga gttaaaaaag ttggatgaca   300
   aaaatttgtt ggtggaagta caactacttg aaagcaaaac atatcatgct ttaagtaatt   360
   taccgaaggc tagagctgca ctaacttcag cacgaaccac agctaacgct atctattgcc   420
   ctccaaagat gcaggcagct ttagatttac aatccggagt attacacgct gcagatgaga   480
   agganttcaa aacagcttat tcttattttt atgaagcttt tgaaggtttt gatagtgttg   540
   aatccccaaa agc                                                      553
<210> 214
   <211> 22
   <212> DNA
   <213> Artificial Sequence
<220>
   <223> primer
<220>
   <221> misc_feature
   <222> (1)..(22)
   <223> forward primer
<400> 214
   caaaggaact cgcagagctt at 22
<210> 215
   <211> 22
   <212> DNA
   <213> Artificial Sequence
<220>
   <223> primer
<220>
   <221> misc_feature
   <222> (1)..(22)
   <223> reverse primer
<400> 215
   gcttttgggg attcaacact at 22
<210> 216
   <211> 1860
   <212> DNA
   <213> Diabrotica virgifera
<220>
   <221> misc_feature
   <222> (1)..(1860)
   <223> WCRW transcript ta03917.001_diavv
<400> 216
   gggtcagatc cgatcatatt aacgtgaaaa gcatatttat gacagacaaa ttaatggaag      60
   tttagtagtg agtagtgaaa aaatatggcc ggagcaatgt tatttgaaag agccccgtct     120
   gtttcttctc aacacgagaa gcttttaaaa ttatccaggg attcagatga tgaagatcaa     180
   aatattgtca ataaagaaca agacatcctt aatcttggtg agaaatataa aaaggagggc     240
   aaggcaaagg aactcgcaga gcttataaag gccaccaggc cttttttgag tttaataagc     300
   aaagcaaaag ctgctaaatt ggtaaggtct ctagttgact acttcctaga tttagaagct     360
   ggtattggta tcgaagtaca actctgcaaa gaatgtatag aatgggcaaa ggaagagaga     420
   agaacttttc tacgtcagtc ccttgaagcc aggctgattg ctctttattt tgacactgga     480
   atgtacagcg aagcgctaca actggaatcg accttactga aagagttaaa aaagttggat     540
   gacaaaaatt tgttggtgga agtacaacta cttgaaagca aaacatatca tgctttaagt     600
   aatttaccga aggctagagc tgcactaact tcagcacgaa ccacagctaa cgctatctat     660
   tgccctccaa agatgcaggc agctttagat ttacaatccg gagtattaca cgctgcagat     720
   gagaaggatt tcaaaacagc ttattcttat ttttatgaag cttttgaagg ttttgatagt     780
   gttgaatccc caaaagccct cacagccctt aaatacatgc ttttgtcaaa aatcatgata     840
   aacagtccgg aagacgtcca acaaatcgtt agtggtaaat tggcctttaa atacgctggt     900
   caagatatag aggctatgaa atcagttgca caggctgcac acaaaagatc actagcagat     960
   ttccaacaag ctgtaaaaca gtttaaacat gaattagaag atgatgttat tgttagagca    1020
   catttaggaa cgttgtatga caatatgttg gagcagaacc tttgcagaat tatcgaacca    1080
   tattctaggg tacaagtaga gtatgtagca aaaaccataa aactcccagt actacaagta    1140
   gagaagaaat tgtcacagat gattttagat gccaagtttc atggaatttt ggaccaaggc    1200
   gaaggtgtcc tcatagtatt cgaagaaagt gcagtagaca aaacatatga aatggcccta    1260
   gaaacgatcc agagtatgag caaagtagta gatactcttt atcaaaaagc aaagaaattg    1320
   tcataggtat aatgtagccg agaatgttat gaaaataata aaatgactgt aaatttttca    1380
   gaactagatt ttttgtagac actgtgttta tataacctat ttaatttgct tttgttaatt    1440
   cttgtctttt taaggattct tataactcat agttttcgga gcttgagggt tttggatgtg    1500
   atggagtttt tttgttaata cagtatagat attttttaag atacttaaag cttatctaaa    1560
   tctaggaaaa tatcatgcct aaagtcataa gagattcata atccatatgt aactaactca    1620
   tagaactatt ttctttataa tattttttac aagcaatgaa ataaaagcct caaaaaatag    1680
   actgaacttt agagacttta ggcattgagt tttggtcttt attgggaaaa tattaataca    1740
   aaagttctat aagaaatatc tttgtatggt ttattaattc attttttaac ctgccaagac     1800
   attgtgatct ctattactat cagtgtcatt ggcttgtttt catacaattt ttccctaata    1860
<210> 217
   <211> 469
   <212> DNA
   <213> Diabrotica virgifera
<220>
   <221> misc_feature
   <222> (1)..(469)
   <223> RNAi target sequence
<220>
   <221> misc_feature
   <222> (294)..(294)
   <223> n can be any nucleotide
<220>
   <221> misc_feature
   <222> (295)..(295)
   <223> n can be any nucleotide
<220>
   <221> misc_feature
   <222> (296)..(296)
   <223> n can be any nucleotide
<220>
   <221> misc_feature
   <222> (385)..(385)
   <223> n can be any nucleotide
<220>
   <221> misc_feature
   <222> (386)..(386)
   <223> n can be any nucleotide
<400> 217
   gtcaatacct tttcacccga aggaagatta tttcaagtac aatatgccat acaagctatt       60
   aagttaggat ctaccgctat tggaatatgc accagtggtg gagtagtgct tgctgtacaa      120
   aaacgcatta cttccccatt aatggaacct actacgattg agaaaattgt agaagttgac      180
   aaacatattg gatgtgcggt ttcaggactt atggcagatt caaggactat gatagataga      240
   gctcgtgtgg aatgtcacaa tcattggttt gtctacaatg aaaatataag tgtnnnatcc      300
   tgtgcacaag gtgtatcaaa tttggctatt cagttcggat attctgatga agatggtgca      360
   gccatgtcta gaccttttgg agtannaatt ttgtttgctg gtatagatga aaaaggacct      420
   cagttgtacc atatggatcc atcaagaaca tttgttcagt ttgacgcaa                  469
<210> 218
   <211> 22
   <212> DNA
   <213> Artificial Sequence
<220>
   <223> primer
<220>
   <221> misc_feature
   <222> (1)..(22)
   <223> forward primer
<400> 218
   gtcaatacct tttcacccga ag 22
<210> 219
   <211> 21
   <212> DNA
   <213> Artificial Sequence
<220>
   <223> primer
<220>
   <221> misc_feature
   <222> (1)..(21)
   <223> reverse primer
<400> 219
   ttgcgtcaaa ctgaacaaat g 21
<210> 220
   <211> 850
   <212> DNA
   <213> Diabrotica virgifera
<220>
   <221> misc_feature
   <222> (1)..(850)
   <223> WCRW transcript ta09615.001_diavv
<400> 220
   gctattacaa gtttcttttg tataagtttt ataaatattg ttaatcgtta acatagtcct      60
   gtttacaaaa tgtttcttac aagaagtgaa tacgataggg gagtcaatac cttttcaccc     120
   gaaggaagat tatttcaagt agaatatgcc atagaagcta ttaagttagg atctaccgct     180
   attggaatat gcaccagtgg tggagtagtg cttgctgtag aaaaacgcat tacttcccca     240
   ttaatggaac ctactacgat tgagaaaatt gtagaagttg acaaacatat tggatgtgcg     300
   gtttcaggac ttatggcaga ttcaaggact atgatagata gagctcgtgt ggaatgtcag     360
   aatcattggt ttgtctacaa tgaaaatata agtgtagaat cctgtgcaca aggtgtatca     420
   aatttggcta ttcagttcgg agattctgat gaagatggtg cagccatgtc tagacctttt     480
   ggagtagcaa ttttgtttgc tggtatagat gaaaaaggac ctcagttgta ccatatggat     540
   ccatcaggaa catttgttca gtttgacgca aaggcgatag gatcaggatc cgagggtgca     600
   cagcaaagtt tacaggaggc atatcatcgc agtatgaccc tggaagaagc taccacaact     660
   gctttaacaa ttctaaaaca agttatggaa gaaaagttga attcttcaaa tgttgaggtt     720
   atgactatga ctccaaaaga tatgttccac atgttcacaa aggaacaagt ggaagaggta     780
   attgctaaat tgtcttaact taggaagtaa gctaaatttt gaaaagggtt aattttatat     840
   aagttttttt                                                            850
<210> 221
   <211> 404
   <212> DNA
   <213> Diabrotica virgifera
<220>
   <221> misc_feature
   <222> (1)..(404)
   <223> RNAi target sequence
<220>
   <221> misc_feature
   <222> (300)..(300)
   <223> n can be any nucleotide
<220>
   <221> misc_feature
   <222> (301)..(301)
   <223> n can be any nucleotide
<220>
   <221> misc_feature
   <222> (302)..(302)
   <223> n can be any nucleotide
<220>
   <221> misc_feature
   <222> (338)..(338)
   <223> n can be any nucleotide
<220>
   <221> misc_feature
   <222> (339)..(339)
   <223> n can be any nucleotide
<220>
   <221> misc_feature
   <222> (340)..(340)
   <223> n can be any nucleotide
<220>
   <221> misc_feature
   <222> (341)..(341)
   <223> n can be any nucleotide
<400> 221
   ttcggaagta aaacaagtgc tgcaggatgt ggatactgaa gatttgtaca ctaaatataa      60
   aaaacttcaa caacagttgg agttcttgga ggttcaagaa gagtacatta aagatgagca     120
   aaggaatctc aaaaaggagt atttacatgc tcaggaagaa gtgaagcgta tccagtctgt     180
   cccgcttgtt attggacagt ttttagaagc tgtagatcaa aacactggca ttgttggctc     240
   aactacaggt tcaaattatt atgttagaat cttatcaaca attgatagag aactcttaan     300
   nnccttctgc tagtgttgct cttcataaac acagcaannn ncttagtaga tgtcctacca     360
   ccagaggcag attcttcaat aagcatgctc caggctgatg aaaa                      404
<210> 222
   <211> 22
   <212> DNA
   <213> Artificial Sequence
<220>
   <223> primer
<220>
   <221> misc_feature
   <222> (1)..(22)
   <223> forward primer
<400> 222
   ttcggaagta aaacaagtgc tg 22
<210> 223
   <211> 20
   <212> DNA
   <213> Artificial Sequence
<220>
   <223> primer
<220>
   <221> misc_feature
   <222> (1)..(20)
   <223> reverse primer
<400> 223
   ttttcatcag cctggagcat 20
<210> 224
   <211> 1364
   <212> DNA
   <213> Diabrotica virgifera
<220>
   <221> misc_feature
   <222> (1)..(1364)
   <223> WCRW transcript ta03370.001_diavv
<400> 224
   aaaaaagtca aaatttgaag aagaaaacaa ttttacaagt catcaaccat ggaggctatg 60
   gatattgtta taccagaaaa agaagaaggt agtccttcgg aagtaaaaca agtgctgcag 120
   gatgtggata ctgaagattt gtacactaaa tataaaaaac ttcaacaaca gttggagttc 180
   ttggaggttc aagaagagta cattaaagat gagcaaagga atctcaaaaa ggagtattta 240
   catgctcagg aagaagtgaa gcgtatccag tctgtcccgc ttgttattgg acagttttta 300
   gaagctgtag atcaaaacac tggcattgtt ggctcaacta caggttcaaa ttattatgtt 360
   agaatcttat caacaattga tagagaactc ttaaaacctt ctgctagtgt tgctcttcat 420
   aaacacagca atgccttagt agatgtccta ccaccagagg cagattcttc aataagcatg 480
   ctccaggctg atgaaaaacc agatgttcaa tacagtgaca ttggaggtat ggatatgcag 540
   aaacaagaaa ttagggaagc tgtagagtta cctttgactc acttcgaatt gtataaacag 600
   attggtattg atccccccag aggtgtgttg atgtatggac ctcctggatg cgggaagact 660
   atgttggcca aagctgttgc tcatcatact acagctgcct ttattcgtgt agtcggatca 720
   gaattcgtac agaaatatct tggtgaaggt cctcgaatgg ttagagacgt gttccgtcta 780
   gcaaaagaaa attctccagc aatcatcttt attgatgaaa ttgatgcaat cgctaccaaa 840
   cgttttgatg cccagacagg tgctgatcgt gaagtccaga gaattttatt ggaactgctt 900
   aaccagatgg atggttttga tcagacaaca aatgttaagg ttatcatggc aactaacaga 960
   gctgatacat tagatcctgc acttctgaga cctggtcgtc tggacagaaa aatcgaattt 1020
   cctttaccag atagaagaca aaagcgtttg attttcagca ctatcacatc taaaatgaat 1080
   ttgtcagagg aggtcgactt ggaggactat gtagctagac cagacagaat atcaggagca 1140
   gacataaacg ccatctgcca ggaagccggt atgcatgccg tccgagaaaa ccgttacatc 1200
   gttttaccta aagatttcga gaagggttat aagaataaca ttaagaagga tgagagcgag 1260
   cacgagtttt acaaatagtt tatttatatt ttttattatt actaatctaa taaagacttg 1320
   taaaatgtat ttgatgtcat tgaatcaagt ttacattaaa aaaa 1364
<210> 225
   <211> 455
   <212> DNA
   <213> Diabrotica virgifera
<220>
   <221> misc_feature
   <222> (1)..(455)
   <223> RNAi target sequence
<400> 225
   cggaattaaa tagtgcccaa gatgaatatc acatctgcag cgggaattat ttccctcttg 60
   gatgaaccaa gaccagaatt aaaagtattt gcactgaaga aactagattc catagtgggt 120
   gagttttggc cggagatctc agaagctatc gaaaaaatcg aaattttgca tgaagacaaa 180
   gttttccagc aacaccagtt agctgcactt gtggcaagta aggtatatta ccatttaggc 240
   gcttttgaag actctttaac ttatgctctt ggtgcgggtg atctctttga tgtcaatgcc 300
   cggagtgagt atgtagaaac tattctagct aaatgtatcg actattacac tcagcaacgt 360
   attgctgtag ctgaaggatt ggcagatgcc aaacccatag actctcgcct tgaagctatt 420
   gttaatcgaa tgtttcagag gtgtcttgat gatgg 455
<210> 226
   <211> 22
   <212> DNA
   <213> Artificial Sequence
<220>
   <223> primer
<220>
   <221> misc_feature
   <222> (1)..(22)
   <223> forward primer
<400> 226
   cggaattaaa tagtgcccaa ga 22
<210> 227
   <211> 22
   <212> DNA
   <213> Artificial Sequence
<220>
   <223> primer
<220>
   <221> misc_feature
   <222> (1)..(22)
   <223> reverse primer
<400> 227
   ccatcatcaa gacacctctg aa 22
<210> 228
   <211> 3242
   <212> DNA
   <213> Diabrotica virgifera
<220>
   <221> misc_feature
   <222> (1)..(3242)
   <223> WCRW transcript ta00483.001_diavv
<400> 228
   tgaatatcca catcaaaaat tgcattcatt caatgactgt gttgtttttc cgacgtgaaa      60
   gtttaaaaat tacatttttc ggaattaaat agtgcccaag atgaatatca catctgcagc     120
   gggaattatt tccctcttgg atgaaccaag accagaatta aaagtatttg cactgaagaa     180
   actagattcc atagtgggtg agttttggcc ggagatctca gaagctatcg aaaaaatcga     240
   aattttgcat gaagacaaag ttttccagca acaccagtta gctgcacttg tggcaagtaa     300
   ggtatattac catttaggcg cttttgaaga ctctttaact tatgctcttg gtgcgggtga 360
   tctctttgat gtcaatgccc ggagtgagta tgtagaaact attctagcta aatgtatcga 420
   ctattacact cagcaacgta ttgctgtagc tgaaggattg gcagatgcca aacccataga 480
   ctctcgcctt gaagctattg ttaatcgaat gtttcagagg tgtcttgatg atggtcaata 540
   tcgtcaagct atgggcctgg ctctagaaac cagacgaatg gatatctttg agtcatctat 600
   tactcagtct gatgatatta tcagcatgtt gacttatgca tttcaagtag cgatgagttt 660
   gattcaaaat cgagcattta gaaacactgt tttgagatct cttgttggac tttatagagg 720
   actggttaca cctgattacg ttaatatgtg ccagtgctta attttcctcg atgatccatt 780
   gtctgtggct gaaattttag acaggcttgt aactggcaag gaggatactg aacttatggc 840
   atatcaaatt gcttttgatc tctatgagtc agctactcaa cagtttttgg aaagagtatt 900
   agttgcatta agagccacag caccagttcc atctttgctg gaagaaaaag caaagccaaa 960
   gccttctggt gaaggagagg gtgctatgtc aaccgagact ggagaaccat ctacagaaac 1020
   aaaagaagaa aaattaaaag aagaaaaatc tctggatagt ttgtctgatc aggataagga 1080
   acatcagaaa agaattgaga aacttcatac tatactctca ggggaggtct ctatagaact 1140
   gcaccttcaa ttcctcatca ggtcaaatca tgctgatcta ctgattctta aacaaacaaa 1200
   agagactgtc agagttagta tttgccacac tgctactgta atcgcaaacg catttatgca 1260
   tagtggtact actagcgatc aatttttaag agataactta gaatggttag ccagagcaac 1320
   gaattgggcc aaacttacag ccacagcctc gctaggagtt attcatagag gccatgaaca 1380
   agaagcatta acactaatgc aaagttactt gccgaaagaa gttggaccca gctcaggtta 1440
   ttccgaaggg ggcggccttt atgctcttgg tcttatacat gccaaccatg gtacaaatat 1500
   cattgattac cttcttggac aacttaagga tgcacagaat gagatggtga gacatggagg 1560
   ttgtcttggt ctaggtttgg ctgctatggg cactcataga caggatgttt atgaacaact 1620
   caaattcaat ttataccaag atgatgccaa tactggtgaa gcagctggta ttgcaatggg 1680
   aatggtaatg ttgggttcaa gtaattctac cacaattatg gatatggttg catacgcaca 1740
   agagagtcaa catgagaaaa ttcttagagg cttagctgtt ggtattagtt tttccatgta 1800
   tggtagatta gaagaagcag atccactgat tcaacaactt acctctgata aagatcctat 1860
   cctcagaaga tcaggaatgt atactttggg tatggcatat tgtggtactg gacacaacca 1920
   agcaattaga aaactacttc atgtagctgt gtcagatgtc aacgatgatg ttagaagagc 1980
   tgcagttaca gctttaggtt ttctgttatt cagaactcct gaacaatgtc ctagtgtagt 2040
   atcccttcta gctgagagtt acaatccaca tgtacgttat ggagctgcta tggctcttgg 2100
   tattgcatgt gcaggaactg gtctacgtga agctatagct cttctcgaac ctatggttat 2160
   gtttgatcct gtaaacttcg taagacaggg tgctctcatt gcttctgcta tgattttaat 2220
   tcaacagact gaacaaactt gccccaaagt cacattcttc agacagacat atgctcaagt 2280
   catttccaac aaacacgaag atgtgatggc caagtttgga gctatcttag ctcaaggaat 2340
   catcgatgca ggtggaagaa atgtaacttt gtcgctacag tccagaactg gccacacaaa 2400
   tatgttagct gtagttggta cactggtgtt cactcagtac tggtactggt tccctctatc 2460
   acattgttta gctttagcat tcactccaac atgtgtgatt ggtcttaatg cacaattgaa 2520
   gatgcccaaa ttagacttca aatccaatgc caaacctagt ctatatgctt atccagctcc 2580
   aatggaagaa aagaaaagag aagaaagaga aaaggtaact actgccgttt taagtattgc 2640
   tgcccgccaa cgtagaagag accatgaaag aaagcatcgt gacgaaaaga tggaagtaga 2700
   tgaagacaaa gatgacaaga aaacagaaaa agaagacaag gagaagaagt ctgaagagaa 2760
   agataaaaag gatgaaaaga aagctgatga caaagataaa gataaaaaag cagatgacaa 2820
   gaaagcatct acttctactg gcgatgataa gaagaaggat gaaaaggaag aaaagaagaa 2880
   agaacctgaa ccaaacttcg aagttttaca gaatcctgct agagttatga ggcaacaatt 2940
   aaaggtgatt acacttaatg atagcaatca acatgtttcg atgaaagatg tttctattgg 3000
   aggaataatc atggtgagaa atttgaagcc tggagatgag gaactggttg aacctgttgc 3060
   agcttttggt cccaagggcg aggatgaaaa ggaaccagaa cctccagagc cgtttgaatg 3120
   gacagaagat tagatatcat cattaacatt tttataattt ttcatttaat ttactataaa 3180
   tagatgtaat tatacgaaaa acgacttttg ttaacattta atattaaatt attagtttag 3240
   cg 3242
<210> 229
   <211> 701
   <212> DNA
   <213> Diabrotica virgifera
<220>
   <221> misc_feature
   <222> (1)..(701)
   <223> RNAi target sequence
<400> 229
   agctcgcttt tatatgagat ccgcaacgtt cataggcatt tcatctatct ttgtattata      60
   gaactgttcg atatccttaa gcgtgcgttt atcatcgtcc gttacgaaat ttattgctac     120
   acccttacga ccaaatcgac cacctcgacc aatcctgtga atgtaatttt ctctattaga     180
   aggcaaatca taattaataa ctagggaaac ttgctgtaca tctatacctc tcgctaacag     240
   atcagtggtt attaaaaccc tagatgatcc tgttctgaac tgacgcataa taacgtcacg     300
   ctctctttgt tccatatcac catgcatagc ggaaacagtg aagtctctct tgtgcatgtt     360
   ttctgttaac caatcgacct tgcgacgggt attgcaaaag attacagcct gcgtaatcga     420
   aagagtatca tagagatcac ataatgtctc taatttccaa tcttcttttt ctacatatac     480
   ataaaattgt ttaatacctt ctaaggtaag ttcctctttt ttgacaagta tccgaacagg     540
   actcctcata aaggattttg tcacgtctag cacatcgttt ggcatggtag ccgaaagtaa     600
   aattacctgt acgtcagtgt tcaacatttt gaacacgtca tgaatttgat ctttaaaacc     660
   ccttgacaac atttcatcgg cttcatccaa aacaaacatt t                         701
<210> 230
   <211> 27
   <212> DNA
   <213> Artificial Sequence
<220>
   <223> primer
<220>
   <221> misc_feature
   <222> (1)..(27)
   <223> forward primer
<400> 230
   agctcgcttt tatatgagat ccgcaac 27
<210> 231
   <211> 28
   <212> DNA
   <213> Artificial Sequence
<220>
   <223> primer
<220>
   <221> misc_feature
   <222> (1)..(28)
   <223> reverse primer
<400> 231
   aaatgtttgt tttggatgaa gccgatga 28
<210> 232
   <211> 2338
   <212> DNA
   <213> Diabrotica virgifera
<220>
   <221> misc_feature
   <222> (1)..(2338)
   <223> WCRW transcript ta03651.003_diavv
<400> 232
   aaacgaccac agacagactc actttcaggt aaaaaaatcc taacttattt gttaccacaa 60
   gttagaagtg gttaaaagca tcctagaagc cactaagtga attgcaatac ctgggcggag 120
   cgacagtatt cctatcactt caaacttcaa ttcagggggc ttttccgttt agctatacgc      180
   gaagtagaca tgtctatgcc agatcgtcgt gaagagtggg acaagaatgg cccagctaat      240
   gaagacagcc agccccaagg ctatgacggc ccaccaggta tggagcctga tggcattatt      300
   gaatccaact gggaagaggt ggtcgataat ttcgacgaca tgaacctaaa agaggaactt      360
   ttaagaggaa tttatgccta tggttttgaa aaaccttctg ctatccaaca gagagccatc      420
   attccttgtg taaagggaca cgatgttatt gctcaggctc agtcaggtac tggtaagact      480
   gcaacattct ccatctctat tttacaacaa attgacacgt cagtgaggga gtgccaagct      540
   cttatattgg caccaactcg tgaattggct caacagatcc aaaaagtagt aattgcccta      600
   ggagatttca tgtcagcgca gtgtcatgca tgtattggtg gtaccaatgt acgagaagac      660
   ataagaaaac ttgaaactgg cgtacatgtt gttgttggta cacctggtag agtttacgat      720
   atgataacga ggagagccct aaggccgact catattaaaa tgtttgtttt ggatgaggct      780
   gatgaaatgt tgtcaagggg ttttaaagat caaattcatg acgtgttcaa aatgttgaac      840
   actgacgtac aggtaatttt actttcggct accatgccaa acgatgtgct agacgtgaca      900
   aaatccttta tgaggagtcc tgttcggata cttgtcaaaa aagaggaact taccttagaa      960
   ggtattaaac aattttatgt atatgtagaa aaagaagatt ggaaattaga gacattatgt     1020
   gatctctatg atactctttc gattacgcag gctgtaatct tttgcaatac ccgtcgcaag     1080
   gtcgattggt tgacagaaaa catgcacaag agagacttca ctgtttccgc tatgcatggt     1140
   gatatggaac aaagagagcg tgacgttatt atgcgtcagt tcagaacagg atcatctagg     1200
   gttttaataa ccactgatct gttagcgaga ggtatagatg tacaacaagt ttccctagtt     1260
   attaattatg atttgccttc taatagagaa aattacattc acaggattgg tcgaggtggt     1320
   cgatttggtc gtaagggtgt agcaataaat ttcgtaacgg acgatgataa acgcacgctt     1380
   aaggatatcg aacagttcta taatacaaag atagatgaaa tgcctatgaa cgttgcggat     1440
   ctcatataaa agcgagctaa taaagagtgt tggtgtgttc tacgacgtct cctgaagtat     1500
   ctttggtgtg agtattagct ttctatgcta aatctgctgc gacatatata tgcgaatatt     1560
   gtgcgctgcg ccccatcacg tttctttagg acaacgccgt tcgaggcaca atcatccacg     1620
   aactctctga gtgtcaattt ttaaatatat ttaattgttt cggtttaatc tctagtgtat     1680
   ttttaatttt ttgatataca tatagttagt gtttattatc attcgggaga cgtgtggctg     1740
   tatggaggcc cgaggtcccc tcgtttcgag gggtaccgac ggtcgtaggc gtgttgtgat     1800
   ttgctccaca cagttcgttc gtctctgtaa gggtttagtt ggtatctgga aaggttatta     1860
   taatcgtaaa agatgtagaa ggaaattctg gattatcaca tagacttttt gtacattaag     1920
   acttaataaa agtggtgata aaattcgttg ccttttcttt atgttttatt ataactggtg     1980
   tgtctaaaga tttgttgaat tgtgaatctg catatatact gtttagagag agactcacag 2040
   gtgagtaatc gtactgtttg aacagaaact ttatagtaca ttcttagttt cattctgtgt 2100
   tatatttgtt taataatcac agttaaagtt aacacaaaat gcaaaaaaca ctaacataaa 2160
   cttttgatgt tgatagtttg ttgaaattat cttccgtgtt tgttatagtt ttttttggct 2220
   ctagcttttg taaaagtaat aatcattagc gaactagtag gcctatggct tgattgacca 2280
   ttcttcatcc tttcttgttc attgctttca tcctccaatt tgtgatgttg agtttttt 2338
<210> 233
   <211> 669
   <212> DNA
   <213> Diabrotica virgifera
<220>
   <221> misc_feature
   <222> (1)..(669)
   <223> RNAi target sequence
<400> 233
   gtgaagagtg ggacaagaat ggcccagcta atgaagacag ccagccccaa ggctatgacg 60
   gcccaccagg tatggagcct gatggcatta ttgaatccaa ctgggaagag gtggtcgata 120
   atttcgacga catgaaccta aaagaggaac ttttaagagg aatttatgcc tatggttttg 180
   aaaaaccttc tgctatccaa cagagagcca tcattccttg tgtaaaggga cacgatgtta 240
   ttgctcaggc tcagtcaggt actggtaaga ctgcaacatt ctccatctct attttacaac 300
   aaattgacac gtcagtgagg gagtgccaag ctcttatatt ggcaccaact cgtgaattgg 360
   ctcaacagat ccaaaaagta gtaattgccc taggagattt catgtcagcg cagtgtcatg 420
   catgtattgg tggtaccaat gtacgagaag acataagaaa acttgaaact ggcgtacatg 480
   ttgttgttgg tacacctggt agagtttacg atatgataac gaggagagcc ctaaggccga 540
   ctcatattaa aatgtttgtt ttggatgagg ctgatgaaat gttgtcaagg ggttttaaag 600
   atcaaattca tgacgtgttt aaaatgttga acactgacgt acaggtaatt ttactttcgg 660
   ctaccatgc 669
<210> 234
   <211> 22
   <212> DNA
   <213> Artificial Sequence
<220>
   <223> primer
<220>
   <221> misc_feature
   <222> (1)..(22)
   <223> forward primer
<400> 234
   gtgaagagtg ggacaagaat gg 22
<210> 235
   <211> 22
   <212> DNA
   <213> Artificial Sequence
<220>
   <223> primer
<220>
   <221> misc_feature
   <222> (1)..(22)
   <223> reverse primer
<400> 235
   gcatggtagc cgaaagtaaa at 22
<210> 236
   <211> 1764
   <212> DNA
   <213> Diabrotica virgifera
<220>
   <221> misc_feature
   <222> (1)..(1764)
   <223> WCRW transcript 454run12_isotig05267
<400> 236
   ttttcaccac ttttattaag tcttaatgta caaaaagtct atgtgataat ccagaatttc      60
   cttctacatc ttttacgatt ataataacct ttccagatac caactaaacc cttacagaga     120
   cgaacgaact gtgtggagca aatcacaaca cgcctacgac cgtcggtacc cctcgaaacg     180
   aggggacctc gggcctccat acagccacac gtctcccgaa tgataataaa cactaactat     240
   atgtatatca aaaaattaaa aatacactag agattaaacc gaaacaatta aatatattta     300
   aaaattgaca ctcagagagt tcgtggatga ttgtgcctcg aacggcgttg tcctaaagaa     360
   acgtgatggg gcgcagcgca caatattcgc atatatatgt cgcagcagat ttagcataga     420
   aagctaatac tcacaccaaa gatacttcag gagacgtcgt agaacacacc aacactcttt     480
   attagctcgc ttttatatga gatccgcaac gttcataggc atttcatcta tctttgtatt     540
   atagaactgt tcgatatcct taagcgtgcg tttatcatcg tccgttacga aatttattgc     600
   tacaccctta cgaccaaatc gaccacctcg accaatcctg tgaatgtaat tttctctatt     660
   agaaggcaaa tcataattaa taactaggga aacttgctgt acatctatac ctctcgctaa     720
   cagatcagtg gttattaaaa ccctagatga tcctgttctg aactgacgca taataacgtc     780
   acgctctctt tgttccatat caccatgcat agcggaaaca gtgaagtctc tcttgtgcat     840
   gttttctgtt aaccaatcga ccttgcgacg ggtattgcaa aagattacag cctgcgtaat     900
   cgaaagagta tcatagagat cacataatgt ctctaatttc caatcttctt tttctacata     960
   tacataaaat tgtttaatac cttctaaggt aagttcctct tttttgacaa gtatccgaac    1020
   aggactcctc ataaaggatt ttgtcacgtc tagcacatcg tttggcatgg tagccgaaag    1080
   taaaattacc tgtacgtcag tgttcaacat tttaaacacg tcatgaattt gatctttaaa    1140
   accccttgac aacatttcat cagcctcatc caaaacaaac attttaatat gagtcggcct    1200
   tagggctctc ctcgttatca tatcgtaaac tctaccaggt gtaccaacaa caacatgtac    1260
   gccagtttca agttttctta tgtcttctcg tacattggta ccaccaatac atgcatgaca    1320
   ctgcgctgac atgaaatctc ctagggcaat tactactttt tggatctgtt gagccaattc    1380
   acgagttggt gccaatataa gagcttggca ctccctcact gacgtgtcaa tttgttgtaa    1440
   aatagagatg gagaatgttg cagtcttacc agtacctgac tgagcctgag caataacatc    1500
   gtgtcccttt acacaaggaa tgatggctct ctgttggata gcagaaggtt tttcaaaacc    1560
   ataggcataa attcctctta aaagttcctc ttttaggttc atgtcgtcga aattatcgac    1620
   cacctcttcc cagttggatt caataatgcc atcaggctcc atacctggtg ggccgtcata    1680
   gccttggggc tggctgtctt cattagctgg gccattcttg tcccactctt cacgacgatc    1740
   tggcatagac atgtctactc cgcg                                           1764
<210> 237
   <211> 628
   <212> DNA
   <213> Diabrotica virgifera
<220>
   <221> misc_feature
   <222> (1)..(628)
   <223> RNAi target sequence
<400> 237
   tcattgtgtc ctaaaggcat gatcataacg ataagtcagc agatgcaaac atccaactta      60
   gaacatgcca atttctcttt tttctagttt tgtaattttt tcttcattta tatctgggat     120
   taaaaacttt attcctttaa atgtaacgtt aatatatgaa tctcataact agtgttaccc     180
   cttagattta accaagagag aaaataacgc tctggtctac atataagtaa acattgccta     240
   atttaaattg atgtttcaga acattcgtcg cagaactcag tttctatgtt aaacaacttg     300
   gaagaactat tcatggaagg gatactggat ctaataaaca gactgcaagt aaaagttgtg     360
   ccctatcagt tgtgcgacaa ctttttcact tgaaagtcat tgaagctttt tcaggtatgt     420
   agaaatgtat atctttcttt ttaattcctt tctcttttta tacagagagg ggctaaattg     480
   tggagtgaat tgattttatc tagaatggac cactttggag agaaatcgcg aaacgggtcg     540
   atttttattt ttgaattaca atttttttgc atatgtttca tactggtgac gtcatccatc     600
   tgggcgtgat gacgtagtcg gtgatttt                                        628
<210> 238
   <211> 22
   <212> DNA
   <213> Artificial Sequence
<220>
   <223> primer
<220>
   <221> misc_feature
   <222> (1)..(22)
   <223> forward primer
<400> 238
   tcattgtgtc ctaaaggcat ga 22
<210> 239
   <211> 22
   <212> DNA
   <213> Artificial Sequence
<220>
   <223> primer
<220>
   <221> misc_feature
   <222> (1)..(22)
   <223> reverse primer
<400> 239
   aaaatcaccg actacgtcat ca 22
<210> 240
   <211> 697
   <212> DNA
   <213> Diabrotica virgifera
<220>
   <221> misc_feature
   <222> (1)..(697)
   <223> WCRW transcript ta41281.001_diavv
<400> 240
   ttaaaaaaat caccgactac gtcatcacgc ccagatggat gacgtcacca gtatgaaaca 60
   tatgcaaaaa aattgtaatt caaaaataaa aatcgacccg tttcgcgatt tctctccaaa 120
   gtggtccatt ctagataaaa tcaattcact ccacaattta gcccctctct gtataaaaag 180
   agaaaggaat taaaaagaaa gatatacatt tctacatacc tgaaaaagct tcaatgactt 240
   tcaagtgaaa aagttgtcgc acaactgata gggcacaact tttacttgca gtctgtttat 300
   tagatccagt atcccttcca tgaatagttc ttccaagttg tttaacatag aaactgagtt 360
   ctgcgacgaa tgttctgaaa catcaattta aattaggcaa tgtttactta tatgtagacc 420
   agagcgttat tttctctctt ggttaaatct aaggggtaac actagttatg agattcatat 480
   attaacgtta catttaaagg aataaagttt ttaatcccag atataaatga agaaaaaatt 540
   acaaaactag aaaaaagaga aattggcatg ttctaagttg gatgtttgca tctgctgact 600
   tatcgttatg atcatgcctt taggacacaa tgaagaaatg taactcacag tttacttctt 660
   ttatttaggt ttttggataa ggattttttt gaatccc 697
<210> 241
   <211> 610
   <212> DNA
   <213> Diabrotica virgifera
<220>
   <221> misc_feature
   <222> (1)..(610)
   <223> RNAi target sequence
<220>
   <221> misc_feature
   <222> (560)..(560)
   <223> n can be any nucleotide
<400> 241
   ccttcaccaa tttcccctta ctcgggggta aatacaacag cggacagtac acttacaaaa 60
   tctaccattt ggccagcaaa gtaccggcgt tcataaggct cctggcaccg aaggggagtt 120
   tggagataca cgaagaggct tggaatgctt acccatattg taggaccgtc attacgaatc 180
   ctggttatac gaaggagaat tttgttattt gtattgaaag tatgcatatt gctgataatg 240
   gtcatcaggt taacgttcat gacttaccca gtgacaaatt gaaacaaaga gaagtggtac 300
   acatcgacat cgctaatgat agtgtatcgt cgggagatta caaagatgac gaagatccta 360
   cgaagtttaa gtctcaaaag acgggcaggg gaccgctcgt gggtcctgat tggaaggaca 420
   aagtcaatcc tgtgatgacg tgttataaat tagtaactgt agaattcaaa tggtttggtt 480
   tacagacgag gatagaaagt tttatacaaa aatctgaaag gcggctattt acaaatttcc 540
   atagacaagt tttctgttgn atggaccgct ggcacggtct gacgatggaa gacatccgtg 600
   caatagaaga 610
<210> 242
   <211> 22
   <212> DNA
   <213> Artificial Sequence
<220>
   <223> primer
<220>
   <221> misc_feature
   <222> (1)..(22)
   <223> forward primer
<400> 242
   ccttcaccaa tttcccctta ct 22
<210> 243
   <211> 23
   <212> DNA
   <213> Artificial Sequence
<220>
   <223> primer
<220>
   <221> misc_feature
   <222> (1)..(23)
   <223> reverse primer
<400> 243
   tcttctattg cacggatgtc ttc 23
<210> 244
   <211> 800
   <212> DNA
   <213> Diabrotica virgifera
<220>
   <221> misc_feature
   <222> (1)..(800)
   <223> WCRW transcript ta20868..001_diavv
<220>
   <221> misc_feature
   <222> (715)..(715)
   <223> n can be any nucleotide
<400> 244
   aattgcagca aagaaaacat gctgattaaa gaataccgtg tgaccctacc cctcacagtc        60
   gaagagtacc aagtggctca actattcagc gtggcagagg cttccaaaga caacactggc       120
   ggcggagagg ggatagaagt attgaaaaat gaacccttca ccaatttccc cttactcggg       180
   ggtaaataca acagcggaca gtacacttac aaaatctacc atttggccag caaagtaccg       240
   gcgttcataa ggctcctggc accgaagggg agtttggaga tacacgaaga ggcttggaat       300
   gcttacccat attgtaggac cgtcattacg aatcctggtt atacgaagga gaattttgtt       360
   atttgtattg aaagtatgca tattgctgat aatggtcatc aggataacgt tcatgactta       420
   cccagtgaca aattgaaaca aagagaagtg gtacacatcg acatcgctaa tgatagtgta       480
   tcgtcgggag attacaaaga tgacgaagat cctacgaagt ttaagtctca aaagacgggc       540
   agaggaccgc tcgtgggtcc tgattggaaa ggacaaagtc aatcctgtga tgacgtgtta       600
   taaattagta actgtagaat tcaaatggtt tggtttacag acgaggatag aaagttttat       660
   acaaaaatct gaaaggcggc tatttacaaa tttccataga caagttttct gttgnatgga       720
   ccgctggcac ggtctgacga tggaagacat ccgtgcaata gaagataaaa caaaagaaga       780
   actagaagct ctccgtcaaa                                                   800
<210> 245
   <211> 524
   <212> DNA
   <213> Diabrotica virgifera
<220>
   <221> misc_feature
   <222> (1)..(524)
   <223> RNAi target sequence
<400> 245
   ggttgccgtc gtttagtaga agtgtcattt ttgtctgctc tcgttttcat cttttgtgta      60
   taaaattggt gctaaggtag ataaactata ttcgaatagg cgtttttatg tcgcaagaaa     120
   gtgttttgta tatttgtgat acgtgatctc gtagagggag tccagaaatt cataatttat     180
   agttaagtaa aaatggcggc aaacagaact ggacctgctc agagaccaaa tggcgctacc     240
   caaggaaaga tatgtcagtt caaactggtc ctactaggcg aaagtgccgt cggtaagtcg     300
   agtttggtac tgaggttcgt caaaggacag ttccacgaat accaggagag taccatagga     360
   gcagctttcc ttacacaaac catatgcctc gacgatacaa ctgttaaatt tgaaatttgg     420
   gacacagcgg gtcaagaaag gtaccacagt ttagctccta tgtactatag gggcgcacag     480
   gcagctatag tcgtctacga cataaccaat caagacacat tcgg                      524
<210> 246
   <211> 22
   <212> DNA
   <213> Artificial Sequence
<220>
   <223> primer
<220>
   <221> misc_feature
   <222> (1)..(22)
   <223> forward primer
<400> 246
   ggttgccgtc gtttagtaga ag 22
<210> 247
   <211> 22
   <212> DNA
   <213> Artificial Sequence
<220>
   <223> primer
<220>
   <221> misc_feature
   <222> (1)..(22)
   <223> reverse primer
<400> 247
   ccgaatgtgt cttgattggt ta 22
<210> 248
   <211> 3182
   <212> DNA
   <213> Diabrotica virgifera
<220>
   <221> misc_feature
   <222> (1)..(3182)
   <223> WCRW transcript ta01834.005_diavv
<400> 248
   ctcaaatatt ggcagccttg ttgacgtcgt ttagtagaag tgtcattttt gtctgctctc      60
   gttttcatct tttgtgtata aaattggtgc taaggtagat aaactatatt cgaataggcg     120
   tttttatgtc gcaagaaagt gttttgtata tttgtgatac gtgatctcgt agagggagtc     180
   cagaaattca taatttatag ttaagtaaaa aatggcggca aacagaactg gacctgctca     240
   gagaccaaat ggcgctaccc aaggaaagat atgtcagttc aaactggtcc tactaggcga     300
   aagtgccgtc ggtaagtcga gtttggtact gaggttcgtc aaaggacagt tccacgaata     360
   ccaggagagt accataggag cagctttcct tacacaaacc atatgcctcg acgatacaac     420
   tgttaaattt gaaatttggg acacagcggg tcaagaaagg taccacagtt tagctcctat     480
   gtactatagg ggcgcacagg cagctatagt cgtctacgac ataaccaatc aagacacatt     540
   cggcagggcg aaaacgtggg tgaaggaact tcaaaggcag gccagtccga cgatcgtgat     600
   agctttggcc ggcaacaagc aagatttggc caacaaacgt atggtagaat acgaagaggc     660
   gcagacgtat gctgacgaaa acggcttact ttttatggaa acttccgcaa agacggcaat     720
   gaacgtcaac gatatatttt tagcaatagc taagaaactg cccaagaatg aacaaaccac     780
   aggtcaaggc ggcagtgccc aaggcaggcg gctagcggag ggcgattcgg gcgccaaggc     840
   acccggaaat tgttgcaagt gatggtatac gcctgcaggt cgagtgttgt tattaaaccg     900
   tcacgagaaa ggactggcaa gtgcagcggc actctagtga tatctatgtg taataaaggt     960
   ccttctatta acaaaaaaaa attaataaaa aaatatatta aacttcatat acactgtcac    1020
   atattccatt tagatgatga aaaacaaaag agcagaagca ttttggtctc taacggtcat    1080
   gttgagttgg aatgtctcgt ggggcattta aatttgtgat aattgtacct atattttgtt 1140
   tttttatata tataattaat atatctgaca gtgtaagtta agcgtaaact gtttatatcg 1200
   atcgtattag cagcaccaat taaaaaaata aataaaattg aagatctttt ttattgtttt 1260
   tgtaatttta actctttggg gaaggttaca aaagaattga taacgttgtg ttgaaaacat 1320
   tgcattataa taaagtaatt ccacccactt acgaattatt ttgaaaggag aatcaaaaat 1380
   gtgtcaattg tcacctgtca tcttatgatg acactctatg agagtggggg caattgcagc 1440
   gcgcgcatac tattcagtct agcttcatta tgtcaattaa atatcgatgt gcattagaaa 1500
   tactgcctaa ttgaatattt tcgacatatg attaaaattt catcgattag tttgacagtt 1560
   cccaaatgaa aaaacagtta cttccgcata aaaatgtatt gcttcaaaac ttactttcag 1620
   tggcatttca gtgggtttta gccatcttat ggagagtggt ctaacacact ttctaggtac 1680
   agctgtggtt tttaaaagga aatataatga atcgcttttg ttttcgaaat ttacattggg 1740
   aatcccttag tgtccttata tttgccctac agagtttcat ttaaactgaa attgggtaat 1800
   ttacatagac ctaaagcttt cacgtctata tagcctaatt agaccacaaa attgacttgc 1860
   tttacgtgaa atgtatgaac cattcaaaca tatttatatg acaacaatat aatataatac 1920
   aatataatat aaataactga caacatttaa aattagaatt tttacccaca atttttctct 1980
   gccatgtgtc atctaataca agaagtatgg caatttagcc ctactcctct tattgtacat 2040
   ttcttgtaat tcctcatttt ctttttcaat tacttcgatc ttttaaactt aattcaccaa 2100
   gtttatttgc aatattgttc ttgaagtcta tagtttgaat tccttcaata tatttgtaat 2160
   gtttttcttc agtgtcaatc cttgcattcc atgaaagaac acgaaaaaga tgcagcactt 2220
   tatttgtcca tattcctgct acatagttat ttttcgatta acacattcac tgccagcgta 2280
   tagtaaactg gcactttact atacgctaaa caaaaatttg gccttgcaga ttttcctaaa 2340
   acacgtagca gctacgccac tgaatggact taggaccccc tatcgtttcg ctggttgcaa 2400
   tagtccggta actagagaca aaagtgggtc attttgcaca cttcatataa ttctcgtttt 2460
   ctgtacttca cgagtatttt ttttgtcgga tatgtattga atagtatcgg tgacaacaca 2520
   ctgccctgcg tgacacccct ttcaaaacct atgtgggaac aatatgtatc tttagagaac 2580
   tttttgtacc cttcccctaa aaactaaata ttttttcact tagtgcaatt ttaatattag 2640
   tctcacctta tttctagaat acggtcgatc taaatgtata atctcaactt tcagcttata 2700
   cttcgaaaac cgagaagtat aaactgtaag aatatttaac gttatatgta ttttattagg 2760
   ataatgattg ttgaaagtat gtttcccaac acgtgtaagt tacatttcgt actctttcca 2820
   tttttattac aaaaaaaaat gtttatttag ataaactgtt gcggaacata ctttctcgat 2880
   gcatgactga aaagaacgtt aacatttata cgtatttgtt tttattttgt gtctgatgtg 2940
   acaacccaga tcttagcgaa agcgtagagg acaaaagatg agtttttaag tttcccattg 3000
   aactttttcc gtccttaaat caaatacaac aactattcac ttaagttttc taaacatttt 3060
   tgctaatatg ggcccctgca ccattcccga cttaacagtc ttcatttgtt tttattaggt 3120
   ttgttcgtag tacgatccaa acgatcggca accgttgcca accattagac gcatgcgcgt 3180
   tc 3182
<210> 249
   <211> 529
   <212> DNA
   <213> Diabrotica virgifera
<220>
   <221> misc_feature
   <222> (1)..(529)
   <223> RNAi target sequence
<400> 249
   atttttgagg ttgtcggcta gaagttcgca aagtaaatct aaattccaac aattaaaccg 60
   aaatagttct ttaaaaaaga tgtttcgaaa ccaatacgat agtgatgtga cagtgtggag 120
   cccccagggc cgtctgcatc aggtagaata tgccatggaa gcagttaacc taggttcagc 180
   cacagtggga ctgaaaagta aaactcatgc tgtacttatc gctttgaaga gggcttcttc 240
   agaactgtct gcctaccaga agaaaattat caacatagac gatcacattg gtatcaccat 300
   ctcgggactt acagctgatg ctaggatcct tagtagatat atgcgaaacg aatgcttgaa 360
   ttataagtac tctttggata cttatatgcc attgaatagg cttatcagta ttttaggtaa 420
   caaaatgcag acatgtaccc aaagatacga ccgcagacca tttggtgtag gtcttctagt 480
   agctggttat gacgaatctg gttctcatat ctaccaaacg tgcccttct 529
<210> 250
   <211> 22
   <212> DNA
   <213> Artificial Sequence
<220>
   <223> primer
<220>
   <221> misc_feature
   <222> (1)..(22)
   <223> forward primer
<400> 250
   atttttgagg ttgtcggcta ga 22
<210> 251
   <211> 22
   <212> DNA
   <213> Artificial Sequence
<220>
   <223> primer
<220>
   <221> misc_feature
   <222> (1)..(22)
   <223> reverse primer
<400> 251
   agaagggcac gtttggtaga ta 22
<210> 252
   <211> 1051
   <212> DNA
   <213> Diabrotica virgifera
<220>
   <221> misc_feature
   <222> (1)..(1051)
   <223> WCRW transcript 454run3_isotig10716
<400> 252
   cgatgttctg ccgtgactgt ctcggtctgc tttagactaa cagagacagc cacggcaata      60
   ttcctttcca ataccccctc tcctgatcaa cgggtggctt taatgcttta tctaatcctt     120
   ctattctgtg gtatccatgg cagaagtttc cacagctggg actgcatccc tgggctcgtc     180
   ccccgcatca tctctcaacg gatctgcagg aaggtcctca attgcagcag ctgcagctcc     240
   tcctcctcta cgttcttcac cctcaataag ggacaagtag ggggaagttt cttcttcttc     300
   gaggatttta aaggtctgac ctttgccagt gtatcctatg gagacatttt ttgttgtgag     360
   gtcaacctct ggaggtagag tatctctaag agccctcaat ccatgtttga taagttcttc     420
   taaagaacta gatggcaatt catccagatg tttttctaag tatgtcctgg cactctggga     480
   tcgagctcca atggacatag ctttgcaatc gtagaaatta gcagaagggc acgtttggta     540
   gatatgagaa ccagattcgt cataaccagc tactagaaga cctacaccaa atggtctgcg     600
   gtcgtatctt tgggtacatg tctgcatttt gttacctaaa atactgataa gcctattcaa     660
   tggcatataa gtatccaaag agtacttata attcaagcat tcgtttcgca tatatctact     720
   aaggatccta gcatcagctg taagtcccga gatggtgata ccaatgtgat cgtctatgtt     780
   gataattttc ttctggtagg cagacagttc tgaagaagcc ctcttcaaag cgataagtac     840
   agcatgagtt ttacttttca gtcccactgt ggctgaacct aggttaactg cttccatggc     900
   atattctacc tgatgcagac ggccctgggg gctccacact gtcacatcac tatcgtattg     960
   gtttcgaaac atctttttta aagaactatt tcggtttaat tgttggaatt tagatttact     1020
   ttgcgaactt ctagccgaca acctcaaaaa t                                   1051
<210> 253
   <211> 533
   <212> DNA
   <213> Diabrotica virgifera
<220>
   <221> misc_feature
   <222> (1)..(533)
   <223> RNAi target sequence
<400> 253
   aggcataacg gtcaaagagg tagaggttat tataataata atcgggattc caggtatggc    60
   gatagagact atcgaggcag aggcagaccg tacgggttcc aaaatagagg taatcgaaga   120
   caacagcgcg gtagatatgg ttctagagga gggggaggat acagagatta tagggatcgc   180
   agatattcaa gaagcagatc ccgcgaccgc gatcacagca ggtcatacag tcagagcaac   240
   tcagacgcct ctcctccatc gaaacgagac aggtcttctc ctccatccga gaaagaaaag   300
   atcaacaagt attctgacgg agacgcgccc atccagcatg aaggaccgct ttctgagggc   360
   gaggacaggg acgattacat cagcaccgat accagcaagt tgatcgatag ggaaatcggt   420
   ggtaaatggg ctgataaaga agctgacact agtaaggagg aagccaaaga gagtgggcaa   480
   gggaatactg tggaagtaaa ggagtaaaga ggtggacttt cttttaagtc gtt          533
<210> 254
   <211> 22
   <212> DNA
   <213> Artificial Sequence
<220>
   <223> primer
<220>
   <221> misc_feature
   <222> (1)..(22)
   <223> forward primer
<400> 254
   aggcataacg gtcaaagagg ta 22
<210> 255
   <211> 24
   <212> DNA
   <213> Artificial Sequence
<220>
   <223> primer
<220>
   <221> misc_feature
   <222> (1)..(24)
   <223> reverse primer
<400> 255
   aacgacttaa aagaaagtcc acct 24
<210> 256
   <211> 3832
   <212> DNA
   <213> Diabrotica virgifera
<220>
   <221> misc_feature
   <222> (1)..(3832)
   <223> WCRW transcript ta01498.002_diavv
<400> 256
<210> 257
   <211> 583
   <212> DNA
   <213> Diabrotica virgifera
<220>
   <221> misc_feature
   <222> (1)..(583)
   <223> RNAi target sequence
<400> 257
   tgggccagtt aagttcgtat tccacaacaa aagaatggac ccgtggaaga aaagcacttt 60
   tgggaagctt agggcttatt gctggtggta ctggtgcctt gctgtttgct ttggaccaat 120
   cagttaaagc atctgatctt gaattgcatc ctccaaagaa tgtttggagt cattctggaa 180
   ctttcaattc cttggatcat gctagtgtaa gaagaggtta tgaagtatac aaacaagtat 240
   gtgctgcctg ccactcaatg cgcttcattg cttaccgtaa cttaattgga gtttcccaca 300
   ctgaagcaga agccaaagct gaagctgaag aacaaatgat tgaagatggt cccgatgaca 360
   cgggtaactt tttcaaaaga ccaggaaagt tatctgacta cttccccagt ccatacccca 420
   atgaagaagc agctcgtgct gccaacaatg gttcctaccc tccagatttg agctatattg 480
   tatctgccag acatggaggc gaagattaca tatttgccct tttgacgggt tactgcgacg 540
   cgccagcggg cgtggtcctc agagacggac aatacttcaa ccc 583
<210> 258
   <211> 22
   <212> DNA
   <213> Artificial Sequence
<220>
   <223> primer
<220>
   <221> misc_feature
   <222> (1)..(22)
   <223> forward primer
<400> 258
   tgggccagtt aagttcgtat tc 22
<210> 259
   <211> 22
   <212> DNA
   <213> Artificial Sequence
<220>
   <223> primer
<220>
   <221> misc_feature
   <222> (1)..(22)
   <223> reverse primer
<400> 259
   gggttgaagt attgtccgtc tc 22
<210> 260
   <211> 1332
   <212> DNA
   <213> Diabrotica virgifera
<220>
   <221> misc_feature
   <222> (1)..(1332)
   <223> WCRW transcript ta01835.001_diavv
<400> 260
   ggggggtacg tgctaggtgc agactggtat tacgtaaagt gtcaaaaaat tgtatatttt 60
   ttcttagtag cttcctaaaa gtgcttaaat tctatcaaac tagctttcta tcacctaaat 120
   acttcatgtt actctaatct aagggtacga ggggtacaaa ataaatttca cataacctat 180
   ctgcatagtt tttgagggga ccctttaggc aacatggctg caacaatagg acgaatttgt 240
   ggagctggtc ttttaaaacc aacaaactta tcctctgtgg gccagttaag ttcgtattcc 300
   acaacaaaag aatggacccg tggaagaaaa gcacttttgg gaagcttagg gcttattgct 360
   ggtggtactg gtgccttgct gtttgctttg gaccaatcag ttaaagcatc tgatcttgaa 420
   ttgcatcctc caaagaatgt ttggagtcat tctggaactt tcaattcctt ggatcatgct 480
   agtgtaagaa gaggttatga agtatacaaa caagtgtgtg ctgcctgcca ctcaatgcgc 540
   ttcattgctt accgtaactt aattggagtt tcccacactg aagcagaagc caaagctgaa 600
   gctgaagaac aaatgattga agatggtccc gatgacacgg gtaacttttt caaaagacca 660
   ggaaagttat ctgactactt ccccagtcca taccccaatg aagaagcagc tcgtgctgcc 720
   aacaatggtt cctaccctcc agatttgagc tatattgtat ctgccagaca tggaggcgaa 780
   gattacatat ttgccctttt gacgggttac tgcgacgcgc cagcgggcgt ggtcctcaga 840
   gacggacaat acttcaaccc ctatttcccc ggtggtgcca tttcgatggc acaagcccta 900
   tataatgaaa ccatagaata cagtgacgga acaccagcaa ctgccagtca actcgccaaa 960
   gacgtcgctg tgttcctaaa atggacgtca gagccagagc atgatgacag aaagaaaatg 1020
   ttaatcaaag tgatcggcat cttttctctt cttattggta tcacttacta tgctaagaaa 1080
   ctcaaattct cttcactgaa atcgagaaag attgaattta ggcccaagag taaataaata 1140
   agtattgtag atgaatttta aacaaggttt agcgtgacta ccatagttat tattatctta 1200
   aggtctaatt ttattgtaca tatttacaga ctttccaact gactggggtt tgcatttgcc 1260
   accttggttt atttattttt tccaggcttt attattattg tgtcacgtta tgtttcaata 1320
   aaaaaaaata at 1332
<210> 261
   <211> 546
   <212> DNA
   <213> Diabrotica virgifera
<220>
   <221> misc_feature
   <222> (1)..(546)
   <223> RNAi target sequence
<400> 261
   gatcccctgc acttggatta tgttattgct gcagctaatt tgaaggctga agtttatggt 60
   ataccaacta gtagaaatag agaacaaatc gcagaagtgg ttatgcaagt caatgttcca 120
   gaattcatcc ctaaatctgg cgtaaagata gcagttacgg attctcaaat ggctatgaat 180
   ggtaataatg tggatttgga tcgtgtttcc caaattaaag aagaattacc ctcagtagca 240
   gaactaggtg gactacgtct tactccttta gaatttgaaa aggacgatga caccaatttc 300
   cacatggact ttatagttgc tgcttcaaat cttcgtgcag ctaattacaa aataccgcct 360
   gctgatagac ataaatcaaa acttattgct ggtaagatta ttcccgccat agcaacaaca 420
   acctcagtag ttgctggaat ggtttgcttg gaactctaca aactcaccag aagctataca 480
   tccctaactc cattcaaaaa taatttcatt aacctcgctc tgcctttctt cagttgctca 540
   gaacca 546
<210> 262
   <211> 21
   <212> DNA
   <213> Artificial Sequence
<220>
   <223> primer
<220>
   <221> misc_feature
   <222> (1)..(21)
   <223> forward primer
<400> 262
   gatcccctgc acttggatta t 21
<210> 263
   <211> 22
   <212> DNA
   <213> Artificial Sequence
<220>
   <223> primer
<220>
   <221> misc_feature
   <222> (1)..(22)
   <223> reverse primer
<400> 263
   tggttctgag caactgaaga aa 22
<210> 264
   <211> 4199
   <212> DNA
   <213> Diabrotica virgifera
<220>
   <221> misc_feature
   <222> (1)..(4199)
   <223> WCRW transcript ta05794.001_diavv
<400> 264
   tgagaacttg cgtggttcat ttctttcctt tgtcccatca ttctgattca ttttcagctt      60
   aatttttcca tgtagtgccg gttaattgaa gtgatatatg atttatttat gtgattacat     120
   ctaacagcag actcataaaa ttgtgtcttt ttgtgtccga tgtctagtgg tagtttagca     180
   gacaactcca tcgatcctcc agccaaaaag cggaagcttg aatcaaagat tgctaccctc     240
   gaaatggcga acaatgaagc ttcaggatcc tccaccagtg atattgatga gggactttac     300
   tcgagacaac tgtacgtttt ggggcacgaa gctatgcgcc gcatggcaaa tagtgatgtt     360
   ctaatatcag gtttaggtgg tttgggtgta gaagtagcta aaaatgtaat tcttggtggt     420
   gttaaatctg ttacccttca cgatgaggct ctttgtacta ttaaggacct gtcatcccaa     480
   ttttatttaa cagaggggga cattggtaaa aaccgcgcag aagcctgctg taagcagtta     540
   tctgaactca acacttatgt ccccacaaag tcttacacag ggccccttga tgaagctttc     600
   atcttaaagt tcagtgctgt tgtcttaacc acaagcaatc gtgaagagca actaagaatc     660
   agtgagataa cacacgaaaa caacattgca ttaattatag cagacacgaa aggccttttt     720
   gcacaagtat tttgtgattt cggaaaatcc tttactgttg tagatgtaaa cggagaacct 780
   ttagtctctg cgatgatagc agacatttcg aatgatgtcg aaagtgtagt cacttgcatt 840
   gacgatacaa ggcatggtat ggaggatgga gactatgtta ccttctcgga agttcaggga 900
   atgactgaat taaataactg tgaaccaatc aaaatcaaag tattaggacc atacaccttt 960
   agtattggcg ataccaccaa gttttctaaa tacaccagag gtggtattgt aagtcaagtt 1020
   aaaatgccga aacatctgga attcaaatcg tttaaagatt ctcttgaaaa tcctgagata 1080
   ccaattactg attattcaaa aatggatttt cctacgcagc ttcatctagc ttttaccacc 1140
   ttacataagt ttgtagaaaa gtctgcaagg ctacctaaac catggaacaa tgaagatgca 1200
   acagaatttg ttaatttagc taaatcgtta gctgcaaagg gtgattctga aggagaagtt 1260
   aatgtagacc ttttagacac atttgccaag gtatcggctg gagatttaaa tcctataaat 1320
   gctactattg gtggtattgt agcccaagaa gtaatgaagg catgttctgg aaaattccat 1380
   cccattttcc aatggttcta ctatgatgcc atagaatgcc taccacaaga taaatcagaa 1440
   atcactgaag aactagctgc acccactggt agcagatatg atggccaagt tgctgttttt 1500
   ggaaaagaat tccaaaagaa gttagcatca ctgaaatatt ttattgttgg tgctggagct 1560
   attggttgtg aattgttgaa gaactttgct atgatgggtg taggaacaca aggagaagtt 1620
   acagtaactg atatggattt aattgaaaaa tctaatttga acaggcagtt ccttttcaga 1680
   ccacataacg tccaacaacc aaaatctgcc acagctgcaa aggtcattaa aaagatgaat 1740
   cctgatatca atgttgttgc acatgaaaat cgtgttggac ctgaaactga aagtgtctac 1800
   aatgacgatt tctttgaaaa gcttgatgga gttgctaatg cactggataa cgtagatgct 1860
   agaatttata tggatagaag atgtgtctac taccgcaagc ctcttctaga gtctggaacc 1920
   ctaggaacta agggaaatac tcaagtggtg gcacctttcc tctctgaatc atacagttct 1980
   tctcaagatc ctccagagaa aagcattcca atctgtactt taaagaattt ccctaatgct 2040
   atcgaacata ctctacaatg ggcaagagat atgtttgaag gattgtttaa acaaaatgcc 2100
   gaaaatgctt cgttgtacat aacagaccct gatttcctcg ataggactgt taagctacct 2160
   ggtgtacaac ctttagaggt attggaatcc gtaaaggctg ccttaattga tgagagacct 2220
   aaagatttaa atgattgcat cgcttgggca cgtaaccatt ggcaagaaca gtattccaac 2280
   caaattaggc agttgttgtt caactttcct cctgatcaat taaccagtac ggggcagttg 2340
   ttctggtcgg gaccgaagag atgcccagaa gcgcttactt ttgacgtaaa cgatcccctg 2400
   cacttggatt atgttattgc tgcagctaat ttgaaggctg aagtttatgg tataccaact 2460
   agtagaaata gagaacaaat cgcagaagtg gttatgcaag tcaatgttcc agaattcatc 2520
   cctaaatctg gcgtaaagat agcagttacg gattctcaaa tggctatgaa tggtaataat 2580
   gtggatttgg atcgtgtttc ccaaattaaa gaagaattac cctcagtagc agaactaggt 2640
   ggactacgtc ttactccttt agaatttgaa aaggacgatg acaccaattt ccacatggac 2700
   tttatagttg ctgcttcaaa tcttcgtgca gctaattaca aaataccgcc tgctgataga 2760
   cataaatcaa aacttattgc tggtaagatt attcccgcca tagcaacaac aacctcagta 2820
   gttgctggaa tggtttgctt ggaactctac aaactcacca gaagctatac atccctaact 2880
   ccattcaaaa ataatttcat taacctcgct ctgcctttct tcagttgctc agaaccaatc 2940
   gcagcccaac aaatggaata ctgtggtaac aaatggacac tctgggaccg cttcgacgtt 3000
   cagggagaga tgacacttag tgaatttttg gaatacttca aggaaaaaca cggtcttgaa 3060
   attacgatgt tgtcccaggg cgtctgcatg ctgtattcgt tcttcatggc aaaagctaaa 3120
   gctcaagaac gtttcggtct ttcgatgtca gaagtcgtca gacgggtgtc aaagaagaaa 3180
   atcgaacctc acgtaaaagc attggtattc gaactgtgtt gcaacgattc cactggtgaa 3240
   gacgtggaag ttccatatgt taaatacaat ttatgttgag ccgactcagt tcaaaactca 3300
   acttaaaaga cttaagagtt ttgttaggtc agtttctgga ttaagagtac gggaaaacta 3360
   gccacgtttt actatttaca cattgtgaga ttgctgacca atagttggaa tacatattag 3420
   cagcaagagc ttgtgtcttt tattatttat tatttatcag ctgtttttta atatttgcag 3480
   acctttaaat attttttata acttttaaat gtcctttttg tattaacgtc atggcaacca 3540
   ctgttagtat ttaacccttc cagctccgct gtaatatgtc cagcatcatg acgcacttac 3600
   gcgtcaaata cactggccaa gtcatctttg gacgcgcgta tgcgtcaccg tagctgaaag 3660
   agttaaatct ctgtaatttc tccaattttc gtttacccaa aaactgatct atactgaatt 3720
   tgggtacttg actggaaagt gtgatctaaa ttattcttct caaagaaatt gtttgctgtc 3780
   attttgagat aggttaatga aattaataac ggaaaggatt agcatttaaa cacgttgatg 3840
   caatatactt ataataactt gtaggagtat gtttattttt atgaaaatag tagttttcag 3900
   gtaacagtta ttattttctt aagaaatttt catgtgtgag aataaaataa atatttacaa 3960
   aaagttcctt gttttcctta tatacctaca atcgtcgacg actgtagctc aacaacaata 4020
   tactaagtcg attctatgct ccaacaacat tttcattttt aaaaatgata aaacaccaaa 4080
   agtacacatt tgttctcatc ttactaaggc acgtcgaata atatatcgct tatactattt 4140
   cagtgactta aaacgtaaga acagtaaacg taacagtaag aaattttcat gtgtgagaa 4199
<210> 265
   <211> 644
   <212> DNA
   <213> Diabrotica virgifera
<220>
   <221> misc_feature
   <222> (1)..(644)
   <223> RNAi target sequence
<400> 265
   gaatttgatt tggcattttg gtatgtagaa ataaaattat cttgtcaata aataatcaga 60
   aatattgaaa gtagaatcgt tctaaataat tagaaattga aagtagaaca gtttgttcgt 120
   tttactaggt agtaaagagt taaatagaca acaggacgct gttaaaaggt aagctattgc 180
   cgccatactt ttcctgactg aatttattaa ttttttgttt acctaaaaac attattgttt 240
   ttaaacacat tttgtttttg ataacatatt taatctaata ctagaaaagc caaaaattac 300
   aaaataaggt aaagaatcta aatggtttat tactattagg gcccccaggc attaggcagt 360
   ctatgtacat gtaacagcca gagccaattc gtgggcaagt tgtatccttc aagggatgta 420
   gaagtgcaag aggtcataat tatttactat ttctctcgta gaaaactcaa catataagca 480
   tcgaattcaa attcagcctc ttcttttata acatcacttg aaaggcattt caattatatt 540
   ttggaagcat gtttgtaggt tcccagatat acaaaaatgt cagttcaatc gtttgataat 600
   cttctgtata aattaattca atcaaggcca ctgatactcc tatg 644
<210> 266
   <211> 22
   <212> DNA
   <213> Artificial Sequence
<220>
   <223> primer
<220>
   <221> misc_feature
   <222> (1)..(22)
   <223> forward primer
<400> 266
   gaatttgatt tggcattttg gt 22
<210> 267
   <211> 22
   <212> DNA
   <213> Artificial Sequence
<220>
   <223> primer
<220>
   <221> misc_feature
   <222> (1)..(22)
   <223> reverse primer
<400> 267
   cataggagta tcagtggcct tg 22
<210> 268
   <211> 712
   <212> DNA
   <213> Diabrotica virgifera
<220>
   <221> misc_feature
   <222> (1)..(712)
   <223> WCRW transcript ta41459.001_diavv
<400> 268
   tcagccagat ctatccccaa tgtttttaaa atgatttttg atactgaaaa attaaaatta      60
   attcacatag gagtatcagt ggccttgatt gaattaattt atacagaaga ttatcaaacg     120
   attgaactga catttttgta tatctgggaa cctacaaaca tgcttccaaa atataattga     180
   aatgcctttc aagtgatgtt ataaaagaag aggctgaatt tgaattcgat gcttatatgt     240
   tgagttttct acgagagaaa tagtaaataa ttatgacctc ttgcacttct acatcccttg     300
   aaggatacaa cttgcccacg aattggctct ggctgttaca tgtacataga ctgcctaatg     360
   cctgggggcc ctaatagtaa taaaccattt agattcttta ccttattttg taatttttgg     420
   cttttctagt attagattaa atatgttatc aaaaacaaaa tgtgtttaaa aacaataatg     480
   tttttaggta aacaaaaaat taataaattc agtcaggaaa agtatggcgg caatagctta     540
   ccttttaaca gcgtcctgtt gtctatttaa ctctttacta cctagtaaaa cgaacaaact     600
   gttctacttt caatttctaa ttatttagaa cgattctact ttcaatattt ctgattattt     660
   attgacaaga taattttatt tctacatacc aaaatgccaa atcaaattcc cc             712
<210> 269
   <211> 629
   <212> DNA
   <213> Diabrotica virgifera
<220>
   <221> misc_feature
   <222> (1).. (629)
   <223> RNAi target sequence
<220>
   <221> misc_feature
   <222> (568)..(568)
   <223> n can be any nucleotide
<220>
   <221> misc_feature
   <222> (594)..(594)
   <223> n can be any nucleotide
<400> 269
   tgagaaaaac tgtcctttgc atttgcagcg ttaaagatcg gtctcagtaa taaaattatt        60
   tgtagagttt gcaatttcct gaaaatagtg acctctgata aagttcacaa cagttcaata       120
   attggtagtg gcgcatatca gtgtctttga cgaagccatg attaatatag ctggtggtgc       180
   agtgatttct tcgtttgcgg tgtatttgtt attaagatac aggactagaa tgacgtccct       240
   tttgacatgg attattggaa atattagatc tcaacaagct gtcagcagta catcacaaga       300
   agaagagaaa tctgacaaat ccagcagcaa taaacccaag caacagttta aaggtggcaa       360
   agttcgacag aggcctaaaa aacttaacga agatcccaca tttagtacaa gtttatcaac       420
   ccccattgaa gatataatag aggaaacaat caatgaagaa gcagtagcag ttaataagaa       480
   aaaaggaaaa tcatccaaaa agcaaactga agaattgcca gtgaaaaagg aaaaacccac       540
   aaaagatcta gatgatgaca taggttantc atttttcaaa gttaaaactg tttnccaaag       600
   gaaatccaga agccaagcct aagaaacaa                                         629
<210> 270
   <211> 22
   <212> DNA
   <213> Artificial Sequence
<220>
   <223> primer
<220>
   <221> misc_feature
   <222> (1)..(22)
   <223> forward primer
<400> 270
   tgagaaaaac tgtcctttgc at 22
<210> 271
   <211> 22
   <212> DNA
   <213> Artificial Sequence
<220>
   <223> primer
<220>
   <221> misc_feature
   <222> (1)..(22)
   <223> reverse primer
<400> 271
   ttgtttctta ggcttggctt ct 22
<210> 272
   <211> 1750
   <212> DNA
   <213> Diabrotica virgifera
<220>
   <221> misc_feature
   <222> (1)..(1750)
   <223> WCRW transcript ta03339.001_diavv
<220>
   <221> misc_feature
   <222> (633)..(633)
   <223> n can be any nucleotide
<400> 272
   aaaagtagcg aaggtacaac gtgtagagaa tagtcagtct gagaaaaact gtcctttgca 60
   tttgcagcgt taaagatcgg tctcagtaat aaaattattt gtagagtttg caatttcctg 120
   aaaatagtga cctctgataa agttcacaac agttcaataa ttggtagtgg cgcatatcag 180
   tgtctttgac gaagccatga ttaatatagc tggtggtgca gtgatttctt cgtttgcggt 240
   gtatttgtta ttaagataca ggactagaat gacgtccctt ttgacatgga ttattggaaa 300
   tattagatct caacaagctg tcagcagtac atcacaagaa gaagagaaat ctgacaaatc 360
   cagcagcaat aaacccaagc aacagtttaa aggtggcaaa gttcgacaga ggcctaaaaa 420
   acttaacgaa gatcccacat ttagtacaag tttatcaacc cccattgaag atataataga 480
   ggaaacaatc aatgaagaag cagtagcagt taataagaaa aaaggaaaat catccaaaaa 540
   gcaaactgaa gaattgccag tgaaaaagga aaaacccaca aaagatctag atgatgacat 600
   aggttattca tttttcaaag ttaaaactgt ttnccaaagg aaatccagaa gccaagccta 660
   agaaacaagt ttctgttgac ctaaatgaag ataacacagc agatgatata ggtttctcat 720
   ttttacctgt caaagaaaaa ggcaaaaaat ataaatctaa gagtaatcca gaaaataaag 780
   accaagaact gactttaaac gcagtggtag atcagacact gaggaaagct aaatatgata 840
   catcaaagcc gtcaagaata gaacattaca aaaagagaac aactgacaaa gctttttcca 900
   aaaaaggctg caacaaaatt tgaaagtcct tcagtatcta caatcgtaga aaagagattg 960
   ttgtatgaaa gcaaacttaa aagtgaaact aaggagagca aaaaagttac atcttctatt 1020
   tcctttgcta gcatagtcag taatgctact gacgcccctt caggttctga tattgaacaa 1080
   aattcccagt tactaaatgg acctaagcct gatattcaac agcatatctt tgagtcttta 1140
   gagtcagtaa aatctagtgc tgagtccgta gaagtaattg atgatagcaa ttctgaatat 1200
   caaaacagtc aatccaccaa ttttgagaca atcaaagaaa aattaaatac tttactcgat 1260
   gaggattctg attttaatca aactcaaata gagcaatcaa atgtatatga aatagatcaa 1320
   tcttcaaagc attcttctgt atatattgta ggaagtaacg aggtattgga agaatcatcc 1380
   tccacacaag attcttatat tggtcataca ggttttgaac aagaacctga acaaacattc 1440
   agtcagaaaa ctctagaagc ttctatagac aacagattta taacagatgc cagtatatta 1500
   gaaaaaagta catccactga agcttttctt agttttgaaa gacatatcga atcacaagat 1560
   ctaacacaca gtcttgaatc cagtaattat tatcaaaata attattcaca gttaaatttt 1620
   gacaatcaac ttcaaaactc ttcagaaagc catatttctg attacagcta ttcccaaaat 1680
   caacagtccc taagctcaag cgacttttct agagactaca attctacaga ccattccttt 1740
   acagaactag 1750
<210> 273
   <211> 700
   <212> DNA
   <213> Diabrotica virgifera
<220>
   <221> misc_feature
   <222> (1)..(700)
   <223> RNAi target sequence
<220>
   <221> misc_feature
   <222> (608)..(608)
   <223> n can be any nucleotide
<400> 273
   gagagcactt ttcgcagtta ccacagcgga ccggcttttc aacaaacaga aacaatcaga      60
   ggaccggtgt aaaagtgatg gatccttcat ttgagaatgc tgggcaagtg gatggccttg     120
   aaatatggag aattgaagac ttcaaacctg tgccttatcc taaaaatcaa tacggcaaat     180
   tctacacagg agactcatac attatactaa ataccaaaac tacaagaagt ggtgccaaag     240
   tacatgatct tcatttttgg ttaggtgctg aaactagcca ggatgaacaa ggatcagctg     300
   ccatatatac agttcagttg gacgagcatt tgaacggaga accagtccaa catcgtgaaa     360
   cccaaaacca cgaatctcaa ctgttcttat cttacttcaa aagcggtggt attcgatacc     420
   ttcctggtgg tgttagctct ggcttccacc atgtcgatcc taatgctttc gagaagaggc     480
   tgttccaagt taaaggctct agaaatatta gagttaagca agttacccca tcaatcgcct     540
   ctatgaacac tggtgactgc tttattttgg acgttggaag agatatttat gtctatgttg     600
   gtactaangg ctagaagagt tgaacaactc aagggctatt aatgctgcca accaaattcg     660
   cgaccaagat catgccggaa aggccagagt aaccattgtc                           700
<210> 274
   <211> 22
   <212> DNA
   <213> Artificial Sequence
<220>
   <223> primer
<220>
   <221> misc_feature
   <222> (1)..(22)
   <223> forward primer
<400> 274
   gagagcactt ttcgcagtta cc 22
<210> 275
   <211> 22
   <212> DNA
   <213> Artificial Sequence
<220>
   <223> primer
<220>
   <221> misc_feature
   <222> (1)..(22)
   <223> reverse primer
<400> 275
   gacaatggtt actctggcct tt 22
<210> 276
   <211> 2169
   <212> DNA
   <213> Diabrotica virgifera
<220>
   <221> misc_feature
   <222> (1)..(2169)
   <223> WCRW transcript ta00090.001_diavv
<400> 276
   gggggactag ctgtatgagc tttcgtaacg ttcggttgtg tgacagttca gtgtggaagt      60
   gataattcaa aaaaaaacgc aggcgacaaa aacaaaatag gaactacata atagagagag     120
   accgaggcga ataagatgga ccacccttaa accacccaaa ttagaacatt aaaaggtgtt     180
   tttcaaagct ctcaaattca atagctagcg catgtccaaa atgtggggaa acggagcgtt     240
   tagtcgcggc actttggtgt gtctcgcgct tttagtttat ctgaaagata gtagctgtgt     300
   gactacaaac agacgagagc acttttcgca gttaccacag cggaccggct tttcaacaaa     360
   cagaaacaat cagaggaccg gtgtaaaagt gatggatcct tcatttgaga atgctgggca     420
   agtggatggc cttgaaatat ggagaattga agacttcaaa cctgtgcctt atcctaaaaa     480
   tcaatacggc aaattctaca caggagactc atacattata ctaaatacca aaactacaag     540
   aagtggtgcc aaagtacatg atcttcattt ttggttaggt gctgaaacta gccaggatga     600
   acaaggatca gctgccatat atacagttca gttggacgag catttgaacg gagaaccagt     660
   ccaacatcgt gaaacccaaa accacgaatc tcaactgttc ttatcttact tcaaaagcgg     720
   tggtattcga taccttcctg gtggtgttag ctctggcttc caccatgtcg atcctaatgc     780
   tttcgagaag aggctgttcc aagttaaagg ctctagaaat attagagtta agcaagttac     840
   cccatcaatc gcctctatga acactggtga ctgctttatt ttggacgttg gaagagatat     900
   ttatgtctat gttggtacta aggctagaag agttgaacaa ctcaaggcta ttaatgctgc      960
   caaccaaatt cgcgaccaag atcatgccgg aaaggccaaa gtaaccattg tcgataccta     1020
   cagtccagaa agtgactata ctacattctt tgaagccttg ggtgaaggtt ccagagactc     1080
   agttcctgac tataccacca gtgacgacga tgaatctttc gaacgcacag aggaaaatca     1140
   tgcttcactt tacaaggtat cagatgctac tggctctgtt aaagtaacca aagttggaca     1200
   aaaacctttg gaagctagtt cattggaccc cagcgactgc tttattttgg ataccactga     1260
   tgccttattg tatgtatgga tcggcagcaa atgtgatgaa agagaaaaga aggaagcgat     1320
   gaataaagct gatgggttcc ttaatgcgca caatcaccct aaatggacac atgttcaaag     1380
   aattgttcaa ggagctgagc ctactgcgtt tacacaatat ttcagaaatt ggcaggcctt     1440
   tggagaaacc cacccaagat tactacgttc tgtcagcacc tcagcccacc tttaccattg     1500
   ccaaatccgt tcaagatctc gcagacttaa agtcgaagag attagggact atgagcagga     1560
   agacctctgg gaggatgaca tcatggtctt ggacgctggt gatgttgtgt ttgtttggaa     1620
   cggcaaagga gcttcagatg aagaaaaatc tagaggacct aaatacgtac ataaaatgtt     1680
   gaatcgtcaa ggtcgtgaag acgtaccagt aaaaataatc gagcaaggtg atgaaccaga     1740
   agaattcaca atacacttcc cagcttggtc agcagatcac tgggacaatt taccagatgt     1800
   aagaagctac atcaaaaatg aggaagactg aattctgtta tcatgattga ccaacgcgtc     1860
   acttgtttta aataaggctt ttaagttgaa aaatgtgcgc aaaaatatta tagctttaac     1920
   atttttaaat atgcatttat atagctgttt tagtttttta tatgccaatg ctctcggtta     1980
   aatagaaata gtttcaaaat tattgttaca acactagaaa gaacttatga attgtgccta     2040
   aaaaagattt ttaaatacta tatttaaatg atactttata tatgtactgt tgtgtaccta     2100
   tcagtgttat tttttaaaat gtatttgtta agcttgtgtg gttacaaaaa ctataataaa     2160
   aatattaat                                                             2169
<210> 277
   <211> 608
   <212> DNA
   <213> Diabrotica virgifera
<220>
   <221> misc_feature
   <222> (1)..(608)
   <223> RNAi target sequence
<220>
   <221> misc_feature
   <222> (561)..(561)
   <223> n can be any nucleotide
<220>
   <221> misc_feature
   <222> (562)..(562)
   <223> n can be any nucleotide
<400> 277
   caacctggaa ctttaactgc tgcagctgca acaccaggta aaggtaagaa gaaggaagaa        60
   aagaaacaag agaagaagcc acaaacacct aaagaaaaga aggaaaagga acagccagaa       120
   gaattagatg ctgcagaagc cgctcttgct gaagaaccca agagtaagga ccctttttct       180
   gaactaccta aaggaacctt tgttatggat gactttaaac gtgtctattc aaatgaagaa       240
   gagagcaaat caattgctta tttctgggaa aaattcgacc ctgaaaacta ttcaatctgg       300
   tatggtgaat acaaataccc tgaagagctc tccaaagtgt tcatgagctg taacctaatt       360
   actggcatgc aacagagact agataagatg agaaagcagg cctttgcatc tgtatgcctt       420
   tttggagaag acaacaacag cacaatctcc ggtatctggg tatggagagg acaagaatta       480
   gcttttacct taagtcccga ctggcaaata gattatgaat cctatgaatg gaagaagtta       540
   gatgccaaat cagaagaaac nngaaattag tagagcaata cttctcatgg acaggtactg       600
   acaagcaa                                                                608
<210> 278
   <211> 22
   <212> DNA
   <213> Artificial Sequence
<220>
   <223> primer
<220>
   <221> misc_feature
   <222> (1)..(22)
   <223> forward primer
<400> 278
   caacctggaa ctttaactgc tg 22
<210> 279
   <211> 22
   <212> DNA
   <213> Artificial Sequence
<220>
   <223> primer
<220>
   <221> misc_feature
   <222> (1)..(22)
   <223> reverse primer
<400> 279
   ttgcttgtca gtacctgtcc at 22
<210> 280
   <211> 1508
   <212> DNA
   <213> Diabrotica virgifera
<220>
   <221> misc_feature
   <222> (1)..(1508)
   <223> WCRW transcript ta20625.001_diavv
<400> 280
   ggggctcttt agctagtttt gagtgtagtg agaatggctg ccggaactct ttacacttat      60
   cccgaaaatt tccgggccgc aaaaactctg attgccgcac aatatgccaa ggcaaacgtc     120
   aaagtagcac ccaattttgt ttttggagaa actaataaaa ctaaagagtt caccaagaaa     180
   tttccaagtg gaaaggttcc tgcttttgaa tcaaacgatg gcaagtacct tcaagacagc     240
   aacgccatcg cttactatgt ttccaatgaa caactaagag gaaaaaacga atatgataga     300
   gcacaaattt tgcaatggat tggctttgct gaaggtgaaa ttgttccagc agcatgtgcc     360
   tgggtattcc ctatcttggg aattgttaaa aataacagtg gtaatgatga gactttccaa     420
   agagcaaagg aagaccttaa atcctctttg tctattctca acagccactt acttgcccac     480
   acttacttgg taggagagag aatcactcta gctgacattg tagttgcctg caacctattg     540
   aatgcctaca aatatgtact tgatcccaac tttagaaaat cttttggtaa cgtcaataga     600
   tggttcaata cacttattaa ccagccacaa ttcatagctg tacttggaga agttaaactt     660
   tgcagtgcag ttgctcaacc tggaacttta actgctgcag ctgcaacacc aggtaaaggt     720
   aagaagaagg aagaaaagaa acaagagaag aagccacaaa cacctaaaga aaagaaggaa     780
   aaggaacagc cagaagaatt agatgctgca gaagccgctc ttgctgaaga acccaagagt     840
   aaggaccctt tttctgaact acctaaagga acctttgtta tggatgactt taaacgtgtc     900
   tattcaaatg aagaagagag caaatcaatt gcttatttct gggaaaaatt cgaccctgaa     960
   aactattcaa tctggtatgg tgaatacaaa taccctgaag agctctccaa agtgttcatg    1020
   agctgtaacc taattactgg catgcaacag agactagata agatgagaaa gcaggccttt    1080
   gcatctgtat gcctttttgg agaagacaac aacagcacaa tctccggtat ctgggtatgg    1140
   agaggacaag aattagcttt taccttaagt cccgactggc aaatagatta tgaatcctat    1200
   gaatggaaga agttagatgc caaatcagaa gaaacaaaga aattagtaga gcaatacttc    1260
   tcatggacag gtactgacaa acaaggtaga aaattcaatc aaggaaaaat ctttaaatga    1320
   tctacatcta tttaataaat cttacaaaat gctgtactga tcatgtgaag caagagaaat    1380
   aaatcccaat gttttgttaa taatattttt ttattaatat gtaattgttt tgatcgaagc    1440
   ttattctttg ggctttactc cctgagttta taagacactt aaataaattt aataacaatt    1500
   taaaaaaa                                                             1508
<210> 281
   <211> 724
   <212> DNA
   <213> Diabrotica virgifera
<220>
   <221> misc_feature
   <222> (1)..(724)
   <223> RNAi target sequence
<400> 281
   gtcagtgtca tcatttgacg tgtcaagttt ggggttaaat tttgttaatt tcaagaatta      60
   aaatatttaa cataaaaatg gtttctatat taaattcaat agatactggt catgaagata     120
   tgatacatga tgcagaagta gattattacg gtttaaggtt agccacttgt tcctcagata     180
   acagcatcaa agtttatgat ataaaaagtg gagcttccac actactggat gatctaaaag     240
   gccactatgg acctgtttgg caaatagctt ggagtcatcc taagtttggc aacttattag     300
   cttcgtgttc ttatgatagg aaagtaataa tttggaaaga acagcaaaga aaatggataa     360
   aatattatga gtatgcaaat catgattcca gtgttaactc tgttcagttt gcacctgctg     420
   agctaggttt aattctggct tgtggaagta gcgatggttc aatttctctt ttaacttata     480
   atgttggaag taatatttgg gatgccaaga agattcaaaa tgcccatgca ataggatgta     540
   atgctgtaag ttgggctcca gccataaccc ctgtttttgg agatgagact acacctgtga     600
   aacgtcttgt ttctggagga tgtgacaatt tagtaaaaat atggagggaa gatggtgaca     660
   ggtggattga agagtccaaa cttgaagtac gctctgattg ggtaagagat gtagcttggg     720
   ctcc                                                                  724
<210> 282
   <211> 22
   <212> DNA
   <213> Artificial Sequence
<220>
   <223> primer
<220>
   <221> misc_feature
   <222> (1)..(22)
   <223> forward primer
<400> 282
   gtcagtgtca tcatttgacg tg 22
<210> 283
   <211> 21
   <212> DNA
   <213> Artificial Sequence
<220>
   <223> primer
<220>
   <221> misc_feature
   <222> (1)..(21)
   <223> reverse primer
<400> 283
   gagcccaagc tacatctctt a 21
<210> 284
   <211> 1151
   <212> DNA
   <213> Diabrotica virgifera
<220>
   <221> misc_feature
   <222> (1)..(1151)
   <223> WCRW transcript ta06970.001_diavv
<400> 284 ttaataaaaa t 1151
<210> 285
   <211> 501
   <212> DNA
   <213> Diabrotica virgifera
<220>
   <221> misc_feature
   <222> (1)..(501)
   <223> RNAi target sequence
<400> 285
   tgtccaataa tgtgtgttgt gaagtgtaac aaatatgaaa aagactacac ttagaatatg      60
   agttaaaatt ggttgaagat tcctttgtat ttagtttgta gttacacaac agtacagaca     120
   tggcaaggga gcaggtaacc agaaaatggg aaatatttca aggtagaaat aggttttact     180
   gcaatggaag gctgatgaca gcaccgcatt ctggggtatt tttgttgaca ctttttttta     240
   attactgtaa cgagtgtgtt attttttgta tttgactgta aatatttagc agaaaatgtg     300
   acaatagcca ttccgatcat cggagctttg ctgttcttat ttacagtgtc ttcccttttg      360
   cgtacaagct tgtctgaccc aggtattata cctagggcgt cgtcagaaga ggcagcctac     420
   gttgaaaagc aaatagaagt aacaaactct gccaacagtc ctacatatag accgccaccc     480
   aggacgaaag aagttttggt t                                               501
<210> 286
   <211> 23
   <212> DNA
   <213> Artificial Sequence
<220>
   <223> primer
<220>
   <221> misc_feature
   <222> (1)..(23)
   <223> forward primer
<400> 286
   tgtccaataa tgtgtgttgt gaa 23
<210> 287
   <211> 22
   <212> DNA
   <213> Artificial Sequence
<220>
   <223> primer
<220>
   <221> misc_feature
   <222> (1)..(22)
   <223> reverse primer
<400> 287
   aaccaaaact tctttcgtcc tg 22
<210> 288
   <211> 2197
   <212> DNA
   <213> Diabrotica virgifera
<220>
   <221> misc_feature
   <222> (1)..(2197)
   <223> WCRW transcript ta06058.001_diavv
<400> 288
   gggggtcaaa gtcaactgtc aaaattgttg agtcgtgaat gtcggataat cttcactttt      60
   taggagtgtt tcttgtttat ttgtaaaaaa cgataatatg cgtatctcta aacaagatga     120
   tatattaaat tctgtccaat aatgtgtgtt gtgaagtgta acaaatatga aaaagactac     180
   acttagaata tgagttaaaa ttggttgaag attcctttgt atttagtttg tagttacaca     240
   acagtacaga catggcaagg gagcaggtaa ccagaaaatg ggaaatattt caaggtagaa     300
   ataggtttta ctgcaatgga aggctaatga cagcaccgca ttctggggta tttttgttga     360
   cacttttttt aattactgta acgagtgtgt tattttttgt atttgactgt aaatatttag     420
   cagaaaatgt gacaatagcc attccgatca tcggagcttt gctgttctta tttacagtgt     480
   cttccctttt gcgtacaagc ttgtctgacc caggtattat acctagggcg tcgtcagaag     540
   aggcagccta cgttgaaaag caaatagaag taacaaactc tgccaacagt cctacatata     600
   gaccgccacc caggacgaaa gaagttttgg ttaaaggtca aaccattaaa cttaagtatt     660
   gcttcacttg taaaatattt aggccgccta gagcttcgca ttgtagttta tgtgataatt     720
   gtgtagatag gtttgatcat cactgtccat gggtggggaa ctgtgttggt agaagaaatt     780
   ataggttttt ttacatgttt atagtatcac tggcgttttt agcagtattt atatttgcct     840
   gtgcagttgc gcatcttatt ttaatgacga aggaagatcg agaattccta gatgccgtga     900
   aggaatcccc tcccagcgtg ctagttgcag tcatttgttt ttttagtgtt tggagtattt     960
   tgggcttggc cggattccat acgtacctta cggctagcaa ccaaaccact aatgaagata    1020
   taaaaggatc ttttacaagc aaaagaggcc aagaaaacgt caacccttat tcggagggca    1080
   acgtctgctt aaattgtttt cacattctct gtggaccggt tactccttcg ttaatcgaca    1140
   gaagaggtgt tgtcacagaa agctaccaat acgaaaacag tagacctgtt accaatgcac    1200
   caactttgcc tttgaagaaa tatggattgc ccttgcagaa tggtccccaa caagtgacac    1260
   ctgacgcagc ggaaatgcca ggcatctacc gccaaaccac cgagaacacg gaaagcaaca    1320
   cgggtagcgt aacccactta gtgtctagcg aacagcccct agccgtcgtt cccgccgcag     1380
   tgcatctaca gagaacccac ggggacttgc aacagtacca gtcgcagacc agtctgaacc     1440
   actccccggt ctacaacaac atcaccccaa cgcacgccgc tcccgcgcaa ctcagcaaat     1500
   cacaaagctg tatcgacaat ataaattcca cgtcttcatc gaaagaaaac ggcgcgaaag     1560
   acattacatt gaacatggaa gacctccatt tggatcctgt gatattgagg gagaaccacg     1620
   tactggtcaa catcaacgac aaaggacggc tcgccgattt gaggctagac aacgatatga     1680
   cagtgcaaga caaggagaac agtttgttga gtgttagtag gttgcgactg ttgcaagaca     1740
   cgacgatgat cgagagtgct ttagacttgg attcgttaga tgggtctagt ttaggtacga     1800
   atagtcaggc gggattgatg aaagtagacg tgtagttcat tcgagaactt ttagaagaga     1860
   atgtttgtca tttgtttggc aacagccaac atatagtagt attgaataca gaagaacgta     1920
   aaataactca tgatagtgca ataaaatggg atttctttgt tttcgactta ttttattggt     1980
   caaaagtttt gtttatattt ttataacaga ataaatatga ttaaataatt gatttgcact     2040
   ttaaaatgta taaaaatcat cagcagattt atctgtagca atgcaagaca cttttatagt     2100
   aatctccagc tagggaaaac ttacgcagtg catcttagta tttcagaatt aaattttaaa     2160
   tgaaaaagac ctttataaaa gatatattta ttttaaa                              2197
<210> 289
   <211> 628
   <212> DNA
   <213> Diabrotica virgifera
<220>
   <221> misc_feature
   <222> (1)..(628)
   <223> RNAi target sequence
<400> 289 atagaagaac atactcgatg gaccaaca 628
<210> 290
   <211> 22
   <212> DNA
   <213> Artificial Sequence
<220>
   <223> primer
<220>
   <221> misc_feature
   <222> (1)..(22)
   <223> forward primer
<400> 290
   aatttgtgcc tcggaattgt at 22
<210> 291
   <211> 22
   <212> DNA
   <213> Artificial Sequence
<220>
   <223> primer
<220>
   <221> misc_feature
   <222> (1)..(22)
   <223> reverse primer
<400> 291
   tgttggtcca tcgagtatgt tc 22
<210> 292
   <211> 690
   <212> DNA
   <213> Diabrotica virgifera
<220>
   <221> misc_feature
   <222> (1)..(690)
   <223> WCRW transcript ta41486.001_diavv
<400> 292
   gatcacgtca tgggcaccac aaatttgtgc ctcggaattg tattttttac gttttttagt      60
   ggaatttggg cttcagccga aaaaatacca ctgggagcaa ttttcgaaca gggcacagat     120
   gacgtccaaa cagcatttaa atttgcgatg ttgcagcaca atcaaaatat ttccagtaga     180
   aaatttgaat ttcaagcata tgttgacgtt attaatactg cggatgcatt taaattgtct     240
   agattaattt gcactcaatt ttcaagaggt gtatattcga tgttgggagc agtttctcca     300
   gattcatttg atacgctaca ttcctatagt aatacttttc aaatgccttt tgtaacgcct     360
   tggtttccag aaaaagtatt agccccgtca tcgggattta tggactatgc tatttcaatg     420
   cgacctgaat accacaaagc catcatagat acggttaagt attacggatg gaggcagatt     480
   atatatttat atgattctaa tgatggtctc ctaaggttac aacagatata ccaaggatta     540
   actcctggca gtgagatttt tcaagtcact actgtacgga gaataaacaa tgttagcgaa     600
   gcattatcat ttcttagagg aatagaagaa catactcgat ggaccaacaa atatgtggtt     660
   ctagattgct ctactgagac tgctaaagaa                                      690
<210> 293
   <211> 710
   <212> DNA
   <213> Diabrotica virgifera
<220>
   <221> misc_feature
   <222> (1)..(710)
   <223> RNAi target sequence
<400> 293
   gctctgttat gtgcgtcaag aatttagaaa ttttatatta tccgtaatgg tggtcgaccg      60
   tgtgcattct aaaactatta aataatcatg tcgctagaga ctcgatctaa ttcagctctg     120
   tttaaaagag cagaccaact aaaacgttgg gaagaatcag atacaaatcg agaaccagtg     180
   tcaccgaaac aaaaaactcg gaaaattaaa ttttcttcgg gatgtgtgtt tctcgcggct     240
   tgtgctgctg gtgataaaaa agaagtgttg gaattgttac ataaaggagc tgacatcaat     300
   actgcaaatg tggacggatt aacagcccta catcaggcat gtatcgacga caatctcgac     360
   atggtggagt tcctcgtgga gaacggggcg gacgtcaaca ggggcgacaa cgaaggttgg     420
   acccctttgc acgccactgc cagctgcggg tttctctcga tcgcgaagta cctcatcgaa     480
   aagggggccc acgtagcggc agttaataac gatggacaat tggcacttga tattgccgag     540
   agccaaaaaa tggaaggact actcagggaa gaaattgagg atagaggcat cgactgcgaa     600
   gcagctagga acgaagaaga attggtgatg ttgagggatg ccaaagattg gttagcgaca     660
   aaaagttcct tagttaacgt accacaccct aaaaacggag ctacaccttt                710
<210> 294
   <211> 22
   <212> DNA
   <213> Artificial Sequence
<220>
   <223> primer
<220>
   <221> misc_feature
   <222> (1)..(22)
   <223> forward primer
<400> 294
   gctctgttat gtgcgtcaag aa 22
<210> 295
   <211> 23
   <212> DNA
   <213> Artificial Sequence
<220>
   <223> primer
<220>
   <221> misc_feature
   <222> (1)..(23)
   <223> reverse primer
<400> 295
   aaaggtgtag ctccgttttt agg 23
<210> 296
   <211> 3435
   <212> DNA
   <213> Diabrotica virgifera
<220>
   <221> misc_feature
   <222> (1)..(3435)
   <223> WCRW transcript ta00860.008_diavv
<400> 296
   ttttatatta tccgtaatgg tggccgaccg tgtgcattct aaaactatta aataatcatg      60
   tcgctagaga ctcgatctaa ttcagctctg tttaaaagag cagaccaact aaaacgttgg     120
   gaagaatcag atacaaatcg agaaccagtg tcaccgaaac aaaaaactcg gaaaattaaa     180
   ttttcttcgg gatgtgtgtt tctcgcggct tgtgctgctg gtgataaaaa agaagtgttg     240
   gaattgttac ataaaggagc tgacatcaat actgcaaatg tggacggatt aacagcccta     300
   catcaggcat gtatcgacga caatctcgac atggtggagt tcctcgtgga gaacggggcg     360
   gacgtcaaca ggggcgacaa cgaaggttgg acccctttgc acgccactgc cagctgcggg     420
   tttctctcga tcgcgaagta cctcatcgaa aagggggccc acgtagcggc agttaataac     480
   gatggacaat tggcacttga tattgccgag agccaaaaaa tggaaggact actcagggaa     540
   gaaattgagg atagaggcat cgactgcgaa gcagctagga acgaagaaga attggtgatg     600
   ttgagggatg ccaaagattg gttagcgaca aaaagttcct tagttaacgt accacaccct     660
   aaaaacggag ctacaccttt acatgtagcc tgtgccaaag gttatgccga cgttataaaa     720
   atattattac aatgtggagc tgacattgat gcgcaggatg ttgacggctg gacccctcta     780
   catgcagctg cgcactgggg ccaaaaggaa acgtgccaaa tattggctga aagtttggct     840
   gacatggaag ctaaaaactt cgtaggacaa acagcatttg acttgtgtga tactgacatg 900
   ttatcgacat tggaagatct taaggaaaaa aaaaaaaaaa aaaaaacgaa ttaatagaag 960
   aatggccaag aagagggccg atattaacga taggaatttg gaatctgaga cgcctaccaa 1020
   agtgaaaaag gttgagctcg aagggacgaa agtgattaga aatatagaaa agaaaaataa 1080
   cctgaatgcc aacaatgaaa tcacaacgaa cactactgta ccccagaaga tcgttgaaga 1140
   caaagaggat gttactttaa ggaagaaacc gtatacaaaa cagaattcag atttgagtcc 1200
   aacaaaactg aatagtacga ataatttgac taattccacg gaatctataa agattgaacc 1260
   agaagttact ctcaggaaac ctcctattgt acaaaatgaa aaaccgacta aaaaagaaga 1320
   caacgataca ccggtcagta ccgctgcaac cacaaatacc acaactaaca tatcacaaga 1380
   aaagacttca ccagtgacta gtccacagcc tactactaac aaccaacaac aattgcgaag 1440
   cggtctaaag ttgcaaatgc cacctccgcc tacaacaacg gttaccgccc ctacaccggt 1500
   aacgaattct tccacgccct ccactcctac aacgccggga ggaaccaggc ttcagatgct 1560
   caaaaatttc tttaagtcgt ttgttcctcc agtaagggat gaagaaagtg aaactcaaag 1620
   aaaagcgcat gccaagaggg taagggagac tcgacgatcc actcaggggg taactttaga 1680
   agaaatcaaa tccgccgaac agttagtaaa acagaagaat caacctggtg gcggcaatga 1740
   ggaaaagcct ggagcaaatg gagactcccc caacggaatc gtagttaccg ccaccatcac 1800
   aaccggtact cccacagtta taacaactcg tgaaagtgag gaaccacccg caatacacga 1860
   aaggcgacca tcgtggaggt tgagggtaga caatggtacc agcaagttct tgttagaaga 1920
   cgcttcaaaa ccttcggacc cgccaaacac gccgtcgatt attcgtagga ccatcgcatc 1980
   catccaaaga ccttcttcag cgcctctaga gactgcagat actacaatta cactgccttt 2040
   aagaaaaccc aaatccgtag aagaaaaaga tcaagataag gagaacgata ttagaaatgc 2100
   tcaagcaact ctagccacac aagcagctat tcaaaggaga agaaggccga agaggagatc 2160
   gacaggggta gtttcgtttg ataatgttga tgatttggaa caggagaagg attcttcaag 2220
   tggaaaagac tgtgatgata ctactaaaat gaatcatttg gagagcggca cagaccgaag 2280
   tagccgatcg agaattggta gttcttcgga tattcgaaac gagaacggag aattggacta 2340
   caaaaaactg tatgaacagc aactcgcaga gaacgaccga ctgaaagaca aattaaggaa 2400
   atctgacgag gagttgcgag aaaccaaaca aactttggaa aaaattaact tggtcacaag 2460
   taaaaattcc ttatccgaac tagaaaaacg cgaaaggaga gccatggaga gaaaacttag 2520
   tgaaatggaa gaggaactaa agcaattaca aaagttgaaa gctgagaacg aacgcctaaa 2580
   ggccgacaac agatcactca cacgtgtcat caataaactt accaactctg caatgtaata 2640
   gaatttggaa aatcttaaat ccgagaacca gaggttgaag gatgaaaatg gtgctcttat 2700
   cagagtgatc agcaaactgt caaagtagca taagttttag ttgttatcat tcatgcattt 2760
   tatgttaagg tgactttaat aatttattgt tagaaaataa tgaaaattca ggatttccca 2820
   tttttattca cgttgaaagt tagtttcttt aacacagatt tgtagtagtt aaattgaatc 2880
   gtactattgt ggacgcgaat gaaaatagga cgcgaaaaaa agaatatgaa gctgaaatag 2940
   aggctatcga gatggaaatt gtaaagaaaa tagcagatga agaagaagta acgagatcat 3000
   taggtatata acaggtcaca aacaataaaa gaatataccc agacacaaaa aaataaacta 3060
   atgtgatcgt agattccaga gacaacttta aagtaaatat tactagcatc caagaaagat 3120
   atggatataa cacaccatat aagagatata tccccgaaaa aaagtaaaac tgttaagaga 3180
   ggatctacag tccttatcta aagaaactca agttgtttac cgagttgtgt actttacgag 3240
   ttttttgcgt acttagaggt tttttataaa ctgtacagaa gtaaacaaat acttcataaa 3300
   aacgtttctg tggggattga tactcggcta ttttaaatat atttattaaa taaatacatg 3360
   tttttttgtt tatacctcat aaaagtcatt cttatgatcc aaaaaaaaca aacaatccat 3420
   ttggcatact tattt 3435
<210> 297
   <211> 632
   <212> DNA
   <213> Diabrotica virgifera
<220>
   <221> misc_feature
   <222> (1)..(632)
   <223> RNAi target sequence
<220>
   <221> misc_feature
   <222> (610)..(610)
   <223> n can be any nucleotide
<400> 297 caaaaagaan ggatgaactt gaacgtgtag cc 632
<210> 298
   <211> 21
   <212> DNA
   <213> Artificial Sequence
<220>
   <223> primer
<220>
   <221> misc_feature
   <222> (1)..(21)
   <223> forward primer
<400> 298
   gacagtatgg aagacgattg g 21
<210> 299
   <211> 22
   <212> DNA
   <213> Artificial Sequence
<220>
   <223> primer
<220>
   <221> misc_feature
   <222> (1)..(22)
   <223> reverse primer
<400> 299
   ggctacacgt tcaagttcat cc 22
<210> 300
   <211> 806
   <212> DNA
   <213> Diabrotica virgifera
<220>
   <221> misc_feature
   <222> (1)..(806)
   <223> WCRW transcript ta21523.001_diavv
<400> 300
   gcacgaggag cgattgccgg agtggaagtc gctctcaaaa aaagagaaaa ttagaaaagc      60
   aaccttttcc ggttcacgcc gtcgttcagc aagagcttgc gatttttatt tttgaaaaat      120
   agagagtatt ctctaatatt taaggacagt atggaagacg attgggctgt ggataatcag      180
   agtggtggag ttgtcgcccc aaaaattgca gaactacctg aaattaagtt gttcgctaga      240
   tggaactgcg atgatgtcca agtttcagac atgtcccttc aggactacat tgcagtgaaa      300
   gaaaaaaatg caaagtattt accccattca gctggtagat atgctgcaaa aaggttccgt      360
   aaagcacaat gcccaatcgt tgagaggtta acaaactctc taatgatgca tggacgtaac      420
   aatggtaaaa aattgatggc tgtcagaatt gttaaacatg cttttgaaat tatccattta      480
   ctaactggag aaaatccatt acagatttta gtttctgcta ttatcaattc aggacctaga      540
   gaagattcta ctcgtattgg tagagctggt actgtaagaa gacaagctgt tgatgtgtca      600
   cccttgagaa gggttaacca agcaatttgg ttgctctgca caggtgctag ggaagcagca      660
   ttccgtaata ttaaaactat tgctgaatgt ttggctgatg aattaatcaa tgctgccaag      720
   ggatcatcaa attcatatgc tatcaaaaag aaggatgaac ttgaacgtgt agccaaatcc      780
   aaccgttaaa tttatttctc atttta                                           806
<210> 301
   <211> 546
   <212> DNA
   <213> Diabrotica virgifera
<220>
   <221> misc_feature
   <222> (1)..(546)
   <223> RNAi target sequence
<400> 301
   caagtagggc taaaatgaag caaattgtaa caaaccaaac ggtaaaaatt cctgagggaa        60
   ttacagtaac cactaagtca agggtagtaa ctgtaaaagg accccgaggt aatctgaaga       120
   gatccttcaa acatcttacc ctggacattc gaatgatcaa ccccaggtta ctgaaagtag       180
   agaaatggtt cggtaccaag aaggaattag ctgctgtcag aacagtatgc tctcatgttg       240
   aaaacatgtt aaagggtgtt acaaaaggat accaatacaa gatgagagca gcatatgctc       300
   actttcccat taactgtgtc accactgaag gaaacacagt tatcgaaatt agaaatttct       360
   tgggagaaaa atacatcaga agagtaaaga tggccccagg tgttacagta gtaaactcca       420
   ccaaacaaaa ggatgaactt atcattgaag gaaactcatt ggaggatgtt tcaaagtcag       480
   ctgcccttat tcaacaatct acaacagtta aaaataagga tatccgtaaa ttcttagatg       540
   ggcttt                                                                 546
<210> 302
   <211> 23
   <212> DNA
   <213> Artificial Sequence
<220>
   <223> primer
<220>
   <221> misc_feature
   <222> (1)..(23)
   <223> forward primer
<400> 302
   caagtagggc taaaatgaag caa 23
<210> 303
   <211> 23
   <212> DNA
   <213> Artificial Sequence
<220>
   <223> primer
<220>
   <221> misc_feature
   <222> (1)..(23)
   <223> reverse primer
<400> 303
   aaagcccatc taagaattta cgg 23
<210> 304
   <211> 789
   <212> DNA
   <213> Diabrotica virgifera
<220>
   <221> misc_feature
   <222> (1)..(789)
   <223> 02181.002_diavv
<400> 304
   ggggaaaact cttcggaata gaagtcgctt tttctgtcca ttttatgtca caagtagggc 60
   taaaatgaag caaattgtaa caaaccaaac ggtaaaaatt cctgagggaa ttacagtaac 120
   cactaagtca agggtagtaa ctgtaaaagg accccgaggt aatctgaaga gatccttcaa 180
   acatcttacc ctggacattc gaatgatcaa ccccaggtta ctgaaagtag agaaatggtt 240
   cggtaccaag aaggaattag ctgctgtcag aacagtatgc tctcatgttg aaaacatgtt 300
   aaagggtgtt acaaaaggat accaatacaa gatgagagca gcatatgctc actttcccat 360
   taactgtgtc accactgaag gaaacacagt tatcgaaatt agaaatttct tgggagaaaa 420
   atacatcaga agagtaaaga tggccccagg tgttacagta gtaaactcca ccaaacaaaa 480
   ggatgaactt atcattgaag gaaactcatt ggaggatgtt tcaaagtcag ctgcccttat 540
   tcaacaatct acaacagtta aaaataagga tatccgtaaa ttcttagatg ggctttatgt 600
   atctgaaaaa acaactgttg tacaagaaga ataacttgta tttttgaaat aaatttaagt 660
   ttatatttaa aaaaaaaaaa aaaaaatccg ttcaacaatc tacaacagtt aaaaataagg 720
   atatccgtaa attcttagat gggctttatg tatctgaaaa aacaactgtt gtacaagaag 780
   aataacttg 789
<210> 305
   <211> 409
   <212> DNA
   <213> Diabrotica virgifera
<220>
   <221> misc_feature
   <222> (1)..(409)
   <223> RNAi target sequence
<400> 305
   ctcttttgac aacggccaac gttctaaagt tgcatcatgg cgtcttcaga aggtgcaggc 60
   gtgactacag gtcaaccaaa tttacacaaa caagatttat ctaaattgga tgttaaaaga 120
   ttgacggctt tatctccaga agttataagt agacaagcta ccattaacat tggtacaatt 180
   ggacatgtag cgcatggaaa atcgactgtg gttaaggcca tatctggggt acaaactgtc 240
   agatttaaga atgaactgga aaggaacatt accattaagc taggttatgc taacgcaaaa 300
   atctacaaat gtgataatcc gaaatgcccc aggccctcct gctttatttc gggcagttct 360
   tcaaaggacg acagtttccc ttgtttacgt ccagcatgtt cgggtagat 409
<210> 306
   <211> 20
   <212> DNA
   <213> Artificial Sequence
<220>
   <223> primer
<220>
   <221> misc_feature
   <222> (1)..(20)
   <223> forward primer
<400> 306
   ctcttttgac aacggccaac 20
<210> 307
   <211> 20
   <212> DNA
   <213> Artificial Sequence
<220>
   <223> primer
<220>
   <221> misc_feature
   <222> (1)..(20)
   <223> reverse primer
<400> 307
   atctacccga acatgctgga 20
<210> 308
   <211> 1691
   <212> DNA
   <213> Diabrotica virgifera
<220>
   <221> misc_feature
   <222> (1)..(1691)
   <223> WCRW transcript ta02330.001_diavv
<400> 308
   ggacaagtga cgtgttttca tgaacgttag atgtcaaaat tattggccgc ctcttttgac      60
   aacggccaac gttctaaagt tgcatcatgg cgtcttcaga aggtgcaggc gtgactacag     120
   gtcaaccaaa tttacacaaa caagatttat ctaaattgga tgttaaaaga ttgacggctt     180
   tatctccaga agttataagt agacaagcta ccattaacat tggtacaatt ggacatgtag     240
   cgcatggaaa atcgactgtg gttaaggcca tatctggggt acaaactgtc agatttaaga     300
   atgaactgga aaggaacatt accattaagc taggttatgc taacgcaaaa atctacaaat     360
   gtgataatcc gaaatgcccc aggccctcct gctttatttc gggcagttct tcaaaggacg     420
   acagtttccc ttgtttacgt ccagcatgtt cgggtagatt ccaattggtc agacatgtca     480
   gctttgtaga ttgccctggt cacgacattc ttatggctac catgcttaac ggagccgctg     540
   tgatggacgc agcattgtta ttaatagcag gaaacgaatc atgtcctcaa cctcaaacta     600
   gtgaacattt ggctgctatc gaaatcatga agttgaaaca catcctcatt cttcaaaata     660
   aaatagattt ggtgaaggaa gggcaagcca aggagcaaca tgagcaaatt gtgaaatttg     720
   tccaaggaac tgtagctgaa ggagccccta taattcccat atccgctcag ttgaaatata     780
   acatcgaggt tttgtgcgaa tatatcacta aaaaaattcc aataccaatg agagatttta     840
   cttctgaacc aaggctgatc gtgatcagat cttttgatgt taataaacct ggttgtgaag     900
   ttaacgattt aaaaggaggt gtagctggtg gcagtatttt aagaggagtg ttgaaggtgg     960
   gacaagaaat tgaagtaaga cctggactag ttagcaagga ctcagaagga aagctggcct    1020
   gccgaccaat attctctaga atagtatctc tatataccga acaaaacgaa ctgcagtttg    1080
   cagttccagg aggactcatt ggtgtcggta ccaaaatcga accaaccctt tgcagagccg    1140
   atcgacttgt aggtcaagtt ttgggagcag ttggtgctct acctagtatc tttacagaac    1200
   ttgaggtgag ttactatcta ttgaaacgtt tgctgggagt ccgtacagaa ggagacaaga    1260
   aaggggccaa agtccagaga ttaaccagaa gtgaagtact ccttgtcaac attggatccc    1320
   tcagcacggg aggacgagtt gtagccacta aggccgattt gtgcaagatc gctttaacga    1380
   atccagtctg tacggaaatc ggcgaaaaga ttgcactcag tagacgtgtt gaaaaacatt    1440
   ggcgtctcat cggatggggg cagatccgcg gaggggagac aatcgagcct accacaagct    1500
   aggcttttta tactcgtacc ggtcttttca ggacacttta agccaatttt tgttttctat    1560
   ttaatatcgt tttattaaag aagtgtttac gttttgttta tatcttttag gttgattttt    1620
   attactgcta tagtgtactg attatccgat tcaaatataa tgtctataaa actaaaaaaa    1680
   aaaaaaaaaa a                                                          1691
<210> 309
   <211> 670
   <212> DNA
   <213> Diabrotica virgifera
<220>
   <221> misc_feature
   <222> (1)..(670)
   <223> RNAi target sequence
<400> 309
   attgcagtca ttgctgtagt gcacacgtaa tttaagtatt tatttaagaa ttgtctgcaa      60
   ataattaatt ataagatggc taaagcccca gctgtaggta ttgatttagg aaccacatac     120
   tcctgtgtag gagttttcca acatggcaaa gttgaaatta ttgccaacga ccaaggtaac     180
   agaaccacac catcatatgt ggccttcaca gacacagaac gtctcatcgg agatgctgcc     240
   aagaaccagg tagccatgaa ccccaataac acaatttttg atgccaaacg tcttattggg     300
   cgtcgctttg atgacagtgc tgtacagtct gacatgaaac attggccatt tgaagtagta     360
   aatgatgcag gtaaaccaaa gattaaagtt gcatacaagg gcgaagacaa atccttctac     420
   ccagaagaag tcagttccat ggtccttaca aaaatgaagg aaacagcaga agcatactta     480
   ggaaagaatg tcaccaacgc tgtcatcaca gtacctgcat atttcaatga ctcacaacgt     540
   caagctacca aagatgctgg aaccattgct ggactccagg tattacgtat tattaacgaa     600
   cccactgctg ctgccatcgc ctatggttta gacaaaaagg gccaaggtga aagaaacgtc     660
   ctcattttcg                                                            670
<210> 310
   <211> 22
   <212> DNA
   <213> Artificial Sequence
<220>
   <223> primer
<220>
   <221> misc_feature
   <222> (1)..(22)
   <223> forward primer
<400> 310
   attgcagtca ttgctgtagt gc 22
<210> 311
   <211> 22
   <212> DNA
   <213> Artificial Sequence
<220>
   <223> primer
<220>
   <221> misc_feature
   <222> (1)..(22)
   <223> reverse primer
<400> 311
   cgaaaatgag gacgtttctt tc 22
<210> 312
   <211> 2609
   <212> DNA
   <213> Diabrotica virgifera
<220>
   <221> misc_feature
   <222> (1)..(2609)
   <223> WCRW transcript ta00491.010_diavv
<220>
   <221> misc_feature
   <222> (19)..(19)
   <223> n can be any nucleotide
<220>
   <221> misc_feature
   <222> (49)..(49)
   <223> n can be any nucleotide
<400> 312
   ggcccatgga actgacttnt tctgggtaga aggatttgtc tttcgcccnt tgtatgcaaa      60
   ctttaatctt ttggtttacc ttgcatcatt tattactttc aaatggccaa tgtttcatgt     120
   cagactgtac agcactgtca tcaaagcgac gcccaataag acgtttggca tcaaaaattg     180
   tgttattggg gttcatggct acctggttct tggcagcatc tccgatgaga cgttctgtgt     240
   ctgtgaaggc cacatatgat ggtgtggttc tgttaccttg gtcgttggca ataatttcaa     300
   ctttgccatg ttggaaaact cctacacagg agtatgtggt tcctaaatca atacctacag     360
   ctggggcttt agccatcttg gggggatttg attgcagtca ttgctgtagt gcacacgtaa     420
   tttaagtatt tatttaagaa ttgtctgcaa ataattaatt ataagatggc taaagcccca     480
   gctgtaggta ttgatttagg aaccacatac tcctgtgtag gagttttcca acatggcaaa     540
   gttgaaatta ttgccaacga ccaaggtaac agaaccacac catcatatgt ggccttcaca     600
   gacacagaac gtctcatcgg agatgctgcc aagaaccagg tagccatgaa ccccaataac     660
   acaatttttg atgccaaacg tcttattggg cgtcgctttg atgacagtgc tgtacagtct 720
   gacatgaaac attggccatt tgaagtagta aatgatgcag gtaaaccaaa gattaaagtt 780
   gcatacaagg gcgaagacaa atccttctac ccagaagaag tcagttccat ggtccttaca 840
   aaaatgaagg aaacagcaga agcatactta ggaaagaatg tcaccaacgc tgtcatcaca 900
   gtacctgcat atttcaatga ctcacaacgt caagctacca aagatgctgg aaccattgct 960
   ggactccagg tattacgtat tattaacgaa cccaccgctg ctgccatcgc ctatggttta 1020
   gacaaaaagg gccaaggtga aagaaacgtc ctcattttcg atctgggtgg tggtactttt 1080
   gatgtatcaa tcttaacaat tgaagatggt atctttgaag tcaaatcaac tgctggagac 1140
   actcacttgg gaggtgaaga cttcgacaac agaatggtca atcactttgt tggagaattc 1200
   aagaggaaat acaagaagga ccttaccaca aacaaacgtg ccctccgtcg tttgagaaca 1260
   agctgtgaaa gagctaagcg taccctttcc tcatcaaccc aagccagcat tgaaatcgat 1320
   tctctatttg agggtattga cttctacacc tcaatcacca gggctagatt tgaagaactc 1380
   aacgctgatt tgttcagatc taccatggaa cctgtagaaa aggccatcag ggatgccaag 1440
   atggacaaat ctcaagttca cgatattgtg ttggttggtg gatctacccg tattccaaag 1500
   gtacaaaaac tgttgcaaga tttcttcaat ggtaaagaat tgaacaagtc gatcaacccc 1560
   gatgaagctg ttgcttatgg tgcagccgtc caagccgcca tcttgcacgg agataagtct 1620
   gaggaagtac aggacttgct cttgctcgat gtcactccac tctcattggg tattgaaact 1680
   gccggaggtg ttatgactgc cctcatcaaa cgtaacacta ccattcccac caagcaaacc 1740
   cagaccttca ctacttactc tgacaaccaa cccggagtac ttattcaggt atatgaaggt 1800
   gaacgtgcca tgaccaagga caacaacctc ttgggaaaat tcgaacttac tggcattcct 1860
   cctgcgcccc gtggtgtccc tcagattgaa gtaaccttcg acattgatgc caacggtatc 1920
   ttgaacgtca cagctattga aaaatccacc aacaaagaaa acaagatcac catcaccaat 1980
   gataaaggtc gtctcagcaa agaagacatt gaaagaatgg tcaacgatgc agaaaaatac 2040
   cgtaacgaag atgataagca aaaggctacc atctctgcca agaacgtcct cgaatcatac 2100
   tgtttcaaca tcaagagcac catggaagat gacaaaatca aggacaagat cagcgaaagt 2160
   gacaaaacta ccgtcatgga gaaatgcaac gaggttattg cttggttgga tgctaaccaa 2220
   ttagccgaca aggaagaata tgaacacaaa caaaaggagc tcgaaaacat ctgcaacccc 2280
   atcatcacca agttgtacca aggtgccggt ggagctccag gtggcatgcc cggtggtttc 2340
   ccaggtggag cagctccagg agctgcaggc gccgctggag gtgctggacc aaccattgaa 2400
   gaagtcgatt aaactattca ttccattttt taaacttttg ttaaccgttg tgcagtatta 2460
   cttgcagaca aagcttttta aaattacttt ttttacacaa tttttcaatt gttaaacaac 2520
   gtttttggta acagttcaaa aattactgca ataattatgt tcgttcatga ataaaataaa 2580
   aagtcaaatc cgaaaaaaaa aaaaaaaaa 2609
<210> 313
   <211> 672
   <212> DNA
   <213> Diabrotica virgifera
<220>
   <221> misc_feature
   <222> (1)..(672)
   <223> RNAi target sequence
<220>
   <221> misc_feature
   <222> (648)..(648)
   <223> n can be any nucleotide
<220>
   <221> misc_feature
   <222> (649)..(649)
   <223> n can be any nucleotide
<400> 313
   tacacgtaaa aatggcttcg acgatatcgg caagacaggc taacagagaa catgttttgg 60
   ccgtatccag agacttcata tcccaaccaa gactcactta caaaactgta tctggtgtca 120
   acggtccact agtaatcctc gaagatgtca aatttcctaa gttcaacgaa attgtccaac 180
   tcagattatc tgacggaagt ttaagatcag gtcaagtact tgaagtcagc ggttccaagg 240
   ctgtagtaca agtattcgaa ggtacatcag gtatcgatgc caaacacact gtctgtgagt 300
   tcacaggaga tattctcaga actccagtct ctgaggatat gttgggaaga gtattcaacg 360
   gctctggaaa acctattgac aaaggtcccc caattctggc tgaagactac ctagatatcc 420
   aaggacaacc catcaaccca tggtcccgta tctacccaga ggaaatgatc caaactggta 480
   tcacagctat cgatgtgatg aactcgattg cccgtggtca gaagattccc attttctcag 540
   ctgctggtct gccacataac gatattgctg cccaaatttg taggcaagcc ggtcttgtca 600
   aaatcccagg caaatcagtt ttggatgacc acgaagannc tttgccatcg tgttcgctgc 660
   tatgggtgtt aa 672
<210> 314
   <211> 22
   <212> DNA
   <213> Artificial Sequence
<220>
   <223> primer
<220>
   <221> misc_feature
   <222> (1)..(22)
   <223> forward primer
<400> 314
   tacacgtaaa aatggcttcg ac 22
<210> 315
   <211> 21
   <212> DNA
   <213> Artificial Sequence
<220>
   <223> primer
<220>
   <221> misc_feature
   <222> (1)..(21)
   <223> reverse primer
<400> 315
   ttaacaccca tagcagcgaa c 21
<210> 316
   <211> 2583
   <212> DNA
   <213> Diabrotica virgifera
<220>
   <221> misc_feature
   <222> (1)..(2583)
   <223> WCRW transcript ta00620.001_diavv
<400> 316
   tcttaaactt ccgtcagatg ggggcagttg ctaatttttt actgtctttg atagacgtct      60
   cctgtacacg taaaaatggc ttcgacgata tcggcaagac aggctaacag agaacatgtt     120
   ttggccgtat ccagagactt catatcccaa ccaagactca cttacaaaac tgtatctggt     180
   gtcaacggtc cactagtaat cctcgaagat gtcaaatttc ctaagttcaa cgaaattgtc     240
   caactcagat tatctgacgg aagtttaaga tcaggtcaag tacttgaagt cagcggttcc     300
   aaggctgtag tacaagtatt cgaaggtaca tcaggtatcg atgccaaaca cactgtctgt     360
   gagttcacag gagatattct cagaactcca gtctctgagg atatgttggg aagagtattc     420
   aacggctctg gaaaacctat tgacaaaggt cccccaattc tggctgaaga ctacctagat     480
   attcaaggac aacccatcaa cccatggtcc cgtatctacc cagaggaaat gatccaaact     540
   ggtatcactg ccatcgatgt gatgaactcg attgcccgtg gtcagaagat tcccattttc     600
   tcagctgctg gtctgccaca taacgatatt gctgcccaaa tttgtaggca agccggtctt     660
   gtcaaaatcc caggcaaatc agttttggat gaccacgaag acaactttgc catcgtgttc     720
   gctgctatgg gtgttaacat ggagactgct agattcttca aacaagattt cgaagaaaat     780
   ggttctatgg aaaacgtatg tttgttcttg aacttggcta acgatccaac catcgaaaga 840
   atcatcaccc cacgtcttgc tctaacagcc gctgaattct tggcttacca gtgcgaaaaa 900
   cacgtgttag tcattttaac tgacatgtct tcatacgctg aagctttgcg tgaggtatct 960
   gccgccagag aggaagtacc cggtcgtcgt ggtttccccg gttacatgta caccgatttg 1020
   gccaccatct acgaacgtgc cggtcgtgtc gaaggccgta acggttccat cactcaaatc 1080
   cccatcttga ctatgcctaa cgacgatatt actcacccta ttcccgattt gacaggttat 1140
   attaccgaag gacagatcta cgtcgaccgt caattgcaca acaggcagat ctacccacca 1200
   attaacgtac tgccatctct ctcacgtttg atgaagtctg ctatcggcga aggtatgacc 1260
   aggaaggacc actctgacgt ttctaaccaa ttgtatgctt gttatgctat cggtaaggac 1320
   gtccaggcca tgaaagccgt agtaggtgaa gaagctctaa cacccgacga tcttctctac 1380
   ctcgagttcc taacgaaatt cgaaaagaac ttcatcaccc aaggaaacta cgaaaaccgc 1440
   accgtcttcg agtctttgga catcggctgg caactactcc gtatcttccc taaggaaatg 1500
   ttgaaacgta tccctgcaag cacattggcg gaattctatc caagagactc tcgtcactag 1560
   ttggtttatg tataaaattt atatatattt aggaagtttg ttaggtttgt tacaaatatg 1620
   tccacttatt gtatcgatta ttttcaattg agagatattt taccaaaaca atttgagccg 1680
   atgagattct acggtgtaat ttttgtaaat acttaaagag cgtgtttcaa aaatgcgctt 1740
   aatataatat attatcaata cagagcaacc tttttttctt tattattttt tcttgttgaa 1800
   caatatataa agttcttgaa ctccctaagt ggaacataga gcttcagtga aaatgcacca 1860
   tcacctcatt ccaagacttt ccttgacttc ttaccaattg ttggacaatt ataactcaga 1920
   ttaattttac taggtgacag ttccataagt aaaaatatgc aatggctata tattgcgtac 1980
   taaacttaag aagaaaaatt gtgtcgctct ggaagaaaga ttacttgcat aagaatgaaa 2040
   atcagtgtca tattttgcgt ttatgacatc cctagacgat actatttttt cgttactata 2100
   ttttttttaa agagatcaag agcaacaatt tggagactga tattcttatg cgagttgttt 2160
   tcctactttg tcctattttg aagagtctac aattatttac ttaccatttg attaaattag 2220
   atgaacataa gacaatacta tatttttatc atgtaagttg atattcccta tgtaaattat 2280
   tggcacaatg taacctccaa cttgatttat acatttattt tacttttgtt tgtctatttt 2340
   ttctgtaata gagtggaaga attaacattc cattaaatgt cattgcccaa tgtataaagc 2400
   aatatacctc ttaccattcg atgctttttc agtattttaa ttctagatgg tgaacggaga 2460
   tttttcttga tgtaggtttt gacattttcg atgttactgt aaaaatctaa ttttatgtta 2520
   aatagattag tgtacaaaaa tgtgaataaa cactatacat atgttaaaaa aaaaaaaaaa 2580
   aaa 2583
<210> 317
   <211> 500
   <212> DNA
   <213> Diabrotica virgifera
<220>
   <221> misc_feature
   <222> (1)..(500)
   <223> RNAi target sequence
<400> 317
   actttgacga gaggcgttgt tagttacttc gttgtgtcga catttttctg tttagtgtac      60
   cgattaatct tcaaatatta caatggctga ccaactcacc gaagaacaaa ttgccgaatt     120
   caaagaagct ttctcactat tcgataaaga tggtgatggt acaattacga ctaaagaatt     180
   aggaacagta atgagatctc taggacaaaa tccaacagag gctgaattac aggatgtgac     240
   caatgaagta gatgccgatg gtaacggcac gatcgatttc ccagaatttt taacgatgat     300
   ggcacgtaaa atgaaagata ccgatagtga ggaagaaatt cgtgaagcat tccgagtgtt     360
   cgacaaagac ggcaatggtt tcatctcagc agcagaattg cgccacgtca tgaccaactt     420
   gggtgaaaaa ttgacagacg aagaagtcga tgaaatgatt cgggaggccg atatcgatgg     480
   tgatggtcaa gtcaattacg                                                 500
<210> 318
   <211> 20
   <212> DNA
   <213> Artificial Sequence
<220>
   <223> primer
<220>
   <221> misc_feature
   <222> (1)..(20)
   <223> forward primer
<400> 318
   actttgacga gaggcgttgt 20
<210> 319
   <211> 22
   <212> DNA
   <213> Artificial Sequence
<220>
   <223> primer
<220>
   <221> misc_feature
   <222> (1)..(22)
   <223> reverse primer
<400> 319
   cgtaattgac ttgaccatca cc 22
<210> 320
   <211> 713
   <212> DNA
   <213> Diabrotica virgifera
<220>
   <221> misc_feature
   <222> (1)..(713)
   <223> WCRW transcript ta02046.005_diavv
<400> 320
   gcagcagcag ttcccccctc tcttctctta cgtcgatcct aaacaaaacg agaaaagaca 60
   gttggctggt gcactttgac gagaggcgtt gttagttact tcgttgtgtc gacatttttc 120
   tgtttagtgt accgattaat cttcaaatat tacaatggct gaccaactca ccgaagaaca 180
   aattgctgaa ttcaaagaag ctttctcact attcgataaa gatggtgatg gtacaattac 240
   gactaaagaa ttaggaacag taatgagatc tctaggacaa aatccaacag aggctgaatt 300
   acaggatatg atcaatgaag tagatgccga tggtaacggc acgatcgatt tcccagaatt 360
   tttaacgatg atggcacgta aaatgaaaga taccgatagt gaggaagaaa ttcgtgaagc 420
   attccgagtg ttcgacaaag acggcaatgg tttcatctca gcagcagaat tgcgccacgt 480
   catgaccaac ttgggtgaaa aattgacaga cgaagaagtc gatgaaatga ttcgggaggc 540
   cgatatcgat ggtgatggtc aagtcaatta cgaagagttt attagaagtc acctcttggc 600
   caactaagat attattgatt acaaagaaaa atagaagaat atattataat ttacagatac 660
   tatattaatg ataatagaat aagaataaaa tgtaaataaa tagttttgct cat 713
<210> 321
   <211> 514
   <212> DNA
   <213> Diabrotica virgifera
<220>
   <221> misc_feature
   <222> (1)..(514)
   <223> RNAi target sequence
<400> 321
   tttggcaaca ttgtgtatga tgtcatagga gcggggtcag ataggaatgt tgtagttttg    60
   aatgatatac acatagatat aatggactat atagtgcctg ctagttgtac agatagcgag   120
   tttatgagaa tgtgggcgga atttgaatgg gaaaataagg taaccgttaa cacacccctc   180
   acggaacttt cagaatacct cgaacatcta ctcaaaagca caaatttgaa atgtttaaca   240
   tcagaaaaag ctctgagcgg gcagtgtggt tttatggcag ccaatttata tgcaaaatcc   300
   atttttggag aagacgcttt ggccaactta agtatagaga aaccttttaa taaacccgat   360
   gcgccagtaa gcggtcatat tagaataagg gccaaaagtc agggcatggc cttaagttta   420
   ggagacaaag tcaatatgac acagaagagc acacaacata aagtagtagc tgcataaata   480
   aaaacgtttt tttctgtttc aacccttttc ttca                                514
<210> 322
   <211> 22
   <212> DNA
   <213> Artificial Sequence
<220>
   <223> primer
<220>
   <221> misc_feature
   <222> (1)..(22)
   <223> forward primer
<400> 322
   tttggcaaca ttgtgtatga tg 22
<210> 323
   <211> 23
   <212> DNA
   <213> Artificial Sequence
<220>
   <223> primer
<220>
   <221> misc_feature
   <222> (1)..(23)
   <223> reverse primer
<400> 323
   tgaagaaaag ggttgaaaca gaa 23
<210> 324
   <211> 3271
   <212> DNA
   <213> Diabrotica virgifera
<220>
   <221> misc_feature
   <222> (1)..(3271)
   <223> WCRW transcript ta00934.001_diavv
<400> 324
   gggggtcgca tcaaaacgtc aacaatctga cacgtctgta atgtttagcc tgaattttga        60
   gtaaaattac cgaaaaacat aattaaaatt gatttattaa gagtacataa taacgtacga       120
   aaatgaccgc ggtagaacaa ccttgttaca cactaataaa cttgccaaca gattcggagc       180
   cctacaatga aatgcaacta aaaatggatt tagaaaaggg tgaggttaaa gtaaaaataa 240
   gagcattaga aaaaataatt cacatgattc tggcaggaga aaggttgccg aatggattcc 300
   taatgaccat cataagaaac gttttacctc tacaagatca tttggcaaaa aaactattat 360
   tgattttctg ggaaatagtt ccaaaaacaa atccagaagg taaactacta caagagatga 420
   ttttggtatg tgatgcctat agaaaagatc tgcaacaccc aaatgaattt ttgagaggtt 480
   ctacacttcg cttcttgtgc aaactgaagg aaccagaatt gttggaacca ttaatgccca 540
   gtattagagc ttgtttggat cataggcaca gctatgtgag gaggaatgct gtactggcaa 600
   tttttaccat ttacaaaaat tttgaagccc tcattccaga tgctcctgaa ctgatctcca 660
   attatttgga tggtgagcaa gacatgtctt gtaaaagaaa tgcgttttta atgcttcttc 720
   atgctgacca agaaagggcg ttgtcgtatt tggcatcatg tttagatcaa gtaaattcat 780
   ttggagatat tctacaactg gtcatcgttg agttgatata taaggtgtgt cattccaatc 840
   ctgcggaaag atctagattt attagatgta tatataactt gttgaactca agcagtcctg 900
   ctgtcaggta cgaagctgca ggaactttag tcaccctctc cagtgccccg actgccgtta 960
   aagctgctgc tagctgttac attgagttaa ttatcaaaga aagtgacaac aatgtaaaac 1020
   tcatcgtttt ggacaggctg atagcactta aggagcttcc taatcacgaa agaattctgc 1080
   aggatttagt tatggacata ctgagagtac tctctgctcc tgacttagaa gtccgcaaga 1140
   agactttaag tctagccctt gaattagtct cttcacggaa catagaagaa atggtattag 1200
   tattaacaaa ggaagtgagt aaaacggtag acagtgaaca tgaggataca ggaaagtaca 1260
   ggcaattgtt agtaaggact ctacattcgt gttccattaa gttcccagat atcgcacgta 1320
   gtgttatacc agtcttgatt gaatttttat ccgataataa tgaactggct gccacagatg 1380
   tattgctgtt cttaagggaa gccatacaga agtttaaaga attgcaaccg ttaattattg 1440
   agaaactcat cgaaactttc aaagacatta aattggtcaa agtccataga gcagcaattt 1500
   ggattttggg agaatacgcg agtactgctt ccgatataga agttatcgtt ggagaaatta 1560
   acagattgtt gggtgaagga tccctcgttg aagctgagca gaagttaata gcaggagata 1620
   cggaagagaa tgctcctgca cctgctgcag gcgccaccac tttagttact tccgatggaa 1680
   catatgctac ccaatcagct ttcaacactg tcagccaaac cactaaagaa gcacgacctc 1740
   ctctaagaca atacctcatg gatggtgatt ttttcatcgg agcctctttg gcatctacat 1800
   taaccaaact gtctttgcgg tatgaggacc tcacctctcc tgctgctagc aatggattca 1860
   atgccaaaat tatgcttatt atggctggaa ttcttcactt gggaaaatca ggacttccca 1920
   caaaatcaat aaccaacgac gataaagacc acattctgtt ctgtttacga gtcctatctg 1980
   atcgttctcc aatcattgtt gaaattttca aaaaattgtg ccgctcggca ctaaatgaga 2040
   tgcttctagc taaggaatcg gtagaagcga tctcgcaaaa gagcaaagaa aaaaacaagc 2100
   gtacgattca aactgacgac gctataagct tcctgcaatt agagacagat aaaagtggag 2160
   agctaggaga aaacgtattc gagatgtcgc tgtcacaagc tttagtagga ggtcgaacgg 2220
   gaggtggcga atcagtatta agttccaata aattagataa aatcacacaa ctgactggtt 2280
   tttccgatcc agtttattcc gaagcatacg ttcacgtgaa tcagtacgat atcgtgcttg 2340
   atgtcttaat cgtaaaccaa actaacgata ctttacaaaa ctgcacgcta gagctggcta 2400
   ctttaggcga tttgaagttg gtagagaagc cacaacctgt cgtattggcg cccaaagact 2460
   tttgcaacat taaagctaac gtgaaagtgg cctcaactga aaacggaatt atatttggca 2520
   acattgtgta tgatgtcata ggagcggggt cagataggaa tgttgtagtt ttgaatgata 2580
   tacacataga tataatggac tatatagtgc ctgctagttg tacagatagc gagtttatga 2640
   gaatgtgggc ggaatttgaa tgggaaaata aggtaaccgt taacacaccc ctcacggaac 2700
   tttcagaata cctcgaacat ctactcaaaa gcacaaattt gaaatgttta acatcagaaa 2760
   aagctctgag cgggcagtgt ggttttatgg cagccaattt atatgcaaaa tccatttttg 2820
   gagaagacgc tttggccaac ttaagtatag agaaaccttt taataaaccc gatgcgccag 2880
   taagcggtca tattagaata agggccaaaa gtcagggcat ggccttaagt ttaggagaca 2940
   aagtcaatat gacacagaag agcacacaac ataaagtagt agctgcataa ataaaaacgt 3000
   ttttttctgt ttcaaccctt ttcttcattt taaatcatat tctgtaattt aattaattta 3060
   atatcatatt actggttaca cattgttaga ttataaatat cttttaaaga ataaaattta 3120
   tttagctttt acaaagagcg gaaagcaatc tttatcttct tctaataaga tcaacataaa 3180
   gttttctgga aaatataata tataatttaa aaatgtttag catttattag tgaaatacat 3240
   ggcttactat tttgatgaca aagtcagtgt a 3271
<210> 325
   <211> 606
   <212> DNA
   <213> Diabrotica virgifera
<220>
   <221> misc_feature
   <222> (1)..(606)
   <223> RNAi target sequence
<220>
   <221> misc_feature
   <222> (577)..(577)
   <223> n can be any nucleotide
<400> 325
   gccctcatca aacgtaacac taccattccc accaagcaaa cccagacctt cactacttac      60
   tctgacaacc aacccggagt acttattcag gtatatgaag gtgaacgtgc catgaccaag     120
   gacaacaacc tcttgggaaa attcgaactt actggcattc ctcctgcgcc ccgtggtgtc     180
   cctcagattg aagtaacctt cgacattgat gccaacggta tcttgaacgt cacagctatt     240
   gaaaaatcca ccaacaaaga aaacaagatc accatcacca atgataaagg tcgtctcagc     300
   aaagaagaca ttgaaagaat ggtcaacgat gcagaaaaat accgtaacga agatgataag     360
   caaaaggcta ccatctctgc caagaacgtc ctcgaatcat actgtttcaa catcaagagc     420
   accatggaag atgacaaaat caaggacaag atcagcgaaa gtgacaaaac taccgtcatg     480
   gagaaatgca acgaggttat tgcttggttg gatgctaacc aattagccga caaggaagaa     540
   tatgaacaca aacaaaagga gctcgaaaac atctgcnacc ccatcatcac caagttgtac     600
   caaggt                                                                606
<210> 326
   <211> 22
   <212> DNA
   <213> Artificial Sequence
<220>
   <223> primer
<220>
   <221> misc_feature
   <222> (1)..(22)
   <223> forward primer
<400> 326
   gccctcatca aacgtaacac ta 22
<210> 327
   <211> 22
   <212> DNA
   <213> Artificial Sequence
<220>
   <223> primer
<220>
   <221> misc_feature
   <222> (1)..(22)
   <223> reverse primer
<400> 327
   accttggtac aacttggtga tg 22
<210> 328
   <211> 2209
   <212> DNA
   <213> Diabrotica virgifera
<220>
   <221> misc_feature
   <222> (1)..(2209)
   <223> WCRW transcript 454run12_isotig00560
<400> 328
   gatttgattg cagtcattgc tgtagtgcac acgtaattta agtatttatt taagaattgt 60
   ctgcaaataa ttaattataa gatggctaaa gccccagctg taggtattga tttaggaacc 120
   acatactcct gtgtaggagt tttccaacat ggcaaagttg aaattattgc caatgaccaa 180
   ggtaacagaa ccacaccatc atatgtggcc ttcacagaca cagaacgtct catcggagat 240
   gctgccaaga accaggtagc catgaacccc aataacacaa tttttgatgc caaacgtctt 300
   attgggcgtc gctttgatga cagtgctgta cagtctgaca tgaaacattg gccatttgaa 360
   gtagtaaatg atgcaggtaa accaaagatt aaagttgcat acaagggcga agacaaatcc 420
   ttctacccag aagaagtcag ttccatggtc cttacaaaaa tgaaggaaac agcagaagca 480
   tacttaggaa agaatgtcac caacgctgtc atcacagtac ctgcatattt caatgactca 540
   caacgtcaag ctaccaaaga tgctggaacc attgctggac tccaggtatt acgtattatt 600
   aacgaaccca ctgctgctgc catcgcctat ggtttagaca aaaagggcca aggtgaaaga 660
   aacgtcctca ttttcgatct gggtggtggt acttttgatg tatcaatctt aacaattgaa 720
   gatggtatct ttgaagtcaa atcaactgct ggagacactc acttgggagg tgaagacttc 780
   gacaacagaa tggtcaatca ctttgttgga gaattcaaga ggaaatacaa gaaggacctt 840
   accacaaaca aacgtgccct ccgtcgtttg agaacaagct gtgaaagagc taagcgtacc 900
   ctttcctcat caacccaagc cagcattgaa atcgattctc tatttgaggg tattgacttc 960
   tacacctcaa tcaccagggc tagatttgaa gaactgaacg ctgatttgtt cagatctacc 1020
   atggaacctg tagaaaaggc catcagggat gccaagatgg acaaatctca agttcacgat 1080
   attgtgttgg ttggtggatc tacccgtatt ccaaaggtac aaaaactgtt gcaagatttc 1140
   ttcaatggta aagaattgaa caagtcgatc aaccccgatg aagctgttgc ttatggtgca 1200
   gccgtccaag ccgccatctt gcacggagat aagtctgagg aagtacagga cttgctcttg 1260
   ctcgatgtca ctccactttc attgggtatt gaaactgccg gaggtgttat gactgccctc 1320
   atcaaacgta acactaccat tcccaccaag caaacccaga ccttcactac ttactctgac 1380
   aaccaacccg gagtacttat tcaggtatat gaaggtgaac gtgccatgac caaggacaac 1440
   aacctcttgg gaaaattcga acttaccggc attcctcctg cgccccgtgg tgtccctcag 1500
   attgaagtaa ccttcgacat tgatgccaac ggtatcttga acgtcacagc tattgaaaaa 1560
   tccaccaaca aagaaaacaa gatcaccatc accaatgata aaggtcgtct cagcaaagaa 1620
   gacattgaaa gaatggtcaa cgatgcagaa aaataccgta acgaagatga taagcaaaag 1680
   gctaccatct ctgccaagaa cgtcctcgaa tcatactgtt tcaacatcaa gagcaccatg 1740
   gaagatgaca aaatcaagga caagatcagc gaaagtgaca aaactaccgt catggagaaa 1800
   tgcaacgagg ttattgcttg gttggatgct aaccaattag ccgacaagga agaatatgaa 1860
   cacaaacaaa aggagctcga aaacatctgc aaccccatca tcaccaagtt gtaccaaggt 1920
   gccggtggag ctccaggtgg catgcccggt ggtttcccag gtggagcagc tccaggagct 1980
   gcaggcgccg ctggaggtgc tggaccaacc attgaagaag tcgattaaac tattcattcc 2040
   attttttaaa cttttgttaa ccgttgtgca gtattacttg cagacaaagc tttttaaaat 2100
   tacttttttt acacaatttt tcaattgtta aacaacgttt ttggtaacag ttcaaaaatt 2160
   actgcaataa ttatgttcgt tcatgaataa aataaaaagt caaatccgc 2209
<210> 329
   <211> 718
   <212> DNA
   <213> Diabrotica virgifera
<220>
   <221> misc_feature
   <222> (1)..(718)
   <223> RNAi target sequence
<400> 329
   gttgggattg ggatttaaca aatacgtcgt tctaaatttc taaaactgca tacaaaaaat 60
   ccttaaaaaa ctagtgaaga ttactaaagt gcaatagata caaaaacaat atttttacgt 120
   attcaccata ataatataaa cgtagaaaat gaatcccgag tatgattatt tattcaaact 180
   tctgctgatt ggagattcag gagtaggaaa atcttgtctt ctactgagat ttgcagatga 240
   tacctacaca gaaagctata ttagtaccat tggcgtagat tttaaaatca ggacaatcga 300
   tttagatgga aagacaatta aattgcaaat ttgggataca gcaggtcagg aaaggtttag 360
   aacgattaca tcaagttatt accgaggagc acatggtatt attgtagtgt acgattgcac 420
   agaccaagat tcattcaata acgttaaaca atggctcgaa gaaatcgacc gttatgcgtg 480
   tgacaatgta aacaaattac tggtagggaa taaaagcgat ttgacaacta agaaagttgt 540
   cgacttcact acagccaagg agtatgccga ccaattgggt ataccatttt tggaaacctc 600
   agctaagaat gcaaccaatg tagaacaggc ctttatgact gtggccgctg aaataaaaaa 660
   tagagtagga cctccatctt ctgcggtaga ccaaggaaat aaggttaggt tcgatcaa 718
<210> 330
   <211> 22
   <212> DNA
   <213> Artificial Sequence
<220>
   <223> primer
<220>
   <221> misc_feature
   <222> (1)..(22)
   <223> forward primer
<400> 330
   gttgggattg ggatttaaca aa 22
<210> 331
   <211> 24
   <212> DNA
   <213> Artificial Sequence
<220>
   <223> primer
<220>
   <221> misc_feature
   <222> (1)..(24)
   <223> reverse primer
<400> 331
   ttgatcgaac ctaaccttat ttcc 24
<210> 332
   <211> 4464
   <212> DNA
   <213> Diabrotica virgifera
<220>
   <221> misc_feature
   <222> (1)..(4464)
   <223> WCRW transcript ta00232.001_diavv
<400> 332
   gaatcaaggg gttgggattg ggatttaaca aatacgtcgt tctaaatttc taaaactgca      60
   tacaaaaaat ccttaaaaaa ctagtgaaga ttactaaagt gcaatagata caaaaacaat     120
   atttttacgt attcaccata ataatataaa cgtagaaaat gaatcccgag tatgattatt     180
   tattcaaact tctgctgatt ggagattcag gagtaggaaa atcttgtctt ctactgagat     240
   ttgcagatga tacctacaca gaaagctata ttagtaccat tggcgtagat tttaaaatca     300
   ggacaatcga tttagatgga aagacaatta aattgcaaat ttgggataca gcaggtcagg     360
   aaaggtttag aacgattaca tcaagttatt accgaggagc acatggtatt attgtagtgt     420
   acgattgcac agaccaagat tcattcaata acgttaaaca gtggctcgaa gaaatcgacc     480
   gttatgcgtg tgacaatgta aacaaattac tggtagggaa taaaagcgat ttgacaacta     540
   agaaagttgt cgacttcact acagccaagg agtatgccga ccaattgggt ataccatttt     600
   tggaaacctc agctaagaat gcaaccaatg tagaacaggc ctttatgact atggccgctg     660
   aaataaaaaa tagagtagga cctccatctt ctgcggtaga ccaaggaaat aaggttaggt     720
   tcgatcaaag tcgcccagtc gaaacaacca aatccggttg ctgctgaaaa tatttagttc     780
   tctttggttg aaggtggtca ctcatgtcat ctcttttgta cggtagcagc gctacagtta      840
   ctcttattct agtaatatcc gtctgtatgt atagttattt taaagttttt gatttgattt      900
   tcactttaga tttttttttc aaaaatatag aaaacaccaa tatctcttag cctaatctta      960
   actcccttag ataaatatgt aattaacaaa aatatttata aaaccatttt tactaaacta     1020
   ttagtccaca gtatatcgtt ccttttcagg aatgattgca aaagtcttaa aaatggtttt     1080
   aattgtttcg tttaaacgag tctaccactt tatgaaaaat ttcagaaaga ttatacgttg     1140
   aaataaaata aattattttt gtcggacaaa aaagaggggc aaacacccgc aagatgtcac     1200
   tctagcgcgg tacagttttg ttaattacaa aactcataat tttcaattga aagtattgat     1260
   aattactaaa tattgtggat ctcttgtccg acaaatttgc agcctcgttt ctttagtagg     1320
   acaacggaaa tatgttaatt atggtaggtt atggtttgcg gttacaaaag tcataatttt     1380
   aattttttaa cgttcaaact gttcaaaaag taattgtccg acaaaaaaat tgttcgacgc    1440
   aaacttataa caaaaacagt gacagtctac ttgtgtttgt cggtataatc gcaaattacc     1500
   tacctctagt atagatgctt agtctagtaa ttagtctgtt ccgctctgcg gaattgagta     1560
   aatggaatta attaaaaata agtgtaaact atgaacaata atatatttat ttgtgtcgat     1620
   aaaaagacgt gttttgctcg aaagggtgtg ttgttgacgc accgcatcta tcccgacgcc     1680
   cgctggggtg tgactgccga cgggaggttg taaatcgacc cgtggttatg tatcgactga     1740
   cgctgtggct gaaaaaaacc acaaacgcta atatgtttgt acttctaaca tctggccaga     1800
   aaagcgataa attactacat ctgtagtttt aagaaacgtc ttcgaatgca tttccatgca     1860
   ttgacatgga atctgataag gtaattaatt catggggaat tcagtttgtt aaactgaaca     1920
   caccaaaaat atggttaggg atacatttcg tttcgtaaaa cttcttagca tctggtttgt     1980
   atattaaatg tgaatttata tgaccgtttt gagaaatgat attaaaaaat aaaattagca     2040
   aaaatagtaa ttaaagcgtg tcgccacaag tctatgttta cattcggttt ctatacattt     2100
   tctgacgttc taaacgtcac tggcataaca gtctaaacag tgcaacaagt aaagggtcca     2160
   gggctatgtt agctcaattt atctagtact gtggcggttg cagtaaaaaa agttttgaaa     2220
   ttaatttgtc ggactggcta tctgataccc aatttttgtc ggacattgct ctatttggat     2280
   aaagaaccct ccaatctatc actttcgtgt tcgaaatttt ataatcgcaa tctgcataat     2340
   tgttaattat taacatattt gcgttgttcg gctaaagagg ggaggctgca aatttgtctg     2400
   acaagagatc aacaatctag taattaatta ttaataaact aaaagtgggt agttccctgg     2460
   gttggctgta taccatgtag ttaaaaaact ctaaacatgt agtaaggcca cttctgtgta     2520
   aaaggtgtat gtactgaaat tttaaaaatc ccgatggatt gaataaacta ggttcattca     2580
   gaacgttttc ggacctgtca gtccatcatc agtgaattca tatatacttg cgctaactag     2640
   tcccagaacc aaacaatggt tgtacttata aatcaatcta cattatagtg ttatgtattt 2700
   ggaaaggcta agcgccgatg ttctgatgga ctgataggtc cgaaaacgtt ctgaattgga 2760
   acctatttta atcaattcat tgggattttt agggttgtag tagatatacc ttttacacag 2820
   aagtggtttt atttcatgtt ttttgtccga cgaaaataat ttattttatt ctaacgcgta 2880
   attttttgat taaaattaaa aattatagtt ttgcaatctc aaaacaaccg cgctagagct 2940
   acaggtttaa aaggaagtca tcaaaaccat ttttaagact tttgtaatca tttttgaaaa 3000
   acgacgatat actgtggact acatatatga gatcattaga acgagaaaga tatactcatc 3060
   gactgttaat tttgtattgt ttttagtata cctttctctg ttttataaat ccagatcatg 3120
   atagattgac gttagaaatt attgttcgct tttcaaacgt gtattagcag aattttggca 3180
   aattaaatat aaaaattcga gatcctttat aaaaaggtat atttaaaatt ccctaagtaa 3240
   gggttatatt aagtaacaga acgttttcgg attaaaaaat ccaccatcag tgttacaaaa 3300
   aaaaaatagc atgcctgagg caccgaaatg ttacgggtaa aaacccttta aatgttgaca 3360
   gttgttgtct tatactatga tgttgctaat attcctggat attacccagg gcaacacagg 3420
   actcttccca cgcggttgga atttgagaat ttcaatcacg aatttgacac ttccaaaata 3480
   cgtaaagttt tccacatctt taatctgcat gttgtgaata gtaaatagag tgtcgttcct 3540
   tgcattggtt ctcatggact tgattttact aatattaatt ttcaaatcta ttttattggc 3600
   ttcagtggaa agtgaatttt gaaaaaaaat ctgctaaaaa tagtttaaaa aatatttttt 3660
   tttagttgtg acactataca gataaaataa cgaaaaattt acgaaataat atttatcaaa 3720
   agtggctctt gcagtacaga atataatatc attattatta tctagttaat agactcgtac 3780
   tttttaacga tgtaacttta aaaacaatta acaacgttaa aaaaatactg caaaaaggaa 3840
   gataaataag tgataaatgt gtcacttttc ttgagcacta cgcagattgt ttggaataaa 3900
   gctatcacgt attatactgt gtaaactcca caaacgacac tttatacaca tcttagatga 3960
   acaaatgcct ggcacaagtc gatatgtgac actgtttatg ttcggtgcac cgtcagtttt 4020
   ggttaatcat tagtgtcagc taacgaaaat actttaacta taaacttgac actttttgag 4080
   ataagatttt gtagttttat taaactgttg gtgcaataaa accgttttta aaattttttg 4140
   agttgtgaca ctatttgagc ggaggtgcga catgtggaaa atatttacaa ttatttcata 4200
   tatcacttgt tctatacaat aaaaaaacgt catttctaac atgattttta gtgatttctg 4260
   ttggtccaaa acaatttatt atattatcca tatatacttg ttatttttta tgtaatgatt 4320
   tttcttattc gctacacatt aacgtcattt tttgtttata tcaaataaaa agtaatattg 4380
   taagtaatat gtgattgtaa atcttggtca ataggttttt tgtaaattaa agttattctg 4440
   aactggaaaa aaaaaaaaaa aaaa 4464
<210> 333
   <211> 704
   <212> DNA
   <213> Diabrotica virgifera
<220>
   <221> misc_feature
   <222> (1)..(704)
   <223> RNAi target sequence
<400> 333
   ttttgggtgt gaatctgtct atttaattta ctcctcctgg ttaaaaaata aacattagta      60
   gattatatct agggtgctcg gtaaaaggag tataagtcaa aaatggtcaa aatattattt     120
   ttgcagaata agactataaa ggactatatc tttaccttgg tatcaataga acaagtcaat     180
   atgctaacat aaattaaatc aaattaattg gtctttttag tacaactact taaatgaact     240
   tttaatttgg taacaacagt ttatgtcaac ttatactgtt tatacacttc gagtttgtga     300
   actattcttg gctctaatag tacatgtcga catatgtact gttttgacca ttgatactga     360
   ataatatcca agtaagacgg tcgacttata ctaagacgat cgctttcaga tacgtgtgtc     420
   aagcttacta agcaaatatt actcttttct gtacgagatt ttcaagaaac aactgataac     480
   tattcgcgtt tggctgataa ctaaaccgtg ggaattttac caattaaaga ttacacttaa     540
   tagggacagc gagaataagt aatactaact tcttacgtag gtttaatttg ataaaaatag     600
   atgagtaagt ggtttatttt ctggaccact gtgcaatatg ggtgatcgta acaaacgtgt     660
   tgcatatatt gttagcctta tagactccga accacctaaa atca                      704
<210> 334
   <211> 24
   <212> DNA
   <213> Artificial Sequence
<220>
   <223> primer
<220>
   <221> misc_feature
   <222> (1)..(24)
   <223> forward primer
<400> 334
   ttttgggtgt gaatctgtct attt 24
<210> 335
   <211> 22
   <212> DNA
   <213> Artificial Sequence
<220>
   <223> primer
<220>
   <221> misc_feature
   <222> (1)..(22)
   <223> reverse primer
<400> 335
   tgattttagg tggttcggag tc 22
<210> 336
   <211> 737
   <212> DNA
   <213> Diabrotica virgifera
<220>
   <221> misc_feature
   <222> (1)..(737)
   <223> WCRW transcript ta41917.001_diavv
<400> 336
   gggattattc tagtgacttt tttgggtgtg aatctgtcta tttaatttac tcctcctggt      60
   taaaaaataa acattagtag attatatcta gggtgctcgg taaaaggagt ataagtcaaa     120
   aatggtcaaa atattatttt tgcagaataa gactataaag gactatatct ttaccttggt     180
   atcaatagaa caagtcaata tgctaacata aattaaatca aattaattgg tctttttagt     240
   acaactactt aaatgaactt ttaatttggt aacaacagtt tatgtcaact tatactgttt     300
   atacacttcg agtttgtgaa ctattcttgg ctctaatagt acatgtcgac atatgtactg     360
   ttttgaccat tgatactgaa taatatccaa gtaagacggt cgacttatac taagacgatc     420
   gctttcagat acgtgtgtca agcttactaa gcaaatatta ctcttttctg tacgagattt     480
   tcaagaaaca actgataact attcgcgttt ggctgataac taaaccgtgg gaattttacc     540
   aattaaagat tacacttaat agggacagcg agaataagta atactaactt cttacgtagg     600
   tttaatttga taaaaataga tgagtaagtg gtttattttc tggaccactg tgcaatatgg     660
   gtgatcgtaa caaacgtgtt gcatatattg ttagccttat agactccgaa ccacctaaaa     720
   tcactagaaa aaggacc                                                    737
<210> 337
   <211> 221
   <212> DNA
   <213> Diabrotica virgifera
<220>
   <221> misc_feature
   <222> (1)..(221)
   <223> RNAi target sequence
<400> 337
   aatgaaaaac ataataacgg aagccaaaaa attccgactt aaaataaatg aagaaaagac 60
<210> 338
   <211> 24
   <212> DNA
   <213> Artificial Sequence
<220>
   <223> primer
<220>
   <221> misc_feature
   <222> (1)..(24)
   <223> forward primer
<400> 338
   aatgaaaaac ataataacgg aagc 24
<210> 339
   <211> 20
   <212> DNA
   <213> Artificial Sequence
<220>
   <223> primer
<220>
   <221> misc_feature
   <222> (1)..(20)
   <223> reverse primer
<400> 339
   ttgcctttat ctccccttct 20
<210> 340
   <211> 3813
   <212> DNA
   <213> Diabrotica virgifera
<220>
   <221> misc_feature
   <222> (1)..(3813)
   <223> WCRW transcript ta03247.005_diavv
<400> 340
   cttagcgaga tgcgtagaga gcagaaagaa tatagagatg aaataagaga gctacgccag 60
   gagaatgtaa cattaagaga agaaaacacg aaactaaaac aggcagttta tcaaatagaa 120
   gaacggcttg aaagtttaga aaatcaaaaa aggcggaaaa atataattat acaaggattg 180
   aacatagata caaatgggcc agatatccta aaacatgcaa tgaaaaattt tatgcagaca 240
   gaattaaaag tagatgtaca aatagagaag gctctgaagt taggagaaaa agtttgtctg 300
   gttgagcttg aaaaggaaga agataaaaga aaaatcatgc gaaacaaaac caaattgaga 360
   aatcgagaag aaggtctagt ttatatcaac gatgacctat caaagaacga cagaattgta 420
   cagaaacatg tcagacaaaa agcgcaagaa ctaagaaagg aagggagaaa agtgaaagta 480
   gggtataaga aaatatatgc cgatcatgaa atatggcgct gggatggaat gaaccaaacg 540
   ttggtggtag agaccaaagc aaaaaactaa gcaatagcaa tgaaaggaaa caaatgacga 600
   cgaaaaatgc aaagaatagg attacgaact atggaaatag aaacaaatca agacagcaat 660
   aaaaaagaaa agccgaaaaa tgacaagata cagtttgcaa cagggataaa taagacggaa 720
   acgacgcagg caatggacaa ggcaataagt aaagataaca aaaagaaaac taaagtcaac 780
   ttgggtacat ggaatgtaag aggcacatac gaaacaggaa aactcaaaga attaattaga 840
   gaaattaaaa gatataatgt agaaataata gctttacaag agacaaaaca attggaaaca 900
   gaaatagtag aaatagaaga cgtggtattt tttaaaagcg ggggagaaac cagaaggtta 960
   ggaacaggtt ttataataca gcgaaaatgg aaggaaagag taatggcatt ccaaccaata 1020
   tcagatagat tatgcacaat aagattaaaa ggggacaaac gaaacataag tataataaat 1080
   gtacacgcac cgattgaaga tgccacagaa gaagaaaaag atgaattcta cgagcaattg 1140
   gaaagagaat atgataaatt accagggttt gacatcaaaa taataatggg agactgcaac 1200
   gccaaagtcg gaagagagaa tatatacgaa cacataatag ggaaatatag taagcatgag 1260
   gtgtcgaacg ataatggcca aagaataata ggctttgcaa cagaaaggca aatggtgata 1320
   cgaagcacac aattacgccg aaaagatata tataaaggaa cgtggatttc ccctgataag 1380
   aagacagtaa atcagataga ccatatttta atagagaaga agcatgcaaa caatgtaata 1440
   aatgtaaaga gcatgagagg agcggactgt aactctgacc actacctggt gagagcagaa 1500
   tataaaatcg agcataacat aaagcgaaac aataaaacaa cagaaaaaat tggaaaacaa 1560
   tttaacatag gactactaaa agataacaac acacagaaga agtatgaaca gggaataacc 1620
   aacagactaa tcagggaaca agaaacgtca agcccagaca aacaatggca aaatatcaaa 1680
   gaagcaatct tgaacacaag taaagcaatc ctagcgaaaa ccaaaagaaa acacaaaaca 1740
   aaagattggt atgatgaaga atgtcaagaa gtagcagaag caataagaaa ggtaagactc 1800
   aaaaatctca caaatgacga agaaagcact agagaagaat acagagaatt gagaaaaatc 1860
   atgaaaagaa aatgtagaag caaaaagaga aaatacaacg agaacaaact gaaagaaata 1920
   gaagaaaaat ttcaaaaaaa agaaataaga tcattctacc aggaagcaaa aaaaatggaa 1980
   agaggatatc aaaaaaacaa cccatatatg agagatgata aagggatgtt gctaaatgag 2040
   cctgaaaaaa taatggaaaa ctggcaaaac tatttcacag aactgctaaa taagagcgaa 2100
   gtacaaaagg aaacaaacca tgaaattgaa gatgatcaga tagcaataga aatacccaca 2160
   gaacaagaaa taaagaacga aataaagagc ttgaaaaata acaaaagccc aggaataaca      2220
   gaaatatcgg ccgaaatgat aaaatcagga gggaaaagac tacagaaaga gatatatgac      2280
   ctgataaaaa gaatatggga agtagaaaaa atgccagaag agtggaccat agcaagaata      2340
   tgccctatac ataaaaaagg tgatagaatg ctatgcgaaa actacagagg catcgcacta      2400
   ttagaaatag tttacaaaat cttggcacaa agtataagta aaaggctaag tcattattcg      2460
   gaaaaaatac tgggggaata ccaggcaggt tttagaaaca acagatcaac gactgaccaa      2520
   atatttgcct taaaagaaat acagactacc tgttacgaac ataaaacagt actatatgct      2580
   ttgttcatag actttaagca ggcttacgac acagtaaata gacagcagat gtacgaactg      2640
   atgaaagaat tggggatacc aagtaaaatt gtcaggatga taaagatgac gatggaaaac      2700
   acaacaaacg aaatagcttg gaaagggtat acatccaaaa agtttgaaac caaggaagga      2760
   ttacgacaag gagacccact atcaacaatg gcattcaatc taacattgga aggaataatc      2820
   aggaaaagca gaataaacat gcaagaaacg atatttaaaa acggccacca atgcatagca      2880
   tttgcagatg atctgacgct attagcaaca agcaaaaaag aactacaaaa gttaatgaaa      2940
   aacataataa cagaagccaa aaaattcgga cttaaaataa atgaagaaaa gacaaagtat      3000
   atgataatgg gagagatcca aaaagaaaaa gagaataaca tcatagtaca aatagatgga      3060
   gaaaaaacat actcgtttaa aagagccaaa gaaattattt acctgggagc caaaatagat      3120
   gaaaatggtc atgaagaagg ggagataaag gcaagaatag ctaaaggaaa taaaaaatat      3180
   ggagcattac gcacattatt gaaatccaaa tacgtatcaa gaaaaacaaa aataagaatt      3240
   tataagactg ttatcagacc tacagtaaca tacgcatgcg aaacatgggt gctcaaaaag      3300
   tcagaaacag accttttaga aagatgggag agaaaaatgc aaagagcaat atatggaggt      3360
   gtgaaaatag aaggtcagtg gagaagaaga accaataaag aattggaaga actatatcaa      3420
   gagccaacga ttacgacgac gatcaaagca cagagaatac gatacttggg gcacatcgag      3480
   agaatgggaa gtaaacgaat gccaaaaatg gtactctcac gaagaccaat acaaaagagg      3540
   aggaaaggca ggccaaggaa aagatggaag gacagtgtat atgaagactt gaagaaaagt      3600
   aacattgaga gatggaaaga actagcattg gacagaagga gatggagaga ggtggtgaag      3660
   gagtgtataa aaaaactgaa gtgtaattaa ataaaattta taagttatgt aagttatatt      3720
   aagttataaa caatataacc caaggaataa aaaacctaaa cagcttaaca aaaaaaaaac      3780
   aaacactatt gccctgaaca aaaaaagttt tat                                    3813
<210> 341
   <211> 656
   <212> DNA
   <213> Diabrotica virgifera
<220>
   <221> misc_feature
   <222> (1)..(656)
   <223> RNAi target sequence
<400> 341
   tgcaagtaat catggcttca gagagatata gcttttcttt aactactttc agcccctctg      60
   gaaagttggt tcagattgaa tatgcattag ccgctgtagc ggcgggtgct ccatcagtag     120
   gaattaaagc atcaaatggt gtggttattg caacagagaa caagcacaaa tcaattctgt     180
   atgatgaaca cagtgtccat aaagtagaaa tgatcacaaa acacattggt atggtttatt     240
   ctggtatggg tcctgactat cgccttcttg tgagacaagc acgcaagatg gctcagcagt     300
   attacctagt ataccatgaa cccattccta cagtacagtt ggtgcaaaga gtagcgactg     360
   taatgcaaga atacacccaa tcaggaggag ttagaccatt tggagtgtcg ctactgattt     420
   gtggttggga taacaacaga ccttacttgt tccaatgcga tccttctgga gcttactttg     480
   cttggaaagc tactgcaatg ggaaagaatt atgtcaatgg aaaaacgttc ttagaaaaaa     540
   ggtacagtga agagctagaa ctcgacgatg ctgtgcacac tgctatcctt accctgaaag     600
   aaagttttga aggccagatg acagctgaca acatagaagt tggtatttgc gatgct         656
<210> 342
   <211> 22
   <212> DNA
   <213> Artificial Sequence
<220>
   <223> primer
<220>
   <221> misc_feature
   <222> (1)..(22)
   <223> forward primer
<400> 342
   tgcaagtaat catggcttca ga 22
<210> 343
   <211> 22
   <212> DNA
   <213> Artificial Sequence
<220>
   <223> primer
<220>
   <221> misc_feature
   <222> (1)..(22)
   <223> reverse primer
<400> 343
   agcatcgcaa ataccaactt ct 22
<210> 344
   <211> 857
   <212> DNA
   <213> Diabrotica virgifera
<220>
   <221> misc_feature
   <222> (1)..(857)
   <223> WCRW transcript ta03948.001_diavv
<400> 344
   ggtcaaacat caaatccaca agtcatcaac acgaagagga aagttgaaaa ttgtgtttgt       60
   aattgcaagt aatcatggct tcagagagat atagcttttc tttaactact ttcagcccct      120
   ctggaaagtt ggttcagatt gaatatgcat tagccgctgt agcggcgggt gctccatcag      180
   taggaattaa agcatcaaat ggtgtggtca ttgcaacaga gaacaagcac aaatcaattc      240
   tatatgatga acacagtgtc cataaagtag aaatgatcac aaaacacatt ggtatggttt      300
   attctggtat gggccctgac tatcgccttc tcgtgagaca ggcacgcaag atggctcagc      360
   agtattacct agtataccat gaacccattc ctacagtaca gttggtgcaa agagtagcga      420
   ctgtaatgca agaatacacc caatcaggag gagttagacc atttggagtg tcactactga      480
   tttgtggttg ggataacaac agaccttact tgttccaatg cgatccttct ggagcttact      540
   ttgcttggaa agctactgca atgggaaaga attatgtcaa tggaaaaacg ttcttagaaa      600
   aaaggtacag tgaagagcta gaactcgacg atgctgtgca cactgctatc cttaccctga      660
   aagaaagttt tgaaggccag atgacagctg acaacataga agttggtatt tgcgatgcta      720
   atggattcag aaggtttgat ccatctactg ttaaggattt cttagctaat attccttaaa      780
   tttttttata gtttcgattt tttaatatta cttggattat taaatttact ataagtatca      840
   aaaaaaaaaa aaaaaaa                                                      857
<210> 345
   <211> 512
   <212> DNA
   <213> Diabrotica virgifera
<220>
   <221> misc_feature
   <222> (1)..(512)
   <223> RNAi target sequence
<400> 345
   acattcttaa acccggagac aaagtaggag cttccgaagc cacattactc aacatgttaa 60
   acatttctcc cttctcatat ggtcttcaag ttgagcaagt ctatgactca ggcactgtat 120
   ttgctccagc tattttggac atcaaaccag aagatttgag ggagaaattc ttggctggag 180
   tagccaattt ggctgctgtt tgtttgtcca ttggacaccc aacagttgct tctgctccac 240
   acagcattgc caatggattc aagaacttgt tggctattgc tgctgttact gatgttgact 300
   tcaaggaagc caagaccatc aaagaataca tcaaggatcc cagcaaattt gctgctgctg 360
   ctcctgtagc tgcagccgcc gctgcaccag ctgccgagtc taagaaagaa gagaagaagg 420
   aggaatctga aagtgaagat gacgatatgg gcttgggtct ctttgactag aaagttttat 480
   atttttgata cgttatttgc tgttttgtcg ag 512
<210> 346
   <211> 22
   <212> DNA
   <213> Artificial Sequence
<220>
   <223> primer
<220>
   <221> misc_feature
   <222> (1)..(22)
   <223> forward primer
<400> 346
   acattcttaa acccggagac aa 22
<210> 347
   <211> 22
   <212> DNA
   <213> Artificial Sequence
<220>
   <223> primer
<220>
   <221> misc_feature
   <222> (1)..(22)
   <223> reverse primer
<400> 347
   ctcgacaaaa cagcaaataa cg 22
<210> 348
   <211> 1042
   <212> DNA
   <213> Diabrotica virgifera
<220>
   <221> misc_feature
   <222> (1)..(1042)
   <223> WCRW transcript ta20424.001_diavv
<220>
   <221> misc_feature
   <222> (427)..(427)
   <223> n can be any nucleotide
<400> 348
   atacgagtcc ctaaccactt caaagatggg tagggaggac aaggctactt ggaagtcaaa      60
   atacttcacc aaacttatcc aattgctaga agactaccca aagtgtttca ttgtgggcgc     120
   tgacaatgtc ggctcaaaac aaatgcagca aattcgtatt tcattgcgag gaaatgctgt     180
   cgttcttatg ggaaaaaata ccatgatgcg taaagctatc aagggccata ttgaacacaa     240
   ccctgctctg gaaaagatct tgccccacat caagggtaat gttggttttg ttttcacccg     300
   aggtgacttg gttgaaatca gagacaaact attggaaaac aaagtcaaag cccctgctag     360
   agctggtgcc attgctccat tatctgtcat cattcctgcc caaaacactg gattgttccc     420
   tgagaanact tctttcttcc aggctttgag tagccccacc aagatttcca agggtactat     480
   tgaaatcatc aatgatgtcc acattcttaa acccggagac aaagtaggag cttccgaagc     540
   cacattactc aacatgttaa acatttctcc cttctcatat ggtcttcaag ttgagcaagt     600
   ctatgactca agcactgtat ttgctccagc tattttggac atcaaaccag aagatttgag     660
   ggagaaattc ttggctggag tagccaattt ggctgctggt tgtttgtcca ttggacaccc     720
   aacagttgct tctgctccac acagcattgc caatggattc aagaacttgg tggctattgc     780
   tgctggtact gatgttgact tcaaggaagc caagaccatc aaagaataca tcaaggatcc     840
   cagcaaattt gctgctgctg ctcctgtagc tgcagccgcc gctgcaccag ctgccgagtc     900
   taagaaagaa gagaagaagg aggaatctga aagtgaagat gacgatatgg gcttgggtct     960
   ctttgactag aaagttttat atttttgata cgttatttgc tgttttgtcg agtcagactg    1020
   tcaaataaat gaaacaggtc gc                                             1042
<210> 349
   <211> 700
   <212> DNA
   <213> Diabrotica virgifera
<220>
   <221> misc_feature
   <222> (1)..(700)
   <223> RNAi target sequence
<220>
   <221> misc_feature
   <222> (471)..(471)
   <223> n can be any nucleotide
<400> 349
   ggggagtaca atgtcggcta ccagactgga gaacggtgcg ggtgattata ttcaaacaga      60
   attgatatcg ggcatcgatt tcgttaataa aaacaattct aaaggacatt gcgccaatgg     120
   agaaactggg ttaaatgaac acactaaata caagaatgga tttaaaaagt cggtcatttc     180
   caatggacat gtcacatcat cagacgaaga aacgacaaaa aataggacaa aaaatggtta     240
   caaaacagac acatcaacag acgaaccaaa aatccgacaa atcaaagaat ccttcgaacc     300
   gcctacgttg atcaccaaag cactcaccca catcagtttc tacatcctca tgttcttagg     360
   atacctcagc caggcgctgt ttccacctaa gttggtcaaa gaaaaaaaat cgggaaggct     420
   atcgaccgtt gtctgatcgt tttgccgctt tctattccca gtacgtctac nggcgtatca     480
   aggattgttg gaacttaccc atttgcagcg taccagggga tgaagtcgta cttaaagata     540
   ggattacgag agatcattgc tggacctttg aatttacggg gtccaaaacg aaatgcataa     600
   atttggcttc ctacaactac ttggggttcg cagaagccac aggtccctgc gcagaacatg     660
   cgattcggac gatttacacg gatggcatta gtaccggaag                           700
<210> 350
   <211> 18
   <212> DNA
   <213> Artificial Sequence
<220>
   <223> primer
<220>
   <221> misc_feature
   <222> (1)..(18)
   <223> forward primer
<400> 350
   gagtacaatg tcggctac 18
<210> 351
   <211> 21
   <212> DNA
   <213> Artificial Sequence
<220>
   <223> primer
<220>
   <221> misc_feature
   <222> (1)..(21)
   <223> reverse primer
<400> 351
   cttccggtac taatgccatc c 21
<210> 352
   <211> 2113
   <212> DNA
   <213> Diabrotica virgifera
<220>
   <221> misc_feature
   <222> (1)..(2113)
   <223> WCRW transcript ta00669.001_diavv
<400> 352
   ggggacagac accagccgca gacctccagg catgcgctta ttcagcaagg tggtgtgact 60
   gctgtgagat ggctgtgtcg gctgtacgaa attacaggtt agattaggtt tgggttggga 120
   gttttctttg atttgttcgt gtgttgtgcc taaaaacttc agtacaatgt cggctaccag 180
   actggagaac ggtgcgggtg attatattca aacagaattg atatcgggca tcgatttcgt 240
   taataaaaac aattctaaag gacattgcgc caatggagaa actgggttaa atgaacacac 300
   taaatacaag aatggattta aaaagtcggt catttccaat ggacatgtca catcatcaga 360
   cgaagaaacg acaaaaaata ggacaaaaaa tggttacaaa acagacacat caacagacga 420
   accaaaaatc cgacaaatca aagaatcctt cgaaccgcct acgttgatca ccaaagcact 480
   cacccacatc agtttctaca tcctcatgtt cttaggatac ctcagccagg cgctgtttcc 540
   acctaagttg gtcaaagaaa aaaatcggga aggctatcga ccgttgtctg atcgttttgc 600
   cgctttctat tcccagtacg tctacaggcg tatcaaggat tgttggaact tacccatttg 660
   cagcgtacca ggggatgaag tcgtacttaa agataggatt acgagagatc attgctggac 720
   ctttgaattt acggggtcca aaacgaaatg cataaatttg gcttcctaca actacttggg 780
   gttcgcagaa gccacaggtc cctgcgcaga acatgcgatt cggacgattt acacggatgg 840
   cattagtacc ggaagtacca gacaacagta cggaacgtgt gcactccatg acgaactgga 900
   gcagctgata gccgaattcg taggtgccga agattccatc acctttggaa tgggtttcgc 960
   gacgaactca ctcaacattc caacactcgc atcgcctggt tgtctgatat tgagtgacga 1020
   aaagaatcat gccagcttga tattgggtat caaacttaat catcccaccg ttagagtatt 1080
   taaacacaat aatgttaagc atctagaaaa actcctccaa gaagccatct actacggcca 1140
   acccggccaa cccaacaacg agtacaaacc ttggaagaaa atcattattt ttatcgaagg 1200
   cgtgtatagt atggaaggaa ccataggccg ccttccggaa attatagctc ttaagaaaaa 1260
   gtacaaagct tatttatatt tggacgaggc ccacagcata ggggccatgg gcaagcacgg 1320
   caaaggtgtt gtagattatt tcaactgtga tcccaaagat atcgacattt tgatgggtac 1380
   gtttacgaag agttttggag gcgcgggagg atacattgca ggttcaaagg aattcatatc 1440
   tttcattcgc gaacatagtc acgcgtctgg tcacgcttgg gcgatgtcgc cgccagtcgc 1500
   agcacagatc atatcagttc taaagataat catgggcaaa gatggtacaa acgaaggaca 1560
   aaagagaatc gaaaggttag ctaggaattc cagatacgtc aggttgcgtc tcgaacagat 1620
   gggtttgatc gtacacggta accaagattc tccagtagtt cccattttag tttatcgtta 1680
   ctcaaacgta gcggtagtgg taagaacttt aattaaagaa aaaatagcaa cagtaggcgt 1740
   aggatatcct gcaacgccgc ttgcggaatg cagaataaga atctgcatat cggcgggcca 1800
   cacgaaagag cagctggatt atgccttatc agttatagaa aaagtagcag atgaaatagg 1860
   tattaagtat tctagaaaac cgagagatcc aaaaccgatt gattacaata aaatcaaaat 1920
   atatcaggat ttgttttaga gagagacttt tagatgaagc ttcaagagaa caccagcctg 1980
   cgataggagg ttttgttgtt tttataaatt ttataaatgc atttaatatt tgatttcaaa 2040
   agctatttat taagaatcgt ttttggctct gttttatttt aatactttgc aaaataaaat 2100
   ataaaaacag aaa 2113
<210> 353
   <211> 440
   <212> DNA
   <213> Diabrotica virgifera
<220>
   <221> misc_feature
   <222> (1)..(440)
   <223> RNAi target sequence
<400> 353
   gggtaatgaa agattccgtt gtccagaagc cctcttccaa ccatcctttt tgggaatgga 60
   agccaatggt atacatgaga ctacctacaa ttccatcatg aagtgtgatg ttgatatccg 120
   taaagactta tatgccaata ctgtactctc tggaggtacc accatgtacc ctggtattgc 180
   tgacagaatg cagaaagaaa taacctcctt ggcacccagc acaatgaaaa tcaaaattat 240
   tgctccacca gaaaggaaat actctgtttg gattggtggt tcaattttag cctctctttc 300
   tactttccaa cagatgtgga tttccaaaca ggaatatgat gaatctggac catccattgt 360
   tcacaggaag tgcttttaat ttttatctat gtttaagtat attctttata tatcttgttt 420
   tttatatccc ccatcttggg 440
<210> 354
   <211> 20
   <212> DNA
   <213> Artificial Sequence
<220>
   <223> primer
<220>
   <221> misc_feature
   <222> (1)..(20)
   <223> forward primer
<400> 354
   gtaatgaaag attccgttgt 20
<210> 355
   <211> 21
   <212> DNA
   <213> Artificial Sequence
<220>
   <223> primer
<220>
   <221> misc_feature
   <222> (1)..(21)
   <223> reverse primer
<400> 355
   cccaagatgg gggatataaa a 21
<210> 356
   <211> 1465
   <212> DNA
   <213> Diabrotica virgifera
<220>
   <221> misc_feature
   <222> (1)..(1465)
   <223> WCRW transcript tal5851.001_diavv
<400> 356
   ggagggcatt ccagtcttgt gatatttagt gcttacttgg tacagcagtt tcagtgctgt      60
   gctttagaat aatttatttt ttaacattta tatagaaatc aaatactaac caatcaacat     120
   gtgtgaagaa gaagttgccg ctttagtcgt agacaatgga tccggtatgt gcaaagctgg     180
   ttttgctggg gatgatgcac ctcgtgctgt attcccttca attgttggac gcccaagaca     240
   tcagggtgtg atggtaggaa tgggacaaaa agattcctat gtaggtgatg aagctcaaag     300
   taaaagaggt atccttacct taaaataccc catcgagcac ggaatagtca caaactggga     360
   tgatatggag aaaatttggc atcatacatt ctacaatgaa ctcagagtag ccccagaaga     420
   acaccctgtt ctgttgacag aagctcctct caaccccaag gccaacaggg aaaagatgac     480
   acaaataatg tttgaaactt tcaacacccc agccatgtat gttgccatcc aggctgtact     540
   ctccttgtat gcatctggtc gtactactgg tatcgtattg gattctggtg atggtgtatc     600
   ccacactgtc ccaatctatg aaggttatgc acttccccat gcaatccttc gtttggactt     660
   ggctggcaga gatttaactg attacctcat gaaaatcttg actgaacgtg gctactcttt     720
   caccaccaca gcagaaagag aaattgttag ggatattaaa gaaaaactct gctatgtagc     780
   tttggacttc gaacaagaaa tggcaacggc tgctagttcc agttcccttg aaaagagtta     840
   cgaacttcct gatggacaag tcatcaccat tggtaatgaa agattccgtt gtccagaagc     900
   cctcttccaa ccatcctttt tgggaatgga agccaatggt atacatgaga ctacctacaa     960
   ttccatcatg aagtgtgatg ttgatatccg taaagactta tatgccaata ctgtactctc    1020
   tggaggtacc accatgtacc ctggtattgc tgacagaatg cagaaagaaa taacctcctt    1080
   ggcacccagc acaatgaaaa tcaaaattat tgctccacca gaaaggaaat actctgtttg    1140
   gattggtggt tcaattttag cttctctttc tactttccaa cagatgtgga tttccaaaca    1200
   ggaatatgat gaatctggac catccattgt tcacaggaag tgcttttaat ttttatctat    1260
   gtttaagtat attctttata tatcttgttt tttatatccc ccatcttggg gcaagaactg    1320
   cctatgttat atgagtgatc ttgtgattta ttacacgttc agcataagac ttaatgagct    1380
   ttaattttaa gaagtgtaag ttgaatactc tgtttcatat ttattttacc tttgtaatat    1440
   aataaacaaa aaaaaaaaaa aaaaa                                           1465
<210> 357
   <211> 600
   <212> DNA
   <213> Diabrotica virgifera
<220>
   <221> misc_feature
   <222> (1)..(600)
   <223> RNAi target sequence
<400> 357
   gttcgctagt tccgtagtgt gttgtgcaca tttaacatcg ttctggcctg tagatactcc        60
   taataactta aaaaatggca gatgacaaac accccgtcta tggacccttc tttggagtca       120
   tgggagccgc ggctgctatc attttcagtt cgctgggcgc cgcctatggt acggcaaaat       180
   caggaaccgg tattgccgct atgtcggtca tgaggccgga acttatcatg aaatgtatta       240
   tcccagttgt catggcgggt atcattgcca tctacggtct ggtcatagct gtactgatct       300
   ctggtaacat ccaagacagc tctacatatt cattgtacag tggttttgtc catttgggtg       360
   ctggtctttc agtaggattt tcaggattgg cggcaggttt cgctattggt atcgtaggtg       420
   acgctggtgt aagaggtacc gcacaacagc caagactctt cgtaggaatg atcttgatcc       480
   tcattttcgc cgaagtatta ggtctttatg gtctcatcgt cgccatttac ctctatacta       540
   gagcttaagc gcccagctcc cctaaatttt catctcctaa acacagcgta ccaaaagttg       600
<210> 358
   <211> 23
   <212> DNA
   <213> Artificial Sequence
<220>
   <223> primer
<220>
   <221> misc_feature
   <222> (1)..(23)
   <223> forward primer
<400> 358
   gttcgctagt tccgtagtgt gtt 23
<210> 359
   <211> 22
   <212> DNA
   <213> Artificial Sequence
<220>
   <223> primer
<220>
   <221> misc_feature
   <222> (1)..(22)
   <223> reverse primer
<400> 359
   caacttttgg tacgctgtgt tt 22
<210> 360
   <211> 2938
   <212> DNA
   <213> Diabrotica virgifera
<220>
   <221> misc_feature
   <222> (1)..(2938)
   <223> WCRW transcript tal5854.001_diavv
<400> 360
   ggacctgaaa ttggcggatc gtcatatggg ggattcttgc ggggggcagt cgtgtaagcc      60
   atgggggtca gtgggttcgc tagttccgta gtgtgttgtg cacatttaac atcgttctgg     120
   cctgtaaata ctcctaataa cttaaaaaat ggcagatgac aaacaccccg tctatggacc     180
   cttctttgga gtcatgggag ccgcggctgc tatcattttc agttcgctgg gcgccgccta     240
   tggtacggca aaatcaggaa ccggtattgc cgctatgtcg gtcatgaggc cggaacttat     300
   catgaaatgt attatcccag ttgtcatggc gggtatcatt gccatctacg gtctggtcat     360
   agctgtactg atctctggta acatccaaga cagctctaca tattcattgt acagtggttt     420
   tgtccatttg ggtgctggtc tttcagtagg attttcagga ttggcggcag gtttcgctat     480
   tggtatcgta ggtgacgctg gtgtaagagg taccgcacaa cagccaagac tcttcgtagg     540
   aatgatcttg atcctcattt tcgccgaagt attaggtctt tatggtctca tcgtcgccat     600
   ttacctctat actagagctt aagcgcccag ctcccctaaa ttttcatctc ctaaacacag     660
   cgtaccaaaa gttgcgcatc tcgccccgat cattttgttt ttaatgtcta catgtaaaaa     720
   taaaaactgt ataaatggta gcaggcgatg aaagtgactg aggcgggcaa tgtaaataat     780
   ttagttatga tggggcccgt atttttcgcg tacggggcaa aacaaattaa taattttagt     840
   aaaaaagact gggcctcttc atcgctgctg tgtccacaca cacgactaat aattccttat 900
   ttccatattc atggaaccgg tcgagttttc ttgacaggtt tttgtattaa aacgaatttt 960
   cttcctaggc aaaataaagg tcttagttac taataggttg ttcaaatgaa aatgtacgaa 1020
   acgacactgt gggtataaat gaagtcttta ttcaaagtat tcaccattta cctgagcaca 1080
   acgatgccgt tgattagccg aaggattcat tgatcccaga actcctgtga ccatgaacag 1140
   tcttccccgt atacatccgg cgttgaatag caactggttt aactaacacc ttccgccgtc 1200
   agaaacctga taacgcctca cgaattttgc aacatgaacc ccatgatgtg cccacacgaa 1260
   ctgtcgcgtc aattgaacgg ctctcttata agagcctcta ttctctcctg cgcatgcggg 1320
   cgcccgggaa tgccccgatc gaatccgaag gctacatttg cctccctgga tgtctcacta 1380
   cgttatccca taacggcata ggcaattatg tttgcggtta agttgttacg acgtttcgtg 1440
   ccttttcatt tgatcagtcg cagtataata tacaagagaa gagggaaaac aaattttgat 1500
   ttattttgcc tcgtacgtta caatacctgt gctatcattg taaataattt taaacatact 1560
   tagaagcctc tatttcattt tcatggcggg tcaccactat tattaaatta attgtatagt 1620
   tagtagaatt taggttaccg tttttctttt aatataaaag ctagactcta ttgagcctag 1680
   gaagtatttg aataaagtta aaaaagtagc acaatttatt tttaatttca caaaaatact 1740
   gtacatttgt tttatcatta gtgtgttata cttgatatat cattttcatg gccttacgaa 1800
   ggcttggtct ctttatacaa caaatggttc gggaatgtta tttttgacaa tttttttgaa 1860
   tagcaagttt taagccagta aaactgtggt attttttttc aatgcatcat cttttttaat 1920
   aaaaaaaact ttggttaatg gggcattaca tcgcaaaacc gtaatttcta aataatatcg 1980
   tcttaattta tttatctaac aatgtactgt ctaaataaca ttcgtaaacc ctgtacattt 2040
   ttttaatagt caatttttat ttgttcccat cctcatcagt tttgcttacc tttttttctc 2100
   tctcaaaaat atgatttcaa aataatctat gttccattac cttccaaaaa taatataaaa 2160
   cggttttatt atatcagcat tgttgtaaat atgacaactt atgtgtatcg atggtttttt 2220
   tgatgtccat agctgtacgt gttagcctaa agtgtagtca tagctttggc aaaaaacatc 2280
   cccgttttca acactgacga aacttctatt taatagacag acggtgatgc tttttgactt 2340
   tagcagataa gaatgttata cagggttaac aaaaatgaga atactacgtc tgatatatcc 2400
   tgtaacacac aaaatacttt aaagctcagt tacacgatgc taataacttg ttttactgga 2460
   atataaaaca ccaatttaca caaggagagt actaaaagtc agtacatact cggttttagt 2520
   gactcacaag tgttactagc atggtgtaac tgagccttta tcctggcatt ccaattactg 2580
   gacaatgcta tcatataaat accatgccat ctaaataggt aatctacttg ttagtcctaa 2640
   gagtttatca ctatttaagt aaacgatgaa ttgaattggc tgttttgtgt tacaaggcta 2700
   gtaagttttc tcttttcaag tcaaccctgt agtatctcct ttttatttta gtcatgaatc 2760
   atcatctcag cacgaagctt gttgggcaag tgtctgccgg ttttcggtgg attcggtcca 2820
   tcgaaatagg cagacattgt gtcctattgt tgtagagtat tatattataa tgtagattgg 2880
   ttctgttgta agaagggaac agcaaaatta tgttgaaaat aaataatttg tacattcc 2938
<210> 361
   <211> 525
   <212> DNA
   <213> Diabrotica virgifera
<220>
   <221> misc_feature
   <222> (1)..(525)
   <223> RNAi target sequence
<400> 361
   gggatctcca tcttcctttt tcatttagtg taaagcatgc ctttccaaag gtttgtagaa 60
   accggccgcg tggccgttgt atcggaaggc ccacataagg gcaaacttgt tagtattgta 120
   gacgtaatcg accagacaag ggttttagtt gatggcccca atagcaatgt acccagagga 180
   cagcttaggt taaatgaatt gcatttaaca aagttccgca ttaggtatcc attttcagct 240
   ccaactcgtg ttgtaagaaa agcatggact gatgctaaga ttgatgaaaa atggaatgaa 300
   tccgtctggg ccaaaaaagt agcagccaag gaaaagagat cacaacttac agatcttgac 360
   cggttcaaac ttcgtagggc tcgttcacga agaaaccaca tccgtacagc tactttcctt 420
   tctttaagga aggccgcatc aagaaacggt ttcctctatg gtaaaaagaa gatggctaaa 480
   ggaggtgaca aacctaaacc aagcaagaag gcgaagaaag aaaag 525
<210> 362
   <211> 22
   <212> DNA
   <213> Artificial Sequence
<220>
   <223> primer
<220>
   <221> misc_feature
   <222> (1)..(22)
   <223> forward primer
<400> 362
   gggatctcca tcttcctttt tc 22
<210> 363
   <211> 22
   <212> DNA
   <213> Artificial Sequence
<220>
   <223> primer
<220>
   <221> misc_feature
   <222> (1)..(22)
   <223> reverse primer
<400> 363
   cttttctttc ttcgccttct tg 22
<210> 364
   <211> 689
   <212> DNA
   <213> Diabrotica virgifera
<220>
   <221> misc_feature
   <222> (1)..(689)
   <223> WCRW transcript ta01695.001_diavv
<400> 364
   aagaatacga agagctacta acattactta aataaactat agtttaagat tttaatctgt     60
   caacaactgt gatagccagg ttcctttttc atttagtgta aagcatgcct ttccaaaggt    120
   ttgtagaaac cggccgcgtg gccgttgtat cggaaggccc acataagggc aaacttgtta    180
   gtattgtaga cgtaatcgac caaacaaggg ttttggttga tggccccaac agcaatgtac    240
   ccagaggaca gcttaggtta aacgaattgc atttaacaaa gttccgcatt aggtatccat    300
   tttcagctcc aactcgtgtt gtaagaaaag catggactga tgctaagatt gatgaaaaat    360
   ggaatgaatc tgtctgggcc aaaaaagtag cagccaagga aaagagatca caactcacag    420
   atcttgaccg gttcaaactt cgtagggctc gttcacgaag aaaccacatc cgtacagcaa    480
   ctttccttgc tttaaggaag gccgcatcaa gaaacggttt cctctatggt aaaaagaaga    540
   tggctaaagg aggtgacaaa cctaaaccaa gcaagaaggc gaagaaagaa aagcctgcat    600
   ccaagaagaa ataattttta gattaaatga caatatattt gtttaaaaaa caaaaaaaaa    660
   aaaaaaaaat agaaaccatc attaactat                                      689
<210> 365
   <211> 551
   <212> DNA
   <213> Diabrotica virgifera
<220>
   <221> misc_feature
   <222> (1)..(551)
   <223> RNAi target sequence
<400> 365
   ggggatcttt agtcagtgct ttagaggcca cgaagcaatt tctaggatag acgaacgacg 60
<210> 366
   <211> 19
   <212> DNA
   <213> Artificial Sequence
<220>
   <223> primer
<220>
   <221> misc_feature
   <222> (1)..(19)
   <223> forward primer
<400> 366
   gatctttagt cagtgcttt 19
<210> 367
   <211> 21
   <212> DNA
   <213> Artificial Sequence
<220>
   <223> primer
<220>
   <221> misc_feature
   <222> (1)..(21)
   <223> reverse primer
<400> 367
   tggggctgta gttgagattg a 21
<210> 368
   <211> 6974
   <212> DNA
   <213> Diabrotica virgifera
<220>
   <221> misc_feature
   <222> (1)..(6974)
   <223> WCRW transcript ta35139.001_diavv
<400> 368
   acaatacgac accacgcgcc attttgccat tttctgtgtg tgcagtgaag tgaagtgcta 60
   ctaaaatctt caaaattcaa cccttctaag aaggccgatt tcatgtgttc tatatcgaag 120
   taaaagagca atagcatggg agtggcagag gatttcgctc ccagcttcac gcaaaagcct 180
   caattgaggc aggaggacga tggaaacaaa ctcattttcg aatgccagtt actggctgct 240
   ccgaaaccgg aaatcgaatg gtttcgaagc gatataccac tttcagaaga cagtagaact 300
   aattttaaaa ttcaatccat aggcaccaac aaatttttag tagtactcga attagatgat 360
   gttattgaaa ccgacgctgg cctttacaag gtcaaagcga aaaataccat gggggaaata 420
   gcagcctcca tcaatctcaa cttcagcccc atggacgaac caaaagaaaa acaaatagac 480
   ggcctagcac ccacttttgc gaagaaacca gctattcgcc aagaagatga tggcaaaaaa 540
   ttattattcg aatgtaggat acaggccgat ccccgtccaa ccgtcagttg gtcccacaat 600
   ggcaacgctg ttagcgaagg tccacgtcac aagttgagga tagataaaga tggccattca 660
   tattttgcga cccttgaaat aatcgatgtc acggtagagg atgctggcaa atacaaggtg 720
   accgcaaaaa atgacttggg agaaagtaac gccacaatca gccttaactt tgacagtgga 780
   gatagcgctg atggctttgc gccttctttc cttgagaaac ccaaaatcat acctaatgag 840
   agtggcactc ttattactat gaaatgtaaa tgcaaagcta aacctaaacc tgacgtcacg 900
   tggttccgcg gaaccacagc cgtcaaggaa tcttccaaaa ttaaaatcca gatcgttgat 960
   ctcgaagaag acaaattcga actgtcctta gaaatcaagg atccatcggc agctgatggg 1020
   ggtacttaca gatgccatgt gaagaacgaa tacggagaaa gtaatgcaaa tctgaaccta 1080
   aatatcgaag cagaaccaga accagaagga gaaggaccaa cgttcgtcga aaaacccagg 1140
   ataacctctc acgatggagg caaactcgtt gtcatggagt gtaaagttcg tgctaatcct 1200
   aaacctacta tagtttggta cagagaaagc aaagaagtca cagaatcatc caaaattaag 1260
   atcagtatta aacaaacaga agaagatata tattacgtca aattggaact caatgatccg 1320
   gggattgatg actctggctt gtacaaatgc aatataagga acacacttgg tgaactcaac 1380
   gccaacctca ccttaaacat cgagattatt cctgttatca aagaaaaacc caaagttatt 1440
   aaaatcatta agaagaaaac tgttattgtt gaatgtaaag ttctcagcaa gtttgcacct 1500
   gattgtacat ggtttaagga aagcgatgcc gttaaagaag attcaagaca tactgttcac 1560
   gttgaccaag ttaaagacgg cgaatttact gttaaactcg aaattaatga agttgagaaa 1620
   aaagacaaag gtatgtacaa attggttgct aaaaacgaaa agggtgaggc aacttcacaa 1680
   gtcgttgaag tcactgagtt acctccagag gagaaaccca aaggagacaa gccgaaactg 1740
   accaaactaa ccaatatcgt tactgacgaa ggaaaatcag ttgattttat aacttctctc 1800
   aaaatcgaag acaaaacagt caaaatcaca tggtacaaga acaccactgt gataaccgaa 1860
   tcttcagaaa tcaaaatctc ttttgatggc actgtgacgc gacttagcat tagtaaatgt 1920
   aaagtatcac attccgctac atacaagtgc gttgccaaaa acgaatttgg cgaagacgaa 1980
   ataagcgcta cacttaaagt aaacgaagct aaagaggaag atgaagaaga agaagaatcc 2040
   gaagaggagg ttatcgaaga aaagaaagag gaaaagaaag aagaaaagaa agaagaaaaa 2100
   caggaaaaga aagttgaaga gaaaaaagaa gaaaaaaggt agaaaagaaa gaagagaagg 2160
   ttgaaaagaa agtagaaaag aaagaggaaa agaaaacaga agaaaagaaa gcagttaaaa 2220
   agatagaaaa gaaagaggaa agaaaagaag aggagaaaat gataattaag aaggacgaag 2280
   aaaagaaaaa agacgaaaag attgtggcca aaaaggaaga ggaaaagaaa gatgttgaga 2340
   agaaaataag gcgtacatcg gttcaacaga acgaagaagt aagaaccgaa tcacgcagaa 2400
   gctcaattat acaaaccgaa gaaaaagtta ttaaagatgg aaaagttagt aggaaatctt 2460
   ccatttcaaa agtcaatgag gagagtaaga ctgagtccag gcgcacatca ttagtagatg 2520
   acaagaaaaa gataaccaaa gataccaaaa aattggaaac cacaaaagaa gtgtcagaag 2580
   aaaagctggg agtagccgaa gttaaaaagc tggaaaaacc taaaagggta atacccccta 2640
   aagaagaagc tcaaaagggt tcgttcttga acattcaatt gaaaccagtt caatcggagg 2700
   gtaaaaaagc tgaaaaggcg aataaacttg aagttgactt gaagaaagga gcagttgaag 2760
   agaaaaagac aacagtcaaa aaatcttctg ccaaaaaaga agattatgat attaatgctt 2820
   gggaaaatat ccctgattac gacagaccag tgttagagaa attcgaaaaa catgatgctc 2880
   cagaatatgc tggtagagaa aagagtaaat tctctaagga aagaccatct atctcagtcg 2940
   aaggaaaaga aaaagctcct tctcagtcag ctatatctga acaacctaga cctagccttc 3000
   ctcaaattaa gactccagat gcacccaaaa tcgaagtgag tagggaaaaa actcccgaac 3060
   ccagaaagaa atctctcgag cctggctcag gtccaggaag tcgtagagga tctcttattc 3120
   ctccagaagc catgggcaga cgtgccagtc ttatcatctc cgatgaggga ggaaaattac 3180
   gtcctgggga agtgctagat ccaaagcgtc gtcgtccgag ctccgacgta cgtcgaccaa 3240
   gtttagctga attggaggat ctcgtgaaca aaccctcgac tcctttgaaa ccttccggtc 3300
   caaagggagc agcacctcaa attgtcgatg tccaagaaaa ttactcctca gttgaagatc 3360
   aaactgctta cattactatc caagttgagg gtgatccacc accgacattc aaattctata 3420
   agggtatgac tcaacttatt gagggtggta gattcagatt tgtaaccgat ggtgaaacaa 3480
   acttgatcac actctgcatt agaaaggtaa agcccaatga tgagggtacc tataaagttg 3540
   tcatagccaa cgtccatggt gaagactccg ctgaaatgac cctcttcgta gctggtgctg 3600
   gaggtgttga tttccgttct atgctcatga agagaaagta cgccaaatgg gataaggata 3660
   aggaagaccc caactgggga gacttaaaag aggttgaaaa accagtgcca gcattaaaaa 3720
   aagtcgaaaa gaaacaagaa agtttcctta aaccattggt agatcagttc gctaaagaag 3780
   gaaaagataa gaaagttgtg ttcgacgctg gtttctcaaa aaccaactgt aaaccaaaat 3840
   ggctgttcag gaaagacgaa cttttcccag gatctaaata caaatttaag aatgaaggtg 3900
   acgtgtacca gctgatcatc agcaatccta aagtagagga tactggaaaa tacacaattg 3960
   aaatcggagg agtcagctct accggtttcc tgacagtaga tgaaccagat ccatgttaca 4020
   cctttaccaa agcactgaag aaaaaatatg atggatacac caaacatgaa gttgaactgg 4080
   cttgtactgt aagcagtagt ttggccattg ttggttggta taaaggagag acaaagttgg 4140
   aagataatga taaatttagt atctctaaag atattaacgg agtctgtaga ctgactatta 4200
   aagatgcgac atttgatgat gctggtgatt acacctgcaa actagaaaaa caaaccgaaa 4260
   agacacaaac taaagttaat attgtagact acccatataa attcgtgaaa gtacttaagc 4320
   atcaacaact tatagaaaaa gaaaatgtta cattagtatg tgagctcaac gatgctggtg 4380
   gtgatgtaca gtggtttaag aacggagaag aaatcaaacc agataaacga gtagttatca 4440
   gtaaagatgg tcgcaaacgc aaactggtaa ttaaagacgc caaagttact gatgctggta 4500
   tgtactcttg tactaccaat gcagacaaaa ccgaagctga gcttattgtg aactacttga 4560
   accgctttaa caaaaaactg aaagacactg ttgctgttga acgagaaaaa ctagttttgg 4620
   acattgaact acaagatcaa acagccccgt gcgagtggtt gttcaatggc gaaccaatca 4680
   agccctctga gagagtcgag atcaaaaact tgggaggtgg caagcaccaa ctcatattta 4740
   atggtttaga tatggcagat gacggtgaaa ttaagtgtgt atcaggaaaa cttgaatctt 4800
   ccatgatgct ttccgtcaaa aaaggtgaaa gcaagcctga aattgacttc ccatcagact 4860
   tcgctgcacc aattagcaaa cctattgtcc ttgaagtacc atacaaaatt agtggaacca 4920
   gacagacacc tttggaagct aaacttatca aagacggaaa ggtactacct ctaaaagacg 4980
   tagagacaat aatttcagac gacaaagtca tattcaaaat taaaaaacca tccagagacc 5040
   aatctggtcc ataccagatc aaattatcga acgggcaagg tgaggatgtt aaagatgtca 5100
   agatcaccat gcaggacgtt cctgctccac cagaagacgt taatgtaaca gatatattcc 5160
   aagacaactg taaaatatcc tggaaaccac ccaaagataa tggtggagca cctcttctcc 5220
   attatgttgt tgaacgacaa gatctcagtc tcaaagctgg ttgggaaaat gttggtgaag 5280
   taaaagccaa tgaaccaact acattcaaag ttgaaaaact aattcccaag aaagaatata 5340
   aattcagaat cagagctgtt aaccagctag gtcctagcga acctgctcta ttcggtaaac 5400
   cagtattagc taaagatcct tgggatgaag ccagcaagcc taaagatgta gaagtagttg 5460
   actgggatgt tgaccacgca gacattactt ggaagacacc tgacagcgat ggtggagctc 5520
   ctattactgg ctacgttatt gaattttaaa gaaaaattcg ccaaagattg gaaaaccggt 5580
   gttgaagtac caggtgacca actgaaagga actgttccta aactcaagga aaacagtcag 5640
   tacgaattta gagtaagggc tattaataag gctggaccag gagaacctag tgatcccacc 5700
   aaacctatca tagctaagtg tagattcgta aaaccattta ttattggtaa tgacttaaac 5760
   agtattatta tcaagaaggg acaagttgta aagtacgata ttaagtacgg gggtgaacca 5820
   gaacccgagg tgcaatggtt cctcgataaa aaagaaatca aacccgactt ggaagaaaga 5880
   gttactatcg acaaatatga cagaaacact attattacag tacgcagatg tacaagagct 5940
   gacagtggaa aatatcgttt ggtacttacc aacagctctg gtacttgtga aggggtggcg 6000
   gaagttattg ttttagataa acctacacca cccaaagcta tgaaagtcga ggaaatccgt 6060
   gcagatcacg ttaaagttgc atggcaaaaa ccagaagata tgggtggtac tgacctaact 6120
   ggttatatat tagaaaaaat ggatatggac acaggaagat ggattcctgc aggtgaagtt 6180
   ggtcctgatg aatcaaactt tacgtttaag aacctgacac ctaagaagaa atacaaattt 6240
   agagttcgag ctaaaaataa agaaggagaa tctgaacctt tggaaaccga tgactttatt 6300
   ctagccaaga atccttacga tgagcccgga gcacctggac aaccagaaat ctttgactat 6360
   gacaacaaga gtgccagcct taaatgggct aaaccagata gcgatggtgg gcgccctatc 6420
   acccattaca cagtcgaaat gaaagataag tttgcagctg attggactga ggtagccaag 6480
   acgcctgacg ctactcctga agtggtcgta cctggtttaa aagaaaatat ggtatatcaa 6540
   ttccgtgtga aagcacacaa taaatcagga cctagtgtac cttccgcacc aacagctaac 6600
   cacctctgca aacacaaaaa cttaaaacca agaattgatc gtgaaacatt tagagctgtc 6660
   actataaaag ctggccgtac tcataagtgg gcagttgact ttatgggaga gcctccacca 6720
   tctgtcaaat gggtatggag agatgacatt ccattatcaa atagtgaacg tatcaagata 6780
   gaaaactccg actaccatac ggacttcaca ctcctcaacg ctgttcgtaa agactcagga 6840
   aaatacacgc ttactattga aaacgttaac ggaaaggata ctgaaacagt tgaactgact 6900
   gtattgggca aaccagcagc ccctgaagga cctttagttg tatctgatgt gactgcagaa 6960
   aaagctaaag ttag 6974
<210> 369
   <211> 586
   <212> DNA
   <213> Diabrotica virgifera
<220>
   <221> misc_feature
   <222> (1)..(586)
   <223> RNAi target sequence
<400> 369
   gggggagtag tcggtatgta tttgaattta tcttaatttg tttaatatta gtagttaaat 60
<210> 370
   <211> 19
   <212> DNA
   <213> Artificial Sequence
<220>
   <223> primer
<220>
   <221> misc_feature
   <222> (1)..(19)
   <223> forward primer
<400> 370
   gagtagtcgg tatgtattt 19
<210> 371
   <211> 22
   <212> DNA
   <213> Artificial Sequence
<220>
   <223> primer
<220>
   <221> misc_feature
   <222> (1)..(22)
   <223> reverse primer
<400> 371
   atttctcatc tcctggtttg ga 22
<210> 372
   <211> 683
   <212> DNA
   <213> Diabrotica virgifera
<220>
   <221> misc_feature
   <222> (1)..(683)
   <223> WCRW transcript ta06472.004_diavv
<400> 372
   ggagtagtcg gtatgtattt gaatttatct taatttgttt aatattagta gttaaatagg      60
   aaactatagt tagaagttgt tatatatttg actattagtt gactccccaa aatgcagatc     120
   ttcgttaaaa ccttaacggg taagaccatc actcttgagg tcgagccctc agatactatc     180
   gaaaatgtga aagctaaaat ccaggataaa gaaggaattc ccccagacca gcaacgtctc     240
   atcttcgctg gaaaacaact cgaagatggt cgtaccttgt ctgactataa tattcaaaaa     300
   gaatcaaccc ttcacttggt gttgagattg agaggaggtg ctaagaaacg taagaagaag     360
   aattactcca cccccaagaa aatcaagcac aagaagaaga aggttaagtt agctgtattg     420
   aaattttata aggttgacga aaatggtaaa atccaccgat tgagacgtga atgccccgct     480
   gaacaatgtg gagctggtgt cttcatggca gccatggaag acaggcatta ctgtggcaag     540
   tgcggttaca ctcttgtctt ctccaaacca ggagatgaga aatagatata tgtccttgta     600
   tattgtttaa gaaaaaaata gaaaaacctt tgtttatttg aataaaatat tcgaggaaat     660
   atatctattt ctcatctcct ggt                                             683
<210> 373
   <211> 611
   <212> DNA
   <213> Diabrotica virgifera
<220>
   <221> misc_feature
   <222> (1)..(611)
   <223> RNAi target sequence
<400> 373
   ctccgaagac agacagtcga ttttattcag tgattctgat tgttcagttt ttgtagttgt 60
   acagttttcg tattaggaaa taaatcaaat atggcatcag gagtaactgt agcagatgcc 120
   tgcaaaaagg tccatgaaga aatcaaaagg gacaaaaaac acagatttgc aattttctac 180
   atcaaggatg gcaaacagat tgatgttgaa cacataggag aaagaaatgc agagtatgat 240
   gatttcctaa ctaaactgca agagggagga gctaatgaat gtcgttatgg acttttcgat 300
   tttgaataca tgcaccagtg ccagggaacc tcagagtcaa ccaagaaaca aaagttattc 360
   ctgatgtcgt ggtgtcctga aactgcatct gtcaaaaaga agatggtgta tgctagctca 420
   ttcgaatatc ttaaaaaagc tttggtaata cagaaatcta tccaagcgac agatttagcg 480
   gaagcgagta taacagcggt agaagaaaaa ttacgatcca ctgatcgtca gtaacgcctt 540
   ttatttttat tatttttctt tttgtatctc gactgtctac tcgcgcgccg acctgccgag 600
   gtgtattatt t 611
<210> 374
   <211> 21
   <212> DNA
   <213> Artificial Sequence
<220>
   <223> primer
<220>
   <221> misc_feature
   <222> (1)..(21)
   <223> forward primer
<400> 374
   ctccgaagac agacagtcga t 21
<210> 375
   <211> 22
   <212> DNA
   <213> Artificial Sequence
<220>
   <223> primer
<220>
   <221> misc_feature
   <222> (1)..(22)
   <223> reverse primer
<400> 375
   aaataataca cctcggcagg tc 22
<210> 376
   <211> 1099
   <212> DNA
   <213> Diabrotica virgifera
<220>
   <221> misc_feature
   <222> (1)..(1099)
   <223> WCRW transcript ta00957.001_diavv
<400> 376
   gggaaatcgc gagaatacgg gcgaggtttg aagtttttaa ttttttgttc atatcaccac      60
   ttaaagctat tgttatcatt ttagcatata taaaacaata gcttctgtgt tggggattaa     120
   cagtcgcggt tgagctaaaa aatatatatt tgttaatgtt ataaataata ttcaaaaagt     180
   ttgtttaatt caaaactggc gcatttcgta cgcatgaaaa aatagttcga attgacattg     240
   gaaacatttt gatagttgtg gtgacgtttg ctgtaaatct atttaatttt tggtgattta     300
   ataattgaaa atatttctgg gaattaacgt agtttctaga aaaaatatcg gtgacttatt     360
   ttggcgtagt aaaattgact cgcgcaggcg tacccccagc tgccggtagg acaacgtccc     420
   caacagatct ccgaagacag acagtcgatt ttattcagtg attctgattg ttcagttttt     480
   gtagttgtac agttttcgta ttaggaaata aatcaaatat ggcatcagga gtaactgtag     540
   cagatgcctg caaaaaggtc catgaagaaa tcaaaaggga caaaaaacac agatttgcaa     600
   ttttctacat caaggatggc aaacagattg atgttgaaca cataggagaa agaaatgcag     660
   agtatgatga tttcctaact aaactgcaag agggaggtgc taatgaatgt cgttatggac     720
   ttttcgattt tgaatacatg caccagtgcc agggaacctc agagtcaacc aagaaacaaa     780
   agttattcct gatgtcgtgg tgtcctgaaa ctgcatctgt caaaaagaag atggtgtatg     840
   ctagctcatt cgaatatctt aaaaaagctt tggtaataca gaaatctatc caagcgacag     900
   atttagcgga agcgagtata acagcggtag aagaaaaatt acgatccact gatcgtcagt     960
   aacgcctttt atttttatta tttttctttt tgtatctcga ctgtctactc acgcaccgac    1020
   ctaccgaggt gtattattta tataagtatt tgaatattta ataaagcttc atttataaaa    1080
   aaaaaaaaaa aaaaaaaaa                                                 1099
<210> 377
   <211> 357
   <212> DNA
   <213> Diabrotica virgifera
<220>
   <221> misc_feature
   <222> (1)..(357)
   <223> RNAi target sequence
<400> 377
   aaaatgtcca agagaggacg tggtggttcc gcgggagcga agtttaggat ctcccttgct        60
   cttcctgtag gagcagtaat taattgtgca gataatacag gagcaaagaa cttatatgtt       120
   atcgctgtac aaggtattgg tggacgactt aaccgtctgc cagctgcagg atctggtgac       180
   atgattgtag ccacagtcaa aaagggtaaa ccagaactca ggaaaaaagt catgcctgca       240
   gtagtcatca ggcagcggaa accatttaga aggaaggatg gtgtcttcat ttactttgaa       300
   gataatgctg gggttatagt caataacaaa ggtgaaatga aaggatcagc catcact           357
<210> 378
   <211> 22
   <212> DNA
   <213> Artificial Sequence
<220>
   <223> primer
<220>
   <221> misc_feature
   <222> (1)..(22)
   <223> forward primer
<400> 378
   aaaatgtcca agagaggacg tg 22
<210> 379
   <211> 22
   <212> DNA
   <213> Artificial Sequence
<220>
   <223> primer
<220>
   <221> misc_feature
   <222> (1)..(22)
   <223> reverse primer
<400> 379
   agtgatggct gatcctttca tt 22
<210> 380
   <211> 667
   <212> DNA
   <213> Diabrotica virgifera
<220>
   <221> misc_feature
   <222> (1)..(667)
   <223> WCRW transcript ta01259.001_diavv
<400> 380
   aaggtattgt ctctttaagg aatgcacagg gatctaatga ctggcaggct cgtttgcatc      60
   tggttgcaaa cgtcacgggg gtctgggggc tgtcaaaata agttctttca ataatacaaa     120
   atgtccaaga gaggacgtgg tggttccgcg ggagcgaagt ttaggatctc ccttgctctt     180
   cctgtaggag cagtaattaa ttgtgcagat aatacaggag caaagaactt atatgttatc     240
   gctgtacaag gtattggtgg acgacttaac cgtctgccag ctgcaggatc tggtgacatg     300
   attgtagcca cagtcaaaaa gggtaaacca gaactcagga aaaaagtcat gcctgcagta     360
   gttatcaggc agcggaaacc atttagaagg aaggatggtg tcttcattta ctttgaagat     420
   aatgctgggg ttatagtcaa taacaaaggt gaaatgaaag gatcagccat cactggtcca     480
   gttgcaaaag aatgtgcaga tttatggccc agaattgcat caaatgcaag cagtattgct     540
   tgatgtgtat atgtttaaga aaaataaaat aggatattgt tcttaaaaaa aaaaaaaaaa     600
   aatgcgattt tttatagaac ctatgtaagt ttacaattta ttcatgaaat aatgtaaaaa     660
   tgacagg                                                               667
<210> 381
   <211> 520
   <212> DNA
   <213> Diabrotica virgifera
<220>
   <221> misc_feature
   <222> (1)..(520)
   <223> RNAi target sequence
<400> 381
   ggcccaaaca actgtaagac agggtgctca aggaggaact actgtatacc ccggtatgca      60
   aaacacttac agagctactg cccgcccacc aaatcagtca acaaccatgc gtagcaacat     120
   gagtgtacct aggcaaatta ctggacaaca accacaaaat gcaattgtac ctgctgctgt     180
   taacagaaca gccaacttct aatacacttc aaacatgaga aatccaccac aacctttggg     240
   aggtttacca ggtgctgctg ctccagtaca gcaggctgta cacactcaag gtcaggaacc     300
   attgacagca accatgttgg ctgctgctcc accacaggag caaaaacaga tgttgggtga     360
   aaggttgttt ccacttatcc aacgcatgta tccagacatg gcaggtaaaa tcactggtat     420
   gttattggag attgacaaca ctgagctgct acacatgttg gaacaacagg agtcccttaa     480
   gaacaaagtg gaagaagcag ttgctgtttt acaagcccat                           520
<210> 382
   <211> 20
   <212> DNA
   <213> Artificial Sequence
<220>
   <223> primer
<220>
   <221> misc_feature
   <222> (1)..(20)
   <223> forward primer
<400> 382
   gcccaaacaa ctgtaagaca 20
<210> 383
   <211> 22
   <212> DNA
   <213> Artificial Sequence
<220>
   <223> primer
<220>
   <221> misc_feature
   <222> (1)..(22)
   <223> reverse primer
<400> 383
   atgggcttgt aaaacagcaa ct 22
<210> 384
   <211> 2656
   <212> DNA
   <213> Diabrotica virgifera
<220>
   <221> misc_feature
   <222> (1) .. (2656)
   <223> WCRW transcript ta16449.001_diavv
<400> 384
   cgcatttttt tttttttttt ttgacttctg acaaatagac tttttaaacc tattgtgaag 60
   aaaaactatt aaaaaaacaa aaaaaattgt aaaatagtgt aaaaaagtaa aaaaatatcc 120
   aacgaagatg aatccggctg ccccaaatta tcctatggcg tctttatacg tcggcgattt 180
   acattctgat attactgagg ccatgctttt tgagaaattt tcaaccgcgg gtcctgtgct 240
   ttcgattcgc gtgtgtagag acttgattac acgtcgatcc ttgggttatg catatgtgaa 300
   cttccaacaa cctgcagatg ctgagcgtgc tttggatacc atgaattttg acttgataaa 360
   aggtaggcct atccgcatca tgtggtctca acgtgatcca tcattgagaa aatctggcgt 420
   tggaaatgtt tttatcaaga atttagatcg ttccattgac aacaaggcca tgtacgatac 480
   tttctcagct tttggtaaca ttcttagttg taaggtagcc caagatgaaa acagctctag 540
   taaaggttat gggtttgtcc attttgaaac cgaagaagct gcaaataagt ctattgaaaa 600
   ggtaaatggt atgttgttga acggaaaaaa agtatatgta ggccgcttta ttccacacaa 660
   agaaagagaa aaggaactgg gagaaaaagc caagctgttc acaaatgttt atgttaaaaa 720
   ttttggtgaa gattttgctg aggaacagtt aaaggttatg tttgaaaagt atggtaaaat 780
   taccagctac aagataatga gtaaagaaga tggtaaatca aaaggatttg gttttgtagc 840
   ctttgaaagt tcagaggcag cagaagcggc agttgaagct ttaaatggaa aagagttggt 900
   tgaaggaaaa cctttgtatg ttggaagagc ccaaaagaga gctgagcgtc aacaagaact 960
   aaaacgtcgc tttgaagcct taaaaatgga aagacttaat cgctatcagg gtgtaaatct 1020
   ctatgttaaa aatctagatg atacaataga tgatgaacgt ctccgtaaag agtttactcc 1080
   ttttggaacc ataacatctg ccaaagtgat gttggaagaa ggtagaagca agggttttgg 1140
   ttttgtatgt ttttcctctc cagaagaagc tacaaaggcg gtaacagaaa tgaatggcag 1200
   aattgttggt tctaagcctt tatatgttgc cttagctcag cgtaaagaag atcgtaaagc 1260
   acatttaact tctcagtaca tgcagcgtat ggcaaatatg agaatgcacc agatgggtca 1320
   gttcatacaa ccaggtgcat ctagcggtta ttttgttcct accattcctg ccgcacaaag 1380
   attctatgga ccagcacaga tgactcaaat tcgtgctagt accagatggg ccgcccaaac 1440
   aactgtaaga cagggtgctc aaggaggaac tactgtatac cccggtatgc aaaacactta 1500
   cagagctact gcccgcccac caaatcagtc aacaaccatg cgtagcaaca tgagtgtacc 1560
   taggcaaatt actggacaac aaccacaaaa tgcaattgta cctgctgctg ttaacagaac 1620
   agccaacttc aaatacactt caaacatgag aaatccacca caacctttgg gaggtttacc 1680
   aggtgctgct gctccagtac agcaggctgt acacattcaa ggtcaggaac cattgacagc 1740
   aaccatgttg gctgctgctc caccacagga gcaaaaacag atgttgggtg aaagattgtt 1800
   tccacttatc caacgcatgt atccagacat ggcaggtaaa atcactggta tgttattgga 1860
   gattgacaac actgagctgc tacacatgtt ggaacaacag gagtccctta agaacaaagt 1920
   ggaagaagca gttgctgttt tacaagccca tcaagccaag caagctgcta ctactcagat 1980
   taagaaagag tagttgctga agcagtgatg attttatgtt ttatattttt aagcataaat 2040
   aaaaagtact tgtgcatttt caaaaaaaaa aaaaaaaaat gcgtcaaatt agctgattgt 2100
   agcttaaaca taatctttaa atctaatgtg tttattatta cagtttttgt ttagcatcaa 2160
   acattgctta catctccata ttcacttctt gattattgtt taatagtttg tcatttgatt 2220
   taaattatac aacaaagtta atgctttgtc atttattcag tttttgtatt ataaaaaaat 2280
   atacaattct ggaccttgga ggtaaactac attatgttaa catggtgttg ttttgaataa 2340
   gttcccttaa tccacttatt ttattagttg gattatacaa atttggttat atattattca 2400
   aatatggtta cattgttttt caaacaattg agatttttat aacactcaaa gtgctctact 2460
   gtaatttacc tccgaggtcc agatatgttt ttggaaatcg tcaaagtctc agtacggcta 2520
   gctttgctac atttgtaaca catgattaat ttcatttaga atagttgatt acaaaaatgc 2580
   cacaaggaaa tagcttcaga acatttcatt ttttaattat tataaattca ttttagaaag 2640
   cattatcaaa ttgtag 2656
<210> 385
   <211> 490
   <212> DNA
   <213> Diabrotica virgifera
<220>
   <221> misc_feature
   <222> (1)..(490)
   <223> RNAi target sequence
<400> 385
   tcaggtatgc cagtttattt tggaagatta tgtaatgtct gggcaaggag cctgggccaa     60
   catctgcgtc acacaaccca gacgtatatc agccgtttcg gtatcagaaa gagtggctgc    120
   cgaacgatgt gaagagctcg gtcagtctat cggttactca gtgcgttttg aatcagcttt    180
   acctaaaccg tacggatcag taatgttctg tacagtagga gtgttgttga aaaagttgga    240
   aaatggtctt agaggagtca gccacgttat tgtagatgag attcacgaac gagatgtcaa    300
   tagtgacttt attatggttg ttttgagaga tatgatccac acatatcccg atttaagagt    360
   aattcttatg tctgctacca tcgatacaac tttattctct aaatatttca acaactgccc    420
   cgttatcgaa attccaggaa gagcttttcc tgttaaacag tatttcttgg aagatgctat    480
   tgaactcacg                                                           490
<210> 386
   <211> 23
   <212> DNA
   <213> Artificial Sequence
<220>
   <223> primer
<220>
   <221> misc_feature
   <222> (1)..(23)
   <223> forward primer
<400> 386
   tcaggtatgc cagtttattt tgg 23
<210> 387
   <211> 22
   <212> DNA
   <213> Artificial Sequence
<220>
   <223> primer
<220>
   <221> misc_feature
   <222> (1)..(22)
   <223> reverse primer
<400> 387
   cgtgagttca atagcatctt cc 22
<210> 388
   <211> 4007
   <212> DNA
   <213> Diabrotica virgifera
<220>
   <221> misc_feature
   <222> (1)..(4007)
   <223> WCRW transcript ta01430.001_diavv
<400> 388 attagtatga cagtgtatca gttaatttaa gatttagtgt ttaattt 4007
<210> 389
   <211> 658
   <212> DNA
   <213> Diabrotica virgifera
<220>
   <221> misc_feature
   <222> (1)..(658)
   <223> RNAi target sequence
<400> 389
   gggggacttc attccagtct tgtgatattt agtgcttact tggtacagca gtttcagtgc 60
   tgtgctttag aataatttat tttttaacat ttatatagaa atcaaatact aaccaatcaa 120
   catgtgtgaa gaagaagttg ccgctttagt cgtagacaat ggatccggta tgtgcaaagc 180
   tggttttgct ggggatgatg cacctcgtgc tgtattccct tcaattgttg gacgcccaag 240
   acatcagggt gtgatggtag gaatgggaca aaaagattcc tatgtaggtg atgaagctca 300
   aagtaaaaga ggtatcctta ccttaaaata ccccatcgag cacggaatag tcacaaactg 360
   ggatgatatg gagaaaattt ggcatcatac attctacaat gaactcagag cagccccaga 420
   agaacaccct gttctgttga cagaagctcc tctcaacccc aaggccaaca gggaaaagat 480
   gacacaaata atgtttgaaa ctttcaacac cccagccatg tatgttgcca tccaggctgt 540
   actctccttg tatgcatctg gtcgtacaac tggtattgtg ttggattctg gtgatggtgt 600
   atcccacact gtcccaatct atgaaggtta tgctcttcct catgcaatcc ttcgtttg 658
<210> 390
   <211> 22
   <212> DNA
   <213> Artificial Sequence
<220>
   <223> primer
<220>
   <221> misc_feature
   <222> (1)..(22)
   <223> forward primer
<400> 390
   cttggtacag cagtttcagt gc 22
<210> 391
   <211> 22
   <212> DNA
   <213> Artificial Sequence
<220>
   <223> primer
<220>
   <221> misc_feature
   <222> (1)..(22)
   <223> reverse primer
<400> 391
   gcatgaggaa gagcataacc tt 22
<210> 392
   <211> 1534
   <212> DNA
   <213> Diabrotica virgifera
<220>
   <221> misc_feature
   <222> (1)..(1534)
   <223> WCRW transcript ta02481.002_diavv
<400> 392
   ggattccagt cttgtgatat ttagtgctta cttggtacag cagtttcagt gctgtgcttt      60
   agaataattt attttttaac atttatatag aaatcaaata ctaaccaatc aacatgtgtg     120
   aagaagaagt tgccgcttta gtcgtagaca atggatccgg tatgtgcaaa gctggttttg     180
   ctggggatga tgcacctcgt gctgtattcc cttcaattgt tggacgccca agacatcagg     240
   gtgtgatggt aggaatggga caaaaagatt cctatgtagg tgatgaagct caaagtaaaa     300
   gaggtatcct taccttaaaa taccccatcg agcacggaat agtcacaaac tgggatgata     360
   tggagaaaat ttggcatcat acattctaca atgaactcag agtagcccca gaagaacacc     420
   ctgttctgtt gacagaagct cctctcaacc ccaaggccaa cagggaaaag atgacacaaa     480
   taatgtttga aactttcaac accccagcca tgtatgttgc catccaggct gtactctcct     540
   tgtatgcatc tggtcgtaca actggtattg tgttggattc tggtgatggt gtatcccaca     600
   ctgtcccaat ctatgaaggt tatgctcttc ctcatgcaat ccttcgtttg gacttagctg     660
   gtagagactt gactgattac ctcatgaaaa ttttgactga acgtggctac tctttcacca     720
   ccacagcaga aagagaaatt gttagagata ttaaagaaaa actctgctat gtagctttgg     780
   acttcgaaca agaaatggca acagctgcta gttccagttc ccttgaaaag agttatgaac     840
   ttcctgatgg acaagtcatc accattggta atgaaagatt ccgttgccca gaagctctct     900
   tccaaccatc cttcttgggt atggaagctg ctggtattca tgaaacaacc tacaattcca     960
   tcatgaagtg tgatgttgat atccgtaagg acctatatgc caacactgta ctctctggag    1020
   gtaccaccat gtaccctggt attgctgata gaatgcagaa agaaatcact gccttggctc    1080
   ctagcacaat gaagattaag atcatcgccc caccagaaag aaaatactct gtatggatcg    1140
   gtggttccat tttggcctct ctatccacct tccaacaaat gtggatttcc aaacaagaat    1200
   atgatgaatc tggtccatcc atcgtccaca gaaaatgctt ctaatttgtt tttgtccgca 1260
   tttatattct ttatttctat ttacataagc ccgcccattt tccgggcgcg aactgccgtg 1320
   tttatcgtat gggatcgaga ccataaaata tccacactag gccaaagtat tactatctta 1380
   atttttgtaa tttattacct gtgcaaagta agactattat attattaggc taatactatg 1440
   ggaaaataaa aaaaatagta aaactcaaaa aaaaaaaaaa aaaatcagtt tacctttcta 1500
   ataaaataaa taaaacaaaa aaaaaaaaaa aaaa 1534
<210> 393
   <211> 601
   <212> DNA
   <213> Diabrotica virgifera
<220>
   <221> misc_feature
   <222> (1)..(601)
   <223> RNAi target sequence
<400> 393
   tgctccaaga tgaaggtact cattcttctt tccgttgttg ccttgagcag ctgcttacca 60
   gcatccagaa atcaacaata cgttgatgca gactacaaca gctacaacgc tcatcccgtc 120
   tacaacttct ggaggacttg gaatcagcaa cactatggac aagaaccatg gaaccaatgg 180
   gtacaatatg gtgtccagaa ccaacccggt aactactgga ggcagtacgt gcaacgcagc 240
   aaccaagctg aaaatgtcga tttaccttgg atgtatcact cttgggatgt ccaggatgtg 300
   caaaacttaa ctcccgttca acgtgttcag ttgctactca actacgtcag acttcaccaa 360
   caatggaacc aatggactca tgatgtaact gccaatgaac gtcttcagaa tatgttgtat 420
   caacaccttc ctgaacactt atggagctcc aacgagcaaa ttgtaccata catgatgaac 480
   tacttcaacc aatacccaat gaaatacaac gacattatgc atgaagttat gcaaacccca 540
   tgggctcacc aacaacttat gttccacgtt atgtctgaat tataccagca cttcggtcaa 600
   g 601
<210> 394
   <211> 22
   <212> DNA
   <213> Artificial Sequence
<220>
   <223> primer
<220>
   <221> misc_feature
   <222> (1)..(22)
   <223> forward primer
<400> 394
   tgctccaaga tgaaggtact ca 22
<210> 395
   <211> 22
   <212> DNA
   <213> Artificial Sequence
<220>
   <223> primer
<220>
   <221> misc_feature
   <222> (1)..(22)
   <223> reverse primer
<400> 395
   cttgaccgaa gtgctggtat aa 22
<210> 396
   <211> 1208
   <212> DNA
   <213> Diabrotica virgifera
<220>
   <221> misc_feature
   <222> (1)..(1208)
   <223> WCRW transcript ta08003.008_diavv
<400> 396
   ggggggacat ttcagtacac tgctccaaga tgaaggtact cattcttctt tccgttgttg      60
   ccttgagcag ctgcttacca gcatccagaa atcaacaata cgttgatgta gactacaaca     120
   gctacaacgc tcatcccgtc tacaacttct ggaggacttg gaatcagcaa cactatggac     180
   aagaaccatg gaaccaatgg gtacaatatg gtgtccagaa ccaacccggt aactactgga     240
   ggcagtacgt gcaacgcagc aaccaagctg aaaatgtcga tttaccttgg atgtatcact     300
   cttgggatgt ccaggatgtg caaaacttaa ctcccgttca acgtgttcag ttgctactca     360
   actacgtcag acttcaccaa caatggaacc aatggactca tgatgtaact gccaatgaac     420
   gtcttcagaa tatgttgtat caacaccttc ctgaacactt atggagctcc aacgagcaaa     480
   ttgtaccata catgatgaac tacttcaacc aatacccaat gaaatacaac gacattatgc     540
   atgaagttat gcaaacccca tgggctcacc aacaacttat gttccacgtt atgtctgaat     600
   tataccagca cttcggtcaa gccccatggt cccacgaaca aataatgaac cagatcatgc     660
   aacaccaata cctctacggt acaaccttat cccaacaaca actccaacaa ttggtagaat     720
   tcgtactcca cattcaatac gaacttactc ctttacatta ccaacaactt gaacaagttc     780
   aaccctggaa ccgtgaacaa gtactttcat ggaaccaagg acaagtactc ccatgggcct     840
   acagacaact tcttccattg aactatgaaa ccgttttacc atggacccgc cagtattccg     900
   cacaaaacaa catgaaccta gagtcccaat acaactacaa tctccaccaa caacaaatgc     960
   cattgtccca atactggaac cagagatacg gacaacaata cccacaacat tatgaagcca    1020
   tggaacaacc cggatggtgg aaccaagaat ggacccaaaa cttggttgga caagagaatc    1080
   attatcctct cttggggcaa aatatgccct tagtatatta aaaagtattc tgttctaagc    1140
   atatgattta aaataaaaaa aggcatatat acttgttagt tttattttta acccgtatac    1200
   aaagagtt                                                             1208
<210> 397
   <211> 600
   <212> DNA
   <213> Diabrotica virgifera
<220>
   <221> misc_feature
   <222> (1)..(600)
   <223> RNAi target sequence
<400> 397
   atttctttca aactgggatt cggtgaagac gtgacagctc agggggcgtt taatgaaaaa      60
   actgtgattt attagtgaaa tagtggttaa aacccagcaa aatggggatt aaatttttgg     120
   aagttataaa gcctttctgc agcatactgc ctgaaatagc caagccagaa agaaaaatcc     180
   aattcaggga gaaagtacta tggactgcaa tcactctttt catcttttta gtatgttgtc     240
   agattccatt attcggtata atgagttcag attctgcaga tcctttctac tggattcgtg     300
   ttattcttgc atcaaacaga ggtaccctta tggaacttgg tatttcccca attgtaactt     360
   caggtctgat tatgcagctg ttggccggtg ccaaaattat cgaggttggt gacactccaa     420
   agacagagcc ttattcaatg gtgcccaaaa attgtttggt atggttatta ctgtcggtca     480
   agccatcgtg tatgtaatga caggcatgta tggtgatccg gctgaaattg gagctggagt     540
   gtgccttctc atcatcatcc aactgttcgt cgccggtctc attgtattgc ttctcgatga     600
<210> 398
   <211> 22
   <212> DNA
   <213> Artificial Sequence
<220>
   <223> primer
<220>
   <221> misc_feature
   <222> (1)..(22)
   <223> forward primer
<400> 398
   atttctttca aactgggatt cg 22
<210> 399
   <211> 22
   <212> DNA
   <213> Artificial Sequence
<220>
   <223> primer
<220>
   <221> misc_feature
   <222> (1)..(22)
   <223> reverse primer
<400> 399
   tcatcgagaa gcaatacaat ga 22
<210> 400
   <211> 2110
   <212> DNA
   <213> Diabrotica virgifera
<220>
   <221> misc_feature
   <222> (1)..(2110)
   <223> WCRW transcript ta03313.003_diavv
<400> 400
   ccaacatcat cgatcacgtg acccgtgtgc tgtgctacgt gtacgacgtg tagacgtaaa      60
   acagagacaa gaagtaccgt aatttctttc aaactgggat tcggtgaaga cgtgacagct     120
   caggggggcg tttaatgaaa aaactgtgat ttattagtga aatagtggtt aaaacccagc     180
   aaaatgggga ttaaattttt ggaagttata aagcctttct gcagcatact gcctgaaata     240
   gccaagccag aaagaaaaat ccaattcagg gagaaagtac tatggactgc aatcactctt     300
   ttcatctttt tagtatgttg tcagattcca ttattcggta taatgagttc agattctgca     360
   gatcctttct actggattcg tgttattctt gcatcaaaca gaggtaccct tatggaactt     420
   ggtatttccc caattgtaac ttcaggtctg attatgcagc tgttggccgg tgccaaaatt     480
   atcgaggttg gtgacactcc aaagacagag ccttattcaa tggtgcccaa aaattgtttg     540
   gtatggttat tactgtcggt caagccatcg tgtatgtaat gacaggcatg tatggtgatc     600
   cggctgaaat tggagctgga gtgtgccttc tcatcatcat ccaactgttc gtcgccggtc     660
   tcattgtatt gcttctcgat gaacttctcc agaaaggata tggtttaggt tctggtattt     720
   ccctcttcat tgcaacaaac atttgtgaaa ctattatttg gaaagccttc tcaccagcaa     780
   cagtaaacac tggaagagga acagaatttg aaggagctgt cattgcctta ttccacttgt     840
   tgaccaccag acaagacaaa atcagaggat tacgtgaagc tttctacagg caaaatcttc     900
   ccaatttgat gaacttgttg gcgacagtac tcgtttttgc tatagtaata tacttccaag     960
   gattcagagt agatctaccc atcaagagtg caagataccg tggacaatat tccagctatc 1020
   caatcaaatt gttctacact tcgaacatcc ctattattct acaatctgct cttgtatcta 1080
   acttgtatgt catctcacag atgttggctg ttaaattcca agggaacttc ttaattaatc 1140
   tattaggagt ctgggctgac gttggaggcg gtggcccagc taggtcttac cccattggag 1200
   gattatgtta ctacttatct ccacctgaaa gtgtaagtca cattctagaa gatccaatcc 1260
   acgcaatgct ctacatcgtc ttcatgttgg gatcttgtgc tttcttctcc aaaacatgga 1320
   tcgaagtatc tggaagctca gccaaagatg tcgctaagca attgaaagaa caacagatgg 1380
   taatgagagg acacagagat aactccatga ttcacgaatt gaacaggtac attcccaccg 1440
   ccgcagcttt tggaggtctc tgtattggag ccttgtcagt actcgctgat ttcatgggag 1500
   ccattggttc aggtactggt atcttgttag ctgtcaccat catctaccaa tacttcgaaa 1560
   tcttcgtcaa agaacaaagc gagatgggtg gaatgggagc gttactcttc tagataaatt 1620
   gaagatatca caaagctata gcttgactga tattgtgatt ttgtttggta aatagtaact 1680
   attgtggtta ttatttgttt agtttttgta cctcagtgtc tccactatag ttattattta 1740
   tgtggaacaa cttatatgtc actttgtata aagtgtgaaa catttttttg tactaaaaga 1800
   ttgaaaatag acaaatatat ttcattgtac atttacatga ttttgtgttt aagaagcgag 1860
   ttatttataa tgtgcggatg tcccatacta ttagtttatt ttttacattt tttcattctc 1920
   gttcatgcag tacggttgtg tattgatcat ttattatttg ttgttagata taatttattt 1980
   taaatacaga aatttctgaa atcctgtttt tcatttgatg ctaatgtata ctgtaaatgt 2040
   ttcgagataa gggcatctat catttaaatg ttgaaatatt attgaaatgt acaaatttac 2100
   atgttctttt 2110
<210> 401
   <211> 707
   <212> DNA
   <213> Diabrotica virgifera
<220>
   <221> misc_feature
   <222> (1)..(707)
   <223> RNAi target sequence
<400> 401
   gggggacctt ttatatttga catcctgaaa gatggttgag aaaaaggctg tacagaagcc 60
   cgctggggat gcaccaaaaa aggtgcacaa accaggaaaa cctagaaatt atgatttggg 120
   aaatggagta ctcagatttt ctagatctaa aatgttccac aaaaaagcat tgtacaaatt 180
   tgttggcaag aaggtagaag ctaccaagaa acctcaaaag ccccaggtta ttgaaaaacc 240
   aattggtgga gagaaaaatg gaggaaagag atttgttttg gtcaagaaac gtagggcatc 300
   ctatccaacc caagacaaaa ttaaggccag gccagctagg agaacattta gccaacacac 360
   aaggtctatt agaaatacaa tcactccagg aacagttctc atccttttag ctggttctca 420
   caaaggaaag agggttgtat tccttaaaca actgtactct ggtctacttt tggtaactgg 480
   accttttgga atcaatgctt gcccattgag gcgcattagc caacgatatg taattgctac 540
   atctaccaaa attgacgtta gtggagtaca gctgccagaa aaccttaatg atgaatattt 600
   tgcaagaaag aggcagaaga gatccaagaa agaagaaggt gatattttcc aatccaaaaa 660
   agaaggttac aaagtaagtg aagataggaa agctgaccaa aagaaag 707
<210> 402
   <211> 18
   <212> DNA
   <213> Artificial Sequence
<220>
   <223> primer
<220>
   <221> misc_feature
   <222> (1)..(18)
   <223> forward primer
<400> 402
   gaccttttat atttgaca 18
<210> 403
   <211> 22
   <212> DNA
   <213> Artificial Sequence
<220>
   <223> primer
<220>
   <221> misc_feature
   <222> (1)..(22)
   <223> reverse primer
<400> 403
   ctttcttttg gtcagctttc ct 22
<210> 404
   <211> 1036
   <212> DNA
   <213> Diabrotica virgifera
<220>
   <221> misc_feature
   <222> (1)..(1036)
   <223> WCRW transcript ta17558.001_diavv
<400> **404**
   aaggagaggg aggttttcct gtttgtgcac cttttttgtg cagacagaac gaccacctgg       60
   acacgcatgc tatgggggac cttttatatt tgacatcctg aaagatggtt gagaaaaagg      120
   ctgtacagaa gcccgctggg gatgcaccaa aaaaggtgca caaaccagga aaacctagaa      180
   actatgattt gggaaatgga gtactcagat tctctaggtc taaaatgttc cacaaaaaag      240
   cattatacaa attcgttggc aagaaagtag aagctaccaa gaaacctcaa aagccccagg      300
   ttattgaaaa accaattggt ggagagaaaa atggaggaaa gagatttgtt ttggtcaaga      360
   aacgtagagc atcctatcct acccaagaca aaattaaggc caggccagct aggagaacat      420
   ttagccaaca cacgaggtct attagaaata caatcactcc aggaacagtt ctcatccttt      480
   tagctggttc tcacaaagga aagagggttg tattccttaa acaactgtac tctggtctac      540
   ttttggtaac tggacctttt ggaattaatg cctgtccatt gagacgtatt agccaacgtt      600
   atgtaattgc tacatctacc aaaattgacg ttagtggagt acagctgcca gaaaacctaa      660
   atgatgaata ttttgcaaga aagaggcaga agagatcaaa gaaagaagaa ggtgatattt      720
   tccaatccaa aaaagaaggt tacaaagtaa gcgaagatag gaaagctgac caaaagaaag      780
   tagatacaca agttctggct gcaatcaaga agcaatcaga ccgcaaagta cttttagctt      840
   atttgtcagc catgtgggga ctcagatcat cacagtaccc acacagattg aagttttaga      900
   taagagatac cataaattgt ttatcttttg acttgtatag agaggaaagt gataaataaa      960
   ttatggattt ataaatgagg tttttttatg tgactgttgt tataatacaa taatttaatt     1020
   cttaaaaaaa aaaaaa                                                     1036
<210> 405
   <211> 671
   <212> DNA
   <213> Diabrotica virgifera
<220>
   <221> misc_feature
   <222> (1)..(671)
   <223> RNAi target sequence
<400> 405
   gggaacctta ttttatggtg tcgtgccagc agaaaatttt aaaaattctt gtaattgttt        60
   agacatttcg caacaattgt gaacaatttt ggaaaatatt gcttgaaagc tataattcta       120
   gatgtaagag caaagttttg tagtcaaaat ggctggagta ttcgatttag aactgcatga       180
   tgttgaaact gtagaagtag atgattcaga cgatgcaatc gaaattgaag aggatgaata       240
   tgatcaaaat cctaatatta atgcaattgt agaatccgat gatacagaaa cggttcatct       300
   gactgaacaa attgtgaacc cgggccagga aaaaaccgga ccccatgact ttgaattacg       360
   caaagtcctg ggcaaaggag gttatggcaa agtatttcaa gttagaaaaa ttacggggaa       420
   agatgccaat actgtctttg ccatgaaggt tctccgcaag gcttccatag ttagaaatca       480
   aaaagatact gcccatacga aagcagaacg aaatatttta gaagaagtta agcatccttt       540
   tatagtggat ttgatatatg ccttccaaac gggtggaaaa ttgtacttaa ttttagaata       600
   cctcagcggg ggcgaacttt ttatgcattt agaaagagaa ggaatatttt tagaggatac       660
   cgcttgcttt t                                                            671
<210> 406
   <211> 21
   <212> DNA
   <213> Artificial Sequence
<220>
   <223> primer
<220>
   <221> misc_feature
   <222> (1)..(21)
   <223> forward primer
<400> 406
   gaaccttatt ttatggtgtc g 21
<210> 407
   <211> 22
   <212> DNA
   <213> Artificial Sequence
<220>
   <223> primer
<220>
   <221> misc_feature
   <222> (1)..(22)
   <223> reverse primer
<400> 407
   aaaagcaagc ggtatcctct aa 22
<210> 408
   <211> 2032
   <212> DNA
   <213> Diabrotica virgifera
<220>
   <221> misc_feature
   <222> (1)..(2032)
   <223> WCRW transcript ta00443.001_diavv
<400> 408 acctaggtca gtatgggttg cgaatttaaa atggttataa aaaatatgct ca 2032
<210> 409
   <211> 612
   <212> DNA
   <213> Diabrotica virgifera
<220>
   <221> misc_feature
   <222> (1)..(612)
   <223> RNAi target sequence
<400> 409
   gagcttgcga tttttatttt tgaaaaatag agagtattct ctaatattta aggacagtat 60
   ggaagacgat tgggctgtgg ataatcagag tggtggagtt gtcgccccaa aaattgcaga 120
   actacctgaa attaagttgt tcgctagatg gaactgcgat gatgtccaag tttcagacat 180
   gtcccttcag gactacattg cagtgaaaga aaaaaatgca aagtatttac cccattcagc 240
   tggtagatat gctgcaaaaa ggttccgtaa agcacaatgc ccaatcgttg agaggttaac 300
   aaactctcta atgatgcatg gacgtaacaa tggtaaaaaa ttgatggctg tcagaattgt 360
   taaacatgct tttgaaatta tccatttact aactggagaa aatccattac agattttagt 420
   ttctgctatt atcaattcag gacctagaga agattctact cgtattggta gagctggtac 480
   tgtaagaaga caagctgttg atgtgtcacc cttgagaagg gttaaccaag caatttggtt 540
   gctctgcaca ggtgctaggg aagcagcatt ccgtaatatt aaaactattg ctgaatgttt 600
   ggctgatgaa tt 612
<210> 410
   <211> 23
   <212> DNA
   <213> Artificial Sequence
<220>
   <223> primer
<220>
   <221> misc_feature
   <222> (1)..(23)
   <223> forward primer
<400> 410
   gagcttgcga tttttatttt tga 23
<210> 411
   <211> 22
   <212> DNA
   <213> Artificial Sequence
<220>
   <223> primer
<220>
   <221> misc_feature
   <222> (1)..(22)
   <223> reverse primer
<400> 411
   aattcatcag ccaaacattc ag 22
<210> 412
   <211> 780
   <212> DNA
   <213> Diabrotica virgifera
<220>
   <221> misc_feature
   <222> (1)..(780)
   <223> WCRW transcript 454run3_isotig03469
<400> 412
   aagttttatc catatttatt attggaaata aaatataaaa tgagaaataa atttaacggt 60
   tggatttggc tacacgttca agttcatcct tctttttgat agcatatgaa tttgatgatc 120
   ccttggcagc attgattaat tcatcagcca aacattcagc aatagtttta atattacgga 180
   atgctgcttc cctagcacct gtgcagagca accaaattgc ttggttaacc cttctcaagg 240
   gtgacacatc aacagcttgt cttcttacag taccagctct accaatacga gtagaatctt 300
   ctctaggtcc tgaattgata atagcagaaa ctaaaatctg taatggattt tctccagtta 360
   gtaaatggat aatttcaaaa gcatgtttaa caattctgac agccatcaat tttttaccat 420
   tgttacgtcc atgcatcatt agagagtttg ttaacctctc aacgattggg cattgtgctt 480
   tacggaacct ttttgcagca tatctaccag ctgaatgggg taaatacttt gcattttttt 540
   ctttcactgc aatgtagtcc tgaagggaca tgtctgaaac ttggacatca tcgcagttcc 600
   atctagcgaa caacttaatt tcaggtagtt ctgcaatttt tggggcgaca actccaccac 660
   tctgattatc cacagcccaa tcgtcttcca tactgtcctt aaatattaga gaatactctc 720
   tatttttcaa aaataaaaat cgcaagctct tgctgaacga cggcgtgaac cggaaaaggt 780
<210> 413
   <211> 630
   <212> DNA
   <213> Diabrotica virgifera
<220>
   <221> misc_feature
   <222> (1)..(630)
   <223> RNAi target sequence
<400> 413
   gcttcttttt cgttggatgt ctttgtaggt tttaaaaatg ggagcctaca agtatataca 60
<210> 414
   <211> 22
   <212> DNA
   <213> Artificial Sequence
<220>
   <223> primer
<220>
   <221> misc_feature
   <222> (1)..(22)
   <223> forward primer
<400> 414
   gcttcttttt cgttggatgt ct 22
<210> 415
   <211> 22
   <212> DNA
   <213> Artificial Sequence
<220>
   <223> primer
<220>
   <221> misc_feature
   <222> (1)..(22)
   <223> reverse primer
<400> 415
   agtgagttcc ttctgagcca ag 22
<210> 416
   <211> 1374
   <212> DNA
   <213> Diabrotica virgifera
<220>
   <221> misc_feature
   <222> (1)..(1374)
   <223> WCRW transcript ta18374.001_diavv
<400> 416
   tatataactc ggattaccac agtcaaagtg cataaaactt aacaatacgc aaaaaccgta      60
   gcagtccgtt tatttctttc ctgcggcctt ggctggagcc ttcttggcct tcttaggctt     120
   tgatttctta tctttcaaga tttgtgccct tctcctttct tgcaattttc tggatctaat     180
   cacgacgttg tcggcactca cggtaatacc acgtttcttg gccaaaagtt cttccctgtt     240
   taactgtctc ttttgattct tcaaaatggc ttcacgtttg agtacagcag catatggatt     300
   caacttaagc atggccttag cgttggtcaa tggattgaga cgacgtacac gacgtacaac     360
   cttcttttga ggagcacgta atacagcttt gatttcatca gccttcaaca atctagatag     420
   atcagtgttg gccattttag gctggggtag attgtaaccc ttcttttcca atgatgctgt     480
   tttccatgtg ccgaacaatt tatctaaacg ttggaaagct gattcagtcc aaataacaaa     540
   gcgacctaca tgaccaccag gagcaagttt caacaaattt aacttctgta cgctaatgag     600
   gtcaatgcct gggatgttac ggaaagcacg acgaagacct acaaggggga cttgcgcgtt     660
   gaactttctt tttcgttgga tgtctttgta ggttttaaaa atgggagcct acaagtatat     720
   acaggagttg tatagaaaaa aacaaagtga tgttcttaga tttttactga gagtgagagt     780
   gtggcaatat cgccaattga ctaaactcca cagaactccc agacccagtc ggcctgacaa     840
   ggctcgcaga atgggttaca gggccaagca aggttttgtt atttaccgta ttagagtacg     900
   acgtggagga cgcaagcgtc cagtaccaaa aggtgccacc tacggtaaac ccaaaagcca     960
   tggagttaat gaattgaaac ctgtccgtaa cttacaagct attgctgagg aacgtgtagg    1020
   aagaaggtgt ggtggattaa gagtcctaaa ctcctactgg gtagcccaag attcaacata    1080
   caaatatttt gaaatcattt tggtcgaccc ctcacataac gctattcgca aggaccccaa    1140
   aattaactgg attgtcaact cagtacacaa acacagagaa ctccgtggca aaacttcctc    1200
   tggcaaatca tctcgtggtt tgggcaaggg tcacagatac tctcaaacca agggaggatc    1260
   caggcgtgcc gcttggctca gaaggaactc actgcaattg cgcagaaaac gttaaaattt    1320
   atttttttat aattagacca ataaaattta cggattaaaa aaaaaaaaaa aaaa          1374
<210> 417
   <211> 663
   <212> DNA
   <213> Diabrotica virgifera
<220>
   <221> misc_feature
   <222> (1)..(663)
   <223> RNAi target sequence
<220>
   <221> misc_feature
   <222> (570)..(570)
   <223> n can be any nucleotide
<400> 417
   gggcgaaact gttcttcatt atcctttcta ggtcgtgtaa aatacgtgaa ggccaaaatg      60
   ggtagacgac ctgcgaggtg ctaccgttat tgtaaaaaca aaccctaccc caaaagtcgt     120
   ttctgcagag gtgttccaga tccaaaaatc cgtattttcg atttgggtaa gaagaaggct     180
   tccgtagaag actttcctct atgtgtacat ttagtttctg acgaatatga acagctaagt     240
   tctgaagcat tagaagcagg acgtatctgt tgtaacaaat atttggtaaa gaactgtggt     300
   aaagatcaat tccacattcg tatgagactc catcctttcc atgttatcag aatcaacaag     360
   atgttgtcat gtgctggagc tgatagactc cagactggaa tgagaggagc tttcggcaag     420
   ccccaaggta cagttgctcg tgtccgcatt ggtcaaccta ttatgagtgt acgctctagt     480
   gatcgtttca aggctgcagt tattgaggct ctccgtcgtg ccaagttcaa gttcccaggt     540
   cgtcaaaaga tctatgtatc gaagaaatgn ggattcacca agtatgaacg tgaacaatat     600
   gaaaacttaa aacaacaagg tagattagct cctgatggtt gcaatgttca atacaggcca     660
   gag                                                                   663
<210> 418
   <211> 20
   <212> DNA
   <213> Artificial Sequence
<220>
   <223> primer
<220>
   <221> misc_feature
   <222> (1)..(20)
   <223> forward primer
<400> 418
   gcgaaactgt tcttcattat 20
<210> 419
   <211> 22
   <212> DNA
   <213> Artificial Sequence
<220>
   <223> primer
<220>
   <221> misc_feature
   <222> (1)..(22)
   <223> reverse primer
<400> 419
   ctctggcctg tattgaacat tg 22
<210> 420
   <211> 726
   <212> DNA
   <213> Diabrotica virgifera
<220>
   <221> misc_feature
   <222> (1)..(726)
   <223> WCRW transcript ta04337.003_diavv
<400> 420
   gaaggtttgt ttcttaattt attttaacaa tcttacgttt ttagttgtag tgatatattt      60
   ttataaacta aatactgaat acgtaataac gtatatgtgt tagtttttta atctacatat     120
   ttattttcta ttttcaggcc aaaatgggta gacgacctgc gaggtgctac cgttattgta     180
   aaaacaaacc ctaccccaaa agtcgtttct gcagaggtgt cccagatcca aaaatccgta     240
   ttttcgattt gggtaagaag aaggcttcag tagaagactt tcctctatgt gtacatttag     300
   tttctgacga atatgaacag ctaagttctg aagcattaga agcaggacgt atctgttgta     360
   acaaatattt ggtaaagaac tgtggtaaag atcaattcca cattcgtatg agactccatc     420
   ctttccatgt tatcagaatc aacaagatgt tgtcatgtgc tggagctgat cgactccaga     480
   ctggaatgag aggagctttc ggcaagcccc aaggtacagt tgctcgtgtc cgcattggtc     540
   aacctattat gagtgtacgc tctagtgatc gtttcaaggc tgcagttatc gaggctctcc     600
   gtcgtgccaa gttcaagttc ccaggtcgtc aaaagatcta tgtatcaaag gaaatgggga     660
   ttcaccaagt atgaacgtga acaatatgaa aacttaaaac aacaaggtag attagctcct     720
   gatggt                                                                726
<210> 421
   <211> 625
   <212> DNA
   <213> Diabrotica virgifera
<220>
   <221> misc_feature
   <222> (1)..(625)
   <223> RNAi target sequence
<400> 421
   gcgaaagttg tgtcttgaaa agtaaaaatg acgtctaaag tatctcgtga tactctatac      60
   gagtgtgtga atggagtctt ggaaaatgcc aaggagaaga aaaggaactt tttggaaacc     120
   gtggaactcc agatcggtct taagaactac gatccccaaa aggacaagcg tttcagcgga     180
   accgtcaaac taaagcacat tcctcgtcca aagatgcagg tatgcatctt gggagatcag 240
   caacattgtg atgaagctaa cgctaacaat gttccattca tggatgtaga agccttgaaa 300
   aaattgaaca aaaacaagaa attggttaag aaactagcca agaaatatga tgctttcctc 360
   gcttctgaag ctctcatcaa acagatcccc aggttgttgg gtccaggttt gaacaaggct 420
   ggtaaattcc ctggtcttct ctcccaccag gaatccatga cacagaaaat tgatgaagta 480
   aaagcaacaa tcaaattcca aatgaagaag gtactctgcc tctctgttgc tgttggtcat 540
   gtaggcatga atcaagatga attggttcaa aatgtccatc tttcaatcaa cttcttggtc 600
   tcactgttga agaaacattg gcaga 625
<210> 422
   <211> 22
   <212> DNA
   <213> Artificial Sequence
<220>
   <223> primer
<220>
   <221> misc_feature
   <222> (1)..(22)
   <223> forward primer
<400> 422
   gcgaaagttg tgtcttgaaa ag 22
<210> 423
   <211> 22
   <212> DNA
   <213> Artificial Sequence
<220>
   <223> primer
<220>
   <221> misc_feature
   <222> (1)..(22)
   <223> reverse primer
<400> 423
   tctgccaatg tttcttcaac ag 22
<210> 424
   <211> 1055
   <212> DNA
   <213> Diabrotica virgifera
<220>
   <221> misc_feature
   <222> (1)..(1055)
   <223> WCRW transcript ta02345.005_diavv
<400> **424**
   aagactagta ccttcacata atatggagaa tctcctgggt tgaacataga aaaaaatgta      60
   taatgtatcc ataatagaac aactcaatat aaaagatagg ttgcttaaac aaacacaaca     120
   gagatacagt attttggaca catttcctga agaacaggaa cactttatca ttcctggtgc     180
   atttcgttaa cgtagacaag ccgcaaaagt tgtgtcttga aaaataaaaa tgacgtctaa     240
   agtatctcgt gatactctat acgagtgtgt gaatggagtc ttggaaaatg ccaaggagaa     300
   gaaaaggaac tttttggaaa ccgtggaact ccagatcggt cttaagaact acgatcccca     360
   aaaggataag cgtttcagcg gaaccgtcaa actaaagcac attcctcgtc caaagatgca     420
   ggtatgcatc ttgggagatc agcaacattg tgacgaagct aacgccaaca atgttccatt     480
   catggatgta gaagccttga aaaaattgaa caaaaacaag aaattggtta agaaactagc     540
   caagaaatat gatgctttcc ttgcttctga agccctcatc aaacagatcc ccaggttgtt     600
   gggtccaggt ttgaacaagg ctggtaaatt ccctggtctt ctctcccacc aggaatccat     660
   gacacagaaa attgatgaag taaaagcaac aatcaaattc caaatgaaga aggtactctg     720
   cctttctgtt gctgttggtc atgtaggcat gaatcaagat gaattggttc aaaatgtcca     780
   tctttcaatc aacttcttgg tctcactgtt gaagaaacat tggcagaacg ttaggtctct     840
   ccacgtcaaa tcatctatgg gacccccaca aagattgtat taagttataa tttttatact     900
   gagactttta tttacacact taaatgtggg tatctaaata aacatcgttt ttaagttctt     960
   taaaaaaaaa aaaaatagaa aaaaaaaatg attggatgta catattatac taccctacta    1020
   agtaaaaaaa tacatctaaa aaaaaaaaaa aaaaa                               1055
<210> 425
   <211> 693
   <212> DNA
   <213> Diabrotica virgifera
<220>
   <221> misc_feature
   <222> (1)..(693)
   <223> RNAi target sequence
<400> 425
   gggggacatt ttcgtttgta aataacaaat aacagtaaag taaagaataa aaattgaaga      60
   attttatatg attcctttaa ttttcgtctt caaatagcat ataagttata ttctgagttt     120
   ataaaattaa caccatgcct acgataatat tgctggacgt gtctctttct atgacgaggc     180
   ctgttcaagt tggtgagggc tttgaaacag tctcaagaaa gcaactggcg gaattaggga     240
   ttaatgcatt tttggattat cttagcatac attccaaatt agagtttata tctttgatgg     300
   ctttttcgtc attgtacgag gagaggtgtc cttttaccag agattacaat gctattaaag     360
   cagaattaaa gaatatagaa gactatgata aaacttgcat tgaaacagcg cttcatggtg     420
   ttaatcacat ggttcttgga gagtggggga ataacacggc ttgtcaggta ttattagtaa     480
   cggatggaaa tactggaatt ggaacgaatt ccttgaaaga agcaattgct acaatgaatc     540
   aaaggaataa taaaatacca tttcctgttc ctttttcatt cccaggaaaa ttacatgtgg     600
   tgtgtctcac ttctccctgt gatttaagtt ttattaaatc taaaccactt tttcagaggt     660
   tgattgattt aagtggttac gacggatcaa ttt                                  693
<210> 426
   <211> 21
   <212> DNA
   <213> Artificial Sequence
<220>
   <223> primer
<220>
   <221> misc_feature
   <222> (1)..(21)
   <223> forward primer
<400> 426
   gggggacatt ttcgtttgta a 21
<210> 427
   <211> 22
   <212> DNA
   <213> Artificial Sequence
<220>
   <223> primer
<220>
   <221> misc_feature
   <222> (1)..(22)
   <223> reverse primer
<400> 427
   aaattgatcc gtcgtaacca ct 22
<210> 428
   <211> 1551
   <212> DNA
   <213> Diabrotica virgifera
<220>
   <221> misc_feature
   <222> (1)..(1551)
   <223> WCRW transcript ta10911.001_diavv
<400> 428
   ggacattttc gtttgtaaat aacaaataac agtaaagtaa agaataaaaa ttgaagaatt 60
<210> 429
   <211> 651
   <212> DNA
   <213> Diabrotica virgifera
<220>
   <221> misc_feature
   <222> (1)..(651)
   <223> RNAi target sequence
<400> 429
   gggggctctt agttatccat cttggcttcg tttaggtttt agcttagcac tagcaccacg 60
   ttttcgtttt aagttgactg ttgactttgg ccattgtttc aagtgtagtt gtcttatcgt 120
   tatcgttaca atttgaaatt tggattgagt ttgagttgtt aggaccagga ccaggtttat 180
   aacacaagtg gatatctatt catcatggca attgattggg aaaaaattgt aactgaacta 240
   agtgctgtgt tccaagatga gcaagagaca tttgcgagag ttttgtttta tttaggagaa 300
   gaagaaaagg tgtataaaga tgccgataaa ctaggaccgc agatgaagat cttaatgtca 360
   tctatgaaag tagcatcttt gtttagtcag tacgccaaac ggttagcaga ggacacaaag 420
   agggctgatt cggatgaaaa gttcaaacga gtgatgggtc taatctgggc ccaaatgatg 480
   gacgctaaca aacagacaga tgacaaagat aaaaccccag atgtaagcat tattgccaaa 540
   ttagaacacc tgtctctaga agcttcgcca ggaatggttc gcgccttgag cattattcca 600
   tatgaaagcc aaaagcgttt tttagtaatg ctcaaagaca tcgtaccaac a 651
<210> 430
   <211> 22
   <212> DNA
   <213> Artificial Sequence
<220>
   <223> primer
<220>
   <221> misc_feature
   <222> (1)..(22)
   <223> forward primer
<400> 430
   gggggctctt agttatccat ct 22
<210> 431
   <211> 22
   <212> DNA
   <213> Artificial Sequence
<220>
   <223> primer
<220>
   <221> misc_feature
   <222> (1)..(22)
   <223> reverse primer
<400> 431
   tgttggtacg atgtctttga gc 22
<210> 432
   <211> 1310
   <212> DNA
   <213> Diabrotica virgifera
<220>
   <221> misc_feature
   <222> (1)..(1310)
   <223> WCRW transcript ta05539.003_diavv
<400> 432
   cagagacttc atgctcatgc tccttctctt agttatccat cttggcttcg tttaggtttt      60
   agcttagcac tagcaccacg ttttcgtttt aagttgactg ttgactttgg ccattgtttc     120
   aagtgtagtt gtcttatcgt tatcgttaca atttgaaatt tggattgagt ttgagttgtt     180
   aggaccagga ccaggtttat aacacaagtg gatatctatt catcatggca attgattggg     240
   aaaaaattgt aactgaacta agtgctgtgt tccaagatga gcaagagaca tttgcgagag     300
   ttttgtttta tttaggagaa gaagaaaagg tgtataaaga tgccgataaa ctaggaccgc     360
   agatgaagat cttaatgtca tctatgaaag tagcatcttt gtttagtcag tacgccaaac     420
   ggttagcaga ggacacaaag agggctgatt cggatgaaaa gttcaaacga gtgatgggtc     480
   taatctgggc ccaaatgatg gacgctaaca aacagacaga tgacaaagat aaaaccccag     540
   atgtaagcat tattgccaaa ttagaacacc tgtctctaga agcttcgcca ggaatggttc     600
   gcgccttgag cattattcca tatgaaagcc aaaagcgttt tttagtaatg ctcaaagaca     660
   tcgtaccaac aacagtgcta tcgaatgtag agtatgttgt tgaaactgtt aacaaagtag     720
   gtggtgctgt agtaactgta gctttagcta gtatacaatt ggcatatgaa gctattgtta     780
   acattagaag atggtggaat ggagagattt caggcaagag atgttgtaaa aatattatag     840
   attcacttgc tacaattggt ggtagcgttg gtggaggatt tgctggtgta gctttgggag     900
   cacttggagg tccagttggc atgataatcg gaggtattgg tggaggaatg ttgggcggag     960
   aattggccaa gcgtctgtcg caatggatga ctgaaaaggt atttgatgtt ccaaaatctg    1020
   aggcgctaga aaatgcttat aatttcttta atttgcgcaa tcatgcttcc aatcatcaga    1080
   taaatacagc ttacagacag aagtgtctag aataccatcc tgataaagga ggttctgagg    1140
   aaatgttcca taaagtgcag atggaaatgg cggttattaa agctgccaaa ggagaattgt    1200
   attcataata attagtttgt atttttgtac ttagatttag attatataac atattttgta    1260
   ctatactatt ttatgtacac attatatttg tacagaaata aaaattaacg                1310
<210> 433
   <211> 658
   <212> DNA
   <213> Diabrotica virgifera
<220>
   <221> misc_feature
   <222> (1)..(658)
   <223> RNAi target sequence
<220>
   <221> misc_feature
   <222> (546)..(546)
   <223> n can be any nucleotide
<400> 433
   atgtttttaa gatgggccag tgccagtcac acattgtgtt taagtttatt ttcgtgtttc 60
   ttgctgggta cgaccgagta acgaacaacc taggatatca cgccaaattc acaagtaaca 120
   agttccagtc ctggagtagg agcagccctg gcggcgttag gaagtctcgg tggaatcttc 180
   ggcaatcact tacaaatgtt aggaagctta ccagctttcc agagtccgca gagcctgcaa 240
   cagctgcagc agctggcgat gctgcagcag gctagcggca accagatgaa tccgcaggct 300
   cagtttttat ttcaaaatca gggttttcta caagggttcc ctagaacaag tataccttct 360
   agacccccac atacaatgca ggtacaacaa atagcacaag ctgcccagca actacagcaa 420
   ctgcagaaag tacagcaaaa cacaaaccaa cacaatacca gcattcctca aaatacgacg 480
   ttacctcatc aaacgcagac cacgcctcca aacaaccaca atatacctgc cagcatgctt 540
   actccnagca cgcctagttc tggtggactt acgccgcagc atcttaaaac accctcaagg 600
   atactggaac catctccaga agaaaccaca gatttggaag aacttgaaca gtttgcga 658
<210> 434
   <211> 22
   <212> DNA
   <213> Artificial Sequence
<220>
   <223> primer
<220>
   <221> misc_feature
   <222> (1)..(22)
   <223> forward primer
<400> 434
   atgtttttaa gatgggccag tg 22
<210> 435
   <211> 22
   <212> DNA
   <213> Artificial Sequence
<220>
   <223> primer
<220>
   <221> misc_feature
   <222> (1)..(22)
   <223> reverse primer
<400> 435
   tcgcaaactg ttcaagttct tc 22
<210> 436
   <211> 698
   <212> DNA
   <213> Diabrotica virgifera
<220>
   <221> misc_feature
   <222> (1)..(698)
   <223> WCRW transcript ta21806.001_diavv
<220>
   <221> misc_feature
   <222> (566)..(566)
   <223> n can be any nucleotide
<400> 436
   ggagtgcata ttgcccgcag atgtttttaa gatgggccag tgccagtcac acattgtgtt      60
   taagtttatt ttcgtgtttc ttgctgggta cgaccgagta acgaacaacc taggatatca     120
   cgccaaattc acaagtaaca agttccagtc ctggagtagg agcagccctg gcggcgttag     180
   gaagtctcgg tggaatcttc ggcaatcact tacaaatgtt aggaagctta ccagctttcc     240
   agagtccgca gagcctgcaa cagctgcagc agctggcgat gctgcagcag gctagcggca     300
   accagatgaa tccgcaggct cagtttttat ttcaaaatca gggttttcta caagggttcc     360
   ctagaacaag tataccttct agacccccac atacaatgca ggtacaacaa atagcacaag     420
   ctgcccagca actacagcaa ctgcagaaag tacagcaaaa cacaaaccaa cacaatacca     480
   gcattcctca aaatacgacg ttacctcatc aaacgcagac cacgcctcca aacaaccaca     540
   atatacctgc cagcatgctt actccnagca cgcctagttc tggtggactt acgccgcagc     600
   atcttaaaac accctcaagg atactggaac catctccaga agaaaccaca gatttggaag     660
   aacttgaaca gtttgcgaag acgttcaaac agagaagg                              698
<210> 437
   <211> 647
   <212> DNA
   <213> Diabrotica virgifera
<220>
   <221> misc_feature
   <222> (1)..(647)
   <223> RNAi target sequence
<220>
   <221> misc_feature
   <222> (524)..(524)
   <223> n can be any nucleotide
<400> 437
   aatttcagcc gtcatctgtt gtgttggtgt gtctgtattt ttgtgaattt cgcaaatatt 60
<210> 438
   <211> 22
   <212> DNA
   <213> Artificial Sequence
<220>
   <223> primer
<220>
   <221> misc_feature
   <222> (1)..(22)
   <223> forward primer
<400> 438
   aatttcagcc gtcatctgtt gt 22
<210> 439
   <211> 22
   <212> DNA
   <213> Artificial Sequence
<220>
   <223> primer
<220>
   <221> misc_feature
   <222> (1)..(22)
   <223> reverse primer
<400> 439
   tgttcaacca aacgagcata tc 22
<210> 440
   <211> 727
   <212> DNA
   <213> Diabrotica virgifera
<220>
   <221> misc_feature
   <222> (1)..(727)
   <223> WCRW transcript ta21805.001_diavv
<220>
   <221> misc_feature
   <222> (540)..(540)
   <223> n can be any nucleotide
<400> 440
   ggcaatcgat accaaaaatt tcagccgtca tctgttgtgt tggtgtgtct gtatttttgt        60
   gaatttcgca aatatttttg attctaataa caaaaccttt tgctaaattc agtgaatcat       120
   agtgctactt tcttgttaaa ttcataaggg aaagaagcat caaccaacaa aaaattgtga       180
   atgttctcct agagagattt caactaactt cagagcaact ctatatcatt caaggaaata       240
   aggagttttt attgactcct gaaatattta atgtactaga taagatccaa gaaatacgca       300
   atgattgtaa aatacttatg cagtctggtc ttcaaacgtt ggctttagat attatggagc       360
   aaatgaccac atatcaggag gcagggttgg aaaaattata tagatggacc caaagtcact       420
   gtagaaatgt tgaaaaccct gaattaacag atctagttac acaatcaatg gcaaggcttc       480
   aacacagaca agttttgttt aagtatgtaa tcgatgaata ctgcatatgt cgcagaagcn       540
   agataactga tgattttatc aacgctctaa cacgaggagg gccatctgga aatcctgctc       600
   ctatagaaat gagagctcac gattctcaga tctatgtcac agatatgctc gtttggttga       660
   acaaagcgat tgctttggaa aagcagaatg ttagtttatt gttgaaattg tgcaataaag       720
   tagagct                                                                 727
<210> 441
   <211> 711
   <212> DNA
   <213> Diabrotica virgifera
<220>
   <221> misc_feature
   <222> (1)..(711)
   <223> RNAi target sequence
<400> 441
   gggggataag ataataaatc gaacatacct gtttcgactc tatgtggaat ttgtcagtgc        60
   atggttatgc aatttagtta aaggttaagg ataataatta gtggaaaaaa tgagttgcgg       120
   ttttgattca caaagaattt atatgctagg tgaaaaatat tcaggtctaa aagctagagg       180
   tgctctccaa aattatgaag aaattagaaa gaaatatctg ctgaacaatg cattattcga       240
   agactcacaa tttcaagtag aaaaagtaga actcggacaa gagtgggaca atcataagaa       300
   caacattaag tggctccgac cccaggaaat ggtttccgat ccagtttttc tagaagctca       360
   tgaagacagg ttcgatatta aacaaggaac tttgggcgat tgctggtttt tagcagcttt 420
   ggcaaacata acagacaata ataaaataat gcagcgtatt ataccagaca atcaaagctt 480
   ttctcaagat tatgcaggag tatttcattt caggttttgg caatatggta gatggataga 540
   cgtggttatt gacgacaaat tacctactat agatggtaaa cttatcttta ctaagaccaa 600
   atttagcaac gtattttgga cacctttatt ggaaaaagca tatgcaaaac tgtatggatc 660
   ttataaaaat attgaggggg gctttatttc tgaagctctg gaagatttca c 711
<210> 442
   <211> 24
   <212> DNA
   <213> Artificial Sequence
<220>
   <223> primer
<220>
   <221> misc_feature
   <222> (1)..(24)
   <223> forward primer
<400> 442
   gggggataag ataataaatc gaac 24
<210> 443
   <211> 23
   <212> DNA
   <213> Artificial Sequence
<220>
   <223> primer
<220>
   <221> misc_feature
   <222> (1)..(23)
   <223> reverse primer
<400> 443
   gtgaaatctt ccagagcttc aga 23
<210> 444
   <211> 2478
   <212> DNA
   <213> Diabrotica virgifera
<220>
   <221> misc_feature
   <222> (1)..(2478)
   <223> WCRW transcript ta05594.001_diavv
<400> 444
   gggggataag ataataaatc gaacatacct gtttcgactc tatgtggaat ttgtcagtgc 60
<210> 445
   <211> 579
   <212> DNA
   <213> Diabrotica virgifera
<220>
   <221> misc_feature
   <222> (1)..(579)
   <223> RNAi target sequence
<400> 445
   cgccattttt gttttcgtag ttggaggcga tgtagggaac tcaatcgatt catacaaaac 60
   ttttactgga atgaagcatt tcagcggaaa cctcatgttt ctgtcctgag ccgtttagaa 120
   gttgtacgta gttgaaatgg gggaaaatat aagtggacac ccggtataat atgcagatga 180
   atgtagaaaa aacacaatat ttatgcctag gtgaagattc aactgatatg atattggaca 240
   atataatcag aaaataacta gttgtggtaa atataagtat ttgggaatta atttcagcaa 300
   agaagatgcg gacgatgcag aaattaagag cacagtcaat aaagaaagaa cgataattag 360
   agatataatt aaatcattga acggagtttt gtggagtacc aagatagaga aacaaagaaa 420
   aatgagaata tacaacacca tgattaagag cacattggta tagggatccg aaacatggag 480
   agtaaaggaa caccaaaaaa gtaaaataga agctaccgag atggacgccc taagaagagc 540
   aagtagaaca tcgagattgg accgggttag aaatgagta 579
<210> 446
   <211> 22
   <212> DNA
   <213> Artificial Sequence
<220>
   <223> primer
<220>
   <221> misc_feature
   <222> (1)..(22)
   <223> forward primer
<400> 446
   cgccattttt gttttcgtag tt 22
<210> 447
   <211> 22
   <212> DNA
   <213> Artificial Sequence
<220>
   <223> primer
<220>
   <221> misc_feature
   <222> (1)..(22)
   <223> reverse primer
<400> 447
   tactcatttc taacccggtc ca 22
<210> 448
   <211> 628
   <212> DNA
   <213> Diabrotica virgifera
<220>
   <221> misc_feature
   <222> (1)..(628)
   <223> WCRW transcript ta21896.001_diavv
<400> 448
   gtaaattcat tatttgcttt acttactcat ttctaacccg gtccaatctc gatgttctac       60
   ttgctcttct tagggcgtcc atctcggtag cttctatttt acttttttgg tgttccttta     120
   ctctccatgt ttcggatccc tataccaatg tgctcttaat catggtgttg tatattctca     180
   tttttctttg tttctctatc ttggtactcc acaaaactcc gttcaatgat ttaattatat     240
   ctctaattat cgttctttct ttattgactg tgctcttaat ttctgcatcg tccgcatctt     300
   ctttgctgaa attaattccc aaatacttat atttaccaca actagttatt ttctgattat     360
   attgtccaat atcatatcag ttgaatcttc acctaggcat aaatattgtg ttttttctac     420
   attcatctgc atattatacc gggtgtccac ttatattttc ccccatttca actacgtaca     480
   acttctaaac ggctcaggac agaaacatga ggtttccgct gaaatgcttc attccagtaa     540
   aagttttgta tgaatcgatt gagttcccta catcgcctcc aactacgaaa acaaaaatgg     600
   cggatttttg aaaaaaactc tgttgccc                                        628
<210> 449
   <211> 539
   <212> DNA
   <213> Diabrotica virgifera
<220>
   <221> misc_feature
   <222> (1)..(539)
   <223> RNAi target sequence
<400> 449
   ctatatgaga cgaggccaga caccatatgg aggagtacat cttgctgatg gcgttgtagc      60
   tgatggttta tgggacgttt acaacaaatt ccatatgggt aactgcacag aaaacacagc     120
   aaagaagctg aacatctcca gggctgaaca ggatgagtat gctatcaaca gttacaagaa     180
   aagtgccgaa gcatacaaaa acaatgcttt tgcttctgaa ttagtatcag ttaatgtacc     240
   acaaaggaaa ggacaaccag atttggtggt ttcagtagat gaagagtaca aaagggttaa     300
   ctttgaaaag tttaacaaac tgccaacggt tttccaaaag gaaaacggca cagtaacagc     360
   tggaaacgct tcaactttaa acgacggtgc agctgctttg atcttaacca ctgccgaaaa     420
   tgccaagagg ttaaacgctg aacctctagc tcgagttgtt ggttaccaag acggtgaatg     480
   tgacccaatc gatttcccaa tagcccctgc cgtagctatt ccaaaattgc tcgaaaaga      539
<210> 450
   <211> 22
   <212> DNA
   <213> Artificial Sequence
<220>
   <223> primer
<220>
   <221> misc_feature
   <222> (1)..(22)
   <223> forward primer
<400> 450
   ctatatgaga cgaggccaga ca 22
<210> 451
   <211> 22
   <212> DNA
   <213> Artificial Sequence
<220>
   <223> primer
<220>
   <221> misc_feature
   <222> (1)..(22)
   <223> reverse primer
<400> 451
   tcttttcgag caattttgga at 22
<210> 452
   <211> 1541
   <212> DNA
   <213> Diabrotica virgifera
<220>
   <221> misc_feature
   <222> (1)..(1541)
   <223> WCRW transcript ta05758.001_diavv
<400> 452 atgaactgat attataattt tataaactat tttaaatcct g 1541
<210> 453
   <211> 603
   <212> DNA
   <213> Diabrotica virgifera
<220>
   <221> misc_feature
   <222> (1)..(603)
   <223> RNAi target sequence
<400> 453
   ggggtgtgta tctctgtgct ataagtttag tgtaaatact gtaaccttga aaattttgaa      60
   gtaacctgtg ttttctagaa aattaaggaa acatcaatga aactttggat cccaagttga     120
   ccttgagcga ttatcagatc accgaaattt acgtggtcgg aaaaggaaca cacagtctta     180
   ctcaagcatt ttccagtagc gacattatgg cattaagaaa agaagaagaa aggaaacaaa     240
   tgcataacaa gactggagga ggtgtattca acttaatttt taggagagga aaatcgagta     300
   tgggttcggg ttcaatctcc agcgatacac gttctatttc gccgactcac agcgacgatt     360
   cccgctcagt tacaccacct gcagtcattc agcaacctat tctgccaccg ccgaagcaag     420
   atccgcctcc cgaacgaccg aaacctccac aacgtaaacg gagacccgct ccgagacctc     480
   cgcagctaac ccctccacag caggagatac ctaaggagga aactgccatt gatgctgaag     540
   attctaaaga atctcgtcga aattcggaag aaaaaccagc gagcaccgtt tcagaagtta     600
   gtg                                                                   603
<210> 454
   <211> 22
   <212> DNA
   <213> Artificial Sequence
<220>
   <223> primer
<220>
   <221> misc_feature
   <222> (1)..(22)
<400> 454
   ggggtgtgta tctctgtgct at 22
<210> 455
   <211> 22
   <212> DNA
   <213> Artificial Sequence
<220>
   <223> primer
<220>
   <221> misc_feature
   <222> (1)..(22)
   <223> reverse primer
<400> 455
   cactaacttc tgaaacggtg ct 22
<210> 456
   <211> 736
   <212> DNA
   <213> Diabrotica virgifera
<220>
   <221> misc_feature
   <222> (1)..(736)
   <223> WCRW transcript ta14242.001_diavv
<220>
   <221> misc_feature
   <222> (627)..(627)
   <223> n can be any nucleotide
<220>
   <221> misc_feature
   <222> (629)..(629)
   <223> n can be any nucleotide
<220>
   <221> misc_feature
   <222> (630)..(630)
   <223> n can be any nucleotide
<220>
   <221> misc_feature
   <222> (631)..(631)
   <223> n can be any nucleotide
<400> 456
   ggtgtgtatc tctgtgctat aagtttagtg taaatactgt aaccttgaaa attttgaagt      60
   aacctgtgtt ttctagaaaa ttaaggaaac atcaatgaaa ctttggatcc caagttgacc     120
   ttgagcgatt atcagatcac cgaaatttac gtggtcggaa aaggaacaca cagtcttact     180
   caagcatttt ccagtagcga cattatggca ttaagaaaag aagaagaaag gaaacaaatg     240
   cataacaaga ctggaggagg tgtattcaac ttaattttta ggagaggaaa atcgagtatg     300
   ggttcgggtt caatctccag cgatacacgt tctatttcgc cgactcacag cgacgattcc     360
   cgctcagtta caccacctgc agtcattcag caacctattc tgccaccgcc gaagcaagat     420
   ccgcctcccg aacgaccgaa acctccacaa cgtaaacgga gacccgctcc gagacctccg     480
   cagctaaccc ctccacagca ggagatacct aaggaggaaa ctgccattga tgctgaagat     540
   tctaaagaat ctcgtcgaaa ttcggaagaa aaaccagcga gcaccgtttc agaagctagt     600
   gagttgcagt cgaaaaaaat cgaaacngnn ntagtcatct gtcattccgg aaatagttca     660
   gatagttcag gataccatga agcatcagtt ttaagtgaca actgcaacac gtcgttgcca     720
   cgaaggccaa aatcgg                                                    736
<210> 457
   <211> 519
   <212> DNA
   <213> Diabrotica virgifera
<220>
   <221> misc_feature
   <222> (1)..(519)
   <223> RNAi target sequence
<400> 457
   cctcgttggt ggagtagtca attcagcatt gtacaatgtt gatggaggtc acagggcagt      60
   tattttcgac aggttcgccg gtattaagaa acaagttata ggcgaaggta cacatttctt     120
   tataccttgg gtacagaaac ctattatctt tgatgtacgg tctcgcccta gaaatatccc     180
   ggtaataaca ggtagcaaag atctgcaaaa cgtaaatatc acactccgta ttctcttcag     240
   accagttcca gacgaattac caaagatcta cacaatttta ggccaggatt atgaagaaag     300
   agttttacca tcaatcacta atgaagtcct aaaagctgtg gtggcacaat tcgatgccag     360
   tgaactgatt actcagagag atgtagtttc ccaaaaagtc agtgaagatt taactgaaag     420
   agctactcaa tttggtgtta tccttgatga tatatcatta acccacttaa cctttggtag     480
   ggaattcaca caagctgtag aattgaaaca agttgccca                            519
<210> 458
   <211> 22
   <212> DNA
   <213> Artificial Sequence
<220>
   <223> primer
<220>
   <221> misc_feature
   <222> (1)..(22)
   <223> forward primer
<400> 458
   cctcgttggt ggagtagtca at **2**2
<210> 459
   <211> 22
   <212> DNA
   <213> Artificial Sequence
<220>
   <223> primer
<220>
   <221> misc_feature
   <222> (1)..(22)
   <223> reverse primer
<400> 459
   tgggcaactt gtttcaattc ta 22
<210> 460
   <211> 1170
   <212> DNA
   <213> Diabrotica virgifera
<220>
   <221> misc_feature
   <222> (1)..(1170)
   <223> WCRW transcript ta06694.001_diavv
<400> 460
   cacaaatcta aatcggcatt ttgatcatca attttgtggt tttttattat aaattcagct      60
   gttaggttga aaaatatttc agtctaaaaa ttacgttata aaagacatcg aaatcttcga     120
   tcgattataa ccataaaagc attttagttt gtttgcattt ttacataact taacctctca     180
   tagtgaactt cacatgtaaa gatgtctgca gctcaaattt tcaacagaat cggccaagtt     240
   ggtctaggaa tagccctcgt tggtggagta gtcaattcag cattgtacaa tgttgatgga     300
   ggtcacaggg cagttatttt cgacaggttc gccggtatta agaaacaagt tataggcgaa     360
   ggtacacatt tctttatacc ttgggtacag aaacctatta tctttgatgt acggtctcgc     420
   cctagaaata tcccagtaat aacaggtagc aaagatctgc aaaacgtaaa tatcacactc     480
   cgtattctct tcagaccagt tccagacgaa ttaccaaaga tctacacaat tttaggccag     540
   gattatgaag aaagagtttt accatcaatc actaatgaag tcctaaaagc tgtggtggca     600
   caattcgatg ccagtgaact gattactcag agagatgtag tttcccaaaa agtcagtgaa     660
   gatttaactg aaagagctac tcaatttggt gttatccttg atgatatatc attaacccac     720
   ttaacctttg gtagggaatt cacacaagct gtagaattga aacaagttgc ccaacaggaa     780
   gctgagaaag ctaggttttt ggtagaaaaa gctgaacaga caaagaaagc aactgtaatc     840
   tcagcggagg gtgatgccca agctgctgtg cttcttgcca aggcttttgg tgatgctgga     900
   gaaggtttag tagaactgag acgtatagaa gcagctgaag atatcgcata ccaactatca     960
   aggtcaaggc aggtttccta cttgcctgga ggacaaaatt tgttgttgaa tgtgccaaca    1020
   cctggtaatt agatggatag agtaacaaac aaaattggag ctagttgatt atttggctct    1080
   atgaatgaga gagttgccca tttagctttt atggttctat tattttttat aaaagtatgt    1140
   taataaattg ttgataaatt ttaaaaaaaa                                     1170
<210> 461
   <211> 602
   <212> DNA
   <213> Diabrotica virgifera
<220>
   <221> misc_feature
   <222> (1)..(602)
   <223> RNAi target sequence
<400> 461
   gggaagactc ccaagatatg gtcgacgagt ccacagatca cgtttccaga acgaacgaaa      60
   aatcgacgat attcgaccag gttaaacagt ttttaaagaa taaccatcaa attcctggca     120
   tggcagattt agaagctaag caagaagaag ttttaaaaca gcttgcagac ctcaagaaac     180
   aaattctctc aattaaattg gatttgaaaa ttaatgcaaa ctcagtagga acaaacaaga     240
   cctgtccaag tatttcagca gaaaagctga atgctcttcc tgatcttgtt gtgaatgcaa     300
   gtccaacaaa tccgccatat tcattactga taattcaaaa gctactacgt gacattgtcg     360
   aattgaaggt tacatcccat atacactcct cagttccaac attgaatgaa aatgccaaac     420
   agctgggtga ctcattggca agttacgtac cgaaacgtgg tgtaccaagt gttaacatta     480
   aattgatatg gaaaaatgtg ggcatcgaca cagaactcat agtatcacac gttccaattt     540
   ttggagaagt caacttatta cggtatctaa ccagggtaat cagttcccca ctcagataca     600
   gt                                                                    602
<210> 462
   <211> 20
   <212> DNA
   <213> Artificial Sequence
<220>
   <223> primer
<220>
   <221> misc_feature
   <222> (1)..(20)
   <223> forward primer
<400> 462
   gaagactccc aagatatggt 20
<210> 463
   <211> 23
   <212> DNA
   <213> Artificial Sequence
<220>
   <223> primer
<220>
   <221> misc_feature
   <222> (1)..(23)
   <223> reverse primer
<400> 463
   actgtatctg agtggggaac tga 23
<210> 464
   <211> 1170
   <212> DNA
   <213> Diabrotica virgifera
<220>
   <221> misc_feature
   <222> (1)..(1170)
   <223> WCRW transcript ta07588.001_diavv
<400> 464
   taattatgta attaggctga gtaaatatga agttttgcct cttttaagga taacatacaa      60
   tatcatctgg accccttttt aagaacgcca gtgaaaaatt atttagcaga cgaagactga      120
   ttaccaaaaa aattatatat cgaatttcta aagttaattg taacatttta gcgaagaaaa     180
   atgaatgggc cgataaaaat gtatcgaaca cgacaaataa ttcaccacga ccaaaagatt     240
   gacaagccga agtgtatgta tgctttaaaa aatatctact caaaagactc ccaagatatg     300
   gtcgacgagt ccacagatca cgtttccaga acgaacgaaa aatcgacgat attcgaccag     360
   gttaaacagt ttttaaagaa taaccatcaa attcctggca tggcagattt agaagctaag     420
   caagaagaag ttttaaaaca gcttgcagac ctcaagaaac aaattctctc aattaaattg     480
   gatttgaaaa ttaatgcaaa ctcagtagga acaaacaaga cctgtccaag tatttcagca     540
   gaaaagctga atgctcttcc tgatcttgtt gtgaatgcaa gtccaacaaa tccgccatat     600
   tcattactga taattcaaaa gctactacat gacattgtcg aattgaaggt tacatcccat     660
   atacactcct cagttccaac attgaatgaa aatgccaaac agctgggtga ctcattggca     720
   agttacgtac cgaaacgtgg tgtaccaagt gttaacatta aattgatatg gaaaaatgtg     780
   ggcatcgaca cagaactcat agtatcacac gttccaattt ttggagaagt caacttatta     840
   cggtatctaa ccagggtaat cagttcccca ctcagataca gttctgactc tgatagtgta     900
   gaaatcgatt cgatattaga cacttgttat cttcttctga gagctaaaac gaaaactgaa     960
   aaggcaggac atattgccac attaaataaa tccttaggaa aatcccaatg gttagcaggt    1020
   agaagtaggc atagtctggg ggatctagcc gcatactctg cccttaaaca ggttacaagt    1080
   ggtagtgaaa ttaatgcaaa tatgacgaag tggtatcagc ggtgtgaaac tgttgtataa    1140
   aattattttt gttataaata taagctttaa                                    1170
<210> 465
   <211> 508
   <212> DNA
   <213> Diabrotica virgifera
<220>
   <221> misc_feature
   <222> (1)..(508)
   <223> RNAi target sequence
<400> 465
   caaaacgaat gtgttgagtg tgtttttgtg gatatggttc tggttaataa ggcgcttgat      60
   ttatctcata gataacataa tttttgttaa aactggtata actgctgatt attattattc     120
   tctttgtcaa caagagtgtt acgtatagtg ttatgtatag aacattcacg aagggttgct     180
   tcagcttccg gtcagaccat gcccgtctta cccacgaccc acgatgaggt agcaagacat     240
   gatttcgaca ccgtcaccat cgattcatat ggaatgagcc ccttggcctt gcagaaaatt     300
   agggagcaga tggctttgag cctggagcga accaagcagt tggaggacca gatcaagagc     360
   atgcacaaat tggaggagga ggtcaacttc ctgaaggatg aaaattgtag gttacgaaac     420
   agtttgaaat cagaaaacgg aaaaaaccaa gccacaataa atgacaatta tatcgaacat     480
   tacaaacgac agaggtctca atcattca                                        508
<210> 466
   <211> 22
   <212> DNA
   <213> Artificial Sequence
<220>
   <223> primer
<220>
   <221> misc_feature
   <222> (1)..(22)
   <223> forward primer
<400> 466
   caaaacgaat gtgttgagtg tg 22
<210> 467
   <211> 22
   <212> DNA
   <213> Artificial Sequence
<220>
   <223> primer
<220>
   <221> misc_feature
   <222> (1)..(22)
   <223> reverse primer
<400> 467
   tgaatgattg agacctctgt cg 22
<210> 468
   <211> 3569
   <212> DNA
   <213> Diabrotica virgifera
<220>
   <221> misc_feature
   <222> (1)..(3569)
   <223> WCRW transcript ta01520.001_diavv
<400> 468
   ggggaactgt gttttgttca acaaaaaata agtacctgtc acaaagtaca tatatggaga      60
   acataaacaa aacgaatgtg ttgagtgtgt ttttgtggat atggttctgg ttaataaggc     120
   gcttgattta tctcatagat aacataattt ttgttaaaac tggtataact gctgattatt     180
   attattctct ttgtcaacaa gagtgttacg tatagtgtta tgtatagaac attcacgaag     240
   ggttgcttca gcttccggtc agaccatgcc cgtcttaccc acgacccacg atgaggtagc     300
   aagacatgat ttcgacaccg tcaccatcga ttcatatgga atgagcccct tggccttgca     360
   gaaaattagg gagcagatgg ctttgagcct ggagcgaacc aagcagttgg aggaccagat     420
   caagagcatg cacaaattgg aggaggaggt caacttcctg aaggatgaaa attgtaggtt     480
   acgaaacagt ttgaaatcag aaaacggaaa aaaccaagcc acaataaatg acaattatat     540
   cgaacattac aaacgacaga ggtctcaatc attcacaaac gtaaacaatt tggagatctc     600
   aatgcgaaaa gaatcatccc caccacctcc accaccacga aaagattttg gagtgatgtg     660
   tggggtgttg acgaggaaca taggagtagg caatcagaca cctaacacaa agaatgtttc     720
   tacggctact tttgaaaatg aattcgctga taagtggctg ccagaaaagg tcaaatttct     780
   taacaatcag aacttgaagt catctagtaa aatcacgata tcaaaagaaa cgcaaaccct     840
   aggacaaaag tcaagctcag ataaaggtgt ccaattccaa acaaagagtc ctacagtgcc     900
   taagttcagc acctacacgc aaactacaac aactaaaaaa caactagtgg ataaagggat     960
   cttagcaact gtcaaagtat ctcatatttc tgtccagaaa tgtgttgaat gttactctat    1020
   tggcacttca gatgacacag taaatgatat catttgcgaa aaatgcaatg ctacaaaaaa    1080
   gatctatagc aataggtcct gaatctaatg aagatagcca tacgtcgcct atatccttag    1140
   cgtcccttaa cttaagaagc aaatcgttca atatgggcga agatcgactt aaccttacca    1200
   ctagaacccg ttccatagca tcacagtacg aatctagtag catcagcaga gcttgtcagg    1260
   cgaaacttaa gaaggattct tctacgaaat cttgccaagc tgaaacgaaa atgactgaca    1320
   aaaacaccca gtacgtaaat tacactgtct caaggcatac cgacactcaa gactttataa    1380
   caccaaagaa aaacgtcagt tgcgaagcag ttgaacaaaa accggaaacc attgaggtgg    1440
   cttgtaatac cgcgactgat ttgctgacct gcgtcaaatg tgaaaaagaa aaagaaaagg 1500
   aggcgctgaa gaaagacgga ggcagcccca ctccatctag aataccgcga ccacaaattc 1560
   cgactactcc cgtagaaaat agaaagttta ggagacaaga tacttatact aaagtatatt 1620
   caccaactga gaaagctaca tcacctacta gtccaagatt gtctggttta gatctatcac 1680
   ctaaagtcat atcaaacgat tcaaatccct tagataaagt taaaagtaac ctcaaactag 1740
   tagaaccaat accacatgct caaaatggtg tcaatttcac agcgcctatt accaaagttc 1800
   aaattcaaga aattacactg ccaacttcag aatctgtttt ccaacccatt acacaaaatc 1860
   aaaataggaa aaaagtcatg ccaagtaagg aaatgcaggg tgcaatgaaa gtcctgaacg 1920
   atcatcttat gaagaaccct tccaaaaaca tcaagaaccc aactgctgaa aatattattc 1980
   aaacagaatg gttcaaaatc tcgagctcag taacagccaa tccattggaa gtggaggatt 2040
   acctggatgc tttcgaagac gtgtccagct tgttactgga atacatagta aacatgagtg 2100
   attatagtgg gaacacagcc atgcactatg ctgtttccca tggcaacttt gatgtcgtat 2160
   ccattttgtt agattctaaa gtttgtgata tcaataaaca aaataaggct ggatatacaa 2220
   gtgttatgtt agtttctttg gcggaagttc ggtcccaaac acatgccaac gttgtccgaa 2280
   gactgttcca gttagctgat gtgaatatta gagcgaaaga gcatggtcaa acagcactga 2340
   tgttagcagt aagccacaat aggttgaaca tggtcaagat gttgttggaa gcaggtgcag 2400
   atataaatat tcaggacgaa gatggcagta cggcgttaat gtgtggtgct gaacacggcc 2460
   atatcgaaat agttaaacat ttcctttccc aacctgactg caattcgacc attatggata 2520
   tggatggcag cacagctcta gcaatagcta tggaagccgg tcaccgacac atcggagtcc 2580
   tgctctacgc tcatgagagg aatagccaaa agtctcataa cgtgaagtta aaaaaagcaa 2640
   aatcaacaaa tagtccgaaa accccttcct caccactggc tgcaagaagt agccatcgtg 2700
   ccttaacaga tacgaaacct aaatagtact aacccatttc taactgtgtt ttaacttaag 2760
   aaggtaatgc gtgttgtttc gctgttaacg attctgttaa ccatcgtgat gtattcatat 2820
   acgatggttc tagatttttg gcatgtaaaa tgcatgttta ttcagtgtca tcaagatagg 2880
   gaacccaaag ttattcatcc ggtccaaaca actctatcta gatgaacata ttcttatgta 2940
   taatggaaaa gaatcttagt cgatatatgg cctggcgcaa atttgaccca accgagacac 3000
   aatacactcc ggggccacgg atgacccttg catttatttt tctactgcag cgcatattga 3060
   acccaaccgt gggctgtcgc cgtggccagt tgctaaaatg tagtgatgtt gattatagtt 3120
   attttcaagt atctatgtat tcgttacttt tgtgtaaagt aataatttac attattcgtt 3180
   acattgatta ctatgaatac ttcgaatatt tatgtatttc actaacagta atcatataga 3240
   aaatcggaat tcctcagtat tttgaatata ccttacaata ttgtaacaca agtgagcgct 3300
   tccaacaaat ttaaacatat tttaagtgat atgcgttact attgataata caaaaataaa 3360
   aaccgtgaat acataaaaac aatacaatat ataataaaac aatgaaatac ataaaaacaa 3420
   tgaaatacat gtaaaattaa agttgttgat tatagtgtag atacattatc atttccacat 3480
   atttttagtc aatctaaagc tattttttat ctaagactaa taatatacaa ccgcgtaacc 3540
   aggatgatcc taaggggggg ggggttacg 3569
<210> 469
   <211> 525
   <212> DNA
   <213> Diabrotica virgifera
<220>
   <221> misc_feature
   <222> (1)..(525)
   <223> RNAi target sequence
<400> 469
   gggaaccatc tgataccatt gagaatgtca aagctaagat ccaagataag gaaggtatcc 60
   caccagacca gcaaagattg atctttgcag gaaaacagct agaagatggt agaaccttgt 120
   ctgactataa catccagaaa gagtccactc ttcacttggt actgagattg agaggaggta 180
   tgcagatctt cgtcaagaca ctaactggaa agactatcac tttggaagta gaaccatctg 240
   atactattga gaatgtgaaa gctaagatcc aagataagga aggtatccca ccagaccagc 300
   aaagattgat ctttgcaggg aaacagctag aagatggtag aaccttgtct gactataaca 360
   tccagaaaga gtccactctt cacttggtac ttcgattgag aggaggtatg cagatcttcg 420
   tcaagacact aactggaaag actatcactt tggaagtaga accatctgat accattgaga 480
   atgtgaaagc caagatccaa gataaggaag gtatcccacc agatc 525
<210> 470
   <211> 20
   <212> DNA
   <213> Artificial Sequence
<220>
   <223> primer
<220>
   <221> misc_feature
   <222> (1)..(20)
   <223> forward primer
<400> 470
   gaaccatctg ataccattga 20
<210> 471
   <211> 22
   <212> DNA
   <213> Artificial Sequence
<220>
   <223> primer
<220>
   <221> misc_feature
   <222> (1)..(22)
   <223> reverse primer
<400> 471
   gatctggtgg gataccttcc tt 22
<210> 472
   <211> 703
   <212> DNA
   <213> Diabrotica virgifera
<220>
   <221> misc_feature
   <222> (1)..(703)
   <223> WCRW transcript ta21603.001_diavv
<400> 472
   gcacgaggct tggtacttag attgagagga ggtatgcaga tttttgtcaa gacattgact 60
   ggtaaaacta tcacattaga aggggaacca tctgatacca ttgagaatgt caaagctaag 120
   atccaagata aggaaggtat cccaccagac cagcaaagat tgatatttgc aggaaaacag 180
   ctagaagatg gtagaacctt gtctgactat aacatccaga aagagtccac tcttcacttg 240
   gtactgagat tgagaggagg tatgcagatc ttcgtcaaga cactaactgg aaagaccatc 300
   accttagaag tagaaccatc agataccatt gagaatgtga aagctaagat ccaagataag 360
   gaaggtatcc caccagacca gcaaagattg atctttgcag ggaaacagct agaagatggt 420
   agaaccttgt ctgactataa catccagaaa gagtccactc ttcacttggt acttcgattg 480
   agaggaggta tgcagatctt cgtcaagaca ctaactggaa agactatcac tttggaagta 540
   gaaccatctg ataccattga gaatgtgaaa gccaagatcc aagataagga aggtatccca 600
   ccagatcagc aaagattgat ctttgcaggt aaacaattgg aagatggaag aaccttatct 660
   gattataaca tccagaaaga gtccactctt catttggtac tta 703
<210> 473
   <211> 653
   <212> DNA
   <213> Diabrotica virgifera
<220>
   <221> misc_feature
   <222> (1)..(653)
   <223> RNAi target sequence
<220>
   <221> misc_feature
   <222> (623)..(623)
   <223> n can be any nucleotide
<400> 473
   gatcagctaa gaggctgaca ccacgccatt ttgctttgag aacacgtttt tggattttat      60
   atacagtaaa ttagtctatt aatttatcaa aatgtctgga gaagaaacat ctgccgatag     120
   aaatgttgag atatggaaaa taaagaaact tataaaaagt ttggaactag caagagggaa     180
   tggaacaagt atgatctcac ttataattcc tccaaaagat caaatatcta gagtaagcaa     240
   aatgttagct gatgaatttg gtacagcctc taatattaag tctcgagtaa atagactttc     300
   agttcttggt gctatcacat cagtccagca ccgtttaaaa ctatatacca aggtccctcc     360
   taatggatta gtcatttact gtggaactat tgtaactgat gaaggaaagg aaaagaaagt     420
   aaacatagac tttgaaccat tcaaaccaat taatacctca ttgtatttat gtgacaataa     480
   gttccataca gaagccctga cagcattatt ggcagatgat aataaatttg gttttatagt     540
   tatggatggt aatggtgcat tattcggtac actacaagga aacacaaggg aggtacttca     600
   caagtttact gtagatttac ccnagaaaca cggtagagga ggtcagtctg cac            653
<210> 474
   <211> 22
   <212> DNA
   <213> Artificial Sequence
<220>
   <223> primer
<220>
   <221> misc_feature
   <222> (1)..(22)
   <223> forward primer
<400> 474
   gatcagctaa gaggctgaca cc 22
<210> 475
   <211> 22
   <212> DNA
   <213> Artificial Sequence
<220>
   <223> primer
<220>
   <221> misc_feature
   <222> (1)..(22)
   <223> reverse primer
<400> 475
   gtgcagactg acctcctcta cc 22
<210> 476
   <211> 2829
   <212> DNA
   <213> Diabrotica virgifera
<220>
   <221> misc_feature
   <222> (1)..(2829)
   <223> WCRW transcript ta00933.001_diavv
<400> 476
   aaacaattta gggtggattt gaaattaaat ttaatcaaaa tttggtggac aaaataaaat      60
   cccaattttc aaatttttgt tttttatgac agatatctga actgaattat caattggata     120
   tgccaaatca cctaaacaga atattgttct ttacctgaat ttacagttaa ggccgtgaat     180
   atccagttaa aaccagttgg tttcagttaa taactagtga aaagtgactt tcctggatcc     240
   tcttgttgat cagctaagag gctgacacca cgccattttg ctttgagaac acgtttttgg     300
   attttatata cagtaaatta gtctattaat ttatcaaaat gtctggagaa gaaacatctg     360
   ccgatagaaa tgttgagata tggaaaataa agaaacttat aaaaagtttg gaactagcaa     420
   gagggaatgg aacaagtatg atctcactta taattcctcc aaaagatcaa atatctagag     480
   taagcaaaat gttagctgat gaatttggta cagcctctaa tattaagtct cgagtaaata     540
   gactttcagt tcttggtgct atcacatcag tccagcaccg tttaaaacta tataccaagg     600
   tccctcctaa tggattagtc atttactgtg gaactattgt aactgatgaa ggaaaggaaa     660
   agaaagtaaa catagacttt gaaccattca aaccaattaa tacctcattg tatttatgtg     720
   acaataagtt ccatacagaa gccctgacag cattattggc agatgataat aaatttggtt     780
   ttatagttat ggatggtaat ggtgcattat tcggtacact acaaggaaac acaagggagg     840
   tacttcacaa gtttactgta gatttaccca agaaacacgg tagaggaggt cagtctgcac     900
   ttcgttttgc ccgtctgaga atggaaaaac gtcataacta tgttcgaaaa gtagcagaag     960
   tagctacgca attatttata acaaatgaca aacctaacat agctggactc atcttggcgg    1020
   gtagtgccga ttttaagact gaacttagtc aatctgatat gtttgatcca aggttacaag    1080
   ctaaagtaat caaactagta gatgtctcgt atggtggtga gaatggattt aatcaagcca    1140
   tcgaattagc agctgaatca cttcaaaatg taaagtttat tcaagaaaag aaactgatag    1200
   gcagatattt cgatgaaatt agccaagata cggggaaata ctgttttgga gttgaagata    1260
   ctcttagggg cttggaatta ggtgctgtag aaacactaat ctgttgggag aatttagata    1320
   ttcagcgata tgtattgaaa aaccatgcta caggttcgga aactatactg catttaactc    1380
   ctgaacaaga aaaagataaa tcacattttt cggataaaga gactggtgta gaattggaat    1440
   tggtcgagtg tcaacctctt cttgaatggt tggcgaataa ttacaagaac ttcggtgcca    1500
   ctttagaaat tattacagac aaatctcaag agggctccca atttgttcga gggtttggtg 1560
   gaattggagg tttgctccgg tacaaagtag acttccaaag tttacaacta gacgatatgg 1620
   ataacggcga agaatttgat ctagatgatt attaaatgta gagacaaatt tgtagaaatt 1680
   ttcatttaaa acaatgtggg cccgttatgt aataaacacg tgtaaaaagt aaatgatggc 1740
   aatatactaa tttatcctaa aggagtataa tatgatgtgc ttttttgtaa tattatttcc 1800
   aaatttatgc tttcacaaac ttgaaagttt attccatgaa aagttttaat tatatcatta 1860
   aattaataag tattattgat taattttatt tattgattaa tctatgttag taaaatgtac 1920
   aaagcacaaa gataaagata gttaggataa ccctttacac caactgaaag tatagaaatt 1980
   tccggattgt ttggtacggc aacaaacaaa ttcacacgaa attccaaatt tgataatgtc 2040
   tactacagtt gaagatgaca atagataatt ccagaccacg acctatacgc tcagaaaaca 2100
   atgtaccatt acaaaacggc tgcgaaagcc tacattcata cttgattcgt atacatactg 2160
   tccatgggcg ctcataccca tgggcagggt agggggcgtg gccccctggc ttttcgggtg 2220
   ctttaaacta tatgtcgtta tccaaagtat acatacctaa ggtaatgtgc aacatcttca 2280
   cgtctgccct ccccctaaaa atttttatat gggcgcccgt gatactgtcc taagttacac 2340
   atccattttt gtccacgtgt gtgaccagga gcgccattac caacggttcc caagactgag 2400
   agtcatagct tactcggagc agtagggatt taaagaagaa aaaacgagct atgtacttgt 2460
   cagtgtgtcg gatcacacac agatcggttt agtagtttgt ataaattata taaaataaaa 2520
   atgtagattt ggaaataata ttacaataat atcggagtga tcatgtgttt tgtataaatt 2580
   atattgtctc aaaaaatgtt taccgatatt tattatttaa ttgttaaaat tatgtatgta 2640
   tattgcaagt gtttataatt aatagtgaaa aagaccagta tttctacagt ggcttgtttt 2700
   tttcattggt ttttatatat ttagaacaga atattttttg tatttacttt ttttgttaac 2760
   gaggtaataa atcaggtttg gaaattcttc attctatttt agtttgtaac cattggcatt 2820
   tttgttttt 2829
<210> 477
   <211> 577
   <212> DNA
   <213> Diabrotica virgifera
<220>
   <221> misc_feature
   <222> (1)..(577)
   <223> RNAi target sequence
<400> 477
   ctgactctgt tcattgggga gtggttgaaa ggggtgtttt ttggagttat tccattattt        60
   gaaggagtga taaagaagat cttggaagcc accatagact taattcacat agaaccagca       120
   tactttctga tcaccaagaa gaaatgactg agtattggtt gatatctgcc ccaggggata       180
   agacctgtca acagacatgg gacacgatga ataatatgac aagtaaacag aataatttgt       240
   caaccaacta caagtttcaa atcccggact tgaaagttgg tactctggat cagttagtag       300
   gactttctga tgatctgggc aagcttgatg ggttcgtgga gcaggttacc aggaaggtag       360
   ctcagtacct tggagaagta cttgaagaac agagggacaa gtggtcagaa aacctgcagg       420
   ccaataacag cgatttgccc acttatttaa cccgatttac ttgggacatc gccaaatatc       480
   ccatcaagca atcgctccgt aacatcgccg atataatcag caaacaagtt ggccaaattg       540
   acgctgatct gaagaccaaa tcgtctgcgt acaataa                                577
<210> 478
   <211> 22
   <212> DNA
   <213> Artificial Sequence
<220>
   <223> primer
<220>
   <221> misc_feature
   <222> (1)..(22)
   <223> forward primer
<400> 478
   ctgactctgt tcattgggga gt 22
<210> 479
   <211> 22
   <212> DNA
   <213> Artificial Sequence
<220>
   <223> primer
<220>
   <221> misc_feature
   <222> (1)..(22)
   <223> reverse primer
<400> 479
   ttattgtacg cagacgattt gg 22
<210> 480
   <211> 2409
   <212> DNA
   <213> Diabrotica virgifera
<220>
   <221> misc_feature
   <222> (1)..(2409)
   <223> WCRW transcript ta01215.004_diavv
<400> 480
   ggtgaaactt caataacgat cagctgggct gactctgttc attggggagt ggttgaaagg      60
   ggtgtttttt ggagttattc cattatttga aggagtgata aagaagatct tggaagccac     120
   catagactta attcacatag aaccagcata ctttctgatc accaagaaga aatgactgag     180
   tattggttga tatctgcccc aggggataag acctgtcaac agacatggga cacgatgaat     240
   aatatgacaa gtaaacagaa taatttgtca accaactaca agtttcaaat cccggacttg     300
   aaagttggta ctctggatca gttagtagga ctttctgatg atctgggcaa gcttgatggg     360
   ttcgtggagc aggttaccag gaaggtagct cagtaccttg gagaagtact tgaagaacag     420
   agggacaagt ggtcagaaaa cctgcaggcc aataacagcg atttgcccac ttatttaacc     480
   cgatttactt gggacatcgc caaatatccc atcaagcaat cgctccgtaa catcgccgat     540
   ataatcagca aacaagttgg ccaaattgac gctgatctga agaccaaatc gtctgcgtac     600
   aataacttga aaggaagtct tcagaatttg gagaagaaac agacaggaag tttactaaca     660
   agaaacttgg cagatttggt gaaaaaggaa cattttattt tggattctga atatttgcag     720
   acattgttgg ttattgttcc aaaagcccaa ttcaacgaat ggaatgcgac atacgaaaag     780
   attaccgaca tgatagtccc tcgttcttca caactgatta aacaggataa cgaatacggt     840
   ctatatactg tatccctatt caaaaaggtc gtcgaagaat tcaagttaca cgctagagaa     900
   aagaagttca tcgttcgtga tttcatatat aacgaagagg aactggcagc tggaaagaac     960
   gagataacca aactcgtcac cgataaaaag aagcaatttg gtccattagt tagatggctt    1020
   aaagtcaact tcagcgagtg tttctgcgcc tggattcacg ttaaagcgtt gagggtattt    1080
   gttgaatctg ttttaagata tggcctcccc gtcaatttcc aagctatttt aatccacccc    1140
   aataagaaac aattaaaacg tttaagagat gtcttgaacc aactttacgg ccacctcgat    1200
   agtagtgctg ccctttcagg acctaacgtt gatagcgttg acattccagg cctcggattc    1260
   ggccaatcag agtattaccc atacgtttac tacaagctga acgtcgacat gttagaatcg    1320
   aagatctaaa ctcacgcatc aatccaagag tttgttatta ataggttcca aacaatttta    1380
   aaaaaacgta ctacagatac aaatagtata tttttgatta ctcactcttt cgaggggctg    1440
   ttttaatgtc ggtattttta agttttgtcc ttactcaatg tccatttagt accccgtcat    1500
   tgtatgtaaa ttaattaaaa atgcgcaaca aaaaatgtag aacttttttt tattttaaaa    1560
   tgcatttaaa attcgtttaa cgatgatata attgctttaa tgtatcatgt aatactccga    1620
   ttgaacatat aaatcgttat taatattatt tcttgttgat aatacaaaag agaccatatt    1680
   attagaaact gcattcgaaa aattaaaaaa atgatagata ttatgacgcg tagatgagct    1740
   gacaagtgac gtgttaagat gtattgatat ctaaacatga aatttcaaca aaccatctat    1800
   gttatagatg tacatatgtt tcgtaaaatt gacgaaaagc tggaaataga ttacatataa    1860
   tttgtcataa aatatttgcg tcagagactt gaggttgtga tacaatatat atttactcct 1920
   acagaatatt tgttgaatat taccgacacc attaaaatac cttatccgtt atgattgtag 1980
   ataaataatt tactctgaat ttaatacaaa acgatgtcat aatttcaatt gtacttgttc 2040
   taataatata actacttatc gaaaaagaat agaaatttat tataaaattg tgtgaaatga 2100
   ctgggtatta taggattgcc ccaacaaaat attttagatg tagtatgatg cttcatcagt 2160
   gacaatatat ttgacgtgtt ttattatggg ccactcttta atgtaaacca gtattgattt 2220
   attgtaaata aatattgtat atatactaca aaccaataaa atattttgcc ttttgaatct 2280
   atttataaaa gatgattcca tattgtaata tagttcaatt agttgactac tttttttgaa 2340
   tttatttcag aaataatttt aagtggttat ttgttcaatg taattgttag aaaataaaaa 2400
   ctattccaa 2409
<210> 481
   <211> 689
   <212> DNA
   <213> Diabrotica virgifera
<220>
   <221> misc_feature
   <222> (1)..(689)
   <223> RNAi target sequence
<220>
   <221> misc_feature
   <222> (338)..(338)
   <223> n can be any nucleotide
<220>
   <221> misc_feature
   <222> (551)..(551)
   <223> n can be any nucleotide
<400> 481
   gggcttcttt ctgtggttaa gtccaaaatg gcaccgacag ctgacaaaac taaaacggtt      60
   aaagggctcc ccgcagtccc agaatcagtc ttaaaacacc gtaaaagacg tgaagcatcc     120
   cgtgctaaac aactccagtc tgtcatcaaa aagaaggcag aacagatcaa aaagaggaaa     180
   gatattttca aaagagctga acagtatgtt aaggaatacc gtttaaaaga aagagacgaa     240
   gttaggctag ttagacaagc caaaaccaga ggaaatttct atgttcctgc tgaggctaaa     300
   ttggcttttg ttgttcgtat taagggtatc aataaagnag cacctaaagt acgcaaagta     360
   ctccaactat tccggttact tcaaatcaac aatggagttt tcgttaagtt gaacaaggct     420
   actattaaca tgcttaggat atgcgaacct tacattacat ggggatatcc taacttaaag     480
   tcagtaagag aattaatcta caaaagagga tttgccaaag ttaatggaca gcgtgtagca     540
   attaccagca ntcaaatcgt tgaagacagg ttggggaaaa tctggaatca tttgtgttga     600
   agatttaatc cacgaaattt tcactgttgg acctaagttc aaatatgcct ccaacttctt     660
   gtggcccttc aagctaaaca cacccactg                                       689
<210> 482
   <211> 20
   <212> DNA
   <213> Artificial Sequence
<220>
   <223> primer
<220>
   <221> misc_feature
   <222> (1)..(20)
   <223> forward primer
<400> 482
   gcttctttct gtggttaagt 20
<210> 483
   <211> 22
   <212> DNA
   <213> Artificial Sequence
<220>
   <223> primer
<220>
   <221> misc_feature
   <222> (1)..(22)
   <223> reverse primer
<400> 483
   cagtgggtgt gtttagcttg aa 22
<210> 484
   <211> 855
   <212> DNA
   <213> Diabrotica virgifera
<220>
   <221> misc_feature
   <222> (1)..(855)
   <223> WCRW transcript ta05864.001_diavv
<400> 484
   ggggggcgtg tggggggtct ttctgtggtt aagtccaaaa tggcaccgac agctgacaaa      60
   actaaaacgg ttaaagggct ccccgcagtc ccagaatcag tcttaaaaca ccgtaaaaga     120
   cgtgaagcat cccgtgctaa acaactccag tctgtcatca aaaagaaggc agaacagatc     180
   aaaaagagga aagatatttt caaaagagct gaacagtatg ttaaggaata ccgtttaaaa     240
   gaaagagacg aagttaggct agttagacaa gccaaaacca gaggaaattt ctatgttcct     300
   gctgaggcta aattggcttt tgttgttcgt attaagggta tcaataaagt agcacctaaa     360
   gtacgcaaag tactccaact attccggtta cttcaaatca acaatggagt tttcgttaag     420
   ttgaacaagg ctactattaa catgcttagg atatgcgaac cttacattac atggggatat     480
   cctaacttaa agtcagtaag agaattaatc tacaaaagag gatttgccaa agttaatgga     540
   cagcgtgtag caattaccag caatcaaatc gttgaagaca ggttgggaaa atctggaatc     600
   atttgtgttg aagatttaat ccacgaaatt ttcactgttg gacctaagtt caaatatgcc     660
   tccaacttct tgtggccctt caagctaaac acacccactg gtggatggcg taagaagact     720
   aaccactatg ttgaaggagg tgactttggt aatagagaag atagaattaa tgaacttctc     780
   agaaggatgg tttaaaccaa tttgtttata aaataaatta tgtataaaac acaaaaaaaa     840
   aaaaaaaaaa tgcga                                                      855
<210> 485
   <211> 327
   <212> DNA
   <213> Diabrotica virgifera
<220>
   <221> misc_feature
   <222> (1)..(327)
   <223> RNAi target sequence
<400> 485
   acacttacac ccttcaaaaa ctcatttata aactaaaaat gggtgcatct gtaattcctt      60
   tcatagtttt cactgtatta tggggaggag ttggagctgt tctacccatt tttgtaccta     120
   aaggacccaa tagaggaatt atccaagtta ttcttatgat aacaggtgtg tcgtgctggt     180
   tgttctggct gtgttgttac gtagctcaaa tgaatcctct gatcggtcct aaactagaac     240
   aaaacatcgt gctgattatg gcaaaacaat ggaaatatgc attaccatcg tcagcttgaa     300
   tgaaggtgtg agtgaccatt ccatctg                                         327
<210> 486
   <211> 24
   <212> DNA
   <213> Artificial Sequence
<220>
   <223> primer
<220>
   <221> misc_feature
   <222> (1)..(24)
   <223> forward primer
<400> 486
   acacttacac ccttcaaaaa ctca 24
<210> 487
   <211> 22
   <212> DNA
   <213> Artificial Sequence
<220>
   <223> primer
<220>
   <221> misc_feature
   <222> (1)..(22)
   <223> reverse primer
<400> 487
   cagatggaat ggtcactcac ac 22
<210> 488
   <211> 656
   <212> DNA
   <213> Diabrotica virgifera
<220>
   <221> misc_feature
   <222> (1)..(656)
   <223> WCRW transcript ta06727.001_diavv
<400> 488
   ttcaaggagt ggttagattg tatgcactga atttctgaag gcagatgatt ttgacagatt 60
   ttagtttact ctacttgtaa agtgaaaatc tattcacgtg aattatttta cacttacacc 120
   cttcaaaaac tcatttataa actaaaaatg ggtgcatctg taattccttt catagttttc 180
   actgtattat ggggaggagt tggagctgtt ctacccattt ttgtacctaa aggacccaat 240
   agaggaatta tccaagttat tcttatgata acaggtgtgt cgtgctggtt gttctggctg 300
   tgttgttacg tagctcaaat gaatcctctg atcggtccta aactagaaca aaacatcgtg 360
   ctgattatgg caaaacaatg gaaatatgca ttaccatcgt cagcttgaat gaaggtgtga 420
   gtgaccattc catctggata gtgtgtagaa atttcaaatt gcacagctat tgcattgtat 480
   tatatttatt attaatttcc tttttgtgat catccaatat ctgtatgtag tatgttttaa 540
   agtgatgtgt ataatattga ttattgttta tatactatca tcaaaaccaa ccattaattg 600
   taaaaaaaat attgttgaat taaattaatt tgtcagttaa aaaaaaaaaa aaaaaa 656
<210> 489
   <211> 526
   <212> DNA
   <213> Diabrotica virgifera
<220>
   <221> misc_feature
   <222> (1)..(526)
   <223> RNAi target sequence
<400> 489
   tgtatttgtg actggaaaag gaaattagag ccctatactt acagttaaac gcttataaat 60
   ttgtagcaag tattttatct ttacaaaaga aagtttattt cttaacattg tagttttata 120
   tttttattac aaatggcaac tggaactact ttgcaaaaag ccattgagtt agtcaccaaa 180
   gccaccgaag aagacagaaa caagaattat gaagaagccc ttcgtctata tgaacatgga 240
   gtagagtact ttctacatgc cattaaatat gaagctcagg gtgacaaagc taaggagagc 300
   attagggcca aatgtgtaca gtatctagaa agggcagaaa aaattaaaga atctgtaagg 360
   aaagggaaga agaagccagt tttagatgga ggttcttcaa aggaagacaa aaagagtgat 420
   agtgatgaag gtgacgaaga tgatcctgac aaaaagaagt tacaaagcaa actggaaggg 480
   gccatagttg tagaaaaacc acatgtcaaa tggagtgatg tggcag 526
<210> 490
   <211> 24
   <212> DNA
   <213> Artificial Sequence
<220>
   <223> primer
<220>
   <221> misc_feature
   <222> (1)..(24)
   <223> forward primer
<400> 490
   tgtatttgtg actggaaaag gaaa 24
<210> 491
   <211> 20
   <212> DNA
   <213> Artificial Sequence
<220>
   <223> primer
<220>
   <221> misc_feature
   <222> (1)..(20)
   <223> reverse primer
<400> 491
   ctgccacatc actccatttg 20
<210> 492
   <211> 2068
   <212> DNA
   <213> Diabrotica virgifera
<220>
   <221> misc_feature
   <222> (1)..(2068)
   <223> WCRW transcript ta02217.001_diavv
<400> 492
   tgtgaagtgt caaaactgtc aaaagaatta tgccaatgtc aaaagtgtaa tttggagggg      60
   tgattataaa attattctta gacattacaa tatttcatat aaagtattta tttaaaagat     120
   gtatttgtga ctggaaaagg aaattagagc cctatactta cagttaaacg cttataaatt     180
   tgtagcaagt attttatctt tacaaaagaa agtttatttc ttaacattgt agttttatat     240
   ttttattaca aatggcaact ggaactactt tgcaaaaagc cattgagtta gtcaccaaag     300
   ccaccgaaga agacagaaac aagaattatg aagaagccct tcgtctatat gaacatggag     360
   tagagtactt tctacatgcc attaaatatg aagctcaggg tgacaaagct aaggagagca     420
   ttagggccaa atgtgtacag tatctagaaa gggcagaaaa aattaaagaa tctgtaagga     480
   aagggaagaa gaagccagtt ttagatggag gttcttcaaa ggaagacaaa aagagtgata     540
   gtgatgaagg tgacgaagat gatcctgaca aaaagaagtt acaaagcaaa ctggaagggg     600
   ccatagttgt agaaaaacca catgtcaaat ggagtgatgt ggcagggctg gaagctgcca     660
   aggaggcttt gaaagaagct gttatattac ctattaggtt tccccatttg ttcagtggaa     720
   agagagtacc ttggaaaggg attttgttat ttgggcctcc cggtactggt aaatcgtact     780
   tagccaaggc tgtagctaca gaagcaaaca attcaacatt tttttctgtt tcgtcgtccg     840
   atctcgtttc caaatggttg ggagagtcag aaaaattagt caagaatctt ttcgaattgg     900
   ctagagccca caaacctagt attatattca ttgatgaaat cgattcttta tgctcatccc     960
   gttccgacaa cgaatcagag tcagccagga gaatcaaaac cgagtttttg gttcagatgc    1020
   aaggtgtggg ccacgatact gaaggtatcc ttgtcctcgg tgctacgaat attccgtggg    1080
   tattggattc tgctattcga agaagatttg agaagagaat ttacatacct ctaccagaag    1140
   aacctgccag agccgttatg tttaaattgc atttaggaaa tactcataca gagctaacag    1200
   atgaagatat taaagagttg gcatctaaaa ccgaagggta ttccggagct gatataagta    1260
   tcgtagttcg tgacgctctt atgcaacccg tccgaaaagt ccaaactgca actcacttca    1320
   agaaggtcag aggtccgtcg ccgcttgatc ctaataccat cgtagatgat ttgttgcttc    1380
   catgttctcc cggagatccg ggagctgtgg aaatgtcttg gatggatatc tccagtgata    1440
   aattagctga gcctcctgtt acagtgaatg acatgctgag atcaatcgca acctccaaac    1500
   caaccgtcaa cgacgaagat ctcacgaaac tcaaaaagtt tatggaagac ttcggccaag    1560
   aaggataaaa tttaggtata caccagattt actatttaaa agagagattc ttgtataata    1620
   tttgtattct tttaatgcat atatagatcg taaaaaccac acaaaacttt caacattttg    1680
   cattaagtgt tattagaaaa caaaagatta atgttgttat actgttgctt taatattatt 1740
   tttttaataa attgaatttt tcttttttat attacggcct tattatacag taagatagta 1800
   tatatttgta ttcttaatga taaatgaagt taatgaaaca tgtttttaga atattcactt 1860
   gttctttgta tataaaataa taaaacaccg attggtttaa aatacttgta ttggcttaaa 1920
   gcaagttatc tataagcttc aaagacatac tcagtgtctt atccgatttc tgttactata 1980
   aacgatctga ttcatttatt tatctgttgt aattaaaatc tgaaataaaa tgtcacaata 2040
   tgtagattgt tttccacctt aggattgg 2068
<210> 493
   <211> 621
   <212> DNA
   <213> Diabrotica virgifera
<220>
   <221> misc_feature
   <222> (1)..(621)
   <223> RNAi target sequence
<400> 493
   gcgattcggt aaaggagaaa gtaaccagaa ttattttagc tgttttcagg aatttgatcg 60
   aaaaacccga agatcaacaa gtttcaaaag aacattgtat agctatggtg caatgcaaag 120
   tattgaaaca attggccatt ttagaacagc gcaagttcga tgatgaagat gtgacagctg 180
   atgtcgaatt tctaactgaa aaactacaga gttctgttca ggatctgagt tccttcgatg 240
   aatattcaac tgaagtcaaa tctggacgtt tggaatggtc tccagtacac aagagcaagt 300
   tctggcggga aaatgcgcag cgcctcaacg agaaaaacta cgaattactc cgtattctca 360
   ttcatttgtt agaaaccagc aaggacccct tggtattgag cgtagctagt ttcgatattg 420
   gagagtatgt tcgccattat cctcgcggca aacacataat cgagcagttg ggaggaaagc 480
   aattagttat gcaactctta gcccacgaag atcccaatgt acgatatgaa gctctgttgg 540
   cagtccaaaa attgatggtc cacaattggg aataccttgg tcgtcaactt gaaaaagagc 600
   aaagtacaga taaaaccgcg c 621
<210> 494
   <211> 21
   <212> DNA
   <213> Artificial Sequence
<220>
   <223> primer
<220>
   <221> misc_feature
   <222> (1)..(21)
   <223> forward primer
<400> 494
   gcgattcggt aaaggagaaa g 21
<210> 495
   <211> 22
   <212> DNA
   <213> Artificial Sequence
<220>
   <223> primer
<220>
   <221> misc_feature
   <222> (1)..(22)
   <223> reverse primer
<400> 495
   gcgcggtttt atctgtactt tg 22
<210> 496
   <211> 1830
   <212> DNA
   <213> Diabrotica virgifera
<220>
   <221> misc_feature
   <222> (1)..(1830)
   <223> WCRW transcript ta02168.002_diavv
<400> 496
   tacttctgtc agtgtcaaaa tttatatcac aagacttcca gttgggagtt gggtcagagt      60
   taatttttta ggtcttctat taaaagggtt gtaattttaa tttaaaaaaa tggcatccaa     120
   tttagttgcg ggagcaaatc ctaaaataca ggatataatg tcgtctttgg acgatgacaa     180
   aacagatatg ttggctgcta caagtgtatt gcagcaaaga gctacagata taagaaatca     240
   acatgtaaac tggcaatcct atttccagtc acaaatgatt tcgcaggaag actacaactt     300
   catagtagct tttgatgtga ctgatagtgc aaaaagagaa gctctcctga aaagcgacag     360
   aaaccaatgt gctcaaacat tgttgaacct tttggggcat gtatccaagg accaaactct     420
   gcagtacatt ttagttttgg ttgatgatat gttacaggag aacagaagtc gtgtagagat     480
   atttcacgaa tatgccaacc aaaagaaaga atctgtttgg ggtccatttt tgaaccttct     540
   taaccgccag gatggattta ttaccaacat gacttctagg atcatcgcta aaattgcttg     600
   ttggtcgcag acacttatgg atcgttctga ccttcatttt tacttgacct ggttaaaaga     660
   ccaactaaaa actcagaaca acgaatacat ccaatcggtt gcaagatgcc ttcaaatgat     720
   gctccgaatt gacgactacc gttttgcctt cgtgtccgtc gatggaatat ccactctgtt     780
   gtcggtactc tctggaagag taaatttcca agttcagtac cagctgatat tctgcttgtg     840
   ggtactcact ttcaacccgt tgttagccga aaagatgaac aaattcaacg ttattcctat 900
   attggcagat attttgagcg attcggtaaa ggagaaagta accagaatta ttttagctgt 960
   tttcaggaat ttgatcgaaa aacccgaaga tcaacaagtt tcaaaagaac attgtatagc 1020
   tatggtgcaa tgcaaagtat tgaaacaatt ggccatttta gaacagcgca agttcgatga 1080
   tgaagatgtg acagctgatg tcgaatttct aactgaaaaa ctacagagtt ctgttcagga 1140
   tctgagttcc ttcgatgaat attcaactga agtcaaatct ggacgtttgg aatggtctcc 1200
   agtacacaag agcaagttct ggcgggaaaa tgcgcagcgc ctcaacgaga aaaactacga 1260
   attactccgt attctcattc atttgttaga aaccagcaag gaccccttgg tattgagcgt 1320
   agctagtttc gatattggag agtatgttcg ccattatcct cgcggcaaac acataatcga 1380
   gcagttggga ggaaagcaat tagttatgca actcttagcc cacgaagatc ccaatgtacg 1440
   atatgaagct ctgttggcag tccaaaaatt gatggtccac aattgggaat accttggtcg 1500
   tcaacttgaa aaagagcaaa gtacagataa aaacgcgcca aagggtggag ctcctgtcgc 1560
   aggcaaggcg taaatactac catgaaaagt atttaaagat atttactgca ataattgtag 1620
   aaactttaaa aataattgta aaaattatat cgatttctgt acatagctgt ataggtatac 1680
   attcctgtca tattttatta ttgttacgat aaccaataat caaccatgca cattttaaac 1740
   atttaaaaat attgctttta aagtaacttt tttctgattg ttagtattat aataaagtat 1800
   atgttgtcgt ttaaaaaaaa aaaaaaaaaa 1830
<210> 497
   <211> 642
   <212> DNA
   <213> Diabrotica virgifera
<220>
   <221> misc_feature
   <222> (1)..(642)
   <223> RNAi target sequence
<400> 497
   gggaaccttt ttctggaggt taggaaaagt acgtgacatt ttcactttat tttccataca 60
   gtttaaacaa tattgtattc ttgtagttat tgcctgaaaa taatcacttt ttaagattag 120
   caatgccagc agctgacgaa ataaagtcca gctgggctga cgaggtagag ttagaaggag 180
   gtgccttacc accacccagt gaagtcttcg aaaatggttt caagattgta actgaatata 240
   aatacaatga agacgacaag aaagttaaga ttgtcaggac gtacaaaatt gagaaacgtg 300
   ctgtgtccaa gtcaattgct ctccgtaaaa catggaaaaa gtttggtgat gttgccaatg 360
   ataaacctgg cccgaatccc gcgactacca tagtagctga agacgtttat atgcagtaca 420
   tcactagtaa ggaagaagat aataagcagg atgatgactc tttggacaaa ctgaaaggaa 480
   tgggtgataa gaaattcttt aaatgtcgta catgtaatgg tgaacattgg actacaaaat 540
   gtccatggaa ggacactatg tttgttggtg gaaagccacc agatgataag aaaatatcag 600
   ctgttggagg ggcagctgtt ggtgaagctg gtaaacaagg at 642
<210> 498
   <211> 20
   <212> DNA
   <213> Artificial Sequence
<220>
   <223> primer
<220>
   <221> misc_feature
   <222> (1)..(20)
   <223> forward primer
<400> 498
   gaaccttttt ctggaggtta 20
<210> 499
   <211> 22
   <212> DNA
   <213> Artificial Sequence
<220>
   <223> primer
<220>
   <221> misc_feature
   <222> (1)..(22)
   <223> reverse primer
<400> 499
   atccttgttt accagcttca cc 22
<210> 500
   <211> 1014
   <212> DNA
   <213> Diabrotica virgifera
<220>
   <221> misc_feature
   <222> (1)..(1014)
   <223> WCRW transcript ta06291.001_diavv
<400> 500
   cggcaacctt tttcttgagg ttaggaaaag tacgtgacat tttcacttta ttttccatac      60
   agtttaaaca atattgtatt cttgtagtta ttgcctggaa ataatcactt tttaagatta     120
   gcaatgccag cagctgacga aataaagtcc agctgggctg acgaggtaga gttagaagga     180
   ggtgccttac caccacccag tgaagtcttc gaaaatggtt tcaagattgt aactgaatat     240
   aaatacaatg aagacgacaa gaaagttaag attgtcagga cgtacaaaat tgagaaacgt     300
   gttgtgtcca agtcaattgc tctccgtaaa acatggaaaa agtttggtga tgttgccaat     360
   gataaacctg gcccgaatcc cgcgactacc atagtagctg aagacgttta tatgcagtac     420
   atcactagta aggaagaaga taataagcag gatgatgact ctttggacaa actgaaagga     480
   atgggtgata agaaattctt taaatgtcgt acatgtaatg gtgaacattg gactacaaaa     540
   tgtccatgga aggacactat gtttgttggt ggaaagccac cagatgataa gaaaatatca     600
   gctgttggag gtgcagctgt tggtgaagct ggtaaacaag gatccaaata tgtaccacct     660
   aatttgagag atggaggtgt gaagagaact gatggatctg gtgtagctaa acgagatgat     720
   agttcctgtg ctatcagaat tgccaatttg agtgaatcca caactgaaac tgatttggaa     780
   gatctggtta aacaatttgg accaattcaa aaactgtatt tggcgaaaga caagataact     840
   ggtggatgta aaggatttgc ttatatacat ttcaaattcc gttctgatgc agctaaggca     900
   atcgcaatgc ttcatggaca tggttatgat catcttattc ttaatgttga ttggtccaag     960
   ccatcaccaa attctaacca ataattgtat caccagattt ttgaataata aaat          1014
<210> 501
   <211> 524
   <212> DNA
   <213> Diabrotica virgifera
<220>
   <221> misc_feature
   <222> (1)..(524)
   <223> RNAi target sequence
<400> 501
   tcaacgtcat ctcagtttgg tttattgttg ggtgtaatgt ttaaataaaa taattattat      60
   tttgtaaacc tgtgccatta aacaaataaa gcgatggtgc taattgcagc agcagtctgc     120
   acgaaagcag gcaaaacaat tgtgtctcga caatttgttg aaatgaccaa agctagaata     180
   gaaggtttgt tggctgcctt tcctaaatta attcctacag gaacccagca tacatttgtg     240
   gaaacagatt cagtacggta tgtttatcaa cccctagaga aactgtatat ggttcttatt     300
   acagctagag ctagcaacat attagaagat cttgaaaccc tccgtctatt cgcaagagtg     360
   attcctgagt actgcaaatc tttggatgaa aatgaaattg cagagaatgc attttcactt     420
   atatttgctt ttgatgaaat agtggcatta ggttataggg aaagtgtcaa tctgtctcaa     480
   attcgcacat ttgtggaaat ggactcgcac gaagaaaaag ttta                      524
<210> 502
   <211> 22
   <212> DNA
   <213> Artificial Sequence
<220>
   <223> primer
<220>
   <221> misc_feature
   <222> (1)..(22)
   <223> forward primer
<400> 502
   tcaacgtcat ctcagtttgg tt 22
<210> 503
   <211> 23
   <212> DNA
   <213> Artificial Sequence
<220>
   <223> primer
<220>
   <221> misc_feature
   <222> (1)..(23)
   <223> reverse primer
<400> 503
   taaacttttt cttcgtgcga gtc 23
<210> 504
   <211> 1812
   <212> DNA
   <213> Diabrotica virgifera
<220>
   <221> misc_feature
   <222> (1)..(1812)
   <223> WCRW transcript ta03896.001-diavv
<400> 504
   cgaagatcct tgtcacgtca ctgctttttc gtgaaggggg ggtacacgtc aacgtcatct      60
   cagtttggtt tattgttggg tgtaatgttt aaataaaata attattattt tgtaaacctg     120
   tgccattaaa caaataaagc gatggtgcta attgcagcag cagtctgcac gaaagcaggc     180
   aaaacaattg tgtctcgaca atttgttgaa atgaccaaag ctagaataga aggtttgttg     240
   gctgcctttc ctaaattaat tcctacagga acccagcata catttgtgga aacagattca     300
   gtacggtatg tttatcaacc cctagagaaa ctgtatatgg ttcttattac aactagagct     360
   agcaacatat tagaagatct tgaaaccctc cgtctattcg caagagtgat tcctgagtac     420
   tgcaaatctt tggatgaaaa tgaaattgca gagaatgcat tttcacttat atttgctttt     480
   gatgaaatag tggcattagg ttatagggaa agtgtcaatc tgtctcaaat tcgcacattt     540
   gtggaaatgg actcgcacga agaaaaagtt tatcaggccg tgagacagac tcaagagcgc     600
   gaggccaaga atatgatgag agaaaaggca aaagaacttc agagacagaa gatcgaagcg 660
   gccaaaaaag gagggaagac ctcgtttggt agtagcggtg gctttggcag ctcgacaggt 720
   tatactccta cgccatctgt tggtgatgta gctaaccaga caaatgatgt taaaacttct 780
   tcatacacac cagcccctgc gcaaaaacct cggggtatga aattaggtgg aaaaggtaga 840
   gatgtagaat cattcgtaga tcagcttaaa tcggaaggag aaaacgtcat tactccaaac 900
   aaaaatagta tttcacagcc aggaactaaa gctccagcta tcaaaactga catcgatgat 960
   gttcatttaa gattggaaga aaaattaata gtgagaatag gtcgtgatgg tggcgtacaa 1020
   caattcgaat tattgggact tgctacttta cacattggag atgagagatg gggtaggata 1080
   cgtgtgcaat tggaaaatca gaatacccac ggtgttcaac ttcaaacgca tcctaatgta 1140
   gataaagaat tattcaagct acgctcacag attggattga aacaaccggc taaacctttt 1200
   cctctaaata cagatgttgg tgtactgaaa tggagattac aaagtactga ggaagctcta 1260
   attccactct taataaattg ctggccttca gaagcgggag atggtagttg cgatgttaat 1320
   atagaatatg agcttgccca cactaatttg gaactagttg atgtcaatat tgttattccc 1380
   ttgccaattg gatgttcacc aatagttggt gaatgtgatg gtatgtacac acacgaagcc 1440
   aagcgtaatc aactggtatg gaatttgcct ctgattgatg ccagtaataa aactggttct 1500
   ttagaattta acgctcctag ggccataccc gctgatttct tcccgctttc cgttggattc 1560
   aattcgaagt catcctatgc gagtattaag attaccgaag ttgtcctagt tgatgatgat 1620
   tctcctataa aatattcagt ggagaccgca ctatatccag ataaatatga agtagtataa 1680
   tggggcagaa gtaaagaatc acttgtaaaa attatataac taatattata tcttatatgc 1740
   aacattgctt tttgtacaaa agttaagtaa cttgtcttta ttttatctat attaataaaa 1800
   taagtaagaa ag 1812
<210> 505
   <211> 612
   <212> DNA
   <213> Diabrotica virgifera
<220>
   <221> misc_feature
   <222> (1)..(612)
   <223> RNAi target sequence
<400> 505
   attctgatcc cagcacacaa attttgtata accggacaat ggcgcatttg ggattgtgtg        60
   catttagaca cgctaacatt aaggatgccc ataactgttt agttgatttg atgatgactg       120
   gaaaaccaaa ggagttactg gcacaaggtt tgcttccaca aagacaacac gaacgcagca       180
   aagaacagga gaaaatcgag aaacagcgtc aaatgccctt ccacatgcac atcaatctcg       240
   aactcttaga atgtgtgtat ttagtctccg ccatgttgat cgaaatcccg tatatggctg       300
   cgcatgaatt cgacgccaga cgcaggatga tttctaagac cttctatcag cagttgcgtt       360
   cttccgaacg tcaaagctta gtgggacctc cggaaagcat gcgagagcac gtcgtcgcgg       420
   cggctaaagc aatgatgaac ggcaattggg cggcatgcaa cacttttatc attaacgaca       480
   agatgaatgc caaagtgtgg gatctgtttt accaagccga ccaagttaga tccatgttga       540
   cgaaattgat aaaagaagag tctttgagga cgtatttgtt tacctactcc cacgtttacg       600
   attcaatttc ga                                                           612
<210> 506
   <211> 22
   <212> DNA
   <213> Artificial Sequence
<220>
   <223> primer
<220>
   <221> misc_feature
   <222> (1)..(22)
   <223> forward primer
<400> 506
   attctgatcc cagcacacaa at 22
<210> 507
   <211> 22
   <212> DNA
   <213> Artificial Sequence
<220>
   <223> primer
<220>
   <221> misc_feature
   <222> (1)..(22)
   <223> reverse primer
<400> 507
   tcgaaattga atcgtaaacg tg 22
<210> 508
   <211> 2785
   <212> DNA
   <213> Diabrotica virgifera
<220>
   <221> misc_feature
   <222> (1)..(2785)
   <223> WCRW transcript ta05048.001_diavv
<400> 508
   caatgactaa tgtcacctcg ctcttttaac tcacgttgca tatgtgaggt attttccaaa 60
   attcgagaaa agtttattta gtgttattat aataagggaa aacagtaaaa atgagtagat 120
   ttttcgctgg atccgattca gattcggata gctcatccga agaagagatt atccaacgtc 180
   cccaagtacc agcttatact ttcagtgacg atgaggagga ggtaaaacgt gtagtaagat 240
   cagcgaaaga aaagcgctat gaagaactta caaatactat taaacaaatc agaaatttta 300
   agaaaattaa ggatatgtct agcatgttga atagttttga ggaattacaa aaggcatacc 360
   aaaaagctgc tcctgttatt gtcaaagaag aaaatggcca gactccaaga ttttttatta 420
   ggtgtttggc cgaaatggaa gactttatta atgaaatgtg ggaagataga gatgggagga 480
   aaaacatgag caaaaacaat agcaaatcac tcacatctat gagacagaag ttgagaaaat 540
   atttgaaaga ttttgaagat gacttggcta aatttaagga aaacccagaa cagcctgatg 600
   atgaggaaga ggaggaaaag cctgaagtag ctgaggattc cgagaacgaa gctccatcca 660
   aaaacgcgtt tctcaaagct ccacccaaac aagttgtaga cgacaaagat gaatcgtcag 720
   actcggatga ctggccatct gactctgaat ctagtctgtc atcatctgat gacgacgaag 780
   gcaaatacca gagcataagg gagcgtttct tgaagagaac tgggcccgct cacgaagaag 840
   atgaagaaag ggaaaggaga aaggaggaga aaaggaagga gagaaaggag aaaggtagga 900
   agttgaaaaa ggatgaggag gaggatggtg aaggcaagtg ggagactgtt aatcgaggtt 960
   tcgccatccc tagtgaaaaa cctaaaatgt tcgccaagga cgccgaaatc gatctaaacc 1020
   ttgtaatcaa gaaactcagc gaaatcatgg cagctagagg caaaaagcgc acagatagac 1080
   gcgaacaaat agaactcctg cacgaactcc aaaatgtttc tgagcaacac caattgggag 1140
   cggctatttt cattaaaatt aaactcgcca tcatatctgc cattttcgat tacaacccca 1200
   aagtttctga cgcaatgaag ccggagtatt ggctgaaatt gctggagagg atgagcgaaa 1260
   tgttgggcat gttgttgaat actaccaatc tagtagtttc tgatggaatt actgaagaaa 1320
   tggaaaatct tgaagaaccc ccgtatcgca ttaggggctg tatactaaca tcagtggaaa 1380
   gactggatga cgagtttact aaactactca aagagtgtga tcctcacagt aacgagtatg 1440
   tagaacggtt aaaggatgaa tgtaaggttt ctagtatcat cgataaaaca atgaaatact 1500
   tggaaaggag taacgtatct tccgaactat gtaggatata tttacgaaaa atcgagcatt 1560
   tgtattacaa attcgatcct agagtattac agcaaaaagc tgatcctctc gtagtaccca 1620
   aagacgagat gaactccgtg caagaaatgg agaaactatg caaatttatc tacgcaaaag 1680
   atagtactga cagattgaga actagagcta ttttgtccca tatatatcat cacgctctgc 1740
   atgacaactg gttccaagca agggatttgg tattgatgtc acatctacaa gaaacgattc 1800
   agcattctga tcccagcaca caaattttgt ataaccggac aatggcgcat ttgggattgt 1860
   gtgcatttag acacgctaac attaaggatg cccataactg tttagttgat ttgatgatga      1920
   ctggaaaacc aaaggagtta ctggcacaag gtttgcttcc acaaagacaa cacgaacgca      1980
   gcaaagaaca ggagaaaatc gagaaacagc gtcaaatgcc cttccacatg cacatcaatc      2040
   tcgaactctt agaatgtgtg tatttagtct ccgccatgtt gatcgaaatc ccgtatatgg      2100
   ctgcgcatga attcgacgcc agacgcagga tgatttctaa gaccttctat cagcagttgc      2160
   gttcttccga acgtcaaagc ttagtgggac ctccggaaag catgcgagag cacgtcgtcg      2220
   cggcggctaa agcaatgagg aacggcaatt gggcggcatg caacactttt atcattaacg      2280
   acaagatgaa tgccaaagtg tgggatctgt tttaccaagc cgaccaagtt agatccatgt      2340
   tgacgaaatt gataaaagaa gagtctttga ggacgtattt gtttacctac tcccacgttt      2400
   acgattcaat ttcgatggcc actttggcgg aaatgttcca gttgggcaaa cccgtcgtgc      2460
   attctattat ttctaagatg atcattaacg aagaattgat ggcatcttta gacgacccga      2520
   ctcaaactgt tgtaatgcac agaagcgaac catcccgtct acaatctttg gctttgcaac      2580
   tggcggataa aatcaacaat ttcgtcgatt ccaacgaaag aatattggaa tccaaacaag      2640
   gaaacttctt cctaagggga gcaaaccagg gcaatttccg aggagatagg cagaactacc      2700
   gcggaaataa ccaaaactgg gataggcaac gtcgagacag ggatagaaag gaaaaagata      2760
   attattaata aacatagatc ttttg                                            2785
<210> 509
   <211> 503
   <212> DNA
   <213> Diabrotica virgifera
<220>
   <221> misc_feature
   <222> (1)..(503)
   <223> RNAi target sequence
<400> 509
   ggggctcttt agttgtcatc tgtcatttaa gtagtaattt cattccaaaa tgaagatcgg 60
   actttgtgct tacagcgggt acaaaattta ccccggacat ggcaaaacaa tggttaaagt 120
   tgatggaaag accttcattt tcctcaactc aaaatgtgaa agggcgcact tgatgaaaag 180
   gaacccccgt aaagtaacat ggactgtctt gtacagacgt aaacataaga agggtcagga 240
   ggaagaagcc accaaaaaac gtaccagaag gacacagaaa ttccaaaggg ctattgtagg 300
   tgcttcttta aatgatatca tggccaagag aaaccaaagg cctgaagtaa ggaaggccca 360
   aagggagcaa gccattcgtg ccgctaagga acagaaaaag agcacaaaga cagccaggaa 420
   ggctacagcc cccgccaagg ccaaacctgc tcctaaacag aaagtaacca agaatcaacc 480
   caaagctgca cctagagtag gag 503
<210> 510
   <211> 19
   <212> DNA
   <213> Artificial Sequence
<220>
   <223> primer
<220>
   <221> misc_feature
   <222> (1)..(19)
   <223> forward primer
<400> 510
   gctctttagt tgtcatctg 19
<210> 511
   <211> 23
   <212> DNA
   <213> Artificial Sequence
<220>
   <223> primer
<220>
   <221> misc_feature
   <222> (1)..(23)
   <223> reverse primer
<400> 511
   ctcctactct aggtgcagct ttg 23
<210> 512
   <211> 2131
   <212> DNA
   <213> Diabrotica virgifera
<220>
   <221> misc_feature
   <222> (1)..(2131)
   <223> WCRW transcript ta01391.001_diavv
<400> 512
   aaagatggaa ggaaatgagg ccaatactta gcccatcgtt cacaagcagc aaaatgaaat      60
   cgatgtttat gctgatgtca gaatgtggcg aaaagttggt caaatttttt atggaaaaag     120
   accagaatgc tatagaaatt gaaatgaaag acacattaac gcgttatact aatgatgtta     180
   ttgcctctgc agctttttgg agtacagatt gattctttaa acaacactga aaatgaattc     240
   tatctcatgg gaaaaaatgc aaccaatttc cgtggtttct ggaggactat gaagttgttt     300
   ggatatatga ttataccaaa aatatttgag ttctttaaag tgacattgtt tgatatgaac     360
   gttcgacgat tttttactga attgattagt ggaacaataa agatcaggga agagaaaggc     420
   attattcgtc ccgatatgat tcacctactc atgcaagcaa gaaaaggaat ccatcaaaaa 480
   gaagaaaagg ttgttgatgc tggatttgct gtagtacaag aatcgaattt agaatcaaaa 540
   gtagttgcga cagaaatgac cgacttagac atctgcgctc aagcattgat tttcttcttt 600
   gctggatttg agacggtctc gtcaaccatg tgctttgtgg ctcatgaatt ggcctgtaat 660
   cctgatgtcc aatcgagatt aagagatgaa atacgtgaaa cactcgatca atgtaatgga 720
   aaagtaacct acgaagctat gttgaaaatg aaatatctgg atatggtaat atcagaatcc 780
   atgaggaaat ggccagcagt tatagctgtt gatcgaattt gcaccaaacc atacacactc 840
   gaaccagcaa cactcgatga gaagcccctc cacttagaca aaggcaccat aatctggttc 900
   cccatttacg gtctccaccg cgatcctaaa cactacccag acccagaacg gtttgatcct 960
   gaaagattca gcgatgaaaa caaaggaaac atcaatccat acgcttacct tccatttgga 1020
   gtcggcccta gaaactgcat cggttccaga tttgctttat tagagatgaa agtattgttt 1080
   ttccatttgt tagccaattt tgagattgtg ccaggtccaa agacggaaat tcctttgcgg 1140
   atttgtaaga aacaattcgg tttgactgca gaaaatggtt tttggttcaa cttgaagaaa 1200
   ctgaagaatt agtctttgat atgtttgata aaagtcttga taaaatattg tttaaccata 1260
   tctacttatt tggtgtaatg tattttctag tcataattct tttaaacttt ttcttaacgt 1320
   ttcagcgggc gcaccctata atttcacatg agaagacgcg tgtcgtagat tatacgtctt 1380
   tgcgtaaata atgtcttctt caaataaatg tttggaaatt gttacaatga tattttcttc 1440
   ttcttcctgc ttccttatag gctcgcatct gttctatctt cggatgcctc ttcatcagat 1500
   atccgcagca cgtccacaca ggtaaaccta aacataaaaa cgggggctct ttagttgtca 1560
   tctgtcattt aagtagtaat ttcattccaa aatgaagatc ggactttgtg cttacagcgg 1620
   gtacaaaatt taccccggac atggcaaaac aatggttaaa gttgatggaa agaccttcac 1680
   tttcctcaac tcaaaatgtg aaagggcgca cttgatgaaa aggaaccccc gtaaagtaac 1740
   atggactgtc ttgtacagac gtaaacataa gaagggtcag gaggaagaag ccaccaaaaa 1800
   acgtaccaga aggacacaga aattccaaag ggctattgta ggtgcttctt taaatgatat 1860
   catggccaag agaaaccaaa ggcctgaagt aaggaaggcc caaagggagc aagccattcg 1920
   tgccgctaag gaacagaaaa agagcacaaa gacagccaag aaggctacag cccccgccaa 1980
   ggccaaacct gctcctaaac agaaagtaac caagaatcaa cccaaagctg cacctagagt 2040
   aggaggaaaa cgttaatatt gtataaacgt tatttaataa aataagtttt tttccataaa 2100
   aaaaaaaaaa aaatgcgaaa aaaaaacatg t 2131
<210> 513
   <211> 502
   <212> DNA
   <213> Diabrotica virgifera
<220>
   <221> misc_feature
   <222> (1)..(502)
   <223> RNAi target sequence
<400> 513
   aaagtttttg agccgtcaac atgggtaagc cccgtggatt gagaaccgct aggaaacatg        60
   tgaatcaccg tcgtgaacaa agatgggctg ataaagatta caaaaaggca catttaggaa       120
   ctagatggaa ggctaatcct tttggaggtg cttctcatgc taaaggaatt gtattggaaa       180
   aagtgggagt tgaagcaaaa caacctaact ctgctattcg caagtgcgtt agggtacagt       240
   taattaaaaa tggtaaaaaa atcacagccc tcgtacctag agatggttgc ttaaaccata       300
   tagaagaaaa cgatgaagtt ctagttgcag gttttggacg taaaggtcat gctgtaggag       360
   atattcccgg agttagattt aaggttgtta aggtcgctaa tgtgtcccta ttagctttat       420
   acaaagaaaa gaaggaaagg ccaagatcat aattaaaatg ttattttttt tatttatttg       480
   cttggcactt ttaattttta aa                                                 502
<210> 514
   <211> 22
   <212> DNA
   <213> Artificial Sequence
<220>
   <223> primer
<220>
   <221> misc_feature
   <222> (1)..(22)
   <223> forward primer
<400> 514
   aaagtttttg agccgtcaac at 22
<210> 515
   <211> 24
   <212> DNA
   <213> Artificial Sequence
<220>
   <223> primer
<220>
   <221> misc_feature
   <222> (1)..(24)
   <223> reverse primer
<400> 515
   tttaaaaatt aaaagtgcca agca 24
<210> 516
   <211> 616
   <212> DNA
   <213> Diabrotica virgifera
<220>
   <221> misc_feature
   <222> (1)..(616)
   <223> WCRW transcript ta00545.001_diavv
<400> 516
   ggggattttc ggttgggggg ttgggagcag ttatgggggc tttccggtca aagtttttga      60
   gccgtcaaca tgggtaagcc ccgtggattg agaaccgcta ggaaacatgt gaatcaccgt     120
   cgtgaacaaa gatgggctga taaagattac aaaaaggcac atttaggaac tagatggaag     180
   gctaatcctt ttggaggtgc ttctcatgct aaaggaattg tattggaaaa agtgggagtt     240
   gaagcaaaac aacctaactc tgctattcgc aagtgcgtta gggtacagtt aattaaaaat     300
   ggtaaaaaaa tcacagcctt cgtacctaga gatggttgct taaaccatat agaagaaaac     360
   gatgaagttc tagttgcagg ttttggacgt aaaggtcatg ctgtaggaga tattcccgga     420
   gttagattta aggttgttaa ggtcgctaat gtgtccctat tagctttata caaagaaaag     480
   aaggaaaggc caagatcata attaaaatgt tatttttttt atttatttgc ttggcacttt     540
   taatttttaa ataaagtgtt aagcatataa ttttgtacag tgttttattt caataaacaa     600
   ttttatacct gaaata                                                     616
<210> 517
   <211> 719
   <212> DNA
   <213> Diabrotica virgifera
<220>
   <221> misc_feature
   <222> (1)..(719)
   <223> RNAi target sequence
<400> 517
   gggtgatcct tcagttttcg tccacgcgtg tcaaaatggc gaaaaagaaa ccagaggata    60
   aaaataatca aagcgatgat gatacaaaaa acgagagtga ttactcgtcg aacgaagaag   120
   aaccaaactt cagtgatcct gaagactacg aagacgatgt ctctgatgaa gaactcttag   180
   gtgatgtttt aaaagaaaag cctaaggaaa ctgatggagt agaaagtgtt atagtagtag   240
   atggagttcc tcaagtaggt cctaatcgta ttgaaaagtt gaagtcagtc attgtcaaaa   300
   tatttgtgaa gtttggaaca atagtaaatg aattctaccc tgtcaatagt gagggccaca   360
   ccaaaggtta tatctttata gaatattcct caccatctga agctgctgag gctgttaagc   420
   tcactaacaa ttttaaattg gataagcacc atacctttca agttaacctc tttactgatt   480
   tttctaagta tgaccaaatt cctaataagt gggaccctcc agagccacaa ccttaccaag   540
   ggcaagcaga cttgcattct tacttattgg aaccggatgc atatgatcag tttgctgtag   600
   ttgttgcaca aacacaagct gtgcaaattt ggcagaatac acagcctgat cctagtctca   660
   ttgaagctag aaacagctgg agtcaaacgt acataaaatg gtcacctctg ggtacattc    719
<210> 518
   <211> 19
   <212> DNA
   <213> Artificial Sequence
<220>
   <223> primer
<220>
   <221> misc_feature
   <222> (1)..(19)
   <223> forward primer
<400> 518
   gtgatccttc agttttcgt 19
<210> 519
   <211> 22
   <212> DNA
   <213> Artificial Sequence
<220>
   <223> primer
<220>
   <221> misc_feature
   <222> (1)..(22)
   <223> reverse primer
<400> 519
   gaatgtaccc agaggtgacc at 22
<210> 520
   <211> 2397
   <212> DNA
   <213> Diabrotica virgifera
<220>
   <221> misc_feature
   <222> (1)..(2397)
   <223> WCRW transcript ta01172.001_diavv
<400> 520
   gggggtgatc cttcagtttt cgtccacgcg tgtcaaaatg gcgaaaaaga aaccagagga        60
   taaaaataat caaagcgatg atgatacaaa aaacgagagt gattactcgt cgaacgaaga       120
   agaaccaaac ttcagtgatc ctgaagacta cgaagacgat gtctctgatg aagaactctt       180
   aggtgatgtt ttaaaagaaa agcctaagga aactgatgga gtagaaagtg ttatagtagt     240
   agatggagtt cctcaagtag gtcctaatcg tattgaaaag ttgaagtcag tcattgtcaa     300
   aatatttgtg aagtttggaa caatagtaaa tgaattctac cctgtcaata gtgagggcca     360
   caccaaaggt tatatcttta tagaatattc ctcaccatct gaagctgctg aggctgttaa      420
   gctcactaac aattttaaat tggataagca ccataccttt caagttaacc tctttactga      480
   tttttctaag tatgaccaaa ttcctaataa gtgggaccct ccagagccac aaccttacca     540
   agggcaagca gacttgcatt cttacttatt ggaaccggat gcatatgatc agtttgctgt     600
   agttgttgca caaacacaag ctgtgcaaat ttggcagaat acacagcctg atcctagtct     660
   cattgaagct agaaacagct ggagtcaaac gtacataaaa tggtcacctc tgggtacatt     720
   cttatgtact tttcacaggt tgggtgttgc attgtgggca ggacctggat ttttgcaata      780
   caaaaaattt acccattcaa atgttcaatt tattgatttc tcgccatgtg aaaagtattt     840
   agtgacttat tccccacaag gagacccaca taatccagaa cagaaaagga tcatcatttg     900
   ggatattagg acaggtcttg aaaagcgttc attcaatcca gaaggtccaa cagcatggcc     960
   aatcttcagg tggtctcatg acgacaaata ttttgctcgt attggtcaaa acaatgatgc    1020
   ccttcaagta tacgaaacac catcttttgg tcttctagat aagaaatcaa tcaaaattaa    1080
   cggcataaga gatttcaatt ggtcgcccac agataatgtt gtagcttatt gggtagccga    1140
   agacaaagat gtgcctgcca gtgtaactct gttagaatta ccaaacagga atgaaattag    1200
   aaagaagaat ttgtttaatg ttgctgactg taagatacac tggcagaagt ctggagatta    1260
   cctgtgtgtc aaggtagaca ggtattcaaa ggttcgcaag gagaagaatg aaaccaagta    1320
   ttcaggtatg tactgcaact ttgaaatatt ccacatgaag gagaagcaaa tacctgtaga    1380
   cagtgtagag tgcaaggaac ccattcaggc gtttgcatgg gagccggttg gttctaaatt    1440
   tgcaatgatc catggcgaat cccccagcat cagtgccagt ttctacgagg ttaggatagg    1500
   acaggctcct atccttctca agaaatttga gaaaagagct tgcaaccatc tgttttggtc    1560
   tcctagtggg cagttcatag ttttagcagg cttgggttcc aacggaggtg gatcgctgga    1620
   atttgttgat actcacgaat ttttaatcat gaacactaca gaccatttcc aattgtcaga    1680
   tgtagagtgg gatccaactg gaagatatgt aattactgga gtctcatttt ggaagaccaa    1740
   ggttgataca ggttattgga tttggtcctt ccaaggaaaa atcctcaagc gcatcaattt    1800
   ggaaaagttc tcccagattc tctggagacc tagaccacca tcattactca gcgatgaaaa    1860
   gcaaaaggag gtcaagaaga gcctaaagaa atactacccg cagttcgaga gcaaagacag    1920
   gatgcgacaa tccaaggctt ccaaggaact tatcgagaag agagctgctc ttatggagaa    1980
   attccaagcc tacagagagg agaggatcga ggagtacttg gaccaaaaat caaggcgttt    2040
   gcagcttaga aataacattg acactgatga attaaatgca gagaacgttg aggaggaaat    2100
   cgtagaattc tttattaaag aagaagtaat tccagtcgac tagcaccatc ttgaaagtcc 2160
   ggtcctttcc ctgcgtgtgc tttgttacaa cacattcagg gagatttaaa gtgaaaacgt 2220
   taaatattat aatataaaaa atatttgtat tttggacatt gacataaata tttgttaatc 2280
   aagtgtctcg ttcaattaat agtgttttat atttttgaca cgcgtaccat ctttccaaaa 2340
   actaatgaac attgttatta aacgccttgt ggttactaaa ataaaaaatt caataag 2397
<210> 521
   <211> 639
   <212> DNA
   <213> Diabrotica virgifera
<220>
   <221> misc_feature
   <222> (1)..(639)
   <223> RNAi target sequence
<400> 521
   ccgtgatatg ggttacaacg tatctatgat ggctgactcg acatcacgtt gggccgaagc 60
   tttgagagaa atttcaggtc gtttggctga aatgcctgcc gattccggtt atccggctta 120
   cttaggtgcc cgtttggctt ccttctacga acgtgctggt cgcgttaaat gtttaggtaa 180
   tccagacaga gaaggatccg tttcaattgt aggagccgta tcacctcctg gtggtgattt 240
   ctcagatcct gttaccactg ctactcttgg tattgtacag gtgttctggg gtttggacaa 300
   gaaacttgcc caacgtaagc acttcccttc agtagactgg cttggatcat attccaaata 360
   tttaagagca ttggacgact tttatgacaa aaacttccaa gagtttattc ctcttagaac 420
   caaagttaag gaaattcttc aggaagaaga tgatctagcc gaaattgtgc agctggtagg 480
   taaagcatct ctggcagaaa cggacaaaat caccttggaa attgccaggc ttcttaaaga 540
   agatttcttg caacaaaact catactcttc ttatgacaga ttctgtccat tctataaaac 600
   tgtcggtatg ttgagaaaca tgatcggttt gtacgacat 639
<210> 522
   <211> 22
   <212> DNA
   <213> Artificial Sequence
<220>
   <223> primer
<220>
   <221> misc_feature
   <222> (1)..(22)
   <223> forward primer
<400> 522
   ccgtgatatg ggttacaacg ta 22
<210> 523
   <211> 22
   <212> DNA
   <213> Artificial Sequence
<220>
   <223> primer
<220>
   <221> misc_feature
   <222> (1)..(22)
   <223> reverse primer
<400> 523
   atgtcgtaca aaccgatcat gt 22
<210> 524
   <211> 2533
   <212> DNA
   <213> Diabrotica virgifera
<220>
   <221> misc_feature
   <222> (1)..(2533)
   <223> WCRW transcript ta01291.009_diavv
<400> 524
   cggagatgaa gagaagggtc agtcagctga cttatcttat gacaagttgt tcagtttggg 60
   aaaactcgtc cataatgggg atagttgagt gggtgaggtt ccaagagaaa cataacatcc 120
   atccacaaat atgtcgaaag taaggatcgg agatgaagag aaggaagggc agtatggtta 180
   tgtccatgct gtctcaggtc cagtcgttac tgctgagaaa atgtctggtt ctgctatgta 240
   cgaactggta cgtgtcggat actatgagct ggtaggagaa atcattagat tggaaggtga 300
   catggctact attcaggtat acgaagaaac atcaggtgta actgttggtg atccagtatt 360
   aagaactggt aaaccacttt cagtagaact tggacctggt attatgggtt ccatttttga 420
   tggtatccaa cgtccattga aagacatttg tgacgctact gatagtattt acatccccaa 480
   gggtattaat gtaccttctt tatcgagaac agcaaaatgg gacttcaacc caatcaacat 540
   caagttggga tctcacttaa ctggaggtga tatatatggt ctagttcatg aaaacaccct 600
   tgtcaaacac aaaatgattc tgcctcctag agctaagggt actgtaacct acattgcaga 660
   accaggaaac tacactgttg atgaagtagt attggaaact gaatttgatg gtgatcgtac 720
   caaatatact atgttgcaag tatggcctgt acgtcaagca aggccagtca gtgaaaaatt 780
   acctgccaac catcctctgc ttacaggaca gcgtgtactt gatgctcttt tcccatgtgt 840
   acagggtggt actactgcca ttcccggagc tttcggttgt ggaaaaactg taatttcaca 900
   atctctttcc aaatattcca actctgatgt cattatctac gtcggttgcg gagaaagagg 960
   taacgaaatg tctgaagtat tgagagattt ccctgaattg actgttgaaa ttgacgggca 1020
   cactgaatct attatgaaac gtaccgcatt ggtcgccaac acatctaaca tgcctgtagc 1080
   tgctcgtgaa gcttctatct atactggtat tactctttct gaatacttcc gtgatatggg 1140
   ttacaacgta tctatgatgg ctgactcgac atcacgttgg gccgaagctt tgagagaaat 1200
   ttcaggtcgt ttggctgaaa tgcctgccga ttccggttat ccggcttact taggtgcccg 1260
   tttggcttcc ttctacgaac gtgctggtcg cgttaaatgt ttaggtaatc cagacagaga 1320
   aggatccgtt tcaattgtag gagccgtatc acctcctggt ggtgatttct cagatcctgt 1380
   taccactgct actcttggta ttgtacaggt gttctggggt ttggacaaga aacttgccca 1440
   acgtaagcac ttcccttcag tagactggct tggatcatat tccaaatatt taagagcatt 1500
   ggacgacttt tatgacaaaa acttccaaga gtttattcct cttagaacca aagttaagga 1560
   aattcttcag gaagaagatg atctagccga aattgtgcag ctggtaggta aagcatctct 1620
   ggcagaaacg gacaaaatca ccttggaaat tgccaggctt cttaaagaag atttcttgca 1680
   acaaaactca tactcttctt atgacagatt ctgtccattc tataaaactg tcggtatgtt 1740
   gagaaacatg atcggtttgt acgacatggc gagacacgct gtagaatcaa ccgcacaatc 1800
   agaaaataag atcacttgga acgtaataag agattcaatg agtggaattt tatatcaact 1860
   tagcagtatg aaatttaagg atcccgtaaa agatggtgaa gctaaaatca aggcagattt 1920
   tgatcaatta tatgaagata ttcagcaggc cttcagaaac ttagaagatt aaatcttttt 1980
   aaggaaattt tcctattttg ttcatcagtg taagtttaaa aatatagcga tatttatcaa 2040
   aaagaataat aaggcctcta tccctcactt ctgtgaatat taatatggcc gtactaatga 2100
   tagtaactaa agataggttt tctctttttt gatattatcc tgtacaaaat aaattatgta 2160
   aattgttgaa tatgtgtata gtttttttgg gtgagggtac agtgcttatt aaatactttt 2220
   taaacatttt tcccgccatt ccaattacta ttaagttttt tcgttttaat acttttttaa 2280
   atatacaggt gcttaatatc gtttatattt tcagtattac ttggttttct tcatgtaaat 2340
   tgttttaaat ttttctttta cccttttaat cttgtatatt acattaccca attaaagtta 2400
   attgtacaga ttaagataaa cgagtatctt ataacatcta ttagattgtt agaatcaata 2460
   aatgtagtgt aattgttctg ttttgaacaa ataaatgcat cattattgtt gttaaaaaaa 2520
   aaaaaaaaaa aaa 2533
<210> 525
   <211> 507
   <212> DNA
   <213> Diabrotica virgifera
<220>
   <221> misc_feature
   <222> (1)..(507)
   <223> RNAi target sequence
<400> 525
   ggggtccttt tactgtgttt cttaggattt ggaaggtaca aaatgaccaa ctctaaaggt      60
   taccgccgag gaaccaggga tctatttgcc cgcaagttta aaaaacgtgg tgtaattcca     120
   ctttccacat atttgagagt ctacaaagtt ggagatattg tagatatcaa gggtaatggt     180
   gcagttcaaa agggtatgcc ccacaaagtg taccatggta agaccggacg tgttttcaat     240
   gttactgcac atgcattagg tgtaattgta aacaaaaggg ttcgaggaag aatcatcccc     300
   aaaagaatca atctccgtat tgaacatgta aaccactcca agtgtcgtca agacttcttg     360
   caaagagtaa aatccaatga aaagctacgt aaagaagcta aagaaaagaa cattaaagta     420
   gaacttagga gacaacctgc ccaacctagg ccagcacata ttgttagcgg aaaggttcca     480
   gcacaggtgc ttgctcctat cccatat                                         507
<210> 526
   <211> 20
   <212> DNA
   <213> Artificial Sequence
<220>
   <223> primer
<220>
   <221> misc_feature
   <222> (1)..(20)
   <223> forward primer
<400> 526
   gtccttttac tgtgtttctt 20
<210> 527
   <211> 21
   <212> DNA
   <213> Artificial Sequence
<220>
   <223> primer
<220>
   <221> misc_feature
   <222> (1)..(21)
   <223> reverse primer
<400> 527
   atatgggata ggagcaagca c 21
<210> 528
   <211> 696
   <212> DNA
   <213> Diabrotica virgifera
<220>
   <221> misc_feature
   <222> (1)..(696)
   <223> WCRW transcript ta01222.003_diavv
<400> 528
   cgataactga gtttccttca atgataagtt catccttttg tttggtggag tttactactg      60
   gaacacctgg ggggagaggg ggacagcgac atttcctccg ccgggtcctt ttactgtgtt     120
   tcttaggatt tggaaggtac aaaatgacca actctaaagg ttaccgccga ggaaccaggg     180
   atctatttgc ccgcaagttt aaaaaacgtg gtgtaattcc actttccaca tatttgagag     240
   tctacaaagt tggagatatt gtagatatca agggtaatgg tgcagttcaa aagggtatgc     300
   cccacaaagt gtaccatggt aagacaggac gtgttttcaa tgttactgca catgcattag     360
   gtgtaattgt aaacaaaagg gttcgaggaa gaatcatccc caaaagaatc aatctccgta     420
   ttgaacatgt aaaccactcc aagtgtcgtc aagacttctt gcaaagagta aaatccaacg     480
   aaaagctacg taaagaagct aaagaaaaga acgttaaagt agaacttagg agacaacctg     540
   cccaacctag gccagcacat attgttagcg gaaaggttcc agcacaggtg cttgctccta     600
   tcccatatga attcattgct taggtttgtt tatcttaaaa taaaatcctt tatataataa     660
   aaaaaaaaag aaaaaatcgg cagatccctt aaaaaa                               696
<210> 529
   <211> 519
   <212> DNA
   <213> Diabrotica virgifera
<220>
   <221> misc_feature
   <222> (1)..(519)
   <223> RNAi target sequence
<400> 529
   ggtcagtgaa gccatcttgg caaacgttag taacgtgaaa taaaacttaa attttttcaa 60
   aatgggtcgt atgcacgcac caggaaaagg tattgcccag tcggcattgc catacagaag 120
   gagtgtacca acatggttga aagtcacacc agaagaagta aaagaccata tttttaaact 180
   tggcaagaaa ggcttgactc catcacaaat tggtgttatc ctcagggatt catatggtgt 240
   tgcccaagta aggtttgttt ctggaaacaa aatcttgcgt atcatgaaag ctatgggtct 300
   tgcccctgat ctaccagaag atttgtacta ccttatcaag aaggcagtag ctatccgcaa 360
   acatttagaa cgtaacagaa aagacaagga cagcaaattc cgtttgattt tggtagaatc 420
   acgtatccac cgtttggcta ggtactacaa aaccaagagc gtattggcac ccaactggaa 480
   gtacgaatca agcacagcat ctgctttggt cgcttaaat 519
<210> 530
   <211> 19
   <212> DNA
   <213> Artificial Sequence
<220>
   <223> primer
<220>
   <221> misc_feature
   <222> (1)..(19)
   <223> forward primer
<400> 530
   gtcagtgaag ccatcttgg 19
<210> 531
   <211> 22
   <212> DNA
   <213> Artificial Sequence
<220>
   <223> primer
<220>
   <221> misc_feature
   <222> (1)..(22)
   <223> reverse primer
<400> 531
   atttaagcga ccaaagcaga tg 22
<210> 532
   <211> 596
   <212> DNA
   <213> Diabrotica virgifera
<220>
   <221> misc_feature
   <222> (1)..(596)
   <223> WCRW transcript ta05572.002_diavv
<400> 532
   ggggctgggc tcagtgaagc catcttggca aacgttagta acgtgaaata aaacttaaat      60
   tttttcaaaa tgggtcgtat gcacgcacca ggaaaaggta ttgcccagtc ggcattgcca     120
   tacagaagga gtgtaccaac atggttgaaa gtcacaccag aagaagtaaa agaccatatt     180
   tttaaacttg gcaagaaagg cttgactcca tcacaaattg gtgttatcct cagggattca     240
   tatggtgttg cccaagtaag gtttgtttct ggaaacaaaa tcttgcgtat catgaaagct     300
   atgggtcttg cccctgatct accagaagat ttgtactacc ttatcaagaa ggcagtagct     360
   atccgcaaac atttagaacg taacagaaaa gacaaggaca gcaaattccg tttgattttg     420
   gtagaatcac gtatccaccg tttggctagg tactacaaaa ccaagagcgt attggcaccc     480
   aactggaagt acgaatcaag cacagcatct gctttggtcg cttaaattgt gcttttatgt     540
   taagtttata aaataaaaat ttctactaaa aaaaaaaaaa aaaaatcgga aaaaaa         596
<210> 533
   <211> 600
   <212> DNA
   <213> Diabrotica virgifera
<220>
   <221> misc_feature
   <222> (1)..(600)
   <223> RNAi target sequence
<400> 533
   ggacagcttt tcactgtcat tcgtcattct gatacatttt tttgggtact ttgggttgta      60
   cttgggataa aagacctttt tgtatttcca aatacattgt catataaatt aaaaaagaaa     120
   tggctgccat cagatctatg tctcgccttc ttattaatag atcacttcaa accactgcaa     180
   agaggaccta tgccgaccaa atgaacttca catttgcagc tggaaatcag gtttactata     240
   ctgcaacttc agttaaacaa gtagatgtgc catctttctc tggtagcttt ggtatcctcc     300
   ccgcccatgt acccacactg gctgtgctaa aacctggagt agtcactgtt tacgaagatg     360
   gtggtaatgt taagaaaatc tttgtttcca gtggtactgt caccatcaat gatgattcat     420
   cagttcagat tcttgctgaa gaagcccacc cagtagaaaa cattgatgtc aatgcagcaa     480
   gagacatcct taccaaagcc cagagccagt tatcatctgc aagtacagat gaggcgaaag     540
   ctgaagcctc cattgccgtt gaagtgggag aagcgttagt caaagcggct gaataataat     600
<210> 534
   <211> 22
   <212> DNA
   <213> Artificial Sequence
<220>
   <223> primer
<220>
   <221> misc_feature
   <222> (1)..(22)
   <223> forward primer
<400> 534
   ggacagcttt tcactgtcat tc 22
<210> 535
   <211> 22
   <212> DNA
   <213> Artificial Sequence
<220>
   <223> primer
<220>
   <221> misc_feature
   <222> (1)..(22)
   <223> reverse primer
<400> 535
   attattattc agccgctttg ac 22
<210> 536
   <211> 797
   <212> DNA
   <213> Diabrotica virgifera
<220>
   <221> misc_feature
   <222> (1)..(797)
   <223> WCRW transcript ta03508.001_diavv
<400> 536
   ggtaggggag tgacagacag tcaggtggac agcttttcac ggggattcgt cattctgata        60
   catttttttt gggtactttg ggttgtactt gggataaaag acctttttgt atttccaaat      120
   acattgtcat ataaattaaa aaagaaatgg ctgccatcag atctatgtct cgccttctta       180
   ttaatagatc acttcaaacc actgcaaaga ggacctatgc cgaccaaatg aacttcacat       240
   ttgcagctgg aaatcaggtt tactatactg caacttcagt taaacaagta gatgtgccat       300
   ctttctctgg tagctttggt atcctccccg cccatgtacc cacactggct gtgctaaaac       360
   ctggagtagt cactgtttac gaagatggtg gtaatgttaa gaaaatcttt gtttccagtg       420
   gtactgtcac catcaatgat gattcatcag ttcagattct tgctgaagaa gcccacccag       480
   tagaaaacat tgatgtcaat gcagcaagag acatccttac caaagcccag agccagttat       540
   catctgcaag tacagatgag gcgaaagctg aagcctccat tgccgttgaa gtgggagaag       600
   cgttagtcaa agcggctgaa taataattta atttttgtaa ttttatgtaa atttaataaa       660
   ttagtgtctc aatggtactg ttcattttaa aacaacaaca tattagatct atacctctat       720
   gagttacaat ttttacaaaa acgagaatca atccaaatac ataatttttg tttaccgtta      780
   aaaaaaaaaa aaaaaaa                                                      797
<210> 537
   <211> 612
   <212> DNA
   <213> Diabrotica virgifera
<220>
   <221> misc_feature
   <222> (1)..(612)
   <223> RNAi target sequence
<400> 537
   gggggtttgt aaatctagtc attacagaaa cagtaataat aaattacctt gttctgatta 60
   ttttgtagaa attgtaaaat ggaaaagtgg tgtttttgtt tgtttcttct ttttatatgg 120
   tttgtttccg cacgtggtgc caacaactcg gagtatcctg acctacggcc agaatggtcc 180
   tattgttatg agttcacttg gtttgggcca gattataata acgtgtccac gtacaataat 240
   acgtgtgccg attatctgga cgaaacaaga gccgaaggta taccctgcgc tgctcccatc 300
   gtaatatcgt atgatggaac gttaccggat atgcagtatc tctgggacaa ccacaaagaa 360
   agcgtactct gcaaacgcag cataaaccag atgtgtgtca agtacactta cctctttaac 420
   cagaaaattg aaaatattac ctacatgtgt tctaaagttc agcatgaagg tgggggaaat 480
   attgacagtg gatgctataa acagaaattg gaaagaggat atgaaacaga agtgtgtgta 540
   tgtcaatcct ccaatggaat ttatcctccc tgcaacagaa gtgcctccat atttggttcg 600
   atcaatgttt tc 612
<210> 538
   <211> 19
   <212> DNA
   <213> Artificial Sequence
<220>
   <223> primer
<220>
   <221> misc_feature
   <222> (1)..(19)
   <223> forward primer
<400> 538
   gtttgtaaat ctagtcatt 19
<210> 539
   <211> 22
   <212> DNA
   <213> Artificial Sequence
<220>
   <223> primer
<220>
   <221> misc_feature
   <222> (1)..(22)
   <223> reverse primer
<400> 539
   gaaaacattg atcgaaccaa at 22
<210> 540
   <211> 893
   <212> DNA
   <213> Diabrotica virgifera
<220>
   <221> misc_feature
   <222> (1)..(893)
   <223> WCRW transcript ta03675.001_diavv
<400> 540
   ggtttgtaaa tctagtcatt acagaaacag taataataaa ttaccttgtt ctgattattt     60
   tgtagaaatt gtaaaatgga aaagtggtgt ttttgtttgt ttcttctttt tatatggttt    120
   gtttccgcac gtggtgccaa caactcggag tatcctgacc tacggccaga atggtcctat    180
   tgttatgagt tcacttggtt tgggccagat tataataacg tgtccacgta caataatacg    240
   tgtgccgatt atctggacga aacaagagcc gaaggtatac cctgcgctgc tcccatcgta    300
   atatcgtatg atggaacgtt accggatatg cagtatctct gggacaacca caaagaaagc    360
   gtactctgca aacgcagcat aaaccagatg tgtgtcaagt acacttacct ctttaaccag    420
   aaaattgaaa atattaccta catgtgttct aaagttcagc atgaaggtgg gggaaatatt    480
   gacagtggat gctataaaca gaaattggaa agaggatatg aaacagaagt gtgtgtatgt    540
   caatcctcca atggaattta tcctccctgc aacagaagtg tctccatatt tggttcgatc    600
   aatgttttcc tagtatttgg aatactatgg ttatttgttt ataaattatt ttgactaaaa    660
   ttgttgttat tcatatctct gtataaaact ttaaaaatct caatacttac taaatttcaa    720
   taaggataat aaattatttt aaatatgcag tattctatta ttttactgac tattgcaaat    780
   gccactatca tattttgcaa cttacacgac ctatttgaaa taccaccttc tatacctttt    840
   tgtcttttag aaattgtcgg tcagatagtc aagagaggca gatgtcatta ata           893
<210> 541
   <211> 558
   <212> DNA
   <213> Diabrotica virgifera
<220>
   <221> misc_feature
   <222> (1)..(558)
   <223> RNAi target sequence
<400> 541
   cgggggagtg attatctttg taaaatggca cctacgaagc caaaagcagc ggataaatcc     60
   gcccccaaga aggaagaaaa gaagaagcct gcggccgcac cagcagccgg agccgctgga    120
   gcttcttcct caagttcaaa aacacccgcc ccagctccaa aagcagcagc caaagctgca    180
   agcaaaccag ctgacaagaa agcatcttcc tcaaaaaccc ctgcacccgc acctaaagct    240
   gcctcagcca aaagctccgc gaaagcgcaa gctgctgccc ccaaaccaaa agccgctgct 300
   gctaaagctg gaaaggctgc aggcaaaggt gctgccaaac ccgcagccaa ggctgcagga 360
   aagaaaccag ctgctgctgg aaaggccaag gctcccgcaa ctcttattgc taagcccaag 420
   aagaatgttc ctgttaaaca acaacagaaa ggtgttggca aaaagccagc tgccagtgca 480
   gtgcaagtag cgagagcact taaaatccaa aagaaagtaa ttaaaggccc caatggaaca 540
   cactcaagga aaatccgt 558
<210> 542
   <211> 22
   <212> DNA
   <213> Artificial Sequence
<220>
   <223> primer
<220>
   <221> misc_feature
   <222> (1)..(22)
   <223> forward primer
<400> 542
   cgggggagtg attatctttg ta 22
<210> 543
   <211> 22
   <212> DNA
   <213> Artificial Sequence
<220>
   <223> primer
<220>
   <221> misc_feature
   <222> (1)..(22)
   <223> reverse primer
<400> 543
   acggattttc cttgagtgtg tt 22
<210> 544
   <211> 1017
   <212> DNA
   <213> Diabrotica virgifera
<220>
   <221> misc_feature
   <222> (1)..(1017)
   <223> WCRW transcript ta00525.004_diavv
<400> 544
   gacgcttagg ggaggggcct tttagtgatt atctttgtaa aatggcacct acgaagccaa 60
<210> 545
   <211> 544
   <212> DNA
   <213> Diabrotica virgifera
<220>
   <221> misc_feature
   <222> (1)..(544)
   <223> RNAi target sequence
<400> 545 taca 544
<210> 546
   <211> 22
   <212> DNA
   <213> Artificial Sequence
<220>
   <223> primer
<220>
   <221> misc_feature
   <222> (1)..(22)
   <223> forward primer
<400> 546
   atgcgtgttg aggtcttttc tt 22
<210> 547
   <211> 22
   <212> DNA
   <213> Artificial Sequence
<220>
   <223> primer
<220>
   <221> misc_feature
   <222> (1)..(22)
   <223> reverse primer
<400> 547
   tgtaccaatg gcttagcatg ac 22
<210> 548
   <211> 793
   <212> DNA
   <213> Diabrotica virgifera
<220>
   <221> misc_feature
   <222> (1)..(793)
   <223> WCRW transcript ta05162.001_diavv
<400> 548
   ggctgtcggg ggctgggggg ctaaccaaaa ttatacacat gcgtgttgag gtcttttctt      60
   ttcccaaaac ttgacaaaaa tgggtatcga catcaaccat aaatacgacc gtaaggttag     120
   gagaacagaa cctagaagtc aagatgtata tttacgtctt ttagtaaaac tgtaccgtta     180
   tttagcccgt cgtacagggg ccaaattcaa taaaataatc ctgaaaagat tgtttatgag     240
   caaaatcaac agaccaccac tatctttatc caagttagtg cgtttgatga agaagcctgg     300
   ccgtgatgga cttattgctg tcgtagtagg aacaattact gatgatgcta ggatttttga     360
   aattcccaaa ttgtctgtat gtgcccttcg cgtgacacaa actgctaggg ccaggatctt     420
   gaaagctgga ggtgaagtca tcaccttcga ccaattggca ctcagggccc caactggcaa     480
   taaaactgta ttgctacaag gcagacgtaa tgcccgtgaa gctgtgaaac actttggtcc     540
   tgctcctggt gtaccacaca gtcatgctaa gccattggta cattccaagg gacgcaaatt     600
   tgagagagca cgaggtcgta ggcgctcatg tggttacaag aaatagactt aagttaatca     660
   ttttgtttct gctgtattca tatttgttgt tcaataaata ggaattgcag tacattggaa     720
   ttttgttttg tattttatag aatataacaa aataaaggaa tttaatataa aaaaaaaaaa     780
   aaaaaaaaaa aaa                                                        793
<210> 549
   <211> 401
   <212> DNA
   <213> Diabrotica virgifera
<220>
   <221> misc_feature
   <222> (1)..(401)
   <223> RNAi target sequence
<400> 549
   aaagaaaccg aaggcagaga aaaaggcacc aaaggagggt gcaactccaa gtgataaacc      60
   ctccgttaaa agacgtcctt tcaaaagaca tggacgttta tatgctaaag ccatcttcac     120
   aggttacaaa agaggtcttc gtaatcaaca cgaaaataca gccctattat ctgtagaagg     180
   aaccagatca aaagatgaga gttggtttta cgtaggaaag aaatgcgtct atgtatacaa     240
   agcaaaaaac aggacatgtg ttcccggtag gcccaaatct gtcaaatcca aagttagggc     300
   tatttgggga aaagtcacca gaccacatgg cacaaggggt gcagtgagag ccaaattcaa     360
   taggaattta ccagccaagg ctatgggaca tcgtattcgt a                         401
<210> 550
   <211> 21
   <212> DNA
   <213> Artificial Sequence
<220>
   <223> primer
<220>
   <221> misc_feature
   <222> (1)..(21)
   <223> forward primer
<400> 550
   aaagaaaccg aaggcagaga a 21
<210> 551
   <211> 22
   <212> DNA
   <213> Artificial Sequence
<220>
   <223> primer
<220>
   <221> misc_feature
   <222> (1)..(22)
   <223> reverse primer
<400> 551
   tacgaatacg atgtcccata gc 22
<210> 552
   <211> 1008
   <212> DNA
   <213> Diabrotica virgifera
<220>
   <221> misc_feature
   <222> (1)..(1008)
   <223> WCRW transcript ta00344.005_diavv
<400> 552
   ccgatttttt tttttttttt ttgtgttgga aaacgtattt tttatttctt atttatattt      60
   ataaaaacaa ctatatctta gatgggtaca gcataatacg aatacgatgt cccatagcct     120
   tggctggtaa attcctattg aatttggctc tcactgcacc ccttgtgcca tgtggtctgg     180
   tgacttttcc ccaaatagcc ctaactttgg atttgacaga tttgggccta ccgggaacac     240
   atgtcctgtt ttttgctttg tatacataga cgcatttctt tcctacgtaa aaccaactct     300
   catcttttga tctggttcct tctacagata atagggctgt attttcgtgt tgattacgaa     360
   gacctctttt gtaacctgtg aagatggctt tagcatataa acgtccatgt cttttgaaag     420
   gacgtctttt aacggagggt ttatcacttg gagttgcacc ctcctttggt gcctttttct    480
   ctgccttcgg tttctttgcc gctgcgtctg ccattccgaa gatctcgttt agaattaaaa     540
   agaagttatt tcatttttct cctccaagtg ataaaccctc cgttaaaaga cgtcctttca     600
   aaagacatgg acgtttatat gctaaagcca tcttcacagg ttacaaaaga ggtcttcgta     660
   atcaacacga aaatacagcc ctattatctg tagaaggaac cagatcaaaa gatgagagtt     720
   ggttttacgt aggaaagaaa tgcgtctatg tatacaaagc aaaaaacagg acatgtgttc     780
   ccggtaggcc caaatctgtc aaatccaaag ttagggctat ttggggaaaa gtcaccagac     840
   cacatggcac aaggggtgca gtgagagcca aattcaatag gaatttacca gccaaggcta     900
   tgggacatcg tattcgtatt atgctgtacc catctaagat atagttgttt ttataaatat     960
   aaataagaaa taaaaaatac gttttccaac aaaaaaaaaa aaaaaaaa                1008
<210> 553
   <211> 542
   <212> DNA
   <213> Diabrotica virgifera
<220>
   <221> misc_feature
   <222> (1)..(542)
   <223> RNAi target sequence
<400> 553
   aatctgtaaa catcggcgta gaatcgccat taatatataa gtattttata tagaattaat 60
   tcaaaatgcc acccaagttc gatcccaccg aaattaaata tgtttacatg agatgtgtcg 120
   gaggagaagt tggagctacc tcttctttgg ctcctaaaat tggtccacta ggtttgtctc 180
   ccaaaaaagt tggtgacgat attgccaaag gcactgctga ctggaaaggt cttaaaatca 240
   cagttcagtt gaaaatccaa aatcgtcaag ctaccatttc tgtagtccca tctgcctcct 300
   ccctgatcat tagggctctt aaagaaccac cacgcgatag gaagaagcaa aagaacatta 360
   aacacactgg taacattact ttcgatgaaa tcatcgctat tgccagacag atgaggccca 420
   ggtcaatggc caaacagtta tccggaactg taaaagaggt tttaggaaca gcccaatcag 480
   ttggatgtac aattgatggt aaagcagcac atgatgttat tgacgaggtt aatgaaggaa 540
   cc 542
<210> 554
   <211> 23
   <212> DNA
   <213> Artificial Sequence
<220>
   <223> primer
<220>
   <221> misc_feature
   <222> (1)..(23)
   <223> forward primer
<400> 554
   aatctgtaaa catcggcgta gaa 23
<210> 555
   <211> 21
   <212> DNA
   <213> Artificial Sequence
<220>
   <223> primer
<220>
   <221> misc_feature
   <222> (1)..(21)
   <223> reverse primer
<400> 555
   gttccttcat taacctcgtc a 21
<210> 556
   <211> 768
   <212> DNA
   <213> Diabrotica virgifera
<220>
   <221> misc_feature
   <222> (1)..(768)
   <223> WCRW transcript ta01789.001_diavv
<400> 556
   ggaagatgat tgaccatgtc aattgtcacg tcatgtgtca ggggtccttt tcgaatttac 60
   cattgtgaaa atattttcac tattgtaaaa atctgtaaac atcggcgtag aatcgccatt 120
   aatatataag tattttatat agaattaatt caaaatgcca cccaagttcg atcccaccga 180
   aattaaatat gtttacatga gatgtgtcgg aggagaagtt ggagctacct cttctttggc 240
   tcctaaaatt ggtcctctag gtttgtctcc caaaaaagtt ggtgacgata ttgccaaagg 300
   tactgctgat tggaaaggtc ttaaaatcac agttcaattg aaaatccaaa atcgtcaagc 360
   taccatttct gtagtcccat ctgcttcctc cctgatcatt agggctctta aagaaccacc 420
   acgcgatagg aagaagcaaa agaacattaa acacactggt aacattactt tcgatgaaat 480
   catcgctatt gccagacaga tgaggcccag gtcgatggcc aaacagttat ccggaactgt 540
   aaaagaggtt ttaggaacag cccaatcagt tggatgtaca attgatggta aagcagcaca 600
   tgatgttatt gacgaggtta atgaaggaac catagaagta ccagatgaat aaactattat 660
   tagaatattt tttctaagtt atcagttgtt ttatttaacc tttatccaaa tgtggcattt 720
   tttaatttgt taagaatgta attgatggat tcgaccgtta cttgatgg 768
<210> 557
   <211> 450
   <212> DNA
   <213> Diabrotica virgifera
<220>
   <221> misc_feature
   <222> (1)..(450)
   <223> RNAi target sequence
<220>
   <221> misc_feature
   <222> (341)..(341)
   <223> n can be any nucleotide
<220>
   <221> misc_feature
   <222> (413)..(413)
   <223> n can be any nucleotide
<220>
   <221> misc_feature
   <222> (426)..(426)
   <223> n can be any nucleotide
<400> 557
   aggcatttca aaaacaacca accgtttttc tcaaccgcaa aagtggagga aagaaaaatt      60
   taagacattc tcgcagtgta gggctcggtt ttaaaactcc cagagaggca attgaaggtt     120
   cttgcattga caagaaatgt cccttcactg gaaatgtatc aatccgtggc cgtattctca     180
   ctggagttgt ccaaaaaatg aagatgcaaa ggactattgt catccgtaga gattatttgc     240
   attatatcag gaaatacaat cgttttgaaa agaggcatag gaatatgtct gtacatttat     300
   caccctgctt cagggatgtg gaaattggag atgtagttac nattggtgaa tgcagaccac     360
   tttcaaaaac agttagattc aacgtattga aagtaactaa aggtagcagt tcnaagaaga     420
   gcttcnggaa gttttgagtg atgaaacatt                                      450
<210> 558
   <211> 22
   <212> DNA
   <213> Artificial Sequence
<220>
   <223> primer
<220>
   <221> misc_feature
   <222> (1)..(22)
   <223> forward primer
<400> 558
   aggcatttca aaaacaacca ac 22
<210> 559
   <211> 24
   <212> DNA
   <213> Artificial Sequence
<220>
   <223> primer
<220>
   <221> misc_feature
   <222> (1)..(24)
   <223> reverse primer
<400> 559
   aatgtttcat cactcaaaac ttcc 24
<210> 560
   <211> 615
   <212> DNA
   <213> Diabrotica virgifera
<220>
   <221> misc_feature
   <222> (1)..(615)
   <223> WCRW transcript ta02086.002_diavv
<400> 560
   cttgtcaatg taagaacctt caattgcctc tctgggagtt ttaaaaccga gccctacact      60
   gcgagaaggt cataaagggg aatcctttct tttactagtg aaaatggcgg atcagaccga     120
   aaaggcattt caaaaacaac caaccgtttt tctcaaccgc aaaagtggag gaaagaaaaa     180
   tttaagacat tctcgcagtg tagggctcgg ttttaaaact cccagagagg caattgaagg     240
   ttcttacatt gacaagaaat gtcccttcac tggaaatgta tcaatccgtg gccgtattct     300
   cactggagtt gtccaaaaaa tgaagatgca aaggactatt gtcatccgta gagattattt     360
   gcattatatc aggaaataca atcgttttga aaagaggcat aggaatatgt ctgtacattt     420
   atcaccctgc ttcagggatg tggaaattgg agatgtagtt acaattggtg aatgcagacc     480
   actttcaaaa acagttagat tcaacgtatt gaaagtaact aaaggtagca gttcaaagaa     540
   gagcttcagg aagttttgag tgatgaaaca tttttaaaat aaaaatctta taaaatgaaa     600
   aaaaaaaaaa aaaaa                                                      615
<210> 561
   <211> 225
   <212> DNA
   <213> Diabrotica virgifera
<220>
   <221> misc_feature
   <222> (1)..(225)
   <223> RNAi target sequence DV-HP2-FRAG1
<400> 561
   gaaacgacaa ggaggaaagt tgtgtcttgc ggagtactag aaacaccgag attcggacga      60
   aagagtactt tcctgttcgt gcccggtacg aaggttacct atgaatgcat ccaagagttc     120
   gtgttggtag gtgatcagcg gagagagtgt caagcggatg gaacatggaa tattcctgaa     180
   tacggataca catattgctt acgtcagcag gaatactctc aacga                     225
<210> 562
   <211> 212
   <212> DNA
   <213> Diabrotica virgifera
<220>
   <221> misc_feature
   <222> (1)..(212)
   <223> RNAi target sequence DV-HP2-FRAG2
<400> 562
   gagcattgtt ccacagggaa ttcggaagga cttcagcgac ttacaacaac aaaacgttca 60
   agcctcaatt cctaatgaat ccggaagatt tcctaccaac caacagatct acagatctta 120
   agcgaatatc cgaaatatgt cctctaaagt tgtacgaatg ttactacgat tacgctatga 180
   cggttgacag ggatttggcg cattacacca ag 212
<210> 563
   <211> 210
   <212> DNA
   <213> Diabrotica virgifera
<220>
   <221> misc_feature
   <222> (1)..(210)
   <223> RNAi target sequence DV-HP2-FRAG3
<400> 563
   cttgggagaa gcagaatttg accacaaacg acaacgctaa catccgaatt tctctctggg 60
   gttacagaga aacgactatg aaaccagtgt ttgtttatat caccgatatc gctgataatg 120
   ttcaaaacat tggagaacac acgattgttc cttctcagtt tagaactaga gtcaatcaat 180
   atctgactga tattaagttt ggattcctcc 210
<210> 564
   <211> 229
   <212> DNA
   <213> Diabrotica virgifera
<220>
   <221> misc_feature
   <222> (1)..(229)
   <223> RNAi target sequence DV-HP2-FRAG4
<400> 564
   aatggaaagg gagaattcgc attggtgaaa tcagttacgc aaacggacaa tctggaaatc 60
   caaggaagat ttgagcaaat ggaccctaac acttatggtg aggttagagc tactcagctg 120
   acttctgtag tagctaaagg taataacaca atagcaattg aagtcagaag gagacctctg 180
   gattctagat ggagatatag gttggatgtt atagcagata atagaagac 229
<210> 565
   <211> 170
   <212> DNA
   <213> Diabrotica virgifera
<220>
   <221> misc_feature
   <222> (1)..(170)
   <223> RNAi target sequence DV-HP2-FRAG5
<400> 565
   gaaacgacaa ggaggaaagt tgtgtcttgc ggagtactag aaacaccgag attcggacga 60
   aagagtactt tcctgttcgt gcccggtacg aaggttacct atgaatgcat ccaagagttc 120
   gtgttggtag gtgatcagcg gagagagtgt caagcggatg gaacatggaa 170
<210> 566
   <211> 233
   <212> DNA
   <213> Diabrotica virgifera
<220>
   <221> misc_feature
   <222> (1)..(233)
   <223> RNAi target sequence DV-HP2-FRAG6
<400> 566
   gaaacgacaa ggaggaaagt tgtgtcttgc ggagtactag aaacaccgag attcggacga 60
   aagagtactt tcctgttcgt gcccggtacg aaggttacct atgaatgcat ccaagagttc 120
   gtgttggtag gtgatcagcg gagagagtgt caagcggatg gaacatggaa tattcctgaa 180
   tacggataca catattgctt acgtcagcag gaatactctc aacgacaagc tgg 233
<210> 567
   <211> 162
   <212> DNA
   <213> Diabrotica virgifera
<220>
   <221> misc_feature
   <222> (1)..(162)
   <223> RNAi target sequence DV-HP2-FRAG7
<400> 567
   tttgttattt ttgttttgtt tagtgttagc gttaaaggac tagatggtga tcttccacca 60
   gatgtcgaaa ctatcgatgt ggagactacg aatgtaagta acgcggatcc agacgcaggg 120
   aaacctattc cggtggaaga tacgcctaat ggagtccctc ca 162
<210> 568
   <211> 473
   <212> DNA
   <213> Diabrotica virgifera
<220>
   <221> misc_feature
   <222> (1)..(473)
   <223> RNAi target sequence DV-HP2-FRAG8
<400> 568
   gtgaatacaa actatgaagt agataaatcc ttagttaatg taaccaatag tttggtaaat 60
   gtgactaaaa atcccagacg gacttatcag acagttaata aaatgggcaa atattcgaca 120
   ttttatgctg cagattacga tcctatgacc tcagacacag ctccaccaga cactgaccaa 180
   cgaggaggag gaggaactcc atacactata tcagaaccga gactggcaga aataaggaaa 240
   cattttatgt acccattttt cgataaagga ggcagtgata ataacttggg agatttccaa 300
   aaggacattc aatcttccat tcctcaggtg cacaagaatt taaacttcca gttgcctttc 360
   tttggattca ggtataatta cacaagagtt tcagtgaatg ggtatctgga attcagcgat 420
   ccacctccaa attatgacta tccattagta tttccaatta aagaatggcc caa 473
<210> 569
   <211> 430
   <212> DNA
   <213> Diabrotica virgifera
<220>
   <221> misc_feature
   <222> (1)..(430)
   <223> RNAi target sequence DV-HP2-FRAG9
<400> 569
   aaagaatgat cctgccttta ttggcatttt cttcagcaga tgcagaatcg gtaatctacg 60
   tgatggagac attgatcaaa gaacacccgg ggtctatttt agaatggaaa gagatttaag 120
   aacgaggcaa gacagaatgg gtgtagaaat tagagaaaga cttaagtggg atatcagaac 180
   aggagtcatt ggatcagaaa cattcgatcc taagcacgcc gttattgtca cttggaaaaa 240
   tgttactttc actggaggat tcgctaatgc taaatacaag acaaacacct tccaaatggt 300
   tcttgcaact gacgaagtct tcacttacgc gatgttcaac tacctcaact tggactggac 360
   ctcccacact gaagcgggtg gtgattccca aaacggggaa ggtggtgtca gtgcctatgt 420
   tggttttaac 430
<210> 570
   <211> 486
   <212> DNA
   <213> Diabrotica virgifera
<220>
   <221> misc_feature
   <222> (1)..(486)
   <223> RNAi target sequence DV-HP2-FRAG10
<400> 570
   ccagaagcta cgaatacaat ccttacagtc aagcttccgt cattcgagat ctcacatcag 60
   ttggatttgg aaatggtttt aagggaagac atattttccg tatagacgaa gacattttgc 120
   ttggatcgtg caacaaagat atagatggtg ccaaccttcc cctgaaattc gctcctgaaa 180
   gtggtaacat gttgggtgga acagtggtaa atataactgg tccatgcttc aatttgaatg 240
   ataggattcg atgcaagttt gacgtatcaa atgaagtatt tgggtatgtt gttgataaaa 300
   acagagctat ctgtgtacaa cctcaacttt atgccgaagg atgggtgaat cttcaaattg 360
   ctgttaattc cgaagcattc aagtggaagg ggaagtatta tgtagaatca cctgctagtg 420
   cgactcaaaa aatcttcttt aaggacatga aattacacga aaaatctccg agcgaattaa 480
   aaatca 486
<210> 571
   <211> 500
   <212> DNA
   <213> Diabrotica virgifera
<220>
   <221> misc_feature
   <222> (1)..(500)
   <223> RNAi target sequence DV-HP2-FRAG11
<400> 571
   aaattaattt aactgagtct atcacagtta atacatatac tacaagccag agcagcatag 60
   aattaacacc ggtaatatgg agccgaccca ttccactcgg ttggtacttc cagttccaat 120
   gggaaaacca gtacggtaaa aattggccca aatatctatg tgacgattgg ctgagaacgg 180
   acagatatct aaaaaacttc gcccacgaac ttgctcaatg tccctgcacc gtcgaacagg 240
   ctttagctga caagggcagg tttatgccag acttcgattg cgacaaagat tcgaacccga 300
   tttgttacta taacaaccaa gcacttcatt gtgtgaaaac tggatctccc actttagaag 360
   gctccgaaca acaatgttgc tatgataaaa acggcttttt gatgctctct tacgatcagc 420
   aatggggttc tagtccgaga cgttgccaca acttgggtaa aatgccttac aatgaagcga 480
   caaaagtgcc tactctctca 500
<210> 572
   <211> 310
   <212> DNA
   <213> Diabrotica virgifera
<220>
   <221> misc_feature
   <222> (1)..(310)
   <223> RNAi target sequence DV-HP2-FRAG12
<400> 572
   ggtgttacta tttacactcc aagctacatc cttaatcaat cagaagtcat cattatgttt        60
   gacaatggag ctggaatgga ggttgtcgac aatcaaggat tcatgagtgc aagagtattt       120
   ttgccttggt cgtttataaa taaaaccata ggcctttttg gaaattggag ttttaacaaa       180
   gaagatgact tcacacttcc agatggttct agagcagcaa tagtgaataa cataaatgac       240
   atggaaaggg tttacaatga ttttggttcc aaatggatgt tggaggatgt gttagatcct       300
   caaaagggta                                                              310
<210> 573
   <211> 1327
   <212> DNA
   <213> Diabrotica virgifera
<220>
   <221> misc_feature
   <222> (1)..(1327)
   <223> RNAi target sequence DV-RYANR-FIS
<400> 573 agagaaa 1327
<210> 574
   <211> 193
   <212> DNA
   <213> Diabrotica virgifera
<220>
   <221> misc_feature
   <222> (1)..(193)
   <223> RNAi target sequence DV-RYANR-FRAG1
<400> 574
   tttacgaaaa tgacaagtat cgagactgtg gggaccattg tcctgaaatt gctgaagttg        60
   gtgatcaatt tgatatgtct catcttgtac cgaaccggat atcaaggcta cttcttggga       120
   gtaggaggaa cctggaatct aaacgaagaa aaaaatcccg atgcagaaat tgtggcttcc       180
   ggcgtattcg tag                                                          193
<210> 575
   <211> 145
   <212> DNA
   <213> Diabrotica virgifera
<220>
   <221> misc_feature
   <222> (1)..(145)
   <223> RNAi target sequence DV-RYANR-FRAG2
<400> 575
   acgaaaatga caagtatcga gactgtgggg accattgtcc tgaaattgct gaagttggtg        60
   atcaatttga tatgtctcat cttgtaccga accggatatc aaggctactt cttgggagta       120
   ggaggaacct ggaatctaaa cgaag                                             145
<210> 576
   <211> 83
   <212> DNA
   <213> Diabrotica virgifera
<220>
   <221> misc_feature
   <222> (1)..(83)
   <223> RNAi target sequence DV-RYANR-FRAG3
<400> 576
   accattgtcc tgaaattgct gaagttggtg atcaatttga tatgtctcat cttgtaccga        60
   accggatatc aaggctactt ctt                                                83
<210> 577
   <211> 292
   <212> DNA
   <213> Diabrotica virgifera
<220>
   <221> misc_feature
   <222> (1)..(292)
   <223> RNAi target sequence DV-RYANR-FRAG4
<400> 577
   atttatgatt tacacattcg tctcgctgat cagcctttgc ttcgctagtg gagatcacaa 60
   aacgacattc actgatattc tgatgaatat agtagggatt tttatgtgga tagctgctgg 120
   agctacagct cttcattatt ggcttgggta cttgtccgaa tacaaataca cgacaataga 180
   ttctgaacga caagttggtt tggcgttagg agcgatgtgt ataataaatg gagcggtcta 240
   tcttgtagac ggagtacttt ccgcaatctt tatcctcaaa gccaaaatgc aa 292
<210> 578
   <211> 502
   <212> DNA
   <213> Diabrotica virgifera
<220>
   <221> misc_feature
   <222> (1)..(502)
   <223> RNAi target sequence DV-RYANR-FRAG5
<400> 578
   ataataagtt cgatttttta cgaaaatgac aagtatcgag actgtgggga ccattgtcct 60
   gaaattgctg aagttggtga tcaatttgat atgtctcatc ttgtaccgaa ccggatatca 120
   aggctacttc ttgggagtag gaggaacctg gaatctaaac gaagaaaaaa atcccgatgc 180
   agaaattgtg gcttccggcg tattcgtagg atttatgatt tacacattcg tctcgctgat 240
   cagcctttgc ttcgctagtg gagatcacaa aacgacattc actgatattc tgatgaatat 300
   agtagggatt tttatgtgga tagctgctgg agctacagct cttcattatt ggcttgggta 360
   cttgtccgaa tacaaataca cgacaataga ttctgaacga caagttggtt tggcgttagg 420
   agcgatgtgt ataataaatg gagcggtcta tcttgtagac ggagtacttt ccgcaatctt 480
   tatcctcaaa gccaaaatgc aa 502
<210> 579
   <211> 199
   <212> DNA
   <213> Diabrotica virgifera
<220>
   <221> misc_feature
   <222> (1)..(199)
   <223> RNAi target sequence DV-RYANR-FRAG6
<400> 579
   gcagctcaag tataccgtga catcccactc atacatcaat gtctataatt gtttcatgac 60
   aaatcattta atagtatttt aaagcattca ttcgttcaac acctttttta catttttcaa 120
   gtattatatc aatcttttta aagaattgtt tggctattaa ccggattcaa tccatatagt 180
   tccgtaaaat aattatgcg 199
<210> 580
   <211> 197
   <212> DNA
   <213> Diabrotica virgifera
<220>
   <221> misc_feature
   <222> (1)..(197)
   <223> RNAi target sequence DV-RYANR-FRAG7
<400> 580
   ctgccgcaac aaattaggga gaatactttt gtctcctgta gttttatctg ttatccaaag 60
   ttcgattaaa ttgtttcaat ttaatgtatt tttccatata acgcaccaaa tagtcttaaa 120
   aaaatagtgc ctgttactac ctaatacttt ctacgtatgc cttatggtta gtagtggctc 180
   caataggtgc attttac 197
<210> 581
   <211> 142
   <212> DNA
   <213> Diabrotica virgifera
<220>
   <221> misc_feature
   <222> (1)..(142)
   <223> RNAi target sequence DV-RYANR-FRAG8
<400> 581
   gatttatgat ttacacattc gtctcgctga tcagcctttg cttcgctagt ggagatcaca 60
   aaacgacatt cactgatatt ctgatgaata tagtagggat ttttatgtgg atagctgctg 120
   gagctacagc tcttcattat tg 142
<210> 582
   <211> 129
   <212> DNA
   <213> Diabrotica virgifera
<220>
   <221> misc_feature
   <222> (1)..(129)
   <223> RNAi target sequence DV-RYANR-FRAG9
<400> 582
   cacattcgtc tcgctgatca gcctttgctt cgctagtgga gatcacaaaa cgacattcac 60
<210> 583
   <211> 152
   <212> DNA
   <213> Diabrotica virgifera
<220>
   <221> misc_feature
   <222> (1)..(152)
   <223> RNAi target sequence DV-RYANR-FRAG10
<400> 583
   ggcttgggta cttgtccgaa tacaaataca cgacaataga ttctgaacga caagttggtt 60
   tggcgttagg agcgatgtgt ataataaatg gagcggtcta tcttgtagac ggagtacttt 120
   ccgcaatctt tatcctcaaa gccaaaatgc aa 152
<210> 584
   <211> 587
   <212> DNA
   <213> Diabrotica virgifera
<220>
   <221> misc_feature
   <222> (1)..(587)
   <223> RNAi target sequence DV-RYANR-RASRHO-FIS
<400> 584
   aactttgtgt gttttggcag gtccgatggc tgcaataaga aaaaaattgg tgattgttgg 60
   tgacggagca tgtggaaaaa catgtctttt aatagtattt agtaaagatc agttcccaga 120
   agtatacgtg cccactgtat tcgaaaacta tgtggcggat attgaagtag atggaaagca 180
   ggtagaactc gccttatggg ataccgcagg tcaagaagat tatgatcgtc tgcggcccct 240
   ttcatatcca gacaccgatg tgattttaat gtgcttctca gtagactccc ccgactcatt 300
   agaaaacatc ccagaaaaat ggaccccaga ggtaaaacac ttttgtccaa atgtgccaat 360
   tattctggta ggcaataaga aggatctgcg taacgaccct actaccatta acgaacttaa 420
   aaagatgaaa caagaaccag taaaacctca agacggaaga tccatggcag agaaaatcaa 480
   tgcttttgca tatctagaat gttctgccaa aagtaaggag ggtgtaagag aggtttttga 540
   aaatgctacc cgagctgcac tgcaagtcaa aaagaagaag aagcccc 587
<210> 585
   <211> 163
   <212> DNA
   <213> Diabrotica virgifera
<220>
   <221> misc_feature
   <222> (1)..(163)
   <223> RNAi target sequence DV-RYANR-RASRHO-FRAG1
<400> 585
   aagacggaag atccatggca gagaaaatca atgcttttgc atatctagaa tgttctgcca 60
   aaagtaagga gggtgtaaga gaggtttttg aaaatgctac ccgagctgca ctgcaagtca 120
   aaaagaagaa gaagccccgt tgtgtaatgt tttgagccac tac 163
<210> 586
   <211> 178
   <212> DNA
   <213> Diabrotica virgifera
<220>
   <221> misc_feature
   <222> (1)..(178)
   <223> RNAi target sequence DV-DEAD-FRAG1
<400> 586
   gaggattcta gtagccacaa acttatttgg acgtggaatg gatatcgaaa gagtgaacat 60
   cgtcttcaac tatgacatgc ccgaagattc tgacacttac ctgcacagag tagcccgagc 120
   tggacgtttc ggtaccaaag gtctagccat tactttcgta tcagaagagg gagatgcc 178
<210> 587
   <211> 152
   <212> DNA
   <213> Diabrotica virgifera
<220>
   <221> misc_feature
   <222> (1)..(152)
   <223> RNAi target sequence DV-HPP15-FRAG1
<400> 587
   ggacaactga ccttcatcac tcaagtgact ggcagtgtaa aataccaaga gaaaacacca 60
   aaaacctttc agcgaaactt cataattaca gcacagggcg ataaatggaa aattgtcagt 120
   gattgtatga ggctgcaaga accattaaac gc 152
<210> 588
   <211> 402
   <212> DNA
   <213> Diabrotica virgifera
<220>
   <221> misc_feature
   <222> (1)..(402)
   <223> RNAi target sequence DV-HPP15-BPSAR2-FRAG1
<400> 588
   ttccattggt aacatgaggt ttacgacgtt cgatttgggg ggccacgagc aggctaggag      60
   agtgtggagg gactactttc cagcagtcga tgccatagtg ttccttgttg atgccaacga     120
   cagctcaaga tttgtagaaa gccaggaaca gctaaatgcc ctcctctcag acgaaactct     180
   atcaaactgt ccaatactta tcttaggtaa taaaattgat ctcccaggtg ctgcttcgga     240
   agatgaatta cgaactagat tcggcttgtt tggccaaacc acaggcaaag gcaaagtagc     300
   cagaaatgat ctacccggta ggcctctaga actatttatg tgctctatac tcaaaagaca     360
   aggttatgga gaaggtttcc gttggttggc acaatatatc ga                         402
<210> 589
   <211> 171
   <212> DNA
   <213> Diabrotica virgifera
<220>
   <221> misc_feature
   <222> (1)..(171)
   <223> RNAi target sequence DV-HPP15-BPSAR2-FRAG2
<400> 589
   ccattggtaa catgaggttt acgacgttcg atttgggggg ccacgagcag gctaggagag      60
   tgtggaggga ctactttcca gcagtcgatg ccatagtgtt ccttgttgat gccaacgaca     120
   gctcaagatt tgtagaaagc caggaacagc taaatgccct cctctcagac g              171
<210> 590
   <211> 137
   <212> DNA
   <213> Diabrotica virgifera
<220>
   <221> misc_feature
   <222> (1)..(137)
   <223> RNAi target sequence DV-HPP15-BPSAR2-FRAG3
<400> 590
   ttgatctccc aggtgctgct tcggaagatg aattacgaac tagattcggc ttgtttggcc      60
   aaaccacagg caaaggcaaa gtagccagaa atgatctacc cggtaggcct ctagaactat     120
   ttatgtgctc tatactc                                                    137
<210> 591
   <211> 135
   <212> DNA
   <213> Diabrotica virgifera
<220>
   <221> misc_feature
   <222> (1)..(135)
   <223> RNAi target sequence DV-HPP15-BPSAR2-FRAG4
<400> 591
   ggccacgagc aggctaggag agtgtggagg gactactttc cagcagtcga tgccatagtg        60
   ttccttgttg atgccaacga cagctcaaga tttgtagaaa gccaggaaca gctaaatgcc       120
   ctcctctcag acgaa                                                        135
<210> 592
   <211> 294
   <212> DNA
   <213> Diabrotica virgifera
<220>
   <221> misc_feature
   <222> (1)..(294)
   <223> RNAi target sequence DV-HPP15-BPSAR2-FRAG5
<400> 592
   ctttataatt tgtcgttgaa aaagataggg ctccaaagtc ctattgccaa caaaaaaaca        60
   acgcaagaca ttaaacaact tttttcaagc ccatctgtat ttaaaaaaac agtgcaaaat       120
   gtttctgtgg gactggttta cgggaatgct gggatatcta ggattgtgga agaaacctgg       180
   aaaactatta ttcttaggac tggataacgc aggcaaaact acccttctac atatgctcaa       240
   ggatgacaga ctggcccagc atcttcccac gttacatccc acatcagagg agct             294
<210> 593
   <211> 407
   <212> DNA
   <213> Diabrotica virgifera
<220>
   <221> misc_feature
   <222> (1)..(407)
   <223> RNAi target sequence DV-HPP15-BPSAR2-FRAG6
<400> 593
   ttaattatgt atttttccat ttcgttctgt cattgagtta ggatattaat gtttgaggaa 60
   ctattggcaa cactgcaact acctgattca tttcagatct taggtacttt acataaatat 120
   ctaaatatag atgttggcaa tgtaattttg aacaacagta tatacattca atgtaaatta 180
   tatattttta gttaagttac cttcttaaat ggtgtttgag tggctatggt actgaaatag 240
   tttgactttt tgtgttcttc gataactaaa aatgatttct tgtggaaagt tacactcaga 300
   attacatagt taacttcttt atcagcagtt gttgtcaaga tttccatttt gagacgattg 360
   ttttgtaaat taggtgggaa tttttaaaat gttggactgt tttttaa 407
<210> 594
   <211> 168
   <212> DNA
   <213> Diabrotica virgifera
<220>
   <221> misc_feature
   <222> (1)..(168)
   <223> RNAi target sequence DV-HP1-FRAG1
<400> 594
   ggggaaggta aatcagtatt cacttgatct tcaaggtaga ttaacgcaag tagaaatcta 60
   aaacatgtct ggacgtggta agggaggcaa agttaaggga aaagcaaagt cccgatcaaa 120
   tcgtgctggt ttacaatttc ctgtaggtcg tattcatcgt ttattgag 168
<210> 595
   <211> 190
   <212> DNA
   <213> Diabrotica virgifera
<220>
   <221> misc_feature
   <222> (1)..(190)
   <223> RNAi target sequence DV-HP1-FRAG2
<400> 595
   ggggaaggta aatcagtatt cacttgatct tcaaggtaga ttaacgcaag tagaaatcta 60
   aaacatgtct ggacgtggta agggaggcaa agttaaggga aaagcaaagt cccgatcaaa 120
   tcgtgctggt ttacaatttc ctgtaggtcg tattcatcgt ttattgagaa aaggaaatta 180
   tgccgaaaga 190
<210> 596
   <211> 159
   <212> DNA
   <213> Diabrotica virgifera
<220>
   <221> misc_feature
   <222> (1)..(159)
   <223> RNAi target sequence DV-HP1-FRAG3
<400> 596
   ttggaattgg caggaaatgc agctagagat aacaaaaaga cccgtataat tcctagacat      60
   ttacaattgg ccataagaaa tgacgaggaa ttgaacaaat tactgtcagg agttaccatc     120
   gcccaaggtg gagtattgcc taatatacaa gcagtactt                            159
<210> 597
   <211> 196
   <212> DNA
   <213> Diabrotica virgifera
<220>
   <221> misc_feature
   <222> (1)..(196)
   <223> RNAi target sequence DV-RNAPOL-FRAG1
<400> 597
   ctgttttaaa gccatggctg atgaagaatt tgacgaaaat gatgtagcag atgatttcga      60
   tgacgacgta gaggatgata atatcgaaga actcgaacaa cccgaggaag atggagataa     120
   catcgatatc cttgctccag gacaagcagg aggtggtgta ccaaaaaaca agaggataac     180
   aactaaatat atgacc                                                      196
<210> 598
   <211> 163
   <212> DNA
   <213> Diabrotica virgifera
<220>
   <221> misc_feature
   <222> (1)..(163)
   <223> RNAi target sequence DV-CPNN-FRAG1
<400> 598
   ctgttggtgc tacttgtctt ccacgcctaa cagctcccac atctcaagaa cttggttacg       60
   cagatattgt atgtgtagaa gaattgggtg atacccctat agtttctttc agattggagg      120
   gcaaagaatc aagaatttcc acaattgtca tcagaggtgc tac                       163
<210> 599
   <211> 236
   <212> DNA
   <213> Diabrotica virgifera
<220>
   <221> misc_feature
   <222> (1)..(236)
   <223> RNAi target sequence DV-CPNN-FRAG2
<400> 599
   cagccaaact catgatacta ggaagtcaaa tgcaagattc tgaggtgggt gatggaacta 60
   actttgtgat tatacttgct ggagcacttt tggagcatgc tgaagaacta attcgtcttg 120
   gagtaactcc cacagaaata gcagaaggtt atgagaaagc actagacaaa tgtctagaaa 180
   tccttcctaa tcttatttgc tacacaatca aggatttcag aaacatcgaa gaagtc 236
<210> 600
   <211> 149
   <212> DNA
   <213> Diabrotica virgifera
<220>
   <221> misc_feature
   <222> (1)..(149)
   <223> RNAi target sequence DV-CPNN-FRAG3
<400> 600
   ttggagcatg ctgaagaact aattcgtctt ggagtaactc ccacagaaat agcagaaggt 60
   tatgagaaag cactagacaa atgtctagaa atccttccta atcttatttg ctacacaatc 120
   aaggatttca gaaacatcga agaagtcaa 149
<210> 601
   <211> 194
   <212> DNA
   <213> Diabrotica virgifera
<220>
   <221> misc_feature
   <222> (1)..(194)
   <223> RNAi target sequence DV-CPNN-FRAG4
<400> 601
   ctgcagatga acttatgaac tttagtaggg gagaagaaaa tcttcttgaa ttacaaatca 60
   aatgtatagc agatgctgga gctaaagttg tagttgcagg agccaaattt ggagatatgg 120
   ctctacatta cttacataaa tatggcatga tggttgtcag gttgaactcc aaatttgatt 180
   tgagaagatt atgc 194
<210> 602
   <211> 785
   <212> DNA
   <213> Diabrotica virgifera
<220>
   <221> misc_feature
   <222> (1)..(785)
   <223> RNAi target sequence DV-TEF2-FRAG1
<400> 602
   tctggtaaca tctgtggttt ggttggtgtt gatcaattct tggttaagac tggtaccatt      60
   accactttca aggatgccca caacttgaag gtcatgaaat tcagtgtatc tcctgtagta     120
   cgtgttgctg ttgaacctaa gaaccctgct gatttgccaa aacttgtaga aggtttgaaa     180
   cgtttgacca aatccgatcc tatggtccaa tgtatcattg aagaatctgg tgagcacatc     240
   attgctggtg ccggagaatt gcatcttgaa atttgcctta aggatcttga agaagatcat     300
   gcctgcattc ccattaagaa atctgaccct gttgtatcct accgtgagac agtaagtgaa     360
   gagtctgatc agatgtgcct ctcaaaatca cccaacaaac acaacagatt gttcatgaaa     420
   gcttgtccta tgcctgatgg acttgctgaa gatattgatg atggtaaagt taacccacgt     480
   gatgacttca aagtccgtgc ccgttatctt actgagaaat atgactatga tataactgaa     540
   gcccgtaaga tctggtgctt tggacctgat ggtactggac ccaacatcct tgttgattgt     600
   actaagggag tgcaatatct taatgaaatc aaagactctg tggtagctgg tttccaatgg     660
   gcaaccaaag aaggtgtctt gtctgaagaa aacttgagag gtgttagatt caacatttat     720
   gatgttacct tacacgctga tgctatccat cgtggaggtg gtcaaatcat tccaacaacc     780
   cgtag                                                                 785
<210> 603
   <211> 144
   <212> DNA
   <213> Diabrotica virgifera
<220>
   <221> misc_feature
   <222> (1)..(144)
   <223> RNAi target sequence DV-SRP54-FRAG1
<400> 603
   tgggaggcat caaaggtctc ttcaaaggtg gcgatatggc taaaaacgtc aaccacaacc      60
   agatggcgaa acttaatcaa caaatggcca aaatgatgga ccctagagtc ctccaacaaa     120
   tgggcggcat ggcaggtttg caaa                                            144
<210> 604
   <211> 364
   <212> DNA
   <213> Diabrotica virgifera
<220>
   <221> misc_feature
   <222> (1)..(364)
   <223> RNAi target sequence DV-SRP54-FRAG2
<400> 604
   acgagttgga caacagggat ggcgcaaaat tgtttactaa acaagttggt agagtggtca       60
   gggtagcaca aggatctggt gtaacagaaa gagaagtaaa agatctcatt acgcagtaca      120
   caaaattcgc agcggtggta aagaaaatgg gaggcatcaa aggtctcttc aaaggtggcg      180
   atatggctaa aaacgtcaac cacaaccaga tggcgaaact taatcaacaa atggccaaaa      240
   tgatggaccc tagagtcctc caacaaatgg gcggcatggc aggtttgcaa aatatgatga      300
   gacaactcca agcaggcgca gctggtggtt tagggggctt aggtaatctt atggggggct      360
   ttgg                                                                   364
<210> 605
   <211> 226
   <212> DNA
   <213> Diabrotica virgifera
<220>
   <221> misc_feature
   <222> (1)..(226)
   <223> RNAi target sequence DV-SRP54-FRAG3
<400> 605
   gtagcacaag gatctggtgt aacagaaaga gaagtaaaag atctcattac gcagtacaca       60
   aaattcgcag cggtggtaaa gaaaatggga ggcatcaaag gtctcttcaa aggtggcgat      120
   atggctaaaa acgtcaacca caaccagatg gcgaaactta atcaacaaat ggccaaaatg      180
   atggacccta gagtcctcca acaaatgggc ggcatggcag gtttgc                     226
<210> 606
   <211> 163
   <212> DNA
   <213> Diabrotica virgifera
<220>
   <221> misc_feature
   <222> (1)..(163)
   <223> RNAi target sequence DV-SRP54-FRAG4
<400> 606
   ttatcagaaa aagaactgga aatcatgttt ggtatgtgca gatacattca gagctggtgc       60
   ctacgatcag gtaaaacaga actgtacaaa agctaggata cccttttatg gaagttacac      120
   agaagttgat cctgtagtca tagcccagga tggtgttgat atg                        163
<210> 607
   <211> 171
   <212> DNA
   <213> Diabrotica virgifera
<220>
   <221> misc_feature
   <222> (1)..(171)
   <223> RNAi target sequence DV-TIF3-FRAG1
<400> 607
   gttgagtgct gtttctggag aagatgccca agaccgttca gatcgtacaa ttcttactcc 60
   gtgggttaag ttcctttggg aatcttactg tcaatgtttg gaacttttgc gtacaaatgc 120
   acatgttgag aacttgtatc atgatatagc caggatggct ttccagtttt g 171
<210> 608
   <211> 232
   <212> DNA
   <213> Diabrotica virgifera
<220>
   <221> misc_feature
   <222> (1)..(232)
   <223> RNAi target sequence DV-TIF3-FRAG2
<400> 608
   atcgtgggga tcgcagggac gatagggaga ggagagacga cagggataac agggatagga 60
   gagacgacag agatcgtaga gatgataggg ataggagaga cgacagggag cgtagagatg 120
   atagagagag aagagatgac cgtggcggaa gagatgataa ccgggaccgc cgagacgatc 180
   gtgatcgtag agacgacagg gaccgaagag gcggggacga aggtacttgg cg 232
<210> 609
   <211> 166
   <212> DNA
   <213> Diabrotica virgifera
<220>
   <221> misc_feature
   <222> (1)..(166)
   <223> RNAi target sequence DV-TIF3-FRAG3
<400> 609
   ggcaaccgcg acagcggaag atttggcgac agagacagag aaggtggtgt cagattcacg 60
   agagataatc gggataatcg tggggatcgc agggacgata gggagaggag agacgacagg 120
   gataacaggg ataggagaga cgacagagat cgtagagatg ataggg 166
<210> 610
   <211> 239
   <212> DNA
   <213> Diabrotica virgifera
<220>
   <221> misc_feature
   <222> (1)..(239)
   <223> RNAi target sequence DV-TIF3-FRAG4
<400> 610
   gcaagtccac aactacaaga tttgtacaat tggctggaag tggagttcca tcctttacta 60
   ttgtgtcaga gagtgcatga ggtaatcaaa tctctcgaat ctgaagaaaa tgcagccctc 120
   caacaatatg tgccagcttt acaagatgta actctggtgc gtctagtgag acagatagca 180
   caagtctatc agaccataga atttgctagg ttgatccaga ttgccaaatt tactacacc 239
<210> 611
   <211> 227
   <212> DNA
   <213> Diabrotica virgifera
<220>
   <221> misc_feature
   <222> (1)..(227)
   <223> RNAi target sequence DV-TIF3-FRAG5
<400> 611
   ggttgattgt gtgagacaca atgatatgca aattcgtatt gatcatggca aagactgtat 60
<210> 612
   <211> 224
   <212> DNA
   <213> Diabrotica virgifera
<220>
   <221> misc_feature
   <222> (1)..(224)
   <223> RNAi target sequence DV-TIF3-FRAG6
<400> 612
   tgttataaga gggtatttgc ggatggcaga agaaagaaca gagaatgcta gagaacagtc 60
   agcacaagca gttattgata tcgatgattt agataatttg gctacacctg aaagtatatt 120
   gttgagtgct gtttctggag aagatgccca agaccgttca gatcgtacaa ttcttactcc 180
   gtgggttaag ttcctttggg aatcttactg tcaatgtttg gaac 224
<210> 613
   <211> 154
   <212> DNA
   <213> Diabrotica virgifera
<220>
   <221> misc_feature
   <222> (1)..(154)
   <223> RNAi target sequence DV-TIF3-FRAG7
<400> 613
   aagactgtat ccactttgga atggacctga gtgaaagcca aagagaggac aaaccagagg      60
   gacccacttt gcaagttatg cctagtgagc aagtcagaaa tcaacttatt aacatgtcta     120
   ctgtattgaa tcaagccata caagtcatta atcc                                 154
<210> 614
   <211> 742
   <212> DNA
   <213> Diabrotica virgifera
<220>
   <221> misc_feature
   <222> (1)..(742)
   <223> RNAi target sequence DV-TIF3-FRAG8
<400> 614
   ctggcaagtc cacaactaca agatttgtac aattggctgg aagtggagtt ccatccttta      60
   ctattgtgtc agagagtgca tgaggtaatc aaatctctcg aatctgaaga aaatgcagcc     120
   ctccaacaat atgtgccagc tttacaagat gtaactctgg tgcgtctagt gagacagata     180
   gcacaagtct atcagaccat agaatttgct aggttgatcc agattgccaa atttactaca     240
   ccttttcatc tggagagact tttggttgat tgtgtgagac acaatgatat gcaaattcgt     300
   attgatcatg gcaaagactg tatccacttt ggaatggacc tgagtgaaag ccaaagagag     360
   gacaaaccag agggacccac tttgcaagtt atgcctagtg agcaagtcag aaatcaactt     420
   attaacatgt ctactgtatt gaatcaagcc atacaagtca ttaatcctaa taagaagaag     480
   cttgaacgcg aaaaacttag agctgttatg gttcagaatt atcacgaaac aaaggttaga     540
   gaacaccaga aaattttaca aagacacaag attattgagg acagaaagga atatattgag     600
   cgtttgaata cagtcaggga agaagaggag cagaaaagat tggaagaaat gcaacggcag     660
   cacttactgg ccgaacaaaa acgattggac caagaaagag aagaaagaga gcggaaacgt     720
   gctctaaatg aaatccagca gg                                              742
<210> 615
   <211> 619
   <212> DNA
   <213> Diabrotica virgifera
<220>
   <221> misc_feature
   <222> (1)..(619)
   <223> RNAi target sequence DV-TIF3-FRAG9
<400> 615
   ccaacaatat gtgccagctt tacaagatgt aactctggtg cgtctagtga gacagatagc      60
   acaagtctat cagaccatag aatttgctag gttgatccag attgccaaat ttactacacc     120
   ttttcatctg gagagacttt tggttgattg tgtgagacac aatgatatgc aaattcgtat     180
   tgatcatggc aaagactgta tccactttgg aatggacctg agtgaaagcc aaagagagga     240
   caaaccagag ggacccactt tgcaagttat gcctagtgag caagtcagaa atcaacttat     300
   taacatgtct actgtattga atcaagccat acaagtcatt aatcctaata agaagaagct     360
   tgaacgcgaa aaacttagag ctgttatggt tcagaattat cacgaaacaa aggttagaga     420
   acaccagaaa attttacaaa gacacaagat tattgaggac agaaaggaat atattgagcg     480
   tttgaataca gtcagggaag aagaggagca gaaaagattg gaagaaatgc aacggcagca     540
   cttactggcc gaacaaaaac gattggacca agaaagagaa gaaagagagc ggaaacgtgc     600
   tctaaatgaa atccagcag                                                  619
<210> 616
   <211> 713
   <212> DNA
   <213> Diabrotica virgifera
<220>
   <221> misc_feature
   <222> (1)..(713)
   <223> RNAi target sequence DV-EIF-4A-FRAG2
<400> 616
   cgtctagcac atcgtttggc atggtagccg aaagtaaaat tacctgtacg tcagtgttca 60
   acattttgaa cacgtcatga atttgatctt taaaacccct tgacaacatt tcatcggctt 120
   catccaaaac aaacattttt atatgagtcg gccttaaggc tctccttgtt atcatatcat 180
   aaactctacc aggtgtacca actacaacat gtacgccagt ttcaagtttt cttatgtctt 240
   ctcgtacatt ggtaccacca atacatgcat gacactgcgc tgacatgaaa tctcctaggg 300
   caattactac tttttggatc tgttgagcca attcacgagt tggtgccaat ataagagctt 360
   ggcactccct cactgacgtg tcaatttgtt gtaaaataga gatggagaat gttgcagtct 420
   taccagtacc tgactgagcc tgagcaataa catcgtgtcc ctttacacaa ggaatgatgg 480
   ctctctgttg gatagcagaa ggtttttcaa aaccataggc ataaattcct cttaaaagtt 540
   cctcttttag gttcatgtcg tcgaaattat cgaccacctc ttcccagttg gattcaataa 600
   tgccatcagg ctccatacct ggtgggccgt catagccttg gggctggctg tcttcattag 660
   ctgggccatt cttgtcccac tcttcacgac gatctggcat agacatgtct act 713
<210> 617
   <211> 200
   <212> DNA
   <213> Diabrotica virgifera
<220>
   <221> misc_feature
   <222> (1)..(200)
   <223> RNAi target sequence DV-EIF-4A-FRAG3
<400> 617
   tctatgccag atcgtcgtga agagtgggac aagaatggcc cagctaatga agacagccag 60
   ccccaaggct atgacggccc accaggtatg gagcctgatg gcattattga atccaactgg 120
   gaagaggtgg tcgataattt cgacgacatg aacctaaaag aggaactttt aagaggaatt 180
   tatgcctatg gttttgaaaa 200
<210> 618
   <211> 270
   <212> DNA
   <213> Diabrotica virgifera
<220>
   <221> misc_feature
   <222> (1)..(270)
   <223> RNAi target sequence DV-PAT3-SS1
<400> 618
   tcaacattga taaacacata ggaatggcat tttcaggctt aatagctgat gcaaggcaaa 60
   tcgttgagat tgctagaaaa gaagcatcaa attatagaca tcaatatggt tcaaatattc 120
   ctcttaaata cctaaatgat agagtaagca tgtacatgca tgcatacact ttatacagtg 180
   ctgttagacc atttggttgc agtgtcatct tggccagtta tgaagatagt gacccatcta 240
   tgtatctgat tgatccatct ggagttagct 270
<210> 619
   <211> 322
   <212> DNA
   <213> Diabrotica virgifera
<220>
   <221> misc_feature
   <222> (1)..(322)
   <223> RNAi target sequence DV-PAT3-FRAG5
<400> 619
   ggggagtcgt caacatcaat ttcaagtttc aagaaaaagc aaatcactac gacttgccgg 60
   attttgtagt agtgttaatt ttgtattaaa aaatcaaaat gagttctatt ggaactgggt 120
   acgatttatc agcttcccaa ttctctcctg atggaagagt atttcaagtt gaatatgcaa 180
   tgaaagcagt tgaaaatagt ggcaccgtaa taggcctccg aggtacagat ggcattgtat 240
   tggctgctga aaagctcatt atgtcaaaat tgcatgaacc aagtacaaat aaacgaattt 300
   tcaacattga taaacacata gg 322
<210> 620
   <211> 322
   <212> DNA
   <213> Diabrotica virgifera
<220>
   <221> misc_feature
   <222> (1)..(322)
   <223> RNAi target sequence DV-PAT3-FRAG6
<400> 620
   aatggcattt tcaggcttaa tagctgatgc aaggcaaatc gttgagattg ctagaaaaga 60
   agcatcaaat tatagacatc aatatggttc aaatattcct cttaaatacc taaatgatag 120
   agtaagcatg tacatgcatg catacacttt atacagtgct gttagaccat ttggttgcag 180
   tgtcatcttg gccagttatg aagatagtga cccatctatg tatctgattg atccatctgg 240
   agttagctat ggatactttg gatgtgctac aggtaaagca aaacagtctg caaagactga 300
   aatagaaaaa ttgaagatgg gg 322
<210> 621
   <211> 161
   <212> DNA
   <213> Diabrotica virgifera
<220>
   <221> misc_feature
   <222> (1)..(161)
   <223> RNAi target sequence DV-PAT3-FRAG7
<400> 621
   ggggagtcgt caacatcaat ttcaagtttc aagaaaaagc aaatcactac gacttgccgg 60
   attttgtagt agtgttaatt ttgtattaaa aaatcaaaat gagttctatt ggaactgggt 120
   acgatttatc agcttcccaa ttctctcctg atggaagagt a 161
<210> 622
   <211> 161
   <212> DNA
   <213> Diabrotica virgifera
<220>
   <221> misc_feature
   <222> (1)..(161)
   <223> RNAi target sequence DV-PAT3-FRAG8
<400> 622
   tttcaagttg aatatgcaat gaaagcagtt gaaaatagtg gcaccgtaat aggcctccga 60
   ggtacagatg gcattgtatt ggctgctgaa aagctcatta tgtcaaaatt gcatgaacca 120
   agtacaaata aacgaatttt caacattgat aaacacatag g 161
<210> 623
   <211> 161
   <212> DNA
   <213> Diabrotica virgifera
<220>
   <221> misc_feature
   <222> (1)..(161)
   <223> RNAi target sequence DV-PAT3-FRAG9
<400> 623
   aatggcattt tcaggcttaa tagctgatgc aaggcaaatc gttgagattg ctagaaaaga 60
   agcatcaaat tatagacatc aatatggttc aaatattcct cttaaatacc taaatgatag 120
   agtaagcatg tacatgcatg catacacttt atacagtgct g 161
<210> 624
   <211> 161
   <212> DNA
   <213> Diabrotica virgifera
<220>
   <221> misc_feature
   <222> (1)..(161)
   <223> RNAi target sequence DV-PAT3-FRAG10
<400> 624
   ttagaccatt tggttgcagt gtcatcttgg ccagttatga agatagtgac ccatctatgt 60
   atctgattga tccatctgga gttagctatg gatactttgg atgtgctaca ggtaaagcaa 120
   aacagtctgc aaagactgaa atagaaaaat tgaagatggg g 161
<210> 625
   <211> 162
   <212> DNA
   <213> Diabrotica virgifera
<220>
   <221> misc_feature
   <222> (1)..(162)
   <223> RNAi target sequence DV-PAT3-FRAG11
<400> 625
   ttgtattaaa aaatcaaaat gagttctatt ggaactgggt acgatttatc agcttcccaa 60
   ttctctcctg atggaagagt atttcaagtt gaatatgcaa tgaaagcagt tgaaaatagt 120
   ggcaccgtaa taggcctccg aggtacagat ggcattgtat tg 162
<210> 626
   <211> 160
   <212> DNA
   <213> Diabrotica virgifera
<220>
   <221> misc_feature
   <222> (1)..(160)
   <223> RNAi target sequence DV-PAT3-FRAG12
<400> 626
   gctgctgaaa agctcattat gtcaaaattg catgaaccaa gtacaaataa acgaattttc 60
   aacattgata aacacatagg aatggcattt tcaggcttaa tagctgatgc aaggcaaatc 120
   gttgagattg ctagaaaaga agcatcaaat tatagacatc 160
<210> 627
   <211> 161
   <212> DNA
   <213> Diabrotica virgifera
<220>
   <221> misc_feature
   <222> (1)..(161)
   <223> RNAi target sequence DV-PAT3-FRAG13
<400> 627
   aatatggttc aaatattcct cttaaatacc taaatgatag agtaagcatg tacatgcatg        60
   catacacttt atacagtgct gttagaccat ttggttgcag tgtcatcttg gccagttatg       120
   aagatagtga cccatctatg tatctgattg atccatctgg a                           161
<210> 628
   <211> 80
   <212> DNA
   <213> Diabrotica virgifera
<220>
   <221> misc_feature
   <222> (1)..(80)
   <223> RNAi target sequence DV-PAT3-FRAG14
<400> 628
   ggggagtcgt caacatcaat ttcaagtttc aagaaaaagc aaatcactac gacttgccgg        60
   attttgtagt agtgttaatt                                                    80
<210> 629
   <211> 81
   <212> DNA
   <213> Diabrotica virgifera
<220>
   <221> misc_feature
   <222> (1)..(81)
   <223> RNAi target sequence DV-PAT3-FRAG15
<400> 629
   ttgtattaaa aaatcaaaat gagttctatt ggaactgggt acgatttatc agcttcccaa        60
   ttctctcctg atggaagagt a                                                  81
<210> 630
   <211> 80
   <212> DNA
   <213> Diabrotica virgifera
<220>
   <221> misc_feature
   <222> (1)..(80)
   <223> RNAi target sequence DV-PAT3-FRAG16
<400> 630
   tttcaagttg aatatgcaat gaaagcagtt gaaaatagtg gcaccgtaat aggcctccga        60
   ggtacagatg gcattgtatt                                                    80
<210> 631
   <211> 81
   <212> DNA
   <213> Diabrotica virgifera
<220>
   <221> misc_feature
   <222> (1)..(81)
   <223> RNAi target sequence DV-PAT3-FRAG17
<400> 631
   ggctgctgaa aagctcatta tgtcaaaatt gcatgaacca agtacaaata aacgaatttt 60
   caacattgat aaacacatag g 81
<210> 632
   <211> 80
   <212> DNA
   <213> Diabrotica virgifera
<220>
   <221> misc_feature
   <222> (1)..(80)
   <223> RNAi target sequence DV-PAT3-FRAG18
<400> 632
   aatggcattt tcaggcttaa tagctgatgc aaggcaaatc gttgagattg ctagaaaaga 60
   agcatcaaat tatagacatc 80
<210> 633
   <211> 81
   <212> DNA
   <213> Diabrotica virgifera
<220>
   <221> misc_feature
   <222> (1)..(81)
   <223> RNAi target sequence DV-PAT3-FRAG19
<400> 633
   aatatggttc aaatattcct cttaaatacc taaatgatag agtaagcatg tacatgcatg 60
   catacacttt atacagtgct g 81
<210> 634
   <211> 80
   <212> DNA
   <213> Diabrotica virgifera
<220>
   <221> misc_feature
   <222> (1)..(80)
   <223> RNAi target sequence DV-PAT3-FRAG20
<400> 634
   ttagaccatt tggttgcagt gtcatcttgg ccagttatga agatagtgac ccatctatgt 60
   atctgattga tccatctgga 80
<210> 635
   <211> 81
   <212> DNA
   <213> Diabrotica virgifera
<220>
   <221> misc_feature
   <222> (1)..(81)
   <223> RNAi target sequence DV-PAT3-FRAG21
<400> 635
   gttagctatg gatactttgg atgtgctaca ggtaaagcaa aacagtctgc aaagactgaa 60
   atagaaaaat tgaagatggg g 81
<210> 636
   <211> 644
   <212> DNA
   <213> Diabrotica virgifera
<220>
   <221> misc_feature
   <222> (1)..(644)
   <223> RNAi target sequence DV-PAT3-FIS (TR)
<400> 636
   ggggagtcgt caacatcaat ttcaagtttc aagaaaaagc aaatcactac gacttgccgg 60
   attttgtagt agtgttaatt ttgtattaaa aaatcaaaat gagttctatt ggaactgggt 120
   acgatttatc agcttcccaa ttctctcctg atggaagagt atttcaagtt gaatatgcaa 180
   tgaaagcagt tgaaaatagt ggcaccgtaa taggcctccg aggtacagat ggcattgtat 240
   tggctgctga aaagctcatt atgtcaaaat tgcatgaacc aagtacaaat aaacgaattt 300
   tcaacattga taaacacata ggaatggcat tttcaggctt aatagctgat gcaaggcaaa 360
   tcgttgagat tgctagaaaa gaagcatcaa attatagaca tcaatatggt tcaaatattc 420
   ctcttaaata cctaaatgat agagtaagca tgtacatgca tgcatacact ttatacagtg 480
   ctgttagacc atttggttgc agtgtcatct tggccagtta tgaagatagt gacccatcta 540
   tgtatctgat tgatccatct ggagttagct atggatactt tggatgtgct acaggtaaag 600
   caaaacagtc tgcaaagact gaaatagaaa aattgaagat gggg 644
<210> 637
   <211> 367
   <212> DNA
   <213> Diabrotica virgifera
<220>
   <221> misc_feature
   <222> (1)..(367)
   <223> RNAi target sequence DV-PAT3-FRAG22
<400> 637
   aatctaacat gcaaagaact tgttaaagaa gcagccaaaa tcatttattt ggtccatgat 60
   gagctgaagg ataagaattt tgaactggaa ctttcatggg tatgcaaaga tacgaatggt 120
   ttacatacca aagtgcctga atcagtgttt gctgatgcag aaaaagctgc caaacaaatg 180
   gaagcagatt cagaatcaga tacagaagat atgtaataac tacatttagt ttttaatatt 240
   tcgctgatgg tggctgttct tacaatattt cgtgtgttat gttcatatat tatgtaatac 300
   tgtgagaatt tccatttcaa ggataggttt ataacttttt tttctaataa atacataact 360
   ttatgtc 367
<210> 638
   <211> 182
   <212> DNA
   <213> Diabrotica virgifera
<220>
   <221> misc_feature
   <222> (1)..(182)
   <223> RNAi target sequence DV-PAT3-FRAG23
<400> 638
   aatctaacat gcaaagaact tgttaaagaa gcagccaaaa tcatttattt ggtccatgat 60
   gagctgaagg ataagaattt tgaactggaa ctttcatggg tatgcaaaga tacgaatggt 120
   ttacatacca aagtgcctga atcagtgttt gctgatgcag aaaaagctgc caaacaaatg 180
   ga 182
<210> 639
   <211> 185
   <212> DNA
   <213> Diabrotica virgifera
<220>
   <221> misc_feature
   <222> (1)..(185)
   <223> RNAi target sequence DV-PAT3-FRAG24
<400> 639
   agcagattca gaatcagata cagaagatat gtaataacta catttagttt ttaatatttc 60
   gctgatggtg gctgttctta caatatttcg tgtgttatgt tcatatatta tgtaatactg 120
   tgagaatttc catttcaagg ataggtttat aacttttttt tctaataaat acataacttt 180
   atgtc 185
<210> 640
   <211> 256
   <212> DNA
   <213> Diabrotica virgifera
<220>
   <221> misc_feature
   <222> (1)..(256)
   <223> RNAi target sequence DV-PAT5-FRAG1
<400> 640
   atcaagaaca tttgttcagt ttgacgcaaa ggcgatagga tcaggatccg agggtgcaca 60
   gcaaagttta caggaggcat atcatcgcag tatgaccctg gaagaagcta ccacaactgc 120
   tttaacaatt ctaaaacaag ttatggaaga aaagttgaat tcttcaaatg ttgaggttat 180
   gactatgact ccaaaagata tgttccacat gttcacaaag gaacaagtgg aagaggtaat 240
   tgctaaattg tcttaa 256
<210> 641
   <211> 200
   <212> DNA
   <213> Diabrotica virgifera
<220>
   <221> misc_feature
   <222> (1)..(200)
   <223> RNAi target sequence DV-PAT6-FRAG1
<400> 641
   aatcatgtct agaggaagca gtgcaggttt tgaccgacac ataacaattt tctcgcccga        60
   aggccgactc tatcaagtag agtatgcttt taaagccatt aaccaagccg gccccacttc       120
   ggtagcagtc cgaggagtag atgctgcggc gtgtgtgacc cagagaaaga tcccggataa       180
   gctgattgat cccaacacaa                                                   200
<210> 642
   <211> 216
   <212> DNA
   <213> Diabrotica virgifera
<220>
   <221> misc_feature
   <222> (1)..(216)
   <223> RNAi target sequence DV-PAT6-FRAG2
<400> 642
   cagaacacac tggatgtgtg atgactggca tgattgctga cagcaagtcc caggtgcaga        60
   gagctagata tgaggctgcc gagttcaaat ataagtttgg atatgagatg ccaatcgatg       120
   ccttgtgtag gagagtatcg gatatttccc aggtttatac gcagaatgct gagatgagac       180
   ctttgggttg ctccatgctt ctgataggat atgacc                                 216
<210> 643
   <211> 200
   <212> DNA
   <213> Diabrotica virgifera
<220>
   <221> misc_feature
   <222> (1)..(200)
   <223> RNAi target sequence DV-PAT6-FRAG3
<400> 643
   cacatctgtt ccagttaaca gaacacactg gatgtgtgat gactggcatg attgctgaca        60
   gcaagtccca ggtgcagaga gctagatatg aggctgccga gttcaaatat aagtttggat       120
   atgagatgcc aatcgatgcc ttgtgtagga gagtatcgga tatttcccag gtttatacgc       180
   agaatgctga gatgagacct                                                   200
<210> 644
   <211> 195
   <212> DNA
   <213> Diabrotica virgifera
<220>
   <221> misc_feature
   <222> (1)..(195)
   <223> RNAi target sequence DV-PAT6-FRAG4
<400> 644
   aggagagtat cggatatttc ccaggtttat acgcagaatg ctgagatgag acctttgggt        60
   tgctccatgc ttctgatagg atatgaccaa gaaatgggac catgtgtcca caaagctgac       120
   cctgctggct attactgtgg ctacagagca gtaagtgtag gatccaaaca aactgaagcc       180
   aacagctacc tagag                                                        195
<210> 645
   <211> 199
   <212> DNA
   <213> Diabrotica virgifera
<220>
   <221> misc_feature
   <222> (1)..(199)
   <223> RNAi target sequence DV-PAT6-FRAG5
<400> 645
   aagaagctaa agaagaaaac tgatctgtcc aaagatgaag tcatccaatt ggcaatcagt        60
   tgtctttcaa gtgttttatc tgtagatttt aaaccaacag aagtcgaagt tggggttgtt       120
   actaaggatg agcccaagtt taggaaattg acggaacaag aaatagattt ccatctgaca       180
   gctattgctg agagggatt                                                    199
<210> 646
   <211> 165
   <212> DNA
   <213> Diabrotica virgifera
<220>
   <221> misc_feature
   <222> (1)..(165)
   <223> RNAi target sequence DV-PAT6-FRAG6
<400> 646
   aggagagtat cggatatttc ccaggtttat acgcagaatg ctgagatgag acctttgggt        60
   tgctccatgc ttctgatagg atatgaccaa gaaatgggac catgtgtcca caaagctgac       120
   cctgctggct attactgtgg ctacagagca gtaagtgtag gatcc                       165
<210> 647
   <211> 157
   <212> DNA
   <213> Diabrotica virgifera
<220>
   <221> misc_feature
   <222> (1)..(157)
   <223> RNAi target sequence DV-PAT6-FRAG7
<400> 647
   gagaagaagc taaagaagaa aactgatctg tccaaagatg aagtcatcca attggcaatc 60
   agttgtcttt caagtgtttt atctgtagat tttaaaccaa cagaagtcga agttggggtt 120
   gttactaagg atgagcccaa gtttaggaaa ttgacgg 157
<210> 648
   <211> 196
   <212> DNA
   <213> Diabrotica virgifera
<220>
   <221> misc_feature
   <222> (1)..(196)
   <223> RNAi target sequence DV-BETA2-FRAG1
<400> 648
   gcactcagaa gcagaagtgc ataccatgta aaccttcttc tagctggata tgatgacaaa 60
   gaaggtcctc agctatatta catggactac ctagcatcca tagcaagcgt tgattatgct 120
   gctcatggtt atggaggatt cttttcactg tcaattatgg atcgtaatta tcggcagaac 180
   cttagtaaag aagaag 196
<210> 649
   <211> 187
   <212> DNA
   <213> Diabrotica virgifera
<220>
   <221> misc_feature
   <222> (1)..(187)
   <223> RNAi target sequence DV-BETA2-FRAG2
<400> 649
   tttcactgtc aattatggat cgtaattatc ggcagaacct tagtaaagaa gaaggttacg 60
   aacttttgaa aaaatgtgtg aaagaagttc agaaaagatt gatcatcaat ttgcctaact 120
   ttaaggtgca gtgtgttgat aaaaatggca tccaggatat gcctaatatc actgctaagg 180
   atttggt 187
<210> 650
   <211> 155
   <212> DNA
   <213> Diabrotica virgifera
<220>
   <221> misc_feature
   <222> (1)..(155)
   <223> RNAi target sequence DV-BETA2-FRAG3
<400> 650
   cagatgcact cagaagcaga agtgcatacc atgtaaacct tcttctagct ggatatgatg       60
   acaaagaagg tcctcagcta tattacatgg actacctagc atccatagca agcgttgatt      120
   atgctgctca tggttatgga ggattctttt cactg                                 155
<210> 651
   <211> 174
   <212> DNA
   <213> Diabrotica virgifera
<220>
   <221> misc_feature
   <222> (1)..(174)
   <223> RNAi target sequence DV-BETA3-FRAG1
<400> 651
   tgtgaagcac tttgggagcc taacctagga cctgaggatc tttttgaaac catctctcaa       60
   gctctcatca atgcctttga cagagatgcc atttctggtt ggggagctac agtgtacatc      120
   atagaaaaaa ataaggtgac tgtaaaaaat ttgaagacac gtatggacta gtta            174
<210> 652
   <211> 203
   <212> DNA
   <213> Diabrotica virgifera
<220>
   <221> misc_feature
   <222> (1)..(203)
   <223> RNAi target sequence DV-PROTB-FRAG1
<400> 652
   taccagagaa caaaacaaac ttttcccact atcaggcact actgttttgg gttgtgcagg       60
   atgttggtgt gacactctaa cattaaccag aatccttaaa tctcgcatgc agatgtacca      120
   acaagagcat aacaaaacaa tgtctacaac tgcatgtgcc cagatgttgt caaccatgct      180
   ctactacaag agattctttc ctt                                              203
<210> 653
   <211> 291
   <212> DNA
   <213> Diabrotica virgifera
<220>
   <221> misc_feature
   <222> (1)..(291)
   <223> RNAi target sequence DV-PBT6.2-FRAG1
<400> 653
   tatagtgtaa aactgatttt tttattttaa tatataccaa aacataccaa agcttaaaaa      60
   ataatcaatt aaccttcata aaatttgggc aattcatttc ctaaaacaac tcttcgttca     120
   ataccagctt cagtgatgat ccccagtctg actacacctc ctgaagaacc atctcttgac     180
   atggcaagtg ccaatgtatt tgtgacaaac ttcacacatt cttccttgct catattgggc     240
   ttgaagttgg catctacgta accataaaca taactggaac ctgatcctcc g              291
<210> 654
   <211> 277
   <212> DNA
   <213> Diabrotica virgifera
<220>
   <221> misc_feature
   <222> (1)..(277)
   <223> RNAi target sequence DV-PBT6.2-FRAG2
<400> 654
   attgacactt cttgtctaac acacatccca ccaattggta tggaatatac ttgtccgcct      60
   ttctttttat cccaacctgc taccagtata ccagccatta gcgaatctct ataattgtag     120
   caaagttctt ggaaaatggc ggcacctact tgtactttgg gttcttcacc aagttccata     180
   ccatgaaaat taagatgata agcaacaatg tctgcaattg cttgtgtatc tgctgcagat     240
   cctgaacgac aacagtatat atggtcagtg actttgg                              277
<210> 655
   <211> 257
   <212> DNA
   <213> Diabrotica virgifera
<220>
   <221> misc_feature
   <222> (1)..(257)
   <223> RNAi target sequence DV-PBT6.2-FRAG3
<400> 655
   tgagtttgtc tgctacccgg tttgcaatgt aggccccagt agttgtgcga gaatctgctc      60
   ctataacaac gcctccatca aactccgcgg ccataataga ggttcctgta ctgtgagcgg     120
   catctctcca atcattagga ccagtcattg caccatactc agtcataaga ggcatttttt     180
   acaagtttaa tgaaaagaat acaagcttag aaaaattaca cttgtaatcc tgcaatgcaa     240
   tattttgcca gtccccc                                                    257
<210> 656
   <211> 273
   <212> DNA
   <213> Diabrotica virgifera
<220>
   <221> misc_feature
   <222> (1)..(273)
   <223> RNAi target sequence DV-PBT6.2-FRAG4
<400> 656
   caggaacctc tattatggcc gcggagtttg atggaggcgt tgttatagga gcagattctc 60
   gcacaactac tggggcctac attgcaaacc gggtagcaga caaactcacc aaagtcactg 120
   accatatata ctgttgtcgt tcaggatctg cagcagatac acaagcaatt gcagacattg 180
   ttgcttatca tcttaatttt catggtatgg aacttggtga agaacccaaa gtacaagtag 240
   gtgccgccat tttccaagaa ctttgctaca att 273
<210> 657
   <211> 219
   <212> DNA
   <213> Diabrotica virgifera
<220>
   <221> misc_feature
   <222> (1)..(219)
   <223> RNAi target sequence DV-PBT6.2-FRAG5
<400> 657
   ggctggtata ctggtagcag gttgggataa aaagaaaggc ggacaagtat attccatacc 60
   aattggtggg atgtgtgtta gacaagaagt gtcaatcgga ggatcaggtt ccagttatgt 120
   ttatggttac gtagatgcca acttcaagcc caatatgagc aaggaagaat gtgtgaagtt 180
   tgtcacaaat acattggcac ttgccatgtc aagagatgg 219
<210> 658
   <211> 151
   <212> DNA
   <213> Diabrotica virgifera
<220>
   <221> misc_feature
   <222> (1)..(151)
   <223> RNAi target sequence DV-PBT6.2-FRAG6
<400> 658
   ttagacaaga agtgtcaatc ggaggatcag gttccagtta tgtttatggt tacgtagatg 60
   ccaacttcaa gcccaatatg agcaaggaag aatgtgtgaa gtttgtcaca aatacattgg 120
   cacttgccat gtcaagagat ggttcttcag g 151
<210> 659
   <211> 229
   <212> DNA
   <213> Diabrotica virgifera
<220>
   <221> misc_feature
   <222> (1)..(229)
   <223> RNAi target sequence DV-BETA7-FRAG1
<400> 659
   ggctacagtt ttgtgtccag aaataccaac gcctggtttt tcattcgaaa attgtaaaag 60
   aaatgcactt ctggaaaata agggccttgc tcttccaaaa gccactaaaa caggtactac 120
   gatcgtagga atagtctaca aagatggaat tattcttgga gcagatacta gagctacaga 180
   agacaccaca gttgcagaca agaactgtga aaagattcac ttccttgca 229
<210> 660
   <211> 285
   <212> DNA
   <213> Diabrotica virgifera
<220>
   <221> misc_feature
   <222> (1)..(285)
   <223> RNAi target sequence DV-BETA7-FRAG2
<400> 660
   gctaaattag aactccacag attatacaca aaccgagtta ctagagtgtg tacagcaaac 60
   caattgttaa aacaatatct atttcgctac cagggatata ttggggctgc tttaatcctg 120
   ggtggtgttg atattgaagg accacatcta tatatgattt atccacatgg atccagtgtt 180
   gatcttcctt atgctacaat gggttctggt tccttagcag ccatatctgt ctttgaatcc 240
   cgctggaagc ctcaaatgga agaggaagat ggtgtccaac tagta 285
<210> 661
   <211> 188
   <212> DNA
   <213> Diabrotica virgifera
<220>
   <221> misc_feature
   <222> (1)..(188)
   <223> RNAi target sequence DV-BETA7-FRAG3
<400> 661
   agacatctgt gtaataagaa agggttcagt agactacaaa cgaacctatg acgaggctaa 60
   caaaaaggga gttaaacaag gatcttatag atatcaaaga ggaaccactg cagtgcttag 120
   tacccaggtg gtggatgttg aggaagtctc agtgattccc attgaagctg aagaaatgga 180
   tgttgctt 188
<210> 662
   <211> 150
   <212> DNA
   <213> Diabrotica virgifera
<220>
   <221> misc_feature
   <222> (1)..(150)
   <223> RNAi target sequence DV-BETA7-FRAG4
<400> 662
   aaggaccaca tctatatatg atttatccac atggatccag tgttgatctt ccttatgcta 60
   caatgggttc tggttcctta gcagccatat ctgtctttga atcccgctgg aagcctcaaa 120
   tggaagagga agatggtgtc caactagtaa 150
<210> 663
   <211> 168
   <212> DNA
   <213> Diabrotica virgifera
<220>
   <221> misc_feature
   <222> (1)..(168)
   <223> RNAi target sequence DV-BETA7-FRAG5
<400> 663
   cattcgaaaa ttgtaaaaga aatgcacttc tggaaaataa gggccttgct cttccaaaag 60
   ccactaaaac aggtactacg atcgtaggaa tagtctacaa agatggaatt attcttggag 120
   cagatactag agctacagaa gacaccacag ttgcagacaa gaactgtg 168
<210> 664
   <211> 156
   <212> DNA
   <213> Diabrotica virgifera
<220>
   <221> misc_feature
   <222> (1)..(156)
   <223> RNAi target sequence DV-BETA7-FRAG6
<400> 664
   gctaaattag aactccacag attatacaca aaccgagtta ctagagtgtg tacagcaaac 60
   caattgttaa aacaatatct atttcgctac cagggatata ttggggctgc tttaatcctg 120
   ggtggtgttg atattgaagg accacatcta tatatg 156
<210> 665
   <211> 220
   <212> DNA
   <213> Diabrotica virgifera
<220>
   <221> misc_feature
   <222> (1)..(220)
   <223> RNAi target sequence DV-NATP1-FRAG1
<400> 665
   tgaatatcac atctgcagcg ggaattattt ccctcttgga tgaaccaaga ccagaattaa 60
   aagtatttgc actgaagaaa ctagattcca tagtgggtga gttttggccg gagatctcag 120
   aagctatcga aaaaatcgaa attttgcatg aagacaaagt tttccagcaa caccagttag 180
   ctgcacttgt ggcaagtaag gtatattacc atttaggcgc 220
<210> 666
   <211> 222
   <212> DNA
   <213> Diabrotica virgifera
<220>
   <221> misc_feature
   <222> (1)..(222)
   <223> RNAi target sequence DV-NATP1-FRAG2
<400> 666
   ttttgaagac tctttaactt atgctcttgg tgcgggtgat ctctttgatg tcaatgcccg 60
   gagtgagtat gtagaaacta ttctagctaa atgtatcgac tattacactc agcaacgtat 120
   tgctgtagct gaaggattgg cagatgccaa acccatagac tctcgccttg aagctattgt 180
   taatcgaatg tttcagaggt gtcttgatga tggtcaatat cg 222
<210> 667
   <211> 204
   <212> DNA
   <213> Diabrotica virgifera
<220>
   <221> misc_feature
   <222> (1)..(204)
   <223> RNAi target sequence DV-NATP1-FRAG3
<400> 667
   tcaagctatg ggcctggctc tagaaaccag acgaatggat atctttgagt catctattac 60
   tcagtctgat gatattatca gcatgttgac ttatgcattt caagtagcga tgagtttgat 120
   tcaaaatcga gcatttagaa acactgtttt gagatctctt gttggacttt atagaggact 180
   ggttacacct gattacgtta atat 204
<210> 668
   <211> 155
   <212> DNA
   <213> Diabrotica virgifera
<220>
   <221> misc_feature
   <222> (1)..(155)
   <223> RNAi target sequence DV-NATP1-FRAG4
<400> 668
   tgtatcgact attacactca gcaacgtatt gctgtagctg aaggattggc agatgccaaa 60
   cccatagact ctcgccttga agctattgtt aatcgaatgt ttcagaggtg tcttgatgat 120
   ggtcaatatc gtcaagctat gggcctggct ctaga 155
<210> 669
   <211> 226
   <212> DNA
   <213> Diabrotica virgifera
<220>
   <221> misc_feature
   <222> (1)..(226)
   <223> RNAi target sequence DV-NATP1-FRAG5
<400> 669
   attaacacta atgcaaagtt acttgccgaa agaagttgga cccagctcag gttattccga 60
   agggggcggc ctttatgctc ttggtcttat acatgccaac catggtacaa atatcattga 120
   ttaccttctt ggacaactta aggatgcaca gaatgagatg gtgagacatg gaggttgtct 180
   tggtctaggt ttggctgcta tgggcactca tagacaggat gtttat 226
<210> 670
   <211> 205
   <212> DNA
   <213> Diabrotica virgifera
<220>
   <221> misc_feature
   <222> (1)..(205)
   <223> RNAi target sequence DV-NATP1-FRAG6
<400> 670
   gaacaactca aattcaattt ataccaagat gatgccaata ctggtgaagc agctggtatt 60
   gcaatgggaa tggtaatgtt gggttcaagt aattctacca caattatgga tatggttgca 120
   tacgcacaag agagtcaaca tgagaaaatt cttagaggct tagctgttgg tattagtttt 180
   tccatgtatg gtagattaga agaag 205
<210> 671
   <211> 236
   <212> DNA
   <213> Diabrotica virgifera
<220>
   <221> misc_feature
   <222> (1)..(236)
   <223> RNAi target sequence DV-NATP1-FRAG7
<400> 671
   cagatccact gattcaacaa cttacctctg ataaagatcc tatcctcaga agatcaggaa 60
   tgtatacttt gggtatggca tattgtggta ctggacacaa ccaagcaatt agaaaactac 120
   ttcatgtagc tgtgtcagat gtcaacgatg atgttagaag agctgcagtt acagctttag 180
   gttttctgtt attcagaact cctgaacaat gtcctagtgt agtatccctt ctagct 236
<210> 672
   <211> 200
   <212> DNA
   <213> Diabrotica virgifera
<220>
   <221> misc_feature
   <222> (1)..(200)
   <223> RNAi target sequence DV-NATP1-FRAG8
<400> 672
   gtatggtaga ttagaagaag cagatccact gattcaacaa cttacctctg ataaagatcc 60
   tatcctcaga agatcaggaa tgtatacttt gggtatggca tattgtggta ctggacacaa 120
   ccaagcaatt agaaaactac ttcatgtagc tgtgtcagat gtcaacgatg atgttagaag 180
   agctgcagtt acagctttag 200
<210> 673
   <211> 199
   <212> DNA
   <213> Diabrotica virgifera
<220>
   <221> misc_feature
   <222> (1)..(199)
   <223> RNAi target sequence DV-NATP2-FRAG1
<400> 673
   tgggcgatac aaaggtagaa cctacaaagg tggataaaac aaaggggact gaggacaaaa 60
   acaaaaatga actgtcggaa gaagacaaac agcttcaaga tgaattaaat ttgtgtgttg 120
   agaaactcca ggataacgat gaaaagagat acctacttgc tttgcaagct ttatcaaatc 180
   atattcaagc ctcaactac 199
<210> 674
   <211> 198
   <212> DNA
   <213> Diabrotica virgifera
<220>
   <221> misc_feature
   <222> (1)..(198)
   <223> RNAi target sequence DV-NATP2-FRAG2
<400> 674
   gcctcaacta cttcgatgac ttctgttcct aagccgttaa aatttatgag gccccattac 60
   gaaaccatga agacagtaca tgaaaaaatt actgatcctg atactaaaaa agtctgtgca 120
   gatattatat ctgttttagc tatgacaatg ggagaaggca gagaatgttt gaaataccgt 180
   cttctttcta atttggat 198
<210> 675
   <211> 190
   <212> DNA
   <213> Diabrotica virgifera
<220>
   <221> misc_feature
   <222> (1)..(190)
   <223> RNAi target sequence DV-NATP2-FRAG3
<400> 675
   acatctacca tagagtgtgc ttatatctca ccagctgtgt accctatgtg gcagatcctg 60
   agaatactac tctactgcag actgctcttt tactgtttag aaagtttgat cagtatcccc 120
   aggcactgag attagcaatg cagttaaatg actttggatt aattgaagaa atttttgtca 180
   actgcccaga 190
<210> 676
   <211> 223
   <212> DNA
   <213> Diabrotica virgifera
<220>
   <221> misc_feature
   <222> (1)..(223)
   <223> RNAi target sequence DV-NATP2-FRAG4
<400> 676
   cggacgtaca aaaacaactg gcatttatgc taggacgaca acagatatat gttgaaattc      60
   ctgagcatac gcctgaatac gaagatctaa tcgagataat ggcaaactgt catcttaata     120
   accacttctt gaacctcgct cgcgagttgg acattatgga tcctaaaacc cccgaagacg     180
   tttacaaatc ccatttagaa aatagtcgtc ctccatttgg tgg                        223
<210> 677
   <211> 165
   <212> DNA
   <213> Diabrotica virgifera
<220>
   <221> misc_feature
   <222> (1)..(165)
   <223> RNAi target sequence DV-NATP2-FRAG5
<400> 677
   tgctaggacg acaacagata tatgttgaaa ttcctgagca tacgcctgaa tacgaagatc      60
   taatcgagat aatggcaaac tgtcatctta ataaccactt cttgaacctc gctcgcgagt     120
   tggacattat ggatcctaaa acccccgaag acgtttacaa atccc                      165
<210> 678
   <211> 156
   <212> DNA
   <213> Diabrotica virgifera
<220>
   <221> misc_feature
   <222> (1)..(156)
   <223> RNAi target sequence DV-NATP2-FRAG6
<400> 678
   catctaccat agagtgtgct tatatctcac cagctgtgta ccctatgtgg cagatcctga      60
   gaatactact ctactgcaga ctgctctttt actgtttaga aagtttgatc agtatcccca     120
   ggcactgaga ttagcaatgc agttaaatga ctttgg                                 156
<210> 679
   <211> 222
   <212> DNA
   <213> Diabrotica virgifera
<220>
   <221> misc_feature
   <222> (1)..(222)
   <223> RNAi target sequence DV-NATP3-FRAG1
<400> 679
   cttgtgcagg ccatgagaaa agcaccccag aatgctgcca ttggtttcag acaggttgtt 60
   caaaagctta ctgttgtagt tgaactactg ctaggagata taccagaacg acagattttt 120
   agacaatcta gtatgaggca ctcccttgcc ccatactttc aactaacaca agcagttaga 180
   atgggcaatt tgcaaagatt tggtgaggtg ttagagaact tc 222
<210> 680
   <211> 222
   <212> DNA
   <213> Diabrotica virgifera
<220>
   <221> misc_feature
   <222> (1)..(222)
   <223> RNAi target sequence DV-NATP3-FRAG2
<400> 680
   ggcccacaat ttagacaaga tcatacattt acactaattt tgagactgcg ccacaatgtt 60
   attaaaacag ctataagatc cataggatta tcatactcca gaatatctcc ccaagacata 120
   gctaagaaac tggggctgga ttcagctgaa gatgcagaat ttatagtagc aaaagcaatc 180
   agagatgggg tgatagaagc tacacttgat ccacagggag gt 222
<210> 681
   <211> 159
   <212> DNA
   <213> Diabrotica virgifera
<220>
   <221> misc_feature
   <222> (1)..(159)
   <223> RNAi target sequence DV-NATP3-FRAG3
<400> 681
   caatctagta tgaggcactc ccttgcccca tactttcaac taacacaagc agttagaatg 60
   ggcaatttgc aaagatttgg tgaggtgtta gagaacttcg gcccacaatt tagacaagat 120
   catacattta cactaatttt gagactgcgc cacaatgtt 159
<210> 682
   <211> 161
   <212> DNA
   <213> Diabrotica virgifera
<220>
   <221> misc_feature
   <222> (1)..(161)
   <223> RNAi target sequence DV-NATP7-FRAG1
<400> 682
   ctttagttag atctattgtc gctctacaca atcttattaa caacaaacta acaaatagag 60
   acgcggaaga aggaaaaaag gaggatagta agaaagaaag caaagataag gacaaggata 120
   aagagaagga aaaggataaa gatgctaaga aggacgagaa g 161
<210> 683
   <211> 151
   <212> DNA
   <213> Diabrotica virgifera
<220>
   <221> misc_feature
   <222> (1)..(151)
   <223> RNAi target sequence DV-NATP8-FRAG1
<400> 683
   ttgccaaaaa ggctttccag ctcaaaccgt ggaagctcaa caatgacttt ttccactttc 60
   ttcctgaaga gaagacccac gaacccatac cctcacagga actggcacag caggctatag 120
   agtatgccaa ggaacttgaa atgattgtct a 151
<210> 684
   <211> 163
   <212> DNA
   <213> Diabrotica virgifera
<220>
   <221> misc_feature
   <222> (1)..(163)
   <223> RNAi target sequence DV-NATP8-FRAG2
<400> 684
   gctggacacc ataagagaag aaattgcaga ctgtttatca gtagcctatg aaaagatctc 60
   agtaaaagac gtagccagaa tgctgtacct gccaaacgaa gaagctgcta aaacatttgc 120
   caaaaaggct ttccagctca aaccgtggaa gctcaacaat gac 163
<210> 685
   <211> 172
   <212> DNA
   <213> Diabrotica virgifera
<220>
   <221> misc_feature
   <222> (1)..(172)
   <223> RNAi target sequence DV-NATP8-FRAG3
<400> 685
   tgtacaagca gttaaaatca gaatggaata ccaacaaccc aaatttaagt aaatgtgaaa 60
   aactactctc aactcttaag cttgagctca ccaatctaat gtttctgccg attaccacat 120
   ctactgcgtc caaacaagaa ctacttctag ccagagatgt tctagagata gg 172
<210> 686
   <211> 673
   <212> DNA
   <213> Diabrotica virgifera
<220>
   <221> misc_feature
   <222> (1)..(673)
   <223> RNAi target sequence DV-NATP14
<400> 686
   actcacggtt gtttttgtaa aacttgtagg taacgaattt tcgtttttag tgaattaaca 60
   aaacaacaga gtgattttcc aacaagaata acttatttat tagtttcatt aatttgataa 120
   acaataaaat ggatagactt cttcgactag gaggtggaat gccaggttta actcaggcac 180
   cgccgccttc tgatgctcct gttgtggata ctgccgaaca agtatacata tcctccttag 240
   cactattaaa aatgttgaag cacggccgag ctggagtacc catggaagta atgggtctta 300
   tgttaggaga attcgtagat gactataccg ttcgtgtaat cgatgtattt gctatgcctc 360
   agactggtac cggtgtcagt gtagaagctg ctgatccagt gtttcaaaca aaaatgcttg 420
   acgtgttaag acagactggt aggccagaaa tggttgtggg gtggtatcat tcccatccag 480
   gtttcggatg ttggttgtct ggagtggata tcaacaccca gcaaagtttt gaggctctca 540
   cggaaagagc tgtagcagta gtagttgatc caatccagtc agtgaaaggc aaagtcgtta 600
   ttgatgcctt taggttaata aatccgaata tgatggtatt gggccaagaa cctcgccaaa 660
   ctacatccaa ctt 673
<210> 687
   <211> 246
   <212> DNA
   <213> Diabrotica virgifera
<220>
   <221> misc_feature
   <222> (1)..(246)
   <223> RNAi target sequence DV-NATP14-FRAG1
<400> 687
   aactttagca gattactctg aaaattgtag tgtgaatgaa aagactgtag ctgatatgtt 60
   agaactagcc aagaattaca acaaggcatt agaagaggag gagaaaatga ctccagaaca 120
   gctcgctatt aaaaatgttg gcaaacaaga tcctaagagg catttagaag aaaaggttga 180
   tattttgatg accaataata tcgtccagtg cttgggagct atgctagata cagttgtttt 240
   taagta 246
<210> 688
   <211> 205
   <212> DNA
   <213> Diabrotica virgifera
<220>
   <221> misc_feature
   <222> (1)..(205)
   <223> RNAi target sequence DV-ATP5-FRAG1
<400> 688
   tgacggtaat taacaagatg gaggtggacg gaggtccgaa gggagaaggt tttcggtcat 60
   attatattac aaagattgaa gaattgcagc ttatagtggc ygaaaagagt caaaatctta 120
   gacgtttgca agctcagaga aatgaactca atgccaaagt tcgtatgttg agagaagaac 180
   tgatgttact tcaagagcaa ggaag 205
<210> 689
   <211> 219
   <212> DNA
   <213> Diabrotica virgifera
<220>
   <221> misc_feature
   <222> (1)..(219)
   <223> RNAi target sequence DV-ATP5-FRAG2
<400> 689
   ctatgtcgga gaagttgtaa agcccatgga taagaaaaaa gtccttgtga aagttcatcc 60
   agaaggaaaa tttgtagttg acattgacaa aaacatagac ataaatgatg ttactccaaa 120
   ttgtcgagta gccctcagaa atgaatctta cacattgcac aaaatacttc caaacaaggt 180
   tgatccttta gtttccttga tgatggtgga gaaagtccc 219
<210> 690
   <211> 244
   <212> DNA
   <213> Diabrotica virgifera
<220>
   <221> misc_feature
   <222> (1)..(244)
   <223> RNAi target sequence DV-ATP5-FRAG3
<400> 690
   tagattggac atcttaaaga ttcactccag aaaaatgaac ctcacaagag gaataaatct      60
   gagaaaaata gctgagttga tgccaggtgc tagtggagct gaagttaagg gtgtgtgtac     120
   ggaagctggt atgtatgcac taagagaacg aagggtgcat gttactcaag aagattttga     180
   aatggccgtc gccaaagtta tgcagaagga ttctgagaag aatatgtcca tcaagaagtt     240
   gtgg                                                                  244
<210> 691
   <211> 163
   <212> DNA
   <213> Diabrotica virgifera
<220>
   <221> misc_feature
   <222> (1)..(163)
   <223> RNAi target sequence DV-ATP5-FRAG4
<400> 691
   gattgaagaa ttgcagctta tagtggcyga aaagagtcaa aatcttagac gtttgcaagc      60
   tcagagaaat gaactcaatg ccaaagttcg tatgttgaga gaagaactga tgttacttca     120
   agagcaagga agctatgtcg gagaagttgt aaagcccatg gat                       163
<210> 692
   <211> 176
   <212> DNA
   <213> Diabrotica virgifera
<220>
   <221> misc_feature
   <222> (1)..(176)
   <223> RNAi target sequence DV-ATP5-FRAG5
<400> 692
   tagctgagtt gatgccaggt gctagtggag ctgaagttaa gggtgtgtgt acggaagctg      60
   gtatgtatgc actaagagaa cgaagggtgc atgttactca agaagatttt gaaatggccg     120
   tcgccaaagt tatgcagaag gattctgaga agaatatgtc catcaagaag ttgtgg         176
<210> 693
   <211> 1432
   <212> DNA
   <213> Diabrotica virgifera
<220>
   <221> misc_feature
   <222> (1)..(1432)
   <223> RNAi target sequence ta00611.03_diaw
<400> 693
   cagaatctta tctgtaaaga taaactaatt atctctattg actcacctca ttttatacgt      60
   agcaggtgaa aatacaccat tgtagtgaaa attattatta gttcgattta taataagttc     120
   gattttttac gaaaatgaca agtatcgaga ctgtggggac cattgtcctg aaattgctga     180
   agttggtgat caatttgata tgtctcatct tgtaccgaac cggatatcaa ggctacttct     240
   tgggagtagg aggaacctgg aatctaaacg aagaaaaaaa tcccgatgca gaaattgtgg     300
   cttccggcgt attcgtagga tttatgattt acacattcgt ctcgctgatc agcctttgct     360
   tcgctagtgg agatcacaaa acgacattca ctgatattct gatgaatata gtagggattt     420
   ttatgtggat agctgttgga gctacagctc ttcattattg gcttgggtac ttgtccgaat     480
   acaaatacac gacaatagat tctgaacgac aagttggttt ggcgttagga gcgatgtgta     540
   taataaatgg agcggtctat cttgtagacg gagtactttc cgcaatcttt atcctcaaag     600
   ccaaaatgca ataactttca tcgtaatata aatatattta tttaggttat atactttact     660
   ttaagcagct caagtatacc gtgacatccc actcatacat caatgtctat aattgtttca     720
   tgacaaatca tttaatagta ttttaaagca ttcattcgtt caacaccttt tttacatttt     780
   tcaagtatta tatcaatctt tttaaagaat tgtttggcta ttaaccggat tcaatccata     840
   tagttccgta aaataattat gcgttttaat aattaatgta attaaaaata gttagtatat     900
   ttttgaaata aaacaactgc cgcaacaaat tagggagaat acttttgtct cctgtagttt     960
   tatctgttat ccaaagttcg attaaattgt ttcaatttaa tgtatttttc catataacgc    1020
   accaaatagt cttaaaaaaa tagtgcctgt tactacctaa tactttctac gtatgcctta    1080
   tggttagtag tggctccaat aggtgcattt tacatttttc atgaaaattc aattcacatt    1140
   attttcctcc cgttctcttc atattatatt tatttattct ttaatatagt tttgggcgtg    1200
   aagcccagta aatacctata tgaagtatat tgatggaact gccatgcttg atgggttttg    1260
   tctgcaatta aaataaaacc aaaacaaaca tttgtacagt attgtaaaat tcacaatatt    1320
   taggtagatg tttaaaattt aatatttttt aagagtttgc atttatgtta atcgtagtta    1380
   agtttttata aaaatttagt agttgtgaga aataaaataa gtaaaagaga at            1432
<210> 694
   <211> 730
   <212> DNA
   <213> Diabrotica undecimpunctata
<220>
   <221> misc_feature
   <222> (1)..(730)
   <223> RNAi target sequence ta01434.01_diaun
<400> 694
   tttatacgta gcaagtgaaa atacaccatc gtagtgaaaa ttaatattag ttaaaacgaa      60
   aagttcgatt ttttacgaaa atgacaagta tcgagactgt ggggacaatt gtcctgaaat     120
   tgctcaaact ggtgatcaat ttgatatgtc tcatcttgta ccgaaccggt tatcagggct     180
   actttttagg agtaggagga acctggaatc taaacgaaga aaaaaatccc gatgcagaaa     240
   tcgtcgcctc cggagtattc gtaggattca tgatttatac attcgtctcg ctgatcagcc     300
   tttgcttcgc tagtggagat cacaaaacga cattcactga tattctgatg aatatagttg     360
   ggatttttat gtggatagct gttggagcta cagctcttca ttattggctt gggtacttgt     420
   ccgaatacaa atacacaaca gtagattctg aacgacaggt cggtttggcg ttaggatcga     480
   tgtgtatagt aaatggagcg gtctatcttg tagacggagt actttccgca atcttcatcc     540
   tcaaagccaa aatgcaataa atttcatcgt aatataaata tatttattta gtttctatat     600
   tttactttaa gcagctcaag tgtaccgtga catccaacac aaatcaatat ccataattgt     660
   ttcatgacaa atcatttaat aatattttaa agcattcatt cattcaacac cttttttata     720
   tttccaagta                                                            730
<210> 695
   <211> 1629
   <212> DNA
   <213> Diabrotica barberi
<220>
   <221> misc_feature
   <222> (1)..(1629)
   <223> RNAi target sequence ta01092.02_diab
<400> 695
   tattgactca cctcatttta tacgtagcag gtgaaaatac accattgtag tgaaaattat 60
   tattagttcg atttataaaa agttcgattt tttacgaaaa tgacaagtat cgagactgtg 120
   gggaccattg tcctgaaact actcaaattg gtgatcaatt tgatatgtct catcttgtac 180
   cgaaccggat atcaaggcca cttcttggga gtaggaggaa cctggaatct aaacgaagaa 240
   aaaaatcccg atgcagaaat tgtggcttcc ggcgtattcg taggatttat gatttacaca 300
   ttcgtctcgc tgatcagcct ttgcttcgct agtggagatc acaaaacgac attcactgat 360
   attctgatga atatagtagg gatttttatg tggatagctg ttggagctac agctcttcat 420
   tattggcttg ggtacttgtc cgaatacaaa tacacaacaa tagattctga acgacaggtt 480
   ggtttggcgt taggatcgat gtgtataata aatggagctg tctatcttgt agacggagta 540
   ctttccgcaa tctttatcct caaagccaaa atgcaataac tttcatcgta atataaatat 600
   atttatttag gttatatact ttactttaag cagctcaagt ataccgtgac gtcccataca 660
   tgcatcaatg tctataattg tttcatgaca aatcatttaa tagtatttta aagcattcat 720
   tcattcaaca ccttttttta catttttcaa gtataatata ttatacctac ctatagttcg 780
   ttcgctaaac tcagacacaa ctggctagtg atttcggtag ataatttttt tgattttgga 840
   caattttacc aaaattacta actacatatt tagtaattat tttttgccaa ttttacgaaa 900
   ttgtcaaaat tacttactaa aatcaatagc cagttgtgtc tgagtttagc gaactgacta 960
   tatcaatctt tttaaagaat tgtttggctg ttaaccggat tcaatccgta tagttccgta 1020
   aaataattat gcattttaat aattaatgta attaaaagta gttagtatat ttctgaaata 1080
   aaacaactgc cgcaacaaat taggaagaat acttttgtcc cctgtatttt tatgttatcc 1140
   aaagttcgat tcaattgttt caatttaatg tatttttcta tataacgcac caaatagcct 1200
   taaaaaaata gtacctacta ataccttcta ctacctaata ctttctacgt atgccttatg 1260
   gttagtcgtg gctacaatag gtgcatttta cattttttcc tgaaaattca attcacattc 1320
   ttttcctccc gtgctcttca tattatattt atttattctt taatatattt ttgggcgtga 1380
   agctctgaag cccagtaaat atatatgaag tatattgatg gaactgccct gcttgatggg 1440
   ttttttctgc aattaaaata aaaccaaaac aaaaatttgt acagtattgt aaaattcaca 1500
   atatttaggt agatgtttaa aatttaatat tttttaagag tttgcattta tgttaatcgt 1560
   agtttaaagt ttttataaaa atgtagtagt tgtgagaaat aaaataaaac caaaacaaaa 1620
   atttgtaca 1629
<210> 696
   <211> 720
   <212> DNA
   <213> Epilachna varivestis
<220>
   <221> misc_feature
   <222> (1)..(720)
   <223> RNAi target sequence ta26722.002_epiva
<400> 696
   gtaaaaactg acttaattag gtttcagtat ttttcttgtg gttaatttgg aaagtgtacg      60
   aaaatgacca gtatagagac agttggtgct atagctattc gtgttctgaa actggtaata     120
   aatattattg tcattatcct ctaccgaacg ggcaaccatg gggcttttct tggtgtcggt     180
   ggaacatgga acttaaacga ggaaaaaacg gctgacgtgg aaattatagc ttctggagta     240
   ttcgttgggt atttgatata cacagcggtc tccttgttga gcttattatt tggaacaaga     300
   gacaacaaag tatatttcac tgatgtcata atggggcttg ttggaatact gatgtggctt     360
   acagttggtg cagctgccct acattactgg attgggttta tcgacgaaca caaatacaca     420
   gctgtatctt cggaacgatc ggttggtcta gcactgggcg ccatgtgcat cctaaattcg     480
   gtcctctatt tgatagacac agtggtttca acgttattcg tagttagaga gaaattaaat     540
   ggctcatttg cttgatgtgt agtaaattct tacaattagg tttactttta tacttttttc     600
   tatttgtttt actgaagatt ttctctatgg agaattcaat cgacccgtga attgtgttcg     660
   cagccatact gttagaatga agcgaaaaac tgaagatttt ctctatggag attcaactga     720
<210> 697
   <211> 1043
   <212> DNA
   <213> Leptinotarsa decemlineata
<220>
   <221> misc_feature
   <222> (1)..(1043)
   <223> RNAi target sequence ta37400.0001_lepde
<400> 697
   acccactgtt tccatactag tcattttggc acaatcttat cagtcgagat aacgtgatta 60
<210> 698
   <211> 648
   <212> DNA
   <213> Orius insidiosus
<220>
   <221> misc_feature
   <222> (1)..(648)
   <223> RNAi target sequence ta01487.01_oriin
<400> 698
   acgacgctct tccgatctgt gaattaaaaa aaaaaggggg acatcaaaaa atcaaagaaa 60
   aatggaaatg gaaactataa ttacgatttt tattaaagtc ataaaatggg tgataaatct 120
   cattgtaatc ttcttatacc gatacggtta ttctgggcaa ttcctcggta ttggaggaac 180
   gtggaatttg aatgaggaga aagatgcgac agcagaagtt gtcgcttcgg gcgtcctcgt 240
   cggctacttt atctacactt cggtcattct aatggcattc tgttttggga cgacaaaaca 300
   taaagcgagc gttgtcgaca taataatgaa cataactggg acgatacttt ttgtcgcagt 360
   cggcggagtg gcacttcact actgggccgg atatcaaaac atgaatcatt tccagccgat 420
   agcgactgaa aaacaagtcg gtttcgccgt cgggagcctc tgcgtcatcg aaggtgccat 480
   ttatttagtc gacaccgtcc taacttttgt tcacatcgtt aaaaagaacg acctctttta 540
   atcaaaagta caaaaaacaa tattttactt taaagaaaaa ttttaatttt tataaatttt 600
   gtattacaac tgaagtatta taataatatt aatctttttt aaaaaaaa 648
<210> 699
   <211> 820
   <212> DNA
   <213> Coleomegilla maculata
<220>
   <221> misc_feature
   <222> (1)..(820)
   <223> RNAi target sequence ta21166.0001_vibdu
<400> 699
   aggatcagct taactatcct aatagctata tctccctgtg ttccactctg atcctatatt 60
   cacttgtttt tgtgtgtggt gtttttcatc ataatttaat tggtaaaaaa aataattttc 120
   gttttttttg tggaaaagtg agtgaaaatg acgagtatcg aaaccattgg agctatagct 180
   attaggatag ttaagctgat cctaaatctc atcattttga taatgtatcg aacaggaaat 240
   cacggaggtt ttcttggtgt tggtggtact tggaacttga acgaagaaaa aacagcagat 300
   gttgaaatca ttgcttcagg agtcttcgtt ggctacttca tctataccgc agtctcactt 360
   atgagtatcc tcttcggtac cagagataat aaagtatatt tcactgacgt cataatggga 420
   gtcattggcg tttttatgtg gatcacagtt ggtgcagcag ctctgcacta ttggcacggg 480
   tacttggatg aacacaaata caaatcagtt tcatcagaaa gaacggtggg attaattatg 540
   ggctcgttgt gcatcctgaa tgcagcttta tatctattag actcagtggt tgccgttctg 600
   ttcgtcgtaa gagaaaaatt aaatggccaa ttttgaaaaa taatttcata attcataagt 660
   gtttttttgt attttttcaa atatgaaatc gacaacgaga gtttaagtcc aaccagaact 720
   gatttccaga atgacaaaag ccccaaaggc tatcctcact gcagttattt cgaaatagtg 780
   gatacttata atttttaatg aagagcaaat atgaaatcga 820
<210> 700
   <211> 5238
   <212> DNA
   <213> Diabrotica undecimpunctata
<220>
   <221> misc_feature
   <222> (1)..(5238)
   <223> RNAi target sequence ta00809.07_diaun
<400> 700
   cgaactttac taaccgtagc tacttttttt gatgtgatct ttatgttgcg ctcatcagta 60
<210> 701
   <211> 4313
   <212> DNA
   <213> Diabrotica barberi
<220>
   <221> misc_feature
   <222> (1)..(4313)
   <223> RNAi target sequence ta06241.06_diab
<400> 701
   aaaacataac tttttgactg aagaatgttt ctaggtacag cggcacaaat actttggtct 60
<210> 702
   <211> 1564
   <212> DNA
   <213> Epilachna varivestis
<220>
   <221> misc_feature
   <222> (1)..(1564)
   <223> RNAi target sequence ta29117.001_epiva
<400> 702
   cttcaatgga aaaggtgaat tcgtgctgat gaagactaac gtggcttcgc agaggttaga 60
   agttcaagct cgtttcgatc aaatgccacc taatgaatac ggtcaagtta gagccaccca 120
   gctctcctcc atagctgtca gaggcaattc ttcgactatt attgaagtta gaataagacc 180
   aaaggaagcc aggtggagat ataaaatgga tgttattgcg gataagcaaa gaagactttt 240
   cttcgaccga ccttcgctta aattccaaca ttttccaggt ctaaccgttt acactccaac 300
   atatatcctg aaccaatctg aagttattat aatgttcgac tcaggagctg gtgtagaaat 360
   tgtagaaagt ttgggattca tgtcagcaag agtttactta ccatggactt tcataaataa 420
   aacaatggga cttctaggaa attggagttt tgacatgacc gatgatttca ctttacctga 480
   tggtacaaaa cgacctgttt ctggtaatat caacgacatg caagacattt acgaaaactt 540
   cggcaagaaa tggatgattg atgactcaga cgatcctcaa aaaggaaaat cgctgttctt 600
   tatcgaaaac ggacggactg cagccttcta caacgaccta aatttcgtac ctgaatacag 660
   catgaatata aactacatac tggcaaacaa aacaggtcgt attgaaatgg caaatcaatg 720
   gtgttccaat aaagactatc agtgtatgta cgactatgcg atgacattag ataaagatat 780
   ggctttttat accacaattt ttaaatcggg cttgcaaaac ctaagagaag cggcaagcaa 840
   aagggtaacg tcttgcggtg tgttggaaac gcccaggttt ggttggaaaa cgaatttctt 900
   cttcacgcct ggaactaaag tcagtttcga ttgtgctcaa gacttcgtat tgagcggtga 960
   tcctagaagg gaatgcctga agaatggaga gtggaatatc ccagaatttg gttatactga 1020
   atgtttacgt gaagaagaat attctctgag acaggcagga actacatttg gaataatact 1080
   tgcagtgatg attcctctta tacttttatt tgtattcttt ggttacaaat atcttcagaa 1140
   gctgacagcg caaaataatg agcaatatga agagaaaatg acattggaac aacaaaaaag 1200
   gttagcacaa gaaagggcag ctaaaaaatt aaccgccgtt gatgaggacc atgatgatga 1260
   tgtgctttcg gaaacaaata cttcatcgcc aagagaaaca acagtgtatt aattcgtaaa 1320
   tttcgattag tcatgagtta ggaaaacgta gtttgccatc attcataata ttttacttca 1380
   aaatttgaat gtgaaaattt tttcaaaaaa tttttaaata tggatatata aaattatagg 1440
   agtttttgtt tgatgattgt taagtagaat ccaatcgctc tctataaatc aaaaaattca 1500
   gagacagaac agctctttta gatttagagg tattttaaat gttcataatc gcattttaca 1560
   tttc 1564
<210> 703
   <211> 5020
   <212> DNA
   <213> Leptinotarsa decemlineata
<220>
   <221> misc_feature
   <222> (1)..(5020)
   <223> RNAi target sequence ta36833.0009_lepde
<400> 703
   ataacaaatt ttatcttgtt acagagcaca cttgtgagaa ggcaatcgag taacgaacct      60
   catacataaa agtacacgta tggtgataga aaacaataaa ttatcaaact gaggctctgt     120
   gccaagtttt ggaaaatgta cgtcaagtgg aaattggttt tgactctagt actgtgtgtt     180
   ggtgtcgtga ttggggaaga tatttcaaca gatataatcc ccttgccaca agacaataca     240
   gcagatgtgg aaatagtggc tacagaaact aggtcaaatt caggatctga agctgctgta     300
   gaagagccca ccaataacac tgggccttca gatactacaa acccaaatta tgctgtagtg     360
   ccccctgtag tttctgatag caaacctgca ataacaaata gtgaaacaaa tgacactaac     420
   aatgatgttg taatgttgag tcctacgaaa tttctcatga aaaagggacg atctggacgt     480
   ttattggaat atccgactga ttacgatcct atgacttccc atattgctcc accggatagt     540
   gatcagagag gatattcagg agtaccatat gtcttgacgg aaaccagact gcaacagatc     600
   cgccaaaatt tcatgtatcc ctactacaac agaggcggta atgcagatga cgaaggagac     660
   taccagaaag aaattcagtc atctattccg caagtgtaca agaacctcaa cttccaactc     720
   cctttcttcg gatttcgatt caattacacg agggtctcct taaacggcta tttggaattc     780
   agcgatcctc ctccaaatta cgactatcct ttggtctttc cagtcaagga atggcctaaa     840
   aagaacgacc cttctttcat cggtatcttt ttcagtaaat gtagaatcgg taacctgagg     900
   gacggagata ttgatcaaag agaccctgga gtgtacttta ggatggaaag ggatctcaga 960
   aataggcagg acaggatggg agtggagatc agagaacgac tgaaatggga tataagggaa 1020
   ggggtgatag ggtcagaaac attcaatccc aaacatgcca ttatagtcac atggaaaaat 1080
   atctctttca atggaggttt tggcaatgct ctctaccaga ctaacacttt ccaaatgatc 1140
   ctcgccactg atgaagtttt cacctacgcc atgttcaact acttgaatct tgactggacc 1200
   acccacactg aagcgggagg cgacacaaga aaaggagaag gaggagttcc cgcttttgtg 1260
   ggattcaacg ctggaaacgg tactagaagt tttgaataca aaccatacag tcaagaatct 1320
   gttattcgag atctcacaca aactggtttc gctaatggtt tcaaaggaag gcacattttc 1380
   cgaatcgacg aaaatatcct aactggaaca tgcaataaag atatagatgg tgctaatcta 1440
   ccgctaatga tatctccaga aagtggaaat atgctgggtg gaacaatagt gaatataaca 1500
   ggaccttgtt tcggcctaga cgaccaagtt aaatgcaaat ttgatgtagc aaatgaaata 1560
   aatggcgtcg ttatagataa aaacagggct atatgcatcc aacctagact gtatgccgaa 1620
   ggatgggtga atttacaaat agccataggg gctggggtat acaaatggaa gggaaaatat 1680
   tatgtcgaat cccccgcagc ggcatctcaa aaaatctact tcaaggacat gaaggttcat 1740
   gaaaaatcgc ctagtgaaat aagaataact tgggaaaaat acaacttgac cactaacgaa 1800
   aacgctaaca ttcgtatctc cttatggggt tacagagaaa caacaataag gccaacgttc 1860
   gtttacatca ctgatatcgc agacagtctc caaaatactg gagagtatac catcgtaccg 1920
   tcccaatata gaacaaaagt taatgagttt ctcacggata tcaaatttgg tttcttgcaa 1980
   attaacttga ctgaatctat caaagtgaac acttatacat ctgtacaaag atcggtggaa 2040
   atagttcctg ttgtatggag tcgacccatt cccctaggat ggtactttca gttccaatgg 2100
   gaaaatatgt atggacgaag ctggcccaaa gcactctgcg atgactggct aagaacagac 2160
   agatacctga aaaactttgc tcatgagtta cctcaatgcc cttgcactgt agaacaggct 2220
   ttggcagaca aagggaggta tatgcccgac tttgattgcg acaaggactc aaatcccgta 2280
   tgctactaca ataaccaagc tctgcactgt gtgaaaacag gatcaccaac gttggaggga 2340
   tcagaacagc agtgctgcta tgacaaaaac gggtatctca tgttatcata cgatcagcag 2400
   tggggttcaa gtccacggcg ttgccacaat ctgggaaaaa tgccctacaa cgaagcaaca 2460
   aaagttccaa ccttatcgca atggttcaac gatatggtac cgaagtatct ttgctgtttg 2520
   tggcaggaag aacaggcggt gggttgcgaa acgctgagat tcgaaagaag accaactcag 2580
   gactgtgtcg cgtaccaagc tccagggatt gctgggattt acggagatcc ccacgtcatc 2640
   actttcgatg acgtcgagta caccttcaac gggaaaggag agtttgctct tgtgaaatct 2700
   gtgacacaaa ctgacaactt ggaggtgcaa ggcagatttg agcaaatgga ccctaacgcc 2760
   tacggagaag tacgtgcaac acaattgact tcaattgtgg caaggggaaa caacaccata 2820
   gcagtggagg tcagaaggag gcccttggat gctaggtgga ggtataggct ggatgtcata 2880
   gctgataata ggaagttgtt cttcgacaga ccctctttga aatttcaaca tttccaagga 2940
   gtgactattt atacacctac ttatatcctc aatcagtctg aagtcatcat tatgtttgat 3000
   aacggagcag gagttcaagt aatggataac cagggattca tgaccgcgag ggtgtatctt 3060
   ccttggtcat tcatcaacaa aactgttggt ctctttggca actggagttt caataaggaa 3120
   gatgacttca ctcttcctga tgagtcgaag gctgccgtcg tgagtaatat caatgatatg 3180
   gaaagggtct acaatgactt tggttccaaa tggatggtgg acgacgtact agatccgaaa 3240
   agaggtagat ccttattttt cagagaattc ggcagatcat cggcaacgta caacaacaaa 3300
   actttcaaac cgcagttcct tatgttacct gaggacataa tacccgcaaa caggtcgata 3360
   cagatacaga gaacttacga catttgtagc acaaaaatgt acgaatgctt ctacgattat 3420
   gccatgacgc tcaacagaga tcttgcccat tttactcaga attataaagc aaccatatat 3480
   caactcaaag aaacgacgag gcagaaggtt gtttcttgcg gagttctgga aacaccgcga 3540
   ttcggtagga agagtacttt tctttttata ccaggaacca aagtcactta cgagtgcaat 3600
   caagacttcg tattggtggg agatcccaga agagaatgtt tggcagatgg cacatggaat 3660
   gctcctgaat atggctacac cgaatgttta cgtcaacaag aatattctca gcgccaatca 3720
   gccattgcct ctggagccgt tctcgcaata attattccac tagttctatt atttgtatat 3780
   ctggcttata tgttcctcaa gaagaaacag aaagaacgag acgaagaaaa tttacaaacg 3840
   caagcgtacg agcaacagaa aagacaagct caggaagctg ctgctagaaa attaactgct 3900
   gccgaagagt acaactcaga tgaagacgat aataacagca acgttacgag cacagcaaaa 3960
   gaaacaacag tgtattagta ttagatatgg taacactttt acgatattat tattttggtt 4020
   ggcatacttc tcagacaaac agtccaaatt attatttgca acaagaacta tttgaatttc 4080
   gaagtttcct ttgaagatcc caagttgctt attatcctaa atttttgcaa aatgtcaaaa 4140
   tcgtactatt ccatcagttg tatattttat ttttctgaga tagttaggcc aacaggtatc 4200
   taaaggagtg aataacagat ttttcatatt ttccagtttt atctgattca ataatgcact 4260
   atgggttttt tctgttaatg gtttttataa cacctctgtt gcaaaataca tattaggtaa 4320
   tataaatata cgtaattaag tcgtaattaa gtattataac aaaatggcgt tctctattat 4380
   ttaaattgta attagatata tattttttat attcataatc acttttgcat gtaaagtagg 4440
   agtccaattt tctgtacttc aaccatcact atcgctactt accccgatca ctttttggat 4500
   gcacatttca tgcacatttt tgcacattgt ttgcacagta agttcacttt gcaaacaatg 4560
   tgcgaagact atgcgggagg acatgtttat gcagtaattt tgtatatatg tgagtaggta 4620
   tatacctact attttcatca tactctggtc ctacttttct tatttacagt gataagatga 4680
   tgtttgaaat cttgaattag gtacaatgaa ttcatttttg tcagttattt gaaacatttt     4740
   cggatatttt aataagactg tgttttatca aagttcagtt tgattgtcca aatactagta     4800
   gatcattaga gtcctactgt agttttagca ttttaataaa gtaattcaat attaatatac     4860
   aagtaatctt gcacttctgc taactcaaaa ttttgcaaaa ttattcactc gaatcagtat     4920
   tcacacgttt ctctttcaga aataaccaaa atatttcacc ccatacacaa atttcattag     4980
   gtttagtgaa atttgtgtat ggggtgaaat attttggtta                           5020
<210> 704
   <211> 3724
   <212> DNA
   <213> Orius insidiosus
<220>
   <221> misc_feature
   <222> (1)..(3724)
   <223> RNAi target sequence ta02139.06_oriin
<400> 704
   tttttaacgt taataaaaca gttttttaat ataaaaaaag tgtacaaaag tttagtatat      60
   gtcggtttct acgctatcgg cgcgacgcat ttgagatttg ggggagtcga acgagctgtc     120
   ggttccgtct tcgaagtcga tgtcggggcg gttcggaagg ggctcgtgtg tattgtaaac     180
   ggcgtcgaaa ttgctcggat tcctaacgag tttatcctct tctttacccc ggccagataa     240
   ggaagaacgc ctcctgctgt acttccagct ttggaccccg ttctgaagac gggctgagtg     300
   acggcgataa aaccacgtcg cgatacagac cagtgtgagg accaacggca aaagaatgcc     360
   caaaatcata agcgacatat tcgccagttg ttgcctgtcg tattcgacat acctgagaca     420
   ttcggtgaac ccttcatccc cttcgatcca tttcccttga gcggagcact tcctttgttg     480
   atctccaaca aaatgaaacc ttgatcgcat tcgaaaataa ctttggtatc aggaacgaag     540
   aagaagttac ttttccttcc aaatcttggc gtttcaagaa ctccacagct caatgttctc     600
   attttgttgg tctgcttgat ggatgaatat tcgctgaagt agtccaaagt ctgtttcgcc     660
   atttcacgat tgaggctcac cgcgtaatcg tatttacatt gatacgagtc gccgcagagg     720
   tcttcagcct tagagacgtc ctgtgtccga ttcggtggta tgatttcctg aggagttttt     780
   ttccattctg gtacaaaagt acgattcgca tagtacgatg ctgaacggcc atactccctt     840
   atgaagaggg gctccccttt tttcggatct gctctatctt ccagcaacca tttgagagca     900
   aaatctttgt gtattcgttc gaaatcttca aggtctccga cttggacgat ttgtccgtca     960
   ggagtggtga agtcatcgga aatgtcgaag ctccaatttc ccatcaaacc tcgcgtttga    1020
   ttgatcatca tccagggcaa gtagacacga caggccatgt atcctttgtt ctcaactacc    1080
   tcaactccta ctccggtttc gaacataatc acgatctgcg attggtccaa aacctgagat    1140
   ggcaaataga cggtgacgcc tcggaagtgc tgaacacgaa gtgcaggacg gtcgaaaaag 1200
   actcgacgtt tgttggcgaa aacgtccaat ctgtatcgcc attgggcgta tcttggtcgc 1260
   aagcggactt cgatgatcgc cgaattattt cctctagctg caacagatgt cagtaaagtg 1320
   gcagcagtgt ctccgtagat attcggcccg acctgttcga atctcccttg tacgtcaagt 1380
   ttgtatttat ctgttgaaga atggagcaaa acgaattcgc ctttaccgtt gaaagtgtat 1440
   tcaagctcgt cgaatgtgac aatgtgagga tcgccgaaga cggtagcgac gtaaggcgct 1500
   tggtatccta cgcagtcttg actgggtcgc cgttcgaaac gtaatgtctc gcatccgaca 1560
   gcttgctcag actgccataa acaacagtgg tagaatgggc ttatatcatg ccaccactgc 1620
   gataatgttg gaactttgtt cggctcagtc cacggtaaaa gacccaaatt tgatgatctc 1680
   ttcggcgaag acccccattt ctggtcgtac gacaacatca aatattcgtt tttgtcataa 1740
   cagcattgtt gttctgctcc ttccaaattc ggagcaccag ttttgacgca atgacgtgca 1800
   cctttgtggt aaaaacagtt cggattcgcg accatatcgc aatctggatc gggcaaaaaa 1860
   cgccctttgt cgtttaacgc ttgctgtaac gtgcacgggc attgggccaa ctcgttagcg 1920
   aaattcttca aaaatctatc gttcataagc cactggtcgc atttcgcttc tgcccacttt 1980
   ttcccgtaga gtctctccca ttgcggagcg aagtaccatc cgagaggtat cggcttactc 2040
   catattacgg gggttatgct aagtccttga tattgggtcg ggtttgtcaa gttgatttga 2100
   atgaagccga aagtgagctc ggacaattca tagttgttcc ttgtccggaa attggccggt 2160
   tgtatgacgt agctgcctgt gtttgccaca cctgattcta acgtgtcaac gtaaagaagt 2220
   ttcggtgcta tattcgtctc tttgtacgcc caaatcgaaa tctgcaaagg agccgcagaa 2280
   ttggaagtta aattgtattt gttccacgtt attttgattt ctccaggata agcatcattc 2340
   acacttccat aatgatcgtt ctcagcaaaa gagatccttt gcgtcgccgt cgctggagtt 2400
   tcgacaaagt atagtccttt ccaaatatat ttgtcgtcgt ttaacgagat ttcgaagggt 2460
   atgtaacctt cagccatgat gaaaggctga acgcatatcg cccggttggt gtcgaccact 2520
   gttcctaaag tcggcgtcgt atcaaacctg catattatct tcatttcttt ggtaaaacaa 2580
   ggtccggtga tgttcacaat agtcccgccc aacatgtttc cagattcagg tgcgaaatta 2640
   aggtccaaac ttgctgcggc tatatccttg ttgcaagatc ccggaagaat gttttcgtca 2700
   atacggaata tgtgacgtcc aggaaatccg ttagcccaac cacgccccgt taaatctcga 2760
   atcgtacttg cctgcgaata aggtttatat tgataagtcc gagtcccgtt tccagcattg 2820
   aatccaacaa acgcaggagt acctccttca cctccagtgg tatcaccatt tgcttcagtg 2880
   tgcgttgtcc attgtatgtc cgcatagttg aacatcgcat atgtgatgac ttcatcagta 2940
   gccaatacaa gttggaatgt attcgttttg tagagactga tgtcgattcc tccggcaaaa 3000
   gatacgtttt tccaggtgat gatgactgcg tgttttggaa cgaatgagtc agaaccgatg 3060
   acgccctgac ggatgtccca cataagacgt tctctaactt caaccccgaa acggtcagtc 3120
   cttccttgta aatctctttc cattctgaag tagacaccag gaggtctttg atccacatct 3180
   gtatctttga tgagaccgat acggcatttg gaatagaaaa taccgataaa actcggatcg 3240
   ttctttttgg gccactcgac cgtcgggaaa tgaagggggt aactgtactg ttctggaggg 3300
   tccgaaaact cgagataacc gtttaagctt acgcgtgtgt agttgaagcg gaaaccgaaa 3360
   aagggaagtt ggaaattgaa gtttttatgg atttgggtag aggaaggatg aatgtctttt 3420
   tgtaggtcac cggaatcatt tggaccacct ttatcgaaga accaataaag gaagttggaa 3480
   cggattttcc gtaaacggtc ttcagttatc acgtacggga gttccccagg ccctgcacgt 3540
   tgcgttggag gtgccgtcct ttcatcatca aaatcgtatg gatttctcgg gttgtagggg 3600
   ttgtaaggag tcaaaaccct cgaaggctgc ctgtagttgt tgctcctcac gttgataact 3660
   tcattgaacc ccgggcgcac actttgtcct ctttggcctt ccgaactttt caaaaagtaa 3720
   aaaa 3724
<210> 705
   <211> 3845
   <212> DNA
   <213> Coleomegilla maculata
<220>
   <221> misc_feature
   <222> (1)..(3845)
   <223> RNAi target sequence ta22533.0010_vibdu
<400> 705
   caaatttcaa aatagaaatg aatctaaaag attagcaaat ccgatcatta gtgatgtaca      60
   atactgatga acggaacatt ttaacatggt gaaaattcga atattagtta ctttttgtat     120
   attcaaattt gttctgggtg aaggaagtgt cagttctaat gatgtggaaa gcattgttgg     180
   tgtgcctatg aaaaaagaag tacttggagc tataggagag gataatactg atacattccc     240
   caatgtaaat ggagccggtc ctgataataa ttacgcatcc gccccgattc ctggagcttc     300
   tgttactttc cataggcctt cagggaaata tataaccaac tatgataaca attacgatcc     360
   aatgcgctcg aatgttgcgc ccccatccgt agatcagagg ggtggttccg gcttacctta     420
   ctacataact gaagataggc tgaaagaaat acgttctctg tttatgtatc catactataa     480
   taagggtgga ggtggtgaca acgatggaga ttatcagaaa caaatccaag cctctacttc     540
   aatcgtacac aagaacctga attttcagct tcccttcttc gggttcaggt acaattacac     600
   tagagtttca ctcaacgggt atttggaatt cagcgatcca ccaccaacct atggcaaata     660
   tccactcatc tttcctagtc ctggatggcc ggaggtgaat gatccagctt tcatcggaat     720
   tttttatagc aaatgtcgta tgggtcttct aagggctgaa gatatagatc agaggaatcc     780
   tggagtgtat ttcaggcttg agcgggattt gaggagcaga accgaccaga tgggtgtcga 840
   aataagagaa aggaccaaat gggacatcag ggaaggtgtg ataggaactg acagtttcga 900
   tcctaagcat gcaattatta taacttggaa aaatgtttct ttcgctgggg gtatatctga 960
   cagtttgatg aggaccaaca ctttccaaat ggtgctcgtg acagatgaag ttttcactta 1020
   tgccattttc aactacctcc acctaggctg gaccactcat acggaagcaa gaggtgatac 1080
   gactacagga gaaggaggta ctccagcatt tgttggattc aatggaggaa atggcactag 1140
   aagttttgaa tacaaaccat acagtcaaga ttcagtaata cgagatctta catcaactgg 1200
   tttcgccaat ggttttccag gaaggcacat attcagaata gatgaaaaca ttttactagg 1260
   aacttgtaat aaggatatag atgccggtcg gttagatctc atgtttgctc cagaaagtgg 1320
   aaacatgtta ggaggaactg ttgttaacat aactggtcct tgcttcaaac cgaatcaaaa 1380
   aatcacctgc atctttgata ttgctccgcc agttattggc gttattgttg atagaaatag 1440
   agccatttgt atacaacctc cgctaatggc tgaaggttgg gttaggttcg agattgcagt 1500
   gggaaacgag aggtttaaat ggaaaggaag atattttgtc gaaacaccca caatggcagc 1560
   gcaaaagata ttcttttcag acatgacgta tcatgacaaa tctcctgcgg aaattcggat 1620
   atcatgggaa aataagaact taacatctaa cgtaaatgcg aatataagaa tttcactatg 1680
   gggttataga gaaacactcg tatctcccca attcgtttat ataacccaat tggcgacgaa 1740
   catacaaaac accggagaat acgtgattgt cccatctcaa tacagattga atgaaaacaa 1800
   gtttcaacaa gatataactg tcggtttcat ccagatcaac ctgactgatt cgatacctgt 1860
   tggtaacagt agcgtgaaaa taacaccgct catatggagc agacccattc ctttggcgtg 1920
   gtactttggt ccacaatggc agaggttata tggaaacagc tggccttcag ttctttgtga 1980
   taattggatc aaggatgatc gttatctgag taatttcgct catgaattac ctcaatgtcc 2040
   ttgtacttta caacaagctt tagttgataa aggaaggtac atgcctgggt tcgattgtga 2100
   cagagactcg aacccattat gctactacca agacaatgcc atacattgtg ttcgatcagg 2160
   tttaccaacg aatgaaggag gggaacagca atgctgctac gataaaaata atttccttat 2220
   gctgtcttat gaccaaatag gaggctcaaa tccgagacgt tctcacaatt taggtttcaa 2280
   cccttacaca gaatctacaa aagtacccgg tttgtcacag tggtaccacg acatgatccc 2340
   gaagtttgtc tgttgtcgct ggcaagacaa ccaagcagtc ggttgtgaaa ccttcaggtt 2400
   tgaaagacga ccaactcaag attgtacagc ttaccaagga cctgccgtgg ctggggtata 2460
   cggagatccc catgtaatca cgtttgatgg attggagtac acattcaacg gaaagggcca 2520
   gttcgttctt gtgaaaacca acgttgcctc gcaaaggtta gaggttcaag caaggttcga 2580
   gcagatgcaa ccaaatcaat tcaatgaggt tagagccact cagttgacgt ctatagccat 2640
   tagagggaat gcttcaacta tcattgaagt tagaataaga ccaaaacatg caaggtggag 2700
   gtatcggatg gatgttatag ccaacaagga gaagagacta ttttttgaca gaccttcatt 2760
   gaagttccaa catttctcag gattgacggt ttacacccca acatacatac tgaaccagtc 2820
   ggaagtgatt gtaatgcttg attctggagc aggtgtggaa atagtcgaaa gtttagggta 2880
   catgagtgca agagttttcc ttccatggtc gtttattaat aaaactgtcg gtctctttgg 2940
   aaactggagt tttgatataa cagatgattt cactcttcca gatggaacga aaaaacctat 3000
   catcaccaac ataaatgact gcaaagtgtt tatgacaact ttggaaaact gtggatgata 3060
   gacgactccg aagattcgca aacaggacaa tctcttttcg ttaacgaatt tggacgtact 3120
   gctgcatact ataacgacct cgactttgta ccagaattcc gaatgaacat tagagatctg 3180
   ctaataccaa ccaacgcatc ggattattat atacagcagg catacaaagc atgcaccaac 3240
   aaagactacc aatgcttgta tgattacgca atgacattgg acaaagactg cgcattctat 3300
   acaacaatat tcaaatcagg tttgaaaaat ataagagaag ctagcggtga acagactgta 3360
   tcatgcggtg ttctggaaac cccaaggttc ggttggaaat cgaatttttc ttcactcctg 3420
   gaagtaaagt tagtttcgaa tgcgctcaag atttcgttct tgacggggat cctagaagag 3480
   aatgcatgga aaatggacaa tggaacctac ccgaattcgg attcacggaa tgtttacgtc 3540
   aagaagaata ttcgttgaga caagcaggaa ctacatatgg aataatcctg gcagtgatga 3600
   ttcctcttat tcttctacta gttttctttg gattcagatt tatgcgtaaa ctatctgaac 3660
   aaaatgacca gaccaatcaa gaaaaactga ctatggagca acagaaaagg ttggcacagg 3720
   agagggcagc gaaaaaatta acagatgtcg atgatgagca taatgatcta ttttcagaac 3780
   attctcaatg ttatcagcaa tatcaatggt tttctacatg aatatatctt caaatgtgtg 3840
   aaaaa 3845
<210> 706
   <211> 982
   <212> DNA
   <213> Diabrotica undecimpunctata
<220>
   <221> misc_feature
   <222> (1)..(982)
   <223> RNAi target sequence ta01950.01_diaun
<400> 706
   tgaaaaagca aatcactacg acttgccgga ttttgtagta gtgttaattt tgtattagaa 60
   aatcaaaatg agttctattg gaactgggta cgatttgtca gcttcccaat tctctcctga 120
   tggaagagtt tttcaagttg aatatgcaat gaaagcagtt gaaaatagtg gcactgtaat 180
   aggtctccga ggtacagatg gaattgtatt agctgctgaa aagcttatta tgtcaaaatt 240
   acatgaaccc agtacaaaca aacgaatttt caacattgat aaacatatag gaatggcatt 300
   ttcaggttta atagctgatg caaggcaaat agttgagatt gctagaaaag aagcatcaaa 360
   ttataggcac caatatggat caaatattcc tcttaaatac ctaaatgata gagtaagcat 420
   gtacatgcat gcatacactt tatacagtgc tgtcagacca tttggttgca gtgtcatatt 480
   gtccagttat gaagataatg acccatctat gtttttgatt gatccatctg gagttagcta 540
   tggatacttt ggatgtgcta caggaaaagc taaacagtct gcaaagactg aaatagaaaa 600
   attgaagatg gggaatttaa catgcaaaga acttgttaaa gaagcagcca aaataattta 660
   tttggtccat gatgaattga aggataagaa ttttgaactg gaactttcat gggtatgcaa 720
   agatacaaat ggtttacata ccaaagtgcc tgagtcagtg tttgctgatg cagaaaaggc 780
   tgctaagcaa gcaatggaag cagattcaga atcagataca gaagatatgt aataactaca 840
   tttagttttt aatatttcac tgatggtggc tgttcttaca atattcgtgt gttatgtcta 900
   taaattatgt aatactgtga gaatttccat ttcaatgata ggtttataac tttttttcta 960
   ataaatacat aattttaaaa aa 982
<210> 707
   <211> 795
   <212> DNA
   <213> Diabrotica barberi
<220>
   <221> misc_feature
   <222> (1)..(795)
   <223> RNAi target sequence ta01738.01_diab
<400> 707
   tgacactgca accaaatggt ctaacagcac cgtaataggc cttcgaggta cagacggcat      60
   tgtattggct gctgaaaagc tcattatgtc aaaattgcat gaaccaagta caaataaacg     120
   aattttcaac attgataaac acataggaat ggcattttca ggcttaatag ctgatgcaag     180
   gcaaattgtt gagattgcta gaaaagaagc atcaaattat agacaccaat atggatcaaa     240
   tattcctctt aaatacctaa atgatagagt aagcatgtac atgcatgcat acactttata     300
   cagtgctgtt agaccatttg gttgcagtgt catcttggcc agttatgaag atagtgaccc     360
   atctatgtat ctgattgatc cgtctggagt tagctatgga tactttggat gtgctacagg     420
   taaagcaaaa cagtctgcaa agactgaaat agaaaaattg aagatgggga atctaacttg     480
   caaagaactt gttaaagaag cagccaaaat catttatttg gtccatgatg agctaaagga     540
   taagaatttt gaactggaac tttcatgggt atgcaaagat acgaatggtt tacataccaa     600
   agtgcctgaa tcagtgtttg ctgatgcaga aaaagctgcc aaacaagcaa tggaagcaga     660
   ttcagaatca gatacagaag atatgtaata actagataca tttagttttt aatatttcgc     720
   tgatgtggct gttcttacaa tatttcatgt gttatgtcca tatattatgt aatactgtga     780
   gaatttccat ttcaa                                                      795
<210> 708
   <211> 993
   <212> DNA
   <213> Epilachna varivestis
<220>
   <221> misc_feature
   <222> (1)..(993)
   <223> RNAi target sequence ta26097.001_epiva
<400> 708
   atttgtatat ttataatcat agataatata taatatattt aggttttcaa tcaagtaata      60
   ccaaattatt aatataaaat gagttctatc ggcactggct atgatctttc ggcttctcaa     120
   ttttctcctg atggtagagt ttttcaagtc gaatatgcta tgaaagctgt ggaaaatagt     180
   ggaacagtta taggtcttaa aggaactgat ggggtagtac ttgcagcaga aaaattaatc     240
   atgtctaaac tacatgaatc tggcaccaac aaaagaattt ataatataga tacacatatt     300
   ggaatgtgtt tttctggtct tattgctgat gctaggcaat tagttgatat agcgagaaag     360
   gaggcatcaa attatagatc acagtatggt gtaagcattc cactcaaata tcttaatgac     420
   agagtcagca tgtatatgca cgcatataca ctttacagtg ctgtaagacc ctacggctgt     480
   agcattattc ttgctagtta tgaggaagat cagcctatga tgtatttaat tgacccctca     540
   ggtgtaaact atggatttaa tggttgtgct actggaaaag caaagcaatc tgctaaaaca     600
   gagattgaaa aactgaaatt gggtgagctg agttgcaaag agttggttag agaagctgcc     660
   aaaattattt acttagtcca tgatgagttg aaggataaaa attttgagct tgagttatca     720
   tgggtgggaa aagatagcaa tggtagacat gaaaaagttc ccagccacgt tttagctgat     780
   gctgagaaag cagcaaaaca agcaatggag gcagattccg attctgatac tgaggatttg     840
   tagatataca tttgattatt ttataagtgc agttctaata aagcgtaatt tctttcaatg     900
   tgtcatctta aataacgccc tttttcttgt aattaacgtc aaagaaaaaa ctcattacaa     960
   acaatttgtt ctgtaccata atgtgttcat tgg                                  993
<210> 709
   <211> 936
   <212> DNA
   <213> Leptinotarsa decemlineata
<220>
   <221> misc_feature
   <222> (1)..(936)
   <223> RNAi target sequence ta37784.0003_lepde
<400> 709
   caactatttg agtatttgac agatgagttg tattttaaga aagaagcaaa tcacgctagt        60
   tcaaaactcc aaaactaaat ttttaaaatc gaatttaaac agtcaatatg agttcaatag       120
   gaactgggta tgacttgtcg gcttcgcaat tctcacctga cggaagagtc ttccaagtcg       180
   aatacgccat gaaagccgta gaaaacagtg ggaccatcat tggcctcaag ggcactgacg       240
   gaatcgtcct ggcagccgag aagctcatca tgtctaaact gcatgaaccg ggaacgaaca       300
   ggagaatctt caatatcgat agacatattg gaatggcgtt ctctggcctg atagcggatg       360
   ctcggcagat cgtcgaaatc gctaggaaag aggcagctaa ttataggctc cagtacggca       420
   ttaacatacc tctgaagtac ttgaacgaca gagtgagcat gtacatgcat gcatatacct       480
   tgtacagtgc cgttcggcct tacggatgca gcgttatatt agctagttac gatgaggatg       540
   agcctcagat gtacctaatt gatccatcag gagttagcta tgggtttcac ggctgcgcaa       600
   cagggaaagc gaaacagtcg gccaaaaccg aaatagagaa gctcaaacta ggcaatttga       660
   catgtaaaga attggtgaaa gaagcagcaa aaataatata catggtccat gacgaactca       720
   aagacaaaaa ctttgaaatg gagctttctt ggatatgtaa agaaagcaat gggttacacg       780
   tgaaagttcc tgaatccgta tatttggagg cagaacgggc cgccaagcaa gccatggaag       840
   ctgattcgga atcggataac gaggacatgt gaggctttgt cagttagtat taagattatg       900
   gcttagttgt aagatgtctt ggctaaatct tttttt                                 936
<210> 710
   <211> 927
   <212> DNA
   <213> Orius insidiosus
<220>
   <221> misc_feature
   <222> (1)..(927)
   <223> RNAi target sequence ta09672.01_oriin
<400> 710
   aggtaacatt acatttgagg ccgcatattt tgctttatca ttactttaaa tttaatttta 60
   cttttatttc ttgttaaaac gtttgtttga catcaaaaat gagttccatc ggtaccgggt 120
   atgatttgtc ggcgtcacaa ttttcgcccg acggacgagt ctttcaagta gaatacgctc 180
   agaaagccat tgaaaacagt gggacggtaa tcggcctgag aggtaaagat ggtgttgtct 240
   tggctgtcga aaagcttgtc acgtcgaaac tgtatgaacc aggagccaat aaaaggatat 300
   tcactattga caaacatatc ggaatggctg ttgctggctt gatatctgat gcgaggcagg 360
   tggcggaagt cgctaggacc gaagctgcta attatcgctc ccaatacgga agtaacatcc 420
   cgttgaagta tttgaacgag cgagtctccg gttatatgca tgcatatact ttatacagcg 480
   ctgtccgacc tttcggctgc agcgtcatcc tcagcactta caacgagcga gatggacccg 540
   aaatgtacat gattgaccca tcaggtgtct catacggtta tttcggatgc gctgtcggca 600
   aagcgaaaca agccgccaaa accgaaatcg aaaaattaaa attgaaagat ttaacatgca 660
   aagaattagt caaagaagct gccagaataa tttatttggt ccatgatgag cttaaagata 720
   aacagtttga acttgaaatg agctggatcg gcgctcatac aaacggtgta aaccaagttg 780
   tcccgccgac cgtccacgcc gaagctgaaa aagatgcgaa agctgctatg gaagaagatt 840
   cagattctga aactgaagat atttaactta attatactat gttctaatta acttttttcc 900
   attaataact ttttttttct attcaaa 927
<210> 711
   <211> 1115
   <212> DNA
   <213> Coleomegilla maculata
<220>
   <221> misc_feature
   <222> (1)..(1115)
   <223> RNAi target sequence ta36121.0001_vibdu
<400> 711 tagaggctgc aacagtgatt tgtagaattt tgcaa 1115
<210> 712
   <211> 853
   <212> DNA
   <213> Diabrotica undecimpunctata
<220>
   <221> misc_feature
   <222> (1)..(853)
   <223> RNAi target sequence ta07256.01_diaun
<400> 712
   cattattgta ccgtgttgtc tagcggatat tttagattta acaatcttat tttataaaat      60
   aattagttaa aatgatgcaa accgccaata atgtatatta tcccgattat tccactgctc     120
   caatgcaacg tcaaattaat ccttatgccg acaatggagg gagtgtagta gcaatagcag     180
   gtgaagactt tgtaataatt ggtgcagata cacgtttgag tgctggattt tccatttaca     240
   ctagagaaca aaacaaacta ttcccgctat caggcactac agttttaggc tgtgcaggat     300
   gttggtgtga tactctaaca ttaaccagaa tccttaaatc tcgcatgcag atgtaccaac     360
   aagagcataa caaaacaatg tctacaactg catgtgccca aatgttgtca accatgctct     420
   actacaagag attctttcct tattatatat caaacattct tgttggttta gataatgaag     480
   gaaagggctg tgtttacagc tatgatccaa tcggacattg tgaaaaagct acttatagag     540
   caggtggttc ggctggagct ctgctccaac cccttttgga caatcagatt ggacaaaaga     600
   acatgcttaa aacatctgga gaacctctta gtcaggagaa agctttggct acactgaaag     660
   atgtatttat ttctgctgct gaaagagata tctacactgg agatagtgta cttataaata     720
   ttattactaa agatggagta aaggaagagt ccttccagtt gagaagggat tagaagcaag     780
   tgtttttgtt tatattattt tcttatgtgt aattcaaata tactttctaa ataaacaatt     840
   ttaagataaa aaa                                                        853
<210> 713
   <211> 856
   <212> DNA
   <213> Diabrotica barberi
<220>
   <221> misc_feature
   <222> (1)..(856)
   <223> RNAi target sequence ta07284.01_diab
<400> 713
   gcataggggt taatttgacg ttgcattgga ccagcggatt ttagattcaa caagcttatt      60
   ttataaaata attagttaaa atgatgcaaa cagctaataa tgcatattat cccgattatt     120
   ccactgctcc aatgcaacgt caaattaacc cctatgcaga taatggaggg agtgtagtag 180
   caatcgcagg tgaagacttt gtaataattg gtgcagatac acgtttgagt actggatttt 240
   ccatctatac cagagaacaa aacaaacttt tcccactatc aggcactaca gttttgggtt 300
   gcgcaggatg ttggtgtgac actctaacat taaccagaat ccttaaatct cgcatgcaga 360
   tgtaccaaca agagcataac aaaacaatgt ctacaactgc atgtgcccag atgttgtcta 420
   ccatgctcta ctacaagaga ttctttcctt attatatatc aaacattcta gtaggtttag 480
   ataatgaagg aaagggctgt gtttacagct atgatcctat tggacattgt gaaaaagcta 540
   cgtatagagc aggtggttca gctggagctc ttcttcagcc tctgttggac aatcaaattg 600
   gacagaagaa catgcttaaa acatctggag aacctcttag tcaggagaaa gctttgtcta 660
   cccttaaaga tgtatttatt tctgctgctg aaagagacat ctacactgga gatagcgtac 720
   ttataaatat tattactaaa gatggagtaa aggaagagtc cttccagttg agacgggatt 780
   agaagcaagt ggttttgttt atattttctt atgtgtaatt caaattaata tactttctaa 840
   ataaacaatt ttaagt 856
<210> 714
   <211> 796
   <212> DNA
   <213> Epilachna varivestis
<220>
   <221> misc_feature
   <222> (1)..(796)
   <223> RNAi target sequence ta27792.001_epiva
<400> 714
   agtgagtttg ttgttctagt agagtaaata ccgaataaaa atgcattcaa taatgaatga      60
   ttctcgtcca tttttcgaag attattctgt taagccaatg cagcgtcaat tcagccccta     120
   cgctgataat ggagggagca ttattgcaat ttctggtaac gacttcgtgg tgattggtgc     180
   agatactcgt ttgagttctg gattttcgat atacactcgg gatcaaaata agttattcaa     240
   actctcagat aatacagttt tgggatgttc tggatgttgg tgtgatactc tttctttaac     300
   tcgtattttg aacgcacgta tgcaaatgta tgaacaagaa cacaataaga aaatgtccac     360
   tccagcttgt gctcaaatgt tatcgaccat gttatattac aagcgattct ttccgtatta     420
   catttctaac atattagttg gtttggattc tgagggaaaa ggatgtgttt atagttacga     480
   tcctattggt catactgaaa gatctagtta tagagctgga ggatcagcag gtgccttact     540
   acaacccctc ttagataatc aagttggtat gaaaaatatc cagaatgcac ataatgaacc     600
   actaactatt gaaagagctg ttggaatttt aaaggataca tttatatctg cagctgaaag     660
   agatatttat acaggtgata gcattaagat caatgtcatc actaaagatg gcatcaaaga     720
   agatactttt gagttgagga aagattaaat tgtatacttt ttaatatctt caataaaaat     780
   ttaattataa aaaaaa                                                     796
<210> 715
   <211> 842
   <212> DNA
   <213> Leptinotarsa decemlineata
<220>
   <221> misc_feature
   <222> (1)..(842)
   <223> RNAi target sequence ta37916.0001_lepde
<400> 715
   tttttttata ttttcggctt gtcattctag ttgacttgtt taaaagtaga aagtatttgt      60
   gtttcctcca ctataaaatg caaacagcaa tgaacgctta ttatcctgat tattcaacgg     120
   taccggttca gcagtctttc agcccctatg ctgataatgg agggagcgta gtggccatag     180
   ctggagatga tttcgtggta gtgggagcag atacacgtct aagcgccggc ttctccatct     240
   acactagaga ccaaaataag ttgtttcctc tttcaaaaac aacggttctc ggatgctcag     300
   gttgctggtg tgacacgcta acgttaactc gtattttgaa gtctcgcatg cagatgtacg     360
   agcaggagca taataaagta atgtcgacca ctgcttgcgc gcagatgctc tccaccatgc     420
   tctactacaa acgctttttt ccttattaca tctctaatgt tctcgtgggt ctcgacaacg     480
   aaggaaaagg ttgtgtttac agctacgacc ctattggaca ttgtgaaaag actacgtaca     540
   gagctggtgg ttctgctggg gctctgctcc agcctctgtt ggataatcaa atcggacaga     600
   agaatatgga gatagtgtct accgagccct tgtcgaaaga gagagctctg gctatattga     660
   aggatgtctt catctctgca gccgagagag acatatacac cggagatagt gtttctatca     720
   atatcataac taaggatggt gtgaaagagg aatcatttga attgaggaaa gattagagga     780
   attttatatt gaattttcct tcatgaattt ttgattatat gatttaagta aaaaaaaaag     840
   tc                                                                    842
<210> 716
   <211> 755
   <212> DNA
   <213> Orius insidiosus
<220>
   <221> misc_feature
   <222> (1)..(755)
   <223> RNAi target sequence ta09310.01_oriin
<400> 716
   cgcttccgat actcgtttga gccaaagcga aaaatttttg tttggtcatc tgctgtacgg      60
   tattagctga agaaatgtct ttttcaccgt attctgataa tggagggagc atcgttgctg     120
   tcgccggtga cgattattcg atcatcgctt ccgatactcg tttgagcgct gggtattcga 180
   tttatacgcg tgagcagtcg aaattgttcc cgctgtcgga aaaagcgatt ttgggaagcg 240
   ctggatgttg gtgtgacgtt ctttctcttg ctagattgct ccagacgcgc attaaaatgt 300
   acttgtatga gcataataag gcgatgtcaa ctccagctat agcgcagatg ctatctatca 360
   taatgtacta taaacgattc tttccgtatt acacgtcgac aattttggcc ggattagatt 420
   ccgagggtaa aggaatcgtc tacagctacg acccaatcgg tcatatggaa agctctaaat 480
   atcgtgctgc aggttctgca ggagcacttc tccagcctct tcttgacagc cagctgggca 540
   agaaaaacca ggagaatgtc gtgaataaat cgtacagctt ggaagaagct ttggcgatcg 600
   tgaaggacac ttttatttcg gctgctgaaa gagatattta tactggagac agcatcgctt 660
   tagccatcat cactcaagat ggcgtcaaaa acgagacttt ccaactgagg aaagattaaa 720
   aacatattat ttgtactatt tttgctcttt ttgaa 755
<210> 717
   <211> 813
   <212> DNA
   <213> Coleomegilla maculata
<220>
   <221> misc_feature
   <222> (1)..(813)
   <223> RNAi target sequence ta23917.0001_vibdu
<400> 717
   gaaaatgtct ataatgaatg attctcgtcc tattttcact gattattctc aaaaacctat      60
   gcagcgacaa tttagtccct atgccgataa cggagggagc atcattgcta tagctggaga     120
   cgattttgtt gttatcggag cagatactcg cttgagtgct ggttattcca tatacacccg     180
   cgatcaaaat aaactcttta aattgtctaa cacaacagta ttgggttgtt ctggttgttg     240
   gtgtgatact cttactctaa caaggattct gaatgcccgt atgcaaatgt atgaacaaga     300
   gcataataag attatgtcta ctcctgcttg tgctcaaatg ctttcaacca tgctatatta     360
   taaacgattc tttccttact atatatccaa tatattggca ggcttggact ctgacggaaa     420
   aggctgcgta tacagttatg atccaatagg tcatacagaa cgctctgctt atagagctgg     480
   tggatccgca ggtgttctac tgcaacctct tttagacaac caaattggcc ttaaaaatat     540
   ggttaatact ccgaatttgc ctctgaccat tgaaagagct gttggaattt taaaagacac     600
   attcatctct gctgcagaaa gagatataca cactggtgat agcatcaaaa ttaacataat     660
   aactaaagat ggaattaagg aagacacttt tgaattgaga aaagattaaa catttttatt    720
   ttttgtcaac aatgggtaaa aattatctaa ttttgaggat gttattgtga gtgtctctca     780
   agtatttaat tataactccg aaatataaaa aaa                                  813
<210> 718
   <211> 785
   <212> DNA
   <213> Diabrotica undecimpunctata
<220>
   <221> misc_feature
   <222> (1)..(785)
   <223> RNAi target sequence ta08127.01_diaun
<400> 718
   tttttttagt gcaaaactta ttttttttat tttaatatat accaaaacat accaaagctt      60
   aaaaaataat taattaacct tcataaaatt tgggcaattc atttcctaaa ataactcttc     120
   gttcaatacc agcttcattg ataattccca gtctgactac acctcctgaa gaaccatctc     180
   tggacatagc aagtgccaat gtatttgtga caaacttcac acattcttct ttgctcatat     240
   tgggtttgaa attggcatct acataaccat aaacataact ggaacctgat cctcctattg     300
   acacttcttg cctcacacac atcccaccaa ttggtatgga atagacttga ccgcctttct     360
   tcttatccca acctgctacc agtattccag ccattagtga atctctataa ttgtagcaaa     420
   gttcttggaa aatggcagca cctacttcta ctttgggttc ttcaccaagt tccataccat     480
   gaaaattaag atgataagca acaatgtctg caattgcttg tgtatctgct gcagatccgg     540
   aacgacaaca gtagatatgg tcagtgactt tggtgagttt gtctgctaca cggtttgcta     600
   tgtaagcccc agtagttgtg cgagaatctg ctcctataac aacgccgccg tcaaactctg     660
   cggccataat agaggtgcct gtactgtgag cagcatctct ccaatcatca ggaccagtca     720
   ttgcaccaaa ctcattcata agaggcattt ttataagttt aatttcaaga atacaagctg     780
   agaaa                                                                 785
<210> 719
   <211> 842
   <212> DNA
   <213> Diabrotica barberi
<220>
   <221> misc_feature
   <222> (1)..(842)
   <223> RNAi target sequence ta05581.01_diab
<400> 719
   tttttggtgt aaacctgatt tttttatttt aatatatacc aaaacatacc aaagcttaaa      60
   aaataattaa ttaaccttca taaaatttgg gcaattcatt tcctaaaaca actcttcgtt     120
   caataccagc ttcagtgatg atccccagtc tgactacacc tcctgaagaa ccatctcttg     180
   acatggcaag tgccaatgta tttgtgacaa acttcacaca ttcctctttg ctcatgttgg     240
   gcttgaagtt ggcatctacg taaccataaa cataactgga gcctgatcct ccaattgaca 300
   cttcttgtct cacacacatc ccaccaattg gtatggaata gacttgtccg cctttctttt 360
   tatcccaacc tgctaccagt ataccagcca ttagtgaatc tctataattg tagcaaagtt 420
   cttggaaaat ggcggcacct acttgtactt tgggttcttc accaagttcc ataccatgaa 480
   aattaagatg ataagcaaca atgtctgcaa ttgcttgtgt atctgctgca gatcctgaac 540
   gacaacagta tatatggtca gtgactttgg tgagtttgtc tgctacccgg tttgcaatgt 600
   aagccccggt agttgtgcga gaatctgctc ctataacaac gcctccatca aactctgcgg 660
   ccataataga ggttcctgta ctgtgagcgg catctctcca atcattagga gcagtcattg 720
   caccatactc agtcataaga ggcatttttt acaagtttaa tgaaaagaat acaagcttag 780
   aaaaattact tgtaatccta caatccaata ttttgccagt caccagctcc tataacaacg 840
   cc 842
<210> 720
   <211> 935
   <212> DNA
   <213> Epilachna varivestis
<220>
   <221> misc_feature
   <222> (1)..(935)
   <223> RNAi target sequence ta23856.001_epiva
<400> 720
   cgatcttaaa aatcggattt atatcgtgat tattgtaata attattctca cagctagccg        60
   aataatttcg tttcgcgtta ctggtaacac tgaaaatgca tttttcattt tgtggttatg       120
   attatttgga gaaaaataat ttgtcatagt cattgagtac aaattgtgtt cgatatattt       180
   gcaatattag attcattaaa cttgatttta tactgaaaat cacaaactac catcgaaaac       240
   atggactccg attggatgaa tgcaccacac agtacaggga cttctattat ggctgctgag       300
   tttaatggtg gagttgttat tggtgccgac actagaacaa ctacaggggc atacatagcc       360
   aaccgagtaa cggataaact aacaaaagta acagatcaca tttattgttg tcgttctggt       420
   tcagcagcag atactcaagc tattgctgat atagtttctt atcatctagg atttcatgga       480
   attgagttgg gagaagagcc acttgtagaa acaagtgcag ctgtgtttca agaactttgt       540
   tacagctatc gtgattcttt aatggctgga attctagtag caggttggga taaacgtaaa       600
   ggtggtcagg tttattctat tcctataggt ggaatgtgtg taagacagag cgtatccata       660
   ggtggttcag gatcaagcta tgtttatggt tatgttgatg ccaattataa acctggaatg       720
   tcaaaagaag cttgtgtaga ctttgtttca aaaactttgg cacttgcaat gtcaagagat       780
   ggttcttctg gtggttgtat tcgtattgga gtcattacaa aggatggcat tgaacgcaag       840
   gttattctgg gttctgaggt ccctaaattc tatgaagatt gattttattt gtattcaatt       900
   aaataataaa taaaaatgtt aacatctaaa aaaaa                                  935
<210> 721
   <211> 1264
   <212> DNA
   <213> Leptinotarsa decemlineata
<220>
   <221> misc_feature
   <222> (1)..(1264)
   <223> RNAi target sequence ta33564.0001_lepde
<400> 721
   tttacataat tacggaatac tgtgccatag attacagaaa tttaaatttt agtgttctgt       60
   ggttctaacc tagcaaataa tttgtagagt cattttttga cttatttttt gttagtgatt      120
   ttgtattcag attgtctccg ttcgtaaaaa tgctactgaa tcaaatatcc atagcaggtg      180
   ccgatgactg gagaaatgcc gctcatagca ctgggacttc tattatggca gcagaattcg      240
   atggaggagt aattattggt gctgattccc gtacaactac aggggcgtac attgcaaatc      300
   gtgtaactga caaactaact aaagtaactg accatatata ttgttgtcga tctggatcag      360
   cagcagatac tcaagccatt gctgatattg tatcgtacca tctgaacttc catggtatgg      420
   aactaggaga agagcccctt gtagaggtgg gggctgctat cttcagggag ctttgctaca      480
   actataggga ttctctcatg gcgggaatcc tagttgctgg ctgggacaaa aaaaaggggg      540
   gacaaattta ttctattcct atcggaggga tgtgtgttag gcaagcggtt tctattggag      600
   gttcaggttc cagttatgtt tatggttatg tagatgctaa tttcaaaccc aagatgtcaa      660
   aggaggaatg tgcgaaattt gtcacaaata ctctggctct tgcaatgtct agagatggat      720
   catctggagg tgttatccgt ctaggtataa ttacggagtc gggaattgaa cgtcgtgtca      780
   tcttaggcga tgatctaccc aagttctatg aaggatagag catataataa tcataatttt      840
   tcattttgta attggtgtca ataaatcaaa taaaataagg ttttctttaa aaaacaatta      900
   tatgattata aaatacatgt taatgaacaa tgaatttgaa agagaaaaaa gatggttgtg      960
   taccatgaag gcactaattc gtatctttat taatgtagtc tgtattttta gagagaatcg     1020
   tgacaaaatt agtcgcatgg cgccatttgg caccaacata ttttgaattc ctttattttt     1080
   tctggaactc tggtaataaa attatttctg ttatatctta gtattcaaaa tgccactgaa     1140
   aaaattgaat cattattttc ttaccaattc tttttcttaa gaagatatcg ttacagcact     1200
   accatcacca caaacagatt taccattttt tttgtcaacc ttctaaattt tacagtaaat     1260
   gtat                                                                  1264
<210> 722
   <211> 912
   <212> DNA
   <213> Orius insidiosus
<220>
   <221> misc_feature
   <222> (1)..(912)
   <223> RNAi target sequence ta09100.01_oriin
<400> 722
   ttcagaatta gctatttaac cgaaggcgac tcagcgaatt tactaagtga aggtgaacta 60
   ttctgtggag aaaaatggcc aaagtcgctg ttcatcatga tcgtttcggt gcccctgcaa 120
   tgactgagga accagtttct actggaacta caattatggc ttgtactttt gacggaggcg 180
   tcatcatcgg tgcggattct aggacgagta gcggacctta cgtcgccaat cgcgtttccg 240
   acaaactgac aaaaatctcc gatcacattt actgttgtag gtcaggatcg gccgctgaca 300
   ctcaagctat cgctgatatc gtcaaatatc acctttcttt ttttaagctt gaaactggtg 360
   aagaaccgct tgttgcaact gctgcagcga ttttccgtga gctttgttat aactacagaa 420
   atcgcttgat ggctggcatc atcgtcgctg gatgggacag agttaaaggc ggacaggttt 480
   atacgatacc tttgggtggt atgataatcg agcagaaatt cgcaatgggc ggttctggaa 540
   gttcttatgt ttatggcttc actgatgcca actatagaga aaacatgagt tcagaagaat 600
   gcaaacagtt cattaaacaa gcaatcgctc ttgccattcg tcgtgacggc tcttccggag 660
   gtgtcataag aatgggtctc atcacaaaag atggtattaa acgagaaatt ttttcgggta 720
   aagattaccc ttcatattta actccagcta aagattagaa tatctttttg tattccttga 780
   gctgtaaaat ttaagtacat tcatttataa ctttttgaaa tgattttctt tcatttatta 840
   acaaaataat ccagtactaa taatactcca gcaagtacta tttcaataca catctttatc 900
   acaaaggcaa aa 912
<210> 723
   <211> 686
   <212> DNA
   <213> Coleomegilla maculata
<400> 723
   cctcacagta caggaacctc tatcatggct gctgaatttg agggtggtgt agtaattggt 60
   gctgattcta gaaccaccac aggaacatat attgccaatc gagtaactga taagcttaca 120
   aaaataactg atcgtatata ttgttgtcga tctggttcag ctgctgatac ccaagccatt 180
   gcagatattg tatcttatca tttaggtttc catgggatgg aattgggaga agaaccactt 240
   gtagaaacta gtgctgcagt attccaagaa ctatgctata attatcgaga ttctttgatg 300
   gctggtatac ttgtagcagg atgggataag cgaaagggag gtcaaattta ctcaattcct 360
   attgggggaa tgtgtgtgag gcaaaaagtc tctattggag gttctggttc tggttttgta 420
   tacggctatg tagatgctaa ctataagtct ggaatgacta aagaagcttg cgttgatttc 480
   gtaattaaaa ctttggcact tgctatgtcg agagatggct catcaggtgg ttgtgttagg 540
   attggagtta taactaaaga tggtatcgaa cgtaaagtaa ttttgggtac agaattaccc 600
   aaattttatg aagattaata tttcataata tattttgtct taattcaata cttcattcaa 660
   taaaattatg aaattttcga aaaaaa 686
<210> 724
   <211> 250
   <212> DNA
   <213> Diabrotica virgifera
<400> 724
   tcaacgtcat ctcagtttgg tttattgttg ggtgtaatgt ttaaataaaa taattattat 60
   tttgtaaacc tgtgccatta aacaaataaa gcgatggtgc taattgcagc agcagtctgc 120
   acgaaagcag gcaaaacaat tgtgtctcga caatttgttg aaatgaccaa agctagaata 180
   gaaggtttgt tggctgcctt tcctaaatta attcctacag gaacccagca tacatttgtg 240
   gaaacagatt 250
<210> 725
   <211> 251
   <212> DNA
   <213> Diabrotica virgifera
<400> 725
   acttcattcc agtcttgtga tatttagtgc ttacttggta cagcagtttc agtgctgtgc 60
   tttagaataa tttatttttt aacatttata tagaaatcaa atactaacca atcaacatgt 120
   gtgaagaaga agttgccgct ttagtcgtag acaatggatc cggtatgtgc aaagctggtt 180
   ttgctgggga tgatgcacct cgtgctgtat tcccttcaat tgttggacgc ccaagacatc 240
   agggtgtgat g 251
<210> 726
   <211> 249
   <212> DNA
   <213> Diabrotica virgifera
<400> 726
   gtaggaatgg gacaaaaaga ttcctatgta ggtgatgaag ctcaaagtaa aagaggtatc 60
   cttaccttaa aataccccat cgagcacgga atagtcacaa actgggatga tatggagaaa 120
   atttggcatc atacattcta caatgaactc agagcagccc cagaagaaca ccctgttctg 180
   ttgacagaag ctcctctcaa ccccaaggcc aacagggaaa agatgacaca aataatgttt 240
   gaaactttc 249
<210> 727
   <211> 250
   <212> DNA
   <213> Diabrotica virgifera
<400> 727
   acacttacac ccttcaaaaa ctcatttata aactaaaaat gggtgcatct gtaattcctt 60
   tcatagtttt cactgtatta tggggaggag ttggagctgt tctacccatt tttgtaccta 120
   aaggacccaa tagaggaatt atccaagtta ttcttatgat aacaggtgtg tcgtgctggt 180
   tgttctggct gtgttgttac gtagctcaaa tgaatcctct gatcggtcct aaactagaac 240
   aaaacatcgt 250
<210> 728
   <211> 211
   <212> DNA
   <213> Diabrotica virgifera
<400> 728
   gataataagt tcgatttttt acgaaaatga caagtatcga gactgtgggg accattgtcc 60
   tgaaattgct gaagttggtg atcaatttga tatgtctcat cttgtaccga accggatatc 120
   aaggctactt cttgggagta ggaggaacct ggaatctaaa cgaagaaaaa aatcccgatg 180
   cagaaattgt ggcttccggc gtattcgtag g 211
<210> 729
   <211> 3114
   <212> DNA
   <213> Diabrotica virgifera
<400> 729
   ggccagaatg gcccggaccg gcggccgccc tgcagctcta gagaaacttc gaaacgcgtg      60
   gaccgaagct tgcatgcctg cagtgcagcg tgacccggtc gtgcccctct ctagagataa     120
   tgagcattgc atgtctaagt tataaaaaat taccacatat tttttttgtc acacttgttt      180
   gaagtgcagt ttatctatct ttatacatat atttaaactt tactctacga ataatataat     240
   ctatagtact acaataatat cagtgtttta gagaatcata taaatgaaca gttagacatg     300
   gtctaaagga caattgagta ttttgacaac aggactctac agttttatct ttttagtgtg     360
   catgtgttct cctttttttt tgcaaatagc ttcacctata taatacttca tccattttat     420
   tagtacatcc atttagggtt tagggttaat ggtttttata gactaatttt tttagtacat     480
   ctattttatt ctattttagc ctctaaatta agaaaactaa aactctattt tagttttttt     540
   atttaataat ttagatataa aatagaataa aataaagtga ctaaaaatta aacaaatacc     600
   ctttaagaaa ttaaaaaaac taaggaaaca tttttcttgt ttcgagtaga taatgccagc     660
   ctgttaaacg ccgtcgacga gtctaacgga caccaaccag cgaaccagca gcgtcgcgtc     720
   gggccaagcg aagcagacgg cacggcatct ctgtcgctgc ctctggaccc ctctcgagag     780
   ttccgctcca ccgttggact tgctccgctg tcggcatcca gaaattgcgt ggcggagcgg 840
   cagacgtgag ccggcacggc aggcggcctc ctcctcctct cacggcaccg gcagctacgg 900
   gggattcctt tcccaccgct ccttcgcttt cccttcctcg cccgccgtaa taaatagaca 960
   ccccctccac accctctttc cccaacctcg tgttgttcgg agcgcacaca cacacaacca 1020
   gatctccccc aaatccaccc gtcggcacct ccgcttcaag gtacgccgct cgtcctcccc 1080
   cccccccctc tctaccttct ctagatcggc gttccggtcc atgcatggtt agggcccggt 1140
   agttctactt ctgttcatgt ttgtgttaga tccgtgtttg tgttagatcc gtgctgctag 1200
   cgttcgtaca cggatgcgac ctgtacgtca gacacgttct gattgctaac ttgccagtgt 1260
   ttctctttgg ggaatcctgg gatggctcta gccgttccgc agacgggatc gatttcatga 1320
   ttttttttgt ttcgttgcat agggtttggt ttgccctttt cctttatttc aatatatgcc 1380
   gtgcacttgt ttgtcgggtc atcttttcat gctttttttt gtcttggttg tgatgatgtg 1440
   gtctggttgg gcggtcgttc tagatcggag tagaattctg tttcaaacta cctggtggat 1500
   ttattaattt tggatctgta tgtgtgtgcc atacatattc atagttacga attgaagatg 1560
   atggatggaa atatcgatct aggataggta tacatgttga tgcgggtttt actgatgcat 1620
   atacagagat gctttttgtt cgcttggttg tgatgatgtg gtgtggttgg gcggtcgttc 1680
   attcgttcta gatcggagta gaatactgtt tcaaactacc tggtgtattt attaattttg 1740
   gaactgtatg tgtgtgtcat acatcttcat agttacgagt ttaagatgga tggaaatatc 1800
   gatctaggat aggtatacat gttgatgtgg gttttactga tgcatataca tgatggcata 1860
   tgcagcatct attcatatgc tctaaccttg agtacctatc tattataata aacaagtatg 1920
   ttttataatt attttgatct tgatatactt ggatgatggc atatgcagca gctatatgtg 1980
   gattttttta gccctgcctt catacgctat ttatttgctt ggtactgttt cttttgtcga 2040
   tgctcaccct gttgtttggt gttacttctg caggtcgact ttaacttagc ctaggatcct 2100
   aactaactag gataataagt tcgatttttt acgaaaatga caagtatcga gactgtgggg 2160
   accattgtcc tgaaattgct gaagttggtg atcaatttga tatgtctcat cttgtaccga 2220
   accggatatc aaggctactt cttgggagta ggaggaacct ggaatctaaa cgaagaaaaa 2280
   aatcccgatg cagaaattgt ggcttccggc gtattcgtag gtaattgaga attcgatatc 2340
   aggtccgcct tgtttctcct ctgtctcttg atctgactaa tcttggttta tgatcttgat 2400
   gatttagctt gactatgcga ttgctttcct ggacccgtgc agctgcccat cgaccctcaa 2460
   ttacctacga atacgccgga agccacaatt tctgcatcgg gatttttttc ttcgtttaga 2520
   ttccaggttc ctcctactcc caagaagtag ccttgatatc cggttcggta caagatgaga 2580
   catatcaaat tgatcaccaa cttcagcaat ttcaggacaa tggtccccac agtctcgata 2640
   cttgtcattt tcgtaaaaaa tcgaacttat tatcctagtt agttaggtta acctagactt     2700
   gtccatcttc tggattggcc aacttaatta atgtatgaaa taaaaggatg cacacatagt     2760
   gacatgctaa tcactataat gtgggcatca aagttgtgtg ttatgtgtaa ttactagtta     2820
   tctgaataaa agagaaagag atcatccata tttcttatcc taaatgaatg tcacgtgtct     2880
   ttataattct ttgatgaacc agatgcattt cattaaccaa atccatatac atataaatat     2940
   taatcatata taattaatat caattgggtt agcaaaacaa atctagtcta ggtgtgtttt     3000
   gcgaattatc gatgggcccc ggccgaagct ggccgcgggc atgtggtacc taagggccca     3060
   taggcgcgcc cggtgcatgc aagcttgctt caagggcccg tttgtacaac ttta           3114
<210> 730
   <211> 159
   <212> PRT
   <213> Diabrotica virgifera
<400> 730
   Met Thr Ser Ile Glu Thr Val Gly Thr Ile Val Leu Lys Leu Leu Lys
   1               5                   10                  15
   Leu Val Ile Asn Leu Ile Cys Leu Ile Leu Tyr Arg Thr Gly Tyr Gln
               20                  25                  30
   Gly Tyr Phe Leu Gly Val Gly Gly Thr Trp Asn Leu Asn Glu Glu Lys
           35                  40                  45
   Asn Pro Asp Ala Glu Ile Val Ala Ser Gly Val Phe Val Gly Phe Met
       50                  55                  60
   Ile Tyr Thr Phe Val Ser Leu Ile Ser Leu Cys Phe Ala Ser Gly Asp
   65                  70                  75                  80
   His Lys Thr Thr Phe Thr Asp Ile Leu Met Asn Ile Val Gly Ile Phe
                   85                  90                  95
   Met Trp Ile Ala Ala Gly Ala Thr Ala Leu His Tyr Trp Leu Gly Tyr
               100                 105                 110
   Leu Ser Glu Tyr Lys Tyr Thr Thr Ile Asp Ser Glu Arg Gln Val Gly
           115                 120                 125
   Leu Ala Leu Gly Ala Met Cys Ile Ile Asn Gly Ala Val Tyr Leu Val
       130                 135                 140
   Asp Gly Val Leu Ser Ala Ile Phe Ile Leu Lys Ala Lys Met Gln
   145                 150                 155
<210> 731
   <211> 162
   <212> PRT
   <213> Drosophila melanogaster
<400> 731
   Met Val Ser Val Glu Thr Val Gly Ser Ile Phe Ile Lys Ala Leu Lys
   1               5                   10                  15
   Leu Ile Ile Asn Leu Val Ile Ile Phe Leu Tyr Arg Trp Gly Asp Gly
               20                  25                  30
   Gly Glu Phe Leu Gly Ile Gly Gly Thr Trp Asn Leu Asn Glu Glu Lys
           35                  40                  45
   Ser Ala Asp Ala Glu Ile Val Ala Ser Gly Val Met Val Gly Phe Leu
       50                  55                  60
   Ile Tyr Thr Gly Cys His Thr Ile Ala Phe Ala Phe Gly Thr Thr Lys
   65                  70                  75                  80
   His Lys Gly Glu Leu Cys Asp Thr Ile Met Asn Val Val Gly Cys Ile
                   85                  90                  95
   Met Trp Ile Ala Val Gly Gly Val Ala Leu His Tyr Trp Lys Gly Tyr
               100                 105                 110
   Met Ser Asp Glu Gly Phe Leu Tyr Val Asn Ser Glu Arg Gln Val Gly
           115                 120                 125
   Ile Ala Met Gly Ser Leu Cys Val Ile Glu Gly Ala Leu Tyr Leu Leu
       130                 135                 140
   Asp Thr Val Leu Ala Cys Ile His Tyr Ser Lys Gly Asp Thr Asp Tyr
   145                 150                 155                 160
   Thr Gln

## Description

### FIELD OF THE INVENTION

The present invention relates generally to methods of molecular biology and gene silencing to control pests.

### BACKGROUND OF THE INVENTION

Insect pests are a serious problem in agriculture. They destroy millions of acres of staple crops such as corn, soybeans, peas, and cotton. Yearly, these pests cause over $100 billion dollars in crop damage in the U.S. alone. In an ongoing seasonal battle, farmers must apply billions of gallons of synthetic pesticides to combat these pests. Other methods employed in the past delivered insecticidal activity by microorganisms or genes derived from microorganisms expressed in transgenic plants. For example, certain species of microorganisms of the genus *Bacillus* are known to possess pesticidal activity against a broad range of insect pests including *Lepidoptera, Diptera, Coleoptera, Hemiptera,* and others. In fact, microbial pesticides, particularly those obtained from *Bacillus* strains, have played an important role in agriculture as alternatives to chemical pest control. Agricultural scientists have developed crop plants with enhanced insect resistance by genetically engineering crop plants to produce insecticidal proteins from *Bacillus.* For example, corn and cotton plants genetically engineered to produce Cry toxins (see, e.g., Aronson (2002) Cell Mol. Life Sci. 59(3):417-425; Schnepf et al. (1998) Microbiol. Mol. Biol. Rev. 62(3):775-806) are now widely used in American agriculture and have provided the farmer with an alternative to traditional insect-control methods. However, these *Bt* insecticidal proteins only protect plants from a relatively narrow range of pests. Moreover, these modes of insecticidal activity provided varying levels of specificity and, in some cases, caused significant environmental consequences. Thus, there is an immediate need for alternative methods to control pests.

### BRIEF SUMMARY OF THE INVENTION

Methods and compositions are provided which employ a silencing element that, when ingested by a pest, such as Coleopteran plant pest including a *Diabrotica* plant pest, is capable of decreasing the expression of a target sequence in the pest. In specific embodiments, the decrease in expression of the target sequence controls the pest and thereby the methods and compositions are capable of limiting damage to a plant. The present invention provides various target polynucleotides as set forth in SEQ ID NOS: 574, 575, 576, 728, or fragments thereof, or complements thereof, wherein a decrease in expression of one or more of the sequences in the target pest controls the pest (i.e., has insecticidal activity). Further provided are silencing elements, which when ingested by the pest, decrease the level of expression of one or more of the target polynucleotides. Plants, plant parts, plant cells, bacteria and other host cells comprising the silencing elements or an active variant or fragment thereof are also provided. Also provided are formulations of sprayable silencing agents for topical applications to pest insects or substrates where pest insects may be found.

In another embodiment, a method for controlling a pest, such as a Coleopteran plant pest or a *Diabrotica* plant pest, is provided. The method comprises feeding to a pest a composition comprising a silencing element, wherein the silencing element, when ingested by the pest, reduces the level of a target sequence in the pest and thereby controls the pest. Further provided are methods to protect a plant from a pest. Such methods comprise introducing into the plant or plant part a silencing element of the invention. When the plant expressing the silencing element is ingested by the pest, the level of the target sequence is decreased and the pest is controlled.

### BRIEF DESCRIPTION OF THE DRAWINGS

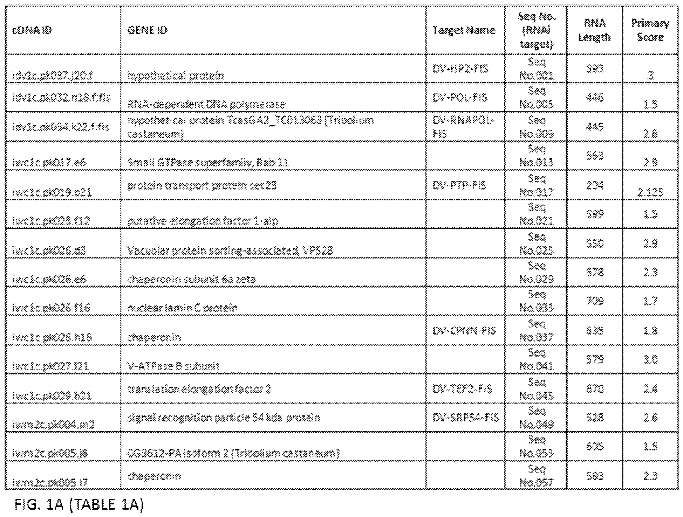

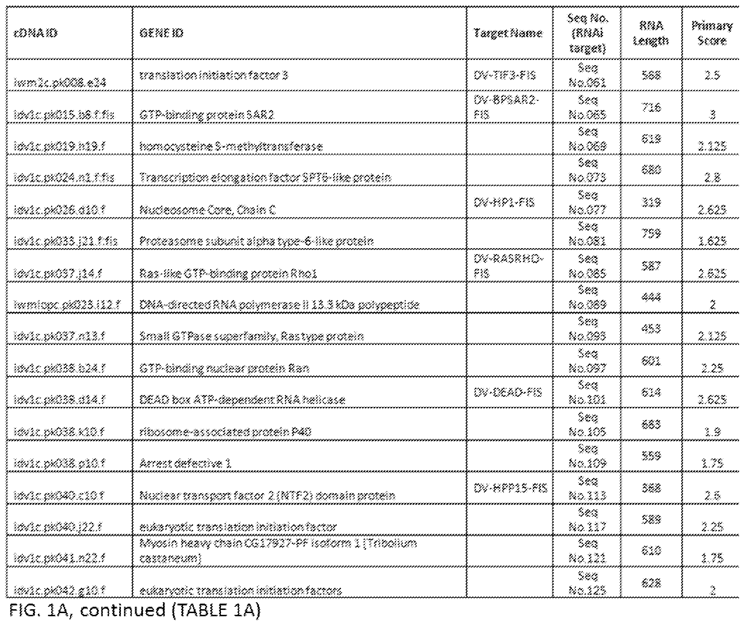

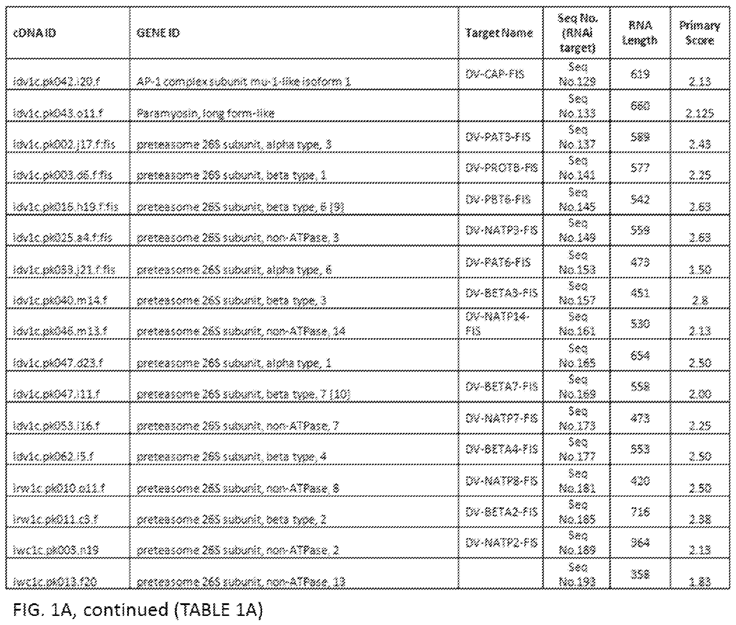

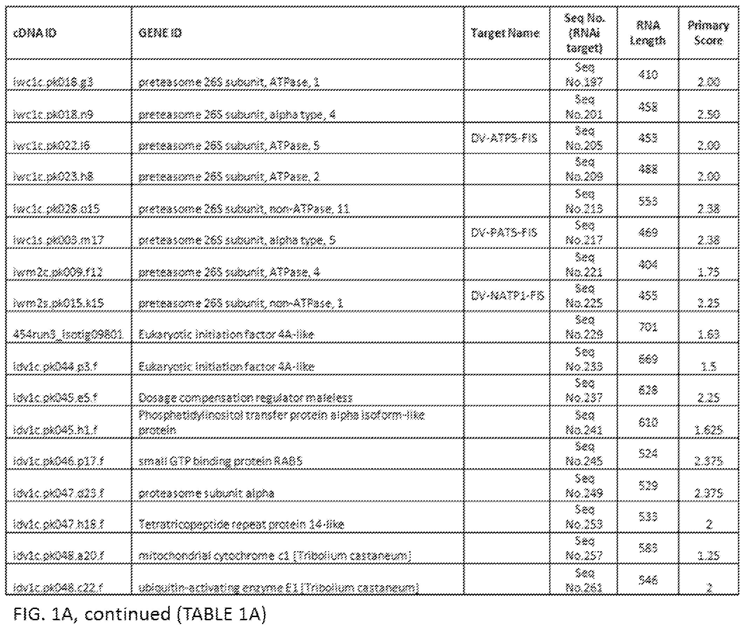

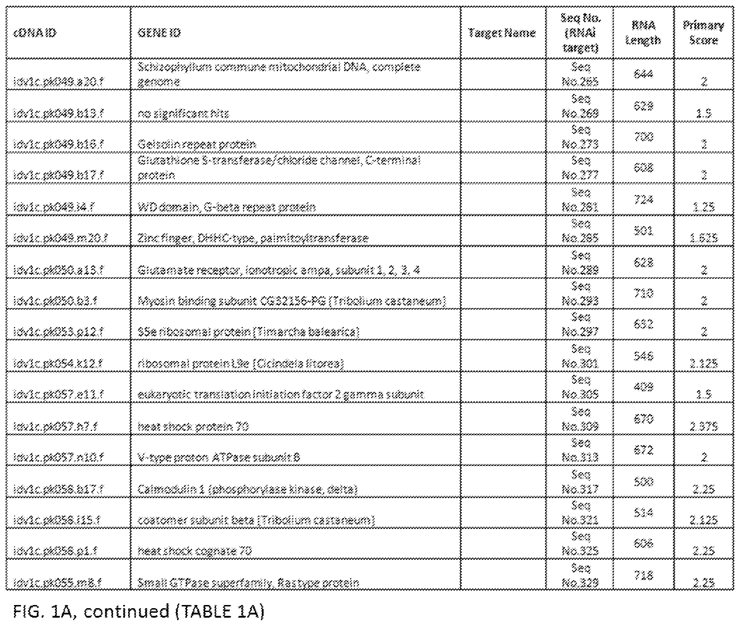

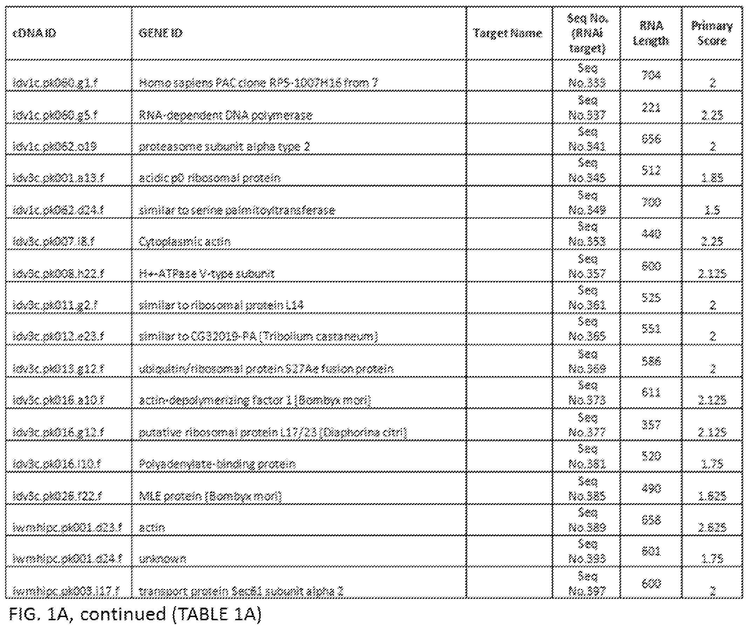

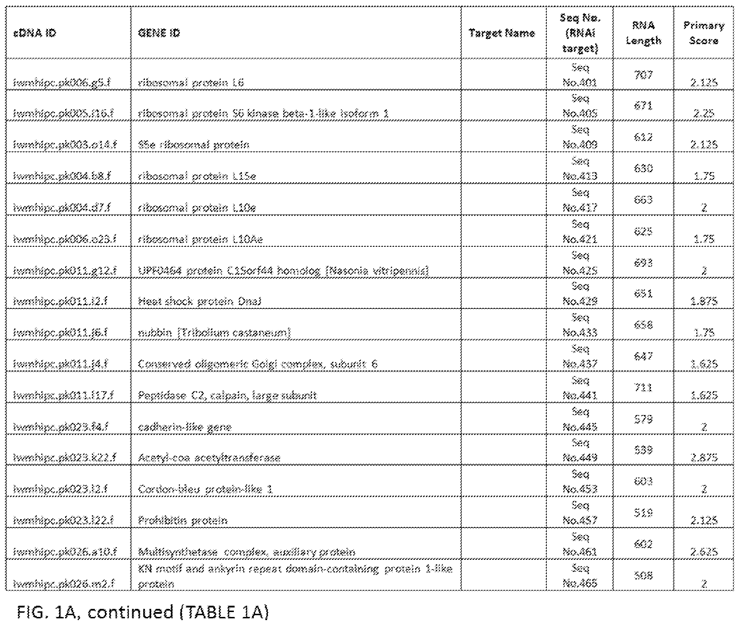

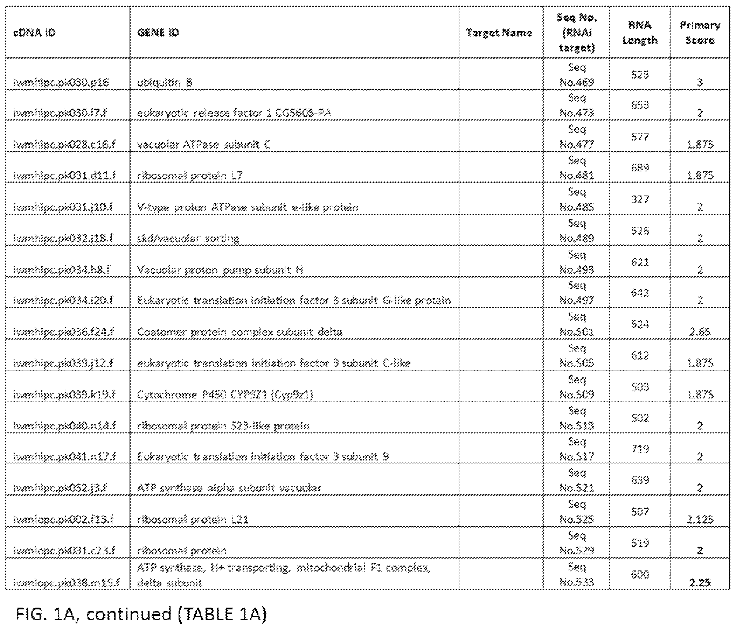

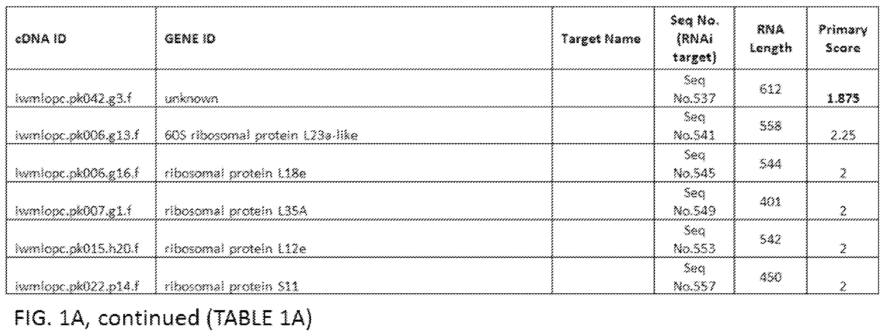

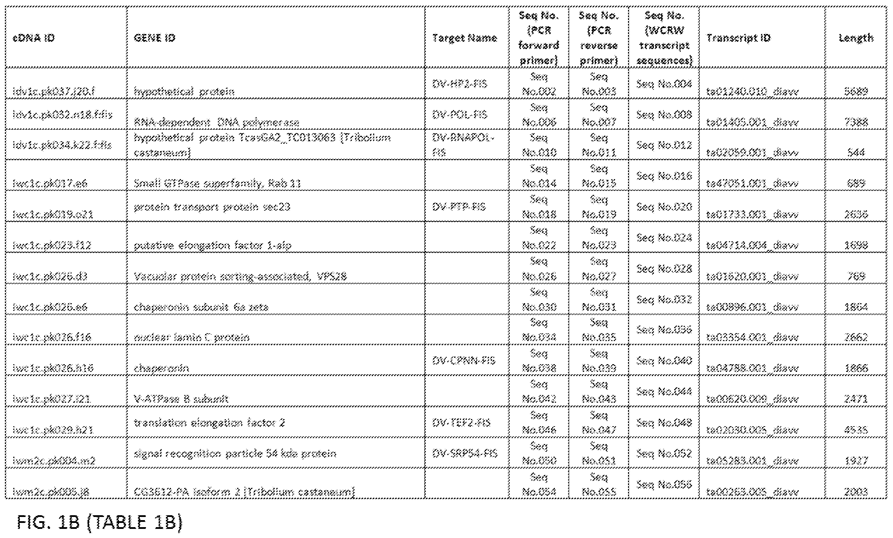

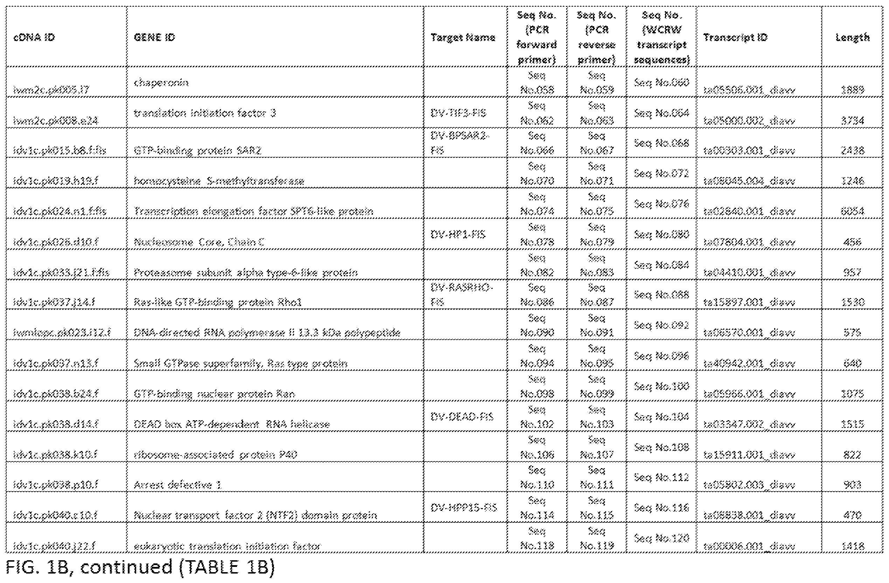

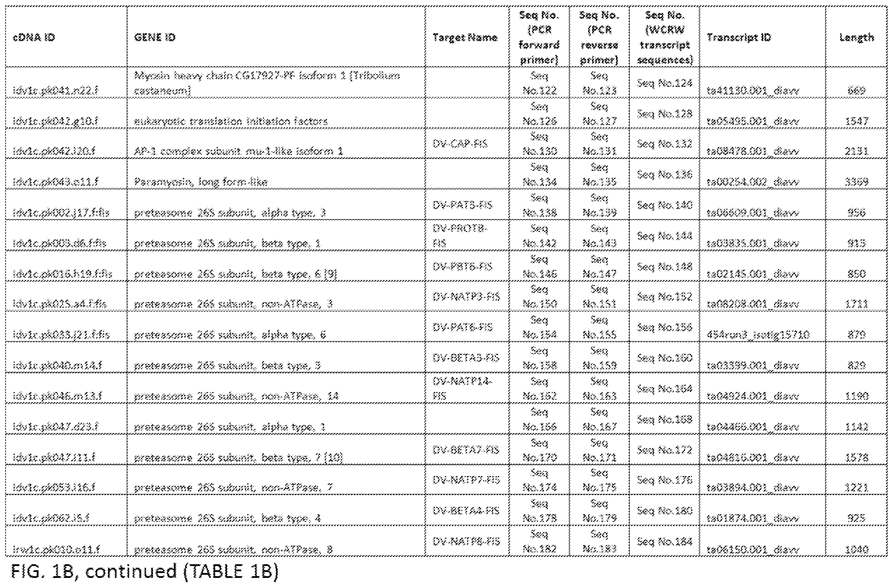

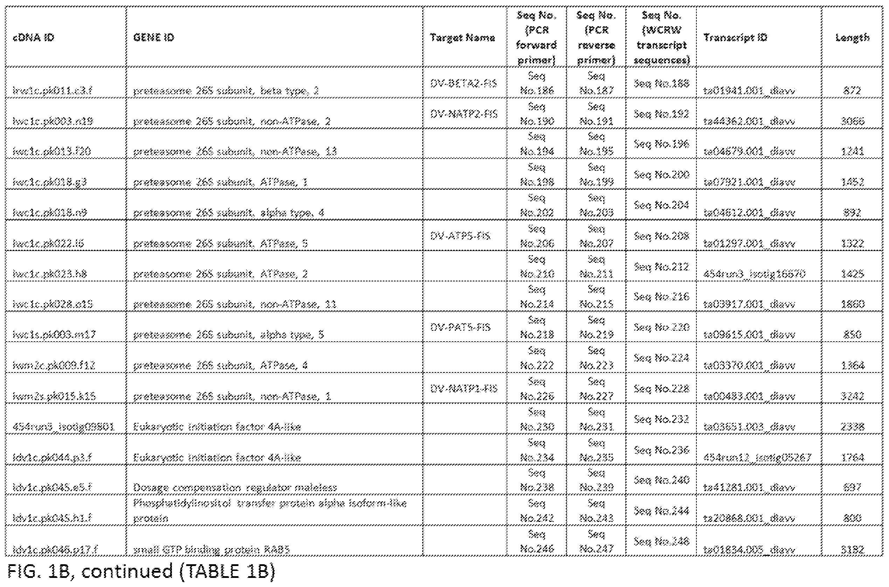

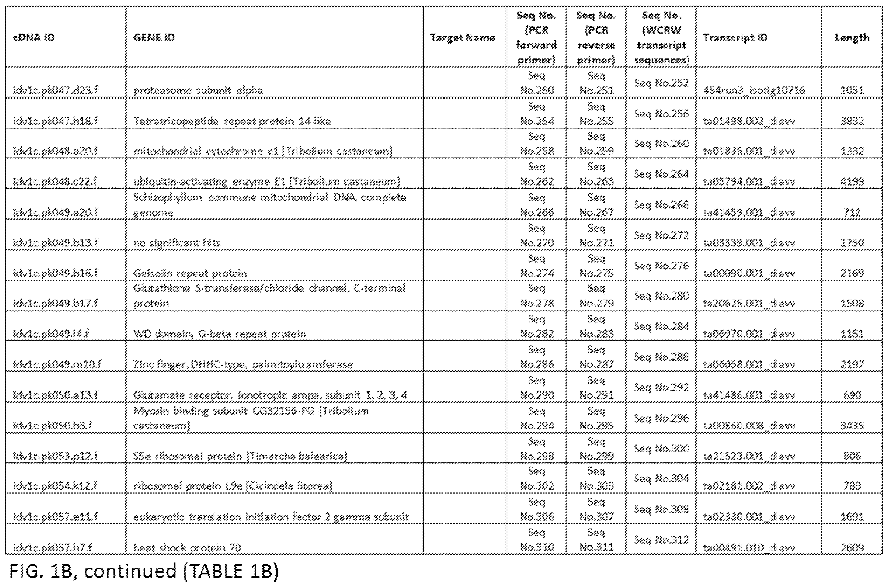

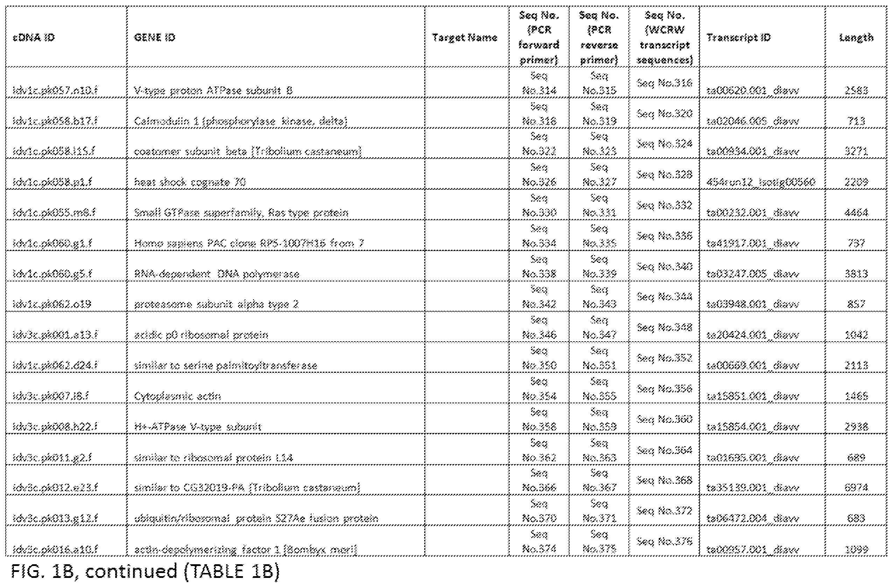

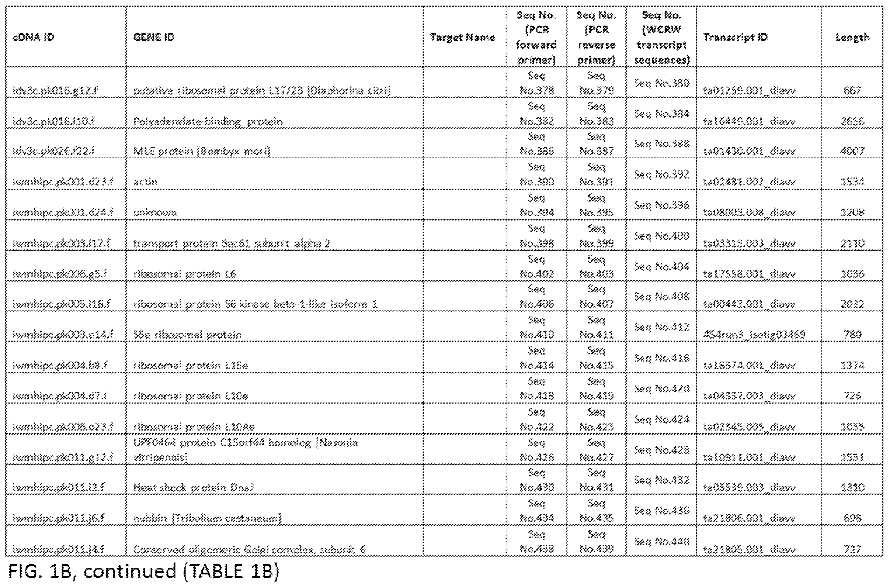

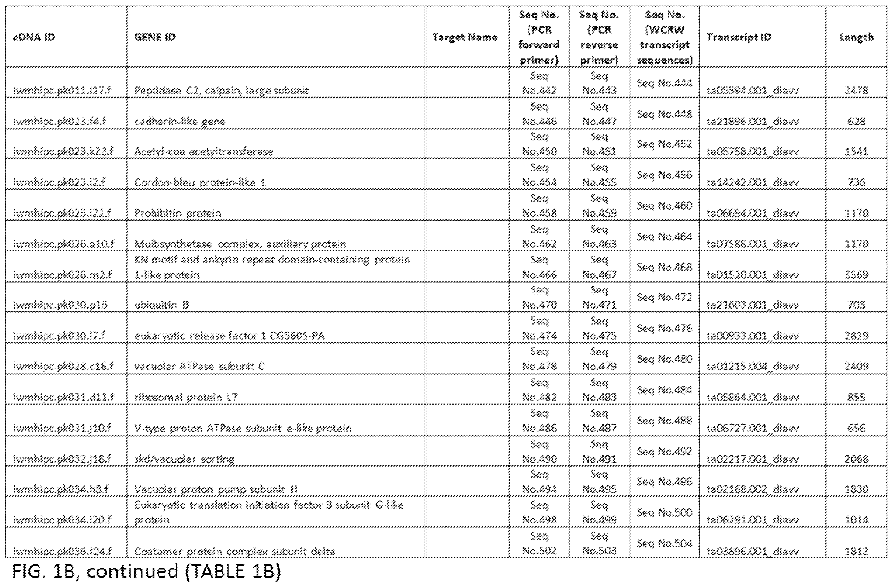

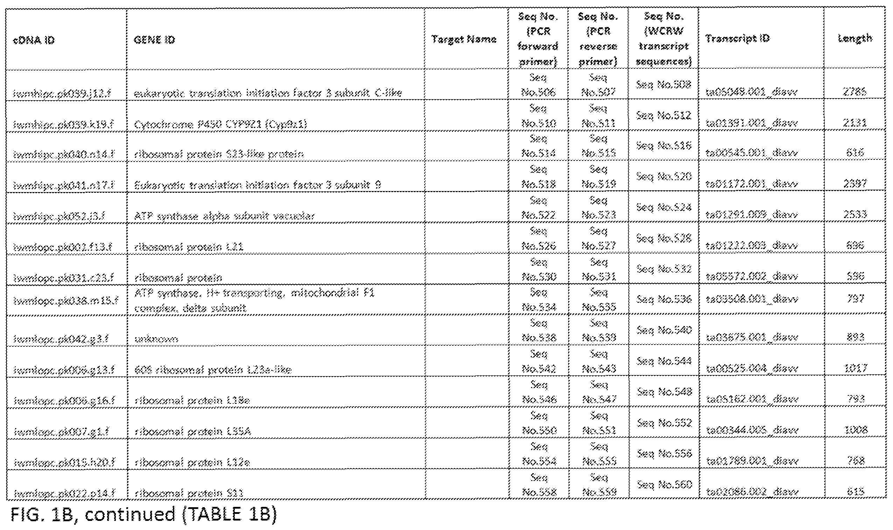

Figure 1 is a table, Tables 1A and 1B, which identifies RNAi active targets in diet assay using dsRNA produced by *in vitro* transcription (IVT).

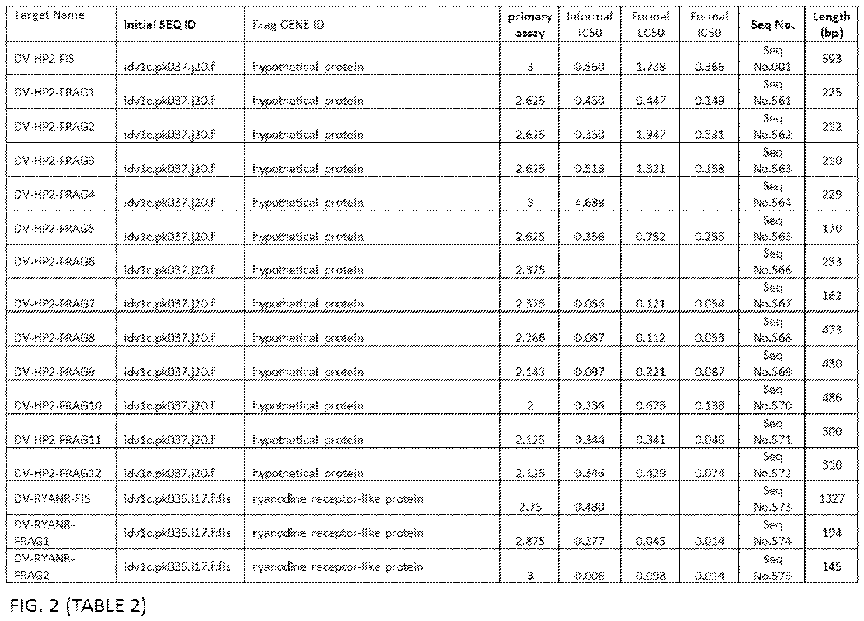

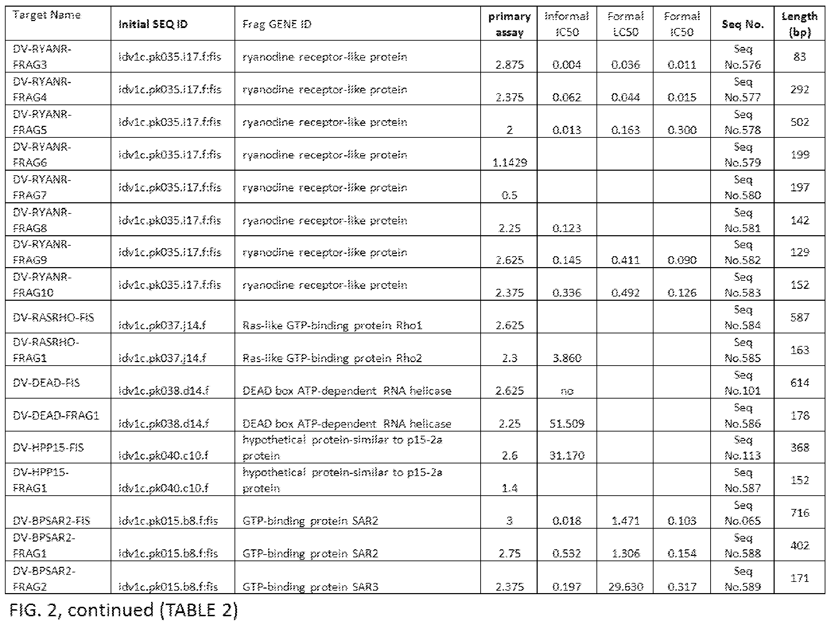

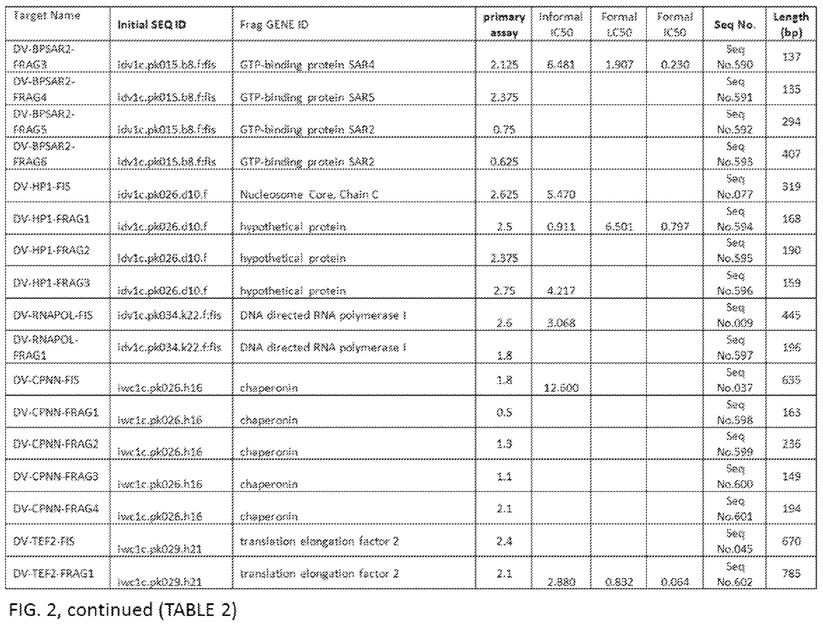

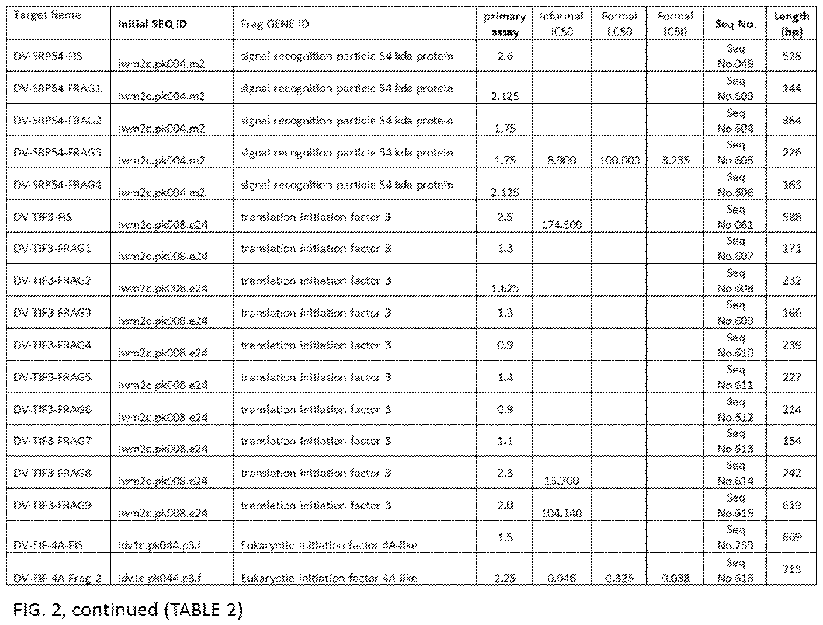

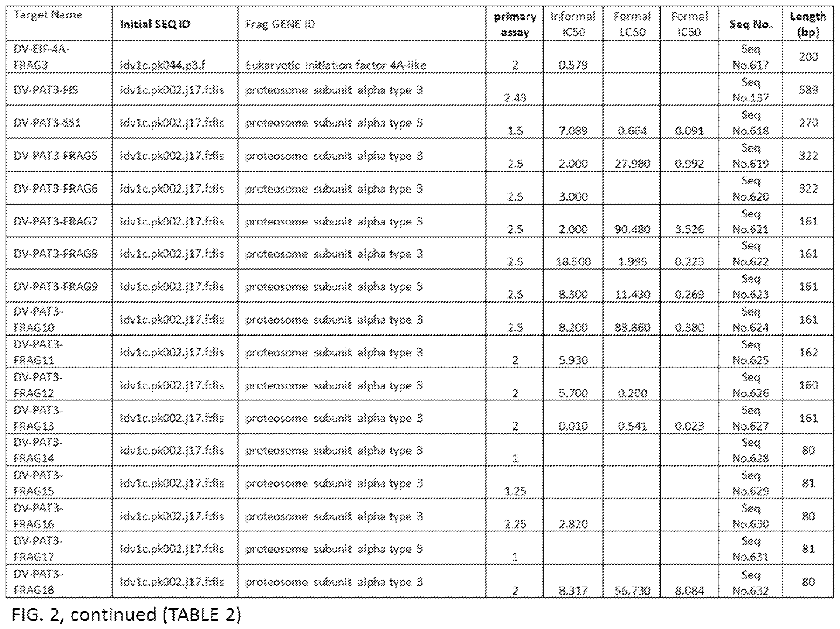

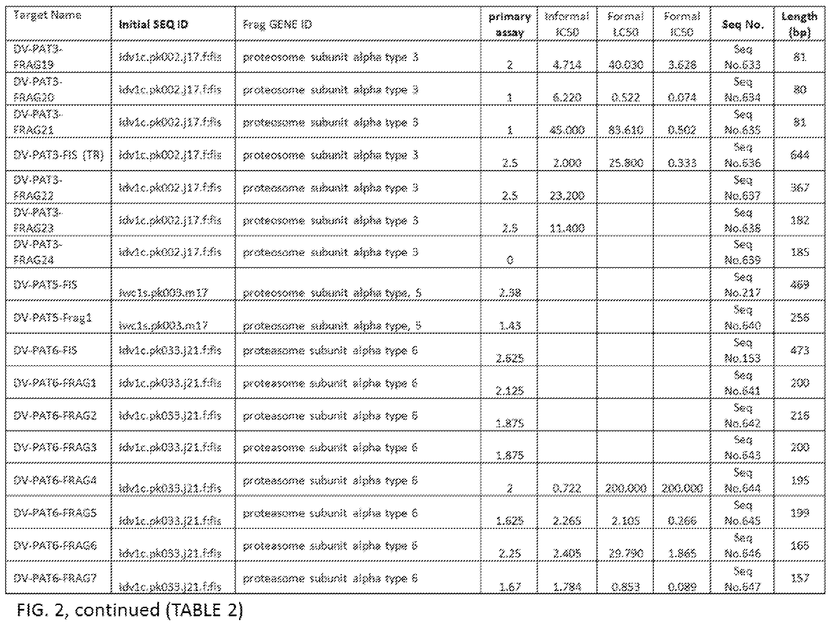

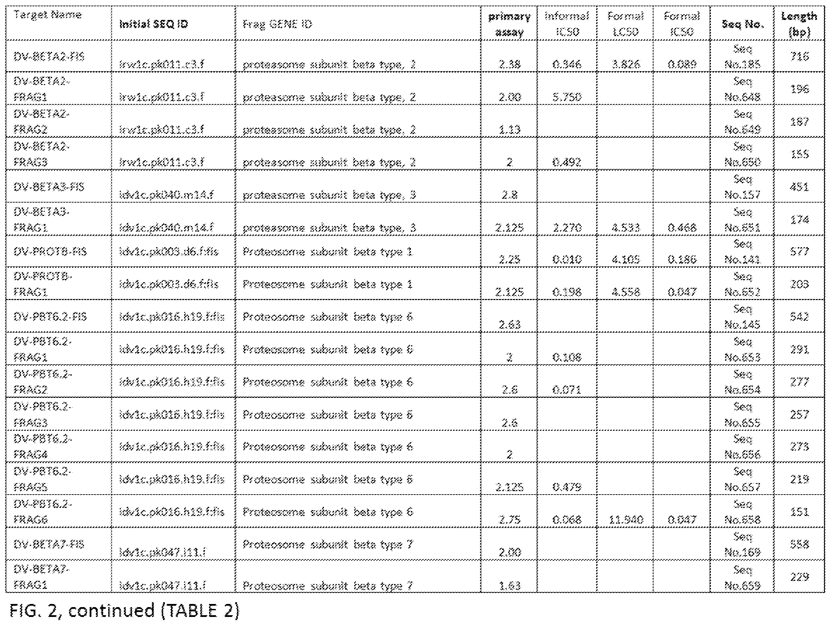

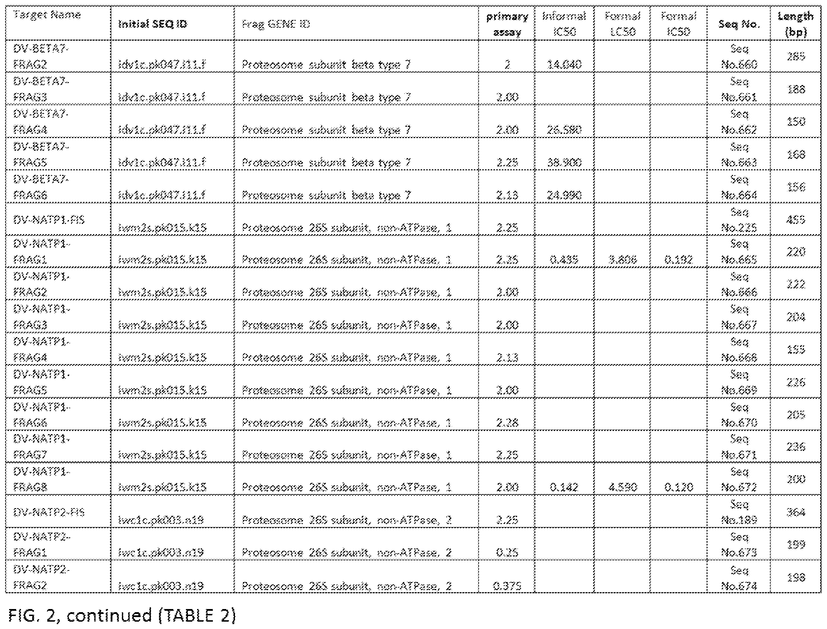

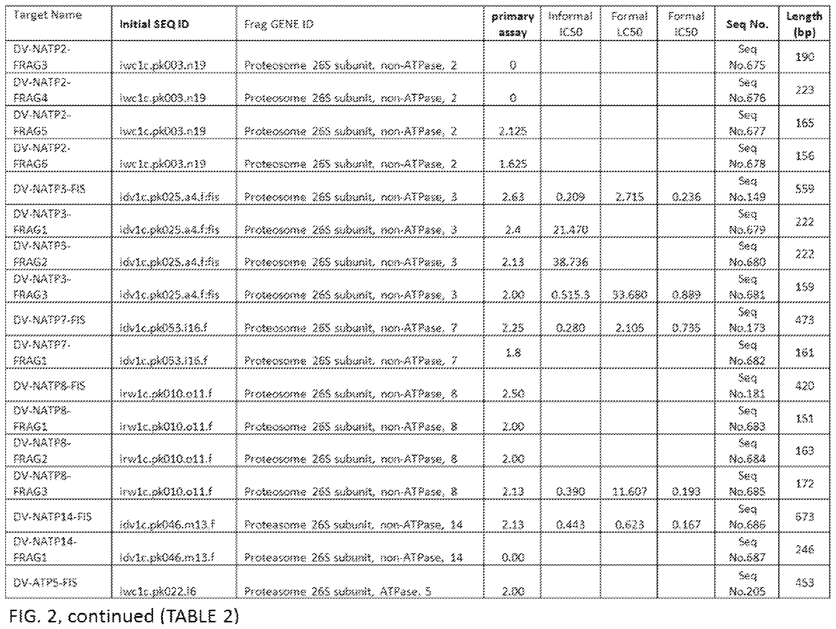

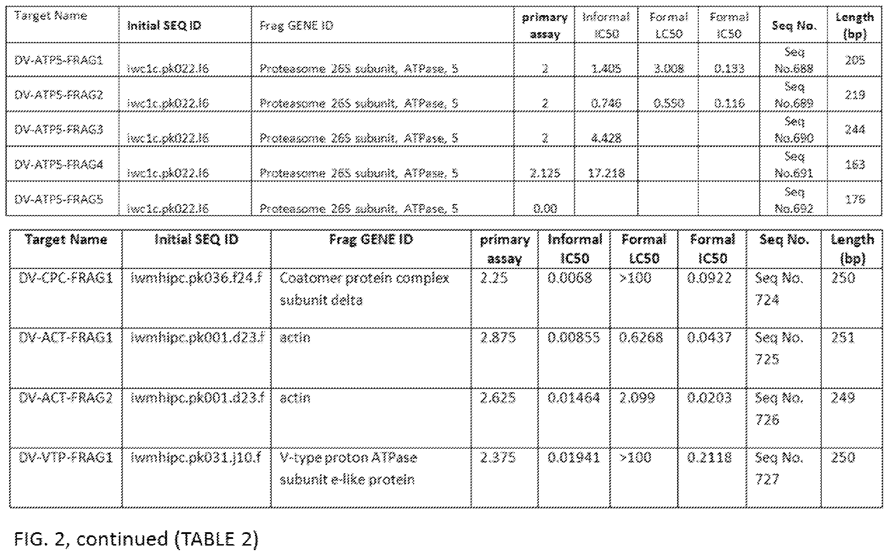

Figure 2 is a table, Table 2, which shows design and identification of RNAi active fragments.

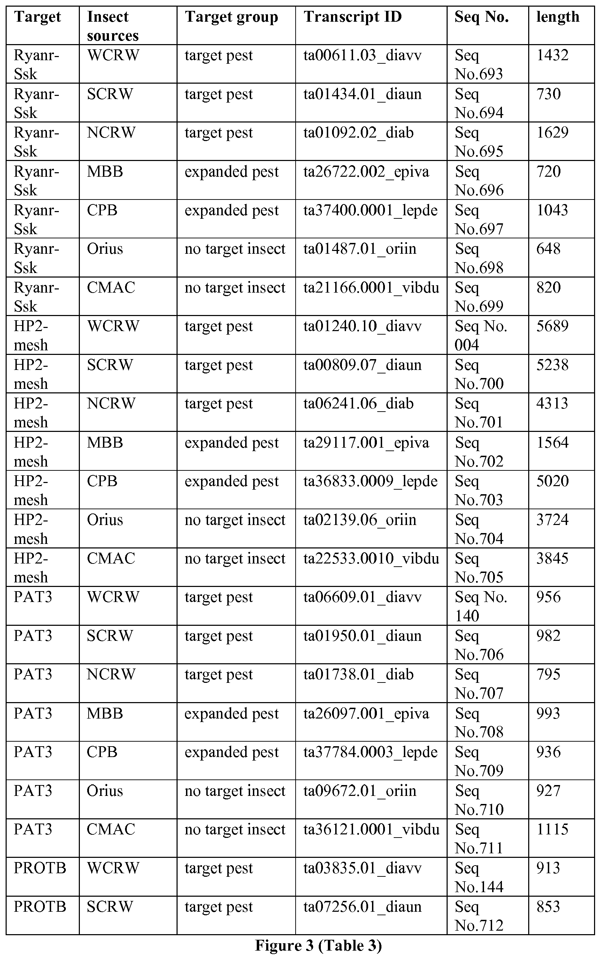

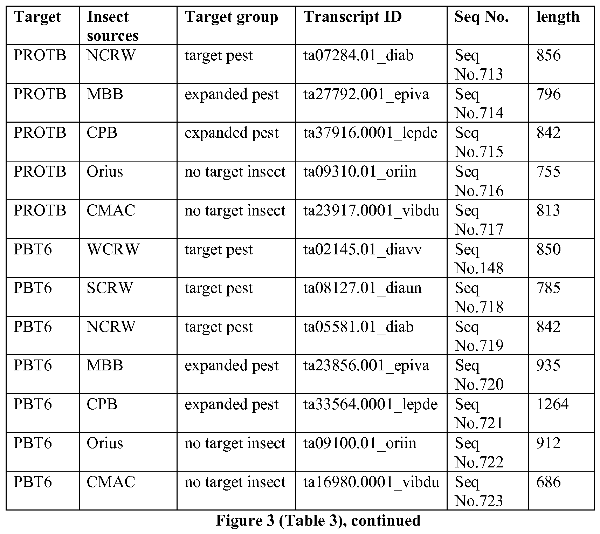

Figure 3 is a table, Table 3 which lists RNAi active targets from target pests, expanded pests and no target insects. Homologous sequences of selected RNAi actives were identified from transcriptome analyses of Western corn rootworm (WCRW, *Diabrotica virgifera*), Northern corn rootworm (NCRW, *Diabrotica barberi*), Southern corn rootworm (SCRW, *Diabrotica undecimpunctata*), Mexican Bean Beetle (MBB, *Epilachna varivestis*), Colorado potato beetle (CPB, *Leptinotarsa decemlineata*), insidious flower bug (Orius, *Orius insidiosus*) and Spotted Lady Beetle (CMAC, *Coleomegilla maculate*).
Figure 4 is a graphic showing a sequence alignment of the amino acid sequences of WCRW Ryanr (SEQ ID NO: 730) and Drosophila Ssk (SEQ ID NO: 731).
Figure 5 is a schematic of PAT3 fragments used in the gene and construct optimization experiment.
Figure 6 is a schematic showing the transgenic region of a representative disclosed construct, PHP58050 (SEQ ID NO: 729).
Figure 7 is a table, Table 4, which shows representative insecticidal activity against corn rootworms for maize plants comprising representative constructs. The representative constructs used in the study to transform maize plants were as shown and described in the table.
Figure 8 shows representative Corn Rootworm Nodal Injury Score ("CRWNIS") data for maize with the constructs described in Figure 7.

### DETAILED DESCRIPTION OF THE INVENTION

The present inventions now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed. Although specific terms are employed herein, they are used in a generic and descriptive sense only and not for purposes of limitation.

### I. Overview

Frequently, RNAi discovery methods rely on evaluation of known classes of sensitive genes (transcription factors, housekeeping genes etc.). In contrast, the target polynucleotides set forth herein were identified based solely on high throughput screens of all singletons and representatives of all gene clusters from a cDNA library of neonate and/or 3^{rd} instar midgut western corn rootworms. This screen allowed for the discovery of many novel sequences, many of which have extremely low or no homology to known sequences. This method provided the advantage of having no built in bias to genes that are frequently highly conserved across taxa. As a result, many novel targets for RNAi as well as known genes not previously shown to be sensitive to RNAi have been identified.

As such, methods and compositions are provided which employ one or more silencing elements that, when ingested by a pest, such as a Coleopteran plant pest or a *Diabrotica* plant pest, is capable of decreasing the expression of a target sequence in the pest. In specific embodiments, the decrease in expression of the target sequence controls the pest and thereby the methods and compositions are capable of limiting damage to a plant or plant part. The present invention provides target polynucleotides as set forth in SEQ ID NOS: 574, 575, 576, 728 and fragments thereof, and complements thereof. Silencing elements comprising sequences, complementary sequences, active fragments or variants of these target polynucleotides are provided which, when ingested by or when contacting the pest, decrease the expression of one or more of the target sequences and thereby controls the pest (i.e., has insecticidal activity).

As used herein, by "controlling a pest" or "controls a pest" is intended any affect on a pest that results in limiting the damage that the pest causes. Controlling a pest includes, but is not limited to, killing the pest, inhibiting development of the pest, altering fertility or growth of the pest in such a manner that the pest provides less damage to the plant, decreasing the number of offspring produced, producing less fit pests, producing pests more susceptible to predator attack, or deterring the pests from eating the plant.

Reducing the level of expression of the target polynucleotide or the polypeptide encoded thereby, in the pest results in the suppression, control, and/or killing the invading pest. Reducing the level of expression of the target sequence of the pest will reduce the pest damage by at least about 2% to at least about 6%, at least about 5% to about 50%, at least about 10% to about 60%, at least about 30% to about 70%, at least about 40% to about 80%, or at least about 50% to about 90% or greater. Hence, the methods of the invention can be utilized to control pests, particularly, Coleopteran plant pests or a *Diabrotica* plant pest.

Assays measuring the control of a pest are commonly known in the art, as are methods to record nodal injury score. See, for example, Oleson et al. (2005) J. Econ. Entomol. 98:1-8. See, for example, the examples below.

The invention is drawn to compositions and methods for protecting plants from a plant pest, such as Coleopteran plant pests or *Diabrotica* plant pests or inducing resistance in a plant to a plant pest, such as Coleopteran plant pests or *Diabrotica* plant pests. As used herein "Coleopteran plant pest" is used to refer to any member of the Coleoptera order. Other plant pests that may be targeted by the methods and compositions of the present invention include, but are not limited to Mexican Bean Beetle (*Epilachna varivestis*), and Colorado potato beetle (*Leptinotarsa decemlineata*),

As used herein, the term *"Diabrotica* plant pest" is used to refer to any member of the *Diabrotica* genus. Accordingly, the compositions and methods are also useful in protecting plants against any *Diabrotica* plant pest including, for example, *Diabrotica adelpha; Diabrotica amecameca; Diabrotica balteata; Diabrotica barberi; Diabrotica biannularis; Diabrotica cristata; Diabrotica decempunctata; Diabrotica dissimilis; Diabrotica lemniscata; Diabrotica limitata* (including, for example, *Diabrotica limitata quindecimpuncata); Diabrotica longicornis; Diabrotica nummularis; Diabrotica porracea; Diabrotica scutellata; Diabrotica sexmaculata; Diabrotica speciosa* (including, for example, *Diabrotica speciosa speciosa); Diabrotica tibialis; Diabrotica undecimpunctata* (including, for example, Southern corn rootworm (*Diabrotica undecimpunctata*), *Diabrotica undecimpunctata duodecimnotata; Diabrotica undecimpunctata howardi* (spotted cucumber beetle); *Diabrotica undecimpunctata undecimpunctata* (western spotted cucumber beetle)); *Diabrotica virgifera* (including, for example, *Diabrotica virgifera virgifera* (western corn rootworm) and *Diabrotica virgifera zeae* (Mexican corn rootworm)); *Diabrotica viridula; Diabrotica wartensis; Diabrotica sp. JJG335; Diabrotica sp. JJG336; Diabrotica sp. JJG341; Diabrotica sp. JJG356; Diabrotica sp. JJG362;* and, *Diabrotica sp. JJG365.*

In specific embodiments, the *Diabrotica* plant pest comprises *D. virgifera virgifera, D. barberi, D. virgifera zeae, D. speciosa,* or *D. undecimpunctata howardi.*

### II. Target Sequences

As used herein, a "target sequence" or "target polynucleotide" comprises any sequence in the pest that one desires to reduce the level of expression thereof. In specific embodiments, decreasing the level of the target sequence in the pest controls the pest. For instance, the target sequence may be essential for growth and development. While the target sequence can be expressed in any tissue of the pest, in specific embodiments, the sequences targeted for suppression in the pest are expressed in cells of the gut tissue of the pest, cells in the midgut of the pest, and cells lining the gut lumen or the midgut. Such target sequences can be involved in, for example, gut cell metabolism, growth or differentiation. Non-limiting examples of target sequences of the invention include a polynucleotide set forth in SEQ ID NOS: 574, 575, 576, 728 and fragments thereof, and complements thereof.As exemplified elsewhere herein, decreasing the level of expression of one or more of these target sequences in a Coleopteran plant pest or a *Diabrotica* plant pest controls the pest.

### III. Silencing Elements

By "silencing element" is intended a polynucleotide which when contacted by or ingested by a pest, is capable of reducing or eliminating the level or expression of a target polynucleotide or the polypeptide encoded thereby. The silencing element employed can reduce or eliminate the expression level of the target sequence by influencing the level of the target RNA transcript or, alternatively, by influencing translation and thereby affecting the level of the encoded polypeptide. Methods to assay for functional silencing elements that are capable of reducing or eliminating the level of a sequence of interest are disclosed elsewhere herein. A single polynucleotide employed in the methods of the invention can comprise one or more silencing elements to the same or different target polynucleotides. The silencing element can be produced *in vivo* (i.e., in a host cell such as a plant or microorganism) or *in vitro.*

A silencing element may comprise a chimeric construction molecule comprising two or more sequences of the present invention. For example, the chimeric construction may be a hairpin or dsRNA as disclosed herein. A chimera may comprise two or more sequences of the present invention. A chimera may contemplate two complementary sequences set forth herein having some degree of mismatch between the complementary sequences such that the two sequences are not perfect complements of one another. Providing at least two different sequences in a single silencing element may allow for targeting multiple genes using one silencing element and/or for example, one expression cassette. Targeting multiple genes may allow for slowing or reducing the possibility of resistance by the pest, and providing the multiple targeting ability in one expressed molecule may reduce the expression burden of the transformed plant or plant product, or provide topical treatments that are capable of targeting multiple hosts with one application.

In specific disclosures, the target sequence is not endogenous to the plant. In other disclosures, while the silencing element controls pests, preferably the silencing element has no effect on the normal plant or plant part.

As discussed in further detail below, silencing elements can include, but are not limited to, a sense suppression element, an antisense suppression element, a double stranded RNA, a siRNA, a amiRNA, a miRNA, or a hairpin suppression element. Silencing elements of the present invention may comprise a chimera where two or more sequences of the present invention or active fragments or variants, or complements thereof, are found in the same RNA molecule. Further, a sequence of the present invention or active fragment or variant, or complement thereof, may be present as more than one copy in a DNA construct, silencing element, DNA molecule or RNA molecule. In a hairpin or dsRNA molecule, the location of a sense or antisense sequence in the molecule, for example, in which sequence is transcribed first or is located on a particular terminus of the RNA molecule, is not limiting to the invention, and the invention is not to be limited by disclosures herein of a particular location for such a sequence. Non-limiting examples of silencing elements that can be employed to decrease expression of these target Coleopteran plant pest sequences or *Diabrotica* plant pest sequences comprise fragments and variants of the sense or antisense sequence or consists of the sense or antisense sequence of the sequence set forth in SEQ ID NOS: 574, 575, 576, 728, and fragments thereof, and complements thereof. The silencing element can further comprise additional sequences that advantageously effect transcription and/or the stability of a resulting transcript. For example, the silencing elements can comprise at least one thymine residue at the 3' end. This can aid in stabilization. Thus, the silencing elements can have at least 1, 2, 3, 4, 5, 6, 7, 8, 9, 10 or more thymine residues at the 3' end. As discussed in further detail below, enhancer suppressor elements can also be employed in conjunction with the silencing elements disclosed herein.

By "reduces" or "reducing" the expression level of a polynucleotide or a polypeptide encoded thereby is intended to mean, the polynucleotide or polypeptide level of the target sequence is statistically lower than the polynucleotide level or polypeptide level of the same target sequence in an appropriate control pest which is not exposed to (i.e., has not ingested or come into contact with) the silencing element. Reducing the polynucleotide level and/or the polypeptide level of the target sequence in a pest according to the invention may result in less than 95%, less than 90%, less than 80%, less than 70%, less than 60%, less than 50%, less than 40%, less than 30%, less than 20%, less than 10%, or less than 5% of the polynucleotide level, or the level of the polypeptide encoded thereby, of the same target sequence in an appropriate control pest. Methods to assay for the level of the RNA transcript, the level of the encoded polypeptide, or the activity of the polynucleotide or polypeptide are discussed elsewhere herein.

### i. Sense Suppression Elements

As used herein, a "sense suppression element" comprises a polynucleotide designed to express an RNA molecule corresponding to at least a part of a target messenger RNA in the "sense" orientation. Expression of the RNA molecule comprising the sense suppression element reduces or eliminates the level of the target polynucleotide or the polypeptide encoded thereby. The polynucleotide comprising the sense suppression element may correspond to all or part of the sequence of the target polynucleotide, all or part of the 5' and/or 3' untranslated region of the target polynucleotide, all or part of the coding sequence of the target polynucleotide, or all or part of both the coding sequence and the untranslated regions of the target polynucleotide.

Typically, a sense suppression element has substantial sequence identity to the target polynucleotide, typically greater than about 65% sequence identity, greater than about 85% sequence identity, about 90%, 91%, 92%, 93%, 94%, 95%, 96%, 97%, 98% or 99% sequence identity. See, U.S. Patent Nos. 5,283,184 and 5,034,323. The sense suppression element can be any length so long as it allows for the suppression of the targeted sequence. The sense suppression element can be, for example, 15, 16, 17, 18, 19, 20, 22, 25, 30, 50, 100, 150, 200, 250, 300, 350, 400, 450, 500, 600, 700, 900, 1000, 1100, 1200, 1300 nucleotides or longer of the target polynucleotides set forth in any of SEQ ID NOS: 574, 575, 576, 728, and fragments thereof, and complements thereof. The sense suppression element can be, for example, about 15-25, 19-35, 19-50, 25-100, 100-150, 150-200, 200-250, 250-300, 300-350, 350-400, 450-500, 500-550, 550-600, 600-650, 650-700, 700-750, 750-800, 800-850, 850-900, 900-950, 950-1000, 1000-1050, 1050-1100, 1100-1200, 1200-1300, 1300-1400, 1400-1500, 1500-1600, 1600-1700, 1700-1800 nucleotides or longer of the target polynucleotides set forth in any of SEQ ID NOS: 574, 575, 576, 728, and fragments thereof, and complements thereof.

### ii. Antisense Suppression Elements

As used herein, an "antisense suppression element" comprises a polynucleotide which is designed to express an RNA molecule complementary to all or part of a target messenger RNA. Expression of the antisense RNA suppression element reduces or eliminates the level of the target polynucleotide. The polynucleotide for use in antisense suppression may correspond to all or part of the complement of the sequence encoding the target polynucleotide, all or part of the complement of the 5' and/or 3' untranslated region of the target polynucleotide, all or part of the complement of the coding sequence of the target polynucleotide, or all or part of the complement of both the coding sequence and the untranslated regions of the target polynucleotide. In addition, the antisense suppression element may be fully complementary (i.e., 100% identical to the complement of the target sequence) or partially complementary (i.e., less than 100% identical to the complement of the target sequence) to the target polynucleotide. The antisense suppression element may comprise at least 85%, 90%, 91%, 92%, 93%, 94%, 95%, 96%, 97%, 98%, or 99% sequence complementarity to the target polynucleotide. Antisense suppression may be used to inhibit the expression of multiple proteins in the same plant. See, for example, U.S. Patent No. 5,942,657. Furthermore, the antisense suppression element can be complementary to a portion of the target polynucleotide. Generally, sequences of at least 15, 16, 17, 18, 19, 20, 22, 25, 50, 100, 200, 300, 400, 450 nucleotides or greater of the sequence set forth in any of SEQ ID NOS: 574, 575, 576, 728, and fragments thereof, and complements thereof. Methods for using antisense suppression to inhibit the expression of endogenous genes in plants are described, for example, in Liu et al (2002) Plant Physiol. 129:1732-1743 and U.S. Patent Nos. 5,759,829 and 5,942,657,.

### iii. Double Stranded RNA Suppression Element

A "double stranded RNA silencing element" or "dsRNA" comprises at least one transcript that is capable of forming a dsRNA either before or after ingestion by a pest. Thus, a "dsRNA silencing element" includes a dsRNA, a transcript or polyribonucleotide capable of forming a dsRNA or more than one transcript or polyribonucleotide capable of forming a dsRNA. "Double stranded RNA" or "dsRNA" refers to a polyribonucleotide structure formed either by a single self-complementary RNA molecule or a polyribonucleotide structure formed by the expression of at least two distinct RNA strands. The dsRNA molecule(s) employed in the methods and compositions of the invention mediate the reduction of expression of a target sequence, for example, by mediating RNA interference "RNAi" or gene silencing in a sequence-specific manner. In the context of the present invention, the dsRNA is capable of reducing or eliminating the level or expression of a target polynucleotide or the polypeptide encoded thereby in a pest.

The dsRNA can reduce or eliminate the expression level of the target sequence by influencing the level of the target RNA transcript, by influencing translation and thereby affecting the level of the encoded polypeptide, or by influencing expression at the pre-transcriptional level (i.e., via the modulation of chromatin structure, methylation pattern, etc., to alter gene expression). See, for example, Verdel et al. (2004) Science 303:672-676; Pal-Bhadra et al. (2004) Science 303:669-672; Allshire (2002) Science 297:1818-1819; Volpe et al. (2002) Science 297:1833-1837; Jenuwein (2002) Science 297:2215-2218; and Hall et al. (2002) Science 297:2232-2237. Methods to assay for functional dsRNA that are capable of reducing or eliminating the level of a sequence of interest are disclosed elsewhere herein. Accordingly, as used herein, the term "dsRNA" is meant to encompass other terms used to describe nucleic acid molecules that are capable of mediating RNA interference or gene silencing, including, for example, short-interfering RNA (siRNA), double-stranded RNA (dsRNA), micro-RNA (miRNA), hairpin RNA, short hairpin RNA (shRNA), post-transcriptional gene silencing RNA (ptgsRNA), and others.

At least one strand of the duplex or double-stranded region of the dsRNA may share sufficient sequence identity or sequence complementarity to the target polynucleotide to allow for the dsRNA to reduce the level of expression of the target sequence. As used herein, the strand that is complementary to the target polynucleotide is the "antisense strand" and the strand homologous to the target polynucleotide is the "sense strand."

In another embodiment, the dsRNA comprises a hairpin RNA. A hairpin RNA comprises an RNA molecule that is capable of folding back onto itself to form a double stranded structure. Multiple structures can be employed as hairpin elements. In specific embodiments, the dsRNA suppression element comprises a hairpin element which comprises in the following order, a first segment, a second segment, and a third segment, where the first and the third segment share sufficient complementarity to allow the transcribed RNA to form a double-stranded stem-loop structure.

The "second segment" of the hairpin comprises a "loop" or a "loop region." These terms are used synonymously herein and are to be construed broadly to comprise any nucleotide sequence that confers enough flexibility to allow self-pairing to occur between complementary regions of a polynucleotide (i.e., segments 1 and 3 which form the stem of the hairpin). The loop region may be substantially single stranded and act as a spacer between the self-complementary regions of the hairpin stem-loop. The loop region can comprise a random or nonsense nucleotide sequence and thus not share sequence identity to a target polynucleotide. The loop region may comprise a sense or an antisense RNA sequence or fragment thereof that shares identity to a target polynucleotide. See, for example, International Patent Publication No. WO 02/00904. The loop region can be optimized to be as short as possible while still providing enough intramolecular flexibility to allow the formation of the base-paired stem region. Accordingly, the loop sequence is generally less than 1000, 900, 800, 700, 600, 500, 400, 300, 200, 100, 50, 25, 20, 19, 18, 17, 16, 15, 10 nucleotides or less.

The "first" and the "third" segment of the hairpin RNA molecule comprise the base-paired stem of the hairpin structure. The first and the third segments are inverted repeats of one another and share sufficient complementarity to allow the formation of the base-paired stem region. In specific embodiments, the first and the third segments are fully complementary to one another. Alternatively, the first and the third segment may be partially complementary to each other so long as they are capable of hybridizing to one another to form a base-paired stem region. The amount of complementarity between the first and the third segment can be calculated as a percentage of the entire segment. Thus, the first and the third segment of the hairpin RNA generally share at least 50%, 60%, 70%, 80%, 85%, 90%, 91%, 92%, 93%, 94%, 95%, 96%, 97%, 98%, 99%, up to and including 100% complementarity.

The first and the third segment are at least about 1000, 500, 475, 450, 425, 400, 375, 350, 325, 300, 250, 225, 200, 175, 150, 125, 100, 75, 60, 50, 40, 30, 25, 22, 20, 19, 18, 17, 16, 15 or 10 nucleotides in length. The length of the first and/or the third segment may be about 10-100 nucleotides, about 10 to about 75 nucleotides, about 10 to about 50 nucleotides, about 10 to about 40 nucleotides, about 10 to about 35 nucleotides, about 10 to about 30 nucleotides, about 10 to about 25 nucleotides, about 10 to about 19 nucleotides, about 10 to about 20 nucleotides, about 19 to about 50 nucleotides, about 50 nucleotides to about 100 nucleotides, about 100 nucleotides to about 150 nucleotides, about 100 nucleotides to about 300 nucleotides, about 150 nucleotides to about 200 nucleotides, about 200 nucleotides to about 250 nucleotides, about 250 nucleotides to about 300 nucleotides, about 300 nucleotides to about 350 nucleotides, about 350 nucleotides to about 400 nucleotides, about 400 nucleotide to about 500 nucleotides, about 600 nt, about 700 nt, about 800 nt, about 900 nt, about 1000 nt, about 1100 nt, about 1200 nt, 1300 nt, 1400 nt, 1500 nt, 1600 nt, 1700 nt, 1800 nt, 1900 nt, 2000 nt or longer. The length of the first and/or the third segment may comprise at least 10-19 nucleotides, 10-20 nucleotides; 19-35 nucleotides, 20-35 nucleotides; 30-45 nucleotides; 40-50 nucleotides; 50-100 nucleotides; 100-300 nucleotides; about 500 - 700 nucleotides; about 700-900nucleotides; about 900-1100 nucleotides; about 1300 -1500 nucleotides; about 1500 - 1700 nucleotides; about 1700 - 1900 nucleotides; about 1900 - 2100 nucleotides; about 2100 - 2300 nucleotides; or about 2300 - 2500 nucleotides. See, for example, International Publication No. WO 0200904.

Hairpin molecules or double-stranded RNA molecules of the present invention may have more than one sequence of the present invention or active fragments, or complements thereof, found in the same portion of the RNA molecule. For example, in a chimeric hairpin structure, the first segment of a hairpin molecule comprises two polynucleotide sections, each with a different sequence of the present invention. For example, reading from one terminus of the hairpin, the first segment is composed of sequences from two separate genes (A followed by B). This first segment is followed by the second segment, the loop portion of the hairpin. The loop segment is followed by the third segment, where the complementary strands of the sequences in the first segment are found (B* followed by A*) in forming the stem-loop, hairpin structure, the stem contains SeqA-A* at the distal end of the stem and SeqB-B* proximal to the loop region.

The first and the third segment may comprise at least 20 nucleotides having at least 85% complementary to the first segment. The first and the third segments which form the stem-loop structure of the hairpin comprises 3' or 5' overhang regions may have unpaired nucleotide residues.

In specific embodiments, the sequences used in the first, the second, and/or the third segments comprise domains that are designed to have sufficient sequence identity to a target polynucleotide of interest and thereby have the ability to decrease the level of expression of the target polynucleotide. The specificity of the inhibitory RNA transcripts is therefore generally conferred by these domains of the silencing element. Thus, the first, second and/or third segment of the silencing element may comprise a domain having at least 10, at least 15, at least 19, at least 20, at least 21, at least 22, at least 23, at least 24, at least 25, at least 30, at least 40, at least 50, at least 100, at least 200, at least 300, at least 500, at least 1000, or more than 1000 nucleotides that share sufficient sequence identity to the target polynucleotide to allow for a decrease in expression levels of the target polynucleotide when expressed in an appropriate cell. The domain may be between about 15 to 50 nucleotides, about 19-35 nucleotides, about 20-35 nucleotides, about 25-50 nucleotides, about 19 to 75 nucleotides, about 20 to 75 nucleotides, about 40-90 nucleotides about 15-100 nucleotides, 10-100 nucleotides, about 10 to about 75 nucleotides, about 10 to about 50 nucleotides, about 10 to about 40 nucleotides, about 10 to about 35 nucleotides, about 10 to about 30 nucleotides, about 10 to about 25 nucleotides, about 10 to about 20 nucleotides, about 10 to about 19 nucleotides, about 50 nucleotides to about 100 nucleotides, about 100 nucleotides to about 150 nucleotides, about 150 nucleotides to about 200 nucleotides, about 200 nucleotides to about 250 nucleotides, about 250 nucleotides to about 300 nucleotides, about 300 nucleotides to about 350 nucleotides, about 350 nucleotides to about 400 nucleotides, about 400 nucleotide to about 500 nucleotides or longer. The length of the first and/or the third segment may comprise at least 10-20 nucleotides, at least 10-19 nucleotides, 20-35 nucleotides, 30-45 nucleotides, 40-50 nucleotides, 50-100 nucleotides, or about 100-300 nucleotides.

The domain of the first, the second, and/or the third segment may have 100% sequence identity to the target polynucleotide. The domain of the first, the second and/or the third segment having homology to the target polypeptide may have at least 50%, 60%, 70%, 80%, 85%, 90%, 91%, 92%, 93%, 94%, 95%, 96%, 97%, 98%, 99%, or greater sequence identity to a region of the target polynucleotide. The sequence identity of the domains of the first, the second and/or the third segments to the target polynucleotide need only be sufficient to decrease expression of the target polynucleotide of interest. See, for example, Chuang and Meyerowitz (2000) Proc. Natl. Acad. Sci. USA 97:4985-4990; Stoutjesdijk et al. (2002) Plant Physiol. 129:1723-1731; Waterhouse and Helliwell (2003) Nat. Rev. Genet. 4:29-38; Pandolfini et al. BMC Biotechnology 3:7, and U.S. Patent Publication No. 20030175965. A transient assay for the efficiency of hpRNA constructs to silence gene expression *in vivo* has been described by Panstruga et al. (2003) Mol. Biol. Rep. 30:135-140.

The amount of complementarity shared between the first, second, and/or third segment and the target polynucleotide or the amount of complementarity shared between the first segment and the third segment (i.e., the stem of the hairpin structure) may vary depending on the organism in which gene expression is to be controlled. Some organisms or cell types may require exact pairing or 100% identity, while other organisms or cell types may tolerate some mismatching. In some cells, for example, a single nucleotide mismatch in the targeting sequence abrogates the ability to suppress gene expression. In these cells, the suppression cassettes of the invention can be used to target the suppression of mutant genes, for example, oncogenes whose transcripts comprise point mutations and therefore they can be specifically targeted using the methods and compositions of the invention without altering the expression of the remaining wild-type allele. In other organisms, holistic sequence variability may be tolerated as long as some 22nt region of the sequence is represented in 100% homology between target polynucleitide and the suppression cassette.

Any region of the target polynucleotide can be used to design the domain of the silencing element that shares sufficient sequence identity to allow expression of the hairpin transcript to decrease the level of the target polynucleotide. For instance, the domain can be designed to share sequence identity to the 5' untranslated region of the target polynucleotide(s), the 3' untranslated region of the target polynucleotide(s), exonic regions of the target polynucleotide(s), intronic regions of the target polynucleotide(s), and any combination thereof. A domain of the silencing element may share sufficient homology to at least about 15, 16, 17, 18, 19, 20, 22, 25 or 30 consecutive nucleotides from about nucleotides 1-50, 25-75, 75-125, 50-100, 125-175, 175-225, 100-150, 150-200, 200-250, 225-275, 275-325, 250-300, 325-375, 375-425, 300-350, 350-400, 425-475, 400-450, 475-525, 450-500, 525-575, 575-625, 550-600, 625-675, 675-725, 600-650, 625-675, 675-725, 650-700, 725-825, 825-875, 750-800, 875-925, 925-975, 850-900, 925-975, 975-1025, 950-1000, 1000-1050, 1025-1075, 1075-1125, 1050-1100, 1125-1175, 1100-1200, 1175-1225, 1225-1275, 1200-1300, 1325-1375, 1375-1425, 1300-1400, 1425-1475, 1475-1525, 1400-1500, 1525-1575, 1575-1625, 1625-1675, 1675-1725, 1725-1775, 1775-1825, 1825-1875, 1875-1925, 1925-1975, 1975-2025, 2025-2075, 2075-2125, 2125-2175, 2175-2225, 1500-1600, 1600-1700, 1700-1800, 1800-1900, 1900-2000 of the target sequence. In some instances to optimize the siRNA sequences employed in the hairpin, the synthetic oligodeoxyribonucleotide/RNAse H method can be used to determine sites on the target mRNA that are in a conformation that is susceptible to RNA silencing. See, for example, Vickers et al. (2003) J. Biol. Chem 278:7108-7118 and Yang et al. (2002) Proc. Natl. Acad. Sci. USA 99:9442-9447. These studies indicate that there is a significant correlation between the RNase-H-sensitive sites and sites that promote efficient siRNA-directed mRNA degradation.

The hairpin silencing element may also be designed such that the sense sequence or the antisense sequence do not correspond to a target polynucleotide, in which case, the sense and antisense sequence flank a loop sequence that comprises a nucleotide sequence corresponding to all or part of the target polynucleotide. Thus, it is the loop region that determines the specificity of the RNA interference. See, for example, WO 02/00904.

In addition, transcriptional gene silencing (TGS) may be accomplished through use of a hairpin suppression element where the inverted repeat of the hairpin shares sequence identity with the promoter region of a target polynucleotide to be silenced. See, for example, Aufsatz et al. (2002) PNAS 99 (Suppl. 4):16499-16506 and Mette et al. (2000) EMBO J 19(19):5194-5201.

The silencing element can comprise a small RNA (sRNA). sRNAs can comprise both micro RNA (miRNA) and short-interfering RNA (siRNA) (Meister and Tuschl (2004) Nature 431:343-349 and Bonetta et al. (2004) Nature Methods 1:79-86). miRNAs are regulatory agents comprising about 19 to about 24 ribonucleotides in length which are highly efficient at inhibiting the expression of target polynucleotides. See, for example Javier et al. (2003) Nature 425: 257-263. For miRNA interference, the silencing element can be designed to express a dsRNA molecule that forms a hairpin structure or partially base-paired structure containing 19, 20, 21, 22, 23, 24 or 25 -nucleotide sequence that is complementary to the target polynucleotide of interest. The miRNA can be synthetically made, or transcribed as a longer RNA which is subsequently cleaved to produce the active miRNA. Specifically, the miRNA can comprise 19 nucleotides of the sequence having homology to a target polynucleotide in sense orientation and 19 nucleotides of a corresponding antisense sequence that is complementary to the sense sequence. The miRNA can be an "artificial miRNA" or "amiRNA" which comprises a miRNA sequence that is synthetically designed to silence a target sequence.

When expressing an miRNA the final (mature) miRNA is present in a duplex in a precursor backbone structure, the two strands being referred to as the miRNA (the strand that will eventually basepair with the target) and miRNA*(star sequence). It has been demonstrated that miRNAs can be transgenically expressed and target genes of interest efficiently silenced (Highly specific gene silencing by artificial microRNAs in Arabidopsis Schwab R, Ossowski S, Riester M, Warthmann N, Weigel D. Plant Cell. 2006 May;18(5):1121-33. Epub 2006 Mar 10 & Expression of artificial microRNAs in transgenic Arabidopsis thaliana confers virus resistance. Niu QW, Lin SS, Reyes JL, Chen KC, Wu HW, Yeh SD, Chua NH. Nat Biotechnol. 2006 Nov;24(11):1420-8. Epub 2006 Oct 22. Erratum in: Nat Biotechnol. 2007 Feb;25(2):254.)

The silencing element for miRNA interference comprises a miRNA primary sequence. The miRNA primary sequence comprises a DNA sequence having the miRNA and star sequences separated by a loop as well as additional sequences flanking this region that are important for processing. When expressed as an RNA, the structure of the primary miRNA is such as to allow for the formation of a hairpin RNA structure that can be processed into a mature miRNA. The miRNA backbone may comprise a genomic or cDNA miRNA precursor sequence, wherein said sequence comprises a native primary in which a heterologous (artificial) mature miRNA and star sequence are inserted.

As used herein, a "star sequence" is the sequence within a miRNA precursor backbone that is complementary to the miRNA and forms a duplex with the miRNA to form the stem structure of a hairpin RNA. The star sequence can comprise less than 100% complementarity to the miRNA sequence. Alternatively, the star sequence can comprise at least 99%, 98%, 97%, 96%, 95%, 90%, 85%, 80% or lower sequence complementarity to the miRNA sequence as long as the star sequence has sufficient complementarity to the miRNA sequence to form a double stranded structure. The star sequence may comprise a sequence having 1, 2, 3, 4, 5 or more mismatches with the miRNA sequence and still has sufficient complementarity to form a double stranded structure with the miRNA sequence resulting in production of miRNA and suppression of the target sequence.

The miRNA precursor backbones can be from any plant. The miRNA precursor backbone may be from a monocot. The miRNA precursor backbone may be from a dicot. The backbone may be from maize or soybean. MicroRNA precursor backbones have been described previously. For example, US20090155910A1 (WO 2009/079532) discloses the following soybean miRNA precursor backbones: 156c, 159, 166b, 168c, 396b and 398b, and US20090155909A1 (WO 2009/079548) discloses the following maize miRNA precursor backbones: 159c, 164h, 168a, 169r, and 396h.

Thus, the primary miRNA can be altered to allow for efficient insertion of heterologous miRNA and star sequences within the miRNA precursor backbone. In such instances, the miRNA segment and the star segment of the miRNA precursor backbone are replaced with the heterologous miRNA and the heterologous star sequences, designed to target any sequence of interest, using a PCR technique and cloned into an expression construct. It is recognized that there could be alterations to the position at which the artificial miRNA and star sequences are inserted into the backbone. Detailed methods for inserting the miRNA and star sequence into the miRNA precursor backbone are described in, for example, US Patent Applications 20090155909A1 and US20090155910A1.

When designing a miRNA sequence and star sequence, various design choices can be made. See, for example, Schwab R, et al. (2005) Dev Cell 8: 517-27. The miRNA sequences disclosed herein can have a "U" at the 5'-end, a "C" or "G" at the 19th nucleotide position, and an "A" or "U" at the 10th nucleotide position. The miRNA design may be such that the miRNA have a high free delta-G as calculated using the ZipFold algorithm (Markham, N. R. & Zuker, M. (2005) Nucleic Acids Res. 33: W577-W581.) Optionally, a one base pair change can be added within the 5' portion of the miRNA so that the sequence differs from the target sequence by one nucleotide.

The methods and compositions of the invention employ silencing elements that when transcribed "form" a dsRNA molecule. Accordingly, the heterologous polynucleotide being expressed need not form the dsRNA by itself, but can interact with other sequences in the plant cell or in the pest gut after ingestion to allow the formation of the dsRNA. For example, a chimeric polynucleotide that can selectively silence the target polynucleotide can be generated by expressing a chimeric construct comprising the target sequence for a miRNA or siRNA to a sequence corresponding to all or part of the gene or genes to be silenced, inwhich case, the dsRNA is "formed" when the target for the miRNA or siRNA interacts with the miRNA present in the cell. The resulting dsRNA can then reduce the level of expression of the gene or genes to be silenced. See, for example, US Application Publication 2007-0130653, entitled "Methods and Compositions for Gene Silencing". The construct can be designed to have a target for an endogenous miRNA or alternatively, a target for a heterologous and/or synthetic miRNA can be employed in the construct. If a heterologous and/or synthetic miRNA is employed, it can be introduced into the cell on the same nucleotide construct as the chimeric polynucleotide or on a separate construct. As discussed elsewhere herein, any method can be used to introduce the construct comprising the heterologous miRNA.

### IV. Variants and Fragments

By "fragment" is intended a portion of the polynucleotide or a portion of the amino acid sequence and hence protein encoded thereby. Fragments of a polynucleotide may encode protein fragments that retain the biological activity of the native protein. Alternatively, fragments of a polynucleotide that are useful as a silencing element do not need to encode fragment proteins that retain biological activity. Thus, fragments of a nucleotide sequence may range from at least about 10, about 15, about 16, about 17, about 18, about 19, nucleotides, about 20 nucleotides, about 22 nucleotides, about 50 nucleotides, about 75 nucleotides, about 100 nucleotides, 200 nucleotides, 300 nucleotides, 400 nucleotides, 500 nucleotides, 600 nucleotides, 700 nucleotides and up to the full-length polynucleotide employed in the invention. Alternatively, fragments of a nucleotide sequence may range from 1-50, 25-75, 75-125, 50-100, 125-175, 175-225, 100-150, 100-300, 150-200, 200-250, 225-275, 275-325, 250-300, 325-375, 375-425, 300-350, 350-400, 425-475, 400-450, 475-525, 450-500, 525-575, 575-625, 550-600, 625-675, 675-725, 600-650, 625-675, 675-725, 650-700, 725-825, 825-875, 750-800, 875-925, 925-975, 850-900, 925-975, 975-1025, 950-1000, 1000-1050, 1025-1075, 1075-1125, 1050-1100, 1125-1175, 1100-1200, 1175-1225, 1225-1275, 1200-1300, 1325-1375, 1375-1425, 1300-1400, 1425-1475, 1475-1525, 1400-1500, 1525-1575, 1575-1625, 1625-1675, 1675-1725, 1725-1775, 1775-1825, 1825-1875, 1875-1925, 1925-1975, 1975-2025, 2025-2075, 2075-2125, 2125-2175, 2175-2225, 1500-1600, 1600-1700, 1700-1800, 1800-1900, 1900-2000 of any one of SEQ ID NOS: 574, 575, 576, 728, and fragments thereof, and complements thereof. Methods to assay for the activity of a desired silencing element are described elsewhere herein.

"Variants" is intended to mean substantially similar sequences. For polynucleotides, a variant comprises a deletion and/or addition of one or more nucleotides at one or more internal sites within the native polynucleotide and/or a substitution of one or more nucleotides at one or more sites in the native polynucleotide. A variant of a polynucleotide that is useful as a silencing element will retain the ability to reduce expression of the target polynucleotide and, may, thereby control a pest of interest. As used herein, a "native" polynucleotide or polypeptide comprises a naturally occurring nucleotide sequence or amino acid sequence, respectively. For polynucleotides, conservative variants include those sequences that, because of the degeneracy of the genetic code, encode the amino acid sequence of one of the polypeptides employed in the invention. Variant polynucleotides also include synthetically derived polynucleotide, such as those generated, for example, by using site-directed mutagenesis, but continue to retain the desired activity. Generally, variants of a particular polynucleotide of the invention (i.e., a silencing element) will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular polynucleotide as determined by sequence alignment programs and parameters described elsewhere herein.

Variants of a particular polynucleotide of the invention (i.e., the reference polynucleotide) can also be evaluated by comparison of the percent sequence identity between the polypeptide encoded by a variant polynucleotide and the polypeptide encoded by the reference polynucleotide. Percent sequence identity between any two polypeptides can be calculated using sequence alignment programs and parameters described elsewhere herein. Where any given pair of polynucleotides employed in the invention is evaluated by comparison of the percent sequence identity shared by the two polypeptides they encode, the percent sequence identity between the two encoded polypeptides is at least about 40%, 45%, 50%, 55%, 60%, 65%, 70%, 75%, 80%, 85%, 90%, 91%, 92%, 93%, 94%, 95%, 96%, 97%, 98%, 99% or more sequence identity.

The following terms are used to describe the sequence relationships between two or more polynucleotides or polypeptides: (a) "reference sequence", (b) "comparison window", (c) "sequence identity", and, (d) "percentage of sequence identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two polynucleotides. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.
   Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by GAP Version 10.
(c) As used herein, "sequence identity" or "identity" in the context of two polynucleotides or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

A method is further provided for identifying a silencing element from the target polynucleotides set forth in SEQ ID NOS: 574, 575, 576, 728, and fragments thereof, and complements thereof. Such methods comprise obtaining a candidate fragment of any one of SEQ ID NOS: 574, 575, 576, 728, and fragments thereof, and complements thereof, which is of sufficient length to act as a silencing element and thereby reduce the expression of the target polynucleotide and/or control a desired pest; expressing said candidate polynucleotide fragment in an appropriate expression cassette to produce a candidate silencing element and determining is said candidate polynucleotide fragment has the activity of a silencing element and thereby reduce the expression of the target polynucleotide and/or controls a desired pest. Methods of identifying such candidate fragments based on the desired pathway for suppression are known. For example, various bioinformatics programs can be employed to identify the region of the target polynucleotides that could be exploited to generate a silencing element. See, for example, Elbahir et al. (2001) Genes and Development 15:188-200, Schwartz et al. (2003) Cell 115:199-208, Khvorova et al. (2003) Cell 115:209-216. See also, siRNA at Whitehead (jura.wi.mit.edu/bioc/siRNAext/) which calculates the binding energies for both sense and antisense siRNAs. See, also genscript.com/ssl-bin/app/rnai?op=known; Block-iT™ RNAi designer from Invitrogen and GenScript siRNA Construct Builder.

### V. DNA constructs

The use of the term "polynucleotide" is not intended to limit the present invention to polynucleotides comprising DNA. Those of ordinary skill in the art will recognize that polynucleotides can comprise ribonucleotides and combinations of ribonucleotides and deoxyribonucleotides. Such deoxyribonucleotides and ribonucleotides include both naturally occurring molecules and synthetic analogues. The polynucleotides of the invention also encompass all forms of sequences including, but not limited to, single-stranded forms, double-stranded forms, hairpins, stem-and-loop structures, and the like.

The polynucleotide encoding the silencing element or in specific embodiments employed in the methods and compositions of the invention can be provided in expression cassettes for expression in a plant or organism of interest. It is recognized that multiple silencing elements including multiple identical silencing elements, multiple silencing elements targeting different regions of the target sequence, or multiple silencing elements from different target sequences can be used. It is recognized that each silencing element can be contained in a single or separate cassette, DNA construct, or vector. As discussed, any means of providing the silencing element is contemplated. A plant or plant cell can be transformed with a single cassette comprising DNA encoding one or more silencing elements or separate cassettes comprising each silencing element can be used to transform a plant or plant cell or host cell. Likewise, a plant transformed with one component can be subsequently transformed with the second component. One or more silencing elements can also be brought together by sexual crossing. That is, a first plant comprising one component is crossed with a second plant comprising the second component. Progeny plants from the cross will comprise both components.

The expression cassette can include 5' and 3' regulatory sequences operably linked to the polynucleotide of the invention. "Operably linked" is intended to mean a functional linkage between two or more elements. For example, an operable linkage between a polynucleotide of the invention and a regulatory sequence (i.e., a promoter) is a functional link that allows for expression of the polynucleotide of the invention. Operably linked elements may be contiguous or non-contiguous. When used to refer to the joining of two protein coding regions, by operably linked is intended that the coding regions are in the same reading frame. The cassette may additionally contain at least one additional polynucleotide to be cotransformed into the organism. Alternatively, the additional polypeptide(s) can be provided on multiple expression cassettes. Expression cassettes can be provided with a plurality of restriction sites and/or recombination sites for insertion of the polynucleotide to be under the transcriptional regulation of the regulatory regions. The expression cassette may additionally contain selectable marker genes.

The expression cassette can include in the 5'-3' direction of transcription, a transcriptional and translational initiation region (i.e., a promoter), a polynucleotide comprising the silencing element employed in the methods and compositions of the invention, and a transcriptional and translational termination region (i.e., termination region) functional in plants. The double stranded RNA may be expressed from a suppression cassette. Such a cassette can comprise two convergent promoters that drive transcription of an operably linked silencing element. "Convergent promoters" refers to promoters that are oriented on either terminus of the operably linked silencing element such that each promoter drives transcription of the silencing element in opposite directions, yielding two transcripts. In such embodiments, the convergent promoters allow for the transcription of the sense and anti-sense strand and thus allow for the formation of a dsRNA. Such a cassette may also comprise two divergent promoters that drive transcription of one or more operably linked silencing elements. "Divergent promoters" refers to promoters that are oriented in opposite directions of each other, driving transcription of the one or more silencing elements in opposite directions. The divergent promoters allow for the transcription of the sense and antisense strands and allow for the formation of a dsRNA. The divergent promoters also allow for the transcription of at least two separate hairpin RNAs. One cassette comprising two or more silencing elements under the control of two separate promoters in the same orientation may be present in a construct. Two or more individual cassettes, each comprising at least one silencing element under the control of a promoter, may be present in a construct in the same orientation.

The regulatory regions (i.e., promoters, transcriptional regulatory regions, and translational termination regions) and/or the polynucleotides employed in the invention may be native/analogous to the host cell or to each other. Alternatively, the regulatory regions and/or the polynucleotide employed in the invention may be heterologous to the host cell or to each other. As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide. As used herein, a chimeric gene comprises a coding sequence operably linked to a transcription initiation region that is heterologous to the coding sequence.

The termination region may be native with the transcriptional initiation region, may be native with the operably linked polynucleotide encoding the silencing element, may be native with the plant host, or may be derived from another source (i.e., foreign or heterologous) to the promoter, the polynucleotide comprising silencing element, the plant host, or any combination thereof. Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acids Res. 15:9627-9639.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

A number of promoters can be used in the practice of the invention. The polynucleotide encoding the silencing element can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

An inducible promoter, for instance, a pathogen-inducible promoter could also be employed. Such promoters include those from pathogenesis-related proteins (PR proteins), which are induced following infection by a pathogen; e.g., PR proteins, SAR proteins, beta-1,3-glucanase, chitinase, etc. See, for example, Redolfi et al. (1983) Neth. J. Plant Pathol. 89:245-254; Uknes et al. (1992) Plant Cell 4:645-656; and Van Loon (1985) Plant Mol. Virol. 4:111-116. See also WO 99/43819.

Additionally, as pathogens find entry into plants through wounds or insect damage, a wound-inducible promoter may be used in the constructions of the invention. Such wound-inducible promoters include potato proteinase inhibitor (pin II) gene (Ryan (1990) Ann. Rev. Phytopath. 28:425-449; Duan et al. (1996) Nature Biotechnology 14:494-498); wun1 and wun2, U.S. Patent No. 5,428,148; win1 and win2 (Stanford et al. (1989) Mol. Gen. Genet. 215:200-208); systemin (McGurl et al. (1992) Science 225:1570-1573); WIP1 (Rohmeier et al. (1993) Plant Mol. Biol. 22:783-792; Eckelkamp et al. (1993) FEBS Letters 323:73-76); MPI gene (Corderok et al. (1994) Plant J. 6(2):141-150); and the like.

Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156).

Tissue-preferred promoters can be utilized to target enhanced expression within a particular plant tissue. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

Leaf-preferred promoters are known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590.

Root-preferred promoters are known and can be selected from the many available from the literature or isolated de novo from various compatible species. See, for example, Hire et al. (1992) Plant Mol. Biol. 20(2):207-218 (soybean root-specific glutamine synthetase gene); Keller and Baumgartner (1991) Plant Cell 3(10):1051-1061 (root-specific control element in the GRP 1.8 gene of French bean); Sanger et al. (1990) Plant Mol. Biol. 14(3):433-443 (root-specific promoter of the mannopine synthase (MAS) gene of *Agrobacterium tumefaciens*); and Miao et al. (1991) Plant Cell 3(1):11-22 (full-length cDNA clone encoding cytosolic glutamine synthetase (GS), which is expressed in roots and root nodules of soybean). See also Bogusz et al. (1990) Plant Cell 2(7):633-641, where two root-specific promoters isolated from hemoglobin genes from the nitrogen-fixing nonlegume *Parasponia andersonii* and the related non-nitrogen-fixing nonlegume *Trema tomentosa* are described. The promoters of these genes were linked to a β-glucuronidase reporter gene and introduced into both the nonlegume *Nicotiana tabacum* and the legume *Lotus corniculatus,* and in both instances root-specific promoter activity was preserved. Leach and Aoyagi (1991) describe their analysis of the promoters of the highly expressed rolC and rolD root-inducing genes of *Agrobacterium rhizogenes* (see Plant Science (Limerick) 79(1):69-76). They concluded that enhancer and tissue-preferred DNA determinants are dissociated in those promoters. Teeri *et al.* (1989) used gene fusion to lacZ to show that the *Agrobacterium* T-DNA gene encoding octopine synthase is especially active in the epidermis of the root tip and that the TR2' gene is root specific in the intact plant and stimulated by wounding in leaf tissue, an especially desirable combination of characteristics for use with an insecticidal or larvicidal gene (see EMBO J. 8(2):343-350). The TR1' gene, fused to *nptII* (neomycin phosphotransferase II) showed similar characteristics. Additional root-preferred promoters include the VfENOD-GRP3 gene promoter (Kuster et al. (1995) Plant Mol. Biol. 29(4):759-772); and rolB promoter (Capana et al. (1994) Plant Mol. Biol. 25(4):681-691. See also U.S. Patent Nos. 5,837,876; 5,750,386; 5,633,363; 5,459,252; 5,401,836; 5,110,732; and 5,023,179.

The plant-expressed promoter may be a vascular-specific promoter such as a phloem-specific promoter. A "vascular-specific" promoter, as used herein, is a promoter which is at least expressed in vascular cells, or a promoter which is preferentially expressed in vascular cells. Expression of a vascular-specific promoter need not be exclusively in vascular cells, expression in other cell types or tissues is possible. A "phloem-specific promoter" as used herein, is a plant-expressible promoter which is at least expressed in phloem cells, or a promoter which is preferentially expressed in phloem cells.

Expression of a phloem-specific promoter need not be exclusively in phloem cells, expression in other cell types or tissues, e.g., xylem tissue, is possible. A phloem-specific promoter is a plant-expressible promoter at least expressed in phloem cells, wherein the expression in non-phloem cells is more limited (or absent) compared to the expression in phloem cells. Examples of suitable vascular-specific or phloem-specific promoters include but are not limited to the promoters selected from the group consisting of: the SCSV3, SCSV4, SCSV5, and SCSV7 promoters (Schunmann et al. (2003) Plant Functional Biology 30:453-60; the rolC gene promoter of *Agrobacterium rhizogenes*(Kiyokawa et al. (1994) Plant Physiology 104:801-02; Pandolfini et al. (2003) BioMedCentral (BMC) Biotechnology 3:7, (www.biomedcentral.com/1472-6750/3/7); Graham et al. (1997) Plant Mol. Biol. 33:729-35; Guivarc'h *et al.* (1996); Almon et al. (1997) Plant Physiol. 115:1599-607; the rolA gene promoter of *Agrobacterium rhizogenes* (Dehio et al. (1993) Plant Mol. Biol. 23:1199-210); the promoter of the *Agrobacterium tumefaciens* T-DNA gene 5 (Korber et al. (1991) EMBO J. 10:3983-91); the rice sucrose synthase RSs1 gene promoter (Shi et al. (1994) J. Exp. Bot. 45:623-31); the CoYMV or Commelina yellow mottle badnavirus promoter (Medberry et al. (1992) Plant Cell 4:185-92; Zhou et al. (1998) Chin. J. Biotechnol. 14:9-16); the CFDV or coconut foliar decay virus promoter (Rohde et al. (1994) Plant Mol. Biol. 27:623-28; Hehn and Rhode (1998) J. Gen. Virol. 79:1495-99); the RTBV or rice tungro bacilliform virus promoter (Yin and Beachy (1995) Plant J. 7:969-80; Yin et al. (1997) Plant J. 12:1179-80); the pea glutamin synthase GS3A gene (Edwards et al. (1990) Proc. Natl. Acad. Sci. USA 87:3459-63; Brears et al. (1991) Plant J. 1:235-44); the inv CD111 and inv CD141 promoters of the potato invertase genes (Hedley et al. (2000) J. Exp. Botany 51:817-21); the promoter isolated from Arabidopsis shown to have phloem-specific expression in tobacco by Kertbundit et al. (1991) Proc. Natl. Acad. Sci. USA 88:5212-16); the VAHOX1 promoter region (Tornero et al. (1996) Plant J. 9:639-48); the pea cell wall invertase gene promoter (Zhang et al. (1996) Plant Physiol. 112:1111-17); the promoter of the endogenous cotton protein related to chitinase of US published patent application 20030106097, an acid invertase gene promoter from carrot (Ramloch-Lorenz et al. (1993) The Plant J. 4:545-54); the promoter of the sulfate transporter geneSultr1; 3 (Yoshimoto et al. (2003) Plant Physiol. 131:1511-17); a promoter of a sucrose synthase gene (Nolte and Koch (1993) Plant Physiol. 101:899-905); and the promoter of a tobacco sucrose transporter gene (Kuhn et al. (1997) Science 275-1298-1300).

Possible promoters also include the Black Cherry promoter for Prunasin Hydrolase (PH DL1.4 PRO) (US Patent No. 6,797, 859), Thioredoxin H promoter from cucumber and rice (Fukuda A et al. (2005). Plant Cell Physiol. 46(11):1779-86), Rice (RSs1) (Shi, T. Wang et al. (1994). J. Exp. Bot. 45(274): 623-631) and maize sucrose synthese -1 promoters (Yang., N-S. et al. (1990) PNAS 87:4144-4148), PP2 promoter from pumpkin Guo, H. et al. (2004) Transgenic Research 13:559-566), At SUC2 promoter (Truernit, E. et al. (1995) Planta 196(3):564-70., At SAM-1 (S-adenosylmethionine synthetase) (Mijnsbrugge KV. et al. (1996) Planr. Cell. Physiol. 37(8): 1108-1115), and the Rice tungro bacilliform virus (RTBV) promoter (Bhattacharyya-Pakrasi et al. (1993) Plant J. 4(1):71-79).

The expression cassette can also comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). Additional selectable markers include phenotypic markers such as β-galactosidase and fluorescent proteins such as green fluorescent protein (GFP) (Su et al. (2004) Biotechnol Bioeng 85:610-9 and Fetter et al. (2004) Plant Cell 16:215-28), cyan florescent protein (CYP) (Bolte et al. (2004) J. Cell Science 117:943-54 and Kato et al. (2002) Plant Physiol 129:913-42), and yellow florescent protein (PhiYFP™ from Evrogen, see, Bolte et al. (2004) J. Cell Science 117:943-54). For additional selectable markers, see generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Sci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724. The above list of selectable marker genes is not meant to be limiting. Any selectable marker gene can be used.

### VI. Compositions Comprising Silencing Elements

One or more of the polynucleotides comprising the silencing element can be provided as an external composition such as a spray or powder to the plant, plant part, seed, a pest, or an area of cultivation. In another example, a plant is transformed with a DNA construct or expression cassette for expression of at least one silencing element. In either composition, the silencing element, when ingested by an insect, can reduce the level of a target pest sequence and thereby control the pest (i.e., a Coleopteran plant pest including a *Diabrotica* plant pest, such as, *D. virgifera virgifera, D. barberi, D. virgifera zeae, D. speciosa,* or *D*. *undecimpunctata howardi*). It is recognized that the composition can comprise a cell (such as plant cell or a bacterial cell), in which a polynucleotide encoding the silencing element is stably incorporated into the genome and operably linked to promoters active in the cell. Compositions comprising a mixture of cells, some cells expressing at least one silencing element are also encompassed. Compositions comprising the silencing elements may not be contained in a cell, in which case the composition can be applied to an area inhabited by a pest. The composition may be applied externally to a plant (i.e., by spraying a field or area of cultivation) to protect the plant from the pest.. Methods of applying nucleotides in such a manner are known to those of skill in the art.

The composition of the invention can further be formulated as bait, in which case the compositions comprise a food substance or an attractant which enhances the attractiveness of the composition to the pest.

The composition comprising the silencing element can be formulated in an agriculturally suitable and/or environmentally acceptable carrier. Such carriers can be any material that the animal, plant or environment to be treated can tolerate. Furthermore, the carrier must be such that the composition remains effective at controlling a pest. Examples of such carriers include water, saline, Ringer's solution, dextrose or other sugar solutions, Hank's solution, and other aqueous physiologically balanced salt solutions, phosphate buffer, bicarbonate buffer and Tris buffer. In addition, the composition may include compounds that increase the half-life of a composition. Various insecticidal formulations can also be found in, for example, US Publications 2008/0275115, 2008/0242174, 2008/0027143, 2005/0042245, and 2004/0127520.

It is recognized that the polynucleotides comprising sequences encoding the silencing element can be used to transform organisms to provide for host organism production of these components, and subsequent application of the host organism to the environment of the target pest(s). Such host organisms include baculoviruses, bacteria, and the like. In this manner, the combination of polynucleotides encoding the silencing element may be introduced via a suitable vector into a microbial host, and said host applied to the environment, or to plants or animals.

The term "introduced" in the context of inserting a nucleic acid into a cell, means "transfection" or "transformation" or "transduction" and includes reference to the incorporation of a nucleic acid into a eukaryotic or prokaryotic cell where the nucleic acid may be stably incorporated into the genome of the cell (e.g., chromosome, plasmid, plastid, or mitochondrial DNA), converted into an autonomous replicon, or transiently expressed (e.g., transfected mRNA).

Microbial hosts that are known to occupy the "phytosphere" (phylloplane, phyllosphere, rhizosphere, and/or rhizoplana) of one or more crops of interest may be selected. These microorganisms are selected so as to be capable of successfully competing in the particular environment with the wild-type microorganisms, provide for stable maintenance and expression of the sequences encoding the silencing element, and desirably, provide for improved protection of the components from environmental degradation and inactivation.

Such microorganisms include bacteria, algae, and fungi. Of particular interest are microorganisms such as bacteria, e.g., *Pseudomonas, Erwinia, Serratia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc,* and *Alcaligenes,* fungi, particularly yeast, e.g., *Saccharomyces, Cryptococcus, Kluyveromyces, Sporobolomyces, Rhodotorula,* and *Aureobasidium.* Of particular interest are such phytosphere bacterial species as *Pseudomonas syringae, Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacter xyli* and *Azotobacter vinlandir,* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces rosues, S. odorus, Kluyveromyces veronae,* and *Aureobasidium pollulans.* Of particular interest are the pigmented microorganisms.

A number of ways are available for introducing the polynucleotide comprising the silencing element into the microbial host under conditions that allow for stable maintenance and expression of such nucleotide encoding sequences. For example, expression cassettes can be constructed which include the nucleotide constructs of interest operably linked with the transcriptional and translational regulatory signals for expression of the nucleotide constructs, and a nucleotide sequence homologous with a sequence in the host organism, whereby integration will occur, and/or a replication system that is functional in the host, whereby integration or stable maintenance will occur.

Transcriptional and translational regulatory signals include, but are not limited to, promoters, transcriptional initiation start sites, operators, activators, enhancers, other regulatory elements, ribosomal binding sites, an initiation codon, termination signals, and the like. See, for example, U.S. Patent Nos. 5,039,523 and 4,853,331; EPO 0480762A2; Sambrook et al. (2000); Molecular Cloning: A Laboratory Manual (3rd ed.; Cold Spring Harbor Laboratory Press, Plainview, NY); Davis et al. (1980) Advanced Bacterial Genetics (Cold Spring Harbor Laboratory, Cold Spring Harbor, NY); and the references cited therein.

Suitable host cells include the prokaryotes and the lower eukaryotes, such as fungi. Illustrative prokaryotes, both Gram-negative and Gram-positive, include *Enterobacteriaceae,* such as *Escherichia, Erwinia, Shigella, Salmonella, and Proteus; Bacillaceae; Rhizobiceae,* such as *Rhizobium; Spirillaceae,* such as photobacterium, *Zymomonas*, *Serratia, Aeromonas, Vibrio, Desulfovibrio, Spirillum; Lactobacillaceae; Pseudomonadaceae,* such as *Pseudomonas* and *Acetobacter; Azotobacteraceae* and *Nitrobacteraceae.* Among eukaryotes are fungi, such as *Phycomycetes* and *Ascomycetes,* which includes yeast, such as *Saccharomyces* and *Schizosaccharomyces;* and *Basidiomycetes* yeast, such as *Rhodotorula, Aureobasidium, Sporobolomyces,* and the like.

Characteristics of particular interest in selecting a host cell for purposes of the invention include ease of introducing the coding sequence into the host, availability of expression systems, efficiency of expression, stability in the host, and the presence of auxiliary genetic capabilities. Characteristics of interest for use as a pesticide microcapsule include protective qualities, such as thick cell walls, pigmentation, and intracellular packaging or formation of inclusion bodies; leaf affinity; lack of mammalian toxicity; attractiveness to pests for ingestion; and the like. Other considerations include ease of formulation and handling, economics, storage stability, and the like.

Host organisms of particular interest include yeast, such as *Rhodotorula spp., Aureobasidium spp., Saccharomyces spp.,* and *Sporobolomyces spp.,* phylloplane organisms such as *Pseudomonas spp., Erwinia spp.,* and *Flavobacterium spp.,* and other such organisms, including *Pseudomonas aeruginosa, Pseudomonas fluorescens, Saccharomyces cerevisiae, Bacillus thuringiensis, Escherichia coli, Bacillus subtilis,* and the like.

The sequences encoding the silencing elements encompassed by the invention can be introduced into microorganisms that multiply on plants (epiphytes) to deliver these components to potential target pests. Epiphytes, for example, can be gram-positive or gram-negative bacteria.

The silencing element can be fermented in a bacterial host and the resulting bacteria processed and used as a microbial spray in the same manner that *Bacillus thuringiensis* strains have been used as insecticidal sprays. Any suitable microorganism can be used for this purpose. By way of example, *Pseudomonas* has been used to express *Bacillus thuringiensis* endotoxins as encapsulated proteins and the resulting cells processed and sprayed as an insecticide Gaertner et al. (1993), in Advanced Engineered Pesticides, ed. L. Kim (Marcel Decker, Inc.).

Alternatively, the components of the invention are produced by introducing heterologous genes into a cellular host. Expression of the heterologous sequences results, directly or indirectly, in the intracellular production of the silencing element. These compositions may then be formulated in accordance with conventional techniques for application to the environment hosting a target pest, e.g., soil, water, and foliage of plants. See, for example, EPA 0192319, and the references cited therein.

In the present invention, a transformed microorganism can be formulated with an acceptable carrier into separate or combined compositions that are, for example, a suspension, a solution, an emulsion, a dusting powder, a dispersible granule, a wettable powder, and an emulsifiable concentrate, an aerosol, an impregnated granule, an adjuvant, a coatable paste, and also encapsulations in, for example, polymer substances.

Such compositions disclosed above may be obtained by the addition of a surface-active agent, an inert carrier, a preservative, a humectant, a feeding stimulant, an attractant, an encapsulating agent, a binder, an emulsifier, a dye, a UV protectant, a buffer, a flow agent or fertilizers, micronutrient donors, or other preparations that influence plant growth. One or more agrochemicals including, but not limited to, herbicides, insecticides, fungicides, bactericides, nematicides, molluscicides, acaracides, plant growth regulators, harvest aids, and fertilizers, can be combined with carriers, surfactants or adjuvants customarily employed in the art of formulation or other components to facilitate product handling and application for particular target pests. Suitable carriers and adjuvants can be solid or liquid and correspond to the substances ordinarily employed in formulation technology, e.g., natural or regenerated mineral substances, solvents, dispersants, wetting agents, tackifiers, binders, or fertilizers. The active ingredients of the present invention (i.e., at least one silencing element) are normally applied in the form of compositions and can be applied to the crop area, plant, or seed to be treated. For example, the compositions may be applied to grain in preparation for or during storage in a grain bin or silo, *etc.* The compositions may be applied simultaneously or in succession with other compounds. Methods of applying an active ingredient or a composition that contains at least one silencing element include, but are not limited to, foliar application, seed coating, and soil application. The number of applications and the rate of application depend on the intensity of infestation by the corresponding pest.

Suitable surface-active agents include, but are not limited to, anionic compounds such as a carboxylate of, for example, a metal; carboxylate of a long chain fatty acid; an N-acylsarcosinate; mono- or di-esters of phosphoric acid with fatty alcohol ethoxylates or salts of such esters; fatty alcohol sulfates such as sodium dodecyl sulfate, sodium octadecyl sulfate, or sodium cetyl sulfate; ethoxylated fatty alcohol sulfates; ethoxylated alkylphenol sulfates; lignin sulfonates; petroleum sulfonates; alkyl aryl sulfonates such as alkyl-benzene sulfonates or lower alkylnaphtalene sulfonates, e.g., butyl-naphthalene sulfonate; salts of sulfonated naphthalene-formaldehyde condensates; salts of sulfonated phenol-formaldehyde condensates; more complex sulfonates such as the amide sulfonates, e.g., the sulfonated condensation product of oleic acid and N-methyl taurine; or the dialkyl sulfosuccinates, e.g., the sodium sulfonate or dioctyl succinate. Non-ionic agents include condensation products of fatty acid esters, fatty alcohols, fatty acid amides or fatty-alkyl- or alkenyl-substituted phenols with ethylene oxide, fatty esters of polyhydric alcohol ethers, e.g., sorbitan fatty acid esters, condensation products of such esters with ethylene oxide, e.g., polyoxyethylene sorbitan fatty acid esters, block copolymers of ethylene oxide and propylene oxide, acetylenic glycols such as 2,4,7,9-tetraethyl-5-decyn-4,7-diol, or ethoxylated acetylenic glycols. Examples of a cationic surface-active agent include, for instance, an aliphatic mono-, di-, or polyamine such as an acetate, naphthenate or oleate; or oxygen-containing amine such as an amine oxide of polyoxyethylene alkylamine; an amide-linked amine prepared by the condensation of a carboxylic acid with a di- or polyamine; or a quaternary ammonium salt.

Examples of inert materials include, but are not limited to, inorganic minerals such as kaolin, phyllosilicates, carbonates, sulfates, phosphates, or botanical materials such as cork, powdered corncobs, peanut hulls, rice hulls, and walnut shells.

The compositions comprising the silencing element can be in a suitable form for direct application or as a concentrate of primary composition that requires dilution with a suitable quantity of water or other dilutant before application.

The compositions (including the transformed microorganisms) can be applied to the environment of an insect pest (such as a Coleoptera plant pest or a *Diabrotica* plant pest) by, for example, spraying, atomizing, dusting, scattering, coating or pouring, introducing into or on the soil, introducing into irrigation water, by seed treatment or general application or dusting at the time when the pest has begun to appear or before the appearance of pests as a protective measure. For example, the composition(s) and/or transformed microorganism(s) may be mixed with grain to protect the grain during storage. It is generally important to obtain good control of pests in the early stages of plant growth, as this is the time when the plant can be most severely damaged. The compositions can conveniently contain another insecticide if this is thought necessary. The composition(s) may be applied directly to the soil, at a time of planting, in granular form of a composition of a carrier and dead cells of a *Bacillus* strain or transformed microorganism of the invention. Also disclosed is a granular form of a composition comprising an agrochemical such as, for example, a herbicide, an insecticide, a fertilizer, in an inert carrier, and dead cells of a *Bacillus* strain or transformed microorganism of the invention.

### VII. Plants, Plant Parts, and Methods of Introducing Sequences into Plants

Disclosed are methods of introducing a polynucleotide into a plant. "Introducing" is intended to mean presenting to the plant the polynucleotide in such a manner that the sequence gains access to the interior of a cell of the plant. The methods of the invention do not depend on a particular method for introducing a sequence into a plant, only that the polynucleotide or polypeptides gains access to the interior of at least one cell of the plant. Methods for introducing polynucleotides into plants are known in the art including, but not limited to, stable transformation methods, transient transformation methods, and virus-mediated methods.

"Stable transformation" is intended to mean that the nucleotide construct introduced into a plant integrates into the genome of the plant and is capable of being inherited by the progeny thereof. "Transient transformation" is intended to mean that a polynucleotide is introduced into the plant and does not integrate into the genome of the plant or a polypeptide is introduced into a plant.

Transformation protocols as well as protocols for introducing polypeptides or polynucleotide sequences into plants may vary depending on the type of plant or plant cell, i.e., monocot or dicot, targeted for transformation. Suitable methods of introducing polypeptides and polynucleotides into plant cells include microinjection (Crossway et al. (1986) Biotechniques 4:320-334), electroporation (Riggs et al. (1986) Proc. Natl. Acad. Sci. USA 83:5602-5606, *Agrobacterium*-mediated transformation (U.S. Patent No. 5,563,055 and U.S. Patent No. 5,981,840), direct gene transfer (Paszkowski et al. (1984) EMBO J. 3:2717-2722), and ballistic particle acceleration (see, for example, U.S. Patent Nos. 4,945,050; U.S. Patent No. 5,879,918; U.S. Patent No. 5,886,244; and, 5,932,782; Tomes et al. (1995) in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); U.S. Patent Nos. 5,240,855; 5,322,783; and, 5,324,646; Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*).

The silencing element sequences of the invention can be provided to a plant using a variety of transient transformation methods. Such transient transformation methods include, but are not limited to, the introduction of the protein or variants and fragments thereof directly into the plant or the introduction of the transcript into the plant. Such methods include, for example, microinjection or particle bombardment. See, for example, Crossway et al. (1986) Mol Gen. Genet. 202:179-185; Nomura et al. (1986) Plant Sci. 44:53-58; Hepler et al. (1994) Proc. Natl. Acad. Sci. 91: 2176-2180 and Hush et al. (1994) The Journal of Cell Science 107:775-784. Alternatively, polynucleotides can be transiently transformed into the plant using techniques known in the art. Such techniques include viral vector systems and the precipitation of the polynucleotide in a manner that precludes subsequent release of the DNA. Thus, the transcription from the particle-bound DNA can occur, but the frequency with which it is released to become integrated into the genome is greatly reduced. Such methods include the use of particles coated with polyethylimine (PEI; Sigma #P3143).

Polynucleotide of the invention may be introduced into plants by contacting plants with a virus or viral nucleic acids. Generally, such methods involve incorporating a nucleotide construct of the invention within a viral DNA or RNA molecule. Further, it is recognized that promoters of the invention also encompass promoters utilized for transcription by viral RNA polymerases. Methods for introducing polynucleotides into plants and expressing a protein encoded therein, involving viral DNA or RNA molecules, are known in the art. See, for example, U.S. Patent Nos. 5,889,191, 5,889,190, 5,866,785, 5,589,367, 5,316,931, and Porta et al. (1996) Molecular Biotechnology 5:209-221.

Methods are known in the art for the targeted insertion of a polynucleotide at a specific location in the plant genome. The insertion of the polynucleotide at a desired genomic location may be achieved using a site-specific recombination system. See, for example, WO99/25821, WO99/25854, WO99/25840, WO99/25855, and WO99/25853. Briefly, the polynucleotide of the invention can be contained in transfer cassette flanked by two non-recombinogenic recombination sites. The transfer cassette is introduced into a plant having stably incorporated into its genome a target site which is flanked by two non-recombinogenic recombination sites that correspond to the sites of the transfer cassette. An appropriate recombinase is provided and the transfer cassette is integrated at the target site. The polynucleotide of interest is thereby integrated at a specific chromosomal position in the plant genome.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide of the invention, for example, an expression cassette of the invention, stably incorporated into their genome.

As used herein, the term plant includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like. Grain is intended to mean the mature seed produced by commercial growers for purposes other than growing or reproducing the species. Progeny, variants, and mutants of the regenerated plants are also included within the scope of the invention, provided that these parts comprise the introduced polynucleotides.

The present invention may be used for transformation of any plant species, including, but not limited to, monocots and dicots. Examples of plant species of interest include, but are not limited to, corn (*Zea mays*), *Brassica* sp. (e.g., *B. napus, B. rapa, B. juncea*), particularly those *Brassica* species useful as sources of seed oil, alfalfa (*Medicago sativa*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanuts (*Arachis hypogaea*), cotton (*Gossypium barbadense, Gossypium hirsutum*), sweet potato (*Ipomoea batatus*), cassava (*Manihot esculenta*), coffee (*Coffea* spp.), coconut (*Cocos nucifera*), pineapple (*Ananas comosus*), citrus trees (*Citrus* spp.), cocoa (*Theobroma cacao*), tea (*Camellia sinensis*), banana (*Musa* spp.), avocado (*Persea americana*), fig (*Ficus casica*), guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum* spp.), oats, barley, vegetables, ornamentals, and conifers.

Vegetables include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (*C*. *sativus*), cantaloupe (*C*. *cantalupensis),* and musk melon (*C*. *melo*). Ornamentals include azalea (*Rhododendron* spp.), hydrangea (*Macrophylla hydrangea*), hibiscus (*Hibiscus rosasanensis*), roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*), and chrysanthemum.

Conifers that may be employed include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*); Douglas-fir (*Pseudotsuga menziesii*); Western hemlock (*Tsuga canadensis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*). In specific embodiments, plants of the present invention are crop plants (for example, alfalfa, sunflower, soybean, cotton, safflower, wheat, millet, tobacco, etc.). In other embodiments, soybean plants are optimal Corn plants, *Brassica,* peanut, sorghum and sugar cane plants may also be used.

Other plants of interest include grain plants that provide seeds of interest, oil-seed plants, and leguminous plants. Seeds of interest include grain seeds, such as corn, wheat, barley, rice, sorghum, rye, etc. Oil-seed plants include cotton, soybean, safflower, sunflower, *Brassica,* maize, alfalfa, palm, coconut, etc. Leguminous plants include beans and peas. Beans include guar, locust bean, fenugreek, soybean, garden beans, cowpea, mungbean, lima bean, fava bean, lentils, chickpea, etc.

### VIII. Stacking of Traits in Transgenic Plant

Transgenic plants may comprise a stack of one or more target polynucleotides as set forth in SEQ ID NOS: 574, 575, 576, 728, or fragments thereof, or complements thereof, as disclosed herein with one or more additional polynucleotides resulting in the production or suppression of multiple polypeptide sequences. Transgenic plants comprising stacks of polynucleotide sequences can be obtained by either or both of traditional breeding methods or through genetic engineering methods. These methods include, but are not limited to, breeding individual lines each comprising a polynucleotide of interest, transforming a transgenic plant comprising an expression construct comprising various target polynucleotides as set forth in SEQ ID NOS: 574, 575, 576, 728, or fragments thereof, or complements thereof, as disclosed herein with a subsequent gene and co-transformation of genes into a single plant cell. As used herein, the term "stacked" includes having the multiple traits present in the same plant (i.e., both traits are incorporated into the nuclear genome, one trait is incorporated into the nuclear genome and one trait is incorporated into the genome of a plastid or both traits are incorporated into the genome of a plastid). In one non-limiting example, "stacked traits" comprise a molecular stack where the sequences are physically adjacent to each other. A trait, as used herein, refers to the phenotype derived from a particular sequence or groups of sequences. Co-transformation of polynucleotides can be carried out using single transformation vectors comprising multiple polynucleotides or polynucleotides carried separately on multiple vectors. If the sequences are stacked by genetically transforming the plants, the polynucleotide sequences of interest can be combined at any time and in any order. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. It is further recognized that polynucleotide sequences can be stacked at a desired genomic location using a site-specific recombination system. *See,* for example, WO 1999/25821, WO 1999/25854, WO 1999/25840, WO 1999/25855 and WO 1999/25853.

In some embodiments the various target polynucleotides as set forth in SEQ ID NOS: 574, 575, 576, 728, or fragments thereof, or complements thereof, as disclosed herein, alone or stacked with one or more additional insect resistance traits can be stacked with one or more additional input traits (e.g., herbicide resistance, fungal resistance, virus resistance, stress tolerance, disease resistance, male sterility, stalk strength, and the like) or output traits (e.g., increased yield, modified starches, improved oil profile, balanced amino acids, high lysine or methionine, increased digestibility, improved fiber quality, drought resistance, and the like). Thus, the polynucleotide embodiments can be used to provide a complete agronomic package of improved crop quality with the ability to flexibly and cost effectively control any number of agronomic pests.

Transgenes useful for stacking include, but are not limited to, to those as described herein below.

### i. Transgenes that Confer Resistance to Insects or Disease

(A) Plant disease resistance genes. Plant defenses are often activated by specific interaction between the product of a disease resistance gene (R) in the plant and the product of a corresponding avirulence (Avr) gene in the pathogen. A plant variety can be transformed with cloned resistance gene to engineer plants that are resistant to specific pathogen strains. *See,* for example, Jones, et al., (1994) Science 266:789 (cloning of the tomato Cf-9 gene for resistance to Cladosporium fulvum); Martin, et al., (1993) Science 262:1432 (tomato Pto gene for resistance to Pseudomonas syringae pv. tomato encodes a protein kinase); Mindrinos, et al., (1994) Cell 78:1089 (Arabidopsis RSP2 gene for resistance to Pseudomonas syringae), McDowell and Woffenden, (2003) Trends Biotechnol. 21(4):178-83 and Toyoda, et al., (2002) Transgenic Res. 11(6):567-82. A plant resistant to a disease is one that is more resistant to a pathogen as compared to the wild type plant.
(B) Genes encoding a Bacillus thuringiensis protein, a derivative thereof or a synthetic polypeptide modeled thereon. *See,* for example, Geiser, et al., (1986) Gene 48:109, who disclose the cloning and nucleotide sequence of a Bt delta-endotoxin gene. Moreover, DNA molecules encoding delta-endotoxin genes can be purchased from American Type Culture Collection (Rockville, Md.), for example, under ATCC Accession Numbers 40098, 67136, 31995 and 31998. Other non-limiting examples of Bacillus thuringiensis transgenes being genetically engineered are given in the following patents and patent applications: U.S. Pat. Nos. 5,188,960; 5,689,052; 5,880,275; 5,986,177; 6,023,013, 6,060,594, 6,063,597, 6,077,824, 6,620,988, 6,642,030, 6,713,259, 6,893,826, 7,105,332; 7,179,965, 7,208,474; 7,227,056, 7,288,643, 7,323,556, 7,329,736, 7,449,552, 7,468,278, 7,510,878, 7,521,235, 7,544,862, 7,605,304, 7,696,412, 7,629,504, 7,705,216, 7,772,465, 7,790,846, 7,858,849 and WO 1991/14778; WO 1999/31248; WO 2001/12731; WO 1999/24581 and WO 1997/40162.
   Genes encoding pesticidal proteins may also be stacked including but are not limited to: insecticidal proteins from Pseudomonas sp. such as PSEEN3174 (Monalysin, (2011) PLoS Pathogens, 7:1-13), from Pseudomonas protegens strain CHAO and Pf-5 (previously fluorescens) (Pechy-Tarr, (2008) Environmental Microbiology 10:2368-2386: Gen Bank Accession No. EU400157); from Pseudomonas Taiwanensis (Liu, et al., (2010) J. Agric. Food Chem. 58:12343-12349) and from Pseudomonas pseudoalcligenes (Zhang, et al., (2009) Annals of Microbiology 59:45-50 and Li, et al., (2007) Plant Cell Tiss. Organ Cult. 89:159-168); insecticidal proteins from Photorhabdus sp. and Xenorhabdus sp. (Hinchliffe, et al., (2010) The Open Toxinology Journal 3:101-118 and Morgan, et al., (2001) Applied and Envir. Micro. 67:2062-2069), U.S. Pat. No. 6,048,838, and U.S. Pat. No. 6,379,946; and .delta.-endotoxins including, but not limited to, the Cry1, Cry2, Cry3, Cry4, Cry5, Cry6, Cry7, Cry8, Cry9, Cry10, Cry11, Cry12, Cry13, Cry14, Cry15, Cry16, Cry17, Cry18, Cry19, Cry20, Cry21, Cry22, Cry23, Cry24, Cry25, Cry26, Cry27, Cry 28, Cry 29, Cry 30, Cry31, Cry32, Cry33, Cry34, Cry35, Cry36, Cry37, Cry38, Cry39, Cry40, Cry41, Cry42, Cry43, Cry44, Cry45, Cry 46, Cry47, Cry49, Cry 51 and Cry55 classes of delta-endotoxin genes and the B. thuringiensis cytolytic Cyt1 and Cyt2 genes. Members of these classes of B. thuringiensis insecticidal proteins include, but are not limited to Cry1Aa1 (Accession #Accession #M11250), Cry1Aa2 (Accession #M10917), Cry1Aa3 (Accession #D00348), Cry1Aa4 (Accession #X13535), Cry1Aa5 (Accession #D17518), Cry1Aa6 (Accession #U43605), Cry1Aa7 (Accession #AF081790), Cry1Aa8 (Accession #126149), Cry1Aa9 (Accession #AB026261), Cry1Aa10 (Accession #AF154676), Cry1Aa11 (Accession #Y09663), Cry1Aa12 (Accession #AF384211), Cry1Aa13 (Accession #AF510713), Cry1Aa14 (Accession #AY197341), Cry1Aa15 (Accession #DQ062690), Cry1Ab1 (Accession #M13898), Cry1Ab2 (Accession #M12661), Cry1Ab3 (Accession #M15271), Cry1Ab4 (Accession #D00117), Cry1Ab5 (Accession #X04698), Cry1Ab6 (Accession #M37263), Cry1Ab7 (Accession #X13233), Cry1Ab8 (Accession #M16463), Cry1Ab9 (Accession #X54939), Cry1Ab10 (Accession #A29125), Cry1Ab11 (Accession #112419), Cry1Ab12 (Accession #AF059670), Cry1Ab13 (Accession #AF254640), Cry1Ab14 (Accession #U94191), Cry1Ab15 (Accession #AF358861), Cry1Ab16 (Accession #AF375608), Cry1Ab17 (Accession #AAT46415), Cry1Ab18 (Accession #AAQ88259), Cry1Ab19 (Accession #AY847289), Cry1Ab20 (Accession #DQ241675), Cry1Ab21 (Accession #EF683163), Cry1Ab22 (Accession #ABW87320), Cry1Ab-like (Accession #AF327924), Cry1Ab-like (Accession #AF327925), Cry1Ab-like (Accession #AF327926), Cry1Ab-like (Accession #DQ781309), Cry1Ac1 (Accession #M11068), Cry1Ac2 (Accession #M35524), Cry1Ac3 (Accession #X54159), Cry1Ac4 (Accession #M73249), Cry1Ac5 (Accession #M73248), Cry1Ac6 (Accession #U43606), Cry1Ac7 (Accession #U87793), Cry1Ac8 (Accession #U87397), Cry1Ac9 (Accession #U89872), Cry1Ac10 (Accession #AJ002514), Cry1Ac11 (Accession #AJ130970), Cry1Ac12 (Accession #112418), Cry1Ac13 (Accession #AF148644), Cry1Ac14 (Accession #AF492767), Cry1Ac15 (Accession #AY122057), Cry1Ac16 (Accession #AY730621), Cry1Ac17 (Accession #AY925090), Cry1Ac18 (Accession #DQ023296), Cry1Ac19 (Accession #DQ195217), Cry1Ac20 (Accession #DQ285666), Cry1Ac21 (Accession #DQ062689), Cry1Ac22 (Accession #EU282379), Cry1Ac23 (Accession #AM949588), Cry1Ac24 (Accession #ABL01535), Cry1Ad1 (Accession #M73250), Cry1Ad2 (Accession #A27531), Cry1Ae1 (Accession #M65252), Cry1Af1 (Accession #U82003), Cry1Ag1 (Accession #AF081248), Cry1Ah1 (Accession #AF281866), Cry1Ah2 (Accession #DQ269474), Cry1Ai1 (Accession #AY174873), Cry1A-like (Accession #AF327927), Cry1Ba1 (Accession #X06711), Cry1Ba2 (Accession #X95704), Cry1Ba3 (Accession #AF368257), Cry1Ba4 (Accession #AF363025), Cry1Ba5 (Accession #AB020894), Cry1Ba6 (Accession #ABL60921), Cry1Bb1 (Accession #L32020), Cry1Bc1 (Accession #Z46442), Cry1Bd1 (Accession #U70726), Cry1Bd2 (Accession #AY138457), Cry1Be1 (Accession #AF077326), Cry1Be2 (Accession #AAQ52387), Cry1Bf1 (Accession #AX189649), Cry1Bf2 (Accession #AAQ52380), Cry1Bg1 (Accession #AY176063), Cry1Ca1 (Accession #X07518), Cry1Ca2 (Accession #X13620), Cry1Ca3 (Accession #M73251), Cry1Ca4 (Accession #A27642), Cry1Ca5 (Accession #X96682), Cry1Ca6 [1] (Accession #AF215647), Cry1Ca7 (Accession #AY015492), Cry1Ca8 (Accession #AF362020), Cry1Ca9 (Accession #AY078160), Cry1Ca10 (Accession #AF540014), Cry1Ca11 (Accession #AY955268), Cry1Cb1 (Accession #M97880), Cry1Cb2 (Accession #AY007686), Cry1Cb3 (Accession #EU679502), Cry1Cb-like (Accession #AAX63901), Cry1Da1 (Accession #X54160), Cry1Da2 (Accession #176415), Cry1Db1 (Accession #Z22511), Cry1 Db2 (Accession #AF358862), Cry1 Dc1 (Accession #EF059913), Cry1Ea1 (Accession #X53985), Cry1Ea2 (Accession #X56144), Cry1Ea3 (Accession #M73252), Cry1Ea4 (Accession #U94323), Cry1Ea5 (Accession #A15535), Cry1Ea6 (Accession #AF202531), Cry1 Ea7 (Accession #AAW72936), Cry1 Ea8 (Accession #ABX11258), Cry1Eb1 (Accession #M73253), Cry1Fa1 (Accession #M63897), Cry1Fa2 (Accession #M73254), Cry1Fb1 (Accession #Z22512), Cry1Fb2 (Accession #AB012288), Cry1Fb3 (Accession #AF062350), Cry1Fb4 (Accession #173895), Cry1Fb5 (Accession #AF336114), Cry1Fb6 (Accession #EU679500), Cry1Fb7 (Accession #EU679501), Cry1Ga1 (Accession #Z22510), Cry1Ga2 (Accession #Y09326), Cry1Gb1 (Accession #U70725), Cry1Gb2 (Accession #AF288683), CrylGc (Accession #AAQ52381), Cry1Ha1 (Accession #Z22513), Cry1Hb1 (Accession #U35780), Cry1H-like (Accession #AF182196), Cry1Ia1 (Accession #X62821), Cry1Ia2 (Accession #M98544), Cry1Ia3 (Accession #L36338), Cry1Ia4 (Accession #L49391), Cry1Ia5 (Accession #Y08920), Cry1Ia6 (Accession #AF076953), Cry1Ia7 (Accession #AF278797), Cry1Ia8 (Accession #AF373207), Cry1Ia9 (Accession #AF521013), Cry1Ia10 (Accession #AY262167), Cry1Ia11 (Accession #AJ315121), Cry1Ia12 (Accession #AAV53390), Cry1Ia13 (Accession #ABF83202), Cry1Ia14 (Accession #EU887515), Cry1Ib1 (Accession #U07642), Cry1Ib2 (Accession #ABW88019), Cry1Ib3 (Accession #EU677422), Cry1Ic1 (Accession #AF056933), Cry1Ic2 (Accession #AAE71691), Cry1Id1 (Accession #AF047579), Cry1Ie1 (Accession #AF211190), Cry1If1 (Accession #AAQ52382), Cry1I-like (Accession #190732), Cry1I-like (Accession #DQ781310), Cry1Ja1 (Accession #L32019), Cry1Jb1 (Accession #U31527), Cry1Jc1 (Accession #190730), Cry1Jc2 (Accession #AAQ52372), Cry1Jd1 (Accession #AX189651), Cry1Ka1 (Accession #U28801), Cry1La1 (Accession #AAS60191), Cry1-like (Accession #190729), Cry2Aa1 (Accession #M31738), Cry2Aa2 (Accession #M23723), Cry2Aa3 (Accession #D86064), Cry2Aa4 (Accession #AF047038), Cry2Aa5 (Accession #AJ 132464), Cry2Aa6 (Accession #AJ 132465), Cry2Aa7 (Accession #AJ132463), Cry2Aa8 (Accession #AF252262), Cry2Aa9 (Accession #AF273218), Cry2Aa10 (Accession #AF433645), Cry2Aa11 (Accession #AAQ52384), Cry2Aa12 (Accession #DQ977646), Cry2Aa13 (Accession #ABL01536), Cry2Aa14 (Accession #ACF04939), Cry2Ab1 (Accession #M23724), Cry2Ab2 (Accession #X55416), Cry2Ab3 (Accession #AF164666), Cry2Ab4 (Accession #AF336115), Cry2Ab5 (Accession #AF441855), Cry2Ab6 (Accession #AY297091), Cry2Ab7 (Accession #DQ119823), Cry2Ab8 (Accession #DQ361266), Cry2Ab9 (Accession #DQ341378), Cry2Ab10 (Accession #EF157306), Cry2Ab11 (Accession #AM691748), Cry2Ab12 (Accession #ABM21764), Cry2Ab13 (Accession #EU909454), Cry2Ab14 (Accession #EU909455), Cry2Ac1 (Accession #X57252), Cry2Ac2 (Accession #AY007687), Cry2Ac3 (Accession #AAQ52385), Cry2Ac4 (Accession #DQ361267), Cry2Ac5 (Accession #DQ341379), Cry2Ac6 (Accession #DQ359137), Cry2Ac7 (Accession #AM292031), Cry2Ac8 (Accession #AM421903), Cry2Ac9 (Accession #AM421904), Cry2Ac10 (Accession #BI 877475), Cry2Ac11 (Accession #AM689531), Cry2Ac12 (Accession #AM689532), Cry2Ad1 (Accession #AF200816), Cry2Ad2 (Accession #DQ358053), Cry2Ad3 (Accession #AM268418), Cry2Ad4 (Accession #AM490199), Cry2Ad5 (Accession #AM765844), Cry2Ae1 (Accession #AAQ52362), Cry2Af1 (Accession #EF439818), Cry2Ag (Accession #ACH91610), Cry2Ah (Accession #EU939453), Cry3Aa1 (Accession #M22472), Cry3Aa2 (Accession #J02978), Cry3Aa3 (Accession #Y00420), Cry3Aa4 (Accession #M30503), Cry3Aa5 (Accession #M37207), Cry3Aa6 (Accession #U10985), Cry3Aa7 (Accession #AJ237900), Cry3Aa8 (Accession #AAS79487), Cry3Aa9 (Accession #AAWO5659), Cry3Aa10 (Accession #AAU29411), Cry3Aa11 (Accession #AY882576), Cry3Aa12 (Accession #ABY49136), Cry3Ba1 (Accession #X17123), Cry3Ba2 (Accession #A07234), Cry3Bb1 (Accession #M89794), Cry3Bb2 (Accession #U31633), Cry3Bb3 (Accession #115475), Cry3Ca1 (Accession #X59797), Cry4Aa1 (Accession #Y00423), Cry4Aa2 (Accession #D00248), Cry4Aa3 (Accession #AL731825), Cry4A-like (Accession #DQ078744), Cry4Ba1 (Accession #X07423), Cry4Ba2 (Accession #X07082), Cry4Ba3 (Accession #M20242), Cry4Ba4 (Accession #D00247), Cry4Ba5 (Accession #AL731825), Cry4Ba-like (Accession #ABC47686), Cry4Ca1 (Accession #EU646202), Cry5Aa1 (Accession #L07025), Cry5Ab1 (Accession #L07026), Cry5Ac1 (Accession #134543), Cry5Ad1 (Accession #EF219060), Cry5Ba1 (Accession #U19725), Cry5Ba2 (Accession #EU121522), Cry6Aa1 (Accession #L07022), Cry6Aa2 (Accession #AF499736), Cry6Aa3 (Accession #DQ835612), Cry6Ba1 (Accession #L07024), Cry7Aal (Accession #M64478), Cry7Ab1 (Accession #U04367), Cry7Ab2 (Accession #U04368), Cry7Ab3 (Accession #BI 1015188), Cry7Ab4 (Accession #EU380678), Cry7Ab5 (Accession #ABX9555), Cry7Ab6 (Accession #FJ194973), Cry7Ba1 (Accession #ABB70817), Cry7Ca1 (Accession #EF486523), Cry8Aa1 (Accession #U04364), Cry8Ab1 (Accession #EU044830), Cry8Ba1 (Accession #U04365), Cry8Bb1 (Accession #AX543924), Cry8Bc1 (Accession #AX543926), Cry8Ca1 (Accession #U04366), Cry8Ca2 (Accession #AAR98783), Cry8Ca3 (Accession #EU625349), Cry8Da1 (Accession #AB089299), Cry8Da2 (Accession #BD133574), Cry8Da3 (Accession #BD133575), Cry8 Db1 (Accession #AB303980), Cry8Ea1 (Accession #AY329081), Cry8Ea2 (Accession #EU047597), Cry8Fa1 (Accession #AY551093), Cry8Ga1 (Accession #AY590188), Cry8Ga2 (Accession #DQ318860), Cry8Ga3 (Accession #FJ198072), Cry8Ha1 (Accession #EF465532), Cry81a1 (Accession #EU381044), Cry8Ja1 (Accession #EU625348), Cry8 like (Accession #ABS53003), Cry9Aa1 (Accession #X58120), Cry9Aa2 (Accession #X58534), Cry9Aa like (Accession #AAQ52376), Cry9Ba1 (Accession #X75019), Cry9Bb1 (Accession #AY758316), Cry9Ca1 (Accession #Z37527), Cry9Ca2 (Accession #AAQ52375), Cry9Da1 (Accession #D85560), Cry9Da2 (Accession #AF042733), Cry9 Db1 (Accession #AY971349), Cry9Ea1 (Accession #AB011496), Cry9Ea2 (Accession #AF358863), Cry9Ea3 (Accession #EF157307), Cry9Ea4 (Accession #EU760456), Cry9Ea5 (Accession #EU789519), Cry9Ea6 (Accession #EU887516), Cry9Eb1 (Accession #AX189653), Cry9Ec1 (Accession #AF093107), Cry9Ed1 (Accession #AY973867), Cry9 like (Accession #AF093107), Cry10Aa1 (Accession #M12662), Cry10Aa2 (Accession #E00614), Cry10Aa3 (Accession #AL731825), Cry10A like (Accession #DQ167578), Cry1IAa1 (Accession #M31737), Cry1IAa2 (Accession #M22860), Cry1IAa3 (Accession #AL731825), Cry1IAa-like (Accession #DQ166531), Cry11Ba1 (Accession #X86902), Cry11Bb1 (Accession #AF017416), Cry12Aa1 (Accession #L07027), Cry13Aa1 (Accession #L07023), Cry14Aa1 (Accession #U13955), Cry15Aa1 (Accession #M76442), Cry16Aa1 (Accession #X94146), Cry17Aa1 (Accession #X99478), Cry18Aa1 (Accession #X99049), Cry18Ba1 (Accession #AF169250), Cry18Ca1 (Accession #AF169251), Cry19Aa1 (Accession #Y07603), Cry19Ba1 (Accession #D88381), Cry20Aa1 (Accession #U82518), Cry21Aa1 (Accession #132932), Cry21Aa2 (Accession #166477), Cry21Ba1 (Accession #AB088406), Cry22Aa1 (Accession #134547), Cry22Aa2 (Accession #AX472772), Cry22Aa3 (Accession #EU715020), Cry22Ab1 (Accession #AAK50456), Cry22Ab2 (Accession #AX472764), Cry22Ba1 (Accession #AX472770), Cry23Aa1 (Accession #AAF76375), Cry24Aa1 (Accession #U88188), Cry24Ba1 (Accession #BAD32657), Cry24Ca1 (Accession #AM158318), Cry25Aa1 (Accession #U88189), Cry26Aa1 (Accession #AF122897), Cry27Aa1 (Accession #AB023293), Cry28Aa1 (Accession #AF132928), Cry28Aa2 (Accession #AF285775), Cry29Aa1 (Accession #AJ251977), Cry30Aa1 (Accession #AJ251978), Cry30Ba1 (Accession #BAD00052), Cry30Ca1 (Accession #BAD67157), Cry30Da1 (Accession #EF095955), Cry30 Db1 (Accession #BAE80088), Cry30Ea1 (Accession #EU503140), Cry30Fa1 (Accession #EU751609), Cry30Ga1 (Accession #EU882064), Cry31Aa1 (Accession #AB031065), Cry31Aa2 (Accession #AY081052), Cry31Aa3 (Accession #AB250922), Cry31Aa4 (Accession #AB274826), Cry31Aa5 (Accession #AB274827), Cry31Ab1 (Accession #AB250923), Cry31Ab2 (Accession #AB274825), Cry31Ac1 (Accession #AB276125), Cry32Aa1 (Accession #AY008143), Cry32Ba1 (Accession #BAB78601), Cry32Ca1 (Accession #BAB78602), Cry32Da1 (Accession #BAB78603), Cry33Aa1 (Accession #AAL26871), Cry34Aa1 (Accession #AAG50341), Cry34Aa2 (Accession #AAK64560), Cry34Aa3 (Accession #AY536899), Cry34Aa4 (Accession #AY536897), Cry34Ab1 (Accession #AAG41671), Cry34Ac1 (Accession #AAG50118), Cry34Ac2 (Accession #AAK64562), Cry34Ac3 (Accession #AY536896), Cry34Ba1 (Accession #AAK64565), Cry34Ba2 (Accession #AY536900), Cry34Ba3 (Accession #AY536898), Cry35Aa1 (Accession #AAG50342), Cry35Aa2 (Accession #AAK64561), Cry35Aa3 (Accession #AY536895), Cry35Aa4 (Accession #AY536892), Cry35Ab1 (Accession #AAG41672), Cry35Ab2 (Accession #AAK64563), Cry35Ab3 (Accession #AY536891), Cry35Ac1 (Accession #AAG50117), Cry35Ba1 (Accession #AAK64566), Cry35Ba2 (Accession #AY536894), Cry35Ba3 (Accession #AY536893), Cry36Aa1 (Accession #AAK64558), Cry37Aa1 (Accession #AAF76376), Cry38Aa1 (Accession #AAK64559), Cry39Aa1 (Accession #BAB72016), Cry40Aa1 (Accession #BAB72018), Cry40Ba1 (Accession #BAC77648), Cry40Ca1 (Accession #EU381045), Cry40Da1 (Accession #EU596478), Cry41Aa1 (Accession #AB116649), Cry41Ab1 (Accession #AB116651), Cry42Aa1 (Accession #AB116652), Cry43Aa1 (Accession #AB115422), Cry43Aa2 (Accession #AB176668), Cry43Ba1 (Accession #AB115422), Cry43-like (Accession #AB115422), Cry44Aa (Accession #BAD08532), Cry45Aa (Accession #BAD22577), Cry46Aa (Accession #BAC79010), Cry46Aa2 (Accession #BAG68906), Cry46Ab (Accession #BAD35170), Cry47Aa (Accession #AY950229), Cry48Aa (Accession #AJ841948), Cry48Aa2 (Accession #AM237205), Cry48Aa3 (Accession #AM237206), Cry48Ab (Accession #AM237207), Cry48Ab2 (Accession #AM237208), Cry49Aa (Accession #AJ841948), Cry49Aa2 (Accession #AM237201), Cry49Aa3 (Accession #AM237203), Cry49Aa4 (Accession #AM237204), Cry49Ab1 (Accession #AM237202), Cry50Aa1 (Accession #AB253419), Cry51Aa1 (Accession #DQ836184), Cry52Aa1 (Accession #EF613489), Cry53Aa1 (Accession #EF633476), Cry54Aa1 (Accession #EU339367), Cry55Aa1 (Accession #EU121521), Cry55Aa2 (Accession #AAE33526).
   Examples of delta-endotoxins also include but are not limited to Cry1A proteins of U.S. Pat. Nos. 5,880,275 and 7,858,849; a DIG-3 or DIG-11 toxin (N-terminal deletion of alpha-helix 1 and/or alpha-helix 2 variants of Cry proteins such as Cry1A) of U.S. Pat. Nos. 8,304,604 and 8,304,605, CrylB of U.S. patent application Ser. No. 10/525,318; CrylC of U.S. Pat. No. 6,033,874; CrylF of U.S. Pat. Nos. 5,188,960, 6,218,188; Cry1A/F chimeras of U.S. Pat. Nos. 7,070,982; 6,962,705 and 6,713,063); a Cry2 protein such as Cry2Ab protein of U.S. Pat. No. 7,064,249); a Cry3A protein including but not limited to an engineered hybrid insecticidal protein (eHIP) created by fusing unique combinations of variable regions and conserved blocks of at least two different Cry proteins (US Patent Application Publication Number 2010/0017914); a Cry4 protein; a Cry5 protein; a Cry6 protein; Cry8 proteins of U.S. Pat. Nos. 7,329,736, 7,449,552, 7,803,943, 7,476,781, 7,105,332, 7,378,499 and 7,462,760; a Cry9 protein such as such as members of the Cry9A, Cry9B, Cry9C, Cry9D, Cry9E, and Cry9F families; a Cry15 protein of Naimov, et al., (2008) Applied and Environmental Microbiology 74:7145-7151; a Cry22, a Cry34Ab1 protein of U.S. Pat. Nos. 6,127,180, 6,624,145 and 6,340,593; a CryET33 and CryET34 protein of U.S. Pat. Nos. 6,248,535, 6,326,351, 6,399,330, 6,949,626, 7,385,107 and 7,504,229; a CryET33 and CryET34 homologs of US Patent Publication Number 2006/0191034, 2012/0278954, and PCT Publication Number WO 2012/139004; a Cry35Ab1 protein of U.S. Pat. Nos. 6,083,499, 6,548,291 and 6,340,593; a Cry46 protein, a Cry 51 protein, a Cry binary toxin; a TIC901 or related toxin; TIC807 of US 2008/0295207; ET29, ET37, TIC809, TIC810, TIC812, TIC127, TIC128 of PCT US 2006/033867; AXMI-027, AXMI-036, and AXMI-038 of U.S. Pat. No. 8,236,757; AXMI-***031***, AXMI-039, AXMI-040, AXMI-049 of U.S. Pat. No. 7,923,602; AXMI-018, AXMI-020, and AXMI-021 of WO 2006/083891; AXMI-010 of WO 2005/038032; AXMI-003 of WO 2005/021585; AXMI-008 of US 2004/0250311; AXMI-006 of US 2004/0216186; AXMI-007 of US 2004/0210965; AXMI-009 of US 2004/0210964; AXMI-014 of US 2004/0197917; AXMI-004 of US 2004/0197916; AXMI-028 and AXMI-029 of WO 2006/119457; AXMI-007, AXMI-008, AXMI-0080rf2, AXMI-009, AXMI-014 and AXMI-004 of WO 2004/074462; AXMI-150 of U.S. Pat. No. 8,084,416; AXMI-205 of US20110023184; AXMI-011, AXMI-012, AXMI-013, AXMI-015, AXMI-019, AXMI-044, AXMI-037, AXMI-043, AXMI-033, AXMI-034, AXMI-022, AXMI-023, AXMI-041, AXMI-063, and AXMI-064 of US 2011/0263488; AXMI-R1 and related proteins of US 2010/0197592; AXMI221Z, AXMI222z, AXMI223z, AXMI224z and AXMI225z of WO 2011/103248; AXMI218, AXMI219, AXMI220, AXMI226, AXMI227, AXMI228, AXMI229, AXMI230, and AXMI231 of WO11/103,247; AXMI-115, AXMI-113, AXMI-005, AXMI-163 and AXMI-184 of U.S. Pat. No. 8,334,431; AXMI-001, AXMI-002, AXMI-030, AXMI-035, and AXMI-045 of US 2010/0298211; AXMI-066 and AXMI-076 of US20090144852; AXMI128, AXMI130, AXMI131, AXMI133, AXMI140, AXMI141, AXMI142, AXMI143, AXMI144, AXMI146, AXMI148, AXMI149, AXMI152, AXMI153, AXMI154, AXMI155, AXMI156, AXMI157, AXMI158, AXMI162, AXMI165, AXMI166, AXMI167, AXMI168, AXMI169, AXMI170, AXMI171, AXMI172, AXMI173, AXMI174, AXMI175, AXMI176, AXMI177, AXMI178, AXMI179, AXMI180, AXMI181, AXMI182, AXMI185, AXMI186, AXMI187, AXMI188, AXMI189 of U.S. Pat. No. 8,318,900; AXMI079, AXMI080, AXMI081, AXMI082, AXMI091, AXMI092, AXMI096, AXMI097, AXMI098, AXMI099, AXMI100, AXMI101, AXMI102, AXMI103, AXMI104, AXMI107, AXMI108, AXMI109, AXMI110, AXMI111, AXMI112, AXMI114, AXMI116, AXMI117, AXMI118, AXMI119, AXMI120, AXMI121, AXMI122, AXMI123, AXMI124, AXMI1257, AXMI1268, AXMI127, AXMI129, AXMI164, AXMI151, AXMI161, AXMI183, AXMI132, AXMI138, AXMI137 of US 2010/0005543; Cry proteins such as Cry1A and Cry3A having modified proteolytic sites of U.S. Pat. No. 8,319,019; and a Cry1Ac, Cry2Aa and CrylCa toxin protein from Bacillus thuringiensis strain VBTS 2528 of US Patent Application Publication Number 2011/0064710. Other Cry proteins are well known to one skilled in the art (see, Crickmore, et al., "Bacillus thuringiensis toxin nomenclature" (2011), at lifesci.sussex.ac.uk/home/Neil_Crickmore/Bt/which can be accessed on the world-wide web using the "www" prefix). The insecticidal activity of Cry proteins is well known to one skilled in the art (for review, see, van Frannkenhuyzen, (2009) J. Invert. Path. 101:1-16). The use of Cry proteins as transgenic plant traits is well known to one skilled in the art and Cry-transgenic plants including but not limited to Cry1Ac, Cry1Ac+Cry2Ab, Cry1Ab, Cry1A.105, Cry1F, Cry1Fa2, Cry1F+Cry1Ac, Cry2Ab, Cry3A, mCry3A, Cry3Bb1, Cry34Ab1, Cry35Ab1, Vip3A, mCry3A, Cry9c and CBI-Bt have received regulatory approval (see, Sanahuja, (2011) Plant Biotech Journal 9:283-300 and the CERA (2010) GM Crop Database Center for Environmental Risk Assessment (CERA), ILSI Research Foundation, Washington D.C. at cera-gmc.org/index.php?action=gm_crop_database which can be accessed on the world-wide web using the "www" prefix). Pesticidal proteins also include insecticidal lipases including lipid acyl hydrolases of U.S. Pat. No. 7,491,869, and cholesterol oxidases such as from Streptomyces (Purcell et al. (1993) Biochem Biophys Res Commun 15:1406-1413). Pesticidal proteins also include VIP (vegetative insecticidal proteins) toxins of U.S. Pat. Nos. 5,877,012, 6,107,279, 6,137,033, 7,244,820, 7,615,686, and 8,237,020, and the like. Other VIP proteins are well known to one skilled in the art (see, lifesci.sussex.ac.uk/home/Neil_Crickmore/Bt/vip.html which can be accessed on the world-wide web using the "www" prefix). Pesticidal proteins also include toxin complex (TC) proteins, obtainable from organisms such as Xenorhabdus, Photorhabdus and Paenibacillus (see, U.S. Pat. Nos. 7,491,698 and 8,084,418). Some TC proteins have "stand alone" insecticidal activity and other TC proteins enhance the activity of the stand-alone toxins produced by the same given organism. The toxicity of a "stand-alone" TC protein (from Photorhabdus, Xenorhabdus or Paenibacillus, for example) can be enhanced by one or more TC protein "potentiators" derived from a source organism of a different genus. There are three main types of TC proteins. As referred to herein, Class A proteins ("Protein A") are stand-alone toxins. Class B proteins ("Protein B") and Class C proteins ("Protein C") enhance the toxicity of Class A proteins. Examples of Class A proteins are TcbA, TcdA, XptA1 and XptA2. Examples of Class B proteins are TcaC, TcdB, XptBlXb and XptC1Wi. Examples of Class C proteins are TccC, XptClXb and XptB1Wi. Pesticidal proteins also include spider, snake and scorpion venom proteins. Examples of spider venom peptides include but are not limited to lycotoxin-1 peptides and mutants thereof (U.S. Pat. No. 8,334,366).
(C) A polynucleotide encoding an insect-specific hormone or pheromone such as an ecdysteroid and juvenile hormone, a variant thereof, a mimetic based thereon or an antagonist or agonist thereof. See, for example, the disclosure by Hammock, et al., (1990) Nature 344:458, of baculovirus expression of cloned juvenile hormone esterase, an inactivator of juvenile hormone.
(D) A polynucleotide encoding an insect-specific peptide which, upon expression, disrupts the physiology of the affected pest. For example, see the disclosures of, Regan, (1994) J. Biol. Chem. 269:9 (expression cloning yields DNA coding for insect diuretic hormone receptor); Pratt, et al., (1989) Biochem. Biophys. Res. Comm. 163:1243 (an allostatin is identified in Diploptera puntata); Chattopadhyay, et al., (2004) Critical Reviews in Microbiology 30(1):33-54; Zjawiony, (2004) J Nat Prod 67(2):300-310; Carlini and Grossi-de-Sa, (2002) Toxicon 40(11):1515-1539; Ussuf, et al., (2001) Curr Sci. 80(7):847-853 and Vasconcelos and Oliveira, (2004) Toxicon 44(4):385-403. See also, U.S. Pat. No. 5,266,317 to Tomalski, et al., who disclose genes encoding insect-specific toxins.
(E) A polynucleotide encoding an enzyme responsible for a hyperaccumulation of a monoterpene, a sesquiterpene, a steroid, hydroxamic acid, a phenylpropanoid derivative or another non-protein molecule with insecticidal activity.
(F) A polynucleotide encoding an enzyme involved in the modification, including the post-translational modification, of a biologically active molecule; for example, a glycolytic enzyme, a proteolytic enzyme, a lipolytic enzyme, a nuclease, a cyclase, a transaminase, an esterase, a hydrolase, a phosphatase, a kinase, a phosphorylase, a polymerase, an elastase, a chitinase and a glucanase, whether natural or synthetic. *See,* PCT Application WO 1993/02197 in the name of Scott, et al., which discloses the nucleotide sequence of a callase gene. DNA molecules which contain chitinase-encoding sequences can be obtained, for example, from the ATCC under Accession Numbers 39637 and 67152. *See* also, Kramer, et al., (1993) Insect Biochem. Molec. Biol. 23:691, who teach the nucleotide sequence of a cDNA encoding tobacco hookworm chitinase and Kawalleck, et al., (1993) Plant Molec. Biol. 21:673, who provide the nucleotide sequence of the parsley ubi4-2 polyubiquitin gene, and U.S. Pat. Nos. 6,563,020; 7,145,060 and 7,087,810.
(G) A polynucleotide encoding a molecule that stimulates signal transduction. For example, see the disclosure by Botella, et al., (1994) Plant Molec. Biol. 24:757, of nucleotide sequences for mung bean calmodulin cDNA clones, and Griess, et al., (1994) Plant Physiol. 104:1467, who provide the nucleotide sequence of a maize calmodulin cDNA clone.
(H) A polynucleotide encoding a hydrophobic moment peptide. *See,* PCT Application WO 1995/16776 and U.S. Pat. No. 5,580,852 disclosure of peptide derivatives of Tachyplesin which inhibit fungal plant pathogens) and PCT Application WO 1995/18855 and U.S. Pat. No. 5,607,914 (teaches synthetic antimicrobial peptides that confer disease resistance).
(I) A polynucleotide encoding a membrane permease, a channel former or a channel blocker. For example, see the disclosure by Jaynes, et al., (1993) Plant Sci. 89:43, of heterologous expression of a cecropin-beta lytic peptide analog to render transgenic tobacco plants resistant to Pseudomonas solanacearum.
(J) A gene encoding a viral-invasive protein or a complex toxin derived therefrom. For example, the accumulation of viral coat proteins in transformed plant cells imparts resistance to viral infection and/or disease development effected by the virus from which the coat protein gene is derived, as well as by related viruses. See, Beachy, et al., (1990) Ann. Rev. Phytopathol. 28:451. Coat protein-mediated resistance has been conferred upon transformed plants against alfalfa mosaic virus, cucumber mosaic virus, tobacco streak virus, potato virus X, potato virus Y, tobacco etch virus, tobacco rattle virus and tobacco mosaic virus. Id.
(K) A gene encoding an insect-specific antibody or an immunotoxin derived therefrom. Thus, an antibody targeted to a critical metabolic function in the insect gut would inactivate an affected enzyme, killing the insect. Cf. Taylor, et al., Abstract #497, SEVENTH INT'L SYMPOSIUM ON MOLECULAR PLANT-MICROBE INTERACTIONS (Edinburgh, Scotland, 1994) (enzymatic inactivation in transgenic tobacco via production of single-chain antibody fragments).
(L) A gene encoding a virus-specific antibody. See, for example, Tavladoraki, et al., (1993) Nature 366:469, who show that transgenic plants expressing recombinant antibody genes are protected from virus attack.
(M) A polynucleotide encoding a developmental-arrestive protein produced in nature by a pathogen or a parasite. Thus, fungal endo alpha-1,4-D-polygalacturonases facilitate fungal colonization and plant nutrient release by solubilizing plant cell wall homo-alpha-1,4-D-galacturonase. See, Lamb, et al., (1992) Bio/Technology 10:1436. The cloning and characterization of a gene which encodes a bean endopolygalacturonase-inhibiting protein is described by Toubart, et al., (1992) Plant J. 2:367.
(N) A polynucleotide encoding a developmental-arrestive protein produced in nature by a plant. For example, Logemann, et al., (1992) Bio/Technology 10:305, have shown that transgenic plants expressing the barley ribosome-inactivating gene have an increased resistance to fungal disease.
(O) Genes involved in the Systemic Acquired Resistance (SAR) Response and/or the pathogenesis related genes. Briggs, (1995) Current Biology 5(2), Pieterse and Van Loon, (2004) Curr. Opin. Plant Bio. 7(4):456-64 and Somssich, (2003) Cell 113(7):815-6.
(P) Antifungal genes (Cornelissen and Melchers, (1993) Pl. Physiol. 101:709-712 and Parijs, et al., (1991) Planta 183:258-264 and Bushnell, et al., (1998) Can. J. of Plant Path. 20(2):137-149. Also see, U.S. patent application Ser. Nos. 09/950,933; 11/619,645; 11/657,710; 11/748,994; 11/774,121 and U.S. Pat. Nos. 6,891,085 and 7,306,946. LysM Receptor-like kinases for the perception of chitin fragments as a first step in plant defense response against fungal pathogens (US 2012/0110696).
(Q) Detoxification genes, such as for fumonisin, beauvericin, moniliformin and zearalenone and their structurally related derivatives. For example, see, U.S. Pat. Nos. 5,716,820; 5,792,931; 5,798,255; 5,846,812; 6,083,736; 6,538,177; 6,388,171 and 6,812,380.
(R) A polynucleotide encoding a Cystatin and cysteine proteinase inhibitors. See, U.S. Pat. No. 7,205,453.
(S) Defensin genes. See, WO 2003/000863 and U.S. Pat. Nos. 6,911,577; 6,855,865; 6,777,592 and 7,238,781.
(T) Genes conferring resistance to nematodes. See, e.g., PCT Application WO 1996/30517; PCT Application WO 1993/19181, WO 2003/033651 and Urwin, et al., (1998) Planta 204:472-479, Williamson, (1999) Curr Opin Plant Bio. 2(4):327-31; U.S. Pat. Nos. 6,284,948 and 7,301,069 and miR164 genes (WO 2012/058266).
(U) Genes that confer resistance to Phytophthora Root Rot, such as the Rps 1, Rps 1-a, Rps 1-b, Rps 1-c, Rps 1-d, Rps 1-e, Rps 1-k, Rps 2, Rps 3-a, Rps 3-b, Rps 3-c, Rps 4, Rps 5, Rps 6, Rps 7 and other Rps genes. See, for example, Shoemaker, et al., Phytophthora Root Rot Resistance Gene Mapping in Soybean, Plant Genome IV Conference, San Diego, Calif. (1995).
(V) Genes that confer resistance to Brown Stem Rot, such as described in U.S. Pat. No. 5,689,035.
(W) Genes that confer resistance to Colletotrichum, such as described in US Patent Application Publication US 2009/0035765. This includes the Rcg locus that may be utilized as a single locus conversion.

### ii. Transgenes that Confer Resistance to a Herbicide.

(A) A polynucleotide encoding resistance to a herbicide that inhibits the growing point or meristem, such as an imidazolinone or a sulfonylurea. Exemplary genes in this category code for mutant ALS and AHAS enzyme as described, for example, by Lee, et al., (1988) EMBO J. 7:1241 and Miki, et al., (1990) Theor. Appl. Genet. 80:449, respectively. See also, U.S. Pat. Nos. 5,605,011; 5,013,659; 5,141,870; 5,767,361; 5,731,180; 5,304,732; 4,761,373; 5,331,107; 5,928,937 and 5,378,824; U.S. patent application Ser. No. 11/683,737 and International Publication WO 1996/33270.
(B) A polynucleotide encoding a protein for resistance to Glyphosate (resistance imparted by mutant 5-enolpyruvl-3-phosphikimate synthase (EPSP) and aroA genes, respectively) and other phosphono compounds such as glufosinate (phosphinothricin acetyl transferase (PAT) and Streptomyces hygroscopicus phosphinothricin acetyl transferase (bar) genes), and pyridinoxy or phenoxy proprionic acids and cyclohexones (ACCase inhibitor-encoding genes). See, for example, U.S. Pat. No. 4,940,835 to Shah, et al., which discloses the nucleotide sequence of a form of EPSPS which can confer glyphosate resistance. U.S. Pat. No. 5,627,061 to Barry, et al., also describes genes encoding EPSPS enzymes. See also, U.S. Pat. Nos. 6,566,587; 6,338,961; 6,248,876 B1; 6,040,497; 5,804,425; 5,633,435; 5,145,783; 4,971,908; 5,312,910; 5,188,642; 5,094,945, 4,940,835; 5,866,775; 6,225,114 B1; 6,130,366; 5,310,667; 4,535,060; 4,769,061; 5,633,448; 5,510,471; Re. 36,449; RE 37,287 E and 5,491,288 and International Publications EP 1173580; WO 2001/66704; EP 1173581 and EP 1173582.
   Glyphosate resistance is also imparted to plants that express a gene encoding a glyphosate oxido-reductase enzyme as described more fully in U.S. Pat. Nos. 5,776,760 and 5,463,175. In addition glyphosate resistance can be imparted to plants by the over expression of genes encoding glyphosate N-acetyltransferase. See, for example, U.S. Pat. Nos. 7,462,481; 7,405,074 and US Patent Application Publication Number US 2008/0234130. A DNA molecule encoding a mutant aroA gene can be obtained under ATCC Accession Number 39256, and the nucleotide sequence of the mutant gene is disclosed in U.S. Pat. No. 4,769,061 to Comai. EP Application Number 0 333 033 to Kumada, et al., and U.S. Pat. No. 4,975,374 to Goodman, et al., disclose nucleotide sequences of glutamine synthetase genes which confer resistance to herbicides such as L-phosphinothricin. The nucleotide sequence of a phosphinothricin-acetyl-transferase gene is provided in EP Application Numbers 0 242 246 and 0 242 236 to Leemans, et al.; De Greef, et al., (1989) Bio/Technology 7:61, describe the production of transgenic plants that express chimeric bar genes coding for phosphinothricin acetyl transferase activity. See also, U.S. Pat. Nos. 5,969,213; 5,489,520; 5,550,318; 5,874,265; 5,919,675; 5,561,236; 5,648,477; 5,646,024; 6,177,616 B1 and 5,879,903. Exemplary genes conferring resistance to phenoxy proprionic acids and cyclohexones, such as sethoxydim and haloxyfop, are the Acc1-S1, Acc1-S2 and Acc1-S3 genes described by Marshall, et al., (1992) Theor. Appl. Genet. 83:435.
(C) A polynucleotide encoding a protein for resistance to herbicide that inhibits photosynthesis, such as a triazine (psbA and gs+genes) and a benzonitrile (nitrilase gene). Przibilla, et al., (1991) Plant Cell 3:169, describe the transformation of Chlamydomonas with plasmids encoding mutant psbA genes. Nucleotide sequences for nitrilase genes are disclosed in U.S. Pat. No. 4,810,648 to Stalker and DNA molecules containing these genes are available under ATCC Accession Numbers 53435, 67441 and 67442. Cloning and expression of DNA coding for a glutathione S-transferase is described by Hayes, et al., (1992) Biochem. J. 285:173.
(D) A polynucleotide encoding a protein for resistance to Acetohydroxy acid synthase, which has been found to make plants that express this enzyme resistant to multiple types of herbicides, has been introduced into a variety of plants (see, e.g., Hattori, et al., (1995) Mol Gen Genet. 246:419). Other genes that confer resistance to herbicides include: a gene encoding a chimeric protein of rat cytochrome P4507A1 and yeast NADPH-cytochrome P450 oxidoreductase (Shiota, et al., (1994) Plant Physiol 106:17), genes for glutathione reductase and superoxide dismutase (Aono, et al., (1995) Plant Cell Physiol 36:1687) and genes for various phosphotransferases (Datta, et al., (1992) Plant Mol Biol 20:619).
(E) A polynucleotide encoding resistance to a herbicide targeting Protoporphyrinogen oxidase (protox) which is necessary for the production of chlorophyll. The protox enzyme serves as the target for a variety of herbicidal compounds. These herbicides also inhibit growth of all the different species of plants present, causing their total destruction. The development of plants containing altered protox activity which are resistant to these herbicides are described in U.S. Pat. Nos. 6,288,306 B1; 6,282,837 B1 and 5,767,373 and International Publication WO 2001/12825.
(F) The aad-1 gene (originally from Sphingobium herbicidovorans) encodes the aryloxyalkanoate dioxygenase (AAD-1) protein. The trait confers tolerance to 2,4-dichlorophenoxyacetic acid and aryloxyphenoxypropionate (commonly referred to as "fop" herbicides such as quizalofop) herbicides. The aad-1 gene, itself, for herbicide tolerance in plants was first disclosed in WO 2005/107437 (see also, US 2009/0093366). The aad-12 gene, derived from Delftia acidovorans, which encodes the aryloxyalkanoate dioxygenase (AAD-12) protein that confers tolerance to 2,4-dichlorophenoxyacetic acid and pyridyloxyacetate herbicides by deactivating several herbicides with an aryloxyalkanoate moiety, including phenoxy auxin (e.g., 2,4-D, MCPA), as well as pyridyloxy auxins (e.g., fluoroxypyr, triclopyr).
(G) A polynucleotide encoding a herbicide resistant dicamba monooxygenase disclosed in US Patent Application Publication 2003/0135879 for imparting dicamba tolerance.
(H) A polynucleotide molecule encoding bromoxynil nitrilase (Bxn) disclosed in U.S. Pat. No. 4,810,648 for imparting bromoxynil tolerance.
(I) A polynucleotide molecule encoding phytoene (crtl) described in Misawa, et al., (1993) Plant J. 4:833-840 and in Misawa, et al., (1994) Plant J. 6:481-489 for norflurazon tolerance.

### iii. Transgenes that Confer or Contribute to an Altered Grain Characteristic

(A) Altered fatty acids, for example, by (1) Down-regulation of stearoyl-ACP to increase stearic acid content of the plant. See, Knultzon, et al., (1992) Proc. Natl. Acad. Sci. USA 89:2624 and WO 1999/64579 (Genes to Alter Lipid Profiles in Corn); (2) Elevating oleic acid via FAD-2 gene modification and/or decreasing linolenic acid via FAD-3 gene modification (see, U.S. Pat. Nos. 6,063,947; 6,323,392; 6,372,965 and WO 1993/11245); (3) Altering conjugated linolenic or linoleic acid content, such as in WO 2001/12800; (4) Altering LEC1, AGP, Dek1, Superal1, mil ps, various Ipa genes such as Ipa1, Ipa3, hpt or hggt. For example, see, WO 2002/42424, WO 1998/22604, WO 2003/011015, WO 2002/057439, WO 2003/011015, U.S. Pat. Nos. 6,423,886, 6,197,561, 6,825,397 and US Patent Application Publication Numbers US 2003/0079247, US 2003/0204870 and Rivera-Madrid, et al., (1995) Proc. Natl. Acad. Sci. 92:5620-5624; (5) Genes encoding delta-8 desaturase for making long-chain polyunsaturated fatty acids (U.S. Pat. Nos. 8,058,571 and 8,338,152), delta-9 desaturase for lowering saturated fats (U.S. Pat. No. 8,063,269), Primula .DELTA.6-desaturase for improving omega-3 fatty acid profiles; (6) Isolated nucleic acids and proteins associated with lipid and sugar metabolism regulation, in particular, lipid metabolism protein (LMP) used in methods of producing transgenic plants and modulating levels of seed storage compounds including lipids, fatty acids, starches or seed storage proteins and use in methods of modulating the seed size, seed number, seed weights, root length and leaf size of plants (EP 2404499); (7) Altering expression of a High-Level Expression of Sugar-Inducible 2 (HSI2) protein in the plant to increase or decrease expression of HSI2 in the plant. Increasing expression of HSI2 increases oil content while decreasing expression of HSI2 decreases abscisic acid sensitivity and/or increases drought resistance (US Patent Application Publication Number 2012/0066794); (8) Expression of cytochrome b5 (Cb5) alone or with FAD2 to modulate oil content in plant seed, particularly to increase the levels of omega-3 fatty acids and improve the ratio of omega-6 to omega-3 fatty acids (US Patent Application Publication Number 2011/0191904); and (9) Nucleic acid molecules encoding wrinkled1-like polypeptides for modulating sugar metabolism (U.S. Pat. No. 8,217,223).
(B) Altered phosphorus content, for example, by the (1) introduction of a phytase-encoding gene would enhance breakdown of phytate, adding more free phosphate to the transformed plant. For example, see, Van Hartingsveldt, et al., (1993) Gene 127:87, for a disclosure of the nucleotide sequence of an Aspergillus niger phytase gene; and (2) modulating a gene that reduces phytate content. In maize, this, for example, could be accomplished, by cloning and then re-introducing DNA associated with one or more of the alleles, such as the LPA alleles, identified in maize mutants characterized by low levels of phytic acid, such as in WO 2005/113778 and/or by altering inositol kinase activity as in WO 2002/059324, US Patent Application Publication Number 2003/0009011, WO 2003/027243, US Patent Application Publication Number 2003/0079247, WO 1999/05298, U.S. Pat. No. 6,197,561, U.S. Pat. No. 6,291,224, U.S. Pat. No. 6,391,348, WO 2002/059324, US Patent Application Publication Number 2003/0079247, WO 1998/45448, WO 1999/55882, WO 2001/04147.
(C) Altered carbohydrates affected, for example, by altering a gene for an enzyme that affects the branching pattern of starch or, a gene altering thioredoxin such as NTR and/or TRX (see, U.S. Pat. No. 6,531,648) and/or a gamma zein knock out or mutant such as cs27 or TUSC27 or en27 (see, U.S. Pat. No. 6,858,778 and US Patent Application Publication Number 2005/0160488, US Patent Application Publication Number 2005/0204418). See, Shiroza, et al., (1988) J. Bacteriol. 170:810 (nucleotide sequence of Streptococcus mutant fructosyltransferase gene), Steinmetz, et al., (1985) Mol. Gen. Genet. 200:220 (nucleotide sequence of Bacillus subtilis levansucrase gene), Pen, et al., (1992) Bio/Technology 10:292 (production of transgenic plants that express Bacillus licheniformis alpha-amylase), Elliot, et al., (1993) Plant Molec. Biol. 21:515 (nucleotide sequences of tomato invertase genes), Sogaard, et al., (1993) J. Biol. Chem. 268:22480 (site-directed mutagenesis of barley alpha-amylase gene) and Fisher, et al., (1993) Plant Physiol. 102:1045 (maize endosperm starch branching enzyme II), WO 1999/10498 (improved digestibility and/or starch extraction through modification of UDP-D-xylose 4-epimerase, Fragile 1 and 2, Ref1, HCHL, C4H), U.S. Pat. No. 6,232,529 (method of producing high oil seed by modification of starch levels (AGP)). The fatty acid modification genes mentioned herein may also be used to affect starch content and/or composition through the interrelationship of the starch and oil pathways.
(D) Altered antioxidant content or composition, such as alteration of tocopherol or tocotrienols. For example, see, U.S. Pat. No. 6,787,683, US Patent Application Publication Number 2004/0034886 and WO 2000/68393 involving the manipulation of antioxidant levels and WO 2003/082899 through alteration of a homogentisate geranyl geranyl transferase (hggt).
(E) Altered essential seed amino acids. For example, see, U.S. Pat. No. 6,127,600 (method of increasing accumulation of essential amino acids in seeds), U.S. Pat. No. 6,080,913 (binary methods of increasing accumulation of essential amino acids in seeds), U.S. Pat. No. 5,990,389 (high lysine), WO 1999/40209 (alteration of amino acid compositions in seeds), WO 1999/29882 (methods for altering amino acid content of proteins), U.S. Pat. No. 5,850,016 (alteration of amino acid compositions in seeds), WO 1998/20133 (proteins with enhanced levels of essential amino acids), U.S. Pat. No. 5,885,802 (high methionine), U.S. Pat. No. 5,885,801 (high threonine), U.S. Pat. No. 6,664,445 (plant amino acid biosynthetic enzymes), U.S. Pat. No. 6,459,019 (increased lysine and threonine), U.S. Pat. No. 6,441,274 (plant tryptophan synthase beta subunit), U.S. Pat. No. 6,346,403 (methionine metabolic enzymes), U.S. Pat. No. 5,939,599 (high sulfur), U.S. Pat. No. 5,912,414 (increased methionine), WO 1998/56935 (plant amino acid biosynthetic enzymes), WO 1998/45458 (engineered seed protein having higher percentage of essential amino acids), WO 1998/42831 (increased lysine), U.S. Pat. No. 5,633,436 (increasing sulfur amino acid content), U.S. Pat. No. 5,559,223 (synthetic storage proteins with defined structure containing programmable levels of essential amino acids for improvement of the nutritional value of plants), WO 1996/01905 (increased threonine), WO 1995/15392 (increased lysine), US Patent Application Publication Number 2003/0163838, US Patent Application Publication Number 2003/0150014, US Patent Application Publication Number 2004/0068767, U.S. Pat. No. 6,803,498, WO 2001/79516.

### iv. Genes that Control Male-Sterility

There are several methods of conferring genetic male sterility available, such as multiple mutant genes at separate locations within the genome that confer male sterility, as disclosed in U.S. Pat. Nos. 4,654,465 and 4,727,219 to Brar, et al., and chromosomal translocations as described by Patterson in U.S. Pat. Nos. 3,861,709 and 3,710,511. In addition to these methods, Albertsen, et al., U.S. Pat. No. 5,432,068, describe a system of nuclear male sterility which includes: identifying a gene which is critical to male fertility; silencing this native gene which is critical to male fertility; removing the native promoter from the essential male fertility gene and replacing it with an inducible promoter; inserting this genetically engineered gene back into the plant; and thus creating a plant that is male sterile because the inducible promoter is not "on" resulting in the male fertility gene not being transcribed. Fertility is restored by inducing or turning "on", the promoter, which in turn allows the gene that confers male fertility to be transcribed. Non-limiting examples include: (A) Introduction of a deacetylase gene under the control of a tapetum-specific promoter and with the application of the chemical N-Ac-PPT (WO 2001/29237); (B) Introduction of various stamen-specific promoters (WO 1992/13956, WO 1992/13957); and (C) Introduction of the barnase and the barstar gene (Paul, et al., (1992) Plant Mol. Biol. 19:611-622). For additional examples of nuclear male and female sterility systems and genes, see also, U.S. Pat. Nos. 5,859,341; 6,297,426; 5,478,369; 5,824,524; 5,850,014 and 6,265,640.

### v. Genes that Create a Site for Site Specific DNA Integration.

This includes the introduction of FRT sites that may be used in the FLP/FRT system and/or Lox sites that may be used in the Cre/Loxp system. For example, see, Lyznik, et al., (2003) Plant Cell Rep 21:925-932 and WO 1999/25821. Other systems that may be used include the Gln recombinase of phage Mu (Maeser, et al., (1991) Vicki Chandler, The Maize Handbook ch. 118 (Springer-Verlag 1994), the Pin recombinase of E. coli (Enomoto, et al., 1983) and the R/RS system of the pSRi plasmid (Araki, et al., 1992).

### vi. Genes that affect abiotic stress resistance

Including but not limited to flowering, ear and seed development, enhancement of nitrogen utilization efficiency, altered nitrogen responsiveness, drought resistance or tolerance, cold resistance or tolerance and salt resistance or tolerance and increased yield under stress. Non-limiting examples include: (A) For example, see: WO 2000/73475 where water use efficiency is altered through alteration of malate; U.S. Pat. Nos. 5,892,009, 5,965,705, 5,929,305, 5,891,859, 6,417,428, 6,664,446, 6,706,866, 6,717,034, 6,801,104, WO 2000/060089, WO 2001/026459, WO 2001/035725, WO 2001/034726, WO 2001/035727, WO 2001/036444, WO 2001/036597, WO 2001/036598, WO 2002/015675, WO 2002/017430, WO 2002/077185, WO 2002/079403, WO 2003/013227, WO 2003/013228, WO 2003/014327, WO 2004/031349, WO 2004/076638, WO 199809521; (B) WO 199938977 describing genes, including CBF genes and transcription factors effective in mitigating the negative effects of freezing, high salinity and drought on plants, as well as conferring other positive effects on plant phenotype; (C) US Patent Application Publication Number 2004/0148654 and WO 2001/36596 where abscisic acid is altered in plants resulting in improved plant phenotype such as increased yield and/or increased tolerance to abiotic stress; (D) WO 2000/006341, WO 2004/090143, U.S. Pat. Nos. 7,531,723 and 6,992,237 where cytokinin expression is modified resulting in plants with increased stress tolerance, such as drought tolerance, and/or increased yield. Also see, WO 2002/02776, WO 2003/052063, JP 2002/281975, U.S. Pat. No. 6,084,153, WO 2001/64898, U.S. Pat. No. 6,177,275 and U.S. Pat. No. 6,107,547 (enhancement of nitrogen utilization and altered nitrogen responsiveness); (E) For ethylene alteration, see, US Patent Application Publication Number 2004/0128719, US Patent Application Publication Number 2003/0166197 and WO 2000/32761; (F) For plant transcription factors or transcriptional regulators of abiotic stress, see, e.g., US Patent Application Publication Number 2004/0098764 or US Patent Application Publication Number 2004/0078852; (G) Genes that increase expression of vacuolar pyrophosphatase such as AVP1 (U.S. Pat. No. 8,058,515) for increased yield; nucleic acid encoding a HSFA4 or a HSFA5 (Heat Shock Factor of the class A4 or A5) polypeptides, an oligopeptide transporter protein (OPT4-like) polypeptide; a plastochron2-like (PLA2-like) polypeptide or a Wuschel related homeobox 1-like (WOX1-like) polypeptide (U. Patent Application Publication Number US 2011/0283420); (H) Down regulation of polynucleotides encoding poly (ADP-ribose) polymerase (PARP) proteins to modulate programmed cell death (U.S. Pat. No. 8,058,510) for increased vigor; (I) Polynucleotide encoding DTP21 polypeptides for conferring drought resistance (US Patent Application Publication Number US 2011/0277181); (J) Nucleotide sequences encoding ACC Synthase 3 (ACS3) proteins for modulating development, modulating response to stress, and modulating stress tolerance (US Patent Application Publication Number US 2010/0287669); (K) Polynucleotides that encode proteins that confer a drought tolerance phenotype (DTP) for conferring drought resistance (WO 2012/058528); (L) Tocopherol cyclase (TC) genes for conferring drought and salt tolerance (US Patent Application Publication Number 2012/0272352); (M) CAAX amino terminal family proteins for stress tolerance (U.S. Pat. No. 8,338,661); (N) Mutations in the SAL1 encoding gene have increased stress tolerance, including increased drought resistant (US Patent Application Publication Number 2010/0257633); (O) Expression of a nucleic acid sequence encoding a polypeptide selected from the group consisting of: GRF polypeptide, RAA1-like polypeptide, SYR polypeptide, ARKL polypeptide, and YTP polypeptide increasing yield-related traits (US Patent Application Publication Number 2011/0061133); and (P) Modulating expression in a plant of a nucleic acid encoding a Class III Trehalose Phosphate Phosphatase (TPP) polypeptide for enhancing yield-related traits in plants, particularly increasing seed yield (US Patent Application Publication Number 2010/0024067).

Other genes and transcription factors that affect plant growth and agronomic traits such as yield, flowering, plant growth and/or plant structure, can be introduced or introgressed into plants, see e.g., WO 1997/49811 (LHY), WO 1998/56918 (ESD4), WO 1997/10339 and U.S. Pat. No. 6,573,430 (TFL), U.S. Pat. No. 6,713,663 (FT), WO 1996/14414 (CON), WO 1996/38560, WO 2001/21822 (VRN1), WO 2000/44918 (VRN2), WO 1999/49064 (GI), WO 2000/46358 (FR1), WO 1997/29123, U.S. Pat. No. 6,794,560, U.S. Pat. No. 6,307,126 (GAI), WO 1999/09174 (D8 and Rht) and WO 2004/076638 and WO 2004/031349 (transcription factors).

### vii. Genes that Confer Increased Yield

Non-limiting examples of genes that confer increased yield are: (A) A transgenic crop plant transformed by a 1-AminoCyclopropane-1-Carboxylate Deaminase-like Polypeptide (ACCDP) coding nucleic acid, wherein expression of the nucleic acid sequence in the crop plant results in the plant's increased root growth, and/or increased yield, and/or increased tolerance to environmental stress as compared to a wild type variety of the plant (U.S. Pat. No. 8,097,769); (B) Over-expression of maize zinc finger protein gene (Zm-ZFP1) using a seed preferred promoter has been shown to enhance plant growth, increase kernel number and total kernel weight per plant (US Patent Application Publication Number 2012/0079623); (C) Constitutive over-expression of maize lateral organ boundaries (LOB) domain protein (Zm-LOBDP1) has been shown to increase kernel number and total kernel weight per plant (US Patent Application Publication Number 2012/0079622); (D) Enhancing yield-related traits in plants by modulating expression in a plant of a nucleic acid encoding a VIM1 (Variant in Methylation 1)-like polypeptide or a VTC2-like (GDP-L-galactose phosphorylase) polypeptide or a DUF1685 polypeptide or an ARF6-like (Auxin Responsive Factor) polypeptide (WO 2012/038893); (E) Modulating expression in a plant of a nucleic acid encoding a Ste20-like polypeptide or a homologue thereof gives plants having increased yield relative to control plants (EP 2431472); and (F) Genes encoding nucleoside diphosphatase kinase (NDK) polypeptides and homologs thereof for modifying the plant's root architecture (US Patent Application Publication Number 2009/0064373).

### IX. Methods of Use

Methods of the invention comprise methods for controlling a pest (i.e., a Coleopteran plant pest, including a *Diabrotica* plant pest, such as, *D. virgifera virgifera, D. barberi, D. virgifera zeae, D. speciosa,* or *D. undecimpunctata howardi*). The method comprises feeding or applying to a pest a composition comprising a silencing element of the invention, wherein said silencing element, when ingested or contacted by a pest (i.e., a Coleopteran plant pest including a *Diabrotica* plant pest, such as, *D. virgifera virgifera, D. barberi, D. virgifera zeae, D. speciosa,* or *D. undecimpunctata howardi),* reduces the level of a target polynucleotide of the pest and thereby controls the pest. The pest can be fed the silencing element in a variety of ways. For example, in one embodiment, the polynucleotide comprising the silencing element is introduced into a plant. As the Coleopteran plant pest or *Diabrotica* plant pest feeds on the plant or part thereof expressing these sequences, the silencing element is delivered to the pest. When the silencing element is delivered to the plant in this manner, it is recognized that the silencing element can be expressed constitutively or alternatively, it may be produced in a stage-specific manner by employing the various inducible or tissue-preferred or developmentally regulated promoters that are discussed elsewhere herein. The silencing element may be expressed in the roots, stalk or stem, leaf including pedicel, xylem and phloem, fruit or reproductive tissue, silk, flowers and all parts therein or any combination thereof.

In another method, a composition comprising at least one silencing element of the invention is applied to a plant, in which case, the silencing element can be formulated in an agronomically suitable and/or environmentally acceptable carrier, which is preferably, suitable for dispersal in fields. In addition, the carrier can also include compounds that increase the half life of the composition. The composition comprising the silencing element may be formulated in such a manner such that it persists in the environment for a length of time sufficient to allow it to be delivered to a pest. The composition can be applied to an area inhabited by a pest. The composition may be applied externally to a plant (i.e., by spraying a field) to protect the plant from pests.

The constructs of the present invention can be stacked with any combination of polynucleotide sequences of interest in order to create plants with a desired trait. A trait, as used herein, refers to the phenotype derived from a particular sequence or groups of sequences. For example, the polynucleotides of the present invention may be stacked with any other polynucleotides encoding polypeptides having pesticidal and/or insecticidal activity, such as other *Bacillus thuringiensis* toxic proteins (described in U.S. Patent Nos. 5,366,892; 5,747,450; 5,737,514; 5,723,756; 5,593,881; and Geiser et al. (1986) Gene 48:109), lectins (Van Damme et al. (1994) Plant Mol. Biol. 24:825, pentin (described in U.S. Patent No. 5,981,722), and the like. The combinations generated can also include multiple copies of any one of the polynucleotides of interest. The polynucleotides of the present invention can also be stacked with any other gene or combination of genes to produce plants with a variety of desired trait combinations including, but not limited to, traits desirable for animal feed such as high oil genes (e.g., U.S. Patent No. 6,232,529); balanced amino acids (e.g., hordothionins (U.S. Patent Nos. 5,990,389; 5,885,801; 5,885,802; and 5,703,409); barley high lysine (Williamson et al. (1987) Eur. J. Biochem. 165:99-106; and WO 98/20122) and high methionine proteins (Pedersen et al. (1986) J. Biol. Chem. 261:6279; Kirihara et al. (1988) Gene 71:359; and Musumura et al. (1989) Plant Mol. Biol. 12:123)); increased digestibility (e.g., modified storage proteins (U.S. Application Serial No. 10/053,410, filed November 7, 2001); and thioredoxins (U.S. Application Serial No. 10/005,429, filed December 3, 2001)).

The polynucleotides of the present invention can also be stacked with traits desirable for disease or herbicide resistance (e.g., fumonisin detoxification genes (U.S. Patent No. 5,792,931); avirulence and disease resistance genes (Jones et al. (1994) Science 266:789; Martin et al. (1993) Science 262:1432; Mindrinos et al. (1994) Cell 78:1089); acetolactate synthase (ALS) mutants that lead to herbicide resistance such as the S4 and/or Hra mutations; inhibitors of glutamine synthase such as phosphinothricin or basta (e.g., bar gene); and glyphosate resistance (EPSPS gene)); and traits desirable for processing or process products such as high oil (e.g., U.S. Patent No. 6,232,529); modified oils (e.g., fatty acid desaturase genes (U.S. Patent No. 5,952,544; WO 94/11516)); modified starches (e.g., ADPG pyrophosphorylases (AGPase), starch synthases (SS), starch branching enzymes (SBE), and starch debranching enzymes (SDBE)); and polymers or bioplastics (e.g., U.S. Patent No. 5.602,321; beta-ketothiolase, polyhydroxybutyrate synthase, and acetoacetyl-CoA reductase (Schubert et al. (1988) J. Bacteriol. 170:5837-5847) facilitate expression of polyhydroxyalkanoates (PHAs)). One could also combine the polynucleotides of the present invention with polynucleotides providing agronomic traits such as male sterility (e.g., see U.S. Patent No. 5.583,210), stalk strength, drought resistance (e.g., U.S. Patent No. 7,786,353), flowering time, or transformation technology traits such as cell cycle regulation or gene targeting (e.g., WO 99/61619, WO 00/17364, and WO 99/25821).

These stacked combinations can be created by any method including, but not limited to, cross-breeding plants by any conventional or TopCross methodology, or genetic transformation. If the sequences are stacked by genetically transforming the plants (i.e., molecular stacks), the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system. See, for example, WO99/25821, WO99/25854, WO99/25840, WO99/25855, and WO99/25853.

The following examples are offered by way of illustration and not by way of limitation.

### EXPERIMENTAL

### Example 1: In vitro transcript dsRNA screening method

A cDNA library was produced from neonate western corn rootworm larvae by standard methods. A selected cDNA clone containing an expressed sequence tag was amplified in a PCR using universal primers to the plasmid backbone and flanking the EST insert. The universal primers also contained T7 RNA polymerase sites. The product of the PCR reaction was used as the template for an in vitro transcription (IVT) reaction to produce long double stranded RNAs. Following enzymatic digestion and removal of the DNA template and single stranded RNA, the IVT reaction products were incorporated into artificial insect diet as described below.

Different target selection strategies were used to identify RNAi active targets with insecticidal activities in corn rootworn diet based assay. cDNA libraries were produced from neonate or midgut of 3rd instar western corn rootworm larvae by standard methods. Randomly selected cDNA clones containing an expressed sequence tag (EST) were amplified in a PCR using target specific primers (forward and reverse Table 1), and provided in the sequence listing included herein, to generate DNA templates. The target specific primers also contain T7 RNA polymerase sites (T7 sequence at 5' end of each primer). Second set of cDNA clones was selected based on homology to known lethal genes from other insects, primarily Drosophila melanogaster. A third set of genes was tested based on involvement in proteasome functions. Identification of these genes was based on a progressive homology search beginning with a list of proteosome genes identified in humans cross referenced to the Tribolium genome database. Hits from Tribolium were then used to parse western corn rootworm sequence database. Proteosome genes were categorized as 26S subunit non ATPase, 26S subunit ATPase, alpha type, and beta type genes.

Region(s) of WCRW genes were produced by PCR followed by in vitro transcription 5 (IVT) to produce long double stranded RNAs. The IVT reaction products are quantified in gel and incorporated into artificial insect diet for first-round IVT screening (FIS) as described below.

### Insect bioassays

dsRNAs were incorporated into standard WCRW artificial diet at a final concentration of 50ppm in a 96 well microtiter plate format. 5 µl of the IVT reaction (300 ng/ul) were added to a given well of a 96 well microtiter plate. 25 µl of molten lowmelt Western corn rootworm diet were added to the sample and shaken on an orbital shaker to mix the sample and diet. Once the diet has solidified, eight wells were used for each RNA sample. Preconditioned 1^{st} instar WCRW (neonate insects were placed on neutral diet for 24 hours prior to transfer to test material) were added to the 96 well microtiter plates at a rate of 3-5 insects/well. Plates were sealed with mylar which was then punctured twice above each well of the microtiter plate using a superfine insect collection pin. To prevent drying of the diet, plates were first placed inside a plastic bag with a slightly damp cloth and the bags were placed inside an incubator set at 28 °C and 70% RH. The assay was scored for mortality and stunting affects after 7 days and an average was determined based on assignment of numeric values to each category of impact (3=mortality, 2=severe stunting, 1=stunting, 0= no affect). The number reported in this and all diet assay tables reflect the average score across all observations. A score of 3 represents complete mortality across all observations. A score of 2.5 would indicate half the wells demonstrating mortality and half scored as severe stunting. The assay results can be found in Table 1A.

DNA sequences which encode double stranded RNAs which were shown to have insecticidal activity (average score above 1.5) against corn rootworms using the assay described in Example 1 are listed in Table 1. To identify full length of cDNA or full open-reading frame of RNAi active target gene, full insert sequencing for EST clones and transcriptome analyses of midgut RNA samples were conducted. Sequences of all target transcripts containing full length cDNA or longer transcripts were also listed in Table 1. Some of these sequences were used for RNAi active fragment search.

### Example 2. Sequences Having Insecticidal Activity

DNA sequences which encode double stranded RNAs which were shown to have insecticidal activity against corn rootworms using the assay described in Example 1 are set forth below. Non-limiting examples of target polynucleotides are set forth below in Table 1A and B, and SEQ ID NOS: 574, 575, 576, 728, and fragments thereof, and complements thereof.

Subregions of efficacious dsRNAs were designed to improve insecticidal activities in diet and dsRNA expression in planta. These fragments were assayed in the same manner as the original FIS assays described above. Regions demonstrating a severe impact on larval phenotype (mortality or severe growth retardation) were advanced to informal inhibitory concentration (IC₅₀) assays. IC₅₀ assays used doses starting at 50 ppm and progressing downward by ½ step dilutions through 25, 12.5, 6, 3, 1.5, and 0.75pppm. 12 observations were included for each rate. Assay methods were the same as described above for primary screens. Calculations of inhibition relied on scoring for both mortality and severe stunting. Selected fragments were advanced to formal dose response assays where both LC₅₀ and IC₅₀ values were calculated and described in Table 2 (Seq No. 561 to 728). These assays included an initial range finding assay followed by dose response assays for selected ranges including 3 replications of the experiment. Fragments with confirmed IC₅₀ values below 2 ppm were advanced to plant transformation vector construction.

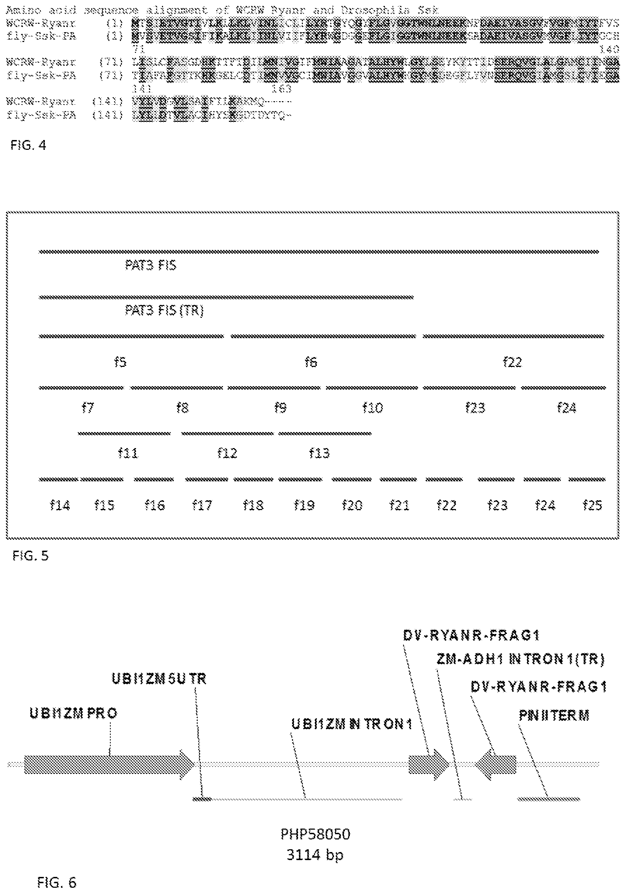

The proteosome alpha subunit type 3 (PAT3) target gene was used as a model for gene and construct optimization. As a first step, the gene was divided into 1/3, 1/6, and 1/12 size fragments (f). In addition, f11-13 represent spanning segments over the boundaries of the first four 1/6^{th} fragments. Figure 5 provides a diagram of the fragments of PAT3.

Plant preferred fragments were identified from active RNAi gene targets and tested in dsRNA artificial diet assays. Selection of these plant preferred regions was based on avoiding destabilizing elements and motifs, or regions with unsuitable base composition. Homology assessments were also employed to avoid potential non target organisms. Finally, fragments with a size range of 150-250 bp were preferred. All rules were considered in selecting fragments but fragments were not excluded from consideration based on any one rule. The Table 2 includes data for initial FIS samples and subsequent fragments insecticidal assay. Selected samples were advanced to IC₅₀ and LC₅₀ determinations.

### Example 3. Identify RNAi active targets from other insects

To identify RNAi active genes from other important corn pests or no-target insects, transcriptome experiments were completed using 3rd instar larvae from Northern corn rootworm (Diabrotica barberi), Southern corn rootworm (*Diabrotica undecimpunctata*), Mexican Bean Beetle (*Epilachna varivestis*), Colorado potato beetle (*Leptinotarsa decemlineata*), Insidious flower bug (*Orius insidiosus*) and Spotted Lady Beetle (*Coleomegilla maculata* , [CMAC]). Homologous transcripts of RNAi active leads were listed in Table 3 (Seq No.693 to 723). This sequence data is important for designing fragments to suppress target pest genes and avoid knockdown same gene in no target insects.

### Example 4. Insecticidal RNA targets in WCRW midgut

Two RNAi active targets Ryanr and HP2 (Table 1 and Table 2) were identified through random cDNA FIS screening. Ryanr was identified in a previous FIS screening (US 2011/0054007A1). Fragments of these targets showed very strong insecticidal activities. Homologous searches reveal that Ryanr and HP2 showed 54% and 49% identity to Drosophila Ssk and Mesh, respectively. The Mesh-Ssk protein complex is required for septate junction formation in the Drosophila midgut. See the amino acid sequence alignment of WCRW Ryanr and Drosophila Ssk in Figure 4.

### Example 5. Transformation of Maize

Immature maize embryos from greenhouse donor plants are bombarded with a plasmid containing the silencing element of the invention operably linked to either a tissue specific, tissue selective, or constitutive promoter and the selectable marker gene PAT (Wohlleben et al. (1988) Gene 70:25-37), which confers resistance to the herbicide Bialaphos. The constructs may express a long double stranded RNA of the target sequence set forth in Table 3. Such a construct can be linked to the UBIZM promoter. Alternatively, the selectable marker gene is provided on a separate plasmid. Transformation is performed as follows. Media recipes follow below.

### Preparation of Target Tissue

The ears are husked and surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5cm target zone in preparation for bombardment.

A plasmid vector comprising the silencing element of interest operably linked to either the tissue specific, tissue selective, or constitutive promoter is made. This plasmid DNA plus plasmid DNA containing a PAT selectable marker is precipitated onto 1.1 µm (average diameter) tungsten pellets using a CaCl2 precipitation procedure as follows: 100 µl prepared tungsten particles in water; 10 µl (1 µg) DNA in Tris EDTA buffer (1 µg total DNA); 100 µl 2.5 M CaC12; and, 10 µl 0.1 M spermidine.

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment.

The sample plates are bombarded at level #4 in a particle gun. All samples receive a single shot at 650 PSI, with a total of ten aliquots taken from each tube of prepared particles/DNA.

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity.

Plants are monitored and scored for the appropriate marker, such as the control of a Coleoptera plant pest, such as a *Diabrotica* plant pest and have insecticidal activity. For example, R0 plant roots are fed to western corn rootworm larvae (WCR, Diabrotica virgifera). Transgenic corn roots are handed-off in Petri dishes with MSOD medium containing antibiotics and glyphosate for in vitro selection. Two WCR larvae are infested per root in each dish with a fine tip paintbrush. The dishes are sealed with Parafilm to prevent the larvae from escaping. The assays are placed into a 27° C, 60% RH Percival incubator incomplete darkness. Contamination and larval quality are monitored. After six days of feeding on root tissue, the larvae are transferred to WCR diet in a 96 well plate. The larvae are allowed to feed on the diet for eight days making the full assay fourteen days long. Larval mass and survivorship are recorded for analysis. A one-way ANOVA analysis and a Dunnett's test is performed on the larval mass data to look for statistical significance compared to an untransformed negative control (HC69). WCR larvae stunting is measured after feeding on two events and compared to growth of larvae fed on negative control plants.

In other assays, transgenic corn plants (R0) generated are planted into 10-inch pots containing Metromix soil after reaching an appropriate size. When plants reach the V4 growth stage, approximately 400 Western corn rootworm (WCR, Diabrotica virgifera) eggs are infested into the root zone. Non-transgenic corn of the same genotype is infested at a similar growth stage to serve as a negative control. Eggs are pre-incubated so hatch occurs within 24 hours of infestation. Larvae are allowed to feed on the root systems for 3 weeks. Plants are removed from the soil and washed so that the roots can be evaluated for larval feeding. Root damage is rated using a Node Injury Scale (NIS) to score the level of damage where a 0 indicates no damage, a 1 indicates that one node of roots is pruned to within 1.5 inches, a 2 indicates that 2 nodes are pruned, while a 3 indicates that 3 nodes are pruned. Because the plants being used for evaluation are directly out of tissue culture after transformation and because transformation events are unique, only a single plant is evaluated per event at this time. The plants in the assay that present signs or symptoms of larval feeding indicate that a successful infestation is obtained. Negative control plant roots are moderately to severely damaged averaging whereas roots of the transgenic plants provide substantial control of larval feeding, with about 0.2 or less on the Corn Rootworm Nodal Injury Score ("CRWNIS").

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature). Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H2O following adjustment to pH 5.8 with KOH); 3.0 g/l Gelrite (added after bringing to volume with D-I H2O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCl, 0.10 g/l pyridoxine HCl, and 0.40 g/l glycine brought to volume with polished D-I H2O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H2O after adjusting to pH 5.6); 3.0 g/l Gelrite (added after bringing to volume with D-I H2O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCl, 0.10 g/l pyridoxine HCl, and 0.40 g/l glycine brought to volume with polished D-I H2O), 0.1 g/l myo-inositol, and 40.0 g/l sucrose (brought to volume with polished D-I H2O after adjusting pH to 5.6); and 6 g/l bacto-agar (added after bringing to volume with polished D-I H2O), sterilized and cooled to 60 °C.

### Example 6. Agrobacterium-mediated Transformation of Maize

For *Agrobacterium*-mediated maize transformation with the disclosed polynucleotide constructs comprising a silencing element as disclosed herein, the method of Zhao was employed (US Patent Number 5,981,840 and International Patent Publication Number WO 1998/32326). Briefly, immature embryos were isolated from maize and the embryos contacted with an *Agrobacterium* suspension, where the bacteria were capable of transferring the desired disclosed polynucleotide constructs comprising a silencing element as disclosed herein, e.g. the polynucleotide construct shown in Figure 6, to at least one cell of at least one of the immature embryos (step 1: the infection step). In this step the immature embryos were immersed in an *Agrobacterium* suspension for the initiation of inoculation. The embryos were co-cultured for a time with the *Agrobacterium* (step 2: the co-cultivation step). The immature embryos were cultured on solid medium following the infection step. Following this co-cultivation period an optional resting step was contemplated. In this resting step, the embryos were incubated in the presence of at least one antibiotic known to inhibit *Agrobacterium* growth without a plant transformant selective agent (step 3: resting step). The immature embryos were cultured on solid medium with antibiotic, but without a selecting agent, for *Agrobacterium* elimination and for a resting phase for the infected cells. Next, inoculated embryos were cultured on medium containing a selective agent and growing transformed callus is recovered (step 4: the selection step). The immature embryos were cultured on solid medium with a selective agent resulting in the selective growth of transformed cells. The callus was then regenerated into plants (step 5: the regeneration step), and calli grown on selective medium were cultured on solid medium to regenerate the plants.

### Example 7 Expression of Silencing Elements in Maize

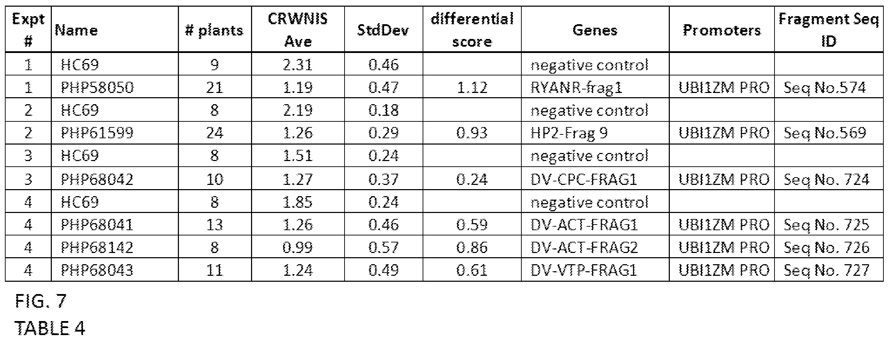

The silencing elements were expressed in a maize plant as hairpins using the transformation techniques described herein above in Example 6, and the plant was tested for insecticidal activity against corn root worms. The data from these studies is shown in Table 4 (Figure 7).

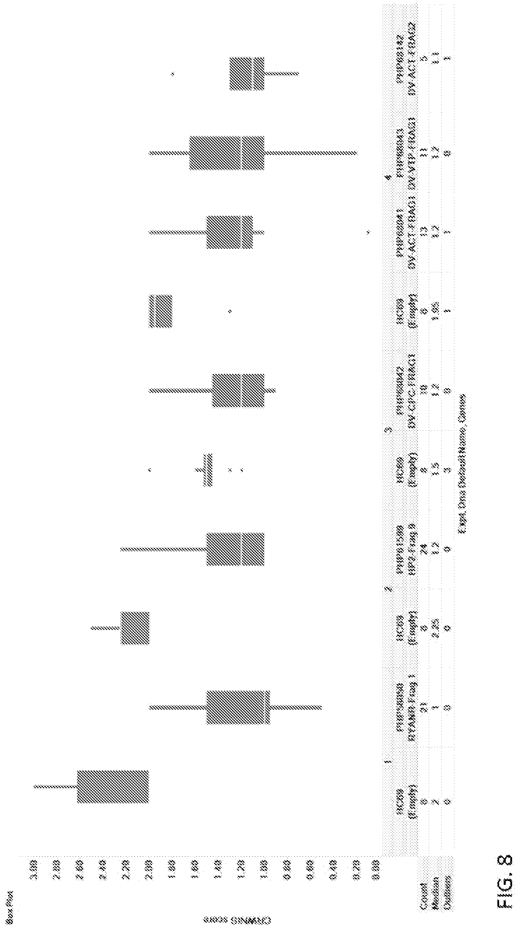

Maize plants were transformed with plasmids containing genes listed in Table 4 (Figure 7), and plants expressing the silencing elements were transplanted from 272V plates into greenhouse flats containing Fafard Superfine potting mix. Approximately 10 to 14 days after transplant, plants (now at growth stage V2-V3) were transplanted into treepots containing Fafard Superfine potting mix. At 14 days post greenhouse send date, plants were infested with 200 eggs of western corn root worms (WCRW)/plant. For later sets, a second infestation of 200 eggs WCRW/plant was done 7 days after the first infestation and scoring was at 14 days after the second infestation. 21 days post infestation, plants were scored using CRWNIS. Those plants with a score of ≤ 0.5 were transplanted into large pots containing SB300 for T1 seed. The data in Table 4 and Figure 8 showed that PHP58050, PHP61599, PHP68041, PHP68142 and PHP68043 showed significant reduced CRWNIS compared to non-transgenic HC6 control plants.

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

All publications and patent applications mentioned in the specification are indicative of the level of those skilled in the art to which this invention pertains.

Although the foregoing invention has been described in some detail by way of illustration and example for purposes of clarity of understanding, certain changes and modifications may be practiced.

## Claims

1. An expression cassette comprising a polynucleotide encoding a sense or antisense strand of a double stranded RNA wherein the polynucleotide consists of:
(a) a nucleotide sequence set forth in any one of SEQ ID NOs: 574, 575, 576, 728, and complements thereof;
(b) a nucleotide sequence having at least 90% sequence identity to any one of nucleotides SEQ ID NOs: 574, 575, 576, 728 and complements thereof; or
(c) at least 19 consecutive nucleotides of any one of SEQ ID NOs: 574, 575, 576, 728 and complements thereof;
wherein said double stranded RNA has insecticidal activity against a Coleoptera plant pest.

2. The expression cassette of claim 1, wherein:
(a) said Coleoptera plant pest is a *Diabrotica* plant pest;
(b) said polynucleotide is operably linked to a heterologous promoter;
(c) said polynucleotide comprises a silencing element which is expressed as a hairpin RNA; or
(d) said polynucleotide is flanked by a first operably linked convergent promoter at one terminus of the polynucleotide and a second operably linked convergent promoter at the opposing terminus of the polynucleotide, wherein the first and the second convergent promoters are capable of driving expression of the polynucleotide.

3. The expression cassette of claim 2(c), wherein the double stranded RNA comprises, in the following order, a first segment, a second segment, and a third segment, wherein:
(a) said first segment consists of a sense or antisense nucleotide sequence having at least 19 nucleotides having at least 90% sequence complementarity to a sequence set forth in any one of SEQ ID NOs: 574, 575, 576, 728, and complements thereof;
(b) said second segment consists of a loop of sufficient length to allow the silencing element to be transcribed as a hairpin RNA; and,
(c) said third segment consists of at least 19 nucleotides having at least 85% complementarity to the first segment.

4. A host cell comprising a heterologous expression cassette of claim 1.

5. A plant cell having stably incorporated into its genome a heterologous polynucleotide encoding a sense or antisense strand of a double stranded RNA, wherein said polynucleotide consists of:
(a) a fragment of at least 19 consecutive nucleotides of any one SEQ ID NOs: 574, 575, 576, 728 and complements thereof; or,
(b) a nucleotide sequence having at least 90% sequence identity to any one of SEQ ID NOs: 574, 575, 576, 728, and complements thereof;
wherein said double stranded RNA, when ingested by a Coleoptera plant pest, reduces the level of a target sequence in said Coleoptera plant pest and thereby controls the Coleoptera plant pest.

6. The plant cell of claim 5, wherein:
(a) the Coleoptera plant pest is a *Diabrotica* plant pest;
(b) said plant cell comprises the expression cassette of claim 3;
(c) said double stranded RNA expresses a hairpin RNA;
(d) said double stranded RNA is operably linked to a heterologous promoter;
(e) said plant cell is from a monocot; optionally wherein said monocot is maize, barley, millet, wheat or rice; or
(f) said plant cell is from a dicot; optionally wherein said plant is soybean, canola, alfalfa, sunflower, safflower, tobacco, *Arabidopsis,* or cotton.

7. The plant cell of claim 5, wherein said double stranded RNA comprises:
(a) a polynucleotide consisting of the sense or antisense sequence of a sequence set forth in any one of SEQ ID NOs: 574, 575, 576, 728, and complements thereof; or
(b) a polynucleotide consisting of at least 130 consecutive nucleotides of the sequence set forth in any one SEQ ID NOs: 574, 575, 576, 728, and complements thereof.

8. The plant cell of claim 6(c), wherein said double stranded RNA comprises, in the following order, a first segment, a second segment, and a third segment, wherein:
(a) said first segment consists of at least 19 nucleotides having at least 90% sequence complementarity to a target sequence set forth in any one of SEQ ID NOs: 574, 575, 576, 728, and complements thereof;
(b) said second segment consists of a loop of sufficient length to allow the silencing element to be transcribed as a hairpin RNA; and,
(c) said third segment consists of at least 19 nucleotides having at least 85% complementarity to the first segment.

9. A plant or plant part comprising a plant cell of claim 5, or a transgenic seed comprising the heterologous polynucleotide as defined in claim 5.

10. A method for controlling a Coleoptera plant pest comprising feeding to a Coleoptera plant pest a composition comprising a double stranded RNA, which when ingested by said Coleoptera plant pest, reduces the level of a target Coleoptera plant pest sequence and thereby controls the Coleoptera plant pest, wherein the sense or antisense sequence of the double stranded RNA consists of a nucleotide sequence having at least 90% sequence identity to the sense or antisense sequence of any one of SEQ ID NOs: 574, 575, 576, 728, and complements thereof.

11. The method of claim 10, wherein:
(I) said Coleoptera plant pest comprises a Diabrotica plant pest; optionally wherein said *Diabrotica* plant pest comprises *D. virgifera virgifera, D. virgifera zeae, D. speciosa, D. barberi, D. virgifera zeae,* or *D*. *undecimpunctata howardi*; or
(II) said composition comprises a plant or plant part having stably incorporated into its genome a polynucleotide comprising said double stranded RNA.

12. The method of claim 11(II), wherein:
(I) said double stranded RNA comprises a hairpin RNA; optionally wherein said polynucleotide encoding the double stranded RNA comprises, in the following order, a first segment, a second segment, and a third segment, wherein:
(a) said first segment consists of at least 19 nucleotides having at least 90% sequence identity to the sense or antisense sequence of any one of SEQ ID NOs: 574, 575, 576, 728, and complements thereof;
(b) said second segment consists of a loop of sufficient length to allow the silencing element to be transcribed as a hairpin RNA; and,
(c) said third segment consists of at least 19 nucleotides having at least 85% complementarity to the first segment;
(II) said silencing element is encoded by a polynucleotide operably linked to a heterologous promoter;
(III) said silencing element is encoded by a polynucleotide, wherein the polynucleotide is flanked by a first operably linked convergent promoter at one terminus of the polynucleotide and a second operably linked convergent promoter at the opposing terminus of the polynucleotide, wherein the first and the second convergent promoters are capable of driving expression of the silencing element;
(IV) said plant is a monocot, optionally wherein said monocot is maize, barley, millet, wheat or rice; or
(V) said plant is a dicot, optionally wherein said plant is soybean, canola, alfalfa, sunflower, safflower, tobacco, *Arabidopsis,* or cotton.

13. An isolated polynucleotide comprising a nucleotide sequence consisting of:
(a) the sequence set forth in any one of SEQ ID NOs: 574, 575, 576, 728, and complements thereof;
(b) the nucleotide sequence having at least 90% sequence identity to any one of nucleotides SEQ ID NOs: 574, 575, 576, 728 and complements thereof; or
(c) at least 19 consecutive nucleotides of any one of SEQ ID NOs: 574, 575, 576, 728, and complements thereof;
wherein said polynucleotide encodes a silencing element which is expressed as a double stranded RNA having insecticidal activity against a Coleoptera plant pest.

14. The isolated polynucleotide of claim 13, wherein said Coleoptera plant pest is a *Diabrotica* plant pest.

15. A double stranded RNA targeting a Coleopteran plant pest target polynucleotide, wherein the double stranded RNA comprises a polynucleotide consisting of:
(a) a sense or antisense sequence of a nucleotide sequence set forth in any one of SEQ ID NO: 574, 575, 576 or 728;
(b) a sense or antisense sequence of a nucleotide sequence having at least 95% sequence identity to SEQ ID NO: 574, 575, 576 or 728; or
(c) a sense or antisense sequence of a nucleotide sequence consisting of at least 25 consecutive nucleotides from nucleotides 50, 25-75,75-125,50-100,125-175, 100-150 or 150-200 of any one of SEQ ID NO: 574, 575, 576 or 728;
wherein said double stranded RNA has insecticidal activity against a Coleopteran plant pest.

16. The double stranded RNA of claim 15, wherein:
(a) said Coleopteran plant pest is a *Diabrotica* plant pest;
(b) the double stranded RNA comprises a hairpin RNA;
(c) the double stranded RNA is expressed in a plant, plant part, or plant cell; or
(d) the double stranded RNA is expressed in a microorganism.

17. A composition comprising the double stranded RNA of claim 15, further comprising an agriculturally acceptable carrier.

## Patentansprüche

1. Expressionskassette, umfassend ein Polynukleotid, das für einen Sense- oder Antisense-Strang einer doppelsträngigen RNA kodiert, wobei das Polynukleotid aus Folgendem besteht:
(a) einer Nukleotidsequenz, die in einer der SEQ ID Nummern: 574, 575, 576, 728 dargelegt ist, und Komplementen davon;
(b) einer Nukleotidsequenz mit mindestens 90 % Sequenzidentität mit einem der Nukleotide der SEQ ID NO: 574, 575, 576, 728 und Komplementen davon;
(c) mindestens 19 aufeinanderfolgenden Nukleotiden von einer der SEQ ID Nummern: 574, 575, 576, 728 und Komplementen davon;
wobei die doppelsträngige RNA eine insektizide Aktivität gegen einen Coleoptera-Pflanzenschädling aufweist.

2. Expressionskassette nach Anspruch 1, wobei:
(a) der Coleoptera-Pflanzenschädling ein *Diabrotica*-Pflanzenschädling ist;
(b) das Polynukleotid operabel mit einem heterologen Promotor verbunden ist;
(c) das Polynukleotid ein Silencing-Element umfasst, das als eine Haarnadel-RNA exprimiert wird; oder
(d) das Polynukleotid von einem ersten operabel verbundenen konvergenten Promotor an einem Terminus des Polynukleotids und einem zweiten operabel verbundenen konvergenten Promotor am entgegengesetzten Terminus des Polynukleotids flankiert wird, wobei der erste und der zweite konvergente Promotor in der Lage sind, die Expression des Polynukleotids anzutreiben.

3. Expressionskassette nach Anspruch 2(c), wobei die doppelsträngige RNA in der folgenden Reihenfolge ein erstes Segment, ein zweites Segment und ein drittes Segment umfasst, wobei:
(a) das erste Segment aus einer Sense- oder Antisense-Nukleotidsequenz mit mindestens 19 Nukleotiden mit mindestens 90 % Sequenzkomplementarität zu einer Sequenz, die in einer der SEQ ID Nummern: 574,575,576, 728 dargelegt ist, und Komplementen davon besteht;
(b) das zweite Segment aus einer Schleife von ausreichender Länge besteht, damit das Silencing-Element als Haarnadel-RNA transkribiert werden kann; und
(c) das dritte Segment aus mindestens 19 Nukleotiden besteht, die eine Komplementarität von mindestens 85 % zum ersten Segment aufweisen.

4. Wirtszelle, die eine heterologe Expressionskassette nach Anspruch 1 umfasst.

5. Pflanzenzelle, die ein heterologes Polynukleotid stabil in ihr Genom integriert hat, das für einen Sense- oder Antisense-Strang einer doppelsträngigen RNA kodiert, wobei das Polynukleotid aus Folgendem besteht:
(a) einem Fragment von mindestens 19 aufeinanderfolgenden Nukleotiden einer der SEQ ID Nummern: 574, 575, 576, 728 und Komplementen davon; oder
(b) einer Nukleotidsequenz mit mindestens 90 % Sequenzidentität mit einer der SEQ ID Nummern: 574, 575, 576, 728 und Komplementen davon;
wobei die doppelsträngige RNA, wenn sie von einem Coleoptera-Pflanzenschädling aufgenommen wird, den Pegel einer Zielsequenz in dem Coleoptera-Pflanzenschädling reduziert und dadurch den Coleoptera-Pflanzenschädling bekämpft.

6. Pflanzenzelle nach Anspruch 5, wobei:
(a) der Coleoptera-Pflanzenschädling ein *Diabrotica*-Pflanzenschädling ist;
(b) die Pflanzenzelle die Expressionskassette nach Anspruch 3 umfasst;
(c) die doppelsträngige RNA eine Haarnadel-RNA exprimiert;
(d) die doppelsträngige RNA operabel mit einem heterologen Promotor verbunden ist;
(e) die Pflanzenzelle von einer Monokotyledone stammt; wobei die Monokotyledone optional Mais, Gerste, Hirse, Weizen oder Reis ist; oder
(f) die Pflanzenzelle von einer Dikotyledone stammt; wobei die Pflanze optional Sojabohne, Raps, Luzerne, Sonnenblume, Saflor, Tabak, *Arabidopsis* oder Baumwolle ist.

7. Pflanzenzelle nach Anspruch 5, wobei die doppelsträngige RNA Folgendes umfasst:
(a) ein Polynukleotid bestehend aus der Sense- oder Antisense-Sequenz einer Sequenz, die in einer von SEQ ID NO: 574, 575, 576, 728 dargelegt ist, und Komplementen davon; oder
(b) ein Polynukleotid bestehend aus mindestens 130 aufeinanderfolgenden Nukleotiden der Sequenz, die in einer von SEQ ID NO: 574, 575, 576, 728 dargelegt ist, und Komplementen davon.

8. Pflanzenzelle nach Anspruch 6(c), wobei die doppelsträngige RNA in der folgenden Reihenfolge ein erstes Segment, ein zweites Segment und ein drittes Segment umfasst, wobei:
(a) das erste Segment aus mindestens 19 Nukleotiden mit mindestens 90 % Sequenzkomplementarität mit einer Zielsequenz, die in einer von SEQ ID NO: 574, 575, 576, 728 dargelegt ist, und Komplementen davon besteht;
(b) das zweite Segment aus einer Schleife von ausreichender Länge besteht, damit das Silencing-Element als Haarnadel-RNA transkribiert werden kann; und
(c) das dritte Segment aus mindestens 19 Nukleotiden besteht, die eine Komplementarität von mindestens 85 % zum ersten Segment aufweisen.

9. Pflanze oder Pflanzenteil, umfassend eine Pflanzenzelle nach Anspruch 5 oder einen transgenen Samen, umfassend das heterologe Polynukleotid, wie definiert im Anspruch 5.

10. Verfahren zur Bekämpfung eines Coleoptera-Pflanzenschädlings, umfassend das Zuführen einer Zusammensetzung an einen Coleoptera-Pflanzenschädling, die eine doppelsträngige RNA umfasst, die, wenn sie von dem Coleoptera-Pflanzenschädling aufgenommen wird, den Pegel einer Ziel-Coleoptera-Pflanzenschädling-Sequenz reduziert und dadurch den Coleoptera-Pflanzenschädling bekämpft, wobei die Sense- oder Antisense-Sequenz der doppelsträngigen RNA aus einer Nukleotidsequenz, die mindestens 90 % Sequenzidentität mit der Sense- oder Antisense-Sequenz einer von SEQ ID NO: 574, 575, 576, 728 aufweist, und Komplementen davon besteht.

11. Verfahren nach Anspruch 10, wobei:
(I) der Coleoptera-Pflanzenschädling einen Diabrotica-Pflanzenschädling umfasst;
wobei der *Diabrotica*-Pflanzenschädling optional *D. virgifera virgifera, D. virgifera zeae, D. speciosa, D. barberi, D. virgifera zeae oder D. undecimpunctata howardi* umfasst; oder
(II) die Zusammensetzung eine Pflanze oder einen Pflanzenteil umfasst, in deren/dessen Genom ein Polynukleotid, das die doppelsträngige RNA umfasst, stabil integriert ist.

12. Verfahren nach Anspruch 11(11), wobei:
(I) die doppelsträngige RNA eine Haarnadel-RNA umfasst; wobei das Polynukleotid, das für die doppelsträngige RNA kodiert, optional in der folgenden Reihenfolge ein erstes Segment, ein zweites Segment und ein drittes Segment umfasst, wobei
(a) das erste Segment aus mindestens 19 Nukleotiden, die mindestens 90 % Sequenzidentität mit der Sense- oder Antisense-Sequenz einer von SEQ ID NO: 574, 575, 576, 728 aufweisen, und Komplementen davon besteht;
(b) das zweite Segment aus einer Schleife von ausreichender Länge besteht, damit das Silencing-Element als Haarnadel-RNA transkribiert werden kann; und
(c) das dritte Segment aus mindestens 19 Nukleotiden mit mindestens 85 % Komplementarität zum ersten Segment besteht;
(II) das Silencing-Element durch ein Polynukleotid kodiert wird, das operabel mit einem heterologen Promotor verbunden ist;
(III) das Silencing-Element durch ein Polynukleotid kodiert wird, wobei das Polynukleotid an einem Terminus des Polynukleotids von einem ersten operabel verbundenen konvergenten Promotor und am entgegengesetzten Terminus des Polynukleotids von einem zweiten operabel verbundenen konvergenten Promotor flankiert wird, wobei der erste und der zweite konvergente Promotor in der Lage sind, die Expression des Silencing-Elements anzutreiben;
(IV) die Pflanze eine Monokotyledone ist, wobei die Monokotyledone optional Mais, Gerste, Hirse, Weizen oder Reis ist; oder
(V) die Pflanze eine Dikotyledone ist, wobei die Pflanze optional Sojabohne, Raps, Luzerne, Sonnenblume, Saflor, Tabak, *Arabidopsis* oder Baumwolle ist.

13. Isoliertes Polynukleotid, umfassend eine Nukleotidsequenz bestehend aus:
(a) der Sequenz, die in einer von SEQ ID NO: 574, 575, 576, 728 dargelegt ist, und Komplementen davon;
(b) der Nukleotidsequenz mit mindestens 90 % Sequenzidentität mit einem der Nukleotide von SEQ ID NO: 574, 575, 576, 728 und Komplementen davon; oder
(c) mindestens 19 aufeinanderfolgenden Nukleotiden von einer von SEQ ID NO: 574, 575, 576, 728 und Komplementen davon;
wobei das Polynukleotid für ein Silencing-Element kodiert, das als doppelsträngige RNA mit insektizider Aktivität gegen einen Coleoptera-Pflanzenschädling exprimiert wird.

14. Isoliertes Polynukleotid nach Anspruch 13, wobei der Coleoptera-Pflanzenschädling ein *Diabrotica-*Pflanzenschädling ist.

15. Doppelsträngige RNA, die auf ein Coleoptera-Pflanzenschädling-Zielpolynukleotid abzielt, wobei die doppelsträngige RNA ein Polynukleotid umfasst, das aus Folgendem besteht:
(a) einer Sense- oder Antisense-Sequenz einer Nukleotidsequenz, die in einer von SEQ ID NO: 574, 575, 576 oder 728 dargelegt ist;
(b) einer Sense- oder Antisense-Sequenz einer Nukleotidsequenz mit mindestens 95 % Sequenzidentität mit SEQ ID NO: 574, 575, 576 oder 728; oder
(c) einer Sense- oder Antisense-Sequenz einer Nukleotidsequenz bestehend aus mindestens 25 aufeinanderfolgenden Nukleotiden aus den Nukleotiden 50, 25-75,75-125,50-100,125-175, 100-150 oder 150-200 von einer von SEQ ID NO: 574, 575, 576 oder 728;
wobei die doppelsträngige RNA eine insektizide Aktivität gegen einen Coleoptera-Pflanzenschädling aufweist.

16. Doppelsträngige RNA nach Anspruch 15, wobei:
(a) der Coleoptera-Pflanzenschädling ein *Diabrotica*-Pflanzenschädling ist;
(b) die doppelsträngige RNA eine Haarnadel-RNA umfasst;
(c) die doppelsträngige RNA in einer Pflanze, einem Pflanzenteil oder einer Pflanzenzelle exprimiert wird; oder
(d) die doppelsträngige RNA in einem Mikroorganismus exprimiert wird.

17. Zusammensetzung, umfassend die doppelsträngige RNA nach Anspruch 15, ferner umfassend einen landwirtschaftlich akzeptablen Träger.

## Revendications

1. Cassette d'expression comprenant un polynucléotide encodant un brin sens ou antisens d'un ARN à double brin, dans laquelle le polynucléotide est constitué de :
(a) une séquence de nucléotides présentée dans un quelconque de SEQ ID n° : 574, 575, 576, 728, et des compléments de ceux-ci ;
(b) une séquence de nucléotides ayant au moins 90 % d'identité de séquence avec un quelconque de nucléotides SEQ ID n° : 574, 575, 576, 728 et des compléments de ceux-ci ; ou
(c) au moins 19 nucléotides consécutifs d'un quelconque de SEQ ID n° : 574, 575, 576, 728 et des compléments de ceux-ci ;
dans laquelle ledit ARN à double brin a une activité insecticide contre un organisme coléoptère nuisible des cultures.

2. Cassette d'expression selon la revendication 1, dans laquelle :
(a) ledit organisme coléoptère nuisible des cultures est un organisme *Diabrotica* nuisible des cultures ;
(b) ledit polynucléotide est fonctionnellement lié à un promoteur hétérologue ;
(c) ledit polynucléotide comprend un élément silencieux qui est exprimé sous forme d'ARN en épingle à cheveux ; ou
(d) ledit polynucléotide est flanqué par un premier promoteur convergent fonctionnellement lié à un terminus du polynucléotide et un second promoteur convergent fonctionnellement lié au terminus opposé du polynucléotide, dans laquelle les premier et second promoteurs convergents sont capables d'entraîner l'expression du polynucléotide.

3. Cassette d'expression selon la revendication 2(c), dans laquelle l'ARN à double brin comprend, dans l'ordre suivant, un premier segment, un deuxième segment, et un troisième segment, dans laquelle :
(a) ledit premier segment est constitué d'une séquence sens ou antisens de nucléotides ayant au moins 19 nucléotides ayant au moins 90 % de complémentarité de séquence avec une séquence présentée dans un quelconque de SEQ ID n° : 574, 575, 576, 728, et des compléments de ceux-ci ;
(b) ledit deuxième segment est constitué d'une boucle de longueur suffisante pour permettre à l'élément silencieux d'être transcrit sous forme d'ARN en épingle à cheveux ; et,
(c) ledit troisième segment est constitué d'au moins 19 nucléotides ayant au moins 85 % de complémentarité avec le premier segment.

4. Cellule hôte, comprenant une cassette d'expression hétérologue selon la revendication 1.

5. Cellule végétale ayant, incorporé de façon stable, dans son génome, un polynucléotide hétérologue encodant un brin sens ou antisens d'un ARN à double brin, dans laquelle ledit polynucléotide est constitué de :
(a) un fragment d'au moins 19 nucléotides consécutifs d'un quelconque de SEQ ID n° : 574, 575, 576, 728 et des compléments de ceux-ci ; ou,
(b) une séquence de nucléotides ayant au moins 90 % d'identité de séquence avec un quelconque de SEQ ID n° : 574, 575, 576, 728, et des compléments de ceux-ci ;
dans laquelle ledit ARN à double brin, lorsqu'il est ingéré par un organisme coléoptère nuisible des cultures, réduit le niveau d'une séquence cible dans ledit organisme coléoptère nuisible des cultures et ainsi contrôle l'organisme coléoptère nuisible des cultures.

6. Cellule végétale selon la revendication 5, dans laquelle :
(a) l'organisme coléoptère nuisible des cultures est un organisme *Diabrotica* nuisible des cultures ;
(b) ladite cellule végétale comprend la cassette d'expression selon la revendication 3 ;
(c) ledit ARN à double brin exprime un ARN en épingle à cheveux ;
(d) ledit ARN à double brin est fonctionnellement lié à un promoteur hétérologue ;
(e) ladite cellule végétale est d'une plante monocotylédone ; optionnellement dans laquelle ladite plante monocotylédone est le maïs, l'orge, le millet, le blé ou le riz ; ou
(f) ladite cellule végétale est d'une plante dicotylédone ; optionnellement dans laquelle ladite plante est le soja, la canola, la luzerne, le tournesol, le carthame, le tabac, l'*arabidopsis,* ou le coton.

7. Cellule végétale selon la revendication 5, dans laquelle ledit ARN à double brin comprend :
(a) un polynucléotide constitué de la séquence sens ou antisens d'une séquence présentée dans un quelconque de SEQ ID n° : 574, 575, 576, 728, et des compléments de ceux-ci ; ou
(b) un polynucléotide constitué d'au moins 130 nucléotides consécutifs de la séquence présentée dans l'un quelconque de SEQ ID n° : 574, 575, 576, 728, et des compléments de ceux-ci.

8. Cellule végétale selon la revendication 6(c), dans laquelle ledit ARN à double brin comprend, dans l'ordre suivant, un premier segment, un deuxième segment, et un troisième segment, dans laquelle :
(a) ledit premier segment est constitué d'au moins 19 nucléotides ayant au moins 90 % de complémentarité de séquence avec une séquence cible présentée dans un quelconque de SEQ ID n° : 574, 575, 576, 728, et des compléments de ceux-ci ;
(b) ledit deuxième segment est constitué d'une boucle de longueur suffisante pour permettre à l'élément silencieux d'être transcrit sous forme d'ARN en épingle à cheveux ; et,
(c) ledit troisième segment est constitué d'au moins 19 nucléotides ayant au moins 85 % de complémentarité avec le premier segment.

9. Plante ou partie de plante, comprenant une cellule végétale selon la revendication 5, ou une graine transgénique comprenant le polynucléotide hétérologue selon la revendication 5.

10. Procédé pour contrôler un organisme coléoptère nuisible des cultures comprenant l'alimentation, à un organisme coléoptère nuisible des cultures, en une composition comprenant un ARN à double brin, qui, lorsqu'il est ingéré par ledit organisme coléoptère nuisible des cultures, réduit le niveau d'une séquence cible d'organisme coléoptère nuisible des cultures et ainsi contrôle l'organisme coléoptère nuisible des cultures, dans lequel la séquence sens ou antisens de l'ARN à double brin est constituée d'une séquence de nucléotides ayant au moins 90 % d'identité de séquence avec la séquence sens ou antisens d'un quelconque de SEQ ID n° : 574, 575, 576, 728, et des compléments de ceux-ci.

11. Procédé selon la revendication 10, dans lequel :
(I) ledit organisme coléoptère nuisible des cultures comprend un organisme *Diabrotica* nuisible des cultures ;
optionnellement dans lequel ledit organisme *Diabrotica* nuisible des cultures comprend *D. virgifera virgifera, D. virgifera zeae, D. speciosa, D. barberi, D. virgifera zeae,* ou *D. undecimpunctata howardi* ; ou
(II) ladite composition comprend une plante ou une partie végétale ayant, incorporé de façon stable dans son génome, un polynucléotide comprenant ledit ARN à double brin.

12. Procédé selon la revendication 11 (II), dans lequel :
(I) ledit ARN à double brin comprend un ARN en épingle à cheveux ; optionnellement dans lequel ledit polynucléotide encodage l'ARN à double brin comprend, dans l'ordre suivant, un premier segment, un deuxième segment, et un troisième segment, dans lequel :
(a) ledit premier segment est constitué d'au moins 19 nucléotides ayant au moins 90 % d'identité de séquence avec la séquence sens ou antisens d'un quelconque de SEQ ID n° : 574, 575, 576, 728, et des compléments de ceux-ci ;
(b) ledit deuxième segment est constitué d'une boucle de longueur suffisante pour permettre à l'élément silencieux d'être transcrit sous forme d'ARN en épingle à cheveux ; et,
(c) ledit troisième segment est constitué d'au moins 19 nucléotides ayant au moins 85 % de complémentarité avec le premier segment ;
(II) ledit élément silencieux est encodé par un polynucléotide fonctionnellement lié à un promoteur hétérologue ;
(III) ledit élément silencieux est encodé par un polynucléotide, dans lequel le polynucléotide est flanqué par un premier promoteur convergent fonctionnellement lié à un terminus du polynucléotide et un second promoteur convergent fonctionnellement lié au terminus opposé du polynucléotide, dans lequel les premier et second promoteurs convergents sont capables d'entraîner l'expression de l'élément silencieux ;
(IV) ladite plante est une plante monocotylédone, optionnellement dans lequel ladite plante monocotylédone est le maïs, l'orge, le millet, le blé ou le riz ; ou
(V) ladite plante est une plante dicotylédone, optionnellement dans lequel ladite plante est le soja, la canola, la luzerne, le tournesol, le carthame, le tabac, l'*arabidopsis*, ou le coton.

13. Polynucléotide isolé comprenant une séquence de nucléotides constituée de :
(a) la séquence présentée dans un quelconque de SEQ ID n° : 574, 575, 576, 728, et des compléments de ceux-ci ;
(b) la séquence de nucléotides ayant au moins 90 % d'identité de séquence avec un quelconque de nucléotides SEQ ID n° : 574, 575, 576, 728 et des compléments de ceux-ci ; ou
(c) au moins 19 nucléotides consécutifs d'un quelconque de SEQ ID n° : 574, 575, 576, 728, et des compléments de ceux-ci ;
dans lequel ledit polynucléotide encode un élément silencieux qui est exprimé sous forme d'ARN à double brin ayant une activité insecticide contre un organisme coléoptère nuisible des cultures.

14. Polynucléotide isolé selon la revendication 13, dans lequel ledit organisme coléoptère nuisible des cultures est un organisme *Diabrotica* nuisible des cultures.

15. ARN à double brin ciblant un polynucléotide cible d'organisme coléoptère nuisible des cultures, dans lequel l'ARN à double brin comprend un polynucléotide constitué de :
(a) une séquence sens ou antisens d'une séquence de nucléotides présentée dans un quelconque de SEQ ID n° : 574, 575, 576 ou 728 ;
(b) une séquence sens ou antisens d'une séquence de nucléotides ayant au moins 95 % d'identité de séquence avec SEQ ID n° : 574, 575, 576 ou 728 ; ou
(c) une séquence sens ou antisens d'une séquence de nucléotides constituée d'au moins 25 nucléotides consécutifs des nucléotides 50, 25 à 75, 75 à 125, 50 à 100, 125 à 175, 100 à 150 ou 150 à 200 d'un quelconque de SEQ ID n° : 574, 575, 576 ou 728 ;
dans lequel ledit ARN à double brin a une activité insecticide contre un organisme coléoptère nuisible des cultures.

16. ARN à double brin selon la revendication 15, dans lequel :
(a) ledit organisme coléoptère nuisible des cultures est un organisme *Diabrotica* nuisible des cultures ;
(b) l'ARN à double brin comprend un ARN en épingle à cheveux ;
(c) l'ARN à double brin est exprimé dans une plante, partie végétale, ou cellule végétale ; ou
(d) l'ARN à double brin est exprimé dans un micro-organisme.

17. Composition comprenant l'ARN à double brin selon la revendication 15, comprenant en outre un vecteur acceptable en agriculture.
